# EUROPEAN PATENT APPLICATION

(11) **EP 1 785 729 A2**
(43) Date of publication of application: **16.05.2007**
(21) Application number: 06076889.2
(22) Date of filing: 16.10.2006
(51) Int. Cl.: G01N 33/68

(54) **A method for the selective isolation of multiply-charged peptides applicable in the quantitative proteomics**

(30) Priority: 17.10.2005 CU 1972005
(71) Applicant: CENTRO DE INGENIERIA GENETICA Y BIOTECNOLOGIA (CIGB), Ciudad de la Habana 10600 (CU)
(72) Inventor: Puente, Aniel Sanchez, Alamar, 11300 Ciudad de la Habana (CU); Gonzalez Lopez, Luis Javier, Municipio Playa, 12100 Habana (CU); Gil Valdes, Jeovanis, Arroyo Naranjo, 10900 Habana (CU); Betancourt Nunez, Lazaro Hiram, 12100 Ciudad de la Habana (CU); Besada Perez, Vladimir Armando, Habana del Este, 19140 Habana (CU); Fernandez de Cossio Dorta-Duque, Jorge, 11300, Ciudad de la Habana (CU); Alvarez Gil, Félix Modesto, Playa, 10600, Ciudad de la Habana (CU); Padron Palomares, Gabriel Ramon, Playa, 12100, Ciudad de la Habana (CU)
(74) Representative: van Loon, C.J.J.

(57) **Abstract**

The present invention describes a method that combines the blocking of primary amino groups and the cation exchange chromatography, to simplify peptide mixtures that can be generated or not by proteolytic or chemical treatments. This method allows the selective isolation of an average of 4 multiply-charged peptides (RH peptides) per protein and the study of 90% of the proteins of the analyzed proteomes. It is applicable to studies of quantitative proteomics without the usage of two-dimensional electrophorsis, it is compatible with any type of isotopic labeling and it is very useful to determine the differential expression of proteins present in multiple conditions (3 to 6 conditions) when using different isotopic labeling in a single experiment. The chromatographic system used also allows the fractionation fraction of the RH peptides to achieve the identification of a greater number of proteins.

## Description

### DESCRIPTIVE MEMORY

The present invention is related with the biotechnology field, particularly with the proteomics. General speaking, we can define proteomics as a set of tools, techniques, interrelated methods for studying the proteomes. The term proteome is used to define the protein complement of the genome.

Today the combination of separation technologies, mainly two-dimensional gel electrophoresis (2DE) in combination with mass spectrometry and the automatic database search have made possible the highthroughout identification of proteins in complex mixtures. 2DE is the most resolving technique for separating complex mixture of proteins, however it has several limitations (Membrane proteins and proteomics: Un amour impossible. Santoni V., Molloy M., Rabilloud T. Electrophoresis. 21, 1054-1070, 2000; Proteome profiling-pitfall and progress. Haynes P.A., Yates III J.R. Yeast. 17, 81-87, 2000):
- Difficulties in the analysis of very hydrophobic proteins. For example, it is well known that membrane proteins which are always attractive candidates for the vaccine development are under-represented in 2DE gels.
- Proteins with extreme p/ can not be focalized in an effective manner.
- The obtantion of reproducible and high quality 2DE gels require high laboriousity and manual skill of the specialist.
- It cannot be directly coupled to mass spectrometers so the high throughput analysis cannot be achievable in a reasonable time.
- The automatic image analysis for detecting differentially expressed proteins is not efficient enough to avoid the manual analysis of an specialist and it is time consuming.

The fact that the sequencing by mass spectrometry of only 3 to 5 amino acids of a peptide is enough to carry out a reliable identification of the source protein (Error-tolerant identification of peptides in sequence databases by peptide sequence tags. Mann M, Wilm M., Anal. Chem. 1994, 66, 4390-4399) and that the proteolytic digestion of a hydrophobic protein can even generate non-hydrophobic peptides easily to be handled, makes preferable the manipulation of peptides instead of intact proteins, arising variants to carry out proteomics without using two-dimensional electrophoresis and allowing a high throughput identification of a great number of proteins in the analyzed mixtures.

The first step towards this direction was done in 1999 by Link *et al.* (Link, A.J. et al. Direct analysis of protein complexes using mass spectrometry. Nat. Biotechnol. 1999, 17, 676-682) when a liquid chromatography system was directly coupled to a mass spectrometer (LC-MS/MS). They packed a microcapilar column followed by a reverse-phase column. Initially, at acidic pH all peptides are retained in the cation exchanger and fractions of these peptides are eluted in batches by increasing the saline concentration of the movile phase. Then, a continuous gradient of acetonitrile, elutes the peptides retained in the reverse phase column and are analyzed by mass spectrometry in order to proceed for their identification in sequence database. This procedure is repeated several times until a complete elution of the peptides retained in the cation exchange chromatography column. This procedure known as MudPiT (Multidimensional Protein Identification Technology) has allowed the identification of 1484 proteins from the total hydrolyzed from *Saccharomyces cerevisiae* (Washburn M. P. et al. Large-scale analysis of the yeast proteome by multidimensional protein identification technology, Nature Biotechnology, 2001, 19, 242-247). This bidimensional fractionation is essential for identifying a great number of proteins due to the complexity of the analyzed peptide mixture and it has promoted the high throughput identification of proteins by using liquid chromatography coupled to mass spectrometry.

However, the relative quantification of proteins in complex mixtures cannot be easily achieved. The first step to solve this problem was done by Washburn MP et al. (Analysis of quantitative proteomic data generated via multidimensional protein identification technology. Anal. Chem. 2002, 74, 1650-1656) when *Saccharomyces cerevisiae* were grown in the normal culture medium containing nitrogen-14 (¹⁴N) and the other cells in an enriched medium containing nitrogen-15 (¹⁵N). By this procedure, all the proteins derived from one conditions are labeled with ¹⁵N while the proteins of the other condition are labeled with ¹⁴N. The proteins derived from both conditions are mixed, and proceed to protein identification through the sequencing of peptides by mass spectrometry. The determination of the relative quantities of the proteins is based on the intensity ratio of the corresponding peptides labeled with ¹⁵N/¹⁴N.

This approach based on the isotopic labeling is not always possible, and only can be performed in simple organisms such as yeast, and bacteria because of high costs of the isotopic ally enriched culture media. On the other hand, it is important to point out, that the ¹⁵N-labeling introduces certain limitations in the identification process because there is a variable mass shifts between the labeled and non-labeled peptides according the number of nitrogen atoms in their sequences.

To overcome this last limitation, other authors restricted the introduction of the isotopic labeling to certain amino acids when the organism under study is grown in a culture medium depleted in a natural amino acid but it is supplemented with its corresponding labeled amino acid.

This strategy is know as SILAC (stable isotope labeling by amino acids in cell culture) and there is a considerable number of publications using the labeling with ¹²C/¹³C y ¹H/²H in culture media supplemented with labeled and non-labeled leucine, arginine or lysine in the two compared conditions (S.E. Ong, B. Blagoev, I. Kratchmarova, D.B. Kristensen, H. Steen, A. Pandey, M. Mann, Mol. Cell Proteomics. 2002, 1, 376-386; Berger SJ, Lee SW, Anderson GA, Lijiana PT, Tolić N, Shen Y, Zhao R, Smith RD High-throughput Global Peptide Proteomic Analysis by Combining Stable Isotope Amino Acid Labeling and Data-Dependent Multiplexed-MS/MS. Analytical Chemistry 2002, 74, 4994-5000; and Precise peptide sequencing and protein quantification in the human proteome through in vivo lysine-Specific Mass Tagging, J. Am. Soc. Mass Spectrom. 2003, 14, 1-7). The mass shift of the light amino acid respect to the heavy amino acid, only is detected if the peptide contain the labeled amino acid, therefore peptides that do not contain the labeled amino acid cannot be used for quantification. SILAC cannot be used universally in all proteomics experiments because its high costs and it is only applicable to biological problems that are studied in cellular culture.

The most universal method to perform the quantification is the ¹⁸O-labeling. It consists in the proteolytic digestion of the whole proteins from one condition in the presence of normal water (H₂O) while the proteins from the other condition are hydrolyzed in presence of ¹⁸O-labeled water (H₂¹⁸O). The peptides obtained in the buffer prepared with H₂¹⁸O can incorporate either one or two ¹⁸O at their C-terminal end, on the contrary, the other peptides show their natural isotopic ion distribution. To perform the quantification, the labeled and non-labeled peptides are mixed and the ratio of the area corresponding to the isotopic ion distribution of the species labeled with ¹⁶O/¹⁸O in the mass spectra is proportional to the relative concentration of protein that originated them in the compared samples.

This method have two limitations: (1) it does not yield a sufficient separation between the isotopic ion distributions of the labeled and non-labeled peptides and (2) the addition of ¹⁸O is not homogeneous, adding one or two ¹⁸O atoms. Both problems make difficult the relative quantification of the species unless software allows the appropriate interpretation of the complex overlapping of the isotopic ion distributions.

To overcome this problem Yao *et. al* (Yao X, Afonso C and Fenselau C. Dissection of proteolytic 18O-labeling: endoprotease-catalyzed 16O-to-18O exchange of truncated peptide substrates. J. Proteome Res. 2003, 2, 147-52) proposed the complete labeling of peptides with ¹⁸O after the proteolytic digestion by a extended incubation of the peptides in the presence of trypsin in order to guaranteed the complete incorporation of two ¹⁸O atoms at the C-terminal end of the peptides and thus exists a separation of 4 Da between the isotopic ion distribution of the labeled and non-labeled peptides.

However, on one hand, there are peptides that are resistant to the additional incorporation of ¹⁸O once they were generated by the cleavage of trypsin an introduce considerable errors in the quantifications, and, on the other hand, the additional incubation for long times to ensure the complete exchange of ¹⁶O by ¹⁸O may introduce non-specific cleavages in the peptide sequence and it affect the protein identification in the sequence database.

Karen *et al.* implemented a new method named inverse ¹⁸O-labeling (Y.Karen Wang, Zhixian Ma, Douglas F.Quin, W.Fu. Inverse 18O Labeling Mass Spectrometry for the Rapid Identification of Marker/Target Proteins. Anal. Chem. 2001, 73, 3742-3750) where to pool of proteins (composed of eight proteins), the control sample and the test sample were prepared. Each pool of proteins is divided in two equal fractions and digested in different buffers prepared with ¹⁸O and ¹⁶O. After finishing the digestions, they make two pools of mixtures, where each group were represented by the fractions labeled with the different isotopes. This original method allows the simplification in the data analysis because the substraction between both experiments eliminate all the proteins that did not changed their expression level; allow the detection of modification originated due to certain perturbations, is suitable for the high throughput analysis; and simplify the analysis because it did not introduce any restriction to the signal that changed their expression level.

The inverse labeling facilitates the identification of proteins with extreme differential expression, however this method require two experiments and it limitates its applicability because it require higher amount of sample and it is very often a limiting factor in proteomics.

The ¹⁸O-labeling also has been used in quantitative proteomics for the specific ¹⁸O-labeling of N-glycopeptides when one of the samples is deglycosylated in a buffer prepared with H₂¹⁸O and the other in a buffer prepared with normal water (Kuster, B and Mann M. 18O-labeling of N-glycosylation sites to improve the identification of gel-separated glycoprotein's using peptide mass mapping and database searching. Anal. Chem. 1999, 71, 1431-1440). After mixing equal proportion of the analyzed samples, the relative quantities of the individual proteins are estimated in the same way, by the ratio ¹⁶O/¹⁸O present at the sidechain of Asp obtained in the deglycosylation reaction of the N-glycopeptides (Gonzalez J, Takao T, Hori H, Besada V, Rodriguez R, Padron G, Shimonishi Y. A Method for Determination of N-Glycosylation Sites in Glycoprotein's by Collision - Induced Dissociation Analysis in Fast Atom Bombardment Mass Spectrometry: Identification of the Positions of Carbohydrate-Linked Asparagine in Recombinant α-Amylase by treatment with PNGase-F in 18O-labeled Water. Anal. Biochem., 1992, 205, 151-158).

In fact, the high specificity of this method for the N-glycopeptides has been proposed as a strategy to discriminate false-positive identifications (Kuster, B and Mann M. 18O-labeling of N-glycosylation sites to improve the identification of gel-separated glycoprotein's using peptide mass mapping and database searching. Anal. Chem. 1999, 71, 1431-1440) by restricting the database search to peptides containing potential N-glycosylation sites (Asn-X-Ser/Thr).

Another variant to perform a quantitative proteomics was introduced by Zappacosta and Annan (Zappacosta F, and Annan RS. N-terminal isotope tagging strategy for quantitative proteomics: results-driven analysis of protein abundance changes. Anal Chem. 2004, 76, 6618-6627) and it comprises the introduction of the labeling in all proteolytic peptides after transforming all the lysine residues into homoarginines and derivatize all the amino terminal groups of proteolytic peptides obtained in one of the compared conditions with a blocking reagent enriched with heavy isotope (particularly deuterium) while the amino terminal groups of the peptides obtained in the other condition are modified with the non-labeled reagent. After mixing both samples, the quantification is performed after estimating the intensity ratio of the isotopic distribution corresponding to the light- and heavy-peptide.

To avoid the overlapping in the isotopic distribution of the labeled and non-labeled species, the derivatization of peptides is performed with deuterated (d₅) and normal propionic anhydride. However, the incorporation of more than 3 atoms of deuterium might introduce errors in the relative quantification of the light and heavy species because of their different retention times in the reversed phase chromatography as demonstrated by Zhang and coworkers (Zhang R, Sioma CS, Wang S, Regnier FE. Fractionation of isotopic ally labeled peptides in quantitative proteomics. Anal Chem. 2001 73: 5142-5149). The errors in the quantification may increase in the same way increase the number of deuterium atoms incorporated in the amino acid sequence of the heavy specie (Zhang R, Sioma CS, Thompson RA, Xiong L, Regnier FE. Controlling deuterium isotope effects in comparative proteomics. Anal Chem. 2002; 74, 3662-3669) and it has been reported that these errors are minimized when the blocking is performed with the ¹³C-labeled reagents (Zhang R, Regnier FE, Minimizing resolution of isotopic ally coded peptides in comparative proteomics. J. Proteome Res. 2002, 1,139-147).

The analysis of the proteolysis of complex protein mixtures constitutes a great challenge since the overhelming number of peptides that are generated, surpasses the resolution power of the current chromatographic systems and the most modern mass spectrometers as well.

To approach this challenge and to carry out quantitative proteomics without the necessity of the usage of the two-dimensional electrophoresis, a trend has been the simplification of the peptide mixture by the development of methods that allow the selective isolation of a subset of peptides that possess a common characteristic, and that its study doesn't affect the representativeness of the original proteins, allowing the characterization the biggest number of proteins present in the initial mixture. The combination of a selective isolation of peptides with appropriate isotope labeling techniques not only allows the identification but also the relative quantification of the proteins present in the compared initial mixtures.

### Selective isolation of cysteine containing peptides

This approach was initiated by Gigy and coworkers (Quantitative analysis of complex protein mixes using isotope-coded affinity tags. Gygi, S. P., Rist, B., Gerber, S. A., Turecek, F., Gelb, M. H., and Aebersold, R. Nat. Biotechnol. 17, 994-999, 1999) when proposing the well-known ICAT method (isotopecode affinity tags) that is based in the selective isolation of cystein-containing peptides. The method combines the affinity chromatography (streptavidinbiotin) and the labeling with the ICAT reagent in its light- and heavy-variants. This reagent consists of three functional elements:
1 - a group that reacts specifically with the thiol groups of the cystein residues.
2 - a group that has an affinity element (biotin) and allows the selective isolation of the peptides that react with ICAT.
3 - an arm that separates the two elements before mentioned, in the heavy variant it has 8 deuterium atoms in its structure (heavy ICAT) and the light variant has 8 atoms of hydrogen (light ICAT). Once the proteins from both conditions are separately reduced in presence of DTT, the free cysteines generated in one of the conditions react with the heavy-ICAT and those generated in the other condition with the light-ICAT. Both mixture of proteins are joined in identical quantities and proceeds the proteolytic digestion. The generated peptides are purified by a streptavidine affinity column and as a consequence, the cystein containing peptides modified with the ICAT reagent are selectively isolated. To proceed to the relative quantification, the relative intensities of the signals corresponding to the peptides labeled with the light and heavy ICAT are measured. The masses of the peptides labeled with these reagents differ in multiples of 8 units of masses depending the number of cysteine residues contained in the sequence.

This methodology presents several limitations:
- The size of the ICAT reagent is considerable and it can affect the ionization efficiency of peptides and the interpretation of the mass spectra.
- The presence of 8 deuterium atoms in the peptide modified with the heavy ICAT can cause big errors in the quantification because its retention time can differ considerably respect to the peptide modified with the light ICAT (Zhang R, Sioma CS, Wang S, Regnier F. Fractionation of isotopic ally labeled peptides in quantitative proteomics. Anal Chem. 2001, 73, 5142-5149) and the intensities ratios are not the same during the elution time of the light and heavy species of the peptide to be quantified.
- The quantification procedure described for the ICAT, is not applicable to other methods of isotopic labeling that do not separate enough the signals to avoid the overlapping of its isotopic distributions.
- If one wants to fractionate the peptide mixture additionally in a similar way as the MudPit, (Washburn M. P. et al. Large-scale analysis of the yeast proteome by multidimensional protein identification technology. Nature Biotechnol. 2001, 19, 242-247) it is necessary to use a chromatography different to the used for the selective isolation of peptides and it causes considerable losses in the manipulation of the samples.
- When using a high affinity chromatography during the selective isolation step, the losses can be considerable for some particular peptides.
- Proteins without cysteines cannot be analyzed by this method.

### Selective isolation of methionine containing peptides.

Another method has been implemented for the selective isolation of methionine-containing peptides by using a combined diagonal chromatography and it is known in the literature as the COFRADIC technology (Gevaert K, Van Damme J, Goethals M, Thomas GR, Hoorelbeke B, Demol H, Martens L, Puype M, Staes E, Vandekerckhove J. Chromatographic isolation of methionine-containing peptides for gel-free proteome analysis: identification of more than 800 Escherichia coli proteins. Mol Cell Proteomics. 2002, 11, 896-903). After the reduction and S-alkylation, all proteins are digested and the peptides are fractionated by reverse phase chromatography and collected in a considerable number of fractions in what the authors denominate as a primary run. Each one of these fractions reacts with a solution of hydrogen peroxide (3%) during 3 minutes, and they are newly analyzed in the same chromatography system under the identical conditions in a second run. The methionine-containing peptides once oxidyzed are isolated selectively because they are transformed in less hydrophobic species, diminish their retention time, so they differ of the rest of the peptides that do not contain methionine whose retention times remain invariable in the second run and they are discarded. According to the authors this method allows a simplification of the complex mixture of peptides in a similar extension to the one obtained by the ICAT and it can be applied to the selective isolation of phosphopeptides, N-terminal peptides of proteins and peptides linked by disulfide bridges (Martens L, Van Damme P, Damme JV, Staes E, Timmerman A, Ghesquiere B, Thomas GR, Vandekerckhove J, Gevaert K. The human platelet proteome mapped by peptide-centric proteomics: To functional protein profile. Proteomics. 2005, 5(12), 3193-3204).

Although the authors outline that the conditions of the oxidation have been optimized to avoid the modification of labile residues like cysteines and tryptophans, if this happened the selectivity of the method would be affected and the simplification degree of the peptide mixture that would be reached would not be similar to that of the ICAT method as the authors claims.

On the other hand, although it is outlined that this method is amenable to be automated, to achieve the selective isolation of all the methionine containing peptides it is necessary to carry out a great number of chromatographic runs and the global yield of the method can it turns be affected.

### Selective isolation of the N-terminal peptides.

A variant of the COFRADIC has also been proposed to isolate selectively the N-terminal peptide of all the proteins (Gevaert K, Goethals M, Martens L, Van Damme J, Staes TO, Thomas GR, Vandekerckhove J. Exploring proteomes and analyzing protein processing by mass spectrometric identification of sorted N-terminal peptides. Nat Biotechnol. 2003; 21, 566-569). The first step of this method consists the blockage of all primary amino groups of the proteins present in the compared complex mixtures, then a specific proteolysis of the modified proteins is performed and by reverse phase chromatography, the peptide mixture is separated in a considerable number of fractions.

The new amino groups of the internal peptides generated during the proteolysis that are present in each one of these fractions react additionally with a highly hydrophobic blocking group and again are separated in the same chromatographic system under conditions identical to that of previously mentioned. The retention time of all internal peptides is increased considerably by the additions of the second blocking reagent, however all the N-terminal end peptides of proteins that were blocked in the first step are selectively isolated when being collected in the same retention time of its original fraction. This strategy have as a disadvantage: to perform a reliable quantification the first step consisting in the blocking of the aminos groups should work in a quantitative way and it is something difficult to achieve when proteins are present in complex mixtures.

Also in this first step during the blocking of amino groups the solubility of the proteins can be diminished considerably to originate precipitations that can affect in turns the quantitativity of the method. Lastly, the fact that a single peptide per protein is isolated is an excessive simplification and it may have a negative impact in the identification and the quantification of the present proteins in the complex mixtures. A method that allows redundancy by isolating a reduced group of 3-4 péptide for proteins can be ideal for proteomics studies without the usage of the two-dimensional electrophoresis since it permits the confirmation of the quantification results by other peptides of the same protein.

### Selective isolation of N-glycopeptides.

It is reported that approximately the 91 percent of the membrane proteins present in the swissprot databases are glycoprotein's (Gahmberg CG, Tolvanen M. Why mammalian cell surface proteins plows glycoprotein's. Trends Biochem. Sci. 1996, 21, 308-311). It has proposed an strategy of work based on the selective isolation of glycopeptides for proteome studies by using the lectin affinity chromatography (Geng M, Zhang X, Bina M, Regnier F. Proteomics of glycoprotein's based on affinity selection of glycopeptides from tryptic digests. J Chromatogr B Biomed Sci Appl. 2001, 752, 293-306; Kaji H, Saito H, Yamauchi AND, Shinkawa T, Taoka M, Hirabayashi J, Kasai K, Takahashi N, Isobe T. Lectin affinity captures, isotope-coded tagging and mass spectrometry to identify N-linked glycoprotein's. Nat. Biotechnol. 2003, 21, 667-672).

The usage of lectins, either a particular lectin or a battery of them immobilized in a chromatographic column, possesses a limitation since it is not able to recognize all the glycoforms present in the sample and to guarantee an efficient selective isolation. To overcome this limitation Zhang and collaborators (Zhang H, Li XJ, Martin DB, Aebersold R. Identification and quantification of N-linked glycoprotein's using hydrazide chemistry, stable isotope labeling and mass spectrometry. Nat Biotechnol. 2003, 21, 627-629) proposed the immobilization of the glycopeptides to a solid support by using the derivatization with hydrazine and then its releasing by the action of the PNGase-F. To carry out this last step in the presence and absence of H₂¹⁸O allows to proceed to the quantification.

This method can be applied to samples of biological interest such as membrane proteins where vaccine and receptor candidates are included.

Also it can be applicable to the serum which is however the most complex proteome, however its applicability is restricted to those samples that are enriched in glycoprotein's.

### Selective isolation of histidine-containing peptides.

The immobilized chelate metal affinity chromatography has been used in proteomics for the selective isolation of peptides that contain histidine. There are several works that evaluate different matrixes and the immobilized metals ions (Ren D, Penner NA, Slentz BE, Inerowicz HD, Rybalko M, Regnier FE. Contributions of commercial sorbents to the selectivity in immobilized metal affinity chromatography with Cu(II). J Chromatogr A. 2004 1031, 87-92) but the results demonstrate that the specificity is still inferior compared with that of the other previously described methods for the selective isolation of peptides (Ren D, Penner NA, Slentz BE, Mirzaei H, Regnier F. Evaluating immobilized metal affinity chromatography for the selection of histidine-containing peptides in comparative proteomics. J Proteome Res. 2003, 2, 321-329; Ren D, Penner NA, Slentz BE, Regnier FE. Histidine-rich peptide selection and quantification in targeted proteomics. J Proteome Res. 2004, 3, 37-45). In fact, variants of chemical modification of peptides before the affinity chromatography have been explored for increasing the specificity.

### Selective isolation of peptides by cation exchange chromatography.

The cation exchange chromatography has been used for the selective isolation of peptides by separating in an easy manner the neutral peptides (zero charge) from the positively-charged peptides (chage: 1+, 2+ ,3+, 4+, etc). Initially it was used to isolate the C-terminal peptide (A new method for the isolation of the C-terminal peptide of proteins by the combination of selective blocking of proteolytic peptides and cation-exchange chromatography. Chapter #12 in: Proteome and Proteome Analysis. (1999) Springer Verlag publishers); and (Isolation and characterization of modified species of a mutated (Cys125-Ala) recombinant human interleukin-2. Moya G, González LJ, Huerta V, García Y. J. Chromatogr. 2002, 971, 129-142) and blocked N-terminal peptides (Selective isolation and identification of N-terminal blocked peptides from tryptic digests. Betancourt L, Besada V, Gonzalez LJ, Morera V, Padron G, Takao T, Shimonishi Y. J. Pept. 2001, 7, 229-237). However, these methods cannot be applied to the massive study of complex mixtures of proteins and proteomes because a single peptide per protein is isolated and for a more reliable quantification of the identified proteins is required the isolation of a bigger number of peptide per proteins, (approximately 3-4 peptides/proteins). On the other hand, if the identification of the proteins is based on a single peptide per protein there is a considerable risk of missing the identification of a high number of proteins since all the peptides do not fragment efficiently in the spectrometer of masses and it is not always possible to obtain structural information. In these two methods of selective isolation, the peptides are obtained as neutral species (chage zero) and they do not have any basic amino acids at their C-terminal end and it does not facilitate the fragmentation in the spectrometer and consequently its identification in the sequence database.

The method for the selective isolation of peptides based on the cation exchange chromatography was also applied in proteomics studies to selectively isolate peptides that do not contain neither histidine nor arginine, denominated by the authors as nHnR peptides (SCAPE: A new tool for the Selective Captures of Peptides in Protein identification. Betancourt L, Gil J, Besada V, González LJ, Fernández-of-Cossio J, García L, Pajón R, Sánchez A, Alvarez F, Census G. J. Proteome Res. 2005, 4, 491-496). This method achieved a considerable simplification of the analyzed peptide mixture in a similar extension to the one achieved by the ICAT (Quantitative analysis of complex protein mixes using isotope-coded affinity tags. Gygi, S. P., Rist, B., Gerber, S. A., Turecek, F., Gelb, M. H., and Aebersold, R. Nat. Biotechnol. 17, 994-999, 1999). The chromatographic system used in this method also separates charged peptides (most of the part composed by peptides that contain arginine and histidine) from neutral composed mainly by the nHnR peptides, completely blocked at their primary amino groups. Before analyzing the nHnR in the mass spectrometer, the attached blocking group is eliminated by means of a hydrolytic treatment to regenerate the free amino groups and to make more favorable and efficient its ionization, fragmentation and consequently its identification in the databases.

This method isolates the nHnR peptides in the non-retained fraction of the cation exchange chromatography and to achieve the identification of a higher number of proteins it requires another chromatographic step for its additional fractionation. These additional chromatographic steps may cause losses during the manipulation and affect the yields of the method.

The discarded fraction in this method is enriched in peptides that possess arginine and histidine. The arginine presumably should be located in the C-terminal end of the peptides because they were generated by the cleavage of trypsin and the histidine residues should be located inside the sequence.

The triptic peptides with these characteristics fragments very well in mass spectrometry because in the ionization process they possess a mobile proton (Paizs B, Suhai S. Towards understanding the tandem mass spectra of protonated oligopeptides: mechanism of amide bond cleavage. J Am Soc Mass Spectrom. 2004 15,103-13; Cordero MM, Houser JJ, Wesdemiotis C. The neutral products formed during backbone fragmentations of protonated peptides in tandem mass spectrometry. Anal. Chem. 1993; 65,1594-1601) within their sequence (located at the histidine) and it can induce multiple fragmentations along the polypeptide chain, having at the same time a fixed proton located at the end C-terminal end of the peptide (located at the arginine). Their ESI-MSMS spectra produced by the collision induced dissociation dissociation experiments is abundant of y¨ₙ series with enough structural information to achieve a highly reliable identification in the databases.

The analysis of peptides with these characteristics is attractive, however, the fraction that is discarded in the SCAPE method is still very complex. The tryptic digest of a complex mixture of proteins of a given proteome generates approximately an average of 18-20 peptides/proteins and if the SCAPE simplifies this complex mixture when isolating selectively 4-5 peptides/protein it implies that the discarded fraction (mentioned above) should have an average of 14-15 peptides/proteins approximately. This last value is excessively high and it is not the optimum to achieve a proteomic study without using two-dimensional electrophoresis and a further simplification of this fraction is still necessary. Therefore, the development of a new 2DE-free method based on the selective isolation of peptides enriched in basic residues: arginine and histidine is required.

In spite of the limitations described for these methods it continues being very necessary to identify and to determine expression levels of the present proteins in complex mixtures, through the selective and specific isolation of a small group of peptides by means of the simplification of the complex mixture to be characterized by mass spectrometry.

### DETAILED DESCRIPTION OF THE INVENTION.

The method for the selective isolation of RH peptides is achieved by combining the blocking of the primary amino groups of proteolytic peptides and a step of cation exchange chromatography at acidic pH. This combination simplifies the complex mixture of peptides by eliminating in an effective and simple way a majority subset of all proteolytic peptides that possess only one or less arginine or hisitidine residues within their sequences (R+H≤1) and it analyzes only a small subset of peptides composed by those that are retained selectively in the cation exchange column and possess multiple positive charges, the peptides that possess in their sequences more than one basic residue arginine or hisitidine (R+H>1).

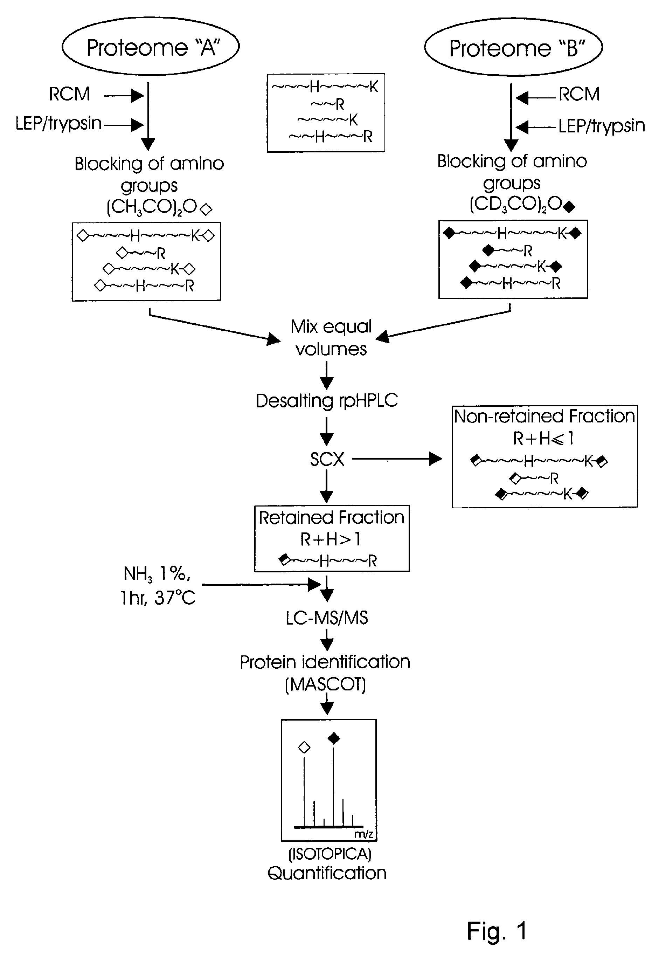

This method can be used for the identification of the proteins constituent of complex mixtures and for the determination of their relative quantities under the compared conditions. For this purpose, the mixture of proteins obtained either by artificial or natural way should be treated according to the steps that are described in the figure 1 and we explain below:
***(1) Reduction and S-alkylation of cyteine residues*** with any of the reagents used for this purpose, for example iodoacetamide, iodoacetic acid, acrylamide, 4-vinylpiridine, etc. This initial step is of a great importance due to several reasons: (a) it assures a bigger efficiency of the next step of the enzymatic hydrolysis of the peptide bonds of proteins present in the analyzed mixture; (b) it avoids the cross-linking of peptides of different proteins that possess cystein residues; (c) it facilitates the identification of the proteins in the databases.
***(2) Hidrolysis of proteins.*** This step can be achieved through an enzymatic digestion using trypsin, or other endoproteinases such as Glu-C, Asp-N, Lys-C, chymotrypsin, termolisin, pepsin, papain, pronase or other proteasas; a chemical hydrolysis can also be used with cyanogen bromide, with organic or inorganic acids. The hydrolysis of the peptide bonds can be carried out by means of the combination of the enzymatic and/or chemical procedures previously described. The peptides generated by these treatments possess an appropriate size for their mass spectrometry identification.
***(3) Blocking Reaction*** consists on the covalent modification of the amino terminal groups and epsilon-amino groups of the lysine residues that contains all the peptides generated by some of the treatments described previously. In this method a wide range of blocking reagents of the amino groups can be used whenever these they do not provide net positive charges to the structure of the modified peptides or they do not possess groups that can be protonated at acidic pH and contribute to the positive charge of the peptide. Among the blocking reagents are: acetic anhydride, N-hydroxysuccinimide, N-acetoxysuccinimide, citacronic anhydride, maleic anhydride, succinic anhydride, ftalic anhydride, tetrahydroftalic anhydride, 9-fluorenylmethyl chloroformate (Fmoc-Cl), 2-methyl sulfonyl ethyl succinimidyl carbonate), urea and reagents that provides protecting amino groups such as: (a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Preferred protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl; (e) photosensitive protective groups which include carbamates derivatives from m-nitrophenyl, 3,5-dimetoxybenzyl, 1-methyl-1(3,5-dimetoxyphenyl)etyl, α-methylnitropiperonyl, o-nitrobenzyl, 3,4-dimetoxy-6-nitrobenzyl, phenyl(o-nitrophenyl)methyl, 2-(2-nitrophenyl)etyl, 6-nitroveratryl, 4-metoxyfenacyl and 3',5'-dimetoxybenzoine In general, all the reagents used in the peptide synthesis for the protection of amino groups or other reagents can also be used if they fulfills the previously explained properties. Examples of this type of reagents and the protocols to carry out the modification of amino groups are easily find in the scientific literature (Protective groups in organic synthesis, Teodora W. Greene and Peter G.M. Wuts, p. 494-654, Ed. John Wiley & Sons, Inc. (1990) and Peptide Chemistry, Bodanszky, N., p. 74-103, Springer-Verlag, New York (1988) and their use is included in the present invention. This blocking reaction of the primary amino groups (alpha-amino terminal and epsilon-amino of lysines) is very important, so that the positive charge of the peptides at acidic pH only depends on the number of two other basic amino acids present in the peptide sequences: arginine (R) and histidine (H).
***(4) Cation exchange chromatography*** of the mixture of modified peptides. The selective isolation of peptides is performed by using a strong cation exchanger at acidic pH (for example a pH between 2 and 4) containing acetonitrile (10 % v/v) in the equilibrium solution. To obtain this pH range, can be used formic, acetic, trifluoroacetic, and heptafluorobutiric acids or other buffers systems or solvents that provides the required pH. This chromatographic step separates in an effective way and with a great selectivity the peptides in two groups: (a) neutral peptides or having a single positive charge (charge 0 and 1+) and (b) multiply-charged peptides (charge 2+, 3+, 4+, etc). For neutral or monocharged peptides it understands those where the sum of the number of arginine residues (R) or hisitidine (H) in their respective sequences is inferior or equal to 1 (R+H ≤ 1). These peptides are not analyzed and they are discarded in the non-retained fraction by the cation exchange column. The multiply-charged ipeptides are those that possess more than one arginine residues and/or histidine within their sequence (R+H>1), they are denominated in the present invention as RH peptides and their selective isolation represents a considerable simplification of the mixture of analyzed peptides without sacrificing considerably the representativeness of the origin proteins (see Figure 1).
   The chromatographic system developed here possesses a double function since it not only allows the simplification of the analyzed complex mixture when the RH peptides are selectively isolated but it also allows its further fractionation by applying a gradient increasing the ionic strength or the pH of the mobile phase before being analyzed by reverse phase chromatography coupled to the mass spectrometer. This additional fractionation by cation exchange chromatography is key for the identification of a great number of proteins (Washburn M. P. et al. Large-scale analysis of the yeast proteome by multidimensional protein identification technology, Nature Biotechnology 2001, 19, 242-247). The method of this invention uses desalting steps in the reverse phase columns before the cation exchange chromatography to eliminate the excess of reagents or to change the working solutions.
***(5) Elimination of side reactions.*** Before being analyzed in the mass spectrometer, the peptides undergo a basic or acidic treatment in presence of a base or acid of organic or inorganic origin to a concentration from 1 to 5% to revert the side reactions in other amino acids (such as tyrosine, serine or threonine) that could have been produced during the blocking reaction. This step can be used additionally to eliminate the blocking group introduced in the step (3) in case of a reversible blocking is used and it is wanted that RH peptides does not have any blocking group at their amino groups before the analysis by mass spectrometry.

To apply this method in the quantitative proteomics it is necessary that the generated peptides in one of the two compared conditions carry one or several heavy isotopes (¹³C, ¹⁵N, ¹⁸O, and/or ²H) in their structure while the peptides generated in the other condition possesses the same elements previously mentioned but with their natural isotopic abundances (¹²C, ¹⁴N, ¹⁶O, and/or ¹H). The elimination of the blocking group of the amino groups of the RH peptides only would be made when this it does not carry the isotopic labeling that is essential to achieve the relative quantification. If the modifier reagent is of a photosensible nature, this can be eliminated by light irradiation of the modified peptides.

The incorporation of the heavy isotopes in the structure of the generated peptides during the proteolysis of proteins in one of the two compared conditions can be carried out in three different ways:
***(a) Isotopic labeling introduced by the cell.*** It is applied to protein extracts from tissues or cells cultivated in media with two isotopic variants of certain essential nutrients for the growing of the cells. The biosintetic machinery incorporates these isotopes in certain amino acids or in all those amino acids that possess the used isotope. Among the used nutrients, labeled compounds that constitute nitrogen source (¹⁴N/¹⁵N); essential amino acids for the cells, labeled in certain positions with isotopes of hydrogen (¹H/²H), nitrogen (¹⁴N/¹⁵N), carbon (¹²C/¹³C), oxygen (¹⁶O/¹⁸O), sulfur, etc. Later on, both extracts of total proteins are mixed, hydrolyzed and the method is continued according to that described in the steps from the 1 to the 5 to achieve the selective isolation of the RH peptides
***(b) Isotope labeling introduced during the proteolysis*.** The protein samples to be compared are hydrolyzed independently, as described in the step 2 of the method, in one case in water enriched with ¹⁸O (H₂ ¹⁸O) and in the other case, with water that possesses their natural isotopic abundance. All poteolytic peptides derived from the first condition are labeled when incorporating one or two atoms of ¹⁸O at their carboxy C-terminal end. Later on, before mixing equal quantities of the digested proteins in both conditions, the proteolytic activity of the enzyme is inhibited by adding of a cocktail of protease inhibitors, or specific inhibitors of the used proteases. The method proceed in the way indicated in the steps 3-5. When this isotopic labeling is used it is advisable a removal of the blocking groups at basic pH because the prolonged treatments of the ¹⁸O-labeled peptides at acidic pH lead to the partial or total loss of the labeling introduced by enzymatic treatment at the carboxy C-terminal end of the peptides and it introduces a great errors in the quantification. This variant of isotopic labeling is frequently used when the heavy isotopes are not introduced at the N-terminal end blocking groups, in other words, when the blocking reagent is just used to modify the primary amino groups of the peptides generated in the compared conditions but not for introducing the labelig responsible for the quantification.
***(c) Isotopic labeling introduced during the blocking reaction*.** The mixtures of proteins to be compare are digested with a protease separately according to the step 2 of the proposed method and the primary amino groups of the obtained peptides are modified using isotopic variants of the blocking reagent. The blocking group of the primary amino groups possesses two variants, given by the presence of light or heavy isotopes to allow the quantification. For example when using the following reagents (C²H₃CO)₂O/(C¹H₃CO)₂O or (¹³CH₃CO)₂O/(¹²CH₃CO)₂O or (¹³CH₃¹³CO)²O/(¹²CH₃¹²CO)²O, the peptides are labeled with ²H₃/¹H₃ or ¹³C₁/¹²C₁ or ¹³C₂/¹²C₂, respectively. This principle is also valid for every of the blocking reagents mentioned in the step (3) of the described method that carry anyone of the isotopes above mentioned as well as ¹⁸O/¹⁶O, ¹⁵N/¹⁴N, ³⁵S/³²S, etc. After labeling, the RH peptides are isolated by cation exchange chromatography as explained in the steps of the described method. In this type labeling the transitory or reversible blocking of the amino groups cannot be used because if it is eliminated before the mass spectrometric analysis, the isotopic labeling is also eliminated and therefore the relative quantification is impossible to be carried out.

The relative quantification by the analysis of the isotopic distribution of the mixture of the labeled and non-labeled RH peptides in the mass spectra is carried out by using an appropriate software (Fernández of Cossío et al. Isotopic, A Web Software for Isotopic Distribution Analysis of Biopolymers by Mass Spectrometry. Nuclei Acid Research 2004, 32, W674-W678 and Fernández of Cossío et al. Automated Interpretation of Mass Spectra of Complex Mixtures by Matching of Isotope Peak Distributions. Rapid Commun. Mass Spectrom. 2004; 18, 2465-2472). This software calculates the theoretical isotopic distributions of the labeled and non-labeled RH peptides and it achieves a such combination so that the resultant area of the theoretical isotopic distribution is adjusted from the best way to the area of the isotopic distribution observed experimentally. The proportions existent between each area corresponding to the peptides labeled with light and heavy isotopes (¹²C/¹³C, ¹⁴N/¹⁵N, ¹⁶O/¹⁸O, and/or ¹H/²H) once normalized correspond with the relative proportion of the proteins that contained them in the compared mixtures.

To carry out the quantification it is necessary to know (a) the elemental composition of the analyzed peptide or its sequence, (b) the type of isotopic labeling used in the experiment and (c) the region of the mass spectrum that contains the experimental isotopic distribution of the RH peptide of interest. All this information is very restrictive allowing to calculate in a very precise way the experimental noise and to discard of the analysis the overlapping of other signals that are not of interest for the quantification. All these information's make the quantification process very robust with the used software and it is independent of the method of isotopic labeling. This proposed method is compatible with the ionization modes more frequently used in the characterization of peptides and proteins: the electrospray ionization for (ESI-MS) and the matrix assisted laser desorption ionizaton (MALDI-MS). These ionization methods give the molecular mass, an important information, however they do not provide structural information that allows a reliable identification in the sequence databases of the selectively isolated RH peptides. To achieve this, the peptides of interest are selected in the mass spectrometer and pass through a collision chamber where by means of a process known as collision induced dissociation, fragments containing enough structural information are generated and it allows the elucidation of the complete or partial sequence of the analyzed peptide.

The mass spectrum that contains this information is known as MSMS spectrum. Each MSMS spectrum is very characteristic of the sequence of the peptide that originated it, can be considered as a fingerprint of the fragments ions and it is useful for the reliable identification of the peptides in the sequence databases with the aid of computer programs. In fact this it is the principle of one of the most popular search engines designs for the identification of proteins in the sequence databases: the program MASCOT (Matrix Science Ltd, UK, Perkins, DN, et al. Probability-based protein identification by searching sequence databases using mass spectrometry data. Electrophoresis 1999, 20, 3551-3567); and the SEQUEST program (Trademark, University of Washington, Seattle Wash, McCormack, A. L. et al. Direct Analysis and Identification of Proteins Mixtures by LC/MS/MS and Database Searching at the Low-Femtomole Level. Anal. Chem. 1996, 69, 767-776; Eng, J. K. et al. An Approach to Correlate Tandem Mass Spectral Dates of Peptides with Amino Acid Sequences in to Protein Database. J. Amer. Soc. Mass. Spectrom. 1994, 5, 976-989; U.S. Pat. No. 5, 538,897 (Jul. 23, 1996) Yates, III et al.)

These programs (MASCOT and SEQUEST) compare the MSMS spectra obtained experimentally with the theoretical MSMS spectra of all the peptides that possess a certain molecular mass in the protein sequences databases and they have been originated by the specific cleavage of the used protease. The MSMS spectrum where a bigger coincidence exists between the mass values of the theoretical fragments and those obtained experimentally, should correspond with the analyzed peptide and from here is inferred the protein that containing it and the identification in the database is carry out.

The following references are related with the application of some mass spectrometry techniques to the identification of proteins, particularly in the proteome analysis: Ideker T, Thorsson V, Ranish J A, Christmas R, Buhler J, Eng J K, Bungarner R, Goodlett D R, Aebersold R, Hood L. Integrated genomic and proteomic analyses of to systematically perturbed metabolic network. Science. 2001, 292, 929-934; Gygi SP, Aebersold R. Mass spectrometry and proteomics. Curr Opin Chem Biol. 2000, 4, 489-494., Gygi S P, Rist B, Aebersold R. Measuring gene expression by quantitative proteome analysis. Curr Opin Biotechnol. 2000, 1, 396-401, Goodlett D R, Bruce J, Anderson GA, Rist B, Pass-Tolic L, Fiehn OR, Smith R D, Aebersold R. Protein identification with to it sails accurate mass of to cysteine-containing peptide and constrained database searching. Anal Chem. 2000, 15; 72, 112-118; and Goodlett D R, Aebersold R, Watts JD. Quantitative in vitro kinase reaction as a guide for phosphoprotein analysis by mass spectrometry. Rapid Commun Mass Spectrom. 2000; 14, 344-348).

Taking into account the high selectivity of the proposed method, the identification can be restricted to databases that only possess RH peptides to guarantee a faster identification, to avoid false positive identification and to obtain a more reliable identification by using the programs MASCOT and SEQUEST.

### DESCRIPTION OF FIGURES.

FIG. 1. Diagram that shows the selective isolation of RH peptides using the method described in this invention for their application in the quantitative proteomics.

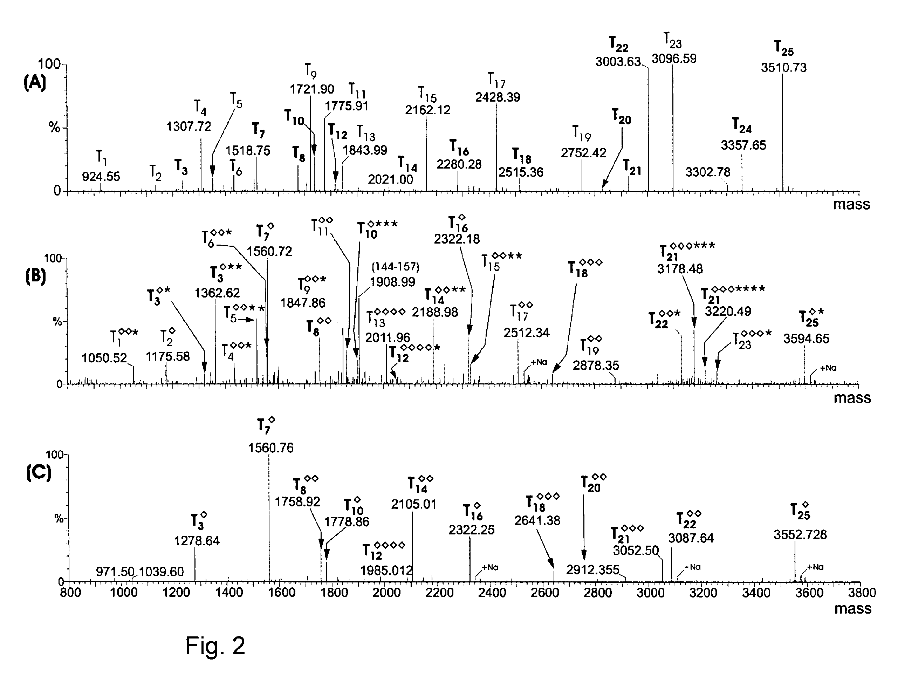

FIG. 2. The selective isolation of the RH peptides is shown in a model protein: the recombinant streptokinase (rSK). (A) ESI-MS spectrum of the mixture of tryptic peptides. (B) ESI-MS spectrum of the peptide mixture after the blocking reaction of the amino groups. (C) ESI-MS spectrum that contains the RH
peptides of the rSK after applying the method object of this invention.

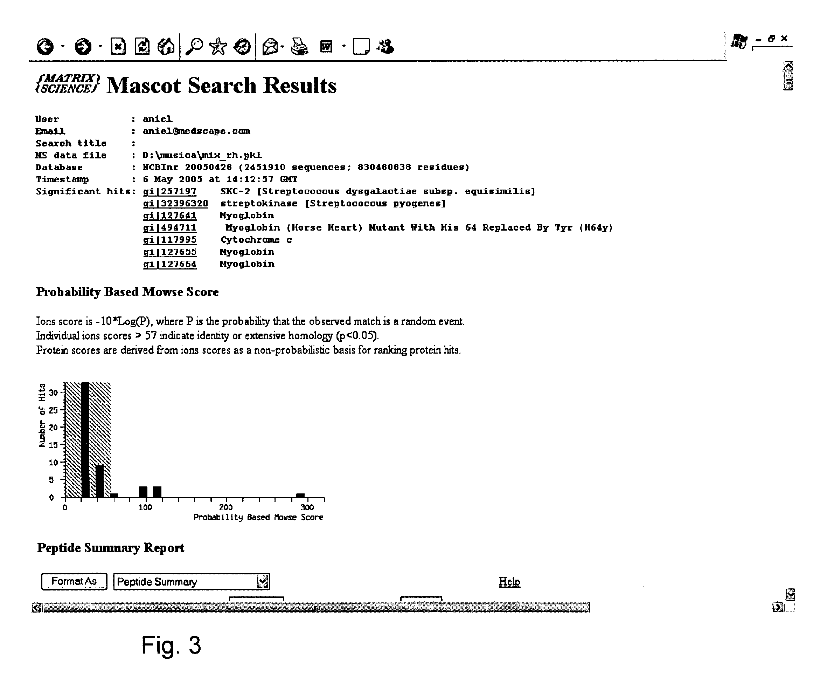

FIG 3. Results provided by the MASCOT program where the three proteins (rSK, myoglobin and cytochrome-C) present in the artificial mixture are automatically identified after the selective isolation of the RH peptides and the analysis by LC-MS/MS.

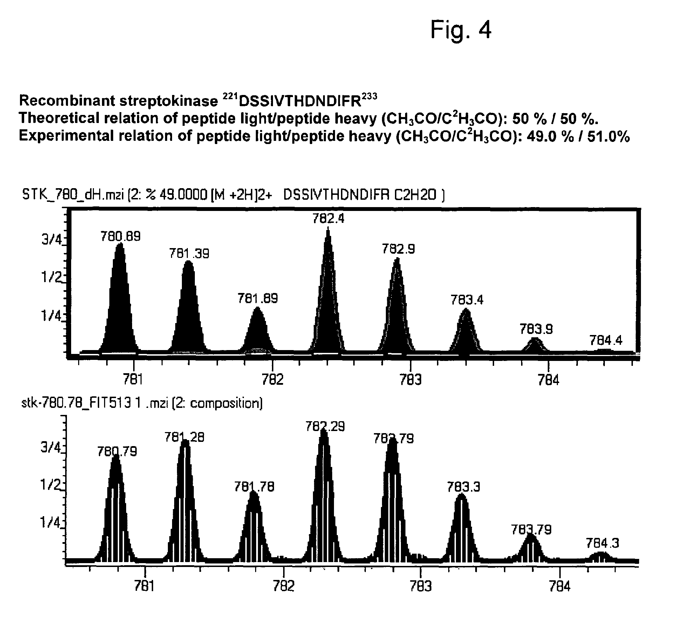

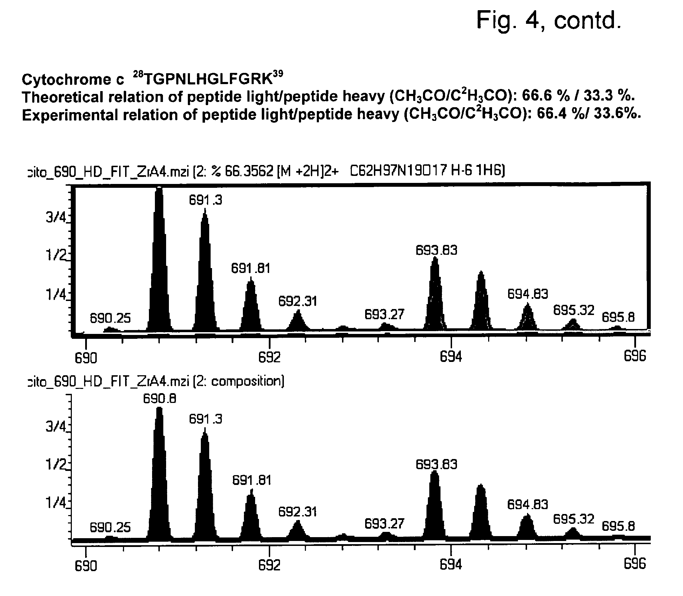

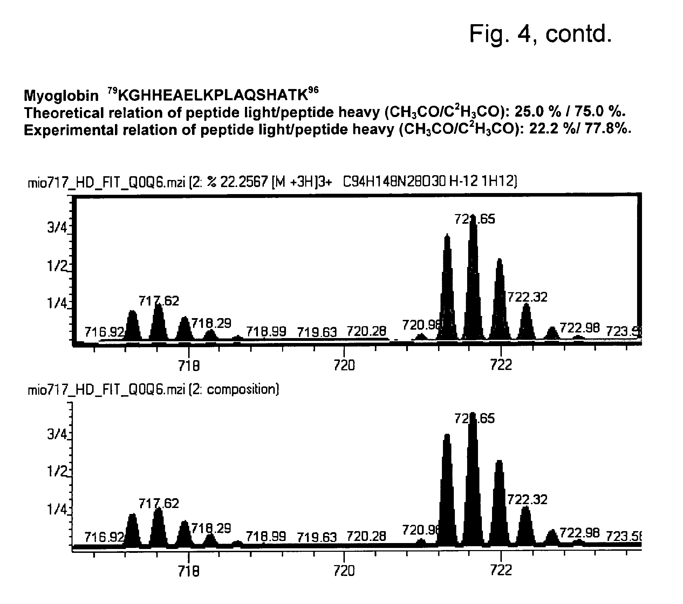

FIG 4. The ESI-MS spectra in black color shows the isotopic distributions of the peptides ²²¹DSSIVTHDNDIFR²³³ (rSK), ²⁸TGPNLHGLFGRK³⁹ (cytochrome-C) and ⁷⁹KGHHEAELKPLAQSHATK⁹⁶ (myoglobin) obtained after applying the method for selective isolation shown in the example 1 to an artificial mixture of three proteins (rSK, myoglobin and cytochrome-C) and using the irreversible blocking of the amino groups with normal acetic anhydride [(CH₃CO)₂O] and deuterated acetic anhydride [(C²H₃CO)₂O] see experiment 1, example 3). In blue and violet colors are shown the contours of the isotopic distributions corresponding to the above mentioned peptides modified with a normal acetyl group (CH₃CO) - and a deuterium labeled acetyl group (C²H₃CO) -, respectively. The contour of the isotopic distribution highlighted in red color show the adjustment carried out by the program after superimposing the isotopic distributions mentioned previously in the proportion (CH₃CO/C²H₃CO) determined by the software as it is indicated for each particular peptide.

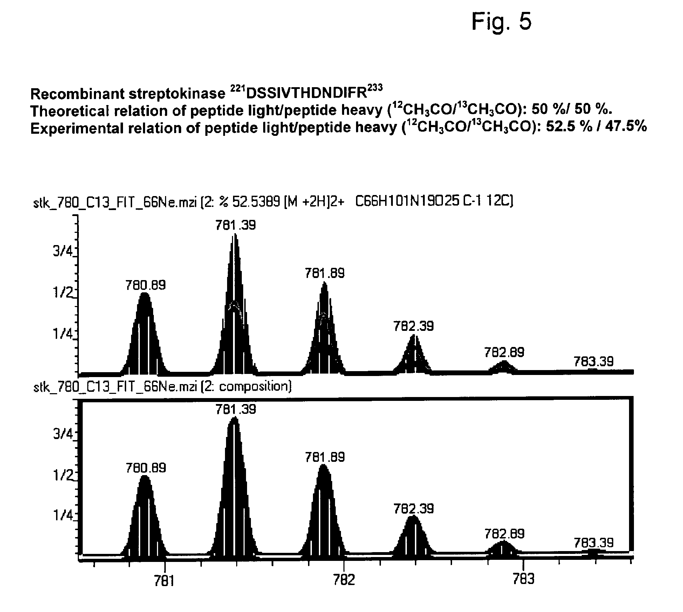

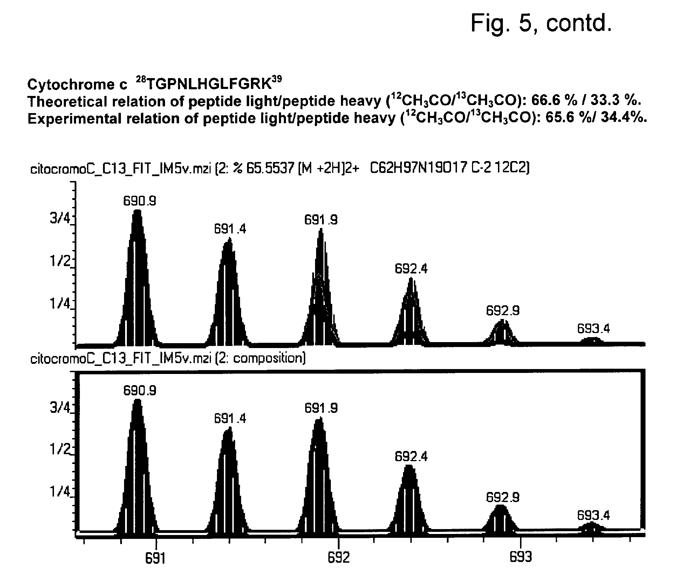

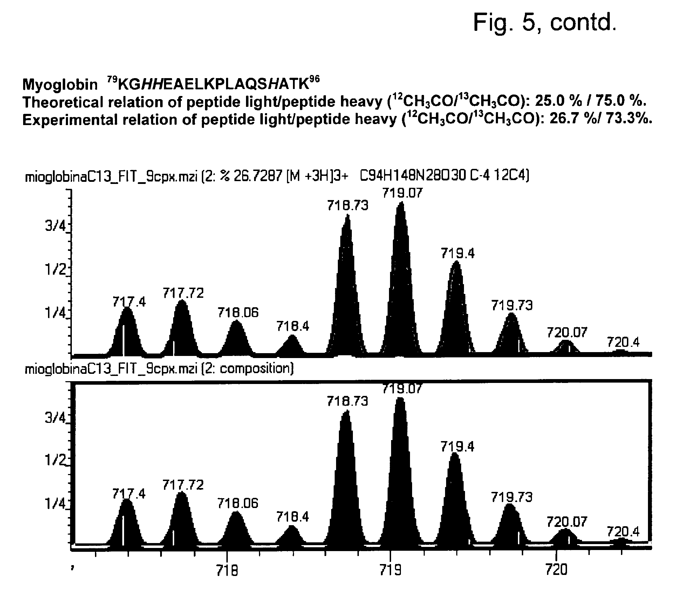

FIG 5. The ESI-MS spectra in black color shows the isotopic distributions of the peptides ²²¹DSSIVTHDNDIFR²³³ (rSK), ²⁸TGPNLHGLFGRK³⁹ (cytochrome-C) and ⁷⁹KGHHEAELKPLAQSHATK⁹⁶ (myoglobin) obtained after applying the method of selective isolation shown in the example 1 to an artificial mixture of three proteins (rSK, myoglobin and cytochrome-C) and using the irreversible blocking of the amino groups with normal acetic anhydride [C²CH₃CO)₂O] and ¹³C-labeled acetic anhydride [(¹³CH₃CO)₂O]. In blue and violet colors are shown the contours of the isotopic distributions corresponding to the peptides previously mentioned with a normal acetyl group (¹²CH₃CO) - and ¹³C-labeled acetyl group (¹³CH₃CO)₂O], respectively.

The contour of the isotopic distribution highlighted in red color show the adjustment carried out by the program after superimposing the isotopic distributions previously mentioned in the proportion (¹²CH₃CO-/¹³CH₃CO-) determined by the software as it is indicated for each particular peptide.

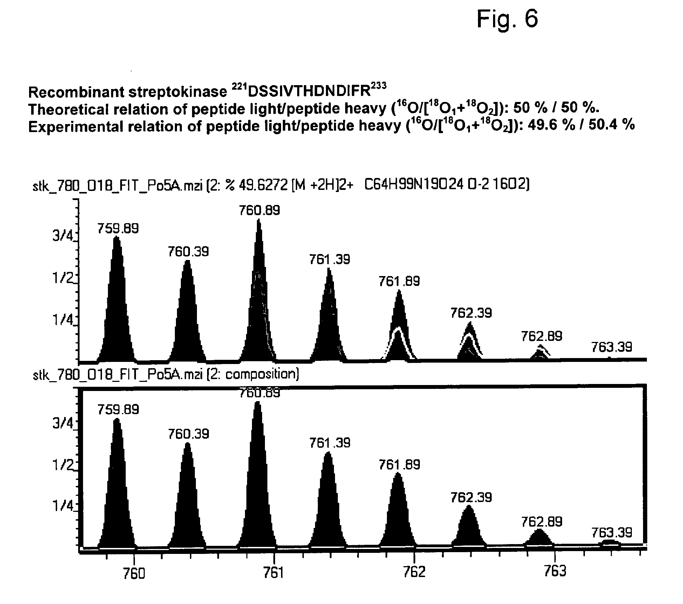

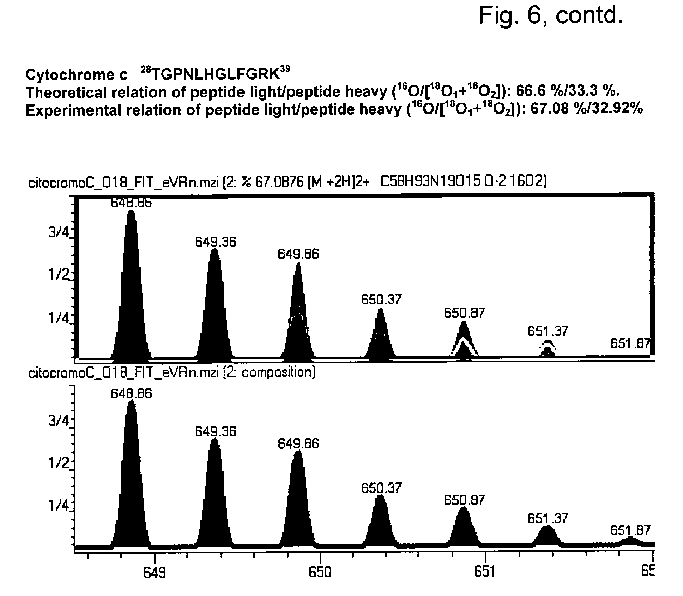

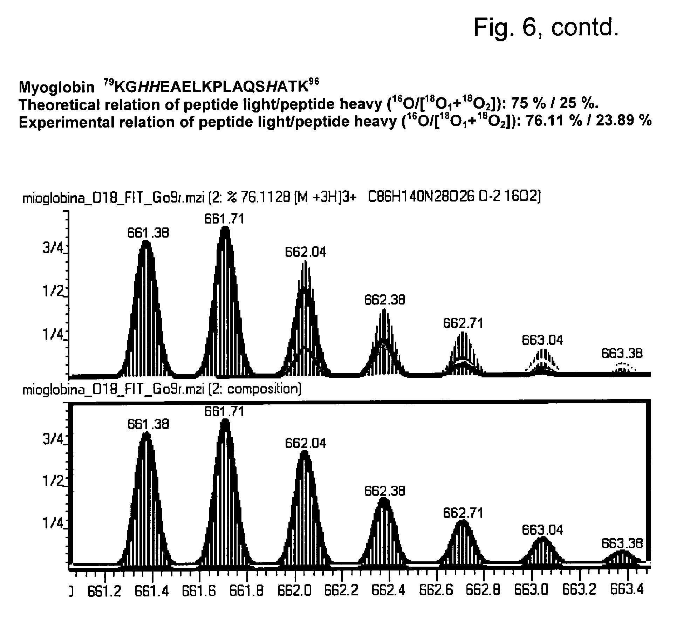

FIG. 6. The ESI-MS spectra in black color show the isotopic distributions of the peptides ²²¹DSSIVTHDNDIFR²³³ (rSK), ²⁸TGPNLHGLFGRK³⁹ (cytochrome-C) and ⁷⁹KGHHEAELKPLAQSHATK⁹⁶ (myoglobin) obtained after applying the method of selective isolation shown in the example 1 to an artificial mixture of three proteins (rSK, myoglobin and cytochrome-C) and using the reversible blocking of the amino groups with 2-(methyl sulfonyl) ethyl succinimidyl carbonate and the labeling with ¹⁸O at the carboxy C-terminal end during the proteolysis of the analyzed mixture of proteins. In blue, violet and yellow colors are shown the contours of the isotopic distributions corresponding to the previously mentioned peptides without incorporating ¹⁸O, after incorporating one atom of ¹⁸O (¹⁸O₁) and when incorporating two atoms of ¹⁸O (¹⁸O₂), respectively. The contour of the isotopic distribution highlighted in red color shows the adjustment carried out by the program after superimposing the isotopic distributions previously mentioned in the proportion (¹⁶O/[¹⁸O₁+¹⁸O₂]) determined by the software as it is indicated for each particular peptide.

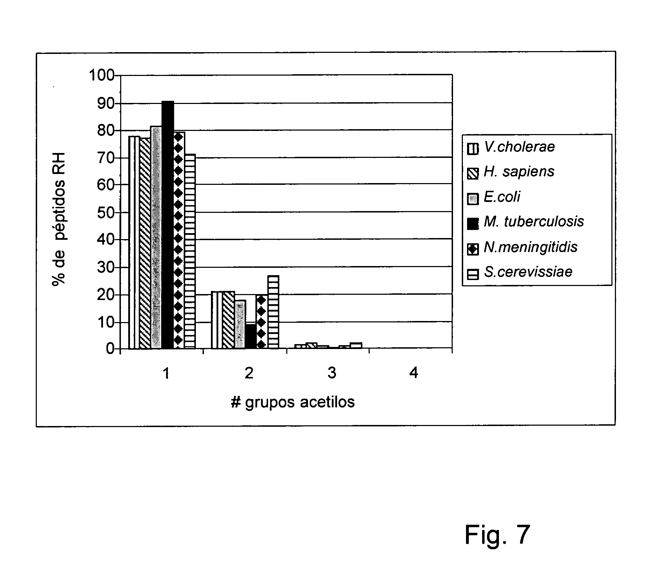

FIG 7. The graph shows the percentage of RH peptides from several proteomes, containing one, two, and three deuterated acetyl groups (C²H₃CO -) in their sequences.

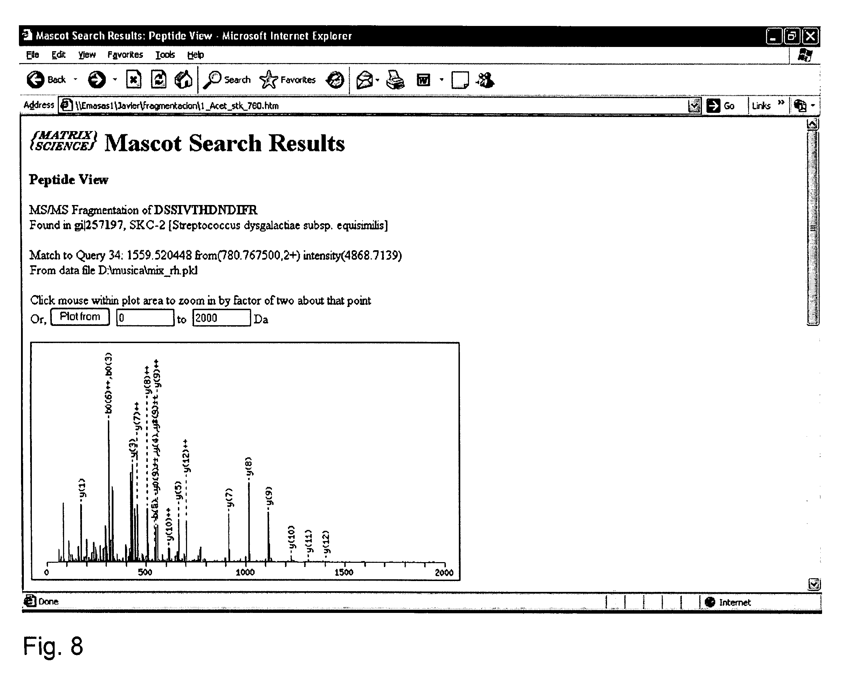

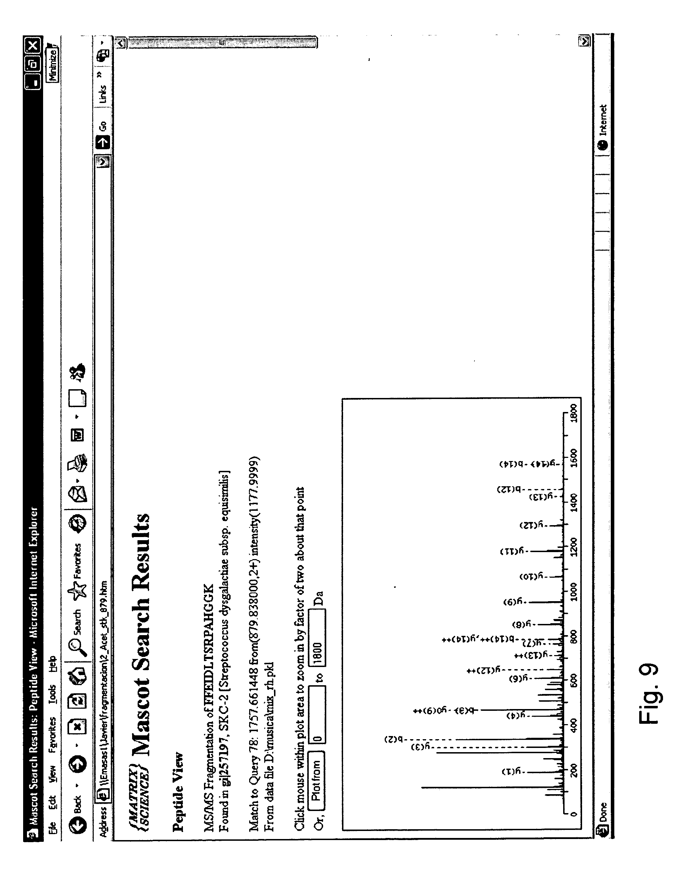

FIG 8. ESI-MSMS spectrum of the doubly-charged RH peptide at m/z 780.77 (²²¹DSSIVTHDNDIFR²³³) of the rSK that contains an acetyl group at their N-terminal end. The fragments ions were assigned automatically by the MASCOT programs according to the nomenclature proposed by Roepstorff P and Fohlman J (Roepstorff P and Fohlman J. Proposal for a common nomenclature for sequence ions in mass spectra of peptides. Biomed Mass Spectrom. 11, 984, 601). FIG 9 ESI-MSMS spectrum of a doubly-charged RH peptide at m/z 879.84 (³⁸FFEIDLTSRPAHGGK⁵²) of the rSK that contains two acetyl groups one located at their N-terminal end and the other one at the amino group of the side chain of lysine residue. The fragments ions were assigned automatically by the MASCOT program according to the nomenclature proposed by Roepstorff P and Fohlman J (Roepstorff P and Fohlman J. Proposal for a common nomenclature for sequence ions in mass spectra of peptides. Biomed Mass Spectrom. 11, 984, 601).

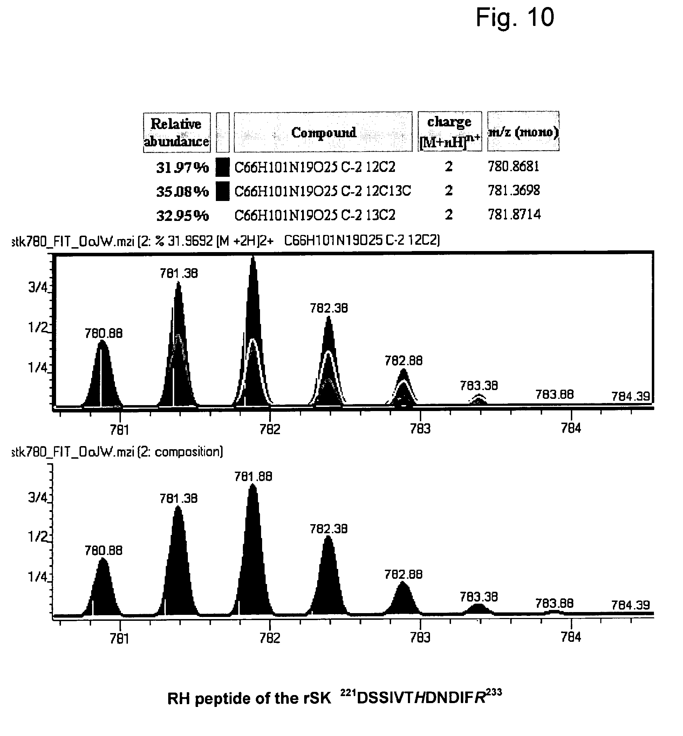

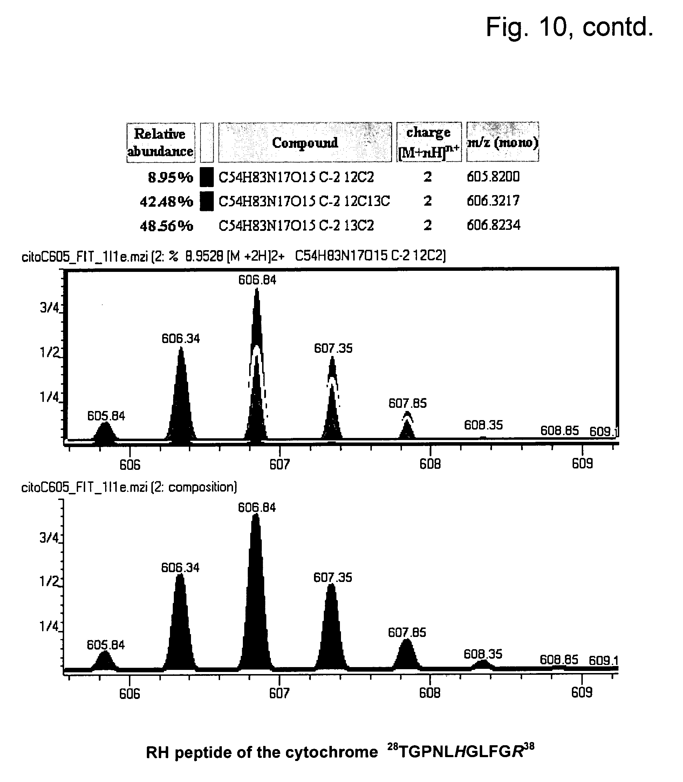

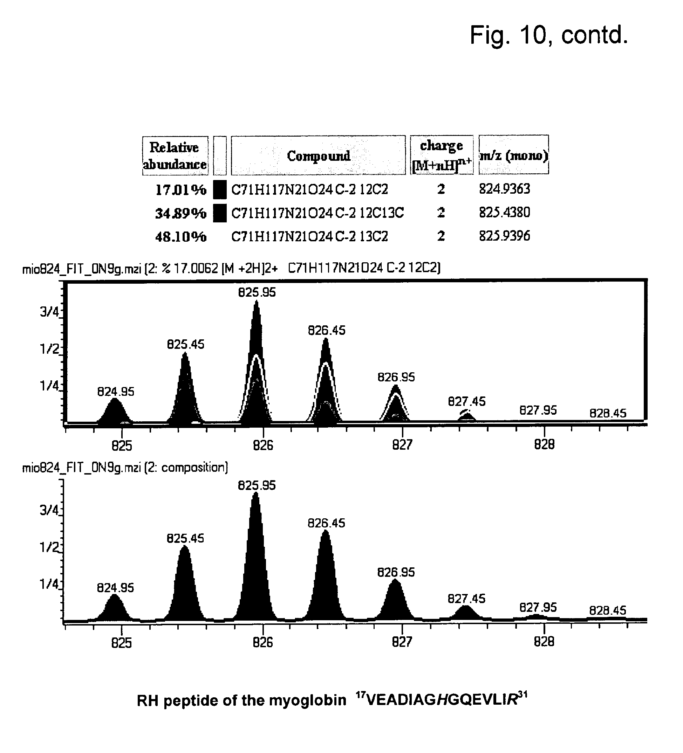

FIG 10. Relative quantification of three proteins (rSK, cytochrome C and myoglobin) present in three compared samples. A peptide of each protein was selected to show the results obtained in the relative quantification using the ISOTOPICA software. The mass spectra highlighted in black color show the overlapping of the experimental isotopic distributions of the RH peptides of each one of the proteins present in the analyzed mixture. The peptides of the conditions "A", "B" and "C" were labeled at their amino terminal end with CH₃CO -, ¹³CH₃CO - and ¹³CH₃¹³CO -, respectively. The contours of the isotopic distribution highlighted in blue, violet and yellow correspond to the RH peptides that do not have ¹³C, one atom of ¹³C and two atoms of ¹³C, respectively. The contour of the isotopic distribution highlighted in red color show the agreement that exists between the experimental isotopic distribution and the one obtained when superimposing the isotopic distributions of the conditions "A", "B" and "C" in the proportions calculated by the ISOTOPICA software. In each one of the spectra the results are also provided by the software for the three labeled species.

### EXAMPLES.

### EXAMPLE 1. In silico demonstration of the simplification of complex mixtures of peptides derived from proteomes of different organisms using the chromatographic system proposed that allows the selective isolation of multiply-charged peptides (2+, 3+, 4+, etc) or RH peptides.

The great efficiency reached by the sequencing of the DNA molecule has made possible that complete sequences of genomes of several organisms are know, and it is possible to predict which are the proteins that are derived from the genomes and what peptides are generated depending the specific proteolytic treatment that is carried out to the studied proteome.

To have an exact idea about the magnitude of the simplification when the RH peptides are selectively isolated a program named SELESTACT was devised. It was written in C for use in PC and calculates from the proteome of a given organism:
1 - the total number of proteins reported in the Swissprot database,
2 - the total number of peptides/proteins that can be generated by an specific proteolytic treatment,
3 - the number of RH peptides/protein that are generated by this specific proteolytic treatment,
4 - the percentage of proteins that possess RH peptides (referred to the total number of proteins reported in particular proteome) that can be successfully identified by the method proposed in the present invention (proteome coverage).

This program also calculates all these parameters for the proteolytic peptides of a given proteome that possess cystein residues and therefore they can be selectively isolated by the ICAT method, one of the pioneering and the most used method in the selective isolation of peptides and its application to proteome studies

**Table 1.** *In silico* analysis of proteomes of different organisms by using of the SELESTACT software to determine the contained RH peptides when applying the method proposed in the Figure 1 that uses a chromatographic system that separates the neutral and singly-charged peptides (charge 0 and 1+) from the multiply-charged peptides (charge 2+, 3+, 4+, etc) or RH peptides. The results for the simplification when applying the well-known method ICAT, are also shown.

| Analyzed Proteome | total # of proteins ^{a)} | Total tryptic peptides/protein ^{b)} | Peptides selectively isolated/protein ^{c)} (RH / \| CAT) | Coverage of the proteome ^{d)}(RH / \| CAT) |
|---|---|---|---|---|
| *N. meningitidis* | 1967 | 14 | 3 / 2 | 80.8 / 81.6 |
| *V. cholerae* | 3785 | 15 | 3 / 2 | 83.9 / 80.3 |
| *M. tuberculosis* | 3875 | 15 | 5 / 2 | 94.6 / 79.0 |
| *E. coli* | 4322 | 15 | 4 / 3 | 87.7 / 83.2 |
| *S. cerevissiae* | 4818 | 24 | 4 / 4 | 88.0 / 89.2 |
| *A. thaliana* | 26003 | 14 | 4 / 5 | 90.3 / 93.2 |
| M. *musculus* | 28959 | 14 | 5 / 6 | 91.9 / 94.7 |
| *H. sapiens* | 47531 | 22 | 6 / 6 | 92.5 / 94.8 |
| **Average** | - | 18 | 4 / 4 | 87.9 / 87.0 |

| | | | | |
|---|---|---|---|---|
| *a) it corresponds to the total number of proteins reported in the Swissprot sequences database.* *b) total number of tryptic peptides*/*proteins that are generated by the specific proteolysis with trypsin of the different analyzed proteomes. It is expressed as a integer number.* *c) total number of multiply-charged peptides* / *analyzed protein when using the chromatographic system described in heading of the this table and the one obtained by the ICA T method. Both data are expressed as integer numbers.* *d) The coverage of the analyzed proteome represents the percentage of the total number of proteins that possess RH peptides and can be isolated when using the chromatographic method described in the heading of the present table. The values correspond to the proposed method and the ICAT.* | | | | |

Table 1 summarizes the results obtained by *in silico* analysis after applying the method to several proteomes including bacteria pathogen bacteria, yeasts, plants, and mammals are shown. As it can appreciate, of an average of 18 tryptic peptides per protein, the mixture would be simplified considerably until reaching an optimum value for these purposes because 4 RH peptides /proteins are selectively isolated at the same extent as the ICAT method. The average for the proteome coverage, that represents the percentage of the proteome of an organism that can be studied with the proposed method (87.9%) it is also very similar to the one achieved with the ICAT (87%). However, when individual proteomes are analyzed a very marked difference it is clearly noticed for the case of *M. tuberculosis* proteome. In the method object of the present invention 94,6% of the complete proteome can be analyzed, on the other hand, when the ICAT method is used only it can analyze less than 80%. When studying particularly this microorganism in proteomics the method of selective isolation object of this invention it should be the method of choice. The usage of the SELESTACT software is of great utility to predict which are the expected results from the method object of the present invention for particular organism and therefore it allows the comparison with other methods of selective isolation of peptides for proteomic studies.

This demonstrates us that the principle of this invention, the separation of peptides with charges 0 and 1+ from the multiply-charged peptides (charges 2+, 3+, 4+, etc) by using a chromatographic system it is of great utility and it can be successfully used for the proteomics study of organisms with different evolutionary grades since it allows an ideal simplification of the complex mixture of peptides and at the same time it guarantees high coverage of the proteome under study.

It only remains to demonstrate in the example 2, a chromatography system that is able to make in a simple and effective way of the proposed separation, the neutral and singly-charged peptides from the multiply-charged peptides.

### EXAMPLE 2. Selective Isolation of the RH peptides of the recombinant streptokinase (rSK). Evaluation of the selectivity and the specificity of the method.

The method proposed in the figure 1 was applied to the rSK protein. This protein was selected as model because its tryptic digest generates a great number of tryptic peptides and RH peptidases well. Therefore it is very easy the evaluation of the specificity and the selectivity of the proposed method.

The steps to follow are shown below:
1. the protein was dissolved in a 500 mM Hepes buffer (pH 8.0) containing guanidium chloride (2 mol/L) and lysyl-endopeptidase was added to a enzyme:substrate ratio 1:200 and the digestion proceeded at 37 °C during 16 h. Next, the digestion buffer was diluted until obtaining a concentration of 1 mol/L of guanidium chloride, and the trypsin was added to a enzyme:substrate ratio of 1:100 and the digestion proceeded additionally during 4h to 37 °C.
2. the mixture of proteolytic peptides were incubated at a temperature between 0-5 °C, and acetic anhydride was added in a molar ratio 10:1 with respect to the primary amino groups (alpha and epsilon). The mixture was shortly stirred and it was incubated again in a ice bathroom. This procedure was repeated additionally twice at intervals of five minutes.
3. the excess of reagent was eliminated by reverse phase chromatography using a RP-C4 column (Vydac, 20 x 2.1 mm) previously equilibrated in a solution of H₂O/TFA 0.05% (v/v) and the peptides are collected in a single fraction when carrying out a fast gradient in 10 minutes by increasing the content of acetonitrile containing 0.05% (v/v) TFA from 1 to 80% of the mobile phase.
4. the peptides blocked at the primary amino groups are applied to strong cation exchange column (for example: Source 30S, 2x20mm, Amersham-Pharmacia, Sweden) previously equilibrated with a solution of H₂O/TFA (0.05% v/v) that contains 15 % of acetonitrile using a flow of 200 microliters/min. The non-retained fraction that contains neutral (R+H=0) or singly-charged peptides (R+H=1) is discarded, on the other hand, the retained fraction that contains to the multiply-charged peptides (RH, R+H>1) is additionally fractioned by increasing the saline concentration of the equilibrium solution.
5. before being analyzed by means of liquid chromatography coupled to mass spectrometry, the RH peptides is treated at basic pH (1 % of triethylamine during 1 hour at 37 °C) to eliminate from its structures the non-specific blockages (O-acylations) present at the tyrosine residues. In this step, the hydrolytic treatment used to eliminate those O-acylations as well as the blocking group (when a reversible blockage is used at the amino groups) should not eliminate the labeling introduced since it is indispensable to carry out the relative quantification of peptides.

The ESI-MS spectrum (figures 2A) shows a considerable number of signals corresponding to the peptides generated during the tryptic digestion of the rSK. The assignments of these signals to the sequence of the rSK (sequence 1 at the end of the document) are shown in the Table 2, as well as the experimental and theoretical mass values for each peptide. In this table the peptides were classified regarding to the number of positive charges that they can have at acidic pH once their primary aminos groups have been acetylated. Logically, the number of positive charges (see Z, Table 2) depend on the number of arginine (R) and/or histidine (H) residues contained within their respective sequences (R+H). As it can be can observed in Table 2, 12 out of the 25 signals correspond to peptides that once blocked the primary amino groups at acidic pH would be neutral (z=0, having neither arginine nor histidine in their respective sequences) or singly-charged (z=1, to have an arginine residue or a histidine residue in their sequences).
Those signals with assignments written in boldfaces correspond to 13 multiply-charged peptides or RH peptides (peptides T₃, T₇₋₈, T₁₀, T₁₂, T₁₄, T₁₆, T₁₈, T₂₀₋₂₂, T₂₄₋₂₅, figure 2A). These peptides fulfills the condition that in their sequences the sum of the number of arginine and/or histidine residues are superior than one (R+H>1).
The primary amino groups (amino terminal and epsilon amino groups of lysines) of tryptic peptides were blocked and it is observed all increased their molecular masses in dependence of the number of acetyl groups added to their structure (see number of added rhombuses, in figure 2B). However, some peptides incorporated additional acetyl groups at the tyrosine residues (figures 2B, peptides 3-6, 9-10, 12,14, 16, 21-22 and 25). This mixture is simplified considerably after passing through the cation exchanger: only 12 peptides out of 25 that had been detected initially. Later on, this undesirable modification at the tyrosine residues (Zappacosta F, and Annan RS. N-terminal isotope tagging strategy for quantitative proteomics: results-driven analysis of protein abundance changes. Anal Chem. 2004, 76, 6618-6627) was eliminated with a basic treatment mentioned in the step 5 of the method (Figures 2C). The non-retained fraction in the cation exchange chromatography was discarded and the retained fraction was eluted in a single step by increasing the concentration of NaCl in the mobile phase to 2 mol/L. Notices, that the expected simplification is achieved for the mixture of peptides derived from the rSK and 12 out of the 13 peptides classified as RH in the figure 2A, were isolated successfully in the figure 2C (peptides T₃, T₇₋₈, T₁₀, T₁₂, T₁₄, T₁₆, T₁₈, T₂₀₋₂₂, T₂₅) for 92 % selectivity. Notice also that in this ESI-MS spectrum any of those neutral peptide (z=0) or singly-charged (z=1) that appear in the Table 2 were not detected in the spectrum of the figure 2A, what is equal to 100% of specificity.

On the other hand, none of the multiply-charged or RH peptides shown in the spectrum of the Figure 2C contain additional acetylations (asterisks) respect to the expected ones, therefore the basic treatment to eliminate these O-acylations at the tyrosine residues worked in a quantitative way.

**Table 2. Summary of the assignment of the signals observed in the Figure 2A for the tryptic peptides of the rSK.**

| Code ^{a)} | Sequence assignment ^{b)} | z ^{c)} | m/z exp ^{d)} | m/z theor.^{e)} |
|---|---|---|---|---|
| T1 | ³⁶⁵IITVYMGK³⁷² | 0 | 924.55 | 924.52 |
| T2 | ³¹¹SEQLLTASE***R***³²⁰ | 1 | 1133.62 | 1133.57 |
| ***T3*** | ²¹¹T***H***PGYTIYE***R***²²⁰ | ***2*** | 1236.64 | 1236.59 |
| T4 | ³⁰⁰YVDVNTNELLK³¹⁰ | 0 | 1307.72 | 1307.68 |
| T5 | ²⁸⁰KGEKPYDPFD***R***²⁹⁰ | 1 | 1351.71 | 1351.66 |
| T6 | ⁴⁰³YTGTPIPDNPNDK⁴¹⁵ | 0 | 1431.71 | 1431.67 |
| ***T7*** | ²²¹DSSIVT***H***DNDIF***R***²³³ | ***2*** | 1518.76 | 1518.71 |
| ***T8*** | ³⁸FFEIDLTS***R***PA***H***GGK⁵² | ***2*** | 1674.92 | 1674.85 |
| T9 | ²³⁴TILPMDQEFTY***H***VK²⁴⁷ | 1 | 1721.90 | 1721.85 |
| ***T10*** | ³⁹⁰YTEEE***R***EVYSYL***R***⁴⁰² | ***2*** | 1736.87 | 1736.81 |
| T11 | ²⁵⁹SGLNEEINNTDLISEK²⁷⁴ | 0 | 1775.91 | 1775.86 |
| ***T12*** | ²⁸⁰KGEKPYDPFD***R***S***H***LK²⁹⁴ | ***2*** | 1816.99 | 1816.93 |
| T13 | ⁶¹SKPFATDSGAMP***H***KLEK⁷⁷ | 1 | 1843.99 | 1843.93 |
| T14 | ³⁷³***R***PEGENASY***H***LAYDKD***R***³⁸⁹ | ***3*** | 2021.04 | 2020.94 |
| T15 | ³³⁶LLYNNLDAFGIMDYTLTGK³⁵⁴ | 0 | 2162.12 | 2162.08 |
| ***T16*** | ¹²³DGSVTLPTQPVQEFLLSG***H***V***R***¹⁴³ | ***2*** | 2280.28 | 2280.19 |
| T17 | ¹⁸⁸TLAIGDTITSQELLAQAQSILNK²¹⁰ | 0 | 2428.39 | 2428.32 |
| ***T18*** | ³⁸FFEIDLTS***R***PA***H***GGKTEQGLSPK⁶⁰ | ***2*** | 2515.36 | 2515.29 |
| T19 | ¹⁵⁸SVDVEYTVQFTPLNPDDDF***R***PGLK¹⁸¹ | 1 | 2752.42 | 2752.34 |
| ***T20*** | ³⁷³***R***PEGENASY***H***LAYDKD***R***YTEEE***R***³⁹⁵ | **4** | 2828.20 | 2828.28 |
| ***T21*** | ³⁶⁵IITVYMGK***R***PEGENASY***H***LAYDKD***R***³⁸⁹ | **3** | 2926.52 | 2926.44 |
| ***T22*** | ¹¹⁷VYFADKDGSVTLPTQPVQEFLLSG***H***V***R***¹⁴³ | **2** | 3003.63 | 3003.55 |
| T23 | **¹⁵⁸**SVDVEYTVQFTPLNPDDDF***R***PGLKDTK¹⁸⁴ | 1 | 3096.59 | 3096.51 |
| ***T24*** | ³³⁶LLYNNLDAFGIMDYTLTGKVEDN***H***DDTN***R***³⁶⁴ | **2** | 3357.65 | 3357.56 |
| ***T25*** | ⁸³AIQEQLIANV***H***SNDDYFEVIDFASDATITD***R***¹¹³ | 2 | 3510.73 | 3510.66 |

| | | | | |
|---|---|---|---|---|
| *a) Code corresponding to the tryptic peptides of the rSK observed in the figure 2A. The codes highlighted in boldfaces and italic correspond to the RH peptides.* *b) The amino acids highlighted in boldfaces and italic are the arginine and histidine residues in the sequence of the tryptic peptides of rSK. The numbering of the amino acids N - and C-terminal correspond with the position of each one of them in the sequence of the rSK.* *c) Z is the number of positive charges holded by the arginine and histidine residues in the peptides dissolved at acidic pH.* *d) experimental mass of the tryptic peptides of the rSK.* *e) theoretical mass of the tryptic peptides of the rSK.* | | | | |

These results demonstrate that the chromatographic system designed is very specific and selective to isolate the multiply-charged or RH peptides and it can be applied with success in the proteomic studies.

### Example 3. Identification and relative quantification of the component proteins of two artificial mixtures (A and B) by means of the selective isolation of RH peptides by using three different isotopic labeling (²H, ¹³C and ¹⁸O).

Two artificial mixtures A and B composed of the proteins rSK, myoglobin and cytochrome-C were prepared in a molar ratio of A with respect to B: 1:1 for the rSK, 2:1 for the cytochrome C and 1:3 for the myoglobin. The sequences of these proteins are shown at the end of the document, sequences 1-3.

The selective isolation of the RH peptides was carried out for the method described in the example 1. Three experiments were performed by using different isotopic labeling methods to demonstrate the compatibility of the proposed method with different kind of labeling (²H, ¹³C and ¹⁸O) and it is demonstrated the possibility of using the reversible blocking of the amino groups in case it is desirable to analyze in the mass spectrometer the peptides with their free amino groups.

### Experiment 1.

The blocking of the primary amino groups of peptides obtained in the tryptic digestion of the mixture "A" was carried out using normal acetic anhydride ((C¹H₃CO)₂O) and the peptides of the mixture "B" were derivatized with deuterated acetic anhydride ((C²H₃CO)₂O) provided by ISOTEC (99% isotopic purity). In this experiment the molecular masses of the peptides obtained under both conditions differed in multiples of 3 units of masses depending the number of acetyl groups they added during the blocking reaction. In this experiment the overlapping of the isotopic distributions of the labeled and non-labeled peptides can be partial in case of a single acetyl group is added and the overlapping decrease as increase the quantity of blocking groups added to the peptides that contain lysine residues within their sequences.

### Experiment 2

The blocking of the primary amino groups of the tryptic peptides of the mixture "A" was carried out using normal acetic anhydride ((¹²CH₃CO)₂O) and those obtained from the mixture "B" were derivatized with ¹³C ((¹³CH₃CO)₂O, commercialized by ISOTEC 99% isotopic purity). In this experiment the overlapping of the isotopic distributions of the labeled and non-labeled peptides is elevated because their mass difference is just 1 Da for each blocking group added to a given peptide.

### Experiment 3

In this experiment some variants were introduced to the method described in the example 1 to demonstrate the combination of the ¹⁸O-labeling with the reversible blocking of the primary amino group of peptides. The mixture of proteins A was digested in a buffer described in the example 1 and the mixture B was digested with in the same buffer but prepared with heavy water (H₂¹⁸O) provided by ISOTEC (99% isotopic purity). By means of this procedure the peptides obtained in this last condition would be labeled with one or with two ¹⁸O atoms at their C-terminal end on the other hand, the other peptides keep their natural isotopic distribution. On the other hand, the amino groups of the tryptic peptides of both conditions were derivatized with a reversible reagent (2 (methyl sulfonyl) ethyl succinimidyl carbonate, marketed by Aldrich) and before mass spectrometric analyze they were eliminated in the same step of the removal of O-acylations at the tyrosine residues using the same conditions described previously in the example 1.

In this case the analysis of the isotopic distributions is a more complex than in the previous cases because the peptides labeled with ¹⁸O can add 1 and 2 atoms of ¹⁸O at their C-terminal end, therefore for calculating the relative quantities of the peptides obtained under both conditions is important to take into account that the relationship is given by the quotient between the areas of the isotopic distributions of the peptides having ¹⁶O divided by the sum of the areas corresponding to the isotopic distributions of the peptides that incorporated one (¹⁸O₁) and two atoms of (¹⁸O₂), according to the expression: (Area ¹⁶O)/(Area ¹⁸O₁+ Area ¹⁸O₂).

In all the above mentioned experiments the relative quantification of the peptides in the analyzed mixtures is carried out by using the ISOTOPICA software as it is explained in the detailed description of the method object of this invention (Fernández of Cossío et al. Isotopica, a Web Software for Isotopic Distribution Analysis of Biopolymers by Mass Spectrometry. Nucleic Acid Research 2004, 32, W674-W678 and Fernández of Cossío et al. Automated Interpretation of Mass Spectra of Complex Mixtures by Matching of Isotope Peak Distributions. Rapid Commun. Mass Spectrom. 2004; 18: 2465-2472).

The RH peptides of the three proteins present in the prepared mixture were isolated and sequenced in a single LC-MS/MS experiment being able to identify automatically without ambiguities by the MASCOT program (it Figures 3) the three proteins component of the artificially prepared mixture (rSK, cytochrome C and myoglobin). To proceed to the quantification, the expanded regions of the isotopic distribution of these peptides were selected and introduced in the ISOTOPICA software together with its global formulas and the type of labeling used. The results for the experiments 1, 2 and 3, where the labeling used were ²H, ¹³C and ¹⁸O, are summarized in the Table 3.

**Table 3. Summary of the selective isolation of the RH peptides and their relative quantification in two artificial mixtures composed by three proteins using three different labeling: ¹H/²H, ¹²C /¹³C and ¹⁶O/¹⁸O.**

| Proteins | Sequence of RH peptides ^{a)} | Quantification | | | |
|---|---|---|---|---|---|
| | | Theor. ^{b)} | Experimental ^{c)} | | |
| | | | ¹H/²H Exp. 1 | ¹²C/¹³C Exp. 2 | ¹⁶O/¹⁸O Exp. 3 |
| rSK | ¹²³DGSVTLPTQPVQEFLLSG***H***V***R***¹⁴³ | 50:50 | 50.2 : 49.8 | 51.2 : 48.8 | 49.7 : 50.3 |
| | ²¹¹T***H***PGYTIYE***R***²²⁰ | | 50.2 : 49.8 | 51.8 : 48.2 | 52.0 : 48.0 |
| | ²²¹DSSIVT***H***DNDIF***R***²³³ | | 49.0 : 51.0 | 52.5 : 47.5 | 49.6 : 50.4 |
| | ²⁸⁴PYDPFD***R***S***H***LK*²⁹⁴ | | 47.4 : 52.6 | 48.6 : 51.4 | 49.2 : 50.8 |
| | ³²¹NLDF***R***DLYDP***R***³³¹ | | 59.5 : 40.5 | 54.8 : 45.2 | 53.7 : 46.3 |
| | ³⁷³***R***PEGENASY***H***LAYDK*³⁸⁷ | | 50.2 : 49.8 | 53.2 : 46.8 | 52.0 : 48.0 |
| | ³⁸⁸D***R***YTEEE***R***EVYSYL***R***⁴⁰² | | 53.9 : 46.1 | 50.5 : 49.5 | 51.2 : 48.8 |
| | | | **51.5 :48.5 (±4.04)** | **51.8: 48.2 (±1.99)** | **51.1: 48.9 (±1.64)** |
| Cytochrome C | ²⁸TGPNL***H***GLFG***R***³⁸ | 66:33 | 65.7 : 34.3 | 64.8 : 35.2 | 66.8 : 33.2 |
| | ²⁸TGPNL***H***GLFG***R***K*³⁹ | | 66.4 **:** 33.6 | 65.6 : 34.4 | 67.1 : 32.9 |
| | | | **66.0:34.0 (±0.49)** | **65.2:34.8 (±0.49)** | 66.9:33.1 **(±0.21)** |
| Myoglobin | ¹⁷VEADIAG***H***GQEVLI***R***³¹ | 25:75 | 25.4 : 74.6 | 24.7 : 75.3 | 24.3 : 75.7 |
| | ⁷⁹K*G***HH***EAELK*PLAQS***H***ATK*⁹⁶ | | 22.2 : 77.8 | 26.7 : 73.3 | 23.9 : 76.1 |
| | | | **24.1:75.9 (±0.49)** | **25.7:74.3 (±1.0)** | **24.1:75.9 (±0.28)** |

| | | | | | |
|---|---|---|---|---|---|
| *a) Sequence of the RH peptides of the automatically identified proteins by the MASCOT software. The asterisks indicate acetylation in the epsilon amino group of lysine.* *b) theoretical proportion of the proteins in the compared artificial mixtures A and B.* *c) experimental value obtained for the relative quantities of the proteins present in the two compared artificial mixtures. In boldface letters is highlighted at the end of each experiment for each protein the average value of their relative quantification and the value of the standard deviation is indicated between parenthesis.* | | | | | |

For the three proteins the average of the experimental values for the relative quantifications are in very good agreement with the theoretical values and the obtained standard deviation was very good (below 5%).

These results demonstrate that the method can be used in the quantitative proteomics for determining with very good accuracy the relative quantities of the proteins in mixtures.

The adjustments of the areas corresponding to the experimental isotopic distributions obtained for one peptide of each one of the analyzed proteins in the three experiments mentioned in the present example are shown in the figures 4, 5 and 6.

Notice that in all the cases a very good adjustment was obtained for the theoretical contour of the isotopic distributions (red line) and the spectrum obtained experimentally (spectrum shadowed in black) independently of the type of labeling used (either ¹³C, ²H or ¹⁸O).

The results obtained for the relative quantification of the same protein in the three experiments were very similar independently of the type of labeling used. This example demonstrates the versatility and robustness of the method due to its compatibility with different types of labeling to obtain a great accuracy in the relative quantification.

### LC-MS/MS and database search.

The measurements were carried out in a hybrid mass spectrometer (quadrupole and time of flight, QTof-2) from the Micromass company (Manchester, United Kingdom). The mass spectrometer was connected online with a HPLC (AKTA Basic, Amersham Pharmacia Biotech, Sweden) through a column of 200 x 1 mm (Vydac, USES). The peptides were eluted with a lineal gradient from 5 to 45 % of the buffer B (0.2% of formic acid in acetonitrile) in 120 minutes.

The mass spectrometer was operated with cone and the capillary voltages of 35 and 3000 volts, respectively. For the acquisition of the MSMS spectra the singly-, doubly-, triply-charged precursory ions were selected automatically, once these they surpassed an intensity of 7 counts/sec. The measurement mode was changed from MSMS to MS when the total ion current (TIC) diminished to 2 counts/sec or when the spectra MS/MS was acquired during 4 seconds. The acquisition and the data processing were carried out by the software MassLynx (version 3.5, Micromass), while the identification of the proteins based on the MSMS spectra was through the version of the MASCOT software freely available in Internet. Among the search parameters, the cytein modification as well as the possible oxidations and desamidations were included.

### Example 4. Application of the proposed method to the quantitative proteomics of complex mixtures derived from the proteome of an organism (Vibrio cholerae) grown in aerobiosis and anaerobiosis conditions.

A colony of the strain of *V. cholerae* O1 biotype El Tor C7258 (Ogawa; Peru, 1991) grew during 16 h at 37°C in a LB medium (Sambrok, J., Fritsch, E. F., Maniatis, T., Molecular cloning: to manual laboratory, Cold Spring Harbor Laboratory Press, New York 1989, A.1) was inoculated in 5 mL of syncase broth (pH 7.5) (Finkelstein, R.A., Attasampunna, A., Chulasamaya, M., Charunmethee, P., J. Immunol. 1966, 96, 440-449) suplemented with 0.4% w/v of glucose and 1% w/v of casein hydrolyzate and grown overnight to 200 rpm at 37°C.

The culture was diluted 1:100 in a fresh syncase medium suplemented and precultured until an OD~0.2 before inoculating in the final aerobics and anaerobics culture used in the proteomics studies. The anaerobic atmosphere was generated using AnaeroGenTM sacks from the Oxoid company (Basingstoke, Hampshire, UK) until reducing the oxygen levels to values lower than 1% to generate a concentration of CO₂ between 9 and 13% during 30 min. Before proceeding to the definitive inoculation in the anaerobic culture, a 1 L erlenmeyer containing 200 mL of syncase was medium preconditioned during 30 minutes in anaerobiosis and later on it was inoculated with 2 mL of the strain C7258 precultivated and stirred to 220 rpm at 37°C until reaching an optic density of approximately 0.5. The cells were collected by centrifugation to 10 000 g during 5 minutes, washed twice with the electroporation buffer (270 mM sucrose, 1.3 mM Na₂HPO₄, 1 mM MgCl₂, pH 7.4), resuspended again in the same buffer, and centrifuged during three minutes at 10 000 g. Aliquot of 10¹⁰ cells were stored at -70 °C. Two aliquots (1x10⁷ cells) from the aerobic and anaerobic culture were dissolved separately in 1 mL of the lysis buffer (500 mM HEPES (pH 8.0), containing 10 mM EDTA, 2 mol/L guanidinium chloride). The complete disruption of the cells was carried out alternating ultrasound cycles (1 minute) and the incubation in a bathroom of ice. This procedure was repeated three times and later on the sample was pipeted continually several times until diminishing the viscosity of the solution. Finally streptomycin sulphate was added (Merck, Germany) until reaching a concentration of 1%, it was incubated during 15 minutes to 0 °C again, it was centrifuged at 500 g and the precipitate was discarded.

Later on, the complex mixture was analyzed following the steps described in the figure 1. The results of the identification and relative quantification are shown in the Table 3. Only two out of the 137 identified peptides do not classify as RH peptides (NPDGEVLR / protein No.62; and TRDNEVVAK */ protein No.72). These results confirm the high grade of specificity (superior to 98%). These results could be used to restrict the search in the protein sequence databases only to RH peptides and this contributes to avoid false-postives in the identifications.

Of the 91 identified proteins using the method of the present invention, 18 (it is equivalent to 19.8%) do not contain cystein in their amino acid sequences (proteins #2, 12, 13, 20, 22, 28, 29, 40, 41, 42, 44, 47, 48, 52, 60, 62, 68, and 88, see Table 4) and therefore they could not be identified using one of the most used methods in the quantitative proteomics, the ICAT (Quantitative analysis of complex protein mixtures using isotope-coded affinity tags. Gygi, S. P., Rist, B., Gerber, S. A., Turecek, F., Gelb, M. H., and Aebersold, R. Nat. Biotechnol. 17, 994-999, 1999; Simplification of complex peptide mixtures for proteomic analysis: reversible biotinylation of cysteinyl peptides. Spahr CS, Susin INC, Bures EJ, Robinson JH, Davis MT, McGinley MD, Kroemer G, Patterson SD. Electrophoresis, 21(9), 1635-1650, 2000). Aomng the ten proteins that changed their expression levels identified by the present method (proteins #8, 9, 33, 43, 46, 55, 58, 70, 88 and 89, Table 4) one of them (10%) could not be identify with the ICAT because it does not have cystein residues in its sequence (protein 88, Table 4) and this valuable information would be lost if the ICAT method is used.

These results demonstrate the superiority of the present method to study complex mixtures of proteins and it allows to identify non identifiable proteins by an established method such as the ICAT.

**Tabla 4. Summary of the selective isolation of the RH peptides present in hydrolysis of the total proteins of Vibrio cholerae grown in aerobic and anaerobic conditions.**

| # | Protein (MW / pl) | Peptides (sequence) ^{a)} | z | Mass (peptide) m/z exp_theor | %(d0_d3) ^{b)} |
|---|---|---|---|---|---|
| 1 | phospho-2-dehydro-3-deoxyheptonate aldolase, tyr-sensitive, VC0695 (39743 / 6.36) | AHQELIPFLHNR | 3 | 506.26_506.23 | 48.84_51.15 |
| | | RQPLVAEDVIHQIR | 3 | 572.60_572.64 | 51.37_48.63 |
| 2 | ribosomal protein L21, gi\|15640462, VC0435, (11542 / 9.99) | VVAEVVQHGR | 2 | 568.30_568.25 | 57.2_42.8 |
| 3 | inositol monophosphate family protein, VC0745 (31638 / 7.66) | AILQ**H**I**R** | 2 | 446.76_446.73 | 61.20_38.79 |
| 4 | ribosomal protein L28, VC0218 (9022 / 11.61) | FLPNLQT**HR** | 2 | 584.27_584.31 | 58.34_41.65 |
| | | **R**PAVGNN**R** | 2 | 463.20_463.25 | 63.0_37.0 |
| 5 | transcriptional activator HlyU, VC0678 (12327 / 8.66) | AMIELL**HR** | 2 | 512.26_512.77 | 55.00_44.99 |
| 6 | elongation factor TU, VC0362 (43141 / 5.04) | E**H**ILLG**R** | 2 | 440.21_440.25 | 55.32_44.37 |
| | | TK*PHVNVGTIGHVDHGK* | 3 | 627.28_627.33 | 54.02_45.98 |
| | | | 2 | 961.46_961.50 | 57.24_42.76 |
| 7 | valyl-tRNA synthetase, VC2503 (108144 / 5.1) | OLWWG**HR** | 2 | 514.22_513.24 | 44.76_55.23 |
| 8 | pyruvate dehydrogenase, E1 component, VC2414 (98951/9.52) | EFEYL**H**A**R** | 2 | 554.22_553.76 | 75.3_24.7 |
| | | AL**H**GYTPQ**R** | 2 | 542.74_542.78 | 72.7_27.3 |
| | | **H**DVDALETQEWLAALESVV**R** | 3 | 774.99_775.06 | 75.45_24.55 |
| 9 | pyruvate dehydrogenase, E2 component, dihydrolipoamide acetyltransferase, VC2413 (66077 / 5.04) | ISGANL**HR** | 2 | 455.21_455.24 | 76.97_23.02 |
| 10 | peptidoglycan-associated lipoprotein, VC1835 (65966 / 4.94) | VTIEG**H**ADE**R** | 2 | 584.73_584.77 | 48.00_51.99 |
| 11 | phosphoribosylformylglycinamidin e synthase, VC0869 (141374 / 5.12) | MPIAVS**H**GEG**R** | 2 | 598.25_598.29 | Espec_Inco |
| 12 | ribosomal protein L22, VC2591 (12233 / 9.91) | **R**SS**H**ITVVVAD**R** | 3 | 461.21_461.25 | 58.45_41.54 |
| 13 | ribosomal protein L1, VC0325 (2644 / 9.61) | GATVLPHGTGR | 2 | 554.24_554.29 | 58.0_42.0 |
| 14 | DNA-directed RNA polymerase alp, VC2571 (36515 / 4.85) | IHTEEDERPIG**R** | 3 | 498.53_498.57 | 59.17_44.82 |
| 15 | ribosomal protein L2gi\|15642588, VC2593 (29849 / 10.83) | GVRPTVR | 2 | 413.70_413.74 | 59.65_40.34 |
| | | GVVMNPVDHPHGGGEGR | 3 | 592.59_592.55 | 63.6_36.4 |
| | | ATIGEVGNAEHM*LR | 2 | 778.30_778.38 | 57.88_42.12 |
| | | ATIGEVGNAEHMLR | 2 | 770.34_770.38 | *52.37*_47.63 |
| 16 | Amidophosphoribosyltransferase, VC1004 (55904 / 5.75) | AISNVHR | 2 | 419.70_419.72 | 60.24_39.75 |
| 17 | spermidine/putrescine ABC transporter, periplasmic spermidine/putrescine-binding protein, VC1425 (40797 / 4.95) | EVF**H**IAL**R** | 2 | 513.73_513.78 | 56.35_43.65 |
| 18 | heat shock protein HtpG gi\|15641000, VC0985 (72194 / 5.26) | GTDIILHLR | 2 | 540.30_540.31 | 39.5_60.5 |
| 19 | rod shape-determining protein MreB, VC0415 (36920 / 5.04) | MLQ**H**FI**R** | 2 | 493.76_493.77 | 46.33_53.67 |
| 20 | conserved hypothetical protein, VC1055 (13395 / 5.72) | VTVTGS**H**SV**R** | 2 | 542.78_542.79 | 54.5_45.5 |
| 21 | ribosomal protein L3, gi\|15642591, VC2596 (22387 / 9.81) | **R**WNF**R** | 2 | 410.66_410.70 | 58.31_42.69 |
| | | TQDMT**H**GNSLSH**R** | 3 | 509.16_509.23 | 63.53_36.47 |
| 22 | ribosomal protein S2, VC2260 (26834 / 7.88) | DMLTAGV**H**FGHQT**R** | 3 | 537.92_538.20 | 56.07_43.93 |
| 23 | ribosomal protein S1, VC1915 (61057 / 4.86) | **R**NNVVVS**R** | 2 | 493.27_493.22 | 59.8_40.2 |
| | | AFLPGSLVDV**R**PI**R** | 2 | 791.39_791.46 | 57.06_42.94 |
| 24 | DNA-directed RNA polymerase, beta subunit, VC0328 (153253 / 5.22) | VIVSQL**HR** | 2 | 497.29_497.23 | 52.7_47.3 |
| | | GESGL**H**P**R** | 2 | 447.69_447.73 | 56.3_43.7 |
| | | TLAH**N**L**R** | 2 | 433.71 _433.75 | 54.26_45.74 |
| 25 | fructose-bisphosphate aldolase, class II, VC0478 (38919 / 4.91) | LNAIS**HR** | 2 | 426.76_426.73 | 43.2_56.8 |
| | | YV**H**NAEAYGVPVIL**H**TDHAAK* | 3 | 825.70_825.76 | 38.95_61.05 |
| 26 | enolase gi\|15642443, VC2447 (45820 / 5.03) | DAGYTAVIS**HR** | 2 | 616.29_616.30 | 34.75_65.4 |
| 27 | ribosomal protein S4, VC2572 (23387 / 9.99) | IDNAPGV**H**GA**R** | 2 | 574.73_574.78 | 48.0_51.9 |
| 28 | ribosomal protein L25, VC1640 (10525 / 6.82) | VE**H**M*DFI**R** | 2 | 552.75_552.71 | 57.65_42.35 |
| | | VE**H**MDFI**R** | 2 | 544.70_544.76 | 57.75_42.25 |
| | | VE**H**MDFI**R**I | 2 | 601.29_601.31 | 61.5_38.5 |
| 29 | ribosomal protein L32, VC2025 (6334 / 11.29) | **H**NVTAEGYY**R** | 2 | 626.22_626.28 | 63.24_36.76 |
| | | S**H**DALTAAALSVDATSGET**H**L**R** | 3 | 755.62_755.71 | 62.04_37.96 |
| 30 | ribosomal protein S21, VC0520 (8524 / 11.33) | VRENEPFDVALR | 2 | 743.88_743.81 | 60.48_39.52 |
| 31 | GDP-mannose 4,6-dehydratase, VC0243 (42053 / 5.93) | LHYGDLTDSSNLTR | 2 | 817.38_817.39 | 49.56_50.44 |
| 32 | lysyl-tRNA synthetase, heat inducible, VC0664 (58341 / 5.43) | HYNPNHAQIQALTEEDIQNR | 3 | 812.70_812.64 | 38.56_61.43 |
| | | LVMLLTNTHTIR | 2 | 727.34_727.41 | 40.49_59.51 |
| 33 | antioxidant, AhpC/Tsa family, VC0731 (22889 / 5.37) | AYDVEHPEAGVAFR | 2 | 801.87_801.79 | 73.61_26.39 |
| 34 | ribosomal protein S11, VC2573 (13923 / 11.33) | QVADGVAHIHASFNNTIVTITDR | 3 | 841.04_841.10 | 57.9_42.1 |
| 35 | elongation factor Tu, VC0321 (43200 / 5.09) | ARDFASIDNAPEER | 2 | 816.78_816.88 | 39.83_61.17 |
| | | TK*PHVNVGTIGHVDHGK* | 2 | 961.38_961.50 | 57.3_42.7 |
| 36 | acetyl-CoA carboxylase, carboxyl transferase alpha subunit, VC2244 (35639 / 5.9) | HIFTEFDELAGDR | 2 | 796.32_796.37 | 48.63_51.37 |
| 37 | DNA-directed RNA polymerase, alpha subunit, VC2571 (36435 / 4.83) | AEAIHYIGDLVQ**R** | 2 | 763.82_763.78 | 52.96_47.04 |
| 38 | ATP synthase F1, alpha subunit, VC2766 (56789 / 5.54) | EAFPGDVFYL**H**S**R** | 2 | 790.28_790.37 | 48.13_51.86 |
| 39 | DNA-directed RNA polymerase, beta'subunit, VC0329(154967 / 6.48) | EGLNVLQYFIST**H**GA**R** | 2 | 923.87_923.98 | 63.46_36.57 |
| | | HIETIVR | 2 | 455.20_455.26 | 55.4_44.6 |
| 40 | Ribosomal protein S16, VC0561 (9119 / 10.47) VC0561 | VN**H**WVSQGASLSD**R** | 2 | 798.80_798.30 | 60.24_39.76 |
| | | K***R**PFYQIVVADS**R** | 2 | 831.84_831.95 | 49.48_50.52 |
| | | | 3 | 554.94_554.97 | 54.45_45.55 |
| 41 | Ribosomal protein S6, VC0366 (14682/5.96) | A**H**YVLM*NVEADQAVVDELETA F**R** | 3 | 893.35_893.43 | 51.41_48.69 |
| | | AHYVLMNVEADQAVVDELETAF R | 3 | 887.99_888.10 | 60.2_39.80 |
| | | **H**YEIVFMV**H**PDOSEOVAGMIE**R** | 3 | 886.47_886.42 | 56.5_43.5 |
| 42 | phosphocarrier protein, VC0966 (9146 / 6.26) | EAVE**H**LVALM*DQL**H** | 2 | 831.81_831.90 | 40.17_59.83 |
| | | EAVE**H**LVALMDQL**H** | 2 | 823.82_823.90 | 37.55_62.45 |
| 43 | Alcohol dehydrogenase/acetaldehyde dehydrogenase, VC2033, (96214 / 6.08) | IMWVMYEHPDTHFEELAM**R** | 3 | 827.28_826.36 | 25.7_74.3 |
| | | *N*GIIFSP**H**P**R** | 2 | 590.75_590.85 | 23.27_76.73 |
| | | QTAFSQYDRPQAR | 2 | 805.39_805.39 | 20.7_79.3 |
| | | RAFLVTDR | 2 | 510.27_510.29 | 21.6_78.4 |
| 44 | ribosomal protein L4, VC2595 (21897 / 9.76) | EFNEALVHQVVVAYAAGAR | 2 | 1044.51_1044.00 | 47.4_52.6 |
| 45 | phosphoribosylamine-glycine ligase, VC0275 (45322/4.82) | RVGDADTGPNTGGMGAYSPAP VVTQDVHD**R** | 3 | 1033.75_1033.47 | 58.7_41.3 |
| 46 | thiamine biosynthesis protein ThiC, VC0061 (72629 / 5.55) | VTQLHYAR | 2 | 515.28_515.27 | 69.88_30.12 |
| 47 | ribosomal protein S7, VC0360 (17703 / 10.21) | VGGSTYQVPVEVRPVRR | 3 | 647.64_647.69 | 60.6_39.4 |
| | | AFAHY**R** | 2 | 403.66_403.70 | 63.11_36.89 |
| 48 | ribosomal protein S10, VC2597 (11742 / 9.69) | **R**TGAQV**R** | 2 | 415.24_415.20 | 59.4_40.6 |
| | | E**R**FTVLISPHVNK* | 2 | 812.38_812.45 | 58.6_41.4 |
| 49 | ATP synthase F1, gamma subunit, VC2765 (31862 / 8.34) | VIG**H**VANASLEY**R** | 2 | 735.85_735.88 | 60.2_39.8 |
| 50 | 2,3,4,5-tetrahydropyridine-2-carboxylate N-succinyltransferase, VC2329 (35647/6.14) | LQLIS**HR** | 2 | 454.74_454.77 | 58.36_31.64 |
| 51 | asparaginyl-tRNA synthetase, VC1297 (52455 / 5.07) | EFGIDPE**H**MDWY**R** | 2 | 868.82_868.87 | 57.3_42.7 |
| 52 | DNA-directed RNA polymerase, omega subunit, VC2709 (10054 / 4.99) | ERQEQQEQEAAELAAVSSIM***H** NR | 3 | 904.70_905.76 | 52.8_47.2 |
| 53 | chaperonin, 60 KDa subunit, VC2664 (57153 / 4.78) | AS**R**PLLIVAEDVEGEALATLVVN NM**R** | 3 | 942.07_941.5 | 42.7_52.3 |
| | | D**R**VEDALHAT**R** | 3 | 442.20_442.22 | 43.20_56.80 |
| 54 | ribosomal protein S9, VC0571 (14686/10.67) | ALMEYDESL**R**PVL**R** | 2 | 867.38_867.44 | 56.74_43.26 |
| | | | 3 | 578.58_578.65 | 56.63_43.37 |
| 55 | formate acetyltransferase, VC1866 (87770 / 6.01) | **R**AGAPFAPGANPMHG**R** | 3 | 550.22_550.28 | 21.94_78.05 |
| | | EEIAEQ**HR** | 2 | 527.22_527.24 | 21.6_78.4 |
| | | **H**MQFFGA**R** | 2 | 518.25_518.20 | 21.17_78.8 |
| | | **R**AIIP*N*GGI**R** | 2 | 555.28_555.32 | 17.8_82.2 |
| | | AGAPFAPGANPMHG**R** | 2 | 746.84_746.86 | 20.7_79.3 |
| 56 | 6-phosphofructokinase, isozyme I, VC2689 (35928/6.52) | ATVLG**H**IQ**R** | 2 | 518.75_518.79 | 38.44_61.56 |
| | | MGNYAV**H**LLMEG**H**GG**R** | 3 | 596.24_595.60 | 48.3_51.7 |
| 57 | ribosomal protein S8, VC2582 (13981 / 10.01) | VS**R**PGL**R** | 2 | 413.71_413.75 | 58.63_41.37 |
| 58 | triosephosphate isomerase, VC2670 (27971 / 5.03) | DFGAS**H**IIIGHSE**R** | 3 | 527.33_528.65 | 33.27_66.22 |
| 59 | inorganic pyrophosphatase, VC2545 (19630 / 4.87) | AQIT**H**FFE**R** | 2 | 595.75_595.79 | 65.68_34.31 |
| 60 | ribosomal protein L19, VC0564 (13278 / 10.23) | GL**H**SAFTV**R** | 2 | 515.22_515.27 | 57.65_42.34 |
| | | TFQT**H**SPVVDSIEV**K*** | 2 | 864.89_864.94 | 53.6_46.4 |
| 61 | Elongation factor Tu-A (EF-Tu-A), VC0321, (43185 / 5.09) | E**H**ILLG**R** | 2 | 440.21_440.25 | 62.03_37.96 |
| | | GY**R**PQFYF**R** | 2 | 638.23_638.32 | 56.57_43.43 |
| 62 | MSHA biogenesis protein MshN, VC0404 (42853 / 6.24) | NPDGEVL**R** | 2 | 471.22_471.24 | 60.43_39.57 |
| 63 | threonyl-tRNA synthetase, VCA0287 (73443 / 5.92) | ALQA**H**LT**R** | 2 | 476.21_476.26 | 55.52_44.27 |
| | | LIPVMI**HR** | 2 | 510.80_510.74 | 52.6_47.4 |
| 64 | thiamin biosynthesis protein ThiC, VC0061 (72629 / 5.55) | VTQL**H**YA**R** | 2 | 515.25_515.27 | 57.52_42.42 |
| 65 | Methylenetetrahydrofolate dehydrogenase/methenyftetrahyd rofolate cyclohydrolase, VC1942 (33589 / 8.19) | DVDGF**H**PYNVG**R** | 2 | 709.29_709.32 | 53.3_46.7 |
| 66 | NADH:ubiquinone oxidoreductase, Na translocating, alpha subunit, VC2295 (50638 / 6.95) | ATGPHAYLGR | 2 | 542.74_542.78 | 62.75_37.25 |
| | | Y**H**LOVSVL**R** | 2 | 578.78_578.83 | 52.82_47.18 |
| 67 | DnaK protein, VC0855 (68761 / 4.82) | NQADQMI**H**AT**R** | 2 | 663.79_663.81 | 53.07_46.93 |
| 68 | 50S ribosomal protein L 18, VC2580 (12685 / 10.97) | SGFOY**H**G**R** | 2 | 497.21_497.23 | 49.3_50.7 |
| 69 | hypothetical protein, VCA0374 (39390/4.93) | K*VITSV**HR** | 2 | 512.25_512.30 | 61.4_38.6 |
| 70 | glyceraldehyde-3-phosphate dehydrogenase, VC2000 (35283 / 6.06) | YDST**H**G**R** | 2 | 439.19-439.16 | 32.7_67.3 |
| 71 | ATP synthase alpha chain, VC2766 (55649 / 5.82) | RQLT**H**GQK* | 2 | 526.26_526.29 | 45.4_54.6 |
| 72 | ribosomal protein L17, VC2570 (14437/11.07) | T**R**DNEVVAK* | 2 | 537.28_537.26 | 54.8_45.2 |
| | | TDSVAN**RR** | 2 | 480.72_480.75 | 60.1_39.9 |
| 73 | 30S ribosomal subunit protein S3, VC2590 (25596/10.63) | VHPNGI**R** | 2 | 417.71_417.73 | 56.7_43.3 |
| | | RAVQNAM**R** | 2 | 494.23_494.26 | 56.2_43.8 |
| 74 | superoxide dismutase, Fe, VC2045 (21483 / 5.39) | **HH**NTYVV**K*** | 2 | 541.25_541.27 | 60.05_39.95 |
| 75 | ribonuclease E, VC2030 (117525 / 7.47) | LV**R**PDFVN**R** | 2 | 579.31_579.32 | 54.82_45.18 |
| 76 | ATP synthase F1, beta subunit, VC2764 (50520 / 4.85) | AIVMGSSDGL**RR** | 2 | 652.30_652.34 | 50.5_49.5 |
| 77 | GMP synthase, VC0768 (57806 / 5.11) | HPFPGPGLGV**R** | 2 | 588.28_588.32 | 51.34_48.66 |
| 78 | NAD(P) transhydrogenase, alpha subunit, VCA0563 (54007 / 6.52) | AWEAAHEFG**R** | 2 | 614.28_614.31 | 46.07_56.93 |
| 79 | elongation factor Ts, VC2259 (30837 / 5.22) | **H**VAM**H**VAAS**R**PEFLTPDDVPAE VVAK* | 3 | 957.41_957.49 | 52.9_47.1 |
| | | **H**VAM***H**VAAS**R**PEFLTPDDVPA EVVAK* | 3 | 962.70_962.82 | 50.26_49.74 |
| 80 | asparagine synthetase B, glutamine-hydrolyzing, VC0991 (62362 / 5.70) | AILAHE**R** | 2 | 426.22_426.24 | 54.02_45.98 |
| 81 | ribosomal protein L35, VCA0289 (7331 / 11.63) | RQLRPNAILPK* | 2 | 695.38_695.42 | 58.8_41.2 |
| | | RHILTK* | 2 | 426.20_426.26 | 66.96_33.04 |
| 82 | 2',3'-cyclic-nucleotide 2'-phosphodiesterase, VC2562 (75032 / 5.47) | ALVEADATMVQALOHDHQGTR | 3 | 778.32_778.38 | 44.25_55.75 |
| 83 | glutamate-ammonia ligase, VC2746 (51751 / 4.99) | YVVHNVAHAFGK* | 2 | 713.33_713.37 | 50.5_49.5 |
| | | EQHISIPAHQIDADFFE***N***GK | 3 | 780.31_780.37 | 50.8_49.1 |
| | | GHRPGVK* | 2 | 471.68_417.73 | 52.3_47.7 |
| 84 | ribosomal protein L14, VC2586 (13581 / 10.99) | **R**PDGSVI**R** | 2 | 471.22_471.26 | 57.21_42.89 |
| 85 | carbamoyl-phosphate synthase, large subunit, VC2389 (117912 / 5.13) | AL**R**EEGY**R** | 2 | 518.20_518.26 | 65.59_34.41 |
| | | AMEIVYDEQDL**RR** | 2 | 840.33_840.41 | 60.04_39.96 |
| | | AM*EIVYDEQDL**RR** | 2 | 848.32_848.40 | 63.55_36.45 |
| 86 | elongation factor G, VC2342 (76482 / 5.06) | VDVFTPEDHVGDVIGDLN**RR** | 3 | 765.64_765.72 | 56.06_43.94 |
| 87 | initiation factor IF-2, VC0643 (98705 / 5.73) | APVVTIM*GHVDHGK* | 2 | 780.82_780.90 | 57.26_42.74 |
| 88 | accessory colonization factor AcfC, VC0841 (28575 / 8.60) | FNIHPVFMR | 2 | 603.29_603.30 | 11.19_88.80 |
| 89 | succinate dehydrogenase, flavoprotein subunit, VC2089 (64510 / 6.45) | LK*LD**H**LG**R** | 2 | 518.24_518.30 | 69.95_30.05 |
| 90 | fructose-1,6-bisphosphatase, VC2544 (37318 / 5.88) | YIGSLVADF**HR** | 2 | 660.31_660.34 | 49.8_50.2 |
| 91 | phenylalanyl-tRNA synthetase, beta Chain, VC1220 (86079 / 5.09) | GVDYALQ**H**AAME**R** | 2 | 751.81_751.86 | 50.6_49.4 |

| | | | | | |
|---|---|---|---|---|---|
| *a) K * represents the acetylated lysine at the epsilon amino group. All the identified peptides possesses acetyl groups in the amino terminal end. N represents a completely desamidated asparagines residue. In boldfaces the basic amino acids arginine and histidine are highlighted.* *b) The peptides obtained in the aerobic and anaerobiosis conditions were derivatized with normal ((CH3CO)2O) and deuterado ((CD3CO)20) acetic anhydrides, respectively. The proportion between both species is expressed by percentages.* *c) The sequences (8, 9, 33, 46, 89) and (43, 55, 58, 70, 88) correspond to the proteins overexpressed in anaerobiosis and aerobiosis conditions, respectively.* | | | | | |

### Example 5. In silico determination of the content of blocking groups grupos in the RH peptides of different genomes.

The presence of several blocking groups in the structure of the RH peptides when the irreversible blocking of the amino groups is used and in particular when deuterated acetic anhydride is used could be a concern mainly for three reasons:
1 - As increase the number of blocking groups in the structure of the RH peptides, the losses during the whole process could be higher because they are transformed into more hydrophobic species.
2 - As increase the number of blocking groups, the number of deuterium atoms in the sequence of the RH peptides will be higher and therefore the separation of the light and heavy species in reverse phase chromatography increases as well as the errors in their relative quantification under the compared conditions (Zhang R, Sioma CS, Wang S, Regnier FE. Fractionation of isotopically labeled peptides in quantitative proteomics. Anal Chem. 2001, 73, 5142-5149; Zhang R, Sioma CS, Thompson RA, Xiong L, Regnier FE. Controlling deuterium isotope effects in comparative proteomics. Anal Chem. 2002, 74, 3662-3669).
3 - The presence of one or several blocking groups can affect the fragmentation of the RH peptides in the collisions induced dissociation experiments in the mass spectrometer.

All the RH peptides should possess a blocking group at least in its N-terminal end and the presence of additional blocking groups is due to the presence of lysine residues (for each lysine residual a blocking group is added). The presence of lysine in the RH peptides can originate for three reasons:
a) an incomplete proteolysis
b) presence of sites resistant to the proteolytic cleavage of trypsin due to the presence of a proline contiguous to the cleavage site (K-P).
c) RH peptides ending with lysine at their C-terminal end and possess inside their sequence multiple histidine and/or arginine residues.
   The first of these factors (a) can be minimized by means of the optimization of the proteolytic digestion using a higher enzyme to substrate ratio or prolonged the digestion times.

The last factors (b and c) are an intrinsic property of each proteome and it depends on each one of the analyzed proteins, on the relative distribution of the proline residues contiguous to lysine residues (K-P) as well as of the presence of several basic residues in the tryptic peptides.

To know which is the abundance of K-P residues and the quantity of blocking groups, the sequences of all the RH peptides of several genomes were analyzed (Figures 7). The results demonstrate that approximately 80% of the RH peptides adds only one blocking group. Around 20% add two blocking groups and the addition of three or more blocking groups is irrelevant.

Of the 137 RH peptides isolated experimentally in the proteome of *V. cholerae* grown in aerobic and anaerobic conditions (Table 4, example 4) it can be appreciate that 119 added only one acetyl group, 16 added two acetyl groups and only 2 RH peptides more than three, that is equivalent to a 86.8, 11.6 and 1.5%, respectively.

These results are similar to those obtained in the predictions carried out by an *in silico* analysis for the genome of this organism and similar to the other genomes shown in the figure 7.

Zhang and coworkers (Zhang R, Sioma CS, Wang S, Regnier FE. Fractionation of isotopically labeled peptides in quantitative proteomics. Anal Chem. 2001, 73, 5142-5149; Zhang R, Sioma CS, Thompson RA, Xiong L, Regnier FE. Controlling deuterium isotope effects in comparative proteomics. Anal Chem. 2002, 74, 3662-3669) they demonstrated that the presence of a single deuterated acetyl group does not affect the relative quantification of the labeled and non-labeled species, when deuterium labeling is used in the quantification it would not be a problem for our method since most of the peptides added one acetyl group (e deuterium atoms) but in the case of ICAT method all peptides adds 8 deuterium atoms and the errors in the quantification could be appreciable (Zhang R, Sioma CS, Wang S, Regnier FE. Fractionation of isotopically labeled peptides in quantitative proteomics. Anal Chem. 2001, 73, 5142-5149). The addition of two acetyl groups (6 deuterium atoms) present in the remaining 20% of the RH peptides of several genomes (Figures 7) it would be even inferior to that added by all the ICAT-labeled peptides (8 deuterium atoms) and the errors using the method object of this invention will be inferior.

Lastly, the MASCOT software was able to identify in an automatic mode all the RH peptides of the rSK protein that possessed one and two blocking groups (figures 8 and 9). This result demonstrates that the presence of multiple blocking groups in the sequence of the RH peptides does not affect the efficiency of its fragmentation in the collision induced dissociation experiments because the RH peptides are multiply-charged species (R+H>1) with multiple sites of protonation and consequently the automatic and efficient identification of the RH peptides is guaranteed in the protein sequence databases by using one of the most popular search engines, the MASCOT program.

### Example 6. Simultaneous relative quantification of proteins present in several simples in a single experiment.

The RH peptides isolated in the proposed method can be blocked at their N-terminal end with light and heavy isotopes to carry out the relative quantification. Taking into account, the results shown in the example 3 of the present invention, where it is demonstrated that the ISOTOPICA software can be used independently of the type of introduced labeling and the degree of overlapping of the isotopic distributions, in this example it is demonstrated that the relative quantification of proteins can be carried out in a simultaneous way under three or more biological conditions in a single experiment whenever a different isotopic labeling is used for the RH peptides in each one of the compared conditions.

Three artificial mixtures (A, B and C) composed by the proteins rSK, cytochrome-C and myoglogin were prepared. The proteins rSK, cytochrome C and myoglobine in the mixtures A, B and C kept the proportion of 1:1:1, 1:2:3 and 1:4:5, respectively. Later on, it proceeded to the isolation of the RH peptides of the proteins present in each one of the samples. The tryptic peptides of the proteins present in the mixtures A, B and C were derivatized with normal acetic anhydride [(CH₃CO)₂O], acetic anhydride labeled with an atom of carbon-13 [C³CH₃CO)₂O and acetic anhydride labeled with two atoms of carbon-13[(C³CH₃¹³CO)₂O], respectively. Before the desalting step, the three mixtures of blocked peptides were mixed and the procedure continued in the same way until the identification process. The blocked tryptic peptides of the proteins present in the mixture "C" are 1 heavier than the the blocked peptides of the mixture "B" and these in turn are 1 Da heavier than the peptides obtained in the mixture "A". When A, B and C are mixed the isotopic distributions of the peptides from the same proteins are completely overlaped and the content of ¹³C is an reflect the relative quantities of the peptides present in each one of the compared conditions.

In the Table 5 are summarized the results obtained when applying the method for the selective isolation of RH peptides present in the artificial mixture of the three proteins. As they can be appreciated a very good agreement exists among the experimental isotopic distributions and those provided by the software when mixing in the appropriate proportions the theoretical distributions of the peptides. Particularly these values keep very good agreement with the expected values for each one of the proteins taking into consideration the proportions in which they were prepared in the three artificial mixtures.

**Table 5. Simultaneous relative quantification of three proteins present in three artificial mixtures when carrying out a single experiment.**

| Proteins | Peptide Sequence ^{a)} | Theoretical ratio (A : B : C) | Experimental ratio |
|---|---|---|---|
| rSK | ¹²³DGSVTLPTQPVQEFLLSG***H***V***R***¹⁴³ | 1 : 1 : 1 | 1 : 0.94 : 0.98 |
| | ²¹¹T***H***PGYTIYE***R***²²⁰ | | 1 : 1.14 : 1.17 |
| | ²²¹DSSIVT***H***DNDIF***R***²³³ | | 1 : 1.06 : 1.03 |
| | ²⁸⁴PYDPFD***R***S***H***LK*²⁹⁴ | | 1 : 0.99 : 1.02 |
| | ³²¹NLDF***R***DLYDP***R***³³¹ | | 1 : 1.13 : 1.09 |
| | ³⁷³***R***PEGENASY***H***LAYDK*³⁸⁷ | | 1 : 0.90 : 0.94 |
| | ³⁸⁸D***R***VTEEE***R***EVYSYL***R***⁴⁰² | | 1 : 0.83 : 0.95 |
| | | | **1 : 0.99 : 1.02** |
| Cytochrome-C | ²⁸TGPNL***H***GLFG***R***³⁸ | 1 : 4 : 5 | 1 : 4.30 : 5.2 |
| | ²⁸TGPNL***H***GLFG***R***K*³⁹ | | 1 : 4.30 : 5.2 |
| | | | 1 : 4.40 : 5.5 |
| | | | **1 : 4.70 : 5.6** |
| Myoglobin | ¹⁷VEADIAG***H***GQEVLI***R***³¹ | 1 : 2 : 3 | 1 : 2.02 : 2.86 |
| | ⁷⁹K*G***HH***EAELK*PLAQS***H***ATK*⁹⁶ | | 1 : 2.18 : 2.99 |
| | | | **1 : 2.08 : 2.88** |

| | | | |
|---|---|---|---|
| *a) In boldface the amino acids arginine and histidine are highlighted.* | | | |

In the figure 8, the good adjustment carried out by the ISOTOPICA program for the peptides of each one of the proteins can be appreciated.

In principle the comparison can be extended to a greater number samples for which it is only necessary to introduce an isotopic labeling in each one of the conditions in a such way that their molecular masses are different in at least in 1 Da. For example, to be able to compare four conditions it would be necessary to use the same three anhydrides shown in this example and the RH peptides derived from the fourth condition should be modified with deuterated acetic anhydride [(C²H₃CO)O]. It is also possible to be compared 5 and 6 conditions if doubly labeled acetic anhydrides with deuterium and ¹³C ( [(¹³C²H₃CO)O] and [C³C²H₃¹³CO)O],) are used, respectively. This application would have great utility in proteomic experiments where it is necessary the comparison of multiple conditions either as internal controls of the system or to study synergistic effect of diverse drugs in cell lines, tumors, mircrorganisms, etc

If it is desirable to compare the expression of proteins under three different conditions using two-dimensional electrophoresis it would be necessary to obtain analytic three gels for each one of the conditions to evaluate the reproducibility of the experiment because of the exquisite manipulation that requires this analytic technique and later on, it would be necessary to obtain two preparative gels to proceed to the identification by mass spectrometry.

It is evident, the great consumption of reagents, samples and time that it would require a study of such a magnitude. If LC-MSMS techniques are used, it would be necessary to carry out two experiments at least, when comparing one of the samples with the other two remaining ones. However, the possibility to introduce different labeling in the RH peptides isolated in each one of the conditions allows to obtain information of the relative quantification of each one of the proteins present in the mixtures in an single experiment.

This minimizes the inter-experiments errors in the relative quantification and brings a considerable simplification of the work to be carry out. Particularly this advantage is more appreciable in the same measure as increase the number of samples to be compared.

## Claims

1. Method for the identification and relative quantification of one or several proteins in complex mixtures, **characterized** because at acidic pH are selectively isolated the multply-charged peptides in those that the sum of the number of hisitidine residues (H) and/or arginine residues (R) in their sequences are higher than one (R+H>1), denominated here as RH peptides, and the determination of the relative concentration of the protein or the proteins is carried out from the ratio between the areas of theoretical sepectra estimated for each RH peptide labeled with different isotopes in the compared samples, which consists of the following steps:
a) Desnaturalizatión and alkylation of the cystein residues of the proteins present in the analyzed complex mixture and proteolytic hidrolysis with trypsin or any other protease or chemical treatment that hidrolyze in a specific or predictable way the peptide bonds.
b) reversible or irreversible chemical modification of the alpha and epsilon amino groups (α and ε-NH₂) of the peptides obtained in the step (a)
c) selective isolation of the RH peptides by cation exchange chromatography of the mixture of modified peptides obtained in the step (b) previous to the analysis by mass spectrometry.
d) Elimination of O-acylations at the tyrosine residues and deblocking groups of the amino groups by an acidic or basic treatment previous to the analysis by mass spectrometry. In particular, the blocking of the amino groups is eliminated when a transitory or a reversible modification is introduced in the step (b).
e) Identification of the proteins by the mass spectromety analysis coupled to the liquid chromatography of the RH peptides selectively isolated in the step (c).
f) differential isotopic labeling of the samples of proteins previous to the step (a) or labeling of peptides during the steps (a) or (b), or during the hydrolysis step described in (d), mixing immediately at least a portion of the compared samples that contain identical quantities of proteins.
g) relative quantification of one or several proteins in the mixtures of the step (e) from the ratio between the areas of estimated theoretical mass spectra of the RH peptides identified in the step (e), as well as for the peptide fragments of these peptides generated in the same step (e) that contain the introduced isotopic labeling.

2. The method described in the claim 1, step (b), characterizaed because the modifying agents of amino groups could be: acetic anhydride, N-hydroxysuccinimide, N-acetoxysuccinimide, citraconic anhydride, maleic anhydride, succinic anhydride, ftalic anhydride, tetrahydroftalic anhydride, 9-fluorenylmethyl chloroformate (Fmoc-CI), 2-methyl sulfonyl ethyl succinimidyl carbonate), urea and reagents that provides protecting amino groups such as:
(a) aromatic urethane-type protecting groups which include benzyloxycarbonyl, 2-chlorobenzyloxycarbonyl, 9-fluorenylmethyloxycarbonyl, isonicotinyloxycarbonyl and 4-methoxybenzyloxycarbonyl; (b) aliphatic urethane-type protecting groups which include t-butoxycarbonyl, t-amyloxycarbonyl, isopropyloxycarbonyl, 2-(4-biphenyl)-2-propyloxycarbonyl, allyloxycarbonyl and methylsulfonylethoxycarbonyl; (c) cycloalkyl urethane-type protecting groups which include adamantyloxycarbonyl, cyclopentyloxycarbonyl, cyclohexyloxycarbonyl and isobornyloxycarbonyl; (d) acyl protecting groups or sulfonyl protecting groups. Preferred protecting groups include benzyloxycarbonyl, t-butoxycarbonyl, acetyl, 2-propylpentanoyl, 4-methylpentanoyl, t-butylacetyl, 3-cyclohexylpropionyl, n-butanesulfonyl, benzylsulfonyl, 4-methylbenzenesulfonyl, 2-naphthalenesulfonyl, 3-naphthalenesulfonyl and 1-camphorsulfonyl; (e) photosensitive protective groups which include carbamates derivatives from m-nitrophenyl, 3,5-dimetoxybenzyl, 1-methyl-1(3,5-dimetoxyphenyl)etyl, α-methylnitropiperonyl, o-nitrobenzyl, 3,4-dimetoxy-6-nitrobenzyl, phenyl(o-nitrophenyl)methyl, 2-(2-nitrophenyl)etyl, 6-nitroveratryl, 4-metoxyfenacyl and 3',5'-dimetoxybenzoine and other reagents used in the pepetide synthesis for the protection reversible or irreversible of primary amino groups.

3. The method in agreement with the claim 1, step (c) **characterized by** the determination of the relative concentration of one or several proteins in the samples from their blocked or non-blocked RH peptides by calculating the ratio of the areas corrresponding to the theoretical spectra of the isotopically labeled and non-labeled species that are adjusted in the better way to the mass spectrum obtained experimentally or from the theoretical spectra of fragments of these peptides generated in the step (f) and that containing the introduced isotopic labeling.

4. the method in agreement with the claim 1, step (b) **characterized by** the usage of multiple modifier agents in a same experiment, so many as conditions to be analyzed.


### Annex to the application documents - subsequently filed sequences listing

LISTA DE SECUENCIAS

<110> CENTRO DE INGENIERIA GENETICA Y BIOTECNOLOGIA

<120> METODO PARA EL ASILAMIENTO SELECTIVO DE PEPTIDOS
      MULTICARGADOS EN MEZCLAS PROTEICAS COMPLEJAS.

<130> Patente

<140>
<141>

<160> 94

<170> PatentIn Ver. 2.1

<210> 1
<211> 415
<212> PRT
<213> Streptococcus equisimilis

<220>
<221> PEPTIDE
<222> (1)..(415)

<400> 1
Met Ile Ala Gly Pro Glu Trp Leu Leu Asp Arg Pro Ser Val Asn Asn
  1               5                  10                  15

Ser Gln Leu Val Val Ser Val Ala Gly Thr Val Glu Gly Thr Asn Gln
             20                  25                  30

Asp Ile Ser Leu Lys Phe Phe Glu Ile Asp Leu Thr Ser Arg Pro Ala
         35                  40                  45

His Gly Gly Lys Thr Glu Gln Gly Leu Ser Pro Lys Ser Lys Pro Phe
     50                  55                  60

Ala Thr Asp Ser Gly Ala Met Pro His Lys Leu Glu Lys Ala Asp Leu
 65                  70                  75                  80

Leu Lys Ala Ile Gln Glu Gln Leu Ile Ala Asn Val His Ser Asn Asp
                 85                  90                  95

Asp Tyr Phe Glu Val Ile Asp Phe Ala Ser Asp Ala Thr Ile Thr Asp
            100                 105                 110

Arg Asn Gly Lys Val Tyr Phe Ala Asp Lys Asp Gly Ser Val Thr Leu
        115                 120                 125

Pro Thr Gln Pro Val Gln Glu Phe Leu Leu Ser Gly His Val Arg Val
    130                 135                 140

Arg Pro Tyr Lys Glu Lys Pro Ile Gln Asn Gln Ala Lys Ser Val Asp
145                 150                 155                 160

Val Glu Tyr Thr Val Gln Phe Thr Pro Leu Asn Pro Asp Asp Asp Phe
                165                 170                 175

Arg Pro Gly Leu Lys Asp Thr Lys Leu Leu Lys Thr Leu Ala Ile Gly
            180                 185                 190

Asp Thr Ile Thr Ser Gln Glu Leu Leu Ala Gln Ala Gln Ser Ile Leu
        195                 200                 205

Asn Lys Thr His Pro Gly Tyr Thr Ile Tyr Glu Arg Asp Ser Ser Ile
    210                 215                 220

Val Thr His Asp Asn Asp Ile Phe Arg Thr Ile Leu Pro Met Asp Gln
225                 230                 235                 240

Glu Phe Thr Tyr His Val Lys Asn Arg Glu Gln Ala Tyr Glu Ile Asn
                245                 250                 255

Lys Lys Ser Gly Leu Asn Glu Glu Ile Asn Asn Thr Asp Leu Ile Ser
            260                 265                 270

Glu Lys Tyr Tyr Val Leu Lys Lys Gly Glu Lys Pro Tyr Asp Pro Phe
        275                 280                 285

Asp Arg Ser His Leu Lys Leu Phe Thr Ile Lys Tyr Val Asp Val Asn
    290                 295                 300

Thr Asn Glu Leu Leu Lys Ser Glu Gln Leu Leu Thr Ala Ser Glu Arg
305                 310                 315                 320

Asn Leu Asp Phe Arg Asp Leu Tyr Asp Pro Arg Asp Lys Ala Lys Leu
                325                 330                 335

Leu Tyr Asn Asn Leu Asp Ala Phe Gly Ile Met Asp Tyr Thr Leu Thr
            340                 345                 350

Gly Lys Val Glu Asp Asn His Asp Asp Thr Asn Arg Ile Ile Thr Val
        355                 360                 365

Tyr Met Gly Lys Arg Pro Glu Gly Glu Asn Ala Ser Tyr His Leu Ala
    370                 375                 380

Tyr Asp Lys Asp Arg Tyr Thr Glu Glu Glu Arg Glu Val Tyr Ser Tyr
385                 390                 395                 400

Leu Arg Tyr Thr Gly Thr Pro Ile Pro Asp Asn Pro Asn Asp Lys
                405                 410                 415


<210> 2
<211> 153
<212> PRT
<213> Equus caballus

<220>
<221> PEPTIDE
<222> (1)..(153)

<400> 2
Val Leu Ser Glu Gly Glu Trp Gln Leu Val Leu His Val Trp Ala Lys
1               5                   10                  15

Val Glu Ala Asp Val Ala Gly His Gly Gln Asp Ile Leu Ile Arg Leu
            20                  25                  30

Phe Lys Ser His Pro Glu Thr Leu Glu Lys Phe Asp Arg Phe Lys His

        35                  40                  45

Leu Lys Thr Glu Ala Glu Met Lys Ala Ser Glu Asp Leu Lys Lys His
    50                  55                  60

Gly Val Thr Val Leu Thr Ala Leu Gly Ala Ile Leu Lys Lys Lys Gly
65                  70                  75                  80

His His Glu Ala Glu Leu Lys Pro Leu Ala Gln Ser His Ala Thr Lys
                85                  90                  95

His Lys Ile Pro Ile Lys Tyr Leu Glu Phe Ile Ser Glu Ala Ile Ile
            100                 105                 110

His Val Leu His Ser Arg His Pro Gly Asp Phe Gly Ala Asp Ala Gln
        115                 120                 125

Gly Ala Met Asn Lys Ala Leu Glu Leu Phe Arg Lys Asp Ile Ala Ala
    130                 135                 140

Lys Tyr Lys Glu Leu Gly Tyr Gln Gly
145                 150

<210> 3
<211> 104
<212> PRT
<213> Equus caballus

<220>
<221> PEPTIDE
<222> (1)..(104)

<400> 3

<210> 4
<211> 357
<212> PRT
<213> Vibrio cholerae

<220>
<221> PEPTIDE
<222> (1)..(357)
<223> >tr|Q9KU34|Q9KU34_VIBCH
      Phospho-2-dehydro-3-deoxyheptonate aldolase,
      tyr-sensitive - Vibrio cholerae (357 aa).

<400> 4
Met Lys Lys Ser Glu Leu Ser Asp Val Asn Ile Ser Asn Glu Gln Ile
1               5                   10                  15

Leu Ile Thr Pro Asp Ala Leu Lys Ala Lys Ile Pro Leu Ser Asp Lys
            20                  25                  30

Ala Arg Lys Phe Ile Arg Glu Ser Arg Gln Thr Val Ala Asp Ile Ile
        35                  40                  45

His Lys Arg Asp Pro Arg Leu Leu Ile Val Cys Gly Pro Cys Ser Ile
    50                  55                  60

His Asp Val Asp Ala Ala Lys Glu Tyr Ala Lys Lys Leu Lys Ala Leu
65                  70                  75                  80

Ser Ala Gln Leu Ser Asp Gln Leu Tyr Ile Val Met Arg Val Tyr Phe
                85                  90                  95

Glu Lys Pro Arg Thr Thr Val Gly Trp Lys Gly Leu Ile Asn Asp Pro
            100                 105                 110

His Leu Asp Gly Ser Phe Asp Ile Glu His Gly Leu His Val Gly Arg
        115                 120                 125

Gln Leu Leu Val Asp Leu Ala Glu Met Glu Ile Pro Leu Ala Thr Glu
    130                 135                 140

Ala Leu Asp Pro Ile Ser Pro Gln Tyr Leu Ala Asp Thr Phe Ser Trp
145                 150                 155                 160

Ala Ala Ile Gly Ala Arg Thr Thr Glu Ser Gln Thr His Arg Glu Met
                165                 170                 175

Ala Ser Gly Leu Ser Met Pro Ile Gly Phe Lys Asn Gly Thr Asp Gly
            180                 185                 190

Asn Leu Ala Thr Ala Ile Asn Ala Met Gln Ala Ala Ser Ser Ser His
        195                 200                 205

Arg Phe Met Gly Ile Asn Arg Glu Gly Gln Val Ala Leu Leu Thr Thr
    210                 215                 220

Gln Gly Asn Pro Asn Gly His Val Ile Leu Arg Gly Gly Lys Gln Thr
225                 230                 235                 240

Asn Tyr Asp Ser Val Ser Val Ala Glu Cys Glu Glu Glu Met Gln Lys
                245                 250                 255

Ala Arg Leu Glu Pro Ser Leu Met Val Asp Cys Ser His Ala Asn Ser
            260                 265                 270

Arg Lys Asp Tyr Arg Arg Gln Pro Leu Val Ala Glu Asp Val Ile His
        275                 280                 285

Gln Ile Arg Glu Gly Asn Arg Ser Ile Ile Gly Leu Met Ile Glu Ser
    290                 295                 300

His Leu Asn Glu Gly Asn Gln Ser Ser Asp Leu Pro Arg Glu Lys Met
305                 310                 315                 320

Gln Tyr Gly Val Ser Ile Thr Asp Gly Cys Ile Asn Trp Ser Thr Thr
                325                 330                 335

Glu Ala Leu Leu Arg Arg Ala His Gln Glu Leu Ile Pro Phe Leu His
            340                 345                 350

Asn Arg Leu Gln Gly
        355

<210> 5
<211> 103
<212> PRT
<213> Vibrio cholerae

<220>
<221> PEPTIDE
<222> (1)..(103)
<223> >tr|Q9KUT0|Q9KUT0_VIBCH Ribosomal protein L21 -
      Vibrio cholerae. (103 aa)

<400> 5
Met Tyr Ala Val Phe Gln Ser Gly Gly Lys Gln His Arg Val Ser Glu
  1               5                  10                  15

Gly Gln Thr Leu Arg Leu Glu Lys Leu Asp Val Glu Thr Gly Ala Thr
             20                  25                  30

Val Glu Phe Asp Lys Val Leu Leu Val Ala Asn Gly Glu Asp Ile Lys
         35                  40                  45

Val Gly Ala Pro Leu Val Glu Gly Gly Lys Val Val Ala Glu Val Val
     50                  55                  60

Gln His Gly Arg Gly Asp Lys Val Lys Ile Val Lys Phe Arg Arg Arg
 65                  70                  75                  80

Lys His Ser Arg Lys Gln Gln Gly His Arg Gln Trp Phe Thr Glu Val
                 85                  90                  95

Lys Ile Thr Gly Ile Asn Ala
            100

<210> 6
<211> 267
<212> PRT
<213> Vibrio cholerae

<220>
<221> PEPTIDE
<222> (1)..(267)
<223> >sp|Q9KTY5|SUHB_VIBCH Inositol-1-monophosphatase
      (EC 3.1.3.25) (IMPase) (Inositol-1- phosphatase)

(I-1-Pase) - Vibrio cholerae. (267 aa)

<400> 6
Met His Pro Met Leu Asn Ile Ala Ile Arg Ala Ala Arg Lys Ala Gly
1               5                   10                  15

Asn His Ile Ala Lys Ser Leu Glu Asn Ala Glu Lys Ile Gln Thr Thr
            20                  25                  30

Gln Lys Gly Ser Asn Asp Phe Val Thr Asn Val Asp Lys Glu Ala Glu
        35                  40                  45

Ala Ile Ile Val Ser Thr Ile Lys Ser Ser Tyr Pro Glu His Cys Ile
    50                  55                  60

Ile Ala Glu Glu Gly Gly Leu Ile Glu Gly Lys Asp Lys Glu Val Gln
65                  70                  75                  80

Trp Ile Ile Asp Pro Leu Asp Gly Thr Thr Asn Phe Val Lys Gly Phe
                85                  90                  95

Pro His Phe Ala Val Ser Ile Ala Val Arg Phe Arg Gly Lys Thr Glu
            100                 105                 110

Val Ala Cys Val Tyr Asp Pro Met Thr Asn Glu Leu Phe Thr Ala Gln
        115                 120                 125

Arg Gly Ala Gly Ala Gln Leu Asn Asn Ala Arg Ile Arg Val Gln Pro
    130                 135                 140

Ile Lys Asp Leu Gln Gly Ala Val Leu Ala Thr Ala Phe Pro Phe Lys
145                 150                 155                 160

Gln Lys Gln His Ser Glu Ser Phe Met Lys Ile Leu Ser Ala Met Phe
                165                 170                 175

Val Glu Cys Ala Asp Phe Arg Arg Thr Gly Ser Ala Ala Leu Asp Leu
            180                 185                 190

Cys Tyr Leu Ala Ala Asn Arg Val Asp Gly Tyr Phe Glu Leu Gly Leu
        195                 200                 205

Lys Pro Trp Asp Met Ala Ala Gly Glu Leu Ile Ala Arg Glu Ala Gly
    210                 215                 220

Ala Ile Val Thr Asp Phe Ala Gly Gly Thr Asp Tyr Met Gln Ser Gly
225                 230                 235                 240

Asn Ile Val Ala Ser Ser Pro Arg Gly Val Lys Ala Ile Leu Gln His
                245                 250                 255

Ile Arg Glu Asn Gly Asn Ser Ala Ile Leu Lys
            260                 265


<210> 7
<211> 78
<212> PRT
<213> Vibrio cholerae

<220>

<221> PEPTIDE
<222> (1)..(76)
<223> >sp|Q9KVC8|RL28_VIBCH 50S ribosomal protein L28 -
      Vibrio cholerae. (76 aa)

<400> 7
Met Ser Arg Val Cys Gln Val Thr Gly Lys Arg Pro Ala Val Gly Asn
1               5                   10                  15

Asn Arg Ser His Ala Lys Asn Ala Thr Lys Arg Arg Phe Leu Pro Asn
            20                  25                  30

Leu Gln Thr His Arg Phe Trp Val Glu Ser Glu Lys Arg Phe Val Lys
        35                  40                  45

Leu Arg Leu Thr Ala Lys Gly Met Arg Ile Ile Asp Lys Lys Gly Ile
    50                  55                  60

Asp Ala Val Leu Val Glu Ile Arg Ala Arg Gly Glu Asn Val
65                  70                  75


<210> 8
<211> 108
<212> PRT
<213> Vibrio cholerae

<220>
<221> PEPTIDE
<222> (1)..(108)
<223> >sp|P52695|HLYU_VIBCH Transcriptional activator
      hlyU - Vibrio cholerae. (108 aa)

<400> 8
Met Pro Tyr Leu Lys Gly Ala Pro Met Asn Leu Gln Glu Met Glu Lys
1               5                   10                  15

Asn Ser Ala Lys Ala Val Val Leu Leu Lys Ala Met Ala Asn Glu Arg
            20                  25                  30

Arg Leu Gln Ile Leu Cys Met Leu Leu Asp Asn Glu Leu Ser Val Gly
        35                  40                  45

Glu Leu Ser Ser Arg Leu Glu Leu Ser Gln Ser Ala Leu Ser Gln His
    50                  55                  60

Leu Ala Trp Leu Arg Arg Asp Gly Leu Val Asn Thr Arg Lys Glu Ala
65                  70                  75                  80

Gln Thr Val Phe Tyr Thr Leu Ser Ser Thr Glu Val Lys Ala Met Ile
                85                  90                  95

Glu Leu Leu His Arg Leu Tyr Cys Gln Ala Asn Gln
            100                 105


<210> 9
<211> 394
<212> PRT
<213> Vibrio cholerae

<220>
<221> PEPTIDE
<222> (1)..(394)
<223> >sp|Q9KUZ6|EFTU2_VIBCH Elongation factor Tu-B
      (EF-Tu-B) - Vibrio cholerae. (394 aa)

<400> 9
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
        35                  40                  45

Ser Ile Asp Asn Ala Pro Glu Glu Arg Glu Arg Gly Ile Thr Ile Asn
    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Pro
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp
        195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala
        355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390

<210> 10
<211> 953
<212> PRT
<213> Vibrio cholerae

<220>
<221> PEPTIDE
<222> (1)..(953)
<223> >sp|Q9KP73|SYV_VIBCH Valyl-tRNA synthetase (EC
      6.1.1.9) (Valine--tRNA ligase) (ValRS) - Vibrio
      cholerae. (953 aa)

<400> 10
Met Glu Lys Thr Tyr Asn Pro Thr Ser Ile Glu Gln Asp Leu Tyr Lys
  1               5                  10                  15

Thr Trp Glu Glu Gln Gly Tyr Phe Lys Pro His Gly Asp Thr Ser Lys
             20                  25                  30

Asp Ala Tyr Ser Ile Met Ile Pro Pro Pro Asn Val Thr Gly Ser Leu
         35                  40                  45

His Met Gly His Ala Phe Gln Asp Thr Ile Met Asp Thr Leu Ile Arg
     50                  55                  60

Cys Gln Arg Met Lys Gly Lys Asn Thr Leu Trp Gln Val Gly Thr Asp
 65                  70                  75                  80

His Ala Gly Ile Ala Thr Gln Met Val Val Glu Arg Lys Ile Ala Ala
                 85                  90                  95

Glu Glu Gly Lys Thr Lys His Asp Tyr Gly Arg Asp Ala Phe Ile Asp
            100                 105                 110

Lys Ile Trp Glu Trp Lys Ala Glu Ser Gly Gly Thr Ile Thr Lys Gln
        115                 120                 125

Leu Arg Arg Leu Gly Ala Ser Val Asp Trp Asp Arg Glu Arg Phe Thr

130                 135                 140

Met Asp Asp Gly Phe Tyr Lys Ala Val Gln Glu Val Phe Val Arg Leu
145                 150                 155                 160

Tyr Lys Asp Asp Leu Ile Tyr Arg Gly Lys Arg Leu Val Asn Trp Asp
                165                 170                 175

Pro Lys Leu His Thr Ala Ile Ser Asp Leu Glu Val Glu Asn Lys Glu
            180                 185                 190

Thr Lys Gly His Met Trp His Phe Arg Tyr Pro Leu Ala Asp Gly Val
        195                 200                 205

Lys Thr Ala Asp Gly Lys Asp Tyr Ile Val Val Ala Thr Thr Arg Pro
    210                 215                 220

Glu Thr Met Leu Gly Asp Thr Gly Val Ala Val Asn Pro Glu Asp Pro
225                 230                 235                 240

Arg Tyr Lys Asp Leu Ile Gly Lys Glu Ile Ile Leu Pro Ile Val Gly
                245                 250                 255

Arg Arg Ile Pro Ile Val Gly Asp Glu His Ala Asp Met Glu Lys Gly
            260                 265                 270

Thr Gly Cys Val Lys Ile Thr Pro Ala His Asp Phe Asn Asp Tyr Glu
        275                 280                 285

Val Gly Lys Arg His Gln Leu Pro Met Ile Asn Ile Leu Thr Phe Asp
    290                 295                 300

Ala Asn Ile Arg Asp Ala Ala Glu Val Phe Asn Ser Asn Gly Glu Ala
305                 310                 315                 320

Ser Asn Ala Tyr Gly Thr Glu Ile Pro Ala Lys Tyr Gln Gly Met Glu
                325                 330                 335

Arg Phe Ala Ala Arg Lys Ala Ile Val Ala Glu Phe Glu Glu Leu Gly
            340                 345                 350

Leu Leu Gln Glu Ile Lys Asp His Asp Leu Thr Val Pro Tyr Gly Asp
        355                 360                 365

Arg Gly Gly Val Val Ile Glu Pro Met Leu Thr Asp Gln Trp Tyr Val
    370                 375                 380

Arg Ala Gly Ile Leu Ala Lys Pro Ala Val Glu Ala Val Glu Asn Gly
385                 390                 395                 400

Asp Ile Gln Phe Val Pro Lys Gln Tyr Glu Asn Met Tyr Phe Ser Trp
                405                 410                 415

Met Arg Asp Ile Gln Asp Trp Cys Ile Ser Arg Gln Leu Trp Trp Gly
            420                 425                 430

His Arg Ile Pro Ala Trp Tyr Asp Glu Gln Gly Asn Val Phe Val Gly
        435                 440                 445

Arg Asn Glu Glu Glu Val Arg Ala Glu Asn Asn Ile Ala Ala Asp Val
    450                 455                 460

Ala Leu Arg Gln Asp Asp Asp Val Leu Asp Thr Trp Phe Ser Ser Ala
465                 470                 475                 480

Leu Trp Thr Phe Gly Thr Leu Gly Trp Pro Glu Lys Thr Pro Glu Leu
                485                 490                 495

Lys Val Phe His Pro Thr Asp Val Leu Val Thr Gly Phe Asp Ile Ile
            500                 505                 510

Phe Phe Trp Val Ala Arg Met Ile Met Met Thr Met His Phe Cys Lys
        515                 520                 525

Asp Glu Asp Gly Lys Ala Gln Val Pro Phe Lys Thr Val Tyr Val Thr
    530                 535                 540

Gly Leu Ile Arg Asp Glu Asn Gly Asp Lys Met Ser Lys Ser Lys Gly
545                 550                 555                 560

Asn Val Leu Asp Pro Ile Asp Met Ile Asp Gly Ile Asp Leu Glu Ser
                565                 570                 575

Leu Val Ala Lys Arg Thr Gly Asn Met Met Gln Pro Gln Leu Ala Ala
            580                 585                 590

Lys Ile Glu Lys Asn Thr Arg Lys Thr Phe Glu Asn Gly Ile Glu Ala
        595                 600                 605

Tyr Gly Thr Asp Ser Leu Arg Phe Thr Leu Ala Ala Met Ala Ser Thr
    610                 615                 620

Gly Arg Asp Ile Asn Trp Asp Met Lys Arg Leu Glu Gly Tyr Arg Asn
625                 630                 635                 640

Phe Cys Asn Lys Leu Trp Asn Ala Ser Arg Tyr Val Leu Met Asn Thr
                645                 650                 655

Glu Glu Gln Asp Cys Gly Phe Ala Ala Gly Ala Glu Leu Glu Tyr Ser
            660                 665                 670

Leu Ala Asp Lys Trp Ile Glu Ser Gln Phe Glu Leu Ala Ala Lys Glu
        675                 680                 685

Phe Asn Gly His Ile Asp Asn Phe Arg Leu Asp Met Ala Ala Asn Thr
    690                 695                 700

Leu Tyr Glu Phe Ile Trp Asn Gln Phe Cys Asp Trp Tyr Leu Glu Leu
705                 710                 715                 720

Thr Lys Pro Val Leu Trp Lys Gly Thr Glu Ala Gln Gln Arg Ala Thr
                725                 730                 735

Arg Arg Thr Leu Ile Thr Val Leu Glu Lys Thr Leu Arg Leu Ala His
            740                 745                 750

Pro Val Ile Pro Tyr Ile Thr Glu Thr Ile Trp Gln Ser Val Lys Pro
        755                 760                 765

Leu Val Asp Gly Val Glu Gly Asp Thr Ile Met Leu Gln Ala Leu Pro
    770                 775                 780

Gln Tyr Asp Val Ala Asn Phe Asn Gln Glu Ala Leu Asp Asp Ile Glu
785                 790                 795                 800

Trp Val Lys Ala Phe Ile Thr Ser Ile Arg Asn Leu Arg Ala Glu Tyr
                805                 810                 815

Asp Ile Asn Pro Gly Lys Pro Leu Glu Val Met Leu Lys Ala Ala Asn
            820                 825                 830

Glu Gln Asp Ala Ala Arg Ile Glu Ala Asn Lys Pro Val Leu Val Ser
        835                 840                 845

Leu Ala Lys Leu Glu Ser Ile Arg Val Leu Ala Asp Gly Glu Ala Thr
    850                 855                 860

Pro Ala Cys Ala Thr Ala Leu Val Gly Lys Ser Glu Leu Met Ile Pro
865                 870                 875                 880

Met Ala Gly Leu Ile Asp Lys Asp Ala Glu Leu Asp Arg Leu Ala Lys
                885                 890                 895

Glu Ile Ala Lys Thr Gln Gly Glu Ile Ala Arg Ile Glu Gly Lys Leu
            900                 905                 910

Gly Asn Glu Gly Phe Val Ala Lys Ala Pro Glu Ala Val Ile Thr Lys
        915                 920                 925

Glu Arg Glu Lys Leu Ala Gly Tyr Gln Glu Ala Leu Val Lys Leu Glu
    930                 935                 940

Gln Gln Lys Ala Thr Ile Ala Ala Leu
945                 950


<210> 11
<211> 886
<212> PRT
<213> Vibrio cholerae

<220>
<221> PEPTIDE
<222> (1)..(886)
<223> >tr|Q9KPF4|Q9KPF4_VIBCH Pyruvate dehydrogenase, E1
      component - Vibrio cholerae. (886 aa)

<400> 11
Met Ser Asp Met Lys His Asp Val Asp Ala Leu Glu Thr Gln Glu Trp
1               5                   10                  15

Leu Ala Ala Leu Glu Ser Val Val Arg Glu Glu Gly Val Glu Arg Ala
            20                  25                  30

Gln Tyr Leu Leu Glu Gln Val Leu Glu Lys Ala Arg Leu Asp Gly Val
        35                  40                  45

Asp Met Pro Thr Gly Val Thr Thr Asn Tyr Ile Asn Thr Ile Pro Ala
    50                  55                  60

Ala Gln Glu Pro Ala Tyr Pro Gly Asp Thr Thr Ile Glu Arg Arg Ile
65                  70                  75                  80

Arg Ser Ile Ile Arg Trp Asn Ala Ile Met Ile Val Leu Arg Ala Ser
                85                  90                  95

Lys Lys Asp Leu Glu Leu Gly Gly His Met Ala Ser Phe Gln Ser Ser
            100                 105                 110

Ala Ala Phe Tyr Glu Thr Cys Phe Asn His Phe Phe Arg Ala Pro Asn
        115                 120                 125

Glu Lys Asp Gly Gly Asp Leu Val Tyr Tyr Gln Gly His Ile Ser Pro
    130                 135                 140

Gly Ile Tyr Ala Arg Ala Phe Val Glu Gly Arg Leu Thr Glu Glu Gln
145                 150                 155                 160

Leu Asp Asn Phe Arg Gln Glu Val Asp Gly Lys Gly Leu Pro Ser Tyr
                165                 170                 175

Pro His Pro Lys Leu Met Pro Glu Phe Trp Gln Phe Pro Thr Val Ser
            180                 185                 190

Met Gly Leu Gly Pro Ile Ser Ala Ile Tyr Gln Ala Arg Phe Leu Lys
        195                 200                 205

Tyr Leu Asn Gly Arg Gly Leu Lys Asp Thr Thr Ala Gln Arg Val Tyr
    210                 215                 220

Ala Phe Leu Gly Asp Gly Glu Met Asp Glu Pro Glu Ser Arg Gly Ala
225                 230                 235                 240

Ile Ser Phe Ala Ala Arg Glu Lys Leu Asp Asn Leu Cys Phe Leu Ile
                245                 250                 255

Asn Cys Asn Leu Gln Arg Leu Asp Gly Pro Val Met Gly Asn Gly Lys
            260                 265                 270

Ile Ile Gln Glu Leu Glu Gly Leu Phe Arg Gly Ala Gly Trp Asn Val
        275                 280                 285

Val Lys Val Ile Trp Gly Asn Gly Trp Asp Lys Leu Leu Ala Lys Asp
    290                 295                 300

Thr Thr Gly Lys Leu Leu Gln Leu Met Asn Glu Thr Ile Asp Gly Asp
305                 310                 315                 320

Tyr Gln Thr Phe Lys Ala Lys Asp Gly Ala Tyr Val Arg Glu His Phe
                325                 330                 335

Phe Gly Lys Tyr Pro Glu Thr Ala Ala Leu Val Ala Asp Met Thr Asp
            340                 345                 350

Asp Glu Ile Phe Ala Leu Lys Arg Gly Gly His Glu Ser Ser Lys Leu
        355                 360                 365

Tyr Ala Ala Phe Lys Asn Ala Gln Asp Thr Lys Gly Arg Pro Thr Val
    370                 375                 380

Ile Leu Ala Lys Thr Val Lys Gly Tyr Gly Met Gly Asp Ala Ala Glu
385                 390                 395                 400

Gly Lys Asn Ile Ala His Gln Val Lys Lys Met Asp Met Thr His Val

405                 410                 415

Leu Ala Met Arg Asn Arg Leu Gly Leu Gln Asp Leu Ile Ser Asp Glu
            420                 425                 430

Glu Val Lys Asn Leu Pro Tyr Leu Lys Leu Glu Glu Gly Ser Lys Glu
        435                 440                 445

Phe Glu Tyr Leu His Ala Arg Arg Lys Ala Leu His Gly Tyr Thr Pro
    450                 455                 460

Gln Arg Leu Pro Asn Phe Thr Gly Glu Phe Ile Val Pro Ala Leu Glu
465                 470                 475                 480

Asp Phe Lys Pro Leu Leu Glu Glu Gln Ser Arg Glu Ile Ser Ser Thr
                485                 490                 495

Met Ala Tyr Val Arg Ser Leu Asn Ile Leu Leu Lys Asp Lys Asn Ile
            500                 505                 510

Gly Gln Asn Ile Val Pro Ile Ile Ala Asp Glu Ala Arg Thr Phe Gly
        515                 520                 525

Met Glu Gly Leu Phe Arg Gln Ile Gly Ile Tyr Asn Pro His Gly Gln
    530                 535                 540

Asn Tyr Thr Pro Gln Asp Arg Asp Ile Val Ser Tyr Tyr Lys Glu Ala
545                 550                 555                 560

Thr Ser Gly Gln Val Leu Gln Glu Gly Ile Asn Glu Leu Gly Ala Met
                565                 570                 575

Ser Ser Trp Val Ala Ala Ala Thr Ser Tyr Ser Thr Asn Asn Leu Pro
            580                 585                 590

Met Ile Pro Phe Tyr Ile Tyr Tyr Ser Met Phe Gly Phe Gln Arg Val
        595                 600                 605

Gly Asp Met Ala Trp Met Ala Gly Asp Gln Gln Ala Arg Gly Phe Leu
    610                 615                 620

Leu Gly Ala Thr Ala Gly Arg Thr Thr Leu Asn Gly Glu Gly Leu Gln
625                 630                 635                 640

His Glu Asp Gly His Ser His Ile Leu Ala Gly Thr Val Pro Asn Cys
                645                 650                 655

Ile Ser Tyr Asp Pro Thr Phe Ala Tyr Glu Val Ala Val Ile Leu Gln
            660                 665                 670

Asp Gly Ile Arg Arg Met Tyr Gly Glu Gln Glu Asn Val Phe Tyr Tyr
        675                 680                 685

Leu Thr Leu Met Asn Glu Ser Tyr Ala His Pro Ala Met Pro Ala Gly
    690                 695                 700

Ala Glu Glu Gly Ile Arg Lys Gly Ile Tyr Lys Leu Glu Thr His Ala
705                 710                 715                 720

Gly Asn Lys Ala Lys Val Gln Leu Met Ser Ser Gly Thr Ile Met Asn
                725                 730                 735

Glu Val Arg Lys Ala Ala Gln Ile Leu Ser Glu Glu Tyr Gly Val Ala
            740                 745                 750

Ser Asp Val Tyr Ser Val Thr Ser Phe Asn Glu Leu Ala Arg Asp Gly
        755                 760                 765

Gln Ala Cys Asp Arg Phe Asn Met Leu His Pro Glu Ala Glu Val Lys
    770                 775                 780

Val Pro Tyr Ile Ala Gln Val Met Gly Thr Glu Pro Ala Ile Ala Ala
785                 790                 795                 800

Thr Asp Tyr Met Lys Asn Tyr Ala Asp Gln Val Arg Ala Phe Ile Pro

                805                 810                 815

Ala Gln Ser Tyr Lys Val Leu Gly Thr Asp Gly Phe Gly Arg Ser Asp
            820                 825                 830

Ser Arg Glu Asn Leu Arg Arg His Phe Glu Val Asn Ala Gly Tyr Val
        835                 840                 845

Val Val Ala Ala Leu Asn Glu Leu Ala Lys Arg Gly Glu Val Glu Lys
    850                 855                 860

Ser Val Val Ala Ala Ala Ile Lys Lys Phe Asp Ile Asp Thr Glu Lys
865                 870                 875                 880

Thr Asn Pro Leu Tyr Ala
                885


<210> 12
<211> 635
<212> PRT
<213> Vibrio cholerae

<220>
<221> PEPTIDE
<222> (1)..(635)
<223> >tr|Q9KPF5|Q9KPF5_VIBCH Pyruvate dehydrogenase, E2
      component, dihydrolipoamide acetyltransferase -
      Vibrio cholerae. (635 aa)

<400> 12
   Met Lys Val Gly Lys Val Met Ala Ile Glu Ile Tyr Val Pro Asp Ile
   1               5                   10                  15
   Gly Ala Asp Glu Val Glu Val Thr Glu Ile Leu Val Lys Val Gly Asp
               20                  25                  30
   Lys Val Ala Glu Glu Gln Ser Leu Ile Thr Val Glu Gly Asp Lys Ala
           35                  40                  45
   Ser Met Glu Val Pro Ala Ser Gln Ala Gly Ile Val Lys Glu Ile Lys
       50                  55                  60
   Val Val Ala Gly Asp Lys Val Ser Thr Gly Ser Leu Ile Met Val Phe
   65                  70                  75                  80
   Glu Ala Glu Gly Ala Ala Ala Ala Ala Pro Ala Pro Ala Pro Gln Ala
                   85                  90                  95
   Ala Ala Pro Val Ala Ala Ala Pro Ala Ala Ala Ala Leu Lys Glu Val
               100                 105                 110
   Gln Val Pro Asp Ile Gly Gly Asp Glu Val Glu Val Thr Glu Ile Met
           115                 120                 125
   Val Lys Val Gly Asp Val Val Ala Glu Glu Gln Ser Leu Ile Thr Val
       130                 135                 140
   Glu Gly Asp Lys Ala Ser Met Glu Val Pro Ala Pro Phe Ala Gly Thr
   145                 150                 155                 160
   Val Lys Glu Ile Lys Ile Ala Ala Gly Asp Lys Val Ser Thr Gly Ser
                   165                 170                 175
   Leu Ile Met Val Phe Glu Val Ala Gly Ala Ala Pro Leu Ala Ala Pro
               180                 185                 190
   Val Gln Ala Ala Ala Pro Ala Ala Ala Ala Ala Pro Ala Val Ala Ala
           195                 200                 205
   Leu Lys Glu Val Gln Val Pro Asp Ile Gly Gly Asp Glu Val Thr Val
       210                 215                 220
   Thr Glu Ile Met Val Asn Val Gly Asp Ser Ile Ser Glu Glu Gln Ser
   225                 230                 235                 240
   Leu Ile Thr Val Glu Gly Asp Lys Ala Ser Met Glu Val Pro Ala Pro
                   245                 250                 255
   Phe Ala Gly Thr Leu Lys Glu Ile Lys Val Ala Thr Gly Asp Lys Val
               260                 265                 270
   Lys Thr Gly Ser Leu Ile Met Val Phe Glu Val Ala Gly Ala Ala Pro
           275                 280                 285
   Val Ala Ala Pro Val Gln Ala Ala Ala Ala Pro Ala Ala Ala Pro Ala
       290                 295                 300
   Gln Ala Ala Thr Pro Ala Ala Ala Ala Pro Ala Thr Ser Gly Glu Phe
   305                 310                 315                 320
   Gln Glu Asn His Glu Tyr Ser His Ala Ser Pro Val Val Arg Arg Leu
                   325                 330                 335
   Ala Arg Glu Phe Gly Val Asn Leu Ala Lys Val Lys Gly Ser Gly Arg
               340                 345                 350
   Lys Asn Arg Ile Leu Lys Glu Asp Val Gln Asn Tyr Val Lys Glu Ala
           355                 360                 365
   Leu Lys Arg Leu Glu Ser Gly Ala Gln Ala Ala Ala Ser Gly Lys Gly
       370                 375                 380
   Asp Gly Ala Ala Leu Gly Leu Leu Pro Trp Pro Lys Val Asp Phe Ser
   385                 390                 395                 400
   Lys Phe Gly Asp Thr Glu Val Gln Pro Leu Ser Arg Ile Lys Lys Ile
   405 410 415
   Ser Gly Ala Asn Leu His Arg Asn Trp Val Met Ile Pro His Val Thr
   420 425 430
   Gln Trp Asp Asn Ala Asp Ile Thr Glu Leu Glu Lys Phe Arg Gln Glu
   435 440 445
   Gln Asn Ala Met Glu Ala Lys Arg Asp Thr Gly Met Lys Ile Thr Pro
   450 455 460
   Leu Val Phe Ile Met Lys Ala Ala Ala Lys Ala Leu Glu Ala Phe Pro
   465 470 475 480
   Ala Phe Asn Ser Ser Leu Ser Asp Asp Gly Glu Ser Leu Ile Leu Lys
   485 490 495
   Lys Tyr Val Asn Ile Gly Ile Ala Val Asp Thr Pro Asn Gly Leu Val
   500 505 510
   Val Pro Val Phe Lys Asp Val Asn Lys Lys Gly Ile Tyr Glu Leu Ser
   515 520 525
   Lys Glu Leu Ala Glu Val Ser Lys Lys Ala Arg Gly Gly Lys Leu Thr
   530 535 540
   Ala Ala Asp Met Gln Gly Gly Cys Phe Thr Ile Ser Ser Leu Gly Gly
   545 550 555 560
   Ile Gly Gly Thr Ala Phe Thr Pro Ile Val Asn Ala Pro Glu Val Ala
   565 570 575
   Ile Leu Gly Val Ser Lys Ser Glu Met Lys Pro Val Trp Asn Gly Lys
   580 585 590
   Glu Phe Ala Pro Arg Leu Gln Leu Pro Leu Ser Leu Ser Tyr Asp His
   595 600 605
   Arg Val Ile Asp Gly Ala Glu Gly Ala Arg Phe Ile Thr Tyr Leu Asn
   610 615 620
   Glu Cys Leu Ser Asp Ile Arg Arg Leu Val Leu
   625 630 635

<210> 13
<211> 172
<212> PRT
<213> Vibrio cholerae

<220>
<221> PEPTIDE
<222> (1)..(172)
<223> >tr|Q9KR12|Q9KR12_VIBCH Peptidoglycan-associated
lipoprotein - Vibrio cholerae. (172 aa)

<400> 13
Met Gln Leu Asn Lys Val Leu Lys Ser Leu Leu Ile Ala Leu Pro Val
1               5                   10                  15

Leu Ala Val Thr Ala Cys Ser Ser Ser Asp Asp Ala Ala Asn Ser Gly

20                  25                  30

Ser Gln Thr Asn Gln Ser Ala Val Ser Thr Val Asp Ser Asn Gly Leu
        35                  40                  45

Asn Ala Gln Gly Gln Leu Thr Glu Gln Glu Leu Lys Glu Gln Ala Leu
    50                  55                  60

Arg Glu Asn Gln Thr Ile Tyr Phe Ala Phe Asp Asn Ala Thr Ile Ala
65                  70                  75                  80

Ser Asp Tyr Glu Ala Met Leu Ala Ala His Ala Ala Tyr Leu Val Lys
                85                  90                  95

Asn Pro Ser Leu Arg Val Thr Ile Glu Gly His Ala Asp Glu Arg Gly
            100                 105                 110

Thr Pro Glu Tyr Asn Ile Ala Leu Gly Glu Arg Arg Ala Gln Ala Val
        115                 120                 125

Ala Lys Tyr Leu Glu Ala Leu Gly Val Gln Ala Gly Gln Leu Ser Ile
    130                 135                 140

Val Ser Tyr Gly Glu Glu Lys Pro Leu Val Leu Gly Gln Ser Glu Glu
145                 150                 155                 160

Ala Tyr Ala Lys Asn Arg Arg Ala Val Leu Val Tyr
                165                 170

<210> 14
<211> 1297
<212> PRT
<213> Vibrio cholerae

<220>
<221> PEPTIDE
<222> (1)..(1297)
<223> >sp|Q9KTN2|PUR4_VIBCH
      Phosphoribosylformylglycinamidine synthase (EC
      6.3.5.3) (FGAM synthase) (FGAMS)
      (Formylglycinamide ribotide amidotransferase)
      (FGARAT) (Formylglycinamide ribotide synthetase)

<400> 14
Met Arg Ile Leu Arg Gly Ser Pro Ala Leu Ser Glu Phe Arg Val Asn
1               5                   10                  15

Lys Leu Leu Thr Ala Cys Arg Glu Gln Gln Leu Pro Val Thr Gly Ile
            20                  25                  30

Tyr Ala Glu Phe Met His Phe Ala Asp Leu Lys Ala Glu Leu Asn Pro
        35                  40                  45

Gln Glu Leu Glu Lys Leu Glu Lys Leu Leu Thr Tyr Gly Pro Thr Ile
    50                  55                  60

Gln Glu His Glu Pro Gln Gly Leu Leu Leu Leu Val Thr Pro Arg Pro
65                  70                  75                  80

Gly Thr Ile Ser Pro Trp Ser Ser Lys Ala Thr Asp Ile Ala His Asn
                85                  90                  95

Cys Gly Leu His Gly Ile Lys Arg Leu Glu Arg Gly Thr Ala Tyr Tyr
            100                 105                 110

Val Glu Ala Glu Thr Ala Leu Thr Ala Ala Gln Ile Ala Thr Leu Glu
        115                 120                 125

Ala Leu Leu His Asp Arg Met Met Glu Val Val Phe Ala Glu Leu Thr
    130                 135                 140

Asp Ala Gln Gln Leu Phe Ser Val Ala Glu Pro Ala Pro Met Ser Gln
145                 150                 155                 160

Val Asp Val Leu Ala Gly Gly Arg Arg Ala Leu Glu Glu Ala Asn Val
                165                 170                 175

Ser Leu Gly Leu Ala Leu Ala Glu Asp Glu Ile Asp Tyr Leu Val Glu
            180                 185                 190

Ser Phe Thr Lys Leu Gly Arg Asn Pro Asn Asp Ile Glu Leu Met Met
        195                 200                 205

Phe Ala Gln Ala Asn Ser Glu His Cys Arg His Lys Ile Phe Asn Ala
    210                 215                 220

Asp Trp Thr Ile Asp Gly Val Lys Gln Asp Lys Ser Leu Phe Lys Met
225                 230                 235                 240

Ile Lys Asn Thr Phe Glu Gln Thr Pro Asp Tyr Val Leu Ser Ala Tyr
                245                 250                 255

Lys Asp Asn Ala Ala Val Met Thr Gly Ser Thr Val Gly Arg Phe Phe
            260                 265                 270

Pro Asp Pro Glu Ser Arg Gln Tyr Thr Tyr His His Glu Asp Ala His
        275                 280                 285

Ile Leu Met Lys Val Glu Thr His Asn His Pro Thr Ala Ile Ser Pro
    290                 295                 300

Trp Pro Gly Ala Ser Thr Gly Ser Gly Gly Glu Ile Arg Asp Glu Gly
305                 310                 315                 320

Ala Thr Gly Ile Gly Gly Lys Pro Lys Ala Gly Leu Val Gly Phe Thr
                325                 330                 335

Thr Ser Asn Leu Arg Ile Pro Gly Phe Glu Gln Pro Trp Glu Ser Asp
            340                 345                 350

Phe Gly Lys Pro Ser Arg Ile Val Asn Ala Leu Asp Ile Met Leu Glu
        355                 360                 365

Gly Pro Leu Gly Gly Ala Ala Phe Asn Asn Glu Phe Gly Arg Pro Asn
    370                 375                 380

Leu Leu Gly Tyr Phe Arg Thr Tyr Glu Glu Lys Val Thr Ser His Ala
385                 390                 395                 400

Gly Glu Glu Val Arg Gly Tyr His Lys Pro Ile Met Ile Ala Gly Gly
                405                 410                 415

Met Gly Asn Ile Arg Ala Glu His Ile Gln Lys Lys Glu Ile Pro Val
            420                 425                 430

Gly Ala Lys Leu Ile Val Leu Gly Gly Pro Ala Met Asn Ile Gly Leu
        435                 440                 445

Gly Gly Gly Ala Ala Ser Ser Met Ala Ser Gly Gln Ser Ala Glu Asp
    450                 455                 460

Leu Asp Phe Ala Ser Val Gln Arg Glu Asn Pro Glu Met Glu Arg Arg
465                 470                 475                 480

Cys Gln Glu Val Ile Asp Arg Cys Trp Gln Leu Gly Asp Lys Asn Pro
                485                 490                 495

Ile Ala Phe Ile His Asp Val Gly Ala Gly Gly Ile Ser Asn Ala Leu
            500                 505                 510

Pro Glu Leu Val Asn Asp Gly Asp Arg Gly Gly Lys Phe Gln Leu Arg
        515                 520                 525

Asn Val Pro Asn Asp Glu Pro Gly Met Ser Pro Leu Glu Ile Trp Cys
    530                 535                 540

Asn Glu Ser Gln Glu Arg Tyr Val Leu Ala Val Ala Ala Glu Asp Met
545                 550                 555                 560

Pro Leu Phe Asp Ala Ile Cys Gln Arg Glu Arg Ala Pro Tyr Ala Val
                565                 570                 575

Val Gly Glu Ala Thr Glu Glu Arg His Leu Thr Leu Glu Asp Ser His
            580                 585                 590

Phe Ala Asn Thr Pro Ile Asp Met Pro Met Asp Ile Leu Leu Gly Lys
        595                 600                 605

Pro Pro Lys Met His Arg Glu Ala Ser Thr Leu Lys Val Ser Ser Pro
    610                 615                 620

Ala Leu Glu Arg Ser Gly Ile Glu Leu Asn Glu Ala Val Asp Arg Val
625                 630                 635                 640

Leu Arg Leu Pro Ala Val Ala Glu Lys Thr Phe Leu Ile Thr Ile Gly
                645                 650                 655

Asp Arg Ser Val Thr Gly Leu Val Ala Arg Asp Gln Met Val Gly Pro
            660                 665                 670

Trp Gln Val Pro Val Ala Asn Cys Ala Val Thr Ala Ala Ser Phe Asp
        675                 680                 685

Ser Tyr His Gly Glu Ala Met Ser Met Gly Glu Arg Thr Pro Val Ala
    690                 695                 700

Leu Leu Asp Phe Gly Ala Ser Ala Arg Leu Ala Val Gly Glu Ala Ile
705                 710                 715                 720

Thr Asn Ile Ala Ala Thr Asp Ile Gly Glu Leu Lys Arg Ile Lys Leu

725                 730                 735

Ser Ala Asn Trp Met Ser Pro Ala Gly His Pro Gly Glu Asp Ala Gly
            740                 745                 750

Leu Tyr Glu Ala Val Lys Ala Val Gly Glu Glu Leu Cys Pro Ala Leu
        755                 760                 765

Gly Ile Thr Ile Pro Val Gly Lys Asp Ser Met Ser Met Lys Thr Lys
    770                 775                 780

Trp Gln Glu Asn Gly Glu Gln Lys Glu Val Thr Ser Pro Leu Ser Leu
785                 790                 795                 800

Ile Ile Thr Ala Phe Ala Arg Val Glu Asp Ile Arg Lys Thr Val Thr
                805                 810                 815

Pro Gln Leu Arg Thr Asp Leu Gly Glu Thr Ser Leu Ile Leu Ile Asp
            820                 825                 830

Leu Gly Asn Gly Gln Asn Arg Leu Gly Ala Thr Ala Leu Ala Gln Val
        835                 840                 845

Tyr Lys Gln Leu Gly Asp Lys Pro Ala Asp Val Asp Asn Ala Ala Gln
    850                 855                 860

Leu Lys Gly Phe Phe Asp Ala Val Gln Thr Leu Val Arg Asn Asp Lys
865                 870                 875                 880

Leu Val Ala Tyr His Asp Lys Gly Asp Gly Gly Leu Leu Val Thr Leu
                885                 890                 895

Ala Glu Met Ala Phe Ala Gly His Cys Gly Ile Lys Ala Asn Ile Glu
            900                 905                 910

Thr Leu Gly Asp Asp Ala Leu Ala Ala Leu Phe Asn Glu Glu Leu Gly
        915                 920                 925

Ala Val Val Gln Val Lys Asn Asp Glu Leu Asn Ala Val Leu Ala Thr
    930                 935                 940

Leu Ala Ala His Gly Leu Glu Ala Cys Ala His Val Ile Gly Glu Val
945                 950                 955                 960

Glu Ala Ser Asp Arg Leu Leu Ile Thr Cys Gly Glu Glu Val Leu Ile
                965                 970                 975

Glu Arg Ser Arg Thr Glu Leu Arg Thr Ile Trp Ala Glu Met Thr His
            980                 985                 990

Lys Met Gln Ala Leu Arg Asp Asn Ser Ala Cys Ala Asp Gln Glu Phe
        995                 1000                1005

Ala Ala Lys Gln Asp Asn Arg Asp Pro Gly Leu Asn Ala Lys Leu Thr
    1010                1015                1020

Tyr Asp Val Gln Ala Asp Val Ala Ala Pro Tyr Ile Ala Lys Gly Val
1025                1030                1035                1040

Arg Pro Lys Met Ala Ile Leu Arg Glu Gln Gly Val Asn Ser His Val
                1045                1050                1055

Glu Met Ala Ala Ala Phe Asp Arg Ala Gly Phe Asp Ala Val Asp Val
        1060                1065                1070

His Met Ser Asp Ile Leu Thr Gly Gln Thr Val Leu Asp Ala Tyr Gln
    1075                1080                1085

Gly Leu Val Ala Cys Gly Gly Phe Ser Tyr Gly Asp Val Leu Gly Ala
1090                1095                1100

Gly Glu Gly Trp Ala Lys Ser Ile Leu Phe Asn Ala Gln Ala Arg Glu
1105                1110                1115                1120

Gln Phe Glu Gln Phe Phe Gln Arg Lys Asp Thr Phe Ser Leu Gly Val
                1125                1130                1135

Cys Asn Gly Cys Gln Met Leu Ser Asn Leu Arg Asp Leu Ile Pro Gly
        1140                1145                1150

Ala Glu Leu Trp Pro Arg Phe Val Arg Asn Glu Ser Asp Arg Phe Glu
    1155                1160                1165

Ala Arg Phe Ser Leu Val Glu Val Gln Lys Ser Pro Ser Leu Phe Phe
1170                1175                1180

Ser Glu Met Ala Gly Ser Arg Met Pro Ile Ala Val Ser His Gly Glu
1185                1190                1195                1200

Gly Arg Val Glu Val Arg Asp Ala Gln His Leu Ala Ala Ile Glu Gln
                1205                1210                1215

Ser Gly Thr Val Ala Ile Arg Phe Val Asp Asn Phe Gly Gln Pro Thr
        1220                1225                1230

Gln Ala Tyr Pro Ser Asn Pro Asn Gly Ser Pro Asn Ala Ile Thr Gly
    1235                1240                1245

Leu Thr Thr Gln Asp Gly Arg Val Thr Ile Met Met Pro His Pro Glu
1250                1255                1260

Arg Val Phe Arg Thr Val Ala Asn Ser Trp His Pro Asp Asn Trp Gly
1265                1270                1275                1280

Glu Asn Gly Ala Trp Met Arg Met Phe Gln Asn Ala Arg Lys Tyr Phe
                1285                1290                1295

Gly

<210> 15
<211> 110
<212> PRT
<213> Vibrio cholerae

<220>
<221> PEPTIDE
<222> (1)..(110)
<223> >sp|Q9KNY9|RL22_VIBCH 50S ribosomal protein L22 -
      Vibrio cholerae. (110 aa)

<400> 15
Met Glu Ala Ile Ala Lys His Asn Phe Ala Arg Ile Ser Pro Gln Lys
1               5                   10                  15

Ala Arg Leu Val Ala Asp Gln Ile Arg Gly Lys Ser Val Asp Gln Ala
            20                  25                  30

Leu Glu Leu Leu Thr Phe Ser Asn Lys Lys Ala Ala Glu Leu Val Lys
        35                  40                  45

Lys Val Leu Glu Ser Ala Ile Ala Asn Ala Glu His Asn Glu Gly Ala
    50                  55                  60

Asp Ile Asp Asp Leu Arg Val Ala Lys Ile Phe Val Asp Glu Gly Pro
65                  70                  75                  80

Val Met Lys Arg Ile Met Pro Arg Ala Lys Gly Arg Ala Asp Arg Ile
                85                  90                  95

Leu Lys Arg Ser Ser His Ile Thr Val Val Val Ala Asp Arg
            100                 105                 110

<210> 16
<211> 233
<212> PRT
<213> Vibrio cholerae

<220>
<221> PEPTIDE
<222> (1)..(233)
<223> >sp|Q9KV33|RL1_VIBCH 50S ribosomal protein L1 -
      Vibrio cholerae. (233 aa)

<400> 16
Met Ala Lys Leu Thr Lys Arg Met Arg Thr Ile Arg Ala Lys Val Asp
1               5                   10                  15

Val Thr Lys Glu Tyr Asp Ile Asn Glu Ala Val Ala Leu Leu Lys Glu
            20                  25                  30

Leu Ala Thr Ala Lys Phe Val Glu Ser Val Asp Val Ala Val Asn Leu
        35                  40                  45

Gly Ile Asp Ala Arg Lys Ser Asp Gln Asn Val Arg Gly Ala Thr Val
    50                  55                  60

Leu Pro His Gly Thr Gly Arg Asp Ile Arg Val Ala Val Phe Thr Gln
65                  70                  75                  80

Gly Ala Asn Ala Glu Ala Ala Lys Ala Ala Gly Ala Glu Leu Val Gly
                85                  90                  95

Met Glu Asp Leu Ala Asp Leu Val Lys Lys Gly Glu Met Asn Phe Asp
            100                 105                 110

Val Val Ile Ala Ser Pro Asp Ala Met Arg Val Val Gly Gln Leu Gly
        115                 120                 125

Thr Ile Leu Gly Pro Arg Gly Leu Met Pro Asn Pro Lys Val Gly Thr

130                 135                 140

Val Thr Pro Asn Val Ala Glu Ala Val Lys Asn Ala Lys Ala Gly Gln
145                 150                 155                 160

Val Arg Tyr Arg Asn Asp Lys Asn Gly Ile Ile His Thr Thr Ile Gly
                165                 170                 175

Lys Val Thr Phe Glu Ala Asp Gln Leu Lys Glu Asn Leu Glu Ala Leu
            180                 185                 190

Leu Val Ala Leu Lys Lys Ala Lys Pro Ser Ser Ala Lys Gly Val Phe
        195                 200                 205

Val Lys Lys Val Ser Ile Ser Thr Thr Met Gly Ala Gly Val Ala Val
    210                 215                 220

Asp Gln Asn Thr Leu Ser Ala Gln Val
225                 230

<210> 17
<211> 330
<212> PRT
<213> Vibrio cholerae

<220>
<221> PEPTIDE
<222> (1)..(330)
<223> >sp|Q9KP08|RPOA_VIBCH DNA-directed RNA polymerase
      alpha chain (EC 2.7.7.6) (RNAP alpha subunit)
      (Transcriptase alpha chain) (RNA polymerase alpha
      subunit) - Vibrio cholerae. (330 aa)

<400> 17
Met Gln Gly Ser Val Thr Glu Phe Leu Lys Pro Arg Leu Val Asp Ile
1               5                   10                  15

Glu Gln Ile Ser Thr Thr His Ala Lys Val Thr Leu Glu Pro Leu Glu
            20                  25                  30

Arg Gly Phe Gly His Thr Leu Gly Asn Ala Leu Arg Arg Ile Leu Leu
        35                  40                  45

Ser Ser Met Pro Gly Cys Ala Val Thr Glu Val Glu Ile Glu Gly Val
    50                  55                  60

Leu His Glu Tyr Ser Thr Lys Glu Gly Val Gln Glu Asp Ile Leu Glu
65                  70                  75                  80

Ile Leu Leu Asn Leu Lys Gly Leu Ala Val Arg Val Ala Glu Gly Lys
                85                  90                  95

Asp Glu Val Phe Ile Thr Leu Asn Lys Ser Gly Ser Gly Pro Val Val
            100                 105                 110

Ala Gly Asp Ile Thr His Asp Gly Asp Val Glu Ile Val Asn Pro Glu
        115                 120                 125

His Val Ile Cys His Leu Thr Ser Asp Asn Ala Ala Ile Ala Met Arg
    130                 135                 140

Ile Lys Val Glu Arg Gly Arg Gly Tyr Val Pro Ala Ser Ala Arg Ile
145                 150                 155                 160

His Thr Glu Glu Asp Glu Arg Pro Ile Gly Arg Leu Leu Val Asp Ala
                165                 170                 175

Thr Phe Ser Pro Val Asp Lys Ile Ala Tyr Ser Val Glu Ala Ala Arg
            180                 185                 190

Val Glu Gln Arg Thr Asp Leu Asp Lys Leu Val Ile Asp Met Glu Thr
        195                 200                 205

Asn Gly Thr Leu Glu Pro Glu Glu Ala Ile Arg Arg Ala Ala Thr Ile
    210                 215                 220

Leu Ala Glu Gln Leu Asp Ala Phe Val Asp Leu Arg Asp Val Arg Val
225                 230                 235                 240

Pro Glu Glu Lys Glu Glu Lys Pro Glu Phe Asp Pro Ile Leu Leu Arg
                245                 250                 255

Pro Val Asp Asp Leu Glu Leu Thr Val Arg Ser Ala Asn Cys Leu Lys
            260                 265                 270

Ala Glu Ala Ile His Tyr Ile Gly Asp Leu Val Gln Arg Thr Glu Val
        275                 280                 285

Glu Leu Leu Lys Thr Pro Asn Leu Gly Lys Lys Ser Leu Thr Glu Ile
    290                 295                 300

Lys Asp Val Leu Ala Ser Arg Gly Leu Ser Leu Gly Met Arg Leu Glu
305                 310                 315                 320

Asn Trp Pro Pro Ala Ser Ile Ala Glu Asp
                325                 330


<210> 18
<211> 274
<212> PRT
<213> Vibrio cholerae

<220>
<221> PEPTIDE
<222> (1)..(274)
<223> >sp|Q9KNY7|RL2_VIBCH 50S ribosomal protein L2 -
      Vibrio cholerae.(274 aa)


<400> 18
Met Ala Ile Val Lys Cys Lys Pro Thr Ser Ala Gly Arg Arg His Val
1               5                   10                  15

Val Lys Val Val Asn Ala Asp Leu His Lys Gly Lys Pro Tyr Ala Pro
            20                  25                  30

Leu Leu Glu Lys Asn Ser Lys Asn Gly Gly Arg Asn Asn Asn Gly Arg
        35                  40                  45

Ile Thr Val Arg His Ile Gly Gly Gly His Lys Gln His Tyr Arg Leu

50                  55                  60

Val Asp Phe Lys Arg Thr Lys Asp Gly Ile Pro Ala Lys Val Glu Arg
65                  70                  75                  80

Leu Glu Tyr Asp Pro Asn Arg Ser Ala Asn Ile Ala Leu Val Leu Tyr
                85                  90                  95

Ala Asp Gly Glu Arg Arg Tyr Ile Ile Ala Pro Lys Gly Leu Gln Ala
            100                 105                 110

Gly Asp Val Ile Gln Ser Gly Pro Asp Ala Pro Ile Lys Ala Gly Asn
        115                 120                 125

Ala Met Pro Met Arg Asn Ile Pro Val Gly Ser Thr Ile His Asn Val
    130                 135                 140

Glu Leu Thr Pro Gly Lys Gly Ala Gln Leu Ala Arg Ser Ala Gly Ala
145                 150                 155                 160

Tyr Ala Gln Leu Val Ala Arg Asp Gly Ala Tyr Val Thr Leu Arg Leu
                165                 170                 175

Arg Ser Gly Glu Met Arg Lys Val Leu Ser Glu Gly Arg Ala Thr Ile
            180                 185                 190

Gly Glu Val Gly Asn Ala Glu His Met Leu Arg Glu Leu Gly Lys Ala
        195                 200                 205

Gly Ala Ala Arg Trp Arg Gly Val Arg Pro Thr Val Arg Gly Val Val
    210                 215                 220

Met Asn Pro Val Asp His Pro His Gly Gly Gly Glu Gly Arg Thr Ser
225                 230                 235                 240

Gly Gly Arg His Pro Val Ser Pro Trp Gly Val Pro Thr Lys Gly Tyr
                245                 250                 255

Lys Thr Arg Ser Asn Lys Arg Thr Asp Lys Tyr Ile Val Arg Arg Arg
            260                 265                 270

Asn Lys

<210> 19
<211> 504
<212> PRT
<213> Vibrio cholerae

<220>
<221> PEPTIDE
<222> (1)..(504)
<223> >tr|Q9KT99|Q9KT99_VIBCH
      Amidophosphoribosyltransferase - Vibrio
      cholerae.(504 aa)

<400> 19
Met Cys Gly Ile Val Gly Ile Val Gly Thr Thr Pro Val Asn Gln Ser
1               5                   10                  15

Ile Tyr Asp Ala Leu Thr Val Leu Gln His Arg Gly Gln Asp Ala Ala
            20                  25                  30

Gly Ile Cys Thr Ile Glu Ser Asn Arg Phe Arg Leu Arg Lys Ala Asn
        35                  40                  45

Gly Leu Val Arg Asp Val Phe Glu Ala Lys His Met Gln Arg Leu Gln
    50                  55                  60

Gly Asn Val Gly Ile Gly His Val Arg Tyr Pro Thr Ala Gly Ser Ser
65                  70                  75                  80

Ser Ala Ser Glu Ala Gln Pro Phe Tyr Val Asn Ser Pro Phe Gly Ile
                85                  90                  95

Thr Leu Ala His Asn Gly Asn Leu Thr Asn Ala Asn Gln Val Arg Gln
            100                 105                 110

Lys Leu Phe Glu Lys Asp Arg Arg His Val Asn Thr Thr Ser Asp Ser
        115                 120                 125

Glu Val Leu Leu Asn Val Leu Ala His Glu Ile Asp Thr Val Lys Gly
    130                 135                 140

Asn Val Thr Ala Asp Asp Val Phe Arg Ala Ile Ser Asn Val His Arg
145                 150                 155                 160

Thr Ile Arg Gly Ala Tyr Ala Val Ala Ala Met Ile Ile Gly His Gly
                165                 170                 175

Met Ile Ala Phe Arg Asp Pro His Gly Ile Arg Pro Leu Cys Leu Gly
            180                 185                 190

Lys Arg Glu Val Asn Gly Gln Leu Glu Tyr Met Val Ala Ser Glu Ser
        195                 200                 205

Val Ala Leu Asp Ala Val Gly Phe Asp Phe Val Arg Asp Val Ala Pro
    210                 215                 220

Gly Glu Ala Ile Tyr Ala Thr Phe Asp Gly Glu Leu Tyr Thr Lys Gln
225                 230                 235                 240

Cys Ala Asp Asn Pro Ala Leu Asn Pro Cys Ile Phe Glu Phe Val Tyr
                245                 250                 255

Phe Ala Arg Pro Asp Ser Phe Ile Asp Lys Ile Ser Val Tyr Ser Ala
            260                 265                 270

Arg Val Glu Met Gly Lys Arg Leu Gly Glu Arg Ile Lys Asn Asp Tyr
        275                 280                 285

Ser Asp Leu Asp Ile Asp Val Val Ile Pro Ile Pro Glu Thr Ser Cys
    290                 295                 300

Asp Ile Ala Leu Gln Ile Ala Gln Ala Ile Asp Lys Pro Tyr Arg Gln
305                 310                 315                 320

Gly Phe Val Lys Asn Arg Tyr Val Gly Arg Thr Phe Ile Met Pro Gly
                325                 330                 335

Gln Gln Gln Arg Lys Lys Ser Val Arg Arg Lys Leu Asn Ala Ile Arg

340                 345                 350

Ser Glu Phe Lys Asp Lys Asn Val Leu Leu Val Asp Asp Ser Ile Val
        355                 360                 365

Arg Gly Thr Thr Ser Glu Gln Ile Ile Glu Met Ala Arg Asp Ser Gly
    370                 375                 380

Ala Lys Lys Val Tyr Ile Val Ser Ala Ala Pro Glu Ile Arg Phe Pro
385                 390                 395                 400

Asn Val Tyr Gly Ile Asp Met Pro Ser Ala Asn Glu Leu Ile Ala His
                405                 410                 415

Gly Arg Asp Asn Asp Ala Ile Cys Lys Gln Ile Gly Ala Asp Ala Leu
            420                 425                 430

Ile Phe Gln Thr Leu Glu Asp Leu Val Glu Ala Val Arg Cys Gly Asn
        435                 440                 445

Pro Asp Ile Val Lys Phe Glu Ala Ser Val Phe Asn Gly Glu Tyr Val
    450                 455                 460

Thr Gly Asp Ile Asp Gln Gln Tyr Leu Asp Phe Leu Glu Ser Met Arg
465                 470                 475                 480

Ser Asp Asp Ala Lys Val Gln Arg Glu Ile Gln Gln Asp Leu Ala Asn
                485                 490                 495

Leu Glu Leu His Asn Glu Gly Ala
            500

<210> 20
<211> 366
<212> PRT
<213> Vibrio cholerae

<220>
<221> PEPTIDE
<222> (1)..(366)
<223> >tr|Q9KS36|Q9KS36_VIBCH Spermidine/putrescine ABC
      transporter, periplasmic
      spermidine/putrescine-binding protein - Vibrio
      cholerae. (366 aa)

<400> 20
Met Thr Glu Val Ile Leu Phe Phe Phe Ala Thr Pro Lys Arg Ala Thr
1               5                   10                  15

Phe Ser Leu Glu Leu Thr Ser Met Lys Lys Leu Ala Thr Leu Leu Ala
            20                  25                  30

Gly Ser Ala Cys Ala Leu Ser Leu Leu Ser Gly Thr Ala Ala Ala Glu
        35                  40                  45

Asp Lys Glu Leu Val Phe Met Asn Trp Gly Pro Tyr Ile Asn Ser Gly
    50                  55                  60

Ile Leu Glu Gln Phe Thr Lys Glu Thr Gly Ile Lys Val Ile Tyr Ser

65                  70                  75                  80

Thr Tyr Glu Ser Asn Glu Thr Leu Tyr Ala Lys Leu Lys Thr His Asn
                85                  90                  95

Gln Gly Tyr Asp Leu Val Val Pro Ser Thr Tyr Phe Val Ala Lys Met
            100                 105                 110

Arg Asp Glu Gly Met Leu Gln Lys Ile Asp Lys Ser Lys Leu Ser Asn
        115                 120                 125

Phe Lys Asn Leu Asp Ser Asn Tyr Leu Asp Lys Pro Tyr Asp Pro Asn
    130                 135                 140

Asn Asp Tyr Ser Ile Pro His Val Val Ala Ile Thr Gly Leu Ala Val
145                 150                 155                 160

Asn Thr Asp Met Tyr Asp Pro Asn Asp Phe Gln Ser Trp Gly Asp Leu
                165                 170                 175

Trp Lys Pro Glu Leu Lys Gly Gln Leu Met Leu Met Asp Asp Thr Arg
            180                 185                 190

Glu Val Phe His Ile Ala Leu Arg Lys Leu Gly Tyr Ser Gly Asn Ser
        195                 200                 205

Thr Asp Asp Lys Gln Ile Asp Glu Ala Tyr Ala Glu Leu Gln Lys Leu
    210                 215                 220

Met Pro Asn Val Leu Val Phe Asn Ser Asp Asn Pro Ala Ala Pro Tyr
225                 230                 235                 240

Leu Ser Gly Glu Val Gly Leu Gly Met Leu Trp Asn Gly Ser Ala Ala
                245                 250                 255

Ala Ala Gln Lys Glu Gly Leu Pro Ile Lys Leu Val Phe Pro Lys Glu
            260                 265                 270

Gly Gly Ile Gly Trp Val Asp Asn Phe Ala Ile Ser Ser Gly Ala Lys
        275                 280                 285

Asn Val Asp Ala Ala His Lys Met Ile Asp Phe Leu Leu Arg Pro Glu
    290                 295                 300

Ile Ala Glu Gln Ile Ser Arg Asp Thr Gly Tyr Leu Thr Gly Val Lys
305                 310                 315                 320

Ala Ser Asn Asp Lys Phe Gln Asp Val Ala Pro Leu Phe Pro Ser Gln
                325                 330                 335

Glu Asp Leu Asp Arg Val Glu Trp Gln Ser Ala Val Gly Asp Lys Thr
            340                 345                 350

Val Lys Tyr Glu Glu Tyr Phe Met Lys Leu Lys Ala Gly Gln
        355                 360                 365

<210> 21
<211> 635
<212> PRT
<213> Vibrio cholerae

<220>
<221> PEPTIDE
<222> (1)..(635)
<223> >sp|P22359|HTPG_VIBCH Chaperone protein htpG (Heat
      shock protein htpG) (High temperature protein G) -
      Vibrio cholerae. (635 aa)

<400> 21
Met Ser Glu Thr Ala Thr Thr Asn Lys Glu Thr Arg Gly Phe Gln Ser
1               5                   10                  15

Glu Val Lys Gln Leu Leu His Leu Met Ile His Ser Leu Tyr Ser Asn
            20                  25                  30

Lys Glu Ile Phe Leu Arg Glu Leu Ile Ser Asn Ala Ser Asp Ala Val
        35                  40                  45

Asp Lys Leu Arg Phe Gln Ala Leu Ser His Pro Asp Leu Tyr Gln Gly
    50                  55                  60

Asp Ala Glu Leu Gly Val Lys Leu Ser Phe Asp Lys Asp Lys Asn Thr
65                  70                  75                  80

Leu Thr Ile Ser Asp Asn Gly Ile Gly Met Thr Arg Asp Glu Val Ile
                85                  90                  95

Glu Asn Leu Gly Thr Ile Ala Lys Ser Gly Thr Ala Glu Phe Phe Ser
            100                 105                 110

Lys Leu Ser Gln Glu Gln Ser Lys Asn Ser Gln Leu Ile Gly Gln Phe
        115                 120                 125

Gly Val Gly Phe Tyr Ser Ala Phe Ile Val Ala Asp Ala Val Thr Val
    130                 135                 140

Arg Thr Arg Ala Ala Gly Ser Ala Pro Ala Asp Ala Val Gln Trp Tyr
145                 150                 155                 160

Ser Lys Gly Glu Gly Glu Tyr Thr Val Glu Thr Ile Asn Lys Glu Ser
                165                 170                 175

Arg Gly Thr Asp Ile Ile Leu His Leu Arg Glu Glu Gly Lys Glu Phe
            180                 185                 190

Leu Ser Glu Trp Arg Leu Arg Asp Val Ile Ser Lys Tyr Ser Asp His
        195                 200                 205

Ile Gly Ile Pro Val Tyr Ile Gln Thr Ser Val Met Asp Glu Glu Gly
    210                 215                 220

Lys Ala Thr Glu Glu Thr Lys Trp Glu Gln Ile Asn Lys Ala Gln Ala
225                 230                 235                 240

Leu Trp Thr Arg Ala Lys Ser Glu Val Thr Asp Glu Glu Tyr Lys Glu
                245                 250                 255

Phe Tyr Lys His Val Ser His Asp Phe Ala Asp Pro Leu Val Trp Ser
            260                 265                 270

His Asn Lys Val Glu Gly Lys Asn Asp Tyr Thr Ser Leu Leu Tyr Ile

275                 280                 285

Pro Ala Lys Ala Pro Trp Asp Leu Phe Asn Arg Glu His Lys His Gly
    290                 295                 300

Leu Lys Leu Tyr Val Gln Arg Val Phe Ile Met Asp Asp Ala Ala Gln
305                 310                 315                 320

Phe Met Pro Ser Tyr Leu Arg Phe Val Arg Gly Leu Ile Asp Ser Asn
                325                 330                 335

Asp Leu Pro Leu Asn Val Ser Arg Glu Ile Leu Gln Asp Asn Lys Ile
            340                 345                 350

Thr Gln Ser Leu Arg Gln Ala Cys Thr Lys Arg Val Leu Thr Met Leu
        355                 360                 365

Glu Arg Met Ala Ser Asn Asp Ala Asp Asn Tyr Gln Lys Phe Trp Lys
    370                 375                 380

Glu Phe Gly Leu Val Met Lys Glu Gly Pro Ala Glu Asp Phe Ala Asn
385                 390                 395                 400

Arg Glu Lys Ile Ala Ser Leu Leu Arg Phe Ala Ser Thr His Ile Asp
                405                 410                 415

Ser Ala Glu Gln Thr Ile Ser Leu Ala Ser Tyr Val Glu Arg Met Lys
            420                 425                 430

Glu Gly Gln Asp Lys Ile Tyr Tyr Leu Thr Ala Asp Ser Tyr Thr Ala
        435                 440                 445

Ala Lys Asn Ser Pro His Leu Glu Gln Phe Lys Ser Lys Gly Ile Glu
    450                 455                 460

Val Ile Leu Met Phe Asp Arg Ile Asp Glu Trp Leu Met Asn Tyr Leu
465                 470                 475                 480

Pro Glu Phe Glu Gly Lys Ala Phe Gln Ser Ile Thr Lys Ala Gly Leu
                485                 490                 495

Asp Leu Ser Gln Phe Glu Asp Glu Ala Glu Lys Glu Lys His Lys Glu
            500                 505                 510

Thr Glu Glu Gln Phe Lys Ser Val Val Glu Arg Leu Lys Gly Tyr Leu
        515                 520                 525

Gly Ser Arg Val Lys Glu Val Arg Thr Thr Phe Lys Leu Ala Asn Thr
    530                 535                 540

Pro Ala Val Val Val Thr Asp Asp Tyr Glu Met Gly Thr Gln Met Ala
545                 550                 555                 560

Lys Leu Leu Ala Ala Ala Gly Gln Pro Val Pro Glu Val Lys Tyr Ile
                565                 570                 575

Leu Glu Val Asn Pro Glu His Ala Leu Val Lys Arg Met Ala Asp Glu
            580                 585                 590

Ala Asp Glu Gln Thr Phe Gly Arg Trp Ala Glu Val Leu Leu Gly Gln
        595                 600                 605

Ala Met Leu Ala Glu Arg Gly Ser Met Glu Asp Pro Ser Gln Phe Leu
    610                 615                 620

Gly Ala Val Asn Gln Leu Leu Ala Pro Ser His
625                 630                 635

<210> 22
<211> 347
<212> PRT
<213> Vibrio cholerae

<220>
<221> PEPTIDE
<222> (1)..(347)
<223> >tr|Q9ZA92|Q9ZA92_VIBCH Rod shape-determining
      protein MreB - Vibrio cholerae. (347 aa)

<400> 22
Met Phe Lys Lys Leu Arg Gly Met Phe Ser Asn Asp Leu Ser Ile Asp
1               5                   10                  15

Leu Gly Thr Ala Asn Thr Leu Ile Tyr Val Lys Gly Gln Gly Ile Val
            20                  25                  30

Leu Asp Glu Pro Ser Val Val Ala Ile Arg Gln Asp Lys Gly Arg Gly
        35                  40                  45

Gly Lys Thr Val Ala Ala Val Gly His Ala Ala Lys Gln Met Leu Gly
    50                  55                  60

Arg Thr Pro Gly Asn Ile Ser Ala Ile Arg Pro Met Lys Asp Gly Val
65                  70                  75                  80

Ile Ala Asp Phe Tyr Val Thr Glu Lys Met Leu Gln His Phe Ile Arg
                85                  90                  95

Gln Val His Asp Asn Ser Val Leu Lys Pro Ser Pro Arg Val Leu Val
            100                 105                 110

Cys Val Pro Cys Gly Ser Thr Gln Val Glu Arg Arg Ala Ile Arg Glu
        115                 120                 125

Ser Ala Leu Gly Ala Gly Ala Arg Glu Val Tyr Leu Ile Asp Glu Pro
    130                 135                 140

Met Ala Ala Ala Ile Gly Ala Gly Leu Arg Val Ser Glu Pro Thr Gly
145                 150                 155                 160

Ser Met Val Ile Asp Ile Gly Gly Gly Thr Thr Glu Val Ala Val Ile
                165                 170                 175

Ser Leu Asn Gly Val Val Tyr Ser Ser Ser Val Arg Ile Gly Gly Asp
            180                 185                 190

Arg Phe Asp Glu Ala Ile Ile Asn Tyr Val Arg Arg Asn Tyr Gly Ser
        195                 200                 205

Leu Ile Gly Glu Ala Thr Ala Glu Lys Ile Lys His Glu Ile Gly Ser
    210                 215                 220

Ala Tyr Pro Gly Asp Asp Val Gln Glu Ile Glu Val Arg Gly Arg Asn
225                 230                 235                 240

Leu Ala Glu Gly Val Pro Arg Ser Phe Thr Leu Asn Ser Asn Glu Ile
                245                 250                 255

Leu Glu Ala Leu Gln Glu Pro Leu Thr Gly Ile Val Ser Ala Val Met
            260                 265                 270

Val Ala Leu Glu Gln Cys Pro Pro Glu Leu Ala Ser Asp Ile Ser Glu
        275                 280                 285

Asn Gly Met Val Leu Thr Gly Gly Gly Ala Leu Leu Lys Asp Leu Asp
    290                 295                 300

Arg Leu Leu Met Glu Glu Thr Gly Ile Pro Val Val Ile Ala Asp Asp
305                 310                 315                 320

Pro Leu Thr Cys Val Ala Arg Gly Gly Gly Lys Ala Leu Glu Met Ile
                325                 330                 335

Asp Met His Gly Gly Asp Leu Phe Ser Glu Glu
            340                 345

<210> 23
<211> 109
<212> PRT
<213> Vibrio cholerae

<220>
<221> PEPTIDE
<222> (1)..(109)
<223> >sp|Q9KT50|Y1055_VIBCH Hypothetical UPF0133
      protein VC1055 - Vibrio cholerae.

<400> 23
Met Phe Gly Lys Gly Gly Met Gly Asn Leu Met Lys Gln Ala Gln Gln
1               5                   10                  15

Met Gln Glu Arg Met Gln Lys Leu Gln Glu Glu Ile Ala Asn Met Glu
            20                  25                  30

Val Thr Gly Glu Ser Gly Ala Gly Leu Val Lys Val Thr Val Thr Gly
        35                  40                  45

Ser His Ser Val Arg Arg Val Asn Ile Asp Glu Ser Leu Met Glu Asp
    50                  55                  60

Asp Lys Glu Met Leu Glu Asp Leu Ile Ala Ala Ala Phe Asn Asp Ala
65                  70                  75                  80

Ala Arg Arg Ile Glu Glu Thr Gln Lys Glu Lys Met Ala Ser Ile Thr
                85                  90                  95

Gly Gly Met Gln Leu Pro Pro Gly Met Lys Met Pro Phe
            100                 105

<210> 24
<211> 209
<212> PRT
<213> Vibrio cholerae

<220>
<221> PEPTIDE
<222> (1)..(209)
<223> >sp|Q9KNY4|RL3_VIBCH 50S ribosomal protein L3 -
      Vibrio cholerae. (209 aa)

<400> 24
Met Ile Gly Leu Ile Gly Arg Lys Val Gly Met Thr Arg Val Phe Thr
1               5                   10                  15

Glu Asp Gly Val Ser Ile Pro Val Thr Val Val Glu Val Glu Ala Asn
            20                  25                  30

Arg Val Ser Gln Val Lys Thr Leu Glu Thr Asp Gly Tyr Ala Ala Ile
        35                  40                  45

Gln Val Thr Thr Gly Ser Lys Lys Ala Asn Arg Val Thr Lys Pro Glu
    50                  55                  60

Ala Gly His Phe Ala Lys Ala Gly Val Glu Ala Gly Arg Gly Leu Trp
65                  70                  75                  80

Glu Phe Arg Leu Glu Asn Gly Glu Glu Phe Ala Val Gly Ser Glu Leu
                85                  90                  95

Thr Val Glu Leu Phe Asn Glu Val Lys Lys Val Asp Val Thr Gly Thr
            100                 105                 110

Ser Lys Gly Lys Gly Phe Gln Gly Ala Val Lys Arg Trp Asn Phe Arg
        115                 120                 125

Thr Gln Asp Met Thr His Gly Asn Ser Leu Ser His Arg Ala Pro Gly
    130                 135                 140

Ser Ile Gly Gln Cys Gln Thr Pro Gly Arg Val Phe Lys Gly Lys Lys
145                 150                 155                 160

Met Ala Gly His Met Gly Ala Glu Arg Val Thr Thr Gln Asn Leu Glu
                165                 170                 175

Ile Val Arg Val Asp Ala Glu Arg Asn Leu Leu Leu Ile Lys Gly Ala
            180                 185                 190

Val Pro Gly Ala Thr Gly Gly Asn Val Ile Val Lys Pro Ala Val Lys
        195                 200                 205

Ala

<210> 25
<211> 242
<212> PRT
<213> Vibrio cholerae

<220>
<221> PEPTIDE
<222> (1)..(242)
<223> >sp|Q9KPV2|RS2_VIBCH 30S ribosomal protein S2 -
      Vibrio cholerae. (242 aa)

<400> 25
Met Ala Ser Val Ser Met Arg Asp Met Leu Thr Ala Gly Val His Phe
1               5                   10                  15

Gly His Gln Thr Arg Tyr Trp Asn Pro Lys Met Lys Gln Phe Ile Phe
            20                  25                  30

Gly Ala Arg Asn Arg Val His Ile Ile Asn Leu Glu Lys Thr Val Pro
        35                  40                  45

Met Phe Asn Glu Ala Leu Ala Glu Leu Ala Lys Val Gly Glu Lys Lys
    50                  55                  60

Gly Lys Val Leu Phe Val Gly Thr Lys Arg Ala Ala Ser Glu Ser Val
65                  70                  75                  80

Lys Glu Ala Ala Leu Ala Ser Asn Gln Tyr Tyr Val Asn Asn Arg Trp
                85                  90                  95

Leu Gly Gly Met Leu Thr Asn Trp Lys Thr Val Arg Gln Ser Ile Lys
            100                 105                 110

Arg Leu Lys Glu Leu Glu Val Gln Ser Thr Asp Gly Thr Phe Asp Lys
        115                 120                 125

Leu Thr Lys Lys Glu Ala Leu Met Arg Thr Arg Glu Met Glu Lys Leu
    130                 135                 140

Glu Lys Ser Leu Gly Gly Ile Lys Asp Met Gly Gly Leu Pro Asp Ala
145                 150                 155                 160

Leu Phe Val Ile Asp Ala Asp His Glu His Ile Ala Ile Lys Glu Ala
                165                 170                 175

Asn Asn Leu Gly Ile Pro Val Phe Ala Val Val Asp Thr Asn Ser Ser
            180                 185                 190

Pro Asp Gly Val Asp Tyr Ile Ile Pro Gly Asn Asp Asp Ala Ile Arg
        195                 200                 205

Ala Val Gln Leu Tyr Leu Asn Ala Ala Ala Gln Ala Ile Asn Glu Gly
    210                 215                 220

Arg Asn Lys Asp Val Ala Ala Val Ala Glu Lys Asp Gly Phe Val Glu
225                 230                 235                 240

Ala Glu

<210> 26
<211> 556
<212> PRT
<213> Vibrio cholerae

<220>
<221> PEPTIDE
<222> (1)..(556)
<223> >tr|Q9KQT3|Q9KQT3_VIBCH Ribosomal protein S1 -
      Vibrio cholerae. (556 aa)

<400> 26
Met Thr Glu Ser Phe Ala Gln Leu Phe Glu Glu Phe Leu Asn Glu Thr
1               5                   10                  15

Gln Phe Gln Gln Gly Thr Ile Val Lys Gly Thr Val Val Ala Ile Glu
            20                  25                  30

Asn Gly Tyr Val Leu Val Asp Ala Gly Leu Lys Ser Glu Ser Ala Ile
        35                  40                  45

Pro Ala Glu Gln Phe Lys Asn Ala Ala Gly Glu Leu Glu Val Gln Val
    50                  55                  60

Gly Ser Gln Val Asp Val Ala Leu Asp Ala Val Glu Asp Gly Phe Gly
65                  70                  75                  80

Glu Thr Gln Leu Ser Arg Glu Lys Ala Lys Arg His Glu Ala Trp Ile
                85                  90                  95

Val Leu Glu Lys Ala Tyr Glu Glu Ala Ala Thr Val Ile Gly Ile Ile
            100                 105                 110

Asn Gly Lys Val Lys Gly Gly Phe Thr Val Glu Leu Asn Gly Ile Arg
        115                 120                 125

Ala Phe Leu Pro Gly Ser Leu Val Asp Val Arg Pro Ile Arg Asp Thr
    130                 135                 140

Ala His Leu Glu Asn Lys Glu Leu Glu Phe Lys Val Ile Lys Leu Asp
145                 150                 155                 160

Gln Lys Arg Asn Asn Val Val Val Ser Arg Arg Ala Val Ile Glu Ser
                165                 170                 175

Glu Ser Ser Val Glu Arg Asp Glu Leu Leu Glu Thr Leu Gln Glu Gly
            180                 185                 190

Thr Glu Val Lys Gly Ile Val Lys Asn Leu Thr Asp Tyr Gly Ala Phe
        195                 200                 205

Val Asp Leu Gly Gly Val Asp Gly Leu Leu His Ile Thr Asp Met Ala
    210                 215                 220

Trp Lys Arg Val Lys His Pro Ser Glu Ile Val Asn Val Gly Asp Glu
225                 230                 235                 240

Ile Leu Val Lys Val Leu Lys Phe Asp Arg Asp Arg Thr Arg Val Ser
                245                 250                 255

Leu Gly Leu Lys Gln Leu Gly Glu Asp Pro Trp Val Ala Ile Ala Lys
            260                 265                 270

Arg Tyr Pro Glu Gly His Lys Leu Ser Gly Arg Val Thr Asn Leu Thr
        275                 280                 285

Asp Tyr Gly Cys Phe Val Glu Ile Glu Glu Gly Val Glu Gly Leu Val
    290                 295                 300

His Val Ser Glu Met Asp Trp Thr Asn Lys Asn Ile His Pro Ser Lys
305                 310                 315                 320

Val Val Asn Val Gly Asp Glu Val Glu Val Met Val Leu Asp Ile Asp
                325                 330                 335

Glu Glu Arg Arg Arg Ile Ser Leu Gly Leu Lys Gln Cys Lys Ala Asn
            340                 345                 350

Pro Trp Gln Ser Phe Ala Glu Ala Gln Ala Lys Gly Asp Lys Val Thr
        355                 360                 365

Gly Lys Ile Lys Ser Ile Thr Asp Phe Gly Ile Phe Ile Gly Leu Glu
    370                 375                 380

Gly Gly Ile Asp Gly Leu Val His Leu Ser Asp Ile Ser Trp Asn Val
385                 390                 395                 400

Pro Gly Glu Glu Ala Val Arg Glu Phe Lys Lys Gly Asp Glu Ile Ser
                405                 410                 415

Ala Val Val Leu Ala Val Asp Ala Glu Arg Glu Arg Ile Ser Leu Gly
            420                 425                 430

Ile Lys Gln Met Glu Asn Asp Pro Phe Asn Ala Tyr Val Ser Asp Asn
        435                 440                 445

Lys Lys Gly Ala Leu Val Asn Gly Thr Val Thr Ala Val Asp Ala Lys
    450                 455                 460

Gly Ala Thr Ile Glu Leu Glu Asp Gly Val Glu Gly Tyr Ile Arg Ala
465                 470                 475                 480

Ser Glu Val Ser Arg Asp Arg Ile Glu Asp Ala Ser Leu Ile Leu Asn
                485                 490                 495

Val Gly Asp Lys Val Glu Ala Lys Phe Thr Gly Val Asp Arg Lys Asn
            500                 505                 510

Arg Val Ile Asn Leu Ser Ile Lys Ala Lys Asp Glu Ala Glu Glu Gln
        515                 520                 525

Glu Val Met Ala Thr Leu Asn Lys Gln Asp Glu Ala Ala Phe Gly Asn
    530                 535                 540

Ala Met Ala Asp Ala Phe Lys Ala Ala Lys Gly Glu
545                 550                 555


<210> 27
<211> 1341
<212> PRT
<213> Vibrio cholerae

<220>
<221> PEPTIDE
<222> (1)..(1341)
<223> >sp|Q9KV30|RPOB_VIBCH DNA-directed RNA polymerase

beta chain (EC 2.7.7.6) (RNAP beta subunit)
(Transcriptase beta chain) (RNA polymerase beta
subunit) - Vibrio cholerae. (1341 aa)

<400> 27
Met Val Tyr Ser Tyr Thr Glu Lys Lys Arg Ile Arg Lys Asp Phe Gly
1               5                   10                  15

Thr Arg Pro Gln Val Leu Asp Ile Pro Tyr Leu Leu Ser Ile Gln Leu
            20                  25                  30

Asp Ser Phe Glu Lys Phe Ile Glu Gln Asp Pro Glu Gly Gln Tyr Gly
        35                  40                  45

Leu Glu Ala Ala Phe Arg Ser Val Phe Pro Ile Gln Ser Tyr Asn Gly
    50                  55                  60

Asn Ser Glu Leu Gln Tyr Val Ser Tyr Arg Leu Gly Glu Pro Val Phe
65                  70                  75                  80

Asp Val Lys Glu Cys Gln Ile Arg Gly Val Thr Tyr Ser Lys Pro Leu
                85                  90                  95

Arg Val Lys Leu Arg Leu Val Ile Phe Asp Lys Asp Ala Pro Ala Gly
            100                 105                 110

Thr Val Lys Asp Ile Lys Glu Gln Glu Val Tyr Met Gly Glu Ile Pro
        115                 120                 125

Leu Met Thr Glu Asn Gly Thr Phe Val Ile Asn Gly Thr Glu Arg Val
    130                 135                 140

Ile Val Ser Gln Leu His Arg Ser Pro Gly Val Phe Phe Asp Ser Asp
145                 150                 155                 160

Lys Gly Lys Thr His Ser Ser Gly Lys Val Leu Tyr Asn Ala Arg Ile
                165                 170                 175

Ile Pro Tyr Arg Gly Ser Trp Leu Asp Phe Glu Phe Asp Pro Lys Asp
            180                 185                 190

Asn Leu Tyr Val Arg Ile Asp Arg Arg Arg Lys Leu Pro Ala Ser Ile
        195                 200                 205

Ile Leu Arg Ala Leu Gly Lys Thr Ser Ala Glu Ile Leu Asp Ile Phe
    210                 215                 220

Phe Glu Lys Val Asn Phe Glu Val Lys Asp Gln Thr Leu Met Met Glu
225                 230                 235                 240

Leu Val Pro Glu Arg Leu Arg Gly Glu Thr Ala Thr Phe Asp Ile Glu
                245                 250                 255

Ala Asp Gly Lys Val Tyr Val Glu Lys Gly Arg Arg Val Thr Ala Arg
            260                 265                 270

His Ile Arg Gln Leu Glu Lys Asp Gly Val Asn Phe Ile Glu Val Pro
        275                 280                 285

Val Glu Tyr Ile Val Gly Lys Val Ser Ala Lys Asp Tyr Val Asn Glu
    290                 295                 300

Ala Thr Gly Glu Leu Ile Ile Thr Ala Asn Gln Glu Ile Ser Leu Glu
305                 310                 315                 320

Ala Leu Ala Asn Leu Ser Gln Ala Gly Tyr Lys Lys Leu Glu Val Leu
                325                 330                 335

Phe Thr Asn Asp Leu Asp His Gly Pro Phe Met Ser Glu Thr Leu Arg
            340                 345                 350

Val Asp Ser Thr Thr Asp Arg Ile Ser Ala Leu Val Glu Ile Tyr Arg
        355                 360                 365

Met Met Arg Pro Gly Glu Pro Pro Thr Lys Glu Ala Ala Glu Ser Leu
    370                 375                 380

Phe Glu Ser Leu Phe Phe Ser Ala Glu Arg Tyr Asp Leu Ser Thr Val
385                 390                 395                 400

Gly Arg Met Lys Phe Asn Ser Ser Ile Gly Arg Glu Asp Ala Glu Glu
                405                 410                 415

Gln Gly Thr Leu Asp Glu Val Asp Ile Ile Glu Val Met Lys Lys Leu
            420                 425                 430

Ile Ser Ile Arg Asn Gly Lys Gly Glu Val Asp Asp Ile Asp His Leu
        435                 440                 445

Gly Asn Arg Arg Ile Arg Ser Val Gly Glu Met Ala Glu Asn Gln Phe
    450                 455                 460

Arg Val Gly Leu Val Arg Val Glu Arg Ala Val Lys Glu Arg Leu Ser
465                 470                 475                 480

Leu Gly Asp Leu Asp Asn Val Met Pro Gln Asp Leu Ile Asn Ala Lys
                485                 490                 495

Pro Ile Ser Ala Ala Val Lys Glu Phe Phe Gly Ser Ser Gln Leu Ser
            500                 505                 510

Gln Phe Met Asp Gln Asn Asn Pro Leu Ser Glu Val Thr His Lys Arg
        515                 520                 525

Arg Ile Ser Ala Leu Gly Pro Gly Gly Leu Thr Arg Glu Arg Ala Gly
    530                 535                 540

Phe Glu Val Arg Asp Val His Val Thr His Tyr Gly Arg Leu Cys Pro
545                 550                 555                 560

Ile Glu Thr Pro Glu Gly Pro Asn Ile Gly Leu Ile Asn Ser Leu Ser
                565                 570                 575

Ala Phe Ala Arg Cys Asn Glu Tyr Gly Phe Leu Glu Thr Pro Tyr Arg
            580                 585                 590

Arg Val Val Asn Gly Val Val Thr Asp Glu Val Asp Tyr Leu Ser Ala
        595                 600                 605

Ile Glu Glu Gly Gln Phe Val Ile Ala Gln Ala Asn Ala Lys Leu Thr
    610                 615                 620

Glu Glu Gly Gly Phe Ala Asp Glu Leu Val Thr Ala Arg Gln Lys Gly
625                 630                 635                 640

Glu Ser Gly Leu His Pro Arg Glu His Val Asp Tyr Met Asp Val Ala
                645                 650                 655

Thr Asn Gln Val Val Ser Ile Ala Ala Ser Leu Ile Pro Phe Leu Glu
            660                 665                 670

His Asp Asp Ala Asn Arg Ala Leu Met Gly Ala Asn Met Gln Arg Gln
        675                 680                 685

Ala Val Pro Thr Leu Arg Ser Glu Lys Pro Leu Val Gly Thr Gly Ile
    690                 695                 700

Glu Arg Asn Val Ala Val Asp Ser Gly Val Thr Ala Val Ala Lys Arg
705                 710                 715                 720

Gly Gly Val Ile Gln Ser Val Asp Ala Ser Arg Ile Val Val Lys Val
                725                 730                 735

Asn Glu Glu Glu Leu Ile Pro Gly Glu Ala Gly Ile Asp Ile Tyr Asn
            740                 745                 750

Leu Thr Lys Tyr Thr Arg Ser Asn Gln Asn Thr Cys Ile Asn Gln Arg
        755                 760                 765

Pro Cys Val Met Pro Gly Glu Pro Val Ala Arg Gly Asp Val Leu Ala
    770                 775                 780

Asp Gly Pro Ser Thr Asp Leu Gly Glu Leu Ala Leu Gly Gln Asn Met
785                 790                 795                 800

Arg Ile Ala Phe Met Pro Trp Asn Gly Tyr Asn Phe Glu Asp Ser Ile
                805                 810                 815

Leu Val Ser Glu Arg Val Val Gln Asp Asp Arg Phe Thr Thr Ile His
            820                 825                 830

Ile Gln Glu Leu Ser Cys Val Ala Arg Asp Thr Lys Leu Gly Ala Glu

        835                 840                 845

Glu Ile Thr Ala Asp Ile Pro Asn Val Gly Glu Ala Ala Leu Ser Lys
    850                 855                 860

Leu Asp Glu Ser Gly Ile Val Tyr Ile Gly Ala Glu Val Lys Gly Gly
865                 870                 875                 880

Asp Ile Leu Val Gly Lys Val Thr Pro Lys Gly Glu Thr Gln Leu Thr
                885                 890                 895

Pro Glu Glu Lys Leu Leu Arg Ala Ile Phe Gly Glu Lys Ala Ser Asp
            900                 905                 910

Val Lys Asp Thr Ser Leu Arg Val Pro Asn Ser Val Ala Gly Thr Val
        915                 920                 925

Ile Asp Val Gln Val Phe Thr Arg Asp Gly Val Glu Lys Asp Lys Arg
    930                 935                 940

Ala Leu Glu Ile Glu Gln Met Gln Leu Lys Glu Ala Lys Lys Asp Leu
945                 950                 955                 960

Thr Glu Glu Phe Gln Ile Leu Glu Gly Gly Leu Leu Ala Arg Val Arg
                965                 970                 975

Ser Val Leu Leu Ala Gly Gly Tyr Thr Glu Ala Lys Leu Gly Ser Ile
            980                 985                 990

Glu Arg Lys Lys Trp Leu Glu Gln Thr Leu Glu Asn Glu Glu Leu Gln
        995                 1000                1005

Asn Gln Leu Glu Gln Leu Ala Glu Gln Tyr Asp Glu Leu Lys Ala Asp
    1010                1015                1020

Phe Asp Lys Lys Phe Glu Ala Lys Arg Arg Lys Ile Thr Gln Gly Asp
1025                1030                1035                1040

Asp Leu Ala Pro Gly Val Leu Lys Ile Val Lys Val Tyr Leu Ala Val
                1045                1050                1055

Lys Arg Arg Ile Gln Pro Gly Asp Lys Met Ala Gly Arg His Gly Asn
            1060                1065                1070

Lys Gly Val Ile Ser Lys Ile Asn Pro Val Glu Asp Met Pro Tyr Asp
        1075                1080                1085

Glu Asn Gly Gln Pro Val Asp Ile Val Leu Asn Pro Leu Gly Val Pro
    1090                1095                1100

Ser Arg Met Asn Ile Gly Gln Ile Leu Glu Val His Leu Gly Leu Ala
1105                1110                1115                1120

Ala Lys Gly Ile Gly Asp Lys Ile Asn Gln Met Ile Lys Glu Gln Gln
                1125                1130                1135

Glu Leu Ala Lys Leu Arg Glu Phe Leu Gln Lys Val Tyr Asp Leu Gly
            1140                1145                1150

Asp Thr Arg Gln Arg Val Asp Ile Ser Glu Leu Ser Asp Glu Asp Val
        1155                1160                1165

Arg Thr Leu Ala His Asn Leu Arg Ala Gly Leu Pro Val Ala Thr Pro
    1170                1175                1180

Val Phe Asp Gly Ala Pro Glu Ser Ser Ile Lys Ala Met Leu Glu Leu
1185                1190                1195                1200

Ala Asp Leu Pro Ala Ser Gly Gln Leu Thr Leu Phe Asp Gly Arg Thr
                1205                1210                1215

Gly Asp Ala Phe Glu Arg Pro Val Thr Val Gly Tyr Met Tyr Met Leu
            1220                1225                1230

Lys Leu Asn His Leu Val Asp Asp Lys Met His Ala Arg Ser Thr Gly
        1235                1240                1245

Ser Tyr Ser Leu Val Thr Gln Gln Pro Leu Gly Gly Lys Ala Gln Phe
    1250                1255                1260

Gly Gly Gln Arg Phe Gly Glu Met Glu Val Trp Ala Leu Glu Ala Tyr

1265                1270                1275                1280

Gly Ala Ala Tyr Thr Leu Gln Glu Met Leu Thr Val Lys Ser Asp Asp
                1285                1290                1295

Val Asn Gly Arg Thr Lys Met Tyr Lys Asn Ile Val Asp Gly Asn His
            1300                1305                1310

Ala Met Glu Pro Gly Met Pro Glu Ser Phe Asn Val Leu Leu Lys Glu
        1315                1320                1325

Ile Arg Ser Leu Gly Ile Asn Ile Glu Leu Glu Asp Glu
    1330                1335                1340


<210> 28
<211> 358
<212> PRT
<213> Vibrio cholerae

<220>
<221> PEPTIDE
<222> (1)..(358)
<223> >tr|Q9KUN7|Q9KUN7_VIBCH Fructose-bisphosphate
      aldolase, class II - Vibrio cholerae. (358 aa)

<400> 28
Met Ser Lys Ile Phe Asp Phe Val Lys Pro Gly Val Ile Ser Gly Asp
1               5                   10                  15

Asp Val Gln Lys Val Phe Glu Val Ala Lys Gln Asn Gly Phe Ala Leu
            20                  25                  30

Pro Ala Val Asn Cys Val Gly Thr Asp Ser Val Asn Gly Val Leu Glu
        35                  40                  45

Ala Ala Ala Lys Val Lys Ala Pro Val Ile Val Gln Phe Ser Asn Gly
    50                  55                  60

Gly Ala Ala Phe Phe Ala Gly Lys Gly Leu Lys Leu Glu Gly Gln Gln
65                  70                  75                  80

Ala Gln Ile Leu Gly Ala Ile Ala Gly Ala Lys Tyr Val His Thr Val
                85                  90                  95

Ala Glu Ala Tyr Gly Val Pro Val Ile Leu His Thr Asp His Ala Ala
            100                 105                 110

Lys Asn Leu Leu Pro Trp Ile Asp Gly Leu Leu Asp Ala Gly Glu Lys
        115                 120                 125

His Phe Ala Glu Thr Gly Lys Pro Leu Phe Ser Ser His Met Ile Asp
    130                 135                 140

Leu Ser Glu Glu Ser Leu Glu Glu Asn Ile Glu Ile Ser Ala Lys Tyr
145                 150                 155                 160

Leu Glu Arg Met Ala Lys Met Asn Met Thr Leu Glu Ile Glu Leu Gly
                165                 170                 175

Cys Thr Gly Gly Glu Glu Asp Gly Val Asp Asn Ser Asp Met Asp Ser
            180                 185                 190

Ser Glu Leu Tyr Thr Ser Pro Glu Asp Val Ala Tyr Ala Tyr Glu Lys
        195                 200                 205

Leu Asn Ala Ile Ser His Arg Phe Thr Ile Ala Ala Ser Phe Gly Asn
    210                 215                 220

Val His Gly Val Tyr Lys Pro Gly Asn Val Val Leu Thr Pro Thr Ile
225                 230                 235                 240

Leu Arg Asp Ser Gln Lys Tyr Val Ser Glu Lys Phe Gly Leu Pro Glu
                245                 250                 255

Asn Ser Leu Asn Phe Val Phe His Gly Gly Ser Gly Ser Ser Leu Glu
            260                 265                 270

Glu Ile Arg Glu Ser Ile Ser Tyr Gly Val Ile Lys Met Asn Ile Asp
        275                 280                 285

Thr Asp Thr Gln Trp Ala Thr Trp Asp Gly Ile Arg Gln Tyr Glu Ala
    290                 295                 300

Lys Asn His Asp Tyr Leu Gln Gly Gln Ile Gly Asn Pro Thr Gly Glu
305                 310                 315                 320

Asp Ala Pro Asn Lys Lys Tyr Tyr Asp Pro Arg Val Trp Leu Arg Ala
                325                 330                 335

Gly Gln Ala Ala Met Val Ala Arg Leu Glu Lys Ala Phe Ser Asp Leu
            340                 345                 350

Asn Ala Ile Asp Val Leu
        355



<210> 29
<211> 433
<212> PRT
<213> Vibrio cholerae

<220>
<221> PEPTIDE
<222> (1)..(433)
<223> >sp|Q9KPC5|ENO_VIBCH Enolase (EC 4.2.1.11)
      (2-phosphoglycerate dehydratase) (2-phospho-D-
      glycerate hydro-lyase) - Vibrio cholerae.

<400> 29
Met Ser Lys Ile Val Lys Val Leu Gly Arg Glu Ile Ile Asp Ser Arg
  1               5                  10                  15

Gly Asn Pro Thr Val Glu Ala Glu Val His Leu Glu Gly Gly Phe Val

             20                  25                  30

Gly Met Ala Ala Ala Pro Ser Gly Ala Ser Thr Gly Ser Arg Glu Ala
        35                  40                  45

Leu Glu Leu Arg Asp Gly Asp Lys Ser Arg Phe Leu Gly Lys Gly Val

50                  55                  60

Leu Lys Ala Leu Ala Ala Val Asn Gly Pro Ile Ala Asp Ala Leu Val
65                  70                  75                  80

Gly Lys Asp Ala Lys Asp Gln Ala Thr Ile Asp Gln Ile Met Ile Asp
                85                  90                  95

Leu Asp Gly Thr Glu Asn Lys Ser Asn Phe Gly Ala Asn Ala Ile Leu
            100                 105                 110

Ala Val Ser Leu Ala Asn Ala Lys Ala Ala Ala Ala Ala Lys Gly Met
        115                 120                 125

Pro Leu Tyr Glu His Ile Ala Glu Leu Asn Gly Thr Pro Gly Val Phe
    130                 135                 140

Ser Met Pro Leu Pro Met Met Asn Ile Ile Asn Gly Gly Glu His Ala
145                 150                 155                 160

Asp Asn Asn Val Asp Ile Gln Glu Phe Met Ile Gln Pro Val Gly Ala
                165                 170                 175

Lys Thr Leu Lys Glu Ala Val Arg Met Gly Ala Glu Val Phe His Asn
            180                 185                 190

Leu Ala Lys Val Leu Lys Ser Lys Gly Tyr Asn Thr Ala Val Gly Asp
        195                 200                 205

Glu Gly Gly Phe Ala Pro Asn Leu Lys Ser Asn Ala Glu Ala Leu Glu
    210                 215                 220

Val Ile Ala Glu Ala Val Ala Ala Ala Gly Tyr Lys Leu Gly Thr Asp
225                 230                 235                 240

Ile Thr Leu Ala Met Asp Cys Ala Ala Ser Glu Phe Tyr Asp Ala Glu
                245                 250                 255

Lys Lys Glu Tyr Asn Leu Lys Gly Glu Gly Arg Ile Phe Thr Ser Asn
            260                 265                 270

Gly Phe Ser Asp Phe Leu Glu Glu Leu Thr Glu Lys Phe Pro Ile Val
        275                 280                 285

Ser Ile Glu Asp Gly Leu Asp Glu Ser Asp Trp Glu Gly Phe Ala Tyr
    290                 295                 300

Gln Thr Glu Lys Leu Gly Lys Lys Ile Gln Ile Val Gly Asp Asp Leu
305                 310                 315                 320

Phe Val Thr Asn Thr Lys Ile Leu Lys Arg Gly Ile Asp Asn Gly Ile
                325                 330                 335

Ala Asn Ser Ile Leu Ile Lys Phe Asn Gln Ile Gly Ser Leu Thr Glu
            340                 345                 350

Thr Leu Ala Ala Ile Lys Met Ala Lys Asp Ala Gly Tyr Thr Ala Val
        355                 360                 365

Ile Ser His Arg Ser Gly Glu Thr Glu Asp Ala Thr Ile Ala Asp Leu
    370                 375                 380

Ala Val Gly Thr Ala Ala Gly Gln Ile Lys Thr Gly Ser Met Ser Arg
385                 390                 395                 400

Ser Asp Arg Val Ala Lys Tyr Asn Gln Leu Ile Arg Ile Glu Glu Ala
                405                 410                 415

Leu Gly Ser Arg Ala Pro Phe Asn Gly Leu Lys Glu Val Lys Gly Gln
            420                 425                 430

Ala

<210> 30
<211> 206
<212> PRT
<213> Vibrio cholerae

<220>
<221> PEPTIDE
<222> (1)..(206)
<223> >sp|Q9KP07|RS4_VIBCH 30S ribosomal protein S4 -
      Vibrio cholerae. (206 aa)

<400> 30
Met Ala Arg Tyr Leu Gly Pro Lys Leu Lys Leu Ser Arg Arg Glu Gly
  1               5                  10                  15

Thr Asp Leu Phe Leu Lys Ser Gly Val Arg Ala Ile Asp Thr Lys Cys
             20                  25                  30

Lys Ile Asp Asn Ala Pro Gly Val His Gly Ala Arg Arg Gly Arg Leu
         35                  40                  45

Ser Glu Tyr Gly Val Gln Leu Arg Glu Lys Gln Lys Val Arg Arg Ile
     50                  55                  60

Tyr Gly Val Leu Glu Lys Gln Phe Arg Asn Tyr Tyr Lys Glu Ala Ala
 65                  70                  75                  80

Arg Leu Lys Gly Asn Thr Gly Glu Asn Leu Leu Gln Leu Leu Glu Gly
                 85                  90                  95

Arg Leu Asp Asn Val Val Tyr Arg Met Gly Phe Gly Ala Thr Arg Ala
            100                 105                 110

Glu Ala Arg Gln Leu Val Ser His Lys Ala Ile Leu Val Asn Gly Lys
        115                 120                 125

Val Val Asn Val Pro Ser Phe Asn Val Ala Ala Asn Asp Val Val Ala
    130                 135                 140

Val Arg Glu Lys Ala Lys Lys Gln Ser Arg Ile Lys Ala Ala Leu Glu
145                 150                 155                 160

Val Ala Glu Gln Arg Glu Lys Pro Thr Trp Ile Glu Val Asp Val Asn
                165                 170                 175

Thr Met Glu Gly Thr Phe Lys Arg Met Pro Glu Arg Ser Asp Leu Ser
            180                 185                 190

Ala Asp Ile Asn Glu Gln Leu Ile Val Glu Leu Tyr Ser Lys
        195                 200                 205

<210> 31
<211> 92
<212> PRT
<213> Vibrio cholerae

<220>
<221> PEPTIDE
<222> (1)..(92)
<223> >sp|Q9KRK0|RL25_VIBCH 50S ribosomal protein L25 -
      Vibrio cholerae.

<400> 31
Met Lys Phe Glu Ala Val Leu Arg Thr Asp Leu Gly Lys Gly Ala Ser
1               5                   10                  15

Arg Arg Leu Arg Asn Thr Gly Tyr Phe Pro Ala Ile Val Tyr Gly Gly
            20                  25                  30

Glu Ala Ala Pro Val Ser Ile Ser Leu Asn His Asp Asp Val Met Asn
        35                  40                  45

Gln Met Asp Lys Pro Glu Phe Tyr Glu Ala Ile Val Leu Val Ile Asp
    50                  55                  60

Gly Gln Glu Val Lys Val Lys Pro Gln Asp Val Gln Arg His Ala Tyr
65                  70                  75                  80

Lys Pro Lys Val Glu His Met Asp Phe Ile Arg Ile
                85                  90

<210> 32
<211> 55
<212> PRT
<213> Vibrio cholerae

<220>
<221> PEPTIDE
<222> (1)..(55)
<223> >sp|Q9KQH3|RL32_VIBCH 50S ribosomal protein L32 -
      Vibrio cholerae. (55 aa)

<400> 32
Ala Val Gln Gln Asn Arg Lys Thr Arg Ser Arg Arg Gly Met Arg Arg
1               5                   10                  15

Ser His Asp Ala Leu Thr Ala Ala Ala Leu Ser Val Asp Ala Thr Ser
            20                  25                  30

Gly Glu Thr His Leu Arg His Asn Val Thr Ala Glu Gly Tyr Tyr Arg
        35                  40                  45

Gly Lys Lys Val Ile Asn Lys
    50                  55

<210> 33
<211> 71
<212> PRT
<213> Vibrio cholerae

<220>
<221> PEPTIDE
<222> (1)..(71)
<223> >sp|P66532|RS21_VIBCH 30S ribosomal protein S21 -
      Vibrio cholerae. (71 aa)

<400> 33
Met Pro Val Val Lys Val Arg Glu Asn Glu Pro Phe Asp Val Ala Leu
1               5                   10                  15

Arg Arg Phe Lys Arg Ser Cys Glu Lys Ala Gly Ile Leu Ser Glu Val
            20                  25                  30

Arg Arg Arg Glu His Tyr Glu Lys Pro Thr Thr Val Arg Lys Arg Ala
        35                  40                  45

Lys Ala Ala Ala Gln Lys Arg His Ala Lys Lys Leu Ala Arg Glu Asn
    50                  55                  60

Ala Arg Arg Val Arg Leu Tyr
65                  70



<210> 34
<211> 373
<212> PRT
<213> Vibrio cholerae

<220>
<221> PEPTIDE
<222> (1)..(373)
<223> >sp|Q06952|GMD1_VIBCH Probable GDP-mannose
      4,6-dehydratase (EC 4.2.1.47) (GDP-D-mannose
      dehydratase) - Vibrio cholerae. (373 aa)

<400> 34
Met Asn Lys Lys Val Ala Leu Ile Thr Gly Ile Thr Gly Gln Asp Gly
1               5                   10                  15

Ser Tyr Leu Ala Glu Phe Leu Leu Glu Lys Gly Tyr Glu Val His Gly
            20                  25                  30

Ile Lys Arg Arg Ser Ser Leu Phe Asn Thr Gln Arg Val Asp His Leu
        35                  40                  45

Tyr Lys Asp Pro His Glu Glu Asp Val Asn Phe Lys Leu His Tyr Gly
    50                  55                  60

Asp Leu Thr Asp Ser Ser Asn Leu Thr Arg Ile Leu Ala Glu Val Gln
65                  70                  75                  80

Pro Asp Glu Val Tyr Asn Leu Gly Ala Gln Ser His Val Ala Val Ser
                85                  90                  95

Phe Gln Ser Pro Glu Tyr Thr Ala Asp Val Asp Ala Ile Gly Thr Leu

            100                 105                 110

Arg Leu Leu Glu Ala Ile Arg Phe Leu Gly Leu Thr Lys Lys Thr Lys
        115                 120                 125

Phe Tyr Gln Ala Ser Thr Ser Glu Leu Tyr Gly Leu Val Gln Glu Ile
    130                 135                 140

Pro Gln Lys Glu Thr Thr Pro Phe Tyr Pro Arg Ser Pro Tyr Ala Val
145                 150                 155                 160

Ala Lys Met Tyr Ala Tyr Trp Ile Thr Ile Asn Tyr Arg Glu Ser Tyr
                165                 170                 175

Gly Ile Tyr Ala Cys Asn Gly Ile Leu Phe Asn His Glu Ser Pro Arg
            180                 185                 190

Arg Gly Glu Thr Phe Val Thr Arg Lys Ile Thr Arg Gly Met Ala Asn
        195                 200                 205

Ile Ala Gln Gly Leu Glu Lys Cys Leu Phe Met Gly Asn Leu Asp Ala
    210                 215                 220

Leu Arg Asp Trp Gly His Ala Lys Asp Tyr Val Lys Met Gln Trp Met
225                 230                 235                 240

Met Leu Gln Gln Asp Glu Pro Arg Asp Phe Val Ile Ala Thr Gly Val
                245                 250                 255

Gln Tyr Ser Val Arg Glu Phe Ile Asp Met Ser Ala Arg Glu Leu Gly
            260                 265                 270

Ile Glu Leu Glu Phe Val Gly Lys Gly Val Asp Glu Lys Ala Val Val
        275                 280                 285

Lys Ser Val Ile Gly Thr Lys Ala Pro Ala Ile Lys Val Gly Asp Ile
    290                 295                 300

Ile Val Ala Val Asp Pro Ala Tyr Phe Arg Pro Ala Glu Val Glu Thr
305                 310                 315                 320

Leu Leu Gly Asp Pro Ser Leu Ala Lys Lys Glu Leu Gly Trp Val Pro
                325                 330                 335

Glu Ile Thr Leu Gln Gln Met Val Ser Glu Met Val Ala Ser Asp Leu
            340                 345                 350

Glu Gln Ala Gln Ser His Ala Leu Leu Lys Lys His Gly Tyr Asn Val
        355                 360                 365

Asn Val Ser Val Glu
    370

<210> 35
<211> 510
<212> PRT
<213> Vibrio cholerae

<220>
<221> PEPTIDE

<222> (1)..(510)
<223> >sp|Q9KU60|SYK_VIBCH Lysyl-tRNA synthetase (EC
      6.1.1.6) (Lysine--tRNA ligase) (LysRS) - Vibrio
      cholerae. (510 aa)

<400> 35
Met Thr Asp Ala Val Gln Asn Glu Ile Asn Gln Glu Gln Ile Ala Gln
1               5                   10                  15

Glu Glu Asn Lys Leu Ile Ala Glu Arg Arg Ser Lys Leu Asp His Ile
            20                  25                  30

Arg Lys Asn Cys Lys Ala Asn Gly His Pro Asn Ser Phe Arg Arg Asp
        35                  40                  45

Ser Leu Ala Gly Asp Leu Gln Lys Lys Phe Gly Glu Lys Ser Lys Glu
    50                  55                  60

Glu Leu Glu Ala Leu Asn His Val Val Ser Ile Ala Gly Arg Val Met
65                  70                  75                  80

Ala Lys Arg Gly Pro Phe Leu Val Ile Gln Glu Thr Ser Gly Arg Ile
                85                  90                  95

Gln Ala Tyr Ala Ser Lys Glu Val Gln Gln Glu Leu Lys Asp Lys Tyr
            100                 105                 110

Gln Gly Leu Asp Ile Gly Asp Ile Ile Gly Val Gln Gly Ala Leu His
        115                 120                 125

Lys Ser Gly Lys Gly Asp Leu Tyr Val Asn Met Glu Gln Phe Gln Leu
    130                 135                 140

Leu Thr Lys Ala Leu Arg Pro Leu Pro Glu Lys Phe His Gly Leu Thr
145                 150                 155                 160

Asp Gln Glu Met Arg Tyr Arg Gln Arg Tyr Val Asp Leu Ile Val Asn
                165                 170                 175

Glu Asn Ser Arg Asn Ala Phe Ile Val Arg Ser Lys Val Met Ser Ala
            180                 185                 190

Ile Arg Asn Phe Met Ile Ser Lys Gln Phe Met Glu Val Glu Thr Pro
        195                 200                 205

Met Met His Val Ile Pro Gly Gly Ala Ser Ala Arg Pro Phe Ile Thr
    210                 215                 220

His His Asn Ala Leu Asp Met Pro Met Tyr Leu Arg Ile Ala Pro Glu
225                 230                 235                 240

Leu Tyr Leu Lys Arg Leu Val Val Gly Gly Phe Asp Arg Val Phe Glu
                245                 250                 255

Ile Asn Arg Asn Phe Arg Asn Glu Gly Leu Ser Pro Arg His Asn Pro
            260                 265                 270

Glu Phe Thr Met Met Glu Phe Tyr Met Ala Tyr Ala Asp Tyr Lys Asp
        275                 280                 285

Leu Met Asp Leu Thr Glu Glu Leu Leu Ser Ser Val Ala Leu Glu Val

290                 295                 300

Leu Gly Ser Thr Ser Met Pro Tyr Gly Glu His Thr Val Glu Phe Gly
305                 310                 315                 320

Gly Lys Tyr Ala Arg Met Ser Met Phe Asp Ala Ile Lys His Tyr Asn
                325                 330                 335

Pro Asn His Ala Gln Ile Gln Ala Leu Thr Glu Glu Asp Ile Gln Asn
            340                 345                 350

Arg Asp Leu Met Val Ser Ile Ala Lys Ser Val His Val Asp Val Glu
        355                 360                 365

Pro Phe Trp Thr Cys Gly Gln Leu Leu Glu Glu Ile Phe Gly Glu Thr
    370                 375                 380

Ala Glu Pro Lys Leu Met Gln Pro Thr Phe Ile Thr Gly Tyr Pro Ala
385                 390                 395                 400

Asp Ile Ser Pro Leu Ala Arg Arg Ser Asp Asp Asn Pro Phe Phe Thr
                405                 410                 415

Asp Arg Phe Glu Phe Phe Ile Gly Gly Arg Glu Val Ala Asn Gly Phe
            420                 425                 430

Ser Glu Leu Asn Asp Ala Glu Asp Gln Asp Ala Arg Phe Lys Ala Gln
        435                 440                 445

Val Glu Ala Lys Glu Ser Gly Asp Asp Glu Ala Met Phe Tyr Asp Ala
    450                 455                 460

Asp Tyr Ile Thr Ala Leu Glu His Gly Leu Pro Pro Thr Ala Gly Gln
465                 470                 475                 480

Gly Ile Gly Ile Asp Arg Leu Val Met Leu Leu Thr Asn Thr His Thr
                485                 490                 495

Ile Arg Asp Val Ile Leu Phe Pro Ala Met Arg Pro Gln Gln
            500                 505                 510


<210> 36
<211> 207
<212> PRT
<213> Vibrio cholerae

<220>
<221> PEPTIDE
<222> (1)..(207)
<223> >tr|Q9KTZ9|Q9KTZ9_VIBCH Antioxidant, AhpC/Tsa
      family - Vibrio cholerae (207 aa)

<400> 36
Met Leu Arg Ser Lys Lys Met Val Leu Val Gly Arg Lys Ala Pro Asp
1               5                   10                  15

Phe Thr Ala Ala Ala Val Leu Gly Asn Gly Glu Ile Val Asp Asn Phe
            20                  25                  30

Asn Phe Ala Glu Phe Thr Lys Gly Lys Lys Ala Val Val Phe Phe Tyr

35                  40                  45

Pro Leu Asp Phe Thr Phe Val Cys Pro Ser Glu Leu Ile Ala Phe Asp
    50                  55                  60

Asn Arg Tyr Glu Asp Phe Leu Ala Lys Gly Val Glu Val Ile Gly Val
65                  70                  75                  80

Ser Ile Asp Ser Gln Phe Ser His Asn Ala Trp Arg Asn Thr Ala Val
                85                  90                  95

Glu Asn Gly Gly Ile Gly Gln Val Lys Tyr Pro Leu Val Ala Asp Val
            100                 105                 110

Lys His Glu Ile Cys Lys Ala Tyr Asp Val Glu His Pro Glu Ala Gly
        115                 120                 125

Val Ala Phe Arg Gly Ser Phe Leu Ile Asp Glu Glu Gly Met Val Arg
    130                 135                 140

His Gln Val Val Asn Asp Leu Pro Leu Gly Arg Asn Ile Asp Glu Met
145                 150                 155                 160

Leu Arg Met Val Asp Ala Leu Asn Phe His Gln Thr His Gly Glu Val
                165                 170                 175

Cys Pro Ala Gln Trp Glu Ala Gly Lys Ala Gly Met Glu Ala Ser Pro
            180                 185                 190

Lys Gly Val Ala Ala Phe Leu Ser Gln Tyr Ser Ala Asp Leu Lys
        195                 200                 205


<210> 37
<211> 129
<212> PRT
<213> Vibrio cholerae

<220>
<221> PEPTIDE
<222> (1)..(129)
<223> >sp|P66367|RS11_VIBCH 30S ribosomal protein S11 -
      Vibrio cholerae. (129 aa)

<400> 37
Met Ala Lys Gln Pro Thr Arg Ala Arg Lys Arg Val Arg Lys Gln Val
1               5                   10                  15

Ala Asp Gly Val Ala His Ile His Ala Ser Phe Asn Asn Thr Ile Val
            20                  25                  30

Thr Ile Thr Asp Arg Gln Gly Asn Ala Leu Ala Trp Ala Thr Ala Gly
        35                  40                  45

Gly Ser Gly Phe Arg Gly Ser Arg Lys Ser Thr Pro Phe Ala Ala Gln
    50                  55                  60

Val Ala Ala Glu Arg Cys Ala Glu Met Ala Lys Glu Tyr Gly Leu Lys
65                  70                  75                  80

Asn Leu Glu Val Met Val Lys Gly Pro Gly Pro Gly Arg Glu Ser Thr

85                  90                  95

Val Arg Ala Leu Asn Ala Ala Gly Phe Arg Ile Thr Asn Ile Val Asp
            100                 105                 110

Ala Thr Pro Ile Pro His Asn Gly Cys Arg Pro Pro Lys Lys Arg Arg
        115                 120                 125

Val

<210> 38
<211> 394
<212> PRT
<213> Vibrio cholerae

<220>
<221> PEPTIDE
<222> (1)..(394)
<223> >sp|Q9KV37|EFTU1_VIBCH Elongation factor Tu-A
      (EF-Tu-A) - Vibrio cholerae. (394 aa)

<400> 38
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
        35                  40                  45

Ser Ile Asp Asn Ala Pro Glu Glu Arg Glu Arg Gly Ile Thr Ile Asn
    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Arg
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp

195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala
        355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390


<210> 39
<211> 319
<212> PRT
<213> Vibrio cholerae


<220>
<221> PEPTIDE
<222> (1)..(319)
<223> >tr|Q9KPW8|Q9KPW8_VIBCH Acetyl-CoA carboxylase,
      carboxyl transferase alpha subunit - Vibrio
      cholerae. (319 aa)

<400> 39
Met Ser Leu Asn Phe Leu Asp Phe Glu Lys Pro Ile Val Glu Leu Glu
1               5                   10                  15

Thr Lys Ile Gln Ala Leu Arg Asp Val Ser Arg His Ser Thr Ser Ala
            20                  25                  30

Ser Val Asp Leu Asp Lys Glu Leu Glu Gln Leu Glu Lys Lys Ser Leu
        35                  40                  45

Glu Leu Lys Lys Lys Ile Phe Ser Asp Leu Gly Ala Trp Gln Val Ala
    50                  55                  60

Gln Leu Ala Arg His Pro Gln Arg Pro Tyr Thr Leu Asp Tyr Leu Lys
65                  70                  75                  80

His Ile Phe Thr Glu Phe Asp Glu Leu Ala Gly Asp Arg Ala Tyr Ala
                85                  90                  95

Asp Asp Lys Ala Ile Val Gly Gly Ile Ala Arg Leu Glu Gly Arg Ser
            100                 105                 110

Val Met Val Ile Gly His Gln Lys Gly Arg Glu Thr Arg Glu Lys Val
        115                 120                 125

Lys Arg Asn Phe Gly Met Pro Lys Pro Glu Gly Tyr Arg Lys Ala Leu
    130                 135                 140

Arg Leu Met Glu Met Ala Glu Arg Phe Asn Met Pro Ile Ile Thr Phe
145                 150                 155                 160

Ile Asp Thr Ala Gly Ala Tyr Pro Gly Val Gly Ala Glu Glu Arg Gly
                165                 170                 175

Gln Ser Glu Ala Ile Ala Lys Asn Leu Lys Val Met Ser Gly Leu Lys
            180                 185                 190

Val Pro Val Ile Cys Asn Val Val Gly Glu Gly Gly Ser Gly Gly Ala
        195                 200                 205

Leu Ala Ile Gly Val Gly Asp Tyr Val Asn Met Leu Gln Tyr Ser Thr
    210                 215                 220

Tyr Ser Val Ile Ser Pro Glu Gly Cys Ala Ser Ile Leu Trp Arg Asp
225                 230                 235                 240

Ser Asp Lys Ala Pro Gln Ala Ala Glu Ala Met Gly Leu Ile Ala Pro
                245                 250                 255

Arg Leu Lys Glu Leu Glu Leu Ile Asp Glu Ile Ile Glu Glu Pro Leu
            260                 265                 270

Gly Gly Ala His Arg Asp His Lys Gln Thr Ala Glu Asn Val Lys Ala
        275                 280                 285

Thr Leu Leu Arg Gln Leu Ala Asp Leu Asp Ala Leu Asp His Glu Asn
    290                 295                 300

Leu Leu Glu Arg Arg Tyr Gln Arg Leu Met Asn Tyr Gly Tyr Cys
305                 310                 315

<210> 40
<211> 330
<212> PRT
<213> Vibrio cholerae

<220>
<221> PEPTIDE
<222> (1)..(330)

<223> >sp|Q9KP08|RPOA_VIBCH DNA-directed RNA polymerase
      alpha chain (EC 2.7.7.6) (RNAP alpha subunit)
      (Transcriptase alpha chain) (RNA polymerase alpha
      subunit) - Vibrio cholerae. (330 aa)

<400> 40
Met Gln Gly Ser Val Thr Glu Phe Leu Lys Pro Arg Leu Val Asp Ile
1               5                   10                  15

Glu Gln Ile Ser Thr Thr His Ala Lys Val Thr Leu Glu Pro Leu Glu
            20                  25                  30

Arg Gly Phe Gly His Thr Leu Gly Asn Ala Leu Arg Arg Ile Leu Leu
        35                  40                  45

Ser Ser Met Pro Gly Cys Ala Val Thr Glu Val Glu Ile Glu Gly Val
    50                  55                  60

Leu His Glu Tyr Ser Thr Lys Glu Gly Val Gln Glu Asp Ile Leu Glu
65                  70                  75                  80

Ile Leu Leu Asn Leu Lys Gly Leu Ala Val Arg Val Ala Glu Gly Lys
                85                  90                  95

Asp Glu Val Phe Ile Thr Leu Asn Lys Ser Gly Ser Gly Pro Val Val
            100                 105                 110

Ala Gly Asp Ile Thr His Asp Gly Asp Val Glu Ile Val Asn Pro Glu
        115                 120                 125

His Val Ile Cys His Leu Thr Ser Asp Asn Ala Ala Ile Ala Met Arg
    130                 135                 140

Ile Lys Val Glu Arg Gly Arg Gly Tyr Val Pro Ala Ser Ala Arg Ile
145                 150                 155                 160

His Thr Glu Glu Asp Glu Arg Pro Ile Gly Arg Leu Leu Val Asp Ala
                165                 170                 175

Thr Phe Ser Pro Val Asp Lys Ile Ala Tyr Ser Val Glu Ala Ala Arg
            180                 185                 190

Val Glu Gln Arg Thr Asp Leu Asp Lys Leu Val Ile Asp Met Glu Thr
        195                 200                 205

Asn Gly Thr Leu Glu Pro Glu Glu Ala Ile Arg Arg Ala Ala Thr Ile
    210                 215                 220

Leu Ala Glu Gln Leu Asp Ala Phe Val Asp Leu Arg Asp Val Arg Val
225                 230                 235                 240

Pro Glu Glu Lys Glu Glu Lys Pro Glu Phe Asp Pro Ile Leu Leu Arg
                245                 250                 255

Pro Val Asp Asp Leu Glu Leu Thr Val Arg Ser Ala Asn Cys Leu Lys
            260                 265                 270

Ala Glu Ala Ile His Tyr Ile Gly Asp Leu Val Gln Arg Thr Glu Val
        275                 280                 285

Glu Leu Leu Lys Thr Pro Asn Leu Gly Lys Lys Ser Leu Thr Glu Ile
    290                 295                 300

Lys Asp Val Leu Ala Ser Arg Gly Leu Ser Leu Gly Met Arg Leu Glu
305                 310                 315                 320

Asn Trp Pro Pro Ala Ser Ile Ala Glu Asp
                325                 330



<210> 41
<211> 513
<212> PRT
<213> Vibrio cholerae

<220>
<221> PEPTIDE
<222> (1)..(513)
<223> >sp|Q9KNH3|ATPA_VIBCH ATP synthase alpha chain (EC
      3.6.3.14) - Vibrio cholerae. (513 aa)

<400> 41
Met Gln Leu Asn Ser Thr Glu Ile Ser Asp Leu Ile Lys Gln Arg Ile
  1               5                  10                  15

Glu Ser Phe Asn Val Val Ser Glu Ala Arg Asn Glu Gly Thr Ile Val
             20                  25                  30

Ser Val Ser Asp Gly Ile Ile Arg Ile His Gly Leu Ala Asp Val Met
         35                  40                  45

Gln Gly Glu Met Ile Glu Leu Pro Gly Asn Arg Tyr Ala Leu Ala Leu
     50                  55                  60

Asn Leu Glu Arg Asp Ser Val Gly Ala Val Val Met Gly Pro Tyr Ala
 65                  70                  75                  80

Asp Leu Arg Glu Gly Met Lys Val Thr Gly Thr Gly Arg Ile Leu Glu
                 85                  90                  95

Val Pro Val Gly Pro Glu Leu Leu Gly Arg Val Val Asn Thr Leu Gly
            100                 105                 110

Glu Pro Ile Asp Gly Lys Gly Pro Ile Gly Ala Lys Gln Thr Ser Pro
        115                 120                 125

Val Glu Val Ile Ala Pro Gly Val Ile Asp Arg Lys Ser Val Asp Gln
    130                 135                 140

Pro Val Gln Thr Gly Tyr Lys Ser Val Asp Ser Met Ile Pro Ile Gly
145                 150                 155                 160

Arg Gly Gln Arg Glu Leu Ile Ile Gly Asp Arg Gln Thr Gly Lys Thr
                165                 170                 175

Ala Leu Ala Ile Asp Ala Ile Ile Asn Gln Lys Asn Ser Gly Ile Tyr
            180                 185                 190

Ser Ile Tyr Val Ala Ile Gly Gln Lys Ala Ser Thr Ile Ala Asn Val
        195                 200                 205

Val Arg Lys Leu Glu Glu His Gly Ala Leu Lys Asn Thr Ile Val Val
    210                 215                 220

Val Ala Ser Ala Ser Glu Ser Ala Ala Leu Gln Tyr Leu Ala Pro Tyr
225                 230                 235                 240

Ser Gly Cys Ala Met Gly Glu Tyr Phe Arg Asp Arg Gly Glu Asp Ala
                245                 250                 255

Leu Ile Val Tyr Asp Asp Leu Ser Lys Gln Ala Val Ala Tyr Arg Gln
            260                 265                 270

Ile Ser Leu Leu Leu Arg Arg Pro Pro Gly Arg Glu Ala Phe Pro Gly
        275                 280                 285

Asp Val Phe Tyr Leu His Ser Arg Leu Leu Glu Arg Ala Ala Arg Val
    290                 295                 300

Asn Glu Glu Tyr Val Glu Arg Phe Thr Lys Gly Glu Val Lys Gly Lys
305                 310                 315                 320

Thr Gly Ser Leu Thr Ala Leu Pro Ile Ile Glu Thr Gln Ala Gly Asp
                325                 330                 335

Val Ser Ala Phe Val Pro Thr Asn Val Ile Ser Ile Thr Asp Gly Gln
            340                 345                 350

Ile Phe Leu Gln Thr Glu Leu Phe Asn Ala Gly Val Arg Pro Ala Val
        355                 360                 365

Asp Pro Gly Ile Ser Val Ser Arg Val Gly Gly Ala Ala Gln Thr Lys
    370                 375                 380

Ile Val Lys Lys Leu Ser Gly Gly Ile Arg Thr Ala Leu Ala Ala Tyr

385                 390                 395                 400

Arg Glu Leu Ala Ala Phe Ala Gln Phe Ser Ser Asp Leu Asp Glu Ala
                405                 410                 415

Thr Lys Arg Gln Leu Thr His Gly Gln Lys Val Thr Glu Leu Met Lys
            420                 425                 430

Gln Lys Gln Tyr Ala Pro Met Ser Val Phe Asp Gln Ala Leu Val Ile
        435                 440                 445

Phe Ala Ala Glu Arg Gly Tyr Leu Thr Asp Val Glu Leu Asn Lys Val
    450                 455                 460

Leu Asp Phe Glu Ala Ala Leu Leu Ser Tyr Ala Arg Ala His Tyr Ala
465                 470                 475                 480

Glu Leu Ala Ala Gln Ile Asp Lys Thr Gly Ala Tyr Asn Asp Glu Ile
                485                 490                 495

Glu Ala Gln Leu Lys Lys Leu Val Asp Asp Phe Lys Ala Thr Gln Thr
            500                 505                 510

Trp

<210> 42
<211> 1401
<212> PRT
<213> Vibrio cholerae

<220>
<221> PEPTIDE
<222> (1)..(1401)
<223> >sp|Q9KV29|RPOC_VIBCH DNA-directed RNA polymerase
      beta' chain (EC 2.7.7.6) (RNAP beta' subunit)
      (Transcriptase beta' chain) (RNA polymerase beta'
      subunit) - Vibrio cholerae. (1401 aa)

<400> 42
Met Lys Asp Leu Leu Asn Phe Leu Lys Ala Gln His Lys Thr Glu Glu
1               5                   10                  15

Phe Asp Ala Ile Lys Ile Gly Leu Ala Ser Pro Asp Met Ile Arg Ser
            20                  25                  30

Trp Ser Phe Gly Glu Val Lys Lys Pro Glu Thr Ile Asn Tyr Arg Thr
        35                  40                  45

Phe Lys Pro Glu Arg Asp Gly Leu Phe Cys Ala Arg Ile Phe Gly Pro
    50                  55                  60

Val Lys Asp Tyr Glu Cys Leu Cys Gly Lys Tyr Lys Arg Leu Lys His
65                  70                  75                  80

Arg Gly Val Ile Cys Glu Lys Cys Gly Val Glu Val Thr Gln Thr Lys
                85                  90                  95

Val Arg Arg Asp Arg Met Gly His Ile Glu Leu Ala Ser Pro Val Ala
            100                 105                 110

His Ile Trp Phe Leu Lys Ser Leu Pro Ser Arg Ile Gly Leu Leu Met
        115                 120                 125

Asp Met Pro Leu Arg Asp Ile Glu Arg Val Leu Tyr Phe Glu Met Tyr
    130                 135                 140

Val Val Thr Glu Pro Gly Met Thr Asp Leu Glu Arg Gly Gln Met Leu
145                 150                 155                 160

Thr Glu Glu Glu Tyr Leu Asp Arg Leu Glu Glu Trp Gly Asp Glu Phe
                165                 170                 175

Thr Ala Lys Met Gly Ala Glu Ala Ile Lys Asp Leu Leu Ala Ser Met
            180                 185                 190

Asp Leu Pro Ala Glu Ala Glu Gln Met Arg Glu Glu Leu Asp Thr Thr
        195                 200                 205

Asn Ser Glu Thr Lys Arg Lys Lys Leu Thr Lys Arg Leu Lys Leu Val
    210                 215                 220

Glu Ala Phe Val Ala Ser Gly Asn Lys Pro Glu Trp Met Ile Leu Thr
225                 230                 235                 240

Val Leu Pro Val Leu Pro Pro Asp Leu Arg Pro Leu Val Pro Leu Asp

                245                 250                 255

Gly Gly Arg Phe Ala Thr Ser Asp Leu Asn Asp Leu Tyr Arg Arg Val
            260                 265                 270

Ile Asn Arg Asn Asn Arg Leu Lys Arg Leu Leu Glu Leu Ala Ala Pro
        275                 280                 285

Asp Ile Ile Val Arg Asn Glu Lys Arg Met Leu Gln Glu Ser Val Asp
    290                 295                 300

Ala Leu Leu Asp Asn Gly Arg Arg Gly Arg Ala Ile Thr Gly Ser Asn
305                 310                 315                 320

Lys Arg Pro Leu Lys Ser Leu Ala Asp Met Ile Lys Gly Lys Gln Gly
                325                 330                 335

Arg Phe Arg Gln Asn Leu Leu Gly Lys Arg Val Asp Tyr Ser Gly Arg
            340                 345                 350

Ser Val Ile Thr Val Gly Pro Tyr Leu Arg Leu His Gln Cys Gly Leu
        355                 360                 365

Pro Lys Lys Met Ala Leu Glu Leu Phe Lys Pro Phe Ile Tyr Ser Lys
    370                 375                 380

Leu Glu Thr Arg Gly Leu Ala Thr Thr Ile Lys Ala Ala Lys Lys Met
385                 390                 395                 400

Val Glu Arg Glu Glu Ala Val Val Trp Asp Ile Leu Asp Glu Val Ile
                405                 410                 415

Arg Glu His Pro Val Leu Leu Asn Arg Ala Pro Thr Leu His Arg Leu
            420                 425                 430

Gly Ile Gln Ala Phe Glu Pro Val Leu Ile Glu Gly Lys Ala Ile Gln
        435                 440                 445

Leu His Pro Leu Val Cys Ala Ala Tyr Asn Ala Asp Phe Asp Gly Asp
    450                 455                 460

Gln Met Ala Val His Val Pro Leu Thr Leu Glu Ala Gln Leu Glu Ala
465                 470                 475                 480

Arg Thr Leu Met Met Ser Thr Asn Asn Ile Leu Ser Pro Ala Ser Gly
                485                 490                 495

Asp Pro Ile Ile Val Pro Ser Gln Asp Val Val Leu Gly Leu Tyr Tyr
            500                 505                 510

Met Thr Arg Glu Lys Ile Asn Ala Lys Gly Glu Gly Met Tyr Leu Thr
        515                 520                 525

Gly Pro Ala Glu Ala Glu Lys Ala Tyr Arg Thr Lys Thr Ala Glu Leu
    530                 535                 540

His Ala Arg Val Lys Val Arg Ile Thr Glu Thr Ile Lys His Glu Asn
545                 550                 555                 560

Gly Lys Leu Thr Thr Glu Thr Lys Met Ile Asp Thr Thr Val Gly Arg
                565                 570                 575

Ala Met Leu Trp Gln Ile Val Pro Lys Gly Leu Pro Tyr Ser Leu Val
            580                 585                 590

Asn Gln Lys Leu Gly Lys Lys Gln Ile Ser Asn Leu Leu Asn Glu Ala
        595                 600                 605

Tyr Arg Lys Leu Gly Leu Lys Asp Thr Val Ile Phe Ala Asp Gln Ile
    610                 615                 620

Met Tyr Thr Gly Phe Ala Tyr Ala Ala Leu Ser Gly Val Ser Val Gly
625                 630                 635                 640

Ile Asp Asp Met Val Val Pro Ala Ala Lys Tyr Thr Glu Ile Ala Glu
                645                 650                 655

Ala Glu Glu Glu Val Arg Glu Ile Gln Glu Gln Phe Gln Ser Gly Leu
            660                 665                 670

Val Thr Ala Gly Glu Arg Tyr Asn Lys Val Ile Asp Ile Trp Ala Ser
        675                 680                 685

Thr Asn Asp Arg Val Ala Lys Ala Met Met Glu Asn Leu Ser Ser Glu
    690                 695                 700

Gln Val Ile Asn Arg Gln Gly Glu Gln Glu Lys Gln Glu Ser Phe Asn
705                 710                 715                 720

Ser Ile Tyr Met Met Ala Asp Ser Gly Ala Arg Gly Ser Ala Ala Gln
                725                 730                 735

Ile Arg Gln Leu Ala Gly Met Arg Gly Leu Met Ala Arg Pro Asp Gly
            740                 745                 750

Ser Ile Ile Glu Thr Pro Ile Thr Ala Asn Phe Lys Glu Gly Leu Asn
        755                 760                 765

Val Leu Gln Tyr Phe Ile Ser Thr His Gly Ala Arg Lys Gly Leu Ala
    770                 775                 780

Asp Thr Ala Leu Lys Thr Ala Asn Ser Gly Tyr Leu Thr Arg Arg Leu
785                 790                 795                 800

Val Asp Val Ala Gln Asp Val Val Val Thr Glu His Asp Cys Gly Thr
                805                 810                 815

Leu Glu Gly Val Val Met Thr Pro His Ile Glu Gly Gly Asp Val Lys
            820                 825                 830

Val Ala Leu Thr Glu Leu Ala Leu Gly Arg Val Val Ser Glu Asp Ile
        835                 840                 845

Leu Lys Pro Gly Thr Asp Glu Val Leu Ile Pro Arg Asn Thr Leu Leu
    850                 855                 860

Asp Glu Lys Trp Cys Lys Val Ile Asn Asp Asn Ser Val Asp Gln Ile
865                 870                 875                 880

Lys Val Arg Ser Val Val Thr Cys Asp Ser Asp Phe Gly Cys Cys Ala
                885                 890                 895

Gln Cys Tyr Gly Arg Asp Leu Ala Arg Gly His Leu Val Asn Gln Gly
            900                 905                 910

Glu Ala Val Gly Val Ile Ala Ala Gln Ser Ile Gly Glu Pro Gly Thr
        915                 920                 925

Gln Leu Thr Met Arg Thr Phe His Ile Gly Gly Ala Ala Ser Thr Ala
    930                 935                 940

Ala Ala Glu Asn Ser Ile Gln Ala Lys Asn Asn Gly Ser Val Lys Leu
945                 950                 955                 960

His Asn Ala Lys Phe Val Thr Asn Lys Asp Gly Lys Leu Val Ile Thr
                965                 970                 975

Ser Arg Ala Ser Glu Leu Thr Ile Ile Asp Glu Phe Gly Arg Thr Lys
            980                 985                 990

Glu Lys His Lys Leu Pro Tyr Gly Ser Met Leu Ser Lys Ala Asp Gly
        995                 1000                1005

Asp Ala Val Ala Ala Gly Glu Thr Val Ala Asn Trp Glu Ala His Thr
    1010                1015                1020

Met Pro Ile Ile Thr Glu Val Ala Gly Arg Val Gln Phe Val Asp Met
1025                1030                1035                1040

Ile Asp Gly Val Thr Val Ser Arg Gln Thr Asp Asp Leu Thr Gly Leu
                1045                1050                1055

Ser Ser Ser Glu Val Thr Glu Ala Ala Ala Arg Pro Ala Ala Gly Lys
            1060                1065                1070

Asp Met Arg Pro Ala Ile Lys Leu Val Asp Ala Asn Gly Lys Asp Val
        1075                1080                1085

Leu Ile Pro Gly Thr Asp Met Pro Ala Gln Tyr Phe Leu Pro Gly Lys
    1090                1095                1100

Ala Ile Val Asn Leu Asp Asp Gly Ala Glu Val Asn Val Gly Asp Thr
1105                1110                1115                1120

Leu Ala Arg Ile Pro Gln Lys Ser Gly Gly Asn Lys Asp Ile Thr Gly
                1125                1130                1135

Gly Leu Pro Arg Val Ala Asp Leu Phe Glu Ala Arg Lys Pro Lys Glu
            1140                1145                1150

Pro Ala Ile Leu Ala Glu His Ser Gly Thr Val Ser Phe Gly Lys Glu
        1155                1160                1165

Thr Lys Gly Lys Arg Arg Leu Ile Ile Thr Arg Asp Ser Gly Asp Thr
    1170                1175                1180

Tyr Glu Glu Met Ile Pro Lys His Arg Gln Leu Asn Val Phe Glu Gly
1185                1190                1195                1200

Glu Arg Ile Glu Arg Gly Asp Val Ile Ala Asp Gly Pro Glu Ser Pro
                1205                1210                1215

His Asp Ile Leu Arg Leu Arg Gly Ile His Ala Val Thr Thr Tyr Ile

1220                1225                1230

Ala Asn Glu Val Gln Glu Val Tyr Arg Leu Gln Gly Val Lys Ile Asn
        1235                1240                1245

Asp Lys His Ile Glu Thr Ile Val Arg Gln Met Leu Arg Lys Cys Thr
    1250                1255                1260

Ile Thr Phe Ala Gly Asp Ser Glu Phe Leu Pro Gly Glu Thr Val Glu
1265                1270                1275                1280

Tyr Ser Gln Val Lys Ile Ala Asn Arg Lys Leu Val Glu Glu Gly Lys
                1285                1290                1295

Glu Pro Ala Arg Phe Glu Arg Glu Leu Leu Gly Ile Thr Lys Ala Ser
            1300                1305                1310

Leu Ala Thr Glu Ser Phe Ile Ser Ala Ala Ser Phe Gln Glu Thr Thr
        1315                1320                1325

Arg Val Leu Thr Glu Ala Ala Val Ser Gly Lys Arg Asp Asp Leu Arg
    1330                1335                1340

Gly Leu Lys Glu Asn Val Ile Val Gly Arg Leu Ile Pro Ala Gly Thr
1345                1350                1355                1360

Gly Phe Ala Tyr His Gln Asp Arg Gln Ala Lys Arg Ala Gln Glu Gln
                1365                1370                1375

Gln Gly Pro Ser Ala Glu Gln Ala Thr Asp Asn Leu Ala Ala Leu Leu
            1380                1385                1390

Asn Ala Gly Phe Ser Ser Asp Asp Glu
        1395                1400


<210> 43
<211> 82
<212> PRT
<213> Vibrio cholerae

<220>
<221> PEPTIDE
<222> (1)..(82)
<223> >sp|Q9KUG0|RS16_VIBCH 30S ribosomal protein S16 -
      Vibrio cholerae. (82 aa)

<400> 43
Met Val Thr Ile Arg Leu Ala Arg His Gly Ala Lys Lys Arg Pro Phe
1               5                   10                  15

Tyr Gln Ile Val Val Ala Asp Ser Arg Asn Ser Ala Thr Gly Arg Phe
            20                  25                  30

Ile Glu Lys Val Gly Phe Phe Asn Pro Thr Ala Thr Gly Gln Glu Glu
        35                  40                  45

Gly Leu Arg Leu Asp Leu Asp Arg Val Asn His Trp Val Ser Gln Gly
    50                  55                  60

Ala Ser Leu Ser Asp Arg Val Ala Gln Leu Val Lys Thr Ala Gln Lys

65                  70                  75                  80

Ala Ala

<210> 44
<211> 122
<212> PRT
<213> Vibrio cholerae

<220>
<221> PEPTIDE
<222> (1)..(122)
<223> >sp|Q9KUZ2|RS6_VIBCH 30S ribosomal protein S6 -
      Vibrio cholerae. (122 aa)

<400> 44
Met Arg His Tyr Glu Ile Val Phe Met Val His Pro Asp Gln Ser Glu
1               5                   10                  15

Gln Val Ala Gly Met Ile Glu Arg Tyr Thr Gly Ser Ile Thr Glu Ala
            20                  25                  30

Gly Gly Lys Ile His Arg Leu Glu Asp Trp Gly Arg Arg Gln Leu Ala
        35                  40                  45

Tyr Pro Ile Asn Lys Leu His Lys Ala His Tyr Val Leu Met Asn Val
    50                  55                  60

Glu Ala Asp Gln Ala Val Val Asp Glu Leu Glu Thr Ala Phe Arg Phe
65                  70                  75                  80

Asn Asp Ala Val Leu Arg Asn Met Ile Met Arg Thr Lys Ala Ala Ile
                85                  90                  95

Thr Glu Pro Ser Ile Met Leu Lys Ala Arg Glu Glu Arg Val Lys Arg
            100                 105                 110

Asp Glu Met Lys Phe Asp Ala Asp Val Glu
        115                 120

<210> 45
<211> 85
<212> PRT
<213> Vibrio cholerae

<220>
<221> PEPTIDE
<222> (1)..(85)
<223> >sp|Q9KTD6|PTHP_VIBCH Phosphocarrier protein HPr
      (Histidine-containing protein) - Vibrio cholerae.
      (85 aa)

<400> 45
Met Tyr Glu Lys Gln Val Glu Ile Thr Ala Glu Asn Gly Leu His Thr
1               5                   10                  15

Arg Pro Ala Ala Gln Phe Val Lys Glu Ala Lys Ala Phe Asp Ala Asp
            20                  25                  30

Ile Thr Val Thr Ser Asn Gly Lys Ser Ala Ser Ala Lys Ser Leu Phe
        35                  40                  45

Lys Leu Gln Thr Leu Gly Leu Val Lys Gly Thr Val Val Thr Ile Ser
    50                  55                  60

Ala Glu Gly Pro Gln Ala Lys Glu Ala Val Glu His Leu Val Ala Leu
65                  70                  75                  80

Met Asp Gln Leu His
                85

<210> 46
<211> 894
<212> PRT
<213> Vibrio cholerae

<220>
<221> PEPTIDE
<222> (1)..(894)
<223> >tr|Q9KQG5|Q9KQG5_VIBCH Alcohol
      dehydrogenase/acetaldehyde dehydrogenase - Vibrio
      cholerae.

<400> 46
Met Pro Val Thr Asn Leu Ala Glu Leu Asp Ala Leu Val Ala Arg Val
1               5                   10                  15

Lys Ala Ala Gln Ala Glu Phe Ala Thr Phe Ser Gln Glu Gln Val Asp
            20                  25                  30

Lys Ile Phe Arg Ala Ala Ser Leu Ala Ala Asn Gln Ala Arg Ile Pro
        35                  40                  45

Leu Ala Gln Met Ala Val Glu Glu Ser Gly Met Gly Ile Val Glu Asp
    50                  55                  60

Lys Val Ile Lys Asn His Phe Ala Ser Glu Phe Ile Tyr Asn Lys Tyr
65                  70                  75                  80

Lys Asp Glu Lys Thr Cys Gly Ile Leu Glu Glu Asp Asp Asn Leu Gly
                85                  90                  95

Thr Met Thr Ile Ala Glu Pro Val Gly Ile Ile Cys Gly Ile Val Pro
            100                 105                 110

Thr Thr Asn Pro Thr Ser Thr Ala Ile Phe Lys Ser Leu Ile Ser Leu
        115                 120                 125

Lys Thr Arg Asn Gly Ile Ile Phe Ser Pro His Pro Arg Ala Lys Asn
    130                 135                 140

Ser Thr Asn Ala Ala Ala Lys Leu Val Leu Asp Ala Ala Ile Ala Ala
145                 150                 155                 160

Gly Ala Pro Lys Asp Ile Ile Gly Trp Ile Asp Gln Pro Ser Val Glu
                165                 170                 175

Leu Ser Asn Ala Leu Met Lys His Asp Gly Ile Ala Leu Ile Leu Ala

            180                 185                 190

Thr Gly Gly Pro Gly Met Val Lys Ala Ala Tyr Ser Ser Gly Lys Pro
        195                 200                 205

Ala Ile Gly Val Gly Ala Gly Asn Val Pro Val Val Ile Asp Glu Thr
    210                 215                 220

Ala Asp Ile Lys Arg Ala Val Ala Ser Ile Leu Met Ser Lys Thr Phe
225                 230                 235                 240

Asp Asn Gly Val Val Cys Ala Ser Glu Gln Ala Ala Ile Val Val Ser
                245                 250                 255

Glu Val Tyr Asp Glu Val Lys Glu Arg Phe Ala Thr His Lys Ala His
            260                 265                 270

Val Leu Ser Lys Ala Asp Ala Asp Lys Val Arg Lys Val Leu Leu Ile
        275                 280                 285

Asp Gly Ala Leu Asn Ala Lys Ile Val Gly Gln Pro Ala Ala Ala Ile
    290                 295                 300

Ala Glu Met Ala Gly Val Lys Val Pro Ala Asp Thr Lys Val Leu Val

305                 310                 315                 320

Gly Glu Gly Leu Gly Lys Val Ser Tyr Asp Asp Glu Phe Ala His Glu
                325                 330                 335

Lys Leu Ser Pro Thr Leu Gly Leu Phe Arg Ala Asp Asn Phe Glu Asp
            340                 345                 350

Ala Val Ala Gln Ala Val Thr Met Val Glu Ile Gly Gly Ile Gly His
        355                 360                 365

Thr Ser Gly Leu Tyr Thr Asn Gln Asp Val Asn Ala Asp Arg Ile Arg
    370                 375                 380

Tyr Phe Gly Asp Lys Leu Lys Thr Ala Arg Ile Leu Val Asn Ile Pro
385                 390                 395                 400

Thr Thr His Gly Gly Ile Gly Asp Leu Tyr Asn Phe Asn Val Ala Pro
                405                 410                 415

Ser Leu Thr Leu Gly Cys Gly Ser Trp Gly Gly Asn Ser Ile Ser Glu
            420                 425                 430

Asn Val Gly Pro Lys His Leu Ile Asn Lys Lys Thr Val Ala Lys Arg
        435                 440                 445

Ala Glu Asn Met Leu Trp His Lys Leu Pro Lys Ser Ile Tyr Phe Arg
    450                 455                 460

Arg Gly Ser Leu Pro Ile Ala Leu Ser Asp Leu Glu Gly Lys Lys Arg
465                 470                 475                 480

Ala Phe Leu Val Thr Asp Arg Phe Leu Phe Asn Asn Gly Tyr Ala Asp
                485                 490                 495

Asp Val Val Ala Leu Leu Lys Ala Gln Gly Met Glu Val Gln Thr Phe

            500                 505                 510

Phe Glu Val Glu Ala Asp Pro Thr Leu Ser Val Val Glu Lys Gly Ala
        515                 520                 525

Ala Ala Met Gln Ser Phe Gln Pro Asp Val Ile Leu Ala Leu Gly Gly
    530                 535                 540

Gly Ser Pro Met Asp Ala Ala Lys Ile Met Trp Val Met Tyr Glu His
545                 550                 555                 560

Pro Asp Thr His Phe Glu Glu Leu Ala Met Arg Phe Met Asp Ile Arg
                565                 570                 575

Lys Arg Ile Tyr Lys Phe Pro Lys Met Gly Lys Lys Ala Glu Leu Val
            580                 585                 590

Cys Ile Thr Thr Thr Ser Gly Thr Gly Ser Glu Val Thr Pro Phe Ala
        595                 600                 605

Val Val Thr Asp Asp Lys Thr Gly Ala Lys Tyr Pro Leu Ala Asp Tyr
    610                 615                 620

Glu Leu Thr Pro Gln Met Ala Ile Val Asp Ala Asn Leu Val Met Asn
625                 630                 635                 640

Met Pro Lys Ser Leu Thr Ala Phe Gly Gly Tyr Asp Ala Val Thr His
                645                 650                 655

Ala Leu Glu Ala Tyr Val Ser Val Leu Ala Asn Glu Tyr Ser Asp Gly
            660                 665                 670

Gln Ala Leu Gln Ala Leu Lys Met Leu Lys Glu Tyr Leu Pro Ser Ser
        675                 680                 685

Tyr Ala Asn Gly Ala Lys Asp Pro Ile Ala Arg Glu Lys Val His Asn
    690                 695                 700

Ala Ala Thr Ile Ala Gly Ile Ala Phe Ala Asn Ala Phe Leu Gly Val
705                 710                 715                 720

Cys His Ser Met Ala His Lys Ile Gly Ala Glu Phe His Leu Pro His
                725                 730                 735

Gly Leu Ala Asn Ala Leu Leu Ile Ala Asn Val Val Arg Tyr Asn Ala
            740                 745                 750

Asn Asp Asn Pro Thr Lys Gln Thr Ala Phe Ser Gln Tyr Asp Arg Pro
        755                 760                 765

Gln Ala Arg Arg Arg Tyr Ala Glu Val Ala Asp His Leu Gly Leu Ser
    770                 775                 780

Gln Pro Gly Asp Arg Thr Ala Gln Lys Ile Glu Arg Leu Leu Thr Trp
785                 790                 795                 800

Leu Asp Glu Leu Lys Val Asn Leu Asp Ile Pro Lys Ser Ile Gln Ala
                805                 810                 815

Ala Gly Val Ala Glu Ala Asp Phe Leu Ala Lys Val Asp Glu Leu Ala
            820                 825                 830

Val Glu Ala Phe Asp Asp Gln Cys Thr Gly Ala Asn Pro Arg Tyr Pro
        835                 840                 845

Leu Ile Ala Glu Leu Lys Glu Val Leu Leu Ala Ser Tyr Tyr Gly Lys
    850                 855                 860

Pro Phe Val Glu Gly Gln Thr Phe Glu Gly Thr Thr Val Ile Val Lys
865                 870                 875                 880

Lys Ala Asp Gln Glu Ala Ala Lys Ala Pro Lys Ala Lys Lys
                885                 890

<210> 47
<211> 200
<212> PRT
<213> Vibrio cholerae

<220>
<221> PEPTIDE
<222> (1)..(200)
<223> >sp|Q9KNY5|RL4_VIBCH 50S ribosomal protein L4 -
      Vibrio cholerae. (200 aa)

<400> 47
Met Glu Leu Met Val Lys Gly Ala Asn Ala Leu Thr Val Ser Glu Thr
  1               5                  10                  15

Thr Phe Gly Arg Glu Phe Asn Glu Ala Leu Val His Gln Val Val Val
             20                  25                  30

Ala Tyr Ala Ala Gly Ala Arg Gln Gly Thr Arg Ala Gln Lys Thr Arg
        35                  40                  45

Ser Glu Val Ser Gly Gly Gly Ala Lys Pro Trp Arg Gln Lys Gly Thr
    50                  55                  60

Gly Arg Ala Arg Ala Gly Thr Ile Arg Ser Pro Ile Trp Arg Thr Gly
 65                  70                  75                  80

Gly Val Thr Phe Ala Ala Lys Pro Gln Asp His Ser Gln Lys Val Asn
                 85                  90                  95

Lys Lys Met Tyr Arg Gly Ala Met Lys Ser Ile Leu Ser Glu Leu Val
            100                 105                 110

Arg Gln Glu Arg Leu Ile Val Val Glu Asn Phe Ser Val Glu Ala Pro
        115                 120                 125

Lys Thr Lys Ala Leu Val Ala Lys Leu Lys Glu Leu Glu Leu Asn Asp
    130                 135                 140

Val Leu Ile Val Thr Gly Glu Val Asp Glu Asn Leu Phe Leu Ala Ala
145                 150                 155                 160

Arg Asn Leu Tyr Lys Val Asp Val Arg Asp Val Thr Gly Ile Asp Pro
                165                 170                 175

Val Ser Leu Ile Ala Phe Asp Lys Val Leu Met Thr Ala Ala Ala Val
            180                 185                 190

Lys Gln Val Glu Glu Met Leu Ala
        195                 200

<210> 48
<211> 429
<212> PRT
<213> Vibrio cholerae

<220>
<221> PEPTIDE
<222> (1)..(429)
<223> >sp|Q9KV81|PUR2_VIBCH Phosphoribosylamine--glycine
      ligase (EC 6.3.4.13) (GARS) (Glycinamide
      ribonucleotide synthetase)
      (Phosphoribosylglycinamide synthetase) - Vibrio
      cholerae. (429 aa)

<400> 48
Met Gln Val Leu Ile Ile Gly Ser Gly Gly Arg Glu His Ala Leu Ala
  1               5                  10                  15

Trp Lys Val Ala Gln Asn Pro Gln Val Asp Thr Ile Tyr Val Ala Pro
             20                  25                  30

Gly Asn Ala Gly Thr Ala Leu Glu His Lys Val Gln Asn Val Asn Ile
         35                  40                  45

Gly Ile Thr Asp Ile Pro Ala Leu Val Ala Phe Ala Gln Asp Lys Ala
     50                  55                  60

Ile Glu Leu Thr Ile Val Gly Pro Glu Ala Pro Leu Val Ile Gly Val
 65                  70                  75                  80

Val Asp Ala Phe Arg Ala Ala Gly Leu Pro Ile Phe Gly Pro Thr Gln
                 85                  90                  95

Gly Ala Ala Gln Leu Glu Gly Ser Lys Ala Phe Thr Lys Asp Phe Leu
            100                 105                 110

Ala Arg His Asn Ile Pro Thr Ala Ala Tyr Ala Asn Phe Thr Glu Ile
        115                 120                 125

Glu Pro Ala Leu Ala Tyr Val Arg Glu Lys Gly Ala Pro Ile Val Val
    130                 135                 140

Lys Ala Asp Gly Leu Ala Ala Gly Lys Gly Val Ile Val Ala Met Thr
145                 150                 155                 160

Leu Gln Glu Ala Glu Asp Ala Ile Gln Asp Met Leu Ala Gly Asn Ala
                165                 170                 175

Phe Gly Ser Ala Gly Ser Arg Val Val Val Glu Glu Phe Leu Asp Gly
            180                 185                 190

Glu Glu Ala Ser Phe Ile Val Met Val Asp Gly Glu Asn Val Leu Pro
        195                 200                 205

Met Ala Thr Ser Gln Asp His Lys Arg Val Gly Asp Ala Asp Thr Gly
    210                 215                 220

Pro Asn Thr Gly Gly Met Gly Ala Tyr Ser Pro Ala Pro Val Val Thr
225                 230                 235                 240

Gln Asp Val His Asp Arg Val Met Arg Glu Val Ile Asp Pro Thr Val
                245                 250                 255

Arg Gly Met Ala Ala Glu Gly Asn Thr Tyr Thr Gly Phe Leu Tyr Ala
            260                 265                 270

Gly Leu Met Ile Asp Ser Thr Gly Ala Pro Lys Val Ile Glu Tyr Asn
        275                 280                 285

Cys Arg Phe Gly Asp Pro Glu Thr Gln Pro Ile Met Met Arg Leu Gln
    290                 295                 300

Ser Asp Leu Val Glu Leu Cys Gln Ala Ala Ile Ala Gly Lys Leu Asp
305                 310                 315                 320

Gln Val Glu Ser Lys Trp Asp Pro Arg Ala Ser Ile Gly Val Val Leu
                325                 330                 335

Ala Ala Gly Gly Tyr Pro Gly Asp Tyr Ala Lys Gly Glu Val Ile Ser
            340                 345                 350

Gly Leu Pro Thr Gln Glu Ser Ala Gly Gln Lys Val Phe His Ala Gly
        355                 360                 365

Thr Glu Thr Gln Gly Asp Gln Val Val Thr Asn Gly Gly Arg Val Leu
    370                 375                 380

Cys Ala Thr Ala Leu Gly Asn Thr Val Leu Glu Ala Gln Gln Arg Ala
385                 390                 395                 400

Tyr Gln Leu Ala Asp Gln Ile His Trp Asn Gly Met Phe Cys Arg Arg
                405                 410                 415

Asp Ile Gly Tyr Arg Ala Ile Ala Arg Glu Gln Ala Lys
            420                 425


<210> 49
<211> 645
<212> PRT
<213> Vibrio cholerae

<220>
<221> PEPTIDE
<222> (1)..(645)
<223> >sp|Q9KVS8|THIC_VIBCH Thiamine biosynthesis
      protein thiC - Vibrio cholerae. (645 aa)

<400> 49
Met Ser Asn Arg Lys Gln Ala Arg Leu Glu Ala Lys Arg Phe Ile Asp
1               5                   10                  15

Thr Leu Ser Val Glu Pro Tyr Pro Asn Ser Gln Lys Ser Tyr Leu Leu
            20                  25                  30

Gly Ser Arg Pro Asp Ile Arg Val Pro Val Arg Glu Ile Thr Leu Ser
        35                  40                  45

Asp Thr Leu Val Gly Gly Ser Lys Asp Ala Pro Ile Phe Glu Pro Asn
    50                  55                  60

Glu Pro Ile Cys Val Tyr Asp Thr Ser Gly Val Tyr Thr Asp Pro Ser
65                  70                  75                  80

His Asp Ile Asp Leu Tyr Lys Gly Leu Pro Lys Leu Arg Glu Glu Trp
                85                  90                  95

Ile Glu Glu Arg Arg Asp Thr His Ile Leu Pro Ser Met Ser Ser His
            100                 105                 110

Phe Ala Arg Glu Arg Leu Ala Asp Glu Thr Leu Asp Glu Leu Arg Tyr
        115                 120                 125

Gly His Leu Pro Arg Ile Arg Arg Ala Met Gly Gln His Arg Val Thr
    130                 135                 140

Gln Leu His Tyr Ala Arg Gln Gly Ile Ile Thr Pro Glu Met Glu Phe
145                 150                 155                 160

Val Ala Ile Arg Glu Asn Ser Arg Arg Leu Ala His Gln Asp Pro Ser
                165                 170                 175

Leu Leu Gln Gln His Ala Gly Gln Asn Phe Gly Ala His Leu Pro Asp
            180                 185                 190

Leu Ile Thr Pro Glu Phe Val Arg Arg Glu Ile Ala Glu Gly Arg Ala
        195                 200                 205

Ile Ile Pro Cys Asn Ile Asn His Pro Glu Ser Glu Pro Met Ile Ile
    210                 215                 220

Gly Arg Asn Phe Leu Val Lys Val Asn Ala Asn Ile Gly Asn Ser Ser
225                 230                 235                 240

Val Ser Ser Ser Ile Glu Glu Glu Val Glu Lys Leu Val Trp Ala Thr
                245                 250                 255

Arg Trp Gly Ala Asp Thr Val Met Asp Leu Ser Thr Gly Arg Asn Ile
            260                 265                 270

His Glu Thr Arg Glu Trp Ile Leu Arg Asn Ser Pro Val Pro Ile Gly
        275                 280                 285

Thr Val Pro Met Tyr Gln Ala Leu Glu Lys Val Asn Gly Val Ala Glu
    290                 295                 300

Asn Leu Thr Trp Glu Val Met Arg Asp Thr Leu Leu Glu Gln Ala Glu
305                 310                 315                 320

Gln Gly Val Asp Tyr Phe Thr Ile His Ala Gly Leu Leu Leu Arg Tyr
                325                 330                 335

Val Pro Met Thr Ala Lys Arg Val Thr Gly Ile Val Ser Arg Gly Gly

            340                 345                 350

Ser Ile Ile Ala Lys Trp Cys Leu Ser His His Gln Glu Asn Phe Leu
        355                 360                 365

Tyr Thr His Phe Arg Glu Ile Cys Glu Ile Cys Ala Gln Tyr Asp Val
    370                 375                 380

Ala Leu Ser Leu Gly Asp Gly Leu Arg Pro Gly Ser Ile Ala Asp Ala
385                 390                 395                 400

Asn Asp Glu Ala Gln Phe Ala Glu Leu Arg Thr Leu Gly Glu Leu Thr
                405                 410                 415

Gln Ile Ala Trp Glu Tyr Asp Val Gln Val Met Ile Glu Gly Pro Gly
            420                 425                 430

His Val Pro Met His Leu Ile Lys Ala Asn Met Asp Glu Gln Leu Lys
    435                 440                 445

His Cys His Glu Ala Pro Phe Tyr Thr Leu Gly Pro Leu Thr Thr Asp
    450                 455                 460

Ile Ala Pro Gly Tyr Asp His Ile Thr Ser Gly Ile Gly Ala Ala Met
465                 470                 475                 480

Ile Gly Trp Phe Gly Cys Ala Met Leu Cys Tyr Val Thr Pro Lys Glu
                485                 490                 495

His Leu Gly Leu Pro Asn Lys Glu Asp Val Lys Thr Gly Leu Ile Thr
            500                 505                 510

Tyr Lys Leu Ala Ala His Ala Ala Asp Leu Ala Lys Gly His Pro Gly
    515                 520                 525

Ala Gln Ile Arg Asp Asn Ala Leu Ser Lys Ala Arg Phe Glu Phe Arg
    530                 535                 540

Trp Glu Asp Gln Phe Asn Leu Ala Leu Asp Pro Val Thr Ala Arg Ala
545                 550                 555                 560

Phe His Asp Glu Thr Leu Pro Gln Glu Ser Gly Lys Val Ala His Phe
                565                 570                 575

Cys Ser Met Cys Gly Pro Lys Phe Cys Ser Met Lys Ile Ser Gln Glu
            580                 585                 590

Val Arg Asp Tyr Ala Asn Asn Gln Thr Leu Asp Thr Thr Val Ile Asp
    595                 600                 605

Leu Val Met Pro Ala Glu Ser Ile Gln Leu Ala Met Gln Asp Lys Ser
    610                 615                 620

Arg Glu Phe Leu Ala Ser Gly Ala Glu Leu Tyr His Pro Leu Val Lys
625                 630                 635                 640

Glu Pro Ile Glu Glu
                645

<210> 50
<211> 156
<212> PRT
<213> Vibrio cholerae

<220>
<221> PEPTIDE
<222> (1)..(156)
<223> >sp|Q9KUZ8|RS7_VIBCH 30S ribosomal protein S7 -
      Vibrio cholerae. (156 aa)

<400> 50
Met Pro Arg Arg Arg Val Ile Gly Gln Arg Lys Ile Leu Pro Asp Pro
1               5                   10                  15

Lys Phe Lys Ser Glu Leu Leu Ala Lys Phe Val Asn Ile Leu Met Val
            20                  25                  30

Asp Gly Lys Lys Ser Thr Ala Glu Lys Ile Val Tyr Thr Ala Leu Asp
        35                  40                  45

Thr Met Ala Glu Lys Ser Gly Lys Asp His Leu Ala Val Phe Glu Glu
    50                  55                  60

Ala Leu Glu Asn Val Arg Pro Ala Val Glu Val Lys Ser Arg Arg Val
65                  70                  75                  80

Gly Gly Ser Thr Tyr Gln Val Pro Val Glu Val Arg Pro Val Arg Arg
                85                  90                  95

Asn Ala Leu Ala Met Arg Trp Leu Val Glu Ala Ala Arg Lys Arg Gly
            100                 105                 110

Glu Lys Ser Met Ala Ala Arg Leu Ala Ala Glu Met Leu Asp Ala Ala
        115                 120                 125

Glu Asn Lys Gly Ser Ala Val Lys Lys Arg Glu Asp Val His Arg Met
    130                 135                 140

Ala Glu Ala Asn Lys Ala Phe Ala His Tyr Arg Trp
145                 150                 155


<210> 51
<211> 103
<212> PRT
<213> Vibrio cholerae

<220>
<221> PEPTIDE
<222> (1)..(103)
<223> >sp|Q9KNY3|RS10_VIBCH 30S ribosomal protein S10 -
      Vibrio cholerae. (103 aa)

<400> 51
Met Gln Asn Gln Arg Ile Arg Ile Arg Leu Lys Ala Phe Asp Tyr Lys
1               5                   10                  15

Leu Ile Asp Ala Ser Thr Ala Glu Ile Val Glu Thr Ala Lys Arg Thr
            20                  25                  30

Gly Ala Gln Val Arg Gly Pro Ile Pro Leu Pro Thr Arg Lys Glu Arg
        35                  40                  45

Phe Thr Val Leu Ile Ser Pro His Val Asn Lys Asp Ala Arg Asp Gln
    50                  55                  60

Tyr Glu Ile Arg Thr His Lys Arg Leu Ile Asp Ile Val Glu Pro Thr
65                  70                  75                  80

Asp Lys Thr Val Asp Ala Leu Met Arg Leu Asp Leu Ala Ala Gly Val
                85                  90                  95

Asp Val Gln Ile Ser Leu Gly
            100

<210> 52
<211> 288
<212> PRT
<213> Vibrio cholerae

<220>
<221> PEPTIDE
<222> (1)..(288)
<223> >tr|Q9KNH4|Q9KNH4_VIBCH ATP synthase F1, gamma
      subunit - Vibrio cholerae. (288 aa)

<400> 52
Met Ala Gly Ala Lys Glu Ile Arg Thr Lys Ile Gly Ser Val Lys Ser
1               5                   10                  15

Thr Gln Lys Ile Thr Lys Ala Met Glu Met Val Ala Ala Ser Lys Met
            20                  25                  30

Arg Arg Ser Gln Asp Ala Met Glu Ser Ser Arg Pro Tyr Ala Gln Thr
        35                  40                  45

Ile Arg Lys Val Ile Gly His Val Ala Asn Ala Ser Leu Glu Tyr Arg
    50                  55                  60

His Pro Tyr Leu Glu Glu Arg Glu Ala Lys Arg Val Gly Tyr Ile Ile
65                  70                  75                  80

Ile Ser Thr Asp Arg Gly Leu Cys Gly Gly Leu Asn Ile Asn Leu Phe
                85                  90                  95

Lys Lys Ala Ile Thr Asp Met Gln Thr Trp Lys Glu Lys Gly Ala Gln
            100                 105                 110

Ile Glu Leu Ala Ile Ile Gly Ser Lys Ala Thr Ala Phe Phe Asn Asn
        115                 120                 125

Ser Gly Ala Lys Val Ala Ala Gln Val Ser Gly Leu Gly Asp Ser Pro
    130                 135                 140

Ser Leu Glu Asp Leu Ile Gly Ser Val Gly Val Met Leu Lys Lys Tyr
145                 150                 155                 160

Asp Lys Gly Glu Leu Asp Arg Leu Tyr Leu Val Phe Asn Gln Phe Val
                165                 170                 175

Asn Thr Met Val Gln Lys Pro Lys Ile Asp Gln Leu Leu Pro Leu Pro
            180                 185                 190

Lys Ser Asp Ser Glu Asp Met Gln Arg Asp His Met Trp Asp Tyr Ile
        195                 200                 205

Tyr Glu Pro Glu Pro Lys Pro Leu Leu Asp Ala Leu Leu Leu Arg Phe
    210                 215                 220

Ile Glu Ser Gln Val Tyr Gln Gly Val Val Glu Asn Leu Ala Cys Glu
225                 230                 235                 240

Gln Ala Ala Arg Met Val Ala Met Lys Ala Ala Thr Asp Asn Ala Ser
                245                 250                 255

Asn Leu Ile Asp Asp Leu Gln Leu Val Tyr Asn Lys Ala Arg Gln Ala
            260                 265                 270

Ala Ile Thr Gln Glu Leu Ser Glu Ile Val Gly Gly Ala Ala Ala Val
        275                 280                 285

<210> 53
<211> 343
<212> PRT
<213> Vibrio cholerae

<220>
<221> PEPTIDE
<222> (1)..(343)
<223> >tr|Q9KPN7|Q9KPN7_VIBCH
      2,3,4,5-tetrahydropyridine-2-carboxylate
      N-succinyltransferase - Vibrio cholerae. (343 aa)

<400> 53
Met Ala Tyr Phe Ala Leu Gly Leu Ala Thr Ala Thr Lys Asn Arg Asp
  1               5                  10                  15

Gly Lys Ile Ile Glu Ala Phe Phe Pro Thr Pro Ile Leu Ala Pro Ser
             20                  25                  30

Asp Ala Leu Val Ala Ala Leu Ala Pro Ile Ala Gly Tyr Gln Glu Gly
         35                  40                  45

Asn Gln Ala Leu Asp Ile Thr Ala Ala Gln Ser Ala Gln Leu Ala Ala
     50                  55                  60

Val Phe Ala Ala His Gln Gln Ala Ala Ser Ala Ala Phe Ala Asp Lys
 65                  70                  75                  80

Ala Ala Asn Ala Lys Gln Pro Leu Val Leu Val Ile Leu Ala Ser Asp
                 85                  90                  95

Asp Lys Pro Gln Ser Val Ala Glu Gly Tyr Leu Lys Leu Gln Leu Ile
            100                 105                 110

Ser His Arg Leu Val Lys Pro His Gly Thr Val Leu Asp Gly Ile Phe
        115                 120                 125

Gly Leu Leu His Asn Ile Ala Trp Thr Asn Glu Gly Pro Ile Asp Leu
    130                 135                 140

Pro Glu Leu Ala Glu Arg Gln Ile Glu Ala Arg Leu Ala Gly Arg Val

145                 150                 155                 160

Leu Thr Val Asp Cys Val Asp Lys Phe Pro Lys Met Val Asp Tyr Val
                165                 170                 175

Val Pro Ala Gly Ile Arg Ile Ala Asp Thr Ser Arg Val Arg Leu Gly
            180                 185                 190

Ala His Val Gly Glu Gly Thr Thr Val Met His Glu Gly Phe Ile Asn
        195                 200                 205

Phe Asn Ala Gly Thr Thr Gly Val Ser Met Val Glu Gly Arg Ile Ser
    210                 215                 220

Ala Gly Val Val Val Gly Asn Gly Ser Asp Ile Gly Gly Gly Ala Ser
225                 230                 235                 240

Ile Met Gly Thr Leu Ser Gly Gly Gly Lys Val Val Val Ser Ile Gly
                245                 250                 255

Glu Asn Ser Leu Leu Gly Ala Asn Ala Gly Leu Gly Phe Pro Leu Gly
            260                 265                 270

Asp Arg Cys Thr Val Glu Ser Gly Leu Tyr Val Thr Ala Gly Thr Lys
        275                 280                 285

Val Arg Thr Leu Asp Lys Asp Gly Asn Gln Val Asp Ile Val Lys Ala
    290                 295                 300

Arg Asp Leu Ala Gly Val Ser Asp Leu Leu Phe Arg Arg Asn Ser Leu
305                 310                 315                 320

Thr Gly Gln Ile Glu Cys Leu Ala Asn Lys Ser Ala Val Glu Leu Asn
                325                 330                 335

Ser Glu Leu His Lys Asn Asn
            340


<210> 54
<211> 466
<212> PRT
<213> Vibrio cholerae

<220>
<221> PEPTIDE
<222> (1)..(466)
<223> >sp|Q9KSF9|SYN_VIBCH Asparaginyl-tRNA synthetase
      (EC 6.1.1.22) (Asparagine--tRNA ligase) (AsnRS) -
      Vibrio cholerae. (466 aa)

<400> 54
Met Thr Tyr Ala Pro Val Asn Asp Val Leu Ser Gly Lys Leu Ala Val
1               5                   10                  15

Asp Ser Glu Val Thr Val Arg Gly Trp Ile Arg Thr Arg Arg Asp Ser
            20                  25                  30

Lys Ala Gly Ile Ser Phe Leu Ala Ile Tyr Asp Gly Ser Cys Phe Asn
        35                  40                  45

Pro Ile Gln Ala Val Val Pro Asn Asn Leu Asn Asn Tyr Asp Asn Glu
    50                  55                  60
Val Leu Lys Leu Thr Thr Gly Cys Ser Val Glu Val Thr Gly Lys Ile
65                  70                  75                  80
Val Glu Ser Pro Ala Ser Gly Gln Ala Phe Glu Leu Ala Ala Ser Asp
                85                  90                  95
Val Lys Val Val Gly Trp Val Glu Asp Ala Asp Thr Tyr Pro Met Ala
            100                 105                 110
Lys Thr Arg His Ser Ile Glu Tyr Leu Arg Glu Val Ala His Leu Arg
        115                 120                 125
Pro Arg Thr Asn Val Ile Gly Ala Val Ala Arg Val Arg Asn Cys Leu
    130                 135                 140
Ala Gln Ala Ile His Arg Phe Tyr His Glu Gln Gly Tyr Phe Trp Val
145                 150                 155                 160
Ser Ala Pro Leu Ile Thr Ala Ser Asp Ala Glu Gly Ala Gly Glu Met
                165                 170                 175
Phe Arg Val Ser Thr Leu Asp Met Glu Asn Leu Pro Arg Thr Asp Ala
            180                 185                 190
Gly Lys Val Asp Tyr Asn Gln Asp Phe Phe Gly Lys Glu Thr Phe Leu
        195                 200                 205
Thr Val Ser Gly Gln Leu Asn Ala Glu Ala Tyr Ala Cys Ala Ile Ser
    210                 215                 220
Lys Val Tyr Thr Phe Gly Pro Thr Phe Arg Ala Glu Asn Ser Asn Thr
225                 230                 235                 240
Ser Arg His Leu Ala Glu Phe Trp Met Val Glu Pro Glu Val Ala Phe
                245                 250                 255
Ala Asp Leu Asn Thr Val Ala Lys Leu Ala Glu Asp Met Leu Lys Tyr
            260                 265                 270
Val Phe Lys Ala Val Leu Ala Glu Arg Arg Asp Asp Leu Glu Phe Phe
        275                 280                 285
Asn Asp Arg Ile Asn Asn Glu Val Ile Ala Arg Leu Glu Gln Phe Val
    290                 295                 300
Glu Ser Asp Phe Ala Gln Val Asp Tyr Thr Asp Ala Ile Glu Ile Leu
305                 310                 315                 320
Lys Asn Cys Gly Lys Thr Phe Glu Phe Pro Val Glu Trp Gly Ile Asp
                325                 330                 335
Leu Ala Ser Glu His Glu Arg Phe Leu Ala Glu Glu His Phe Lys Ala
            340                 345                 350
Pro Val Ile Val Lys Asn Tyr Pro Lys Asp Ile Lys Ala Phe Tyr Met
        355                 360                 365

Arg Met Asn Glu Asp Gly Lys Thr Val Ala Ala Met Asp Val Leu Ala
    370                 375                 380

Pro Gly Ile Gly Glu Ile Ile Gly Gly Ser Gln Arg Glu Glu Arg Leu
385                 390                 395                 400

Asp Ile Leu Asp Ala Arg Met Arg Glu Phe Gly Ile Asp Pro Glu His
                405                 410                 415

Met Asp Trp Tyr Arg Asp Leu Arg Arg Tyr Gly Thr Val Pro His Ala
            420                 425                 430

Gly Phe Gly Leu Gly Phe Glu Arg Leu Val Ser Tyr Val Thr Gly Met
        435                 440                 445

Gly Asn Val Arg Asp Val Ile Pro Phe Pro Arg Thr Pro Arg Ser Ala
    450                 455                 460

Ser Phe
465

<210> 55
<211> 90
<212> PRT
<213> Vibrio cholerae

<220>
<221> PEPTIDE
<222> (1)..(90)
<223> >sp|Q9KNM3|RPOZ_VIBCH DNA-directed RNA polymerase
      omega chain (EC 2.7.7.6) (RNAP omega subunit)
      (Transcriptase omega chain) (RNA polymerase omega
      subunit) - Vibrio cholerae. (90 aa)

<400> 55
Met Ala Arg Val Thr Val Gln Asp Ala Val Glu Lys Ile Gly Asn Arg
  1               5                  10                  15

Phe Asp Leu Val Leu Val Ala Ala Arg Arg Ala Arg Gln Met Gln Ser
             20                  25                  30

Gly Gly Lys Asp Ala Leu Val Pro Glu Glu Asn Asp Lys Pro Thr Val
         35                  40                  45

Ile Ala Leu Arg Glu Ile Glu Glu Gly Leu Ile Thr Lys Asp Val Leu
     50                  55                  60

Asp Ala Arg Glu Arg Gln Glu Gln Gln Glu Gln Glu Ala Ala Glu Leu
 65                  70                  75                  80

Ala Ala Val Ser Ser Ile Met His Asn Arg
                 85                  90

<210> 56
<211> 544
<212> PRT
<213> Vibrio cholerae

<220>

<221> PEPTIDE
<222> (1)..(544)
<223> >sp|Q9KNR7|CH601_VIBCH 60 kDa chaperonin 1
      (Protein Cpn60 1) (groEL protein 1) - Vibrio
      cholerae. (544 aa)

<400> 56
Met Ala Ala Lys Asp Val Arg Phe Gly Asn Asp Ala Arg Val Lys Met
1               5                   10                  15

Leu Glu Gly Val Asn Ile Leu Ala Asp Ala Val Lys Val Thr Leu Gly
            20                  25                  30

Pro Lys Gly Arg Asn Val Val Leu Asp Lys Ser Phe Gly Ala Pro Thr
        35                  40                  45

Ile Thr Lys Asp Gly Val Ser Val Ala Arg Glu Ile Glu Leu Glu Asp
    50                  55                  60

Lys Phe Gln Asn Met Gly Ala Gln Met Val Lys Glu Val Ala Ser Gln
65                  70                  75                  80

Ala Asn Asp Ala Ala Gly Asp Gly Thr Thr Thr Ala Thr Val Leu Ala
                85                  90                  95

Gln Ala Ile Val Asn Glu Gly Leu Lys Ala Val Ala Ala Gly Met Asn
            100                 105                 110

Pro Met Asp Leu Lys Arg Gly Ile Asp Lys Ala Val Ile Ala Ala Val
        115                 120                 125

Glu Glu Leu Lys Ala Leu Ser Val Pro Cys Ala Asp Thr Lys Ala Ile
    130                 135                 140

Ala Gln Val Gly Thr Ile Ser Ala Asn Ser Asp Ser Ser Val Gly Asn
145                 150                 155                 160

Ile Ile Ala Glu Ala Met Glu Lys Val Gly Arg Asp Gly Val Ile Thr
                165                 170                 175

Val Glu Glu Gly Gln Ala Leu Gln Asp Glu Leu Asp Val Val Glu Gly
            180                 185                 190

Met Gln Phe Asp Arg Gly Tyr Leu Ser Pro Tyr Phe Ile Asn Asn Gln
        195                 200                 205

Glu Ser Gly Ser Val Glu Leu Asp Asn Pro Phe Ile Leu Leu Val Asp
    210                 215                 220

Lys Lys Ile Ser Asn Ile Arg Glu Leu Leu Pro Val Leu Glu Gly Val
225                 230                 235                 240

Ala Lys Ala Ser Arg Pro Leu Leu Ile Val Ala Glu Asp Val Glu Gly

                245                 250                 255

Glu Ala Leu Ala Thr Leu Val Val Asn Asn Met Arg Gly Ile Val Lys
            260                 265                 270

Val Ala Ala Val Lys Ala Pro Gly Phe Gly Asp Arg Arg Lys Ala Met
        275                 280                 285

Leu Gln Asp Ile Ala Ile Leu Thr Gly Gly Val Val Ile Ser Glu Glu
    290                 295                 300

Ile Gly Leu Glu Leu Glu Lys Ala Thr Leu Glu Asp Leu Gly Gln Ala
305                 310                 315                 320

Lys Arg Val Ser Ile Thr Lys Glu Asn Ser Thr Ile Ile Asp Gly Ala
                325                 330                 335

Gly Asp Gln Ala Ala Ile Gln Gly Arg Val Ala Gln Ile Arg Gln Gln
            340                 345                 350

Ile Glu Glu Ala Thr Ser Asp Tyr Asp Lys Glu Lys Leu Gln Glu Arg
        355                 360                 365

Val Ala Lys Leu Ala Gly Gly Val Ala Val Ile Lys Val Gly Ala Ala
    370                 375                 380

Thr Glu Val Glu Met Lys Glu Lys Lys Asp Arg Val Glu Asp Ala Leu
385                 390                 395                 400

His Ala Thr Arg Ala Ala Val Glu Glu Gly Val Val Ala Gly Gly Gly
                405                 410                 415

Val Ala Leu Ile Arg Ala Ala Ser Lys Leu Ser Ser Leu Val Gly Asp
            420                 425                 430

Asn Glu Glu Gln Asn Val Gly Ile Arg Val Ala Leu Arg Ala Met Glu
        435                 440                 445

Ala Pro Leu Arg Gln Ile Val Lys Asn Ala Gly Asp Glu Glu Ser Val
    450                 455                 460

Val Ala Asn Asn Val Arg Ala Gly Glu Gly Asn Tyr Gly Tyr Asn Ala
465                 470                 475                 480

Ala Thr Gly Val Tyr Gly Asp Met Ile Glu Met Gly Ile Leu Asp Pro
                485                 490                 495

Thr Lys Val Thr Arg Ser Ala Leu Gln Phe Ala Ala Ser Val Ala Gly
            500                 505                 510

Leu Met Ile Thr Thr Glu Ala Met Ile Thr Glu Leu Pro Lys Lys Asp
        515                 520                 525

Ala Pro Ala Met Pro Asp Met Gly Met Gly Gly Met Gly Gly Met Met
    530                 535                 540

<210> 57
<211> 130
<212> PRT
<213> Vibrio cholerae

<220>
<221> PEPTIDE
<222> (1)..(130)

<223> >sp|Q9KUF0|RS9_VIBCH 30S ribosomal protein S9 -
      Vibrio cholerae. (130 aa)

<400> 57
Met Ala Glu Asn Gln Tyr Tyr Gly Thr Gly Arg Arg Lys Ser Ser Ala
1               5                   10                  15

Ala Arg Val Phe Ile Lys Pro Gly Ser Gly Asn Ile Val Ile Asn Lys
            20                  25                  30

Arg Ser Leu Glu Glu Tyr Phe Gly Arg Pro Thr Ser Cys Met Val Val
        35                  40                  45

Lys Gln Pro Leu Glu Leu Val Asp Met Val Glu Lys Leu Asp Leu Tyr
    50                  55                  60

Ile Thr Val Lys Gly Gly Gly Ile Ser Gly Gln Ala Gly Ala Ile Arg
65                  70                  75                  80

His Gly Ile Thr Arg Ala Leu Met Glu Tyr Asp Glu Ser Leu Arg Pro
                85                  90                  95

Val Leu Arg Ala Ala Gly Tyr Val Thr Arg Asp Ala Arg Arg Val Glu
            100                 105                 110

Arg Lys Lys Val Gly Leu Arg Lys Ala Arg Arg Arg Pro Gln Phe Ser
        115                 120                 125

Lys Arg
    130


<210> 58
<211> 787
<212> PRT
<213> Vibrio cholerae

<220>
<221> PEPTIDE
<222> (1)..(787)
<223> >tr|Q9KQY1|Q9KQY1_VIBCH Formate acetyltransferase
      - Vibrio cholerae. (787 aa)

<400> 58
Met Lys Ser Val Leu Leu Pro Ser Gly Ile Ala Gly Ser Thr Ala Lys
1               5                   10                  15

Thr Val Arg Val Phe Ser Thr His Lys Gly Arg Tyr Val Met Ala Glu
            20                  25                  30

Gln Phe Ala Lys Ala Trp Glu Gly Phe Ala Ala Gly Asp Trp Gln Asn
        35                  40                  45

Glu Val Asn Val Arg Asp Phe Ile Gln Lys Asn Tyr Thr Pro Tyr Glu
    50                  55                  60

Gly Asp Glu Ser Phe Leu Val Ser Glu Gly Thr Glu Ala Thr Asn Lys
65                  70                  75                  80

Leu Trp Ala Lys Val Met Glu Gly Ile Arg Gln Glu Asn Ser Thr His
                85                  90                  95

Ala Pro Val Asp Phe Asp Thr Ser Leu Ile Ser Thr Ile Thr Ala His
            100                 105                 110

Asp Ala Gly Tyr Ile Asn Lys Asp Leu Glu Lys Ile Val Gly Leu Gln
        115                 120                 125

Thr Asp Ala Pro Leu Lys Arg Ala Ile Ile Pro Asn Gly Gly Ile Arg
    130                 135                 140

Met Val Glu Gly Ser Cys Lys Ala Tyr Gly Arg Glu Leu Asp Pro Gln
145                 150                 155                 160

Val Ser Lys Ile Tyr Ser Glu Tyr Arg Lys Thr His Asn Ala Gly Val
                165                 170                 175

Phe Asp Ile Tyr Thr Pro Glu Ile Leu Ala Cys Arg Lys Ser Gly Val
            180                 185                 190

Leu Thr Gly Leu Pro Asp Ala Tyr Gly Arg Gly Arg Ile Ile Gly Asp
        195                 200                 205

Tyr Arg Arg Val Ala Leu Tyr Gly Ile Asp Phe Leu Met Lys Asp Lys
    210                 215                 220

Leu Ala Gln Phe Lys Ser Leu Gln Glu Lys Phe Glu Asn Gly Glu Asp
225                 230                 235                 240

Leu Gln Met Thr Met Gln Leu Arg Glu Glu Ile Ala Glu Gln His Arg
                245                 250                 255

Ala Leu Gly Gln Met Lys Gln Met Ala Ala Lys Tyr Gly Tyr Asp Ile
            260                 265                 270

Ser Arg Pro Ala Glu Thr Ala Gln Glu Ala Ile Gln Trp Thr Tyr Phe
        275                 280                 285

Gly Tyr Leu Ala Ala Val Lys Ser Gln Asn Gly Ala Ala Met Ser Leu
    290                 295                 300

Gly Arg Thr Ser Thr Phe Leu Asp Val Tyr Ile Glu Arg Asp Met Lys
305                 310                 315                 320

Ala Gly Lys Ile Thr Glu Val Glu Ala Gln Glu Met Ile Asp His Phe
                325                 330                 335

Val Met Lys Leu Arg Met Val Arg Phe Leu Arg Thr Pro Glu Tyr Asp
            340                 345                 350

Glu Leu Phe Ser Gly Asp Pro Ile Trp Ala Thr Glu Ser Met Gly Gly
        355                 360                 365

Met Gly Leu Asp Gly Arg Thr Leu Val Thr Arg Thr Asn Phe Arg Phe
    370                 375                 380

Leu Asn Ser Leu Tyr Thr Met Gly Pro Ser Pro Glu Pro Asn Ile Thr
385                 390                 395                 400

Val Leu Trp Ser Glu Gln Leu Pro Glu Gly Phe Lys Lys Phe Cys Ala
                405                 410                 415

Lys Val Ser Ile Asp Thr Ser Ser Ile Gln Tyr Glu Asn Asp Asp Leu
            420                 425                 430

Met Arg Pro Asp Phe Asn Asn Asp Asp Tyr Ala Ile Ala Cys Cys Val
        435                 440                 445

Ser Pro Met Val Ile Gly Lys His Met Gln Phe Phe Gly Ala Arg Ala
    450                 455                 460

Asn Leu Ala Lys Thr Leu Leu Tyr Val Ile Asn Gly Gly Val Asp Glu
465                 470                 475                 480

Lys Leu Lys Ile Gln Val Gly Pro Lys Met Pro Lys Ile Thr Asp Glu
                485                 490                 495

Val Leu Asp Phe Asp Asp Val Trp Gly Lys Leu Asp His Phe Met Gly
            500                 505                 510

Trp Leu Ala Thr Gln Tyr Val Thr Ala Leu Asn Ala Ile His Tyr Met
        515                 520                 525

His Asp Lys Tyr Ser Tyr Glu Ala Ala Leu Met Ala Leu His Asp Arg
    530                 535                 540

Asp Val Arg Arg Thr Met Ala Cys Gly Ile Ala Gly Leu Ser Val Ala
545                 550                 555                 560

Ala Asp Ser Leu Ser Ala Ile Lys Tyr Ala Lys Val Lys Pro Ile Arg
                565                 570                 575

Asp Glu Asp Gly Val Ala Ile Asp Phe Glu Ile Glu Gly Asp Tyr Pro
            580                 585                 590

Lys Phe Gly Asn Asn Asp Ser Arg Val Asp Asp Ile Ala Cys Glu Leu
        595                 600                 605

Val Glu Arg Phe Met Asn Lys Ile Arg Ser Leu Lys Thr Tyr Arg Asn
    610                 615                 620

Ala Val Pro Thr Gln Ser Ile Leu Thr Ile Thr Ser Asn Val Val Tyr
625                 630                 635                 640

Gly Lys Lys Thr Gly Asn Thr Pro Asp Gly Arg Arg Ala Gly Ala Pro
                645                 650                 655

Phe Ala Pro Gly Ala Asn Pro Met His Gly Arg Asp Glu Lys Gly Ala
            660                 665                 670

Val Ala Ser Leu Thr Ser Val Gly Lys Leu Pro Phe Ala His Ala Lys
        675                 680                 685

Asp Gly Ile Ser Tyr Thr Phe Ser Ile Val Pro Asn Ala Leu Gly Lys
    690                 695                 700

Asp Glu Asn Ser Gln Arg Ala Asn Leu Ala Gly Leu Met Asp Gly Tyr
705                 710                 715                 720

Phe His His Glu Ala Gly Ile Glu Gly Gly Gln His Leu Asn Val Asn
                725                 730                 735

Val Leu Asn Arg Asp Thr Leu Leu Asp Ala Val Lys His Pro Glu Lys

            740                 745                 750

Tyr Pro Gln Leu Thr Ile Arg Val Ser Gly Tyr Ala Val Arg Phe Asn
        755                 760                 765

Ser Leu Thr Ala Glu Gln Gln Gln Asp Val Ile Ala Arg Thr Phe Thr
    770                 775                 780

Glu Ser Leu
785

<210> 59
<211> 320
<212> PRT
<213> Vibrio cholerae

<220>
<221> PEPTIDE
<222> (1)..(320)
<223> >sp|Q9KNP2|K6PF_VIBCH 6-phosphofructokinase (EC
2.7.1.11) (Phosphofructokinase)
(Phosphohexokinase) - Vibrio cholerae. (320 aa)

<400> 59
Met Ile Lys Lys Ile Gly Val Leu Thr Ser Gly Gly Asp Ala Pro Gly
1               5                   10                  15

Met Asn Ala Ala Ile Arg Gly Val Val Arg Thr Ala Leu Gly Ala Gly
            20                  25                  30

Leu Glu Val Phe Gly Ile Tyr Asp Gly Tyr Gln Gly Leu Tyr Glu Asp
        35                  40                  45

Arg Ile Lys Gln Leu Asp Arg Ser Ser Val Ser Asp Val Ile Asn Arg
    50                  55                  60

Gly Gly Thr Phe Leu Gly Ser Ala Arg Phe Pro Gln Phe Arg Glu Val
65                  70                  75                  80

Glu Val Arg Glu Lys Ala Ile Glu Asn Leu Lys Lys His Gly Ile Glu
                85                  90                  95

Ala Leu Val Val Ile Gly Gly Asp Gly Ser Tyr Met Gly Ala Lys Lys
            100                 105                 110

Leu Thr Glu Met Gly Phe Pro Cys Ile Gly Leu Pro Gly Thr Ile Asp
        115                 120                 125

Asn Asp Ile Ala Gly Thr Asp Tyr Thr Ile Gly Tyr Leu Thr Ala Leu
    130                 135                 140

Asn Thr Val Ile Glu Ala Ile Asp Arg Leu Arg Asp Thr Ser Ser Ser
145                 150                 155                 160

His Gln Arg Ile Ser Ile Val Glu Ile Met Gly Arg His Cys Gly Asp
                165                 170                 175

Leu Thr Leu Thr Ser Ala Ile Ala Gly Gly Cys Glu Tyr Ile Ile Thr
            180                 185                 190

Pro Glu Thr Gly Leu Asn Met Glu Gln Leu Ile Ser Asn Ile Lys Asp
        195                 200                 205

Gly Ile Ala Lys Gly Lys Lys His Ala Ile Ile Ala Leu Thr Glu Leu
    210                 215                 220

Met Met Asp Ala Asn Lys Leu Ala Lys Glu Ile Glu Ser Ala Thr Gly
225                 230                 235                 240

Arg Glu Thr Arg Ala Thr Val Leu Gly His Ile Gln Arg Gly Gly Lys
                245                 250                 255

Pro Thr Ala Phe Asp Arg Val Leu Ala Ser Arg Met Gly Asn Tyr Ala
            260                 265                 270

Val His Leu Leu Met Glu Gly His Gly Gly Arg Cys Val Gly Ile Gln
        275                 280                 285

Lys Glu Gln Leu Val His His Asp Ile Ile Asp Ala Ile Glu Asn Met
    290                 295                 300

Lys Arg Pro Val Arg Thr Asp Leu Tyr Lys Val Ala Glu Glu Leu Phe
305                 310                 315                 320

<210> 60
<211> 130
<212> PRT
<213> Vibrio cholerae

<220>
<221> PEPTIDE
<222> (1)..(130)
<223> >sp|Q9KNZ8|RS8_VIBCH 30S ribosomal protein S8 -
Vibrio cholerae. (130 aa)

<400> 60
Met Ser Met Gln Asp Pro Ile Ser Asp Met Leu Thr Arg Ile Arg Asn
1               5                   10                  15

Gly Gln Ala Ala Asn Lys Val Ala Val Lys Met Pro Ser Ser Lys Leu
            20                  25                  30

Lys Val Ala Ile Ala Ala Leu Leu Lys Ala Glu Gly Tyr Ile Ala Asp
        35                  40                  45

Phe Ala Val Glu Gly Glu Val Lys Ala Glu Leu Glu Ile Thr Leu Lys
    50                  55                  60

Tyr Phe Gln Ala Lys Pro Val Ile Glu Gln Ile Lys Arg Val Ser Arg
65                  70                  75                  80

Pro Gly Leu Arg Val Tyr Lys Lys Lys Asp Glu Leu Pro Ser Val Met
                85                  90                  95

Gly Gly Leu Gly Val Ala Val Val Ser Thr Ser Lys Gly Leu Met Ser
            100                 105                 110

Asp Arg Ala Ala Arg Lys Ala Gly Leu Gly Gly Glu Ile Ile Cys Tyr
        115                 120                 125

Val Ala
    130



<210> 61
<211> 257
<212> PRT
<213> Vibrio cholerae

<220>
<221> PEPTIDE
<222> (1)..(257)
<223> >sp|Q9KNR1|TPIS_VIBCH Triosephosphate isomerase
      (EC 5.3.1.1) (TIM) (Triose-phosphate isomerase) -
      Vibrio cholerae. (257 aa)

<400> 61
Met Arg Arg Pro Val Val Met Gly Asn Trp Lys Leu Asn Gly Ser Lys
  1               5                  10                  15

Ala Met Val Thr Asp Leu Leu Asn Gly Leu Asn Ala Glu Leu Glu Gly
             20                  25                  30

Val Glu Gly Val Asp Val Val Val Ala Pro Pro Ala Met Tyr Leu Asp
         35                  40                  45

Leu Ala Glu Arg Leu Ile Lys Glu Gly Gly Asn Lys Leu Ile Leu Gly
     50                  55                  60

Ala Gln Asn Thr Asp Thr His Asn Ser Gly Ala Tyr Thr Gly Asp Met
 65                  70                  75                  80

Ser Pro Ala Met Leu Lys Asp Phe Gly Ala Ser His Ile Ile Ile Gly
                 85                  90                  95

His Ser Glu Arg Arg Asp Tyr His Lys Glu Ser Asp Glu Phe Val Ala
            100                 105                 110

Lys Lys Phe Ala Phe Leu Lys Glu Asn Gly Leu Thr Pro Val Phe Cys
        115                 120                 125

Ile Gly Glu Thr Glu Ala Gln Asn Glu Ala Gly Glu Thr Glu Ala Val
    130                 135                 140

Cys Ala Arg Gln Ile Asn Ala Val Ile Asp Ala Tyr Gly Val Glu Ala
145                 150                 155                 160

Leu Asn Gly Ala Ile Ile Ala Tyr Glu Pro Ile Trp Ala Ile Gly Thr
                165                 170                 175

Gly Lys Ala Ala Thr Ala Asp Asp Ala Gln Arg Ile His Ala Ser Ile
            180                 185                 190

Arg Ala Leu Ile Ala Ala Lys Asp Ala Ala Val Ala Glu Gln Val Ile
        195                 200                 205

Ile Gln Tyr Gly Gly Ser Val Lys Pro Glu Asn Ala Ala Ser Tyr Phe

210                 215                 220

Ala Gln Pro Asp Ile Asp Gly Ala Leu Val Gly Gly Ala Ser Leu Asp
225                 230                 235                 240

Ala Lys Gly Phe Ala Ala Ile Ala Lys Ala Ala Ala Glu Ala Lys Lys
                245                 250                 255

Ala

<210> 62
<211> 176
<212> PRT
<213> Vibrio cholerae

<220>
<221> PEPTIDE
<222> (1)..(176)
<223> >sp|Q9KP34|IPYR_VIBCH Inorganic pyrophosphatase
      (EC 3.6.1.1) (Pyrophosphate phospho- hydrolase)
      (PPase) - Vibrio cholerae. (176 aa)

<400> 62
Met Ser Leu Asn Asn Val Pro Ala Gly Lys Ser Leu Pro Asp Asp Ile
1               5                   10                  15

Tyr Val Val Ile Glu Ile Pro Ala Asn Ala Asp Pro Ile Lys Tyr Glu
            20                  25                  30

Val Asp Lys Glu Ser Gly Ala Val Phe Val Asp Arg Phe Met Ser Ala
        35                  40                  45

Pro Met Phe Tyr Pro Cys Asn Tyr Gly Tyr Val Asn His Thr Leu Ser
    50                  55                  60

Leu Asp Gly Asp Pro Val Asp Val Leu Val Pro Thr Pro Tyr Pro Leu
65                  70                  75                  80

Ile Pro Gly Ser Val Ile Arg Cys Arg Pro Val Gly Val Leu Lys Met
                85                  90                  95

Thr Asp Glu Ser Gly Glu Asp Ala Lys Val Val Ala Val Pro His Thr
            100                 105                 110

Lys Ile Ser Lys Glu Tyr Asp His Ile Gln Asp Val Asn Asp Leu Pro
        115                 120                 125

Ala Leu Leu Lys Ala Gln Ile Thr His Phe Phe Glu Arg Tyr Lys Glu
    130                 135                 140

Leu Glu Ser Gly Lys Trp Val Lys Val Asp Gly Trp Glu Asp Ala Ala
145                 150                 155                 160

Ser Ala Arg Ala Glu Ile Leu Ser Ser Tyr Glu Arg Ala Gln Asn Lys
                165                 170                 175

<210> 63
<211> 116
<212> PRT
<213> Vibrio cholerae

<220>
<221> PEPTIDE
<222> (1)..(116)
<223> >sp|Q9KUF7|RL19_VIBCH 50S ribosomal protein L19 -
      Vibrio cholerae. (116 aa)

<400> 63
Ser Asn Ile Ile Lys Ala Leu Glu Gln Glu Gln Met Lys Gln Asp Leu
1               5                   10                  15

Pro Gln Phe Ala Pro Gly Asp Thr Val Val Val Gln Val Lys Val Lys
            20                  25                  30

Glu Gly Asp Arg Glu Arg Leu Gln Ala Phe Glu Gly Ile Val Ile Ala
        35                  40                  45

Ile Arg Asn Arg Gly Leu His Ser Ala Phe Thr Val Arg Lys Ile Ser
    50                  55                  60

Asn Gly Glu Gly Val Glu Arg Thr Phe Gln Thr His Ser Pro Val Val
65                  70                  75                  80

Asp Ser Ile Glu Val Lys Arg Arg Gly Ala Val Arg Arg Ala Lys Leu
                85                  90                  95

Tyr Tyr Leu Arg Asp Leu Ser Gly Lys Ala Ala Arg Ile Lys Glu Lys
            100                 105                 110

Leu Ala Lys Lys
        115

<210> 64
<211> 394
<212> PRT
<213> Vibrio cholerae

<220>
<221> PEPTIDE
<222> (1)..(394)
<223> >sp|Q9KV37|EFTU1_VIBCH Elongation factor Tu-A
      (EF-Tu-A) - Vibrio cholerae. (394 aa)

<400> 64
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
        35                  40                  45

Ser Ile Asp Asn Ala Pro Glu Glu Arg Glu Arg Gly Ile Thr Ile Asn

    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Arg
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp
        195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala
        355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390

<210> 65
<211> 389
<212> PRT
<213> Vibrio cholerae

<220>
<221> PEPTIDE
<222> (1)..(389)
<223> >tr|Q9KUV8|Q9KUV8_VIBCH MSHA biogenesis protein
      MshN - Vibrio cholerae. (389 aa)

<400> 65
Met Gly Met Glu Gln Thr Met Ser Val Val Asn Glu Ala Leu Ala Lys
1               5                   10                  15

Ser Ala Gln Arg Ser His Ser Arg Leu Ser Asn Ile Glu Arg Ile Asp
            20                  25                  30

Val Ala Lys Pro Lys Ala Arg Pro Ala Trp Leu Trp Val Met Leu Gly
        35                  40                  45

Phe Gly Val Ser Leu Ala Val Gly Gly Trp Ser Ile Ser Leu Gln Ser
    50                  55                  60

Val Asp Thr Ile Pro Ser Ser Ala Glu Val Val Arg Pro Glu Val Pro
65                  70                  75                  80

Ser Pro Thr Gln Lys Thr Thr Gln Gln Ser Ile Ala Leu Tyr Gln Ala
                85                  90                  95

Pro Val Asn Ser Glu Ala Leu Val Lys Asn Glu Thr Leu Val Gln Lys
            100                 105                 110

Glu Thr Leu Pro Lys Asn Glu Thr Ser Val Lys Asn Ala Val Leu Ala
        115                 120                 125

Lys Val Glu Gly Ser Pro Thr Ser Leu Lys Gln Thr Ala Asn Arg Gln
    130                 135                 140

Arg Glu Gln Thr Ala Leu Gly Ser Glu Leu Asn Ala Glu Pro Val Leu
145                 150                 155                 160

Asn Leu Val Asp Asn Ser Glu Pro Ser Phe Phe Glu Glu Glu Val Ser
                165                 170                 175

Ser Leu Pro Ser Ser Ser Pro Val Met Ile Val Glu Gln Val Ser Leu
            180                 185                 190

Thr Pro Glu Gln Leu Ala Gln Lys Ala Leu Gln Arg Ala Gln Lys Ala
        195                 200                 205

Met Glu Ser Asn Glu Leu Gln Thr Ala Val Ser Ala Tyr Thr Glu Ala
    210                 215                 220

Leu Arg Tyr Thr Pro His Asp Glu Met Ala Arg Gln Lys Leu Ala Ala
225                 230                 235                 240

Leu Tyr Tyr Gly Lys Gly Asp Gly Arg Lys Ala Phe Asp Leu Met Gln
                245                 250                 255

Ala Gly Ile Glu Arg Asn Pro Asp Gly Glu Val Leu Arg Leu Ala Leu
            260                 265                 270

Ala Lys Leu Leu Val Lys Glu Lys Gln Glu Ala Ser Ala Leu Val Pro
        275                 280                 285

Leu Ala Tyr Leu Pro Ser Gln Pro Ser Ile Glu Tyr Leu Ser Leu Arg
    290                 295                 300

Ala Ala Leu Ala Gln Lys Thr Lys Gln Asp Glu Ile Ala Arg Glu Ser
305                 310                 315                 320

Tyr Gln Gln Leu Thr Glu Lys Asp Pro Asn Asn Gly Arg Trp Trp Leu
                325                 330                 335

Gly Leu Ala Ile Gln Gln Glu Arg Ala Leu Gln Trp Pro Ala Ala Gln
            340                 345                 350

His Ala Tyr Gln Gln Ala Leu Asn Lys Val Gly Leu Ser Ser Gln Ser
        355                 360                 365

Gln Ala Phe Ile His Gln Arg Leu Gln Leu Leu Ala Ser Leu Glu Glu
    370                 375                 380

Thr Gln Ser Ala Asn
385

<210> 66
<211> 642
<212> PRT
<213> Vibrio cholerae

<220>
<221> PEPTIDE
<222> (1)..(642)
<223> >sp|Q9KMN7|SYT_VIBCH Threonyl-tRNA synthetase (EC
      6.1.1.3) (Threonine--tRNA ligase) (ThrRS) - Vibrio
      cholerae. (642 aa)

<400> 66
Met Pro Ile Ile Thr Leu Pro Asp Gly Ser Gln Arg His Phe Asp Asn
  1               5                  10                  15

Pro Val Ser Thr Leu Glu Val Ala Gln Ser Ile Gly Pro Gly Leu Ala
             20                  25                  30

Lys Ala Thr Ile Ala Gly Arg Val Asn Gly Ala Arg Val Asp Ala Cys
         35                  40                  45

Asp Leu Ile Glu His Asp Ala Ser Leu Glu Ile Ile Thr Thr Lys Asp
     50                  55                  60

Glu Val Asp Gly Leu Glu Ile Val Arg His Ser Cys Ala His Leu Leu
 65                  70                  75                  80

Gly His Ala Leu Lys Gln Leu Tyr Pro Asn Ala Lys Met Ala Ile Gly

                85                  90                  95

Pro Thr Ile Asp Ser Gly Phe Tyr Tyr Asp Ile Asp Leu Glu Gln Ser
            100                 105                 110

Leu Ser Gln Glu Asp Leu Glu Lys Ile Glu Ala Arg Met Val Glu Leu
        115                 120                 125

Ala Lys Thr Lys Tyr Ala Val Val Lys Lys Lys Val Ser Trp Gln Glu
    130                 135                 140

Ala Arg Asp Thr Phe Glu Ser Arg Gly Glu Ser Tyr Lys Met Glu Ile
145                 150                 155                 160

Leu Asp Glu Asn Val Ala Arg Asp Asp Arg Pro Gly Leu Tyr His His
                165                 170                 175

Glu Glu Tyr Ile Asp Met Cys Arg Gly Pro His Val Pro His Met Gly
            180                 185                 190

Phe Cys Gln Asn Phe Lys Leu Leu Asn Ile Ala Gly Ala Tyr Trp Arg
        195                 200                 205

Gly Asn Ser Asp Asn Lys Met Leu Gln Arg Ile Tyr Gly Thr Ala Phe
    210                 215                 220

His Asp Lys Lys Ala Leu Gln Ala His Leu Thr Arg Leu Glu Glu Ala
225                 230                 235                 240

Ala Lys Arg Asp His Arg Lys Ile Gly Lys Gln Leu Asp Leu Phe His
                245                 250                 255

Met Gln Gln Glu Ala Pro Gly Met Val Phe Trp His His Asn Gly Trp
            260                 265                 270

Ser Ile Phe Arg Asp Leu Glu Ile Phe Ile Arg Gln Lys Leu Asn Glu
        275                 280                 285

Tyr Gly Tyr Gln Glu Val Lys Gly Pro Leu Met Met Asp Arg Val Leu
    290                 295                 300

Trp Glu Arg Ser Gly His Trp Asp Lys Tyr Ala Asp Ala Met Phe Thr
305                 310                 315                 320

Thr Ser Ser Glu Asn Arg Glu Tyr Ala Ile Lys Pro Met Asn Cys Pro
                325                 330                 335

Gly His Ile Gln Ile Phe Asn Gln Gly Leu Lys Ser Tyr Arg Asp Leu
            340                 345                 350

Pro Leu Arg Met Ala Glu Phe Gly Ser Cys His Arg Asn Glu Pro Ser
        355                 360                 365

Gly Ser Leu His Gly Ile Met Arg Val Arg Gly Phe Thr Gln Asp Asp
    370                 375                 380

Ala His Ile Phe Cys Thr Glu Asp Gln Ile Gln Gln Glu Val Thr Ser
385                 390                 395                 400

Cys Ile Lys Met Val Tyr Asp Thr Tyr Thr Thr Phe Gly Phe Gln Asn
                405                 410                 415

Ile Val Val Lys Leu Ser Thr Arg Pro Glu Lys Arg Val Gly Ser Asp
                420                 425                 430

Glu Ile Trp Asp Lys Ser Glu Gln Ala Leu Ile Asp Ser Leu Lys Ala
            435                 440                 445

Met Asp Ile Pro Phe Glu Ile Gln Glu Gly Glu Gly Ala Phe Tyr Gly
    450                 455                 460

Pro Lys Ile Glu Phe Thr Leu Tyr Asp Cys Leu Asp Arg Ala Trp Gln
465                 470                 475                 480

Cys Gly Thr Val Gln Leu Asp Phe Asn Leu Pro Thr Arg Leu Gly Ala
                485                 490                 495

Thr Tyr Val Gly Glu Ser Asn Glu Arg Leu Ile Pro Val Met Ile His
                500                 505                 510

Arg Ala Ile Leu Gly Ser Leu Glu Arg Phe Ile Gly Ile Leu Ile Glu
            515                 520                 525

Glu Tyr Ala Gly Phe Phe Pro Thr Trp Leu Ala Pro Glu Gln Ala Val
    530                 535                 540

Val Val Asn Ile Thr Asp Lys Gln Ala Asp Tyr Ala His Glu Val Ala
545                 550                 555                 560

Gln Lys Leu Gln Lys Cys Gly Ile Arg Ala Lys Ala Asp Leu Arg Asn
                565                 570                 575

Glu Lys Ile Gly Phe Lys Ile Arg Glu His Thr Leu Lys Arg Val Pro
                580                 585                 590

Tyr Met Leu Val Cys Gly Asp Gln Glu Met Glu Ala Gly Glu Ile Ala
            595                 600                 605

Val Arg Thr Arg Lys Gly Lys Asp Leu Gly Lys Phe Lys Leu Asp Asp
    610                 615                 620

Phe Ile Ala His Ile Gln Ala Glu Ile Ala Ser Arg Lys Leu Asn Leu
625                 630                 635                 640

Glu Glu

<210> 67
<211> 645
<212> PRT
<213> Vibrio cholerae

<220>
<221> PEPTIDE
<222> (1)..(645)
<223> >sp|Q9KVS8|THIC_VIBCH Thiamine biosynthesis
      protein thiC - Vibrio cholerae. (645 aa)

<400> 67
Met Ser Asn Arg Lys Gln Ala Arg Leu Glu Ala Lys Arg Phe Ile Asp
  1               5                  10                  15

Thr Leu Ser Val Glu Pro Tyr Pro Asn Ser Gln Lys Ser Tyr Leu Leu
            20                  25                  30

Gly Ser Arg Pro Asp Ile Arg Val Pro Val Arg Glu Ile Thr Leu Ser
        35                  40                  45

Asp Thr Leu Val Gly Gly Ser Lys Asp Ala Pro Ile Phe Glu Pro Asn
    50                  55                  60

Glu Pro Ile Cys Val Tyr Asp Thr Ser Gly Val Tyr Thr Asp Pro Ser
65                  70                  75                  80

His Asp Ile Asp Leu Tyr Lys Gly Leu Pro Lys Leu Arg Glu Glu Trp
                85                  90                  95

Ile Glu Glu Arg Arg Asp Thr His Ile Leu Pro Ser Met Ser Ser His
            100                 105                 110

Phe Ala Arg Glu Arg Leu Ala Asp Glu Thr Leu Asp Glu Leu Arg Tyr
        115                 120                 125

Gly His Leu Pro Arg Ile Arg Arg Ala Met Gly Gln His Arg Val Thr
    130                 135                 140

Gln Leu His Tyr Ala Arg Gln Gly Ile Ile Thr Pro Glu Met Glu Phe
145                 150                 155                 160

Val Ala Ile Arg Glu Asn Ser Arg Arg Leu Ala His Gln Asp Pro Ser
                165                 170                 175

Leu Leu Gln Gln His Ala Gly Gln Asn Phe Gly Ala His Leu Pro Asp
            180                 185                 190

Leu Ile Thr Pro Glu Phe Val Arg Arg Glu Ile Ala Glu Gly Arg Ala
        195                 200                 205

Ile Ile Pro Cys Asn Ile Asn His Pro Glu Ser Glu Pro Met Ile Ile
    210                 215                 220

Gly Arg Asn Phe Leu Val Lys Val Asn Ala Asn Ile Gly Asn Ser Ser
225                 230                 235                 240

Val Ser Ser Ser Ile Glu Glu Glu Val Glu Lys Leu Val Trp Ala Thr
                245                 250                 255

Arg Trp Gly Ala Asp Thr Val Met Asp Leu Ser Thr Gly Arg Asn Ile
            260                 265                 270

His Glu Thr Arg Glu Trp Ile Leu Arg Asn Ser Pro Val Pro Ile Gly
        275                 280                 285

Thr Val Pro Met Tyr Gln Ala Leu Glu Lys Val Asn Gly Val Ala Glu
    290                 295                 300

Asn Leu Thr Trp Glu Val Met Arg Asp Thr Leu Leu Glu Gln Ala Glu
305                 310                 315                 320

Gln Gly Val Asp Tyr Phe Thr Ile His Ala Gly Leu Leu Leu Arg Tyr
                325                 330                 335

Val Pro Met Thr Ala Lys Arg Val Thr Gly Ile Val Ser Arg Gly Gly
            340                 345                 350

Ser Ile Ile Ala Lys Trp Cys Leu Ser His His Gln Glu Asn Phe Leu
        355                 360                 365

Tyr Thr His Phe Arg Glu Ile Cys Glu Ile Cys Ala Gln Tyr Asp Val
    370                 375                 380

Ala Leu Ser Leu Gly Asp Gly Leu Arg Pro Gly Ser Ile Ala Asp Ala
385                 390                 395                 400

Asn Asp Glu Ala Gln Phe Ala Glu Leu Arg Thr Leu Gly Glu Leu Thr
                405                 410                 415

Gln Ile Ala Trp Glu Tyr Asp Val Gln Val Met Ile Glu Gly Pro Gly
            420                 425                 430

His Val Pro Met His Leu Ile Lys Ala Asn Met Asp Glu Gln Leu Lys
        435                 440                 445

His Cys His Glu Ala Pro Phe Tyr Thr Leu Gly Pro Leu Thr Thr Asp
    450                 455                 460

Ile Ala Pro Gly Tyr Asp His Ile Thr Ser Gly Ile Gly Ala Ala Met
465                 470                 475                 480

Ile Gly Trp Phe Gly Cys Ala Met Leu Cys Tyr Val Thr Pro Lys Glu
                485                 490                 495

His Leu Gly Leu Pro Asn Lys Glu Asp Val Lys Thr Gly Leu Ile Thr
            500                 505                 510

Tyr Lys Leu Ala Ala His Ala Ala Asp Leu Ala Lys Gly His Pro Gly
        515                 520                 525

Ala Gln Ile Arg Asp Asn Ala Leu Ser Lys Ala Arg Phe Glu Phe Arg
    530                 535                 540

Trp Glu Asp Gln Phe Asn Leu Ala Leu Asp Pro Val Thr Ala Arg Ala
545                 550                 555                 560

Phe His Asp Glu Thr Leu Pro Gln Glu Ser Gly Lys Val Ala His Phe
                565                 570                 575

Cys Ser Met Cys Gly Pro Lys Phe Cys Ser Met Lys Ile Ser Gln Glu
            580                 585                 590

Val Arg Asp Tyr Ala Asn Asn Gln Thr Leu Asp Thr Thr Val Ile Asp
        595                 600                 605

Leu Val Met Pro Ala Glu Ser Ile Gln Leu Ala Met Gln Asp Lys Ser
    610                 615                 620

Arg Glu Phe Leu Ala Ser Gly Ala Glu Leu Tyr His Pro Leu Val Lys
625                 630                 635                 640

Glu Pro Ile Glu Glu
                645

<210> 68
<211> 311
<212> PRT
<213> Vibrio cholerae

<220>
<221> PEPTIDE
<222> (1)..(311)
<223> >tr|Q9KQQ6|Q9KQQ6_VIBCH Methylenetetrahydrofolate
      dehydrogenase/methenyltetrahydrofolate
      cyclohydrolase - Vibrio cholerae. (311 aa)

<400> 68
Met Pro Val Asp Val Ile Lys Asn Ile Cys Asp Asn Val Arg Lys Asn
1               5                   10                  15

Phe Arg Ser Lys Pro Val Arg Lys Val Met Thr Ala Gln Asn Ile Asp
            20                  25                  30

Gly Thr Leu Ile Ser Gln Thr Val Arg Ser Glu Val Ala Ala Arg Val
        35                  40                  45

Lys Ala Arg Val Gln Ala Gly Leu Arg Ala Pro Gly Leu Ala Val Val
    50                  55                  60

Leu Val Gly Glu Asp Pro Ala Ser Gln Val Tyr Val Gly Ser Lys Arg
65                  70                  75                  80

Arg Ala Cys Glu Glu Val Gly Phe Val Ser Lys Ser Phe Asp Leu Pro
                85                  90                  95

Ala Thr Ala Ser Glu Glu Ala Leu Leu Ser Leu Val Glu Glu Leu Asn
            100                 105                 110

Asn Asp Pro Gln Ile Asp Gly Ile Leu Val Gln Leu Pro Leu Pro Ala
        115                 120                 125

Gly Met Asp Thr Thr Lys Val Leu Glu Ser Ile His Pro Glu Lys Asp
    130                 135                 140

Val Asp Gly Phe His Pro Tyr Asn Val Gly Arg Leu Ala Gln Arg Ile
145                 150                 155                 160

Pro Lys Leu Arg Ser Cys Thr Pro Lys Gly Ile Ile Thr Leu Leu Glu
                165                 170                 175

Arg Tyr Asn Ile Pro Leu Arg Gly Lys His Ala Val Ile Val Gly Ala
            180                 185                 190

Ser Asn Ile Val Gly Arg Pro Met Thr Leu Glu Leu Leu Leu Ala Gly
        195                 200                 205

Cys Thr Thr Thr Thr Cys His Arg Phe Thr Gln Asp Leu Glu Gly His
    210                 215                 220

Ile Arg Gln Ala Asp Ile Leu Val Val Ala Val Gly Lys Pro Asn Phe
225                 230                 235                 240

Ile Pro Gly Ala Trp Ile Lys Glu Gly Ala Val Val Val Asp Val Gly

245                    250                    255

Ile Asn Arg Leu Asp Thr Gly Lys Leu Val Gly Asp Val Glu Tyr Asp
            260                 265                 270

Val Ala Arg Thr Arg Ala Ser Phe Ile Thr Pro Val Pro Gly Gly Val
        275                 280                 285

Gly Pro Met Thr Val Ala Ser Leu Ile Glu Asn Thr Met Met Ala Cys
    290                 295                 300

Glu Gln Phe His Thr Gln Gly
305                 310


<210> 69
<211> 446
<212> PRT
<213> Vibrio cholerae

<220>
<221> PEPTIDE
<222> (1)..(446)
<223> >sp|Q9KPS1|NQRA_VIBCH Na(+)-translocating
      NADH-quinone reductase subunit A (EC 1.6.5.-)
      (Na(+)-translocating NQR subunit A) (Na(+)-NQR
      subunit A) (NQR complex subunit A) (NQR-1 subunit
      A) - Vibrio cho

<400> 69

<210> 70
<211> 635
<212> PRT
<213> Vibrio cholerae

<220>

<221> PEPTIDE
<222> (1)..(635)
<223> >sp|O34241|DNAK_VIBCH Chaperone protein dnaK (Heat
      shock protein 70) (Heat shock 70 kDa protein)
      (HSP70) - Vibrio cholerae. (635 aa)

<400> 70
Met Gly Lys Ile Ile Gly Ile Asp Leu Gly Thr Thr Asn Ser Cys Val
1               5                   10                  15

Ala Val Leu Asp Gly Asp Lys Pro Arg Val Ile Glu Asn Ala Glu Gly
            20                  25                  30

Glu Arg Thr Thr Pro Ser Val Ile Ala Tyr Thr Asp Gly Glu Thr Leu
        35                  40                  45

Val Gly Gln Pro Ala Lys Arg Gln Ala Val Thr Asn Pro Gln Asn Thr
    50                  55                  60

Leu Phe Ala Ile Lys Arg Leu Ile Gly Arg Arg Phe Glu Asp Glu Glu
65                  70                  75                  80

Val Gln Arg Asp Ile Lys Ile Met Pro Tyr Lys Ile Val Lys Ala Asp
                85                  90                  95

Asn Gly Asp Ala Trp Val Glu Ala Lys Gly Gln Lys Met Ala Ala Pro
            100                 105                 110

Gln Val Ser Ala Glu Val Leu Lys Lys Met Lys Lys Thr Ala Glu Asp
        115                 120                 125

Phe Leu Gly Glu Pro Val Thr Ala Ala Val Ile Thr Val Pro Ala Tyr
    130                 135                 140

Phe Asn Asp Ala Gln Arg Gln Ala Thr Lys Asp Ala Gly Arg Ile Ala
145                 150                 155                 160

Gly Leu Glu Val Lys Arg Ile Ile Asn Glu Pro Thr Ala Ala Ala Leu
                165                 170                 175

Ala Tyr Gly Leu Asp Lys Gln Gly Gly Asp Arg Thr Ile Ala Val Tyr
            180                 185                 190

Asp Leu Gly Gly Gly Thr Phe Asp Ile Ser Ile Ile Glu Ile Asp Glu
        195                 200                 205

Val Glu Gly Glu Lys Thr Phe Glu Val Leu Ser Thr Asn Gly Asp Thr
    210                 215                 220

His Leu Gly Gly Glu Asp Phe Asp Asn Arg Met Ile Asn Tyr Leu Val
225                 230                 235                 240

Asp Glu Phe Lys Lys Asp Gln Gly Ile Asp Leu Arg Asn Asp Pro Leu
                245                 250                 255

Ala Met Gln Arg Leu Lys Glu Ala Ala Glu Lys Ala Lys Ile Glu Leu
            260                 265                 270

Ser Ser Ala Gln Gln Thr Asp Val Asn Leu Pro Tyr Ile Thr Ala Asp
        275                 280                 285

Ala Thr Gly Pro Lys His Met Asn Ile Lys Val Thr Arg Ala Lys Leu
    290                 295                 300

Glu Ala Leu Val Glu Asp Leu Val Gln Arg Ser Leu Glu Pro Leu Lys
305                 310                 315                 320

Val Ala Leu Ala Asp Ala Asp Leu Ser Val Asn Asp Ile Thr Asp Val
                325                 330                 335

Ile Leu Val Gly Gly Gln Thr Arg Met Pro Met Val Gln Lys Lys Val
            340                 345                 350

Ala Glu Phe Phe Gly Lys Glu Pro Arg Lys Asp Val Asn Pro Asp Glu
    355                 360                 365

Ala Val Ala Val Gly Ala Ala Val Gln Gly Gly Val Leu Ala Gly Glu
    370                 375                 380

Val Lys Asp Val Leu Leu Leu Asp Val Thr Pro Leu Ser Leu Gly Ile
385                 390                 395                 400

Glu Thr Met Gly Gly Val Met Thr Lys Leu Ile Glu Lys Asn Thr Thr
                405                 410                 415

Ile Pro Thr Lys Ala Asn Gln Val Phe Ser Thr Ala Glu Asp Asn Gln
            420                 425                 430

Ser Ala Val Thr Ile His Val Leu Gln Gly Glu Arg Lys Gln Ala Met
    435                 440                 445

Tyr Asn Lys Ser Leu Gly Gln Phe Asn Leu Glu Gly Ile Asn Pro Ala
    450                 455                 460

Pro Arg Gly Met Pro Gln Ile Glu Val Ile Phe Asp Leu Asp Ala Asp
465                 470                 475                 480

Gly Ile Leu His Val Ser Ala Lys Asp Lys Gln Thr Gly Lys Glu Gln
                485                 490                 495

Lys Ile Thr Ile Gln Ala Ser Gly Gly Leu Ser Asp Ala Glu Ile Glu
            500                 505                 510

Lys Met Val Gln Glu Ala Glu Ala Asn Lys Glu Ala Asp Lys Lys Phe
    515                 520                 525

Glu Glu Leu Ala Thr Ala Arg Asn Gln Ala Asp Gln Met Ile His Ala
    530                 535                 540

Thr Arg Lys Gln Ile Thr Glu Ala Gly Glu Ala Leu Pro Ala Asp Glu
545                 550                 555                 560

Lys Ala Lys Ile Glu Thr Ala Ile Asn Glu Leu Glu Thr Ala Lys Lys
                565                 570                 575

Gly Glu Asp Lys Ala Glu Ile Asp Ala Lys Val Gln Ala Leu Met Ala
            580                 585                 590

Ala Ala Gln Lys Leu Met Glu Ile Ala Gln Gln Gln Ala Gln Ala Gln
    595                 600                 605

Gly Ala Asn Ala Gly Gln Ser Ser Ala Lys Glu Asp Asp Val Val Asp

610                 615                 620

Ala Glu Phe Glu Glu Val Asn Asp Asp Lys Lys
625                 630                 635

<210> 71
<211> 117
<212> PRT
<213> Vibrio cholerae

<220>
<221> PEPTIDE
<222> (1)..(117)
<223> >sp|Q9KP00|RL18_VIBCH 50S ribosomal protein L18 -
      Vibrio cholerae. (117 aa)

<400> 71
Met Asp Lys Lys Ala Ser Arg Ile Arg Arg Ala Thr Arg Ala Arg Arg
1               5                   10                  15

Lys Ile Ala Glu Leu Gly Ala Thr Arg Leu Val Val His Arg Thr Pro
            20                  25                  30

Arg His Val Tyr Ala Gln Val Ile Ala Ala Asn Gly Ser Glu Val Ile
        35                  40                  45

Ala Ala Ala Ser Thr Val Glu Lys Ala Ile Arg Glu Gln Val Lys Tyr
    50                  55                  60

Thr Gly Asn Ile Asp Ala Ala Lys Ala Val Gly Lys Ala Val Ala Glu
65                  70                  75                  80

Arg Ala Leu Glu Lys Gly Val Thr Val Val Ala Phe Asp Arg Ser Gly
                85                  90                  95

Phe Gln Tyr His Gly Arg Val Ala Ala Leu Ala Asp Ser Ala Arg Glu
            100                 105                 110

Ala Gly Leu Lys Phe
        115

<210> 72
<211> 345
<212> PRT
<213> Vibrio cholerae

<220>
<221> PEPTIDE
<222> (1)..(345)
<223> >tr|Q9KMH9|Q9KMH9_VIBCH Hypothetical protein
      VCA0374 - Vibrio cholerae. (345 aa)

<400> 72
Met Ile Leu Arg Gln Leu Asn Arg Ile Asp Met Ser Ser Phe Asp Phe
1               5                   10                  15

Pro Lys Tyr Gln Ala Asn Gln Glu Val Gly Asn Thr Gly Glu Ala Phe
            20                  25                  30

Phe Glu His Phe Val Thr Lys Glu Leu Gly Cys Ile Tyr Arg Lys Val
        35                  40                  45

His Arg Glu His Asp Phe Gly Ile Asp Gly Tyr Ile Asp Ile Val Asn
    50                  55                  60

Asn Gly Val Val Ser Gly Arg Ser Val Ala Val Gln Ile Lys Cys Gly
65                  70                  75                  80

Asp Ser Tyr Ile Ser Lys Val Thr Ser Gly Gly Ile Lys Tyr Leu Gly
                85                  90                  95

Asp Asn Lys His Leu Asn Phe Leu Ile Asn His Asn Val Pro Val Ile
            100                 105                 110

Leu Val Val Leu Asn Ser Asp Cys Thr Lys Gly Tyr Trp Val Glu Phe
        115                 120                 125

Ser Val Asn Ala Thr Asn Pro Ser Ser Lys Ser Trp Trp Ile Glu Ile
    130                 135                 140

Pro Phe Glu Asn Gln Leu Gly Thr Leu Asp Met Thr Ser Leu Cys Asp
145                 150                 155                 160

Ile Ala Gly Ile Ala Glu Asp Tyr Ser Glu Asp Ile Lys Val Ser Trp
                165                 170                 175

Ala Ile Asn Ser Ala Met Asp Glu Gln Ser Asp Ile Ser Phe Val Leu
            180                 185                 190

Ser Lys Glu Glu Val Glu Glu Leu Asp Leu Ser Gly Ile Asn Gln Met
        195                 200                 205

Met Glu Arg Tyr Phe Arg Thr Lys Asp Leu Leu Ile Ser Asn Arg Asn
    210                 215                 220

Lys Thr Val Phe Trp Ile Phe Gly Tyr Asp Asp Asp Ser Arg Glu Leu
225                 230                 235                 240

Tyr Glu Ile Pro Glu Val Arg Arg Trp Phe Lys Phe Thr Leu Glu Gln
                245                 250                 255

Gly Val Pro Trp Phe Tyr Leu Leu Asp Val Gly Ala Asp His Met Ser
            260                 265                 270

Leu Pro Ile Asn Met Tyr Ser Cys Cys Asn Ile Ser Val Asn Lys Leu
        275                 280                 285

Ser Cys Gly Ser Lys Ser Val Val Ile Ser Ser Val Ser Asp Ile Asn
    290                 295                 300

Asn Trp Val Glu Val Asn Phe Glu Asn Leu Asn Arg Phe Ala Asp Glu
305                 310                 315                 320

Lys Lys Ile Pro Asp Asn Ile Leu Lys Glu Val Ser Glu Lys Val Ile
                325                 330                 335

Thr Ser Val His Arg Leu Val Ala Gly
            340                 345

<210> 73
<211> 331
<212> PRT
<213> Vibrio cholerae

<220>
<221> PEPTIDE
<222> (1)..(331)
<223> >tr|Q9KQJ8|Q9KQJ8_VIBCH Glyceraldehyde 3-phosphate
      dehydrogenase - Vibrio cholerae. (331 aa)

<400> 73
Met Thr Ile Lys Val Gly Ile Asn Gly Phe Gly Arg Ile Gly Arg Phe
1               5                   10                  15

Val Phe Arg Ala Ala Gln Glu Arg Asn Asp Ile Glu Ile Val Gly Ile
            20                  25                  30

Asn Asp Leu Ile Asp Val Asp Tyr Met Ala Tyr Met Leu Lys Tyr Asp
        35                  40                  45

Ser Thr His Gly Arg Phe Asn Gly Thr Val Glu Val Lys Asp Gly Asn
    50                  55                  60

Leu Ile Val Asn Gly Lys Thr Val Arg Val Thr Ala Glu Arg Asn Pro
65                  70                  75                  80

Ala Asp Leu Lys Trp Gly Glu Ile Gly Val Asp Val Val Ala Glu Ala
                85                  90                  95

Thr Gly Leu Phe Leu Thr Asp Glu Thr Ala Arg Lys His Ile Glu Ala
            100                 105                 110

Gly Ala Lys Lys Val Val Leu Thr Gly Pro Ser Lys Asp Lys Thr Pro
        115                 120                 125

Met Phe Val Met Gly Val Asn His Lys Thr Tyr Ala Gly Gln Asp Ile
    130                 135                 140

Val Ser Asn Ala Ser Cys Thr Thr Asn Cys Leu Ala Pro Ile Ala Lys
145                 150                 155                 160

Val Leu Asn Asp Asn Phe Gly Ile Glu Ser Gly Leu Met Thr Thr Val
                165                 170                 175

His Ala Thr Thr Ala Thr Gln Lys Thr Val Asp Gly Pro Ser Ala Lys
            180                 185                 190

Asp Trp Arg Gly Gly Arg Gly Ala Ser Gln Asn Ile Ile Pro Ser Ser
        195                 200                 205

Thr Gly Ala Ala Lys Ala Val Gly Val Val Leu Pro Glu Leu Asn Gly
    210                 215                 220

Lys Leu Thr Gly Met Ala Phe Arg Val Pro Thr Ala Asn Val Ser Val
225                 230                 235                 240

Val Asp Leu Thr Val Asn Leu Gln Lys Ala Ala Ser Tyr Asp Asp Ile
                245                 250                 255

Lys Ala Ala Met Lys Ala Ala Ser Glu Gly Glu Leu Ala Gly Ile Leu

260                 265                 270

Gly Tyr Thr Glu Asp Ala Val Val Ser Thr Asp Phe Asn Gly Asp Thr
        275                 280                 285

Arg Thr Ser Ile Phe Asp Ala Asp Ala Gly Ile Ala Leu Thr Asp Lys
    290                 295                 300

Phe Val Lys Val Val Ser Trp Tyr Asp Asn Glu Ile Gly Tyr Ser Asn
305                 310                 315                 320

Lys Val Leu Asp Leu Ile Ala His Ile Ser Lys
                325                 330


<210> 74
<211> 513
<212> PRT
<213> Vibrio cholerae

<220>
<221> PEPTIDE
<222> (1)..(513)
<223> >sp|Q9KNH3|ATPA_VIBCH ATP synthase alpha chain (EC
      3.6.3.14) - Vibrio cholerae. (513 aa)

<400> 74
Met Gln Leu Asn Ser Thr Glu Ile Ser Asp Leu Ile Lys Gln Arg Ile
1               5                   10                  15

Glu Ser Phe Asn Val Val Ser Glu Ala Arg Asn Glu Gly Thr Ile Val
            20                  25                  30

Ser Val Ser Asp Gly Ile Ile Arg Ile His Gly Leu Ala Asp Val Met
        35                  40                  45

Gln Gly Glu Met Ile Glu Leu Pro Gly Asn Arg Tyr Ala Leu Ala Leu
    50                  55                  60

Asn Leu Glu Arg Asp Ser Val Gly Ala Val Val Met Gly Pro Tyr Ala
65                  70                  75                  80

Asp Leu Arg Glu Gly Met Lys Val Thr Gly Thr Gly Arg Ile Leu Glu
                85                  90                  95

Val Pro Val Gly Pro Glu Leu Leu Gly Arg Val Val Asn Thr Leu Gly
            100                 105                 110

Glu Pro Ile Asp Gly Lys Gly Pro Ile Gly Ala Lys Gln Thr Ser Pro
        115                 120                 125

Val Glu Val Ile Ala Pro Gly Val Ile Asp Arg Lys Ser Val Asp Gln
    130                 135                 140

Pro Val Gln Thr Gly Tyr Lys Ser Val Asp Ser Met Ile Pro Ile Gly
145                 150                 155                 160

Arg Gly Gln Arg Glu Leu Ile Ile Gly Asp Arg Gln Thr Gly Lys Thr
                165                 170                 175

Ala Leu Ala Ile Asp Ala Ile Ile Asn Gln Lys Asn Ser Gly Ile Tyr

            180                 185                 190

Ser Ile Tyr Val Ala Ile Gly Gln Lys Ala Ser Thr Ile Ala Asn Val
        195                 200                 205

Val Arg Lys Leu Glu Glu His Gly Ala Leu Lys Asn Thr Ile Val Val
    210                 215                 220

Val Ala Ser Ala Ser Glu Ser Ala Ala Leu Gln Tyr Leu Ala Pro Tyr
225                 230                 235                 240

Ser Gly Cys Ala Met Gly Glu Tyr Phe Arg Asp Arg Gly Glu Asp Ala
                245                 250                 255

Leu Ile Val Tyr Asp Asp Leu Ser Lys Gln Ala Val Ala Tyr Arg Gln
            260                 265                 270

Ile Ser Leu Leu Leu Arg Arg Pro Pro Gly Arg Glu Ala Phe Pro Gly
        275                 280                 285

Asp Val Phe Tyr Leu His Ser Arg Leu Leu Glu Arg Ala Ala Arg Val
    290                 295                 300

Asn Glu Glu Tyr Val Glu Arg Phe Thr Lys Gly Glu Val Lys Gly Lys
305                 310                 315                 320

Thr Gly Ser Leu Thr Ala Leu Pro Ile Ile Glu Thr Gln Ala Gly Asp
                325                 330                 335

Val Ser Ala Phe Val Pro Thr Asn Val Ile Ser Ile Thr Asp Gly Gln
            340                 345                 350

Ile Phe Leu Gln Thr Glu Leu Phe Asn Ala Gly Val Arg Pro Ala Val
        355                 360                 365

Asp Pro Gly Ile Ser Val Ser Arg Val Gly Gly Ala Ala Gln Thr Lys
    370                 375                 380

Ile Val Lys Lys Leu Ser Gly Gly Ile Arg Thr Ala Leu Ala Ala Tyr
385                 390                 395                 400

Arg Glu Leu Ala Ala Phe Ala Gln Phe Ser Ser Asp Leu Asp Glu Ala
                405                 410                 415

Thr Lys Arg Gln Leu Thr His Gly Gln Lys Val Thr Glu Leu Met Lys
            420                 425                 430

Gln Lys Gln Tyr Ala Pro Met Ser Val Phe Asp Gln Ala Leu Val Ile
        435                 440                 445

Phe Ala Ala Glu Arg Gly Tyr Leu Thr Asp Val Glu Leu Asn Lys Val
    450                 455                 460

Leu Asp Phe Glu Ala Ala Leu Leu Ser Tyr Ala Arg Ala His Tyr Ala
465                 470                 475                 480

Glu Leu Ala Ala Gln Ile Asp Lys Thr Gly Ala Tyr Asn Asp Glu Ile
                485                 490                 495

Glu Ala Gln Leu Lys Lys Leu Val Asp Asp Phe Lys Ala Thr Gln Thr
            500                 505                 510

Trp

<210> 75
<211> 128
<212> PRT
<213> Vibrio cholerae

<220>
<221> PEPTIDE
<222> (1)..(128)
<223> >tr|Q9KP09|Q9KP09_VIBCH Ribosomal protein L17 -
      Vibrio cholerae. (128 aa)

<400> 75
Met Arg His Arg Lys Ser Gly Arg Gln Leu Asn Arg Asn Ser Ser His
1               5                   10                  15

Arg Lys Ala Met Phe Ser Asn Met Ala Ser Ser Leu Val Arg His Glu
            20                  25                  30

Val Ile Lys Thr Thr Leu Pro Lys Ala Lys Glu Leu Arg Arg Val Val
        35                  40                  45

Glu Pro Leu Ile Thr Leu Ala Lys Thr Asp Ser Val Ala Asn Arg Arg
    50                  55                  60

Leu Ala Phe Ala Arg Thr Arg Asp Asn Glu Val Val Ala Lys Leu Phe
65                  70                  75                  80

Asn Glu Leu Gly Pro Arg Phe Ala Ala Arg Gln Gly Gly Tyr Thr Arg
                85                  90                  95

Ile Leu Lys Cys Gly Phe Arg Ala Gly Asp Lys Ala Pro Met Ala Tyr
            100                 105                 110

Ile Glu Leu Val Asp Arg Pro Glu Ala Ala Val Glu Ala Ala Ala Glu
        115                 120                 125

<210> 76
<211> 233
<212> PRT
<213> Vibrio cholerae

<220>
<221> PEPTIDE
<222> (1)..(233)
<223> >sp|Q9KNZ0|RS3_VIBCH 30S ribosomal protein S3 -
      Vibrio cholerae. (233 aa)

<400> 76
Met Gly Gln Lys Val His Pro Asn Gly Ile Arg Leu Gly Ile Val Lys
1               5                   10                  15

Pro Trp Asn Ala Thr Trp Phe Ala Asn Thr Lys Asp Phe Ala Asp Asn

20                          25                          30

Leu Asp Gly Asp Phe Lys Val Arg Gln Tyr Leu Ser Lys Glu Leu Ala
            35                          40                          45

Asn Ala Ser Leu Ser Arg Ile Val Ile Glu Arg Pro Ala Lys Ser Ile
      50                          55                          60

Arg Val Thr Ile His Thr Ala Arg Pro Gly Val Val Ile Gly Lys Lys
 65                          70                          75                          80

Gly Glu Asp Val Glu Lys Leu Arg Ala Ala Val Ala Lys Ile Ala Gly
                        85                          90                          95

Val Pro Ala Gln Ile Asn Ile Ala Glu Val Arg Lys Pro Glu Leu Asp
                  100                         105                         110

Gly Gln Leu Val Ala Asp Ser Ile Ala Ser Gln Leu Glu Arg Arg Val
            115                         120                         125

Met Phe Arg Arg Ala Met Lys Arg Ala Val Gln Asn Ala Met Arg Leu
      130                         135                         140

Gly Ala Lys Gly Ile Lys Val Glu Val Ser Gly Arg Leu Gly Gly Ala
145                         150                         155                         160

Glu Ile Ala Arg Ser Glu Trp Tyr Arg Glu Gly Arg Val Pro Leu His
                        165                         170                         175

Thr Leu Arg Ala Asp Ile Asp Tyr Ala Thr Ser Ser Ala His Thr Thr
                  180                         185                         190

Tyr Gly Val Ile Gly Val Lys Val Trp Ile Phe Lys Gly Glu Ile Leu
            195                         200                         205

Gly Gly Met Pro Ala Ala Thr Glu Ala Ala Glu Pro Lys Ala Asp Lys
      210                         215                         220

Pro Lys Lys Gln Arg Lys Gly Arg Lys
225                         230

<210> 77
<211> 194
<212> PRT
<213> Vibrio cholerae

<220>
<221> PEPTIDE
<222> (1)..(94)

<400> 77
Met Ala Phe Glu Leu Pro Ala Leu Pro Tyr Ala Lys Asp Ala Leu Glu
  1                   5                          10                          15

Pro His Ile Ser Ala Glu Thr Leu Asp Phe His His Gly Lys His His
                  20                          25                          30

Asn Thr Tyr Val Val Lys Leu Asn Gly Leu Ile Pro Gly Thr Glu Phe
            35                          40                          45

Glu Asn Lys Ser Leu Glu Glu Ile Ile Lys Thr Ser Thr Gly Gly Ile
    50                  55                  60

Phe Asn Asn Ala Ala Gln Val Trp Asn His Thr Phe Tyr Trp His Cys
65                  70                  75                  80

Leu Ser Pro Asn Gly Gly Gly Glu Pro Thr Gly Ala Val Ala Glu Ala
                85                  90                  95

Ile Asn Ala Ala Phe Gly Ser Phe Ala Asp Phe Lys Ala Lys Phe Thr
            100                 105                 110

Asp Ser Ala Ile Asn Asn Phe Gly Ser Ser Trp Thr Trp Leu Val Lys
        115                 120                 125

Lys Ala Asp Gly Thr Leu Ala Ile Thr Asn Thr Ser Asn Ala Ala Thr
    130                 135                 140

Pro Leu Thr Glu Glu Gly Val Thr Pro Leu Leu Thr Val Asp Leu Trp
145                 150                 155                 160

Glu His Ala Tyr Tyr Ile Asp Tyr Arg Asn Val Arg Pro Asp Tyr Met
                165                 170                 175

Asn Gly Phe Trp Ala Leu Val Asn Trp Asp Phe Val Ala Gln Asn Leu
            180                 185                 190

Ala Lys

<210> 78
<211> 1052
<212> PRT
<213> Vibrio cholerae

<220>
<221> PEPTIDE
<222> (1)..(1052)
<223> >tr|Q9KQG8|Q9KQG8_VIBCH Ribonuclease E - Vibrio
cholerae. (1052 aa)

<400> 78
Met Lys Arg Met Leu Ile Asn Ala Thr Gln Lys Glu Glu Leu Arg Val
1               5                   10                  15

Ala Leu Val Asp Gly Gln Arg Leu Phe Asp Leu Asp Ile Glu Ser Pro
            20                  25                  30

Gly His Glu Ser Lys Lys Ala Asn Ile Tyr Lys Gly Arg Ile Thr Arg
        35                  40                  45

Ile Glu Pro Ser Leu Glu Ala Ala Phe Val Asp Tyr Gly Ala Glu Arg
    50                  55                  60

His Gly Phe Leu Pro Leu Lys Glu Ile Ala Arg Glu Tyr Phe Pro Asp
65                  70                  75                  80

Gly Tyr Ser Tyr Gln Gly Arg Pro Asn Ile Lys Asp Val Leu Asn Glu
                85                  90                  95

Gly Gln Glu Val Ile Val Gln Ile Glu Lys Glu Glu Arg Gly Ser Lys
            100                 105                 110

Gly Ala Ala Leu Thr Thr Phe Ile Ser Leu Ala Gly Ser Tyr Leu Val
        115                 120                 125

Leu Met Pro Asn Asn Pro Arg Ala Gly Gly Ile Ser Arg Arg Ile Glu
    130                 135                 140

Gly Asp Glu Arg Thr Glu Leu Lys Ser Ala Leu Ser Ser Leu Glu Leu
145                 150                 155                 160

Pro Gln Gly Met Gly Leu Ile Val Arg Thr Ala Gly Val Gly Lys Ser
                165                 170                 175

Ala Glu Glu Leu Glu Trp Asp Leu Asn Val Leu Leu Lys His Trp Ser
            180                 185                 190

Ala Ile Lys Gln Ala Ser Asp Ser Asn Pro Ala Pro Phe Leu Ile His
        195                 200                 205

Gln Glu Ser Asn Val Ile Val Arg Ala Ile Arg Asp Tyr Leu Arg Arg
    210                 215                 220

Asp Ile Gly Glu Ile Leu Ile Asp Ser Asn Ser Ile Tyr Glu Arg Ala
225                 230                 235                 240

Leu Glu His Ile Arg Leu Val Arg Pro Asp Phe Val Asn Arg Val Lys
                245                 250                 255

Lys Tyr Glu Gly Glu Val Pro Leu Phe Ser His Phe Gln Ile Glu Ser
            260                 265                 270

Gln Ile Glu Ser Ala Phe Gln Arg Glu Val Arg Leu Pro Ser Gly Gly
        275                 280                 285

Ser Ile Val Ile Asp Pro Thr Glu Ala Leu Thr Ser Ile Asp Ile Asn
    290                 295                 300

Ser Ala Arg Ala Thr Lys Gly Gly Asp Ile Glu Glu Thr Ala Leu Asn
305                 310                 315                 320

Thr Asn Leu Glu Ala Ala Asp Glu Ile Ala Arg Gln Leu Arg Leu Arg
                325                 330                 335

Asp Leu Gly Gly Leu Val Val Ile Asp Phe Ile Asp Met Thr Pro Val
            340                 345                 350

Arg His Gln Arg Glu Val Glu Asn Arg Leu Arg Glu Ala Val Arg Val
        355                 360                 365

Asp Arg Ala Arg Val Gln Ile Gly Arg Ile Ser Arg Phe Gly Leu Leu
    370                 375                 380

Glu Met Ser Arg Gln Arg Leu Ser Pro Ser Leu Ala Glu Ala Ser His
385                 390                 395                 400

His Ile Cys Pro Arg Cys Lys Gly Thr Gly Val Ile Arg Asp Asn Glu
                405                 410                 415

Ser Leu Ala Leu Ser Val Leu Arg Leu Ile Glu Glu Glu Ala Leu Lys
            420                 425                 430

Asp Asn Thr Ser Gln Val Leu Ala Val Val Pro Val Ser Ile Ala Ser
        435                 440                 445

Tyr Leu Leu Asn Glu Lys Arg Arg Ser Ile Asn His Ile Glu Lys Ala
    450                 455                 460

Gln Gln Val Arg Ile Thr Ile Val Pro Asn Ser Asp Met Glu Thr Pro
465                 470                 475                 480

His Phe Glu Val Ile Arg Val Arg Glu Gly Glu Glu Gln Asp Val Leu
                485                 490                 495

Ser Tyr Leu Ile Pro Lys Lys Leu Glu Ala Met Lys Glu Ala Glu Gly
            500                 505                 510

Lys Glu Val Val Asp Val Glu Leu Lys Pro Lys Arg Ile Glu Glu Pro
        515                 520                 525

Val Leu Lys Gly Phe Ala Ala Pro Ala Glu Ala Val Pro Ala Pro Thr
    530                 535                 540

Pro Lys Pro Lys Ala Glu Pro Gln Pro Val Ala Glu Val Gln Gln Pro
545                 550                 555                 560

Ala Gln Pro Gly Phe Phe Ser Arg Val Phe Lys Ala Ile Ala Ser Leu
                565                 570                 575

Phe Ser Ala Thr Pro Glu Ala Ala Lys Val Glu Ala Pro Val Thr Gln
            580                 585                 590

Asn Ser Glu Gln Asp Lys Pro Arg Arg Glu Arg Asn Asp Arg Asn Asn
        595                 600                 605

Asp Arg Arg Arg Asn Pro Arg Asp Lys Asn Arg Arg Arg Gly Asn Glu
    610                 615                 620

Glu Asn Asn Glu Arg Asn Asp Lys Glu Thr Ser Asn Ser Asn Arg Lys
625                 630                 635                 640

Pro Gln Glu Arg Lys Pro Lys Gln Glu Arg Gly Glu Arg Asn Glu Arg
                645                 650                 655

Asn Asp Arg Asn Glu Arg Pro Glu Thr Glu Arg Pro Glu Arg Gln Asp
            660                 665                 670

Arg Arg Asn Lys Arg Asp Glu Ser Lys Thr Ala Lys Leu Leu Glu Gln
        675                 680                 685

Gly Arg Gln Leu Ala Ala Glu Ala Gln Gln Glu Val Lys Ala Val Glu
    690                 695                 700

Pro Lys Glu Glu Lys Ala Ala Val Val Lys Glu Arg Arg Gln Arg Arg
705                 710                 715                 720

Lys Leu Ser Lys Gln Ile Arg Val Lys Asp Gln Leu Ala Ala Glu Glu
                725                 730                 735

Leu Asp Asn Leu Ser Thr Ala Pro Val Asp Asn Ala Phe Asp Ala Pro
            740                 745                 750

Ala Pro Val Asn Leu Pro Val Thr Asp Asp Phe Ala Asp Ser Ala Gln
        755                 760                 765

Glu Asp Asn Glu Gln Asp Asp Gln Lys Gln Arg Arg Asn Arg Arg Ser
    770                 775                 780

Pro Arg His Leu Arg Ala Ser Gly Gln Arg Arg Arg Arg Gly Arg Asp
785                 790                 795                 800

Arg Arg Pro Asn Pro Phe Arg Leu Arg Lys Gly Gly Val Ala Ser Pro
                805                 810                 815

Glu Met Ala Met Gly Lys Val Met Pro Arg Tyr Asp Leu Ala Pro Arg
            820                 825                 830

Pro Gln Ser Arg His Glu Thr Ala Glu Gly Ala Gln His Val Thr His
        835                 840                 845

Val Thr Pro Thr Ala Ala Thr Ala Val Val Glu Ser Glu Lys Val Ala
    850                 855                 860

Ala Pro Arg Val Leu Gly Gly Val Ala Phe Pro Glu Met Ala Met Gly
865                 870                 875                 880

Lys Val Ile Val Arg Arg Glu Thr Ala Ala Val Gln Pro Glu Pro Val
                885                 890                 895

Val Gln Glu Pro Val Ile Thr Asp Val Ile Ala Glu Thr Leu Ile Ala
            900                 905                 910

Pro Val Glu Pro Val Val Val Glu Ala Thr Val Ile Glu Ala Pro Val
        915                 920                 925

Ile Glu Glu Ser Ala Thr Glu Thr Ala Ala Leu Glu Thr Val Val Ala
    930                 935                 940

Lys Val Ala Glu Val Glu Glu Pro Ala Thr Ile Ser Asp Glu Val Val
945                 950                 955                 960

Ala Gln Thr Thr Val Glu Val Ile Ala Asp Lys Met Ala Glu Pro Ile
                965                 970                 975

Lys Val Val Lys Pro Val Thr Val Ser Ala Thr Ser Leu Thr Ala Lys
            980                 985                 990

Ala Val Ile Gln Gln Pro Tyr Ala Ser Ser Pro Met Thr Lys Ala Pro
        995                 1000                1005

Gly Ser Asp Asp Ile Arg Glu Val Gln Val Asn Ala Ala Pro Leu Arg
    1010                1015                1020

Ala Glu Arg Tyr Gln Ala Arg Gly Ala Gly Ser Gln Val Ala Arg Asn
1025                1030                1035                1040

Gln Ala Ser Ala Gly Met Ser Lys Pro Gln Ser Phe
                1045                1050

<210> 79
<211> 466
<212> PRT
<213> Vibrio cholerae

<220>
<221> PEPTIDE
<222> (1)..(466)
<223> >sp|Q9KNH5|ATPB_VIBCH ATP synthase beta chain (EC
      3.6.3.14) - Vibrio cholerae. (466 aa)

<400> 79
Ala Thr Gly Lys Ile Val Gln Ile Ile Gly Ala Val Val Asp Val Glu
1               5                   10                  15

Phe Pro Gln Ser Glu Val Pro Ser Val Tyr Asp Ala Leu Asn Val Val
            20                  25                  30

Asp Ser Lys Glu Arg Leu Val Leu Glu Val Gln Gln Gln Leu Gly Gly
        35                  40                  45

Gly Val Ile Arg Ala Ile Val Met Gly Ser Ser Asp Gly Leu Arg Arg
    50                  55                  60

Gly Met Thr Val Gln Asn Thr Gly Ala Pro Ile Ser Val Pro Val Gly
65                  70                  75                  80

Thr Lys Thr Leu Gly Arg Ile Met Asn Val Leu Gly Asp Ala Ile Asp
                85                  90                  95

Glu Arg Gly Asp Ile Gly Ala Glu Glu Val Tyr Ser Ile His Arg Pro
            100                 105                 110

Ala Pro Ser Tyr Glu Glu Gln Ser Ser Ala Thr Glu Leu Leu Glu Thr
        115                 120                 125

Gly Val Lys Val Ile Asp Leu Ile Cys Pro Phe Ala Lys Gly Gly Lys
    130                 135                 140

Ile Gly Leu Phe Gly Gly Ala Gly Val Gly Lys Thr Val Asn Met Met
145                 150                 155                 160

Glu Leu Ile Asn Asn Ile Ala Leu Gln His Ser Gly Leu Ser Val Phe
                165                 170                 175

Ala Gly Val Gly Glu Arg Thr Arg Glu Gly Asn Asp Phe Tyr His Glu
            180                 185                 190

Met Gln Glu Ala Gly Val Val Asn Val Glu Gln Pro Glu Leu Ser Lys
        195                 200                 205

Val Ala Met Val Tyr Gly Gln Met Asn Glu Pro Pro Gly Asn Arg Leu
    210                 215                 220

Arg Val Ala Leu Thr Gly Leu Thr Met Ala Glu Lys Phe Arg Asp Glu
225                 230                 235                 240

Gly Arg Asp Val Leu Leu Phe Ile Asp Asn Ile Tyr Arg Tyr Thr Leu
                245                 250                 255

Ala Gly Thr Glu Val Ser Ala Leu Leu Gly Arg Met Pro Ser Ala Val

260                 265                 270

Gly Tyr Gln Pro Thr Leu Ala Glu Glu Met Gly Val Leu Gln Glu Arg
        275                 280                 285

Ile Thr Ser Thr Lys Lys Gly Ser Ile Thr Ser Val Gln Ala Val Tyr
    290                 295                 300

Val Pro Ala Asp Asp Leu Thr Asp Pro Ser Pro Ala Thr Thr Phe Ala
305                 310                 315                 320

His Leu Asp Ala Thr Val Val Leu Asn Arg Asn Ile Ala Ala Met Gly
                325                 330                 335

Leu Tyr Pro Ala Ile Asp Pro Leu Asp Ser Thr Ser Arg Gln Leu Asp
            340                 345                 350

Pro Leu Val Val Gly Gln Glu His Tyr Asp Val Ala Arg Gly Val Gln
        355                 360                 365

Ala Thr Leu Gln Arg Tyr Lys Glu Leu Lys Asp Ile Ile Ala Ile Leu
    370                 375                 380

Gly Met Asp Glu Leu Ser Glu Ala Asp Lys Gln Val Val Ala Arg Ala
385                 390                 395                 400

Arg Lys Ile Glu Arg Phe Leu Thr Gln Pro Tyr His Val Ala Glu Val
                405                 410                 415

Phe Thr Gly Asp Pro Gly Val Tyr Val Pro Leu Lys Glu Thr Leu Arg
            420                 425                 430

Gly Phe Lys Gly Leu Leu Ala Gly Asp Tyr Asp Asp Ile Pro Glu Gln
        435                 440                 445

Ala Phe Met Tyr Cys Gly Thr Ile Asp Asp Ala Ile Glu Asn Ala Lys
    450                 455                 460

Lys Leu
465

<210> 80
<211> 517
<212> PRT
<213> Vibrio cholerae

<220>
<221> PEPTIDE
<222> (1)..(517)
<223> >sp|Q9KTW2|GUAA_VIBCH GMP synthase
      [glutamine-hydrolyzing] (EC 6.3.5.2) (Glutamine
      amidotransferase) (GMP synthetase) - Vibrio
      cholerae. (517 aa)

<400> 80
Met Thr Lys Asn Ile His Asp Gln Arg Ile Leu Ile Leu Asp Phe Gly
1               5                   10                  15

Ser Gln Tyr Thr Gln Leu Val Ala Arg Arg Val Arg Glu Ile Gly Val
            20                  25                  30

Tyr Cys Glu Leu Trp Ser Trp Asp Val Glu Glu Ala Asp Ile Arg Glu
        35                  40                  45

Phe Asn Pro Asp Gly Ile Ile Leu Ser Gly Gly Pro Glu Ser Val Thr
    50                  55                  60

Glu Ala Asn Ser Pro Arg Ala Pro Gln Tyr Val Phe Asp Ser Gly Val
65                  70                  75                  80

Pro Val Phe Gly Val Cys Tyr Gly Met Gln Thr Met Ala Glu Gln Leu
                85                  90                  95

Gly Gly Arg Val Ala Thr Ser Asp Glu Arg Glu Phe Gly Tyr Ala Gln
            100                 105                 110

Val Lys Ile Ser Gly Glu Ser Ala Leu Phe Lys Asp Leu Asp Leu Thr
        115                 120                 125

Gln Asp Val Trp Met Ser His Gly Asp Lys Val Val Glu Ile Pro Ala
    130                 135                 140

Asp Phe Val Lys Ile Gly Glu Thr Asp Thr Cys Pro Tyr Ala Ala Met
145                 150                 155                 160

Ala Asn Glu Glu Lys Lys Tyr Tyr Gly Val Gln Phe His Pro Glu Val
                165                 170                 175

Thr His Thr Lys Asn Gly Leu Gln Met Leu Glu Asn Phe Val Leu Gly
            180                 185                 190

Val Cys Gly Cys Glu Arg Leu Trp Thr Ser Glu Ser Ile Ile Glu Asp
        195                 200                 205

Ala Val Ala Arg Ile Lys Glu Gln Val Gly Asn Asp Glu Val Ile Leu
    210                 215                 220

Gly Leu Ser Gly Gly Val Asp Ser Ser Val Val Ala Met Leu Val His
225                 230                 235                 240

Arg Ala Ile Gly Ser Lys Leu Thr Cys Val Phe Val Asp Asn Gly Leu
                245                 250                 255

Leu Arg Leu Asn Glu Gly Glu Gln Val Met Glu Met Phe Gly Asp Lys
            260                 265                 270

Phe Gly Leu Asn Ile Ile Lys Val Asp Ala Glu Glu Arg Phe Leu Lys
        275                 280                 285

Ala Leu Glu Gly Ile Asp Glu Pro Glu Ala Lys Arg Lys Thr Ile Gly
    290                 295                 300

Arg Val Phe Val Glu Val Phe Asp Glu Glu Ser Lys Lys Leu Ser Asn
305                 310                 315                 320

Ala Lys Trp Leu Ala Gln Gly Thr Ile Tyr Pro Asp Val Ile Glu Ser
                325                 330                 335

Ala Ala Ser Lys Thr Gly Lys Ala His Val Ile Lys Ser His His Asn
            340                 345                 350

Val Gly Gly Leu Pro Asp Asp Met Lys Met Gly Leu Val Glu Pro Leu
        355                 360                 365

Arg Glu Leu Phe Lys Asp Glu Val Arg Lys Ile Gly Leu Glu Leu Gly
    370                 375                 380

Leu Pro Tyr Asn Met Leu Tyr Arg His Pro Phe Pro Gly Pro Gly Leu
385                 390                 395                 400

Gly Val Arg Val Leu Gly Glu Val Lys Lys Glu Tyr Cys Asp Leu Leu
                405                 410                 415

Arg Arg Ala Asp Ala Ile Phe Ile Glu Glu Leu His Ala Ala Asp Leu
            420                 425                 430

Tyr Asn Lys Val Ser Gln Ala Phe Thr Val Phe Leu Pro Val Arg Ser
        435                 440                 445

Val Gly Val Met Gly Asp Gly Arg Lys Tyr Asp Trp Val Val Ser Leu
    450                 455                 460

Arg Ala Val Glu Thr Ile Asp Phe Met Thr Ala His Trp Ala His Leu
465                 470                 475                 480

Pro Tyr Glu Phe Leu Gly Lys Val Ser Asn Arg Ile Ile Asn Glu Val
                485                 490                 495

Asn Gly Ile Ser Arg Val Val Tyr Asp Ile Ser Gly Lys Pro Pro Ala
            500                 505                 510

Thr Ile Glu Trp Glu
        515

<210> 81
<211> 512
<212> PRT
<213> Vibrio cholerae

<220>
<221> PEPTIDE
<222> (1)..(512)
<223> >tr|Q9KM26|Q9KM26_VIBCH NAD(P) transhydrogenase,
alpha subunit - Vibrio cholerae. (512 aa)

<400> 81
Met Gln Ile Gly Val Pro Arg Glu Gln Leu Ala Gly Glu Thr Arg Val
1               5                   10                  15

Ala Ala Ser Pro Ser Ser Val Glu Gln Leu Ile Lys Leu Gly Phe Glu
            20                  25                  30

Val Cys Ile Glu Ser Lys Ala Gly Ala Leu Ala Ser Phe Asp Asp Ala
        35                  40                  45

Ala Tyr Thr Ala Ala Gly Ala Thr Ile Gly Ser Arg Glu Glu Ile Trp
    50                  55                  60

Ala Cys Pro Leu Ile Leu Lys Val Asn Ala Pro Ser Asp Asp Glu Ile
65                  70                  75                  80

Ala Leu Leu Lys Glu Gly Ala Thr Leu Val Ser Phe Ile Trp Pro Ala
                85                  90                  95

Gln Asn Pro Ala Leu Met Glu Lys Leu Ser Ser Lys Asn Ile Asn Val
            100                 105                 110

Leu Ala Met Asp Ala Val Pro Arg Ile Ser Arg Ala Gln Ala Leu Asp
        115                 120                 125

Ala Leu Ser Ser Met Ala Asn Ile Ala Gly Tyr Arg Ala Val Val Glu
    130                 135                 140

Ala Ala His Glu Phe Gly Arg Phe Phe Thr Gly Gln Ile Thr Ala Ala
145                 150                 155                 160

Gly Lys Val Ser Pro Ala Lys Val Phe Val Ala Gly Ala Gly Val Ala
                165                 170                 175

Gly Leu Ala Ala Ile Gly Ala Ala Gly Ser Leu Gly Ala Ile Val Arg
            180                 185                 190

Ala Phe Asp Val Arg Pro Glu Val Lys Glu Gln Val Gln Ser Met Gly
        195                 200                 205

Ala Glu Phe Leu Glu Val Asn Phe Gln Glu Thr Ala Gly Ser Gly Asp
    210                 215                 220

Gly Tyr Ala Lys Glu Met Ser Asp Glu Phe Asn Arg Lys Ala Ala Glu
225                 230                 235                 240

Leu Tyr Ala Ala Gln Ala Lys Asp Val Asp Ile Ile Ile Thr Thr Ala
                245                 250                 255

Leu Ile Pro Gly Lys Pro Ala Pro Lys Leu Ile Thr Lys Glu Met Val
            260                 265                 270

Asp Ser Met Lys Ala Gly Ser Val Ile Val Asp Leu Ala Ala Ala Asn
        275                 280                 285

Gly Gly Asn Cys Glu Tyr Thr Val Lys Asp Gln Val Ile Thr Thr Asp
    290                 295                 300

Asn Gly Val Lys Val Ile Gly Tyr Thr Asp Met Val Gly Arg Leu Pro
305                 310                 315                 320

Thr Gln Ser Ser Gln Leu Tyr Ala Thr Asn Leu Val Asn Leu Leu Lys
                325                 330                 335

Leu Leu Cys Lys Glu Lys Asp Gly Asn Ile Asp Ile Asn Phe Glu Asp
            340                 345                 350

Val Val Leu Arg Gly Val Thr Val Val Lys Ala Gly Glu Ile Thr Trp
        355                 360                 365

Pro Ala Pro Pro Ile Gln Val Ser Ala Gln Pro Gln Gln Lys Ala Lys
    370                 375                 380

Val Gln Pro Thr Lys Ala Gln Lys Lys Glu Pro Glu Pro Thr Ser Pro
385                 390                 395                 400

Val Lys Lys Leu Val Gly Leu Ala Val Gly Val Gly Leu Phe Ala Trp

405                 410                 415

Val Ala Ser Val Ala Pro Ala Ala Phe Leu Gly His Phe Thr Val Phe
            420                 425                 430

Val Leu Ala Cys Val Val Gly Tyr Tyr Val Val Trp Asn Val Thr His
        435                 440                 445

Ala Leu His Thr Pro Leu Met Ser Val Thr Asn Ala Ile Ser Gly Ile
    450                 455                 460

Ile Val Val Gly Ala Leu Leu Gln Ile Gly Gln Gly Ser Gly Ile Val
465                 470                 475                 480

Thr Phe Leu Ala Phe Ile Ala Val Leu Ile Ala Ser Ile Asn Ile Phe
                485                 490                 495

Gly Gly Phe Thr Val Thr Lys Arg Met Leu Glu Met Phe Arg Lys Asn
            500                 505                 510

<210> 82
<211> 280
<212> PRT
<213> Vibrio cholerae

<220>
<221> PEPTIDE
<222> (1)..(280)
<223> >sp|Q9KPV3|EFTS_VIBCH Elongation factor Ts (EF-Ts)
      - Vibrio cholerae. (280 aa)

<400> 82
Met Ala Val Thr Ala Ala Leu Val Lys Glu Leu Arg Glu Arg Thr Gly
1               5                   10                  15

Ala Gly Met Met Glu Cys Lys Lys Ala Leu Val Glu Thr Asn Gly Asp
            20                  25                  30

Ile Glu Leu Ala Ile Glu Asn Met Arg Lys Ser Gly Ala Ala Lys Ala
        35                  40                  45

Ala Lys Lys Ala Gly Asn Ile Ala Ala Glu Gly Thr Ile Met Ile Lys
    50                  55                  60

Glu Gly Glu Gly Ile Ala Ala Leu Val Glu Val Asn Cys Gln Thr Asp
65                  70                  75                  80

Phe Val Ala Lys Asp Ser Asn Phe Val Ala Phe Ala Asn Gln Val Thr
                85                  90                  95

Asp Ala Ala Leu Ala Ser Lys Ala Ser Val Glu Glu Leu Gln Ala Gln
            100                 105                 110

Phe Glu Glu Ala Arg Val Ala Leu Val Ala Lys Ile Gly Glu Asn Ile
        115                 120                 125

Asn Ile Arg Arg Val Gln Tyr Val Glu Gly Glu Ala Leu Ala Thr Tyr

130                 135                 140

Arg His Gly Asp Arg Ile Gly Val Val Val Ala Gly Ser Ala Asp Val
145                 150                 155                 160

Glu Thr Leu Lys His Val Ala Met His Val Ala Ala Ser Arg Pro Glu
                165                 170                 175

Phe Leu Thr Pro Asp Asp Val Pro Ala Glu Val Val Ala Lys Glu Arg
            180                 185                 190

Glu Val Gln Val Gly Ile Ala Met Asn Glu Gly Lys Ser Lys Glu Ile
        195                 200                 205

Ala Glu Lys Met Val Glu Gly Arg Met Lys Lys Phe Thr Gly Glu Val
    210                 215                 220

Ser Leu Thr Gly Gln Pro Phe Val Met Glu Pro Lys Lys Thr Val Gly
225                 230                 235                 240

Glu Ile Leu Ala Glu Lys Gly Ala Thr Val Ser Ala Phe Ile Arg Leu
                245                 250                 255

Glu Val Gly Glu Gly Ile Glu Lys Gln Glu Gly Leu Ser Phe Ala Glu
            260                 265                 270

Glu Val Ala Leu Ala Gln Lys Gly
        275                 280


<210> 83
<211> 554

<212> PRT
<213> Vibrio cholerae

<220>
<221> PEPTIDE
<222> (1)..(554)
<223> >tr|Q9KTB2|Q9KTB2_VIBCH Asparagine synthetase B,
      glutamine-hydrolyzing - Vibrio cholerae. (554 aa)

<400> 83
Met Cys Ser Ile Phe Gly Ile Leu Asp Ile Lys Thr Asp Ala Ala Thr
1               5                   10                  15

Leu Arg Pro Ile Ala Leu Glu Met Ser Lys Lys Leu Arg His Arg Gly
            20                  25                  30

Pro Asp Trp Ser Gly Ile Tyr Ala Ser Asp Lys Ala Ile Leu Ala His
        35                  40                  45

Glu Arg Leu Ala Ile Val Gly Leu Asn Ser Gly Ala Gln Pro Leu Tyr
    50                  55                  60

Ser Pro Asp Arg Lys Leu Ile Leu Ala Val Asn Gly Glu Ile Tyr Asn
65                  70                  75                  80

His Lys Glu Ile Arg Glu Arg Tyr Lys Gly Gln Tyr Glu Phe Gln Thr
                85                  90                  95

Asp Ser Asp Cys Glu Val Ile Leu Ala Leu Tyr Arg Asp Lys Gly Ala
            100                 105                 110

Asp Leu Leu Glu Glu Leu Asn Gly Ile Phe Ala Phe Val Leu Tyr Asp
        115                 120                 125

Glu Glu Lys Asp Glu Tyr Leu Ile Gly Arg Asp His Ile Gly Ile Ile
    130                 135                 140

Pro Leu Tyr Gln Gly His Asp Glu His Gly Asn Phe Tyr Val Ala Ser
145                 150                 155                 160

Glu Met Lys Ala Leu Val Pro Val Cys Lys Thr Leu Ser Glu Phe Pro
                165                 170                 175

Pro Gly Ser Tyr Leu Ser Ser Lys Asp Gln Ala Val Thr Arg Tyr Tyr
            180                 185                 190

Val Arg Asp Trp Asn Ser Phe Asp Glu Val Lys Asp Asn Val Ser Ser
        195                 200                 205

Lys Glu Glu Leu Thr Gln Ala Leu Glu Ala Ala Val Lys Arg Gln Leu
    210                 215                 220

Met Thr Asp Val Pro Tyr Gly Val Leu Leu Ser Gly Gly Leu Asp Ser
225                 230                 235                 240

Ser Ile Thr Ser Ala Ile Ala Lys Arg Phe Ala Ala Met Arg Ile Glu
                245                 250                 255

Asp Asp Glu Lys Ser Ala Ala Trp Trp Pro Gln Leu His Ser Phe Ala
            260                 265                 270

Ile Gly Leu Glu Gly Ala Pro Asp Leu Lys Ala Ala Arg Glu Val Ala
        275                 280                 285

Glu Lys Ile Gly Thr Val His His Glu Met Thr Tyr Thr Ile Gln Glu
    290                 295                 300

Gly Leu Asp Ala Ile Arg Asp Val Ile Tyr His Ile Glu Thr Tyr Asp
305                 310                 315                 320

Val Thr Thr Ile Arg Ala Ser Thr Pro Met Phe Leu Met Gly Arg Lys
                325                 330                 335

Ile Lys Ala Met Gly Ile Lys Met Val Leu Ser Gly Glu Gly Ala Asp
            340                 345                 350

Glu Ile Phe Gly Gly Tyr Leu Tyr Phe His Lys Ala Pro Asn Ala Lys
        355                 360                 365

Glu Phe His Glu Glu Thr Val Arg Lys Leu Leu Ala Leu Asn Leu Phe
    370                 375                 380

Asp Cys Ala Arg Ala Asn Lys Ser Leu Ala Ala Trp Gly Val Glu Gly
385                 390                 395                 400

Arg Val Pro Phe Leu Asp Lys Glu Phe Ile Asp Val Ala Met Arg Leu
                405                 410                 415

Asn Pro Ala Asp Lys Met Cys Gly Asn Gly Lys Met Glu Lys His Ile

420                 425                 430

Leu Arg Glu Cys Phe Glu His Tyr Leu Pro Glu Ser Ile Ala Trp Arg
        435                 440                 445

Gln Lys Glu Gln Phe Ser Asp Gly Val Gly Tyr Gly Trp Ile Asp Thr
    450                 455                 460

Leu Lys Ala Thr Ala Glu Ala Lys Ile Ser Asp Gln Gln Met Glu Thr
465                 470                 475                 480

Ala Lys Phe Arg Phe Pro Tyr Asn Thr Pro Thr Thr Lys Glu Gly Tyr
                485                 490                 495

Val Tyr Arg Glu Ile Phe Glu Glu Leu Phe Pro Leu Glu Ser Ala Ala
            500                 505                 510

Arg Cys Val Pro Gly Gly Pro Ser Val Ala Cys Ser Ser Ala Lys Ala
        515                 520                 525

Ile Glu Trp Asp Glu Ser Phe Lys Asn Cys Ile Asp Pro Ser Gly Arg
    530                 535                 540

Ala Val Lys Ala Val His Lys Gln Ala Tyr
545                 550


<210> 84
<211> 64
<212> PRT
<213> Vibrio cholerae

<220>
<221> PEPTIDE
<222> (1)..(64)
<223> >sp|O68845|RL35_VIBCH 50S ribosomal protein L35 -
      Vibrio cholerae. (64 aa)

<400> 84
Met Pro Lys Met Lys Asn Asn Lys Gly Ala Ala Lys Arg Phe Lys Lys
1               5                   10                  15

Thr Ala Gly Gly Ile Lys Tyr Lys His Ala Thr Lys Arg His Ile Leu
            20                  25                  30

Thr Lys Arg Thr Thr Lys Asn Lys Arg Gln Leu Arg Pro Asn Ala Ile
        35                  40                  45

Leu Pro Lys Cys Glu Leu Ala Ala Val Ala Arg Met Leu Pro Tyr Ala
    50                  55                  60


<210> 85
<211> 681
<212> PRT
<213> Vibrio cholerae

<220>

<221> PEPTIDE
<222> (1)..(681)
<223> >tr|Q9KP17|Q9KP17_VIBCH 2`,3`-cyclic-nucleotide
      2`-phosphodiesterase - Vibrio cholerae. (681 aa)

<400> 85
Met Lys Pro Leu Phe His Arg Leu Leu Leu His Leu Ser Ser Cys Phe
1               5                   10                  15

Tyr Phe Leu Leu Leu Ser Leu Ile Leu Glu Ser Val Met Thr Met Met
            20                  25                  30

Ile Lys Pro Val Ser Leu Ala Val Leu Gly Gly Leu Leu Ala Met Ala
        35                  40                  45

Gly Pro Val Met Ala Asp Thr Ile Lys Leu Arg Ile Val Glu Thr Thr
    50                  55                  60

Asp Ile His Thr Asn Leu Met Asp Tyr Asp Tyr Tyr Lys Asp Lys Ala
65                  70                  75                  80

Ser Asp Gln Ile Gly Leu Thr Arg Ala Ala Thr Leu Val Lys Gln Ala
                85                  90                  95

Arg Ala Glu Val Glu Asn Ser Val Leu Val Asp Asn Gly Asp Leu Ile
            100                 105                 110

Gln Gly Ser Pro Met Gly Asp Tyr Met Ala Ala Lys Gly Ile Lys Ala
        115                 120                 125

Gly Glu Val His Pro Val Tyr Lys Ala Met Asn Pro Leu Gly Tyr Asp
    130                 135                 140

Val Gly Asn Ile Gly Asn His Glu Phe Asn Tyr Gly Leu Glu Phe Leu
145                 150                 155                 160

Lys Glu Thr Ile Asn Asp Ala Ala Phe Pro Tyr Ile Asn Ala Asn Val
                165                 170                 175

Phe Asp Ala Lys Thr Gly Gln His Leu Phe Thr Pro Tyr Val Ile Lys
            180                 185                 190

Glu His Ser Phe Lys Asp Thr Asp Gly Glu Ala His Thr Ile Lys Val
        195                 200                 205

Gly Tyr Ile Gly Phe Val Pro Pro Gln Ile Met Val Trp Asp Lys Lys
    210                 215                 220

Asn Leu Glu Gly Lys Val Thr Ala Ala Asp Ile Lys Gln Thr Ala Glu
225                 230                 235                 240

Lys Leu Ile Pro Glu Met Lys Ala Lys Gly Ala Asp Val Ile Val Ala
                245                 250                 255

Ile Pro His Ser Gly Ile Ser Thr Asp Glu Tyr Gln Ala Gly Ala Glu
            260                 265                 270

Asn Ser Val Tyr Tyr Leu Thr Gln Val Lys Gly Ile Asp Ala Ile Ala
        275                 280                 285

Phe Gly His Ser His Ala Val Phe Pro Gly Lys Asp Phe Ala Asn Val

290                 295                 300

Pro Gly Ala Asp Ile Asp Lys Gly Thr Ile Asn Gly Val Thr Ala Val
305                 310                 315                 320

Met Pro Gly Arg Trp Gly Ser His Val Gly Val Met Asp Leu Thr Leu
                325                 330                 335

Glu Lys Gln Gln Gly Arg Trp Ala Val Thr Ser Gly Gln Ser Glu Ala
            340                 345                 350

Arg Pro Ile Phe Asp Lys Ala Asn Lys Lys Ala Leu Val Glu Ala Asp
        355                 360                 365

Ala Thr Met Val Gln Ala Leu Gln His Asp His Gln Gly Thr Arg Asp
    370                 375                 380

Phe Val Asn Gln Pro Ile Gly Lys Ala Asn Asp Val Met Tyr Ser Phe
385                 390                 395                 400

Leu Ser Leu Val Gln Asp Asp Pro Thr Val Gln Ile Val Asn Leu Ala
                405                 410                 415

Gln Lys Asp Tyr Val Glu Arg Phe Ile Gln Gly Asp Pro Asn Leu Asp
            420                 425                 430

Gly Leu Pro Val Leu Ser Ala Ala Ala Pro Phe Lys Ala Gly Gly Arg
        435                 440                 445

Lys Asp Asp Pro Asn Gly Phe Thr Glu Val Glu Ala Gly Glu Leu Thr
    450                 455                 460

Phe Arg Asn Ala Ala Asp Leu Tyr Leu Tyr Pro Asn Thr Leu Val Thr
465                 470                 475                 480

Met Lys Val Ser Gly Gln Gln Leu Lys Glu Trp Leu Glu Cys Ser Ala

                485                 490                 495

Gly Gln Phe Asn Gln Ile Asp Val Thr Ser Ser Ala Pro Gln Ser Leu
            500                 505                 510

Ile Asn Trp Asp Gly Phe Arg Thr Tyr Asn Phe Asp Val Ile Asp Gly
        515                 520                 525

Val Asn Tyr Gln Ile Asp Val Thr Gln Pro Ala Arg Tyr Asp Gly Asp
    530                 535                 540

Cys Lys Leu Ile Asn Pro Asn Ser Gln Arg Ile Val Gln Leu Thr Tyr
545                 550                 555                 560

Gln Gly Lys Pro Ile Asp Thr Gln Gln Thr Phe Leu Ile Ala Thr Asn
                565                 570                 575

Asn Tyr Arg Ala Tyr Ser Asn Lys Phe Pro Gly Thr Gly Ala Glu Phe
            580                 585                 590

Val Ala Phe Asp Ser Pro Asp Glu Asn Arg Thr Val Val Ala Asp Tyr
        595                 600                 605

Ile Ala Arg Val Ser Lys Asp Lys Gly Glu Val Thr Pro Ser Ala Asp

610                 615                 620

Asn Asn Trp Ser Phe Ala Pro Ile Ser Ser Lys Thr Lys Leu Asp Ile
625                 630                 635                 640

Arg Phe Glu Thr Ser Pro Ser Asp Lys Ala Thr Gln Phe Ile Lys Gln
                645                 650                 655

Lys Gly Gln Tyr Pro Met Thr Arg Val Ala Ser Asp Asp Val Gly Phe
            660                 665                 670

Ala Val Tyr Arg Ile Asp Leu Gln Lys
        675                 680


<210> 86
<211> 469
<212> PRT
<213> Vibrio cholerae

<220>
<221> PEPTIDE
<222> (1)..(469)
<223> >sp|Q9KNJ2|GLNA_VIBCH Glutamine synthetase (EC
      6.3.1.2) (Glutamate--ammonia ligase) - Vibrio
      cholerae. (469 aa)

<400> 86
Met Ser Val Glu Asn Val Leu Ser Leu Ile Gln Glu Asn Glu Val Lys
1               5                   10                  15

Phe Val Asp Leu Arg Phe Thr Asp Thr Lys Gly Lys Glu Gln His Ile
            20                  25                  30

Ser Ile Pro Ala His Gln Ile Asp Ala Asp Phe Phe Glu Asp Gly Lys
        35                  40                  45

Met Phe Asp Gly Ser Ser Val Ala Gly Trp Lys Gly Ile Asn Glu Ser
    50                  55                  60

Asp Met Val Met Met Pro Asp Pro Ser Ser Ala Val Leu Asp Pro Phe
65                  70                  75                  80

Thr Glu Asp Ala Thr Leu Asn Ile Arg Cys Asp Ile Leu Glu Pro Ala
                85                  90                  95

Thr Met Gln Gly Tyr Asp Arg Asp Pro Arg Ser Ile Ala Lys Arg Ala
            100                 105                 110

Glu Glu Tyr Met Arg Ser Thr Gly Ile Ala Asp Thr Val Leu Val Gly
        115                 120                 125

Pro Glu Pro Glu Phe Phe Leu Phe Asp Asp Val Lys Phe Ala Thr Asn
    130                 135                 140

Met Ser Gly Ser Phe Phe Lys Ile Asp Asp Val Glu Ala Ala Trp Asn
145                 150                 155                 160

Thr Gly Thr Glu Tyr Glu Asp Gly Asn Lys Gly His Arg Pro Gly Val
                165                 170                 175

Lys Gly Gly Tyr Phe Pro Val Ala Pro Val Asp Ser Ser Gln Asp Ile
            180                 185                 190

Arg Ser Ala Met Cys Leu Ile Met Glu Glu Met Gly Leu Val Val Glu
        195                 200                 205

Ala His His His Glu Val Ala Thr Ala Gly Gln Asn Glu Ile Ala Thr
    210                 215                 220

Arg Phe Asn Thr Leu Thr Thr Lys Ala Asp Glu Ile Gln Ile Tyr Lys
225                 230                 235                 240

Tyr Val Val His Asn Val Ala His Ala Phe Gly Lys Thr Ala Thr Phe
                245                 250                 255

Met Pro Lys Pro Leu Val Gly Asp Asn Gly Ser Gly Met His Val His
            260                 265                 270

Gln Ser Leu Ala Lys Asp Gly Val Asn Leu Phe Ala Gly Asp Lys Tyr
        275                 280                 285

Gly Gly Leu Ser Glu Thr Ala Leu Tyr Tyr Ile Gly Gly Ile Ile Lys
    290                 295                 300

His Ala Arg Ala Leu Asn Ala Ile Thr Asn Pro Ser Thr Asn Ser Tyr
305                 310                 315                 320

Lys Arg Leu Val Pro His Tyr Glu Ala Pro Val Met Leu Ala Tyr Ser
                325                 330                 335

Ala Arg Asn Arg Ser Ala Ser Ile Arg Ile Pro Val Val Pro Ser Pro
            340                 345                 350

Lys Ala Arg Arg Ile Glu Val Arg Phe Pro Asp Pro Ala Ala Asn Pro
        355                 360                 365

Tyr Leu Ala Phe Ala Ala Met Leu Met Ala Gly Leu Asp Gly Ile Lys
    370                 375                 380

Asn Lys Ile His Pro Gly Glu Ala Met Asp Lys Asp Leu Tyr Asp Leu
385                 390                 395                 400

Pro Ala Glu Glu Ala Ala Glu Ile Pro Lys Val Ala Glu Ser Leu Gln
                405                 410                 415

Gln Ala Leu Gln Tyr Leu Asp Ala Asp Arg Glu Phe Leu Thr Ala Gly
            420                 425                 430

Gly Val Phe Ser Asp Asp Phe Ile Asp Ser Tyr Ile Ala Leu Lys Thr
        435                 440                 445

Lys Asp Val Glu Arg Val Asn Val Ala Val His Pro Leu Glu Phe Glu
    450                 455                 460

Leu Tyr Tyr Ser Val
465

<210> 87

<211> 123
<212> PRT
<213> Vibrio cholerae

<220>
<221> PEPTIDE
<222> (1)..(123)
<223> >tr|Q9KNZ4|Q9KNZ4_VIBCH Ribosomal protein L14 -
      Vibrio cholerae. (123 aa)

<400> 87
Met Ile Gln Met Gln Thr Met Leu Asp Ala Ala Asp Asn Ser Gly Ala
1               5                   10                  15

Arg Ser Val Met Cys Ile Lys Val Leu Gly Gly Ser His Arg Arg Tyr
            20                  25                  30

Ala His Val Gly Asp Ile Ile Lys Val Thr Val Lys Glu Ala Ile Pro
        35                  40                  45

Arg Gly Lys Val Lys Lys Gly Asp Val Leu Lys Ala Val Val Val Arg
    50                  55                  60

Thr Arg Lys Gly Val Arg Arg Pro Asp Gly Ser Val Ile Arg Phe Asp
65                  70                  75                  80

Arg Asn Ala Cys Val Leu Leu Asn Asn Asn Ser Glu Gln Pro Ile Gly
                85                  90                  95

Thr Arg Ile Phe Gly Pro Val Thr Arg Glu Leu Arg Asn Ala Lys Phe
            100                 105                 110

Met Lys Ile Val Ser Leu Ala Pro Glu Val Leu
        115                 120


<210> 88
<211> 1076
<212> PRT
<213> Vibrio cholerae

<220>
<221> PEPTIDE
<222> (1)..(1076)
<223> >sp|Q9KPH9|CARB_VIBCH Carbamoyl-phosphate synthase
      large chain (EC 6.3.5.5) (Carbamoyl- phosphate
      synthetase ammonia chain) - Vibrio cholerae. (1076
      aa)

<400> 88
Met Pro Lys Arg Thr Asp Ile Gln Ser Ile Leu Ile Leu Gly Ala Gly
1               5                   10                  15

Pro Ile Val Ile Gly Gln Ala Cys Glu Phe Asp Tyr Ser Gly Ala Gln
            20                  25                  30

Ala Cys Lys Ala Leu Arg Glu Glu Gly Tyr Arg Val Ile Leu Val Asn
        35                  40                  45

Ser Asn Pro Ala Thr Ile Met Thr Asp Pro Glu Met Ala Asp Ala Thr
    50                  55                  60

Tyr Ile Glu Pro Ile His Trp Glu Val Val Arg Lys Ile Ile Glu Lys
65                  70                  75                  80

Glu Arg Pro Asp Ala Ile Leu Pro Thr Met Gly Gly Gln Thr Ala Leu
                85                  90                  95

Asn Cys Ala Leu Ala Leu Glu Lys His Gly Val Leu Ala Glu Phe Gly
            100                 105                 110

Val Glu Met Ile Gly Ala Thr Ala Asp Ala Ile Asp Lys Ala Glu Asp
        115                 120                 125

Arg Ser Arg Phe Asp Lys Ala Met Lys Ser Ile Gly Leu Glu Cys Pro
    130                 135                 140

Arg Ala Asp Thr Ala Lys Ser Met Glu Glu Ala Tyr Lys Val Leu Asp
145                 150                 155                 160

Met Val Gly Phe Pro Cys Ile Ile Arg Pro Ser Phe Thr Met Gly Gly
                165                 170                 175

Ser Gly Gly Gly Ile Ala Tyr Asn Arg Glu Glu Phe Glu Glu Ile Cys
            180                 185                 190

Thr Arg Gly Leu Asp Leu Ser Pro Thr Asn Glu Leu Leu Ile Asp Glu
        195                 200                 205

Ser Leu Ile Gly Trp Lys Glu Tyr Glu Met Glu Val Val Arg Asp Lys
    210                 215                 220

Asn Asp Asn Cys Ile Ile Val Cys Ala Ile Glu Asn Phe Asp Pro Met
225                 230                 235                 240

Gly Ile His Thr Gly Asp Ser Ile Thr Val Ala Pro Ala Gln Thr Leu
                245                 250                 255

Thr Asp Lys Glu Tyr Gln Ile Met Arg Asn Ala Ser Leu Ala Val Leu
            260                 265                 270

Arg Glu Ile Gly Val Glu Thr Gly Gly Ser Asn Val Gln Phe Gly Ile
        275                 280                 285

Asn Pro Lys Asp Gly Arg Met Val Ile Ile Glu Met Asn Pro Arg Val
    290                 295                 300

Ser Arg Ser Ser Ala Leu Ala Ser Lys Ala Thr Gly Phe Pro Ile Ala
305                 310                 315                 320

Lys Val Ala Ala Lys Leu Ala Val Gly Phe Thr Leu Asp Glu Leu Met
                325                 330                 335

Asn Asp Ile Thr Gly Gly Ala Thr Pro Ala Ser Phe Glu Pro Thr Ile
            340                 345                 350

Asp Tyr Val Val Thr Lys Ile Pro Arg Phe Asn Phe Glu Lys Phe Ala
        355                 360                 365

Gly Ala Asn Asp Arg Leu Thr Thr Gln Met Lys Ser Val Gly Glu Val
    370                 375                 380

Met Ala Ile Gly Arg Asn Gln Gln Glu Ser Leu Gln Lys Ala Leu Arg
385                 390                 395                 400

Gly Leu Glu Val Gly Ala Ala Gly Leu Asp Glu Lys Val Asp Leu Asp
                405                 410                 415

Ala Pro Asp Ala Leu Thr Lys Ile Arg Tyr Glu Leu Lys Glu Ala Gly
            420                 425                 430

Ala Glu Arg Ile Trp Tyr Ile Ala Asp Ala Phe Arg Ala Gly Met Ser
        435                 440                 445

Val Asp Gly Val Phe Asn Leu Thr Asn Ile Asp Arg Trp Phe Leu Val
    450                 455                 460

Gln Ile Glu Glu Leu Val Lys Leu Glu Ala Glu Val Lys Ala Gly Gly
465                 470                 475                 480

Phe Ala Gly Leu Asn Gln Asp Val Leu Arg Lys Met Lys Arg Lys Gly
                485                 490                 495

Phe Ser Asp Ala Arg Leu Ser Lys Leu Leu Gly Val Ser Glu Asn Glu
            500                 505                 510

Ile Arg Arg Leu Arg Asp Gln Tyr Asn Ile His Pro Val Tyr Lys Arg
        515                 520                 525

Val Asp Thr Cys Ala Ala Glu Phe Lys Ser Asp Thr Ala Tyr Met Tyr
    530                 535                 540

Ser Thr Tyr Asp Glu Glu Cys Glu Ala Asn Pro Thr Asp Lys Asp Lys
545                 550                 555                 560

Ile Met Val Leu Gly Gly Gly Pro Asn Arg Ile Gly Gln Gly Ile Glu
                565                 570                 575

Phe Asp Tyr Cys Cys Val His Ala Ala Leu Ala Leu Arg Glu Asp Gly
            580                 585                 590

Tyr Glu Thr Ile Met Val Asn Cys Asn Pro Glu Thr Val Ser Thr Asp
        595                 600                 605

Tyr Asp Thr Ser Asp Arg Leu Tyr Phe Glu Pro Val Thr Leu Glu Asp
    610                 615                 620

Val Leu Ala Ile Val Arg Val Glu Lys Pro Lys Gly Val Ile Val Gln
625                 630                 635                 640

Tyr Gly Gly Gln Thr Pro Leu Lys Leu Ala Arg Ala Leu Glu Ala Ala
                645                 650                 655

Gly Val Pro Val Ile Gly Thr Ser Pro Asp Ala Ile Asp Arg Ala Glu
            660                 665                 670

Asp Arg Glu Arg Phe Gln Gln Ala Val Gln Arg Leu Gly Leu Lys Gln
        675                 680                 685

Pro Asp Asn Ala Thr Val Thr Ala Ile Glu Gln Ala Ile Glu Lys Ser
    690                 695                 700

Arg Glu Ile Gly Phe Pro Leu Val Val Arg Pro Ser Tyr Val Leu Gly

705                 710                 715                 720

Gly Arg Ala Met Glu Ile Val Tyr Asp Glu Gln Asp Leu Arg Arg Tyr
                725                 730                 735

Phe Asn Glu Ala Val Ser Val Ser Asn Glu Ser Pro Val Leu Leu Asp
            740                 745                 750

Arg Phe Leu Asp Asp Ala Thr Glu Val Asp Val Asp Ala Ile Cys Asp
        755                 760                 765

Gly Glu Arg Val Val Ile Gly Gly Ile Met Glu His Ile Glu Gln Ala
    770                 775                 780

Gly Val His Ser Gly Asp Ser Ala Cys Ser Leu Pro Ala Tyr Thr Leu
785                 790                 795                 800

Ser Gln Glu Ile Gln Asp Lys Met Arg Glu Gln Val Glu Lys Leu Ala
                805                 810                 815

Phe Glu Leu Gly Val Arg Gly Leu Met Asn Ile Gln Phe Ala Val Lys
            820                 825                 830

Asp Asn Glu Val Tyr Leu Ile Glu Val Asn Pro Arg Ala Ala Arg Thr
        835                 840                 845

Val Pro Phe Val Ser Lys Ala Thr Gly Ala Pro Leu Ala Lys Ile Ala
    850                 855                 860

Ala Arg Val Met Val Gly Gln Thr Leu Glu Gln Gln Gly Phe Thr Lys
865                 870                 875                 880

Glu Ile Ile Pro Pro Tyr Tyr Ser Val Lys Glu Val Val Leu Pro Phe
                885                 890                 895

Asn Lys Phe Pro Gly Val Asp Pro Leu Leu Gly Pro Glu Met Arg Ser
            900                 905                 910

Thr Gly Glu Val Met Gly Val Gly Ala Thr Phe Ala Glu Ala Tyr Ala
        915                 920                 925

Lys Ala Glu Leu Gly Cys Gly Ser Val Tyr Pro Glu Gly Gly Arg Ala
    930                 935                 940

Leu Leu Ser Val Arg Glu Gly Asp Lys Gln Arg Val Val Asp Leu Ala
945                 950                 955                 960

Ser Lys Leu Val Lys Leu Gly Tyr Gln Leu Asp Ala Thr His Gly Thr
                965                 970                 975

Ala Val Ile Leu Gly Glu Ala Gly Ile Asn Pro Arg Leu Val Asn Lys
            980                 985                 990

Val His Glu Gly Arg Pro His Ile Leu Asp Arg Ile Lys Asn His Glu
        995                 1000                1005

Tyr Thr Tyr Ile Val Asn Thr Ala Ser Gly Arg Gln Ala Ile Glu Asp
    1010                1015                1020

Ser Lys Val Leu Arg Arg Gly Ala Leu Ala His Lys Val Asn Tyr Thr
1025                1030                1035                1040

Thr Thr Leu Asn Ala Ala Phe Ala Thr Cys Met Ser His Thr Ala Asp
                1045                1050                1055

Ala Lys Ala Ser Val Thr Ser Val Gln Glu Leu His Ala Arg Val Lys
            1060                1065                1070

Ala Asn Gln Ala
        1075

<210> 89
<211> 695
<212> PRT
<213> Vibrio cholerae

<220>
<221> PEPTIDE
<222> (1)..(695)
<223> >sp|Q9KPM5|EFG2_VIBCH Elongation factor G 2 (EF-G
      2) - Vibrio cholerae. (695 aa)

<400> 89
Met Ala Asp Leu Ser Leu Tyr Arg Asn Ile Gly Ile Phe Ala His Val
1               5                   10                  15

Asp Ala Gly Lys Thr Thr Thr Thr Glu Arg Ile Leu Lys Leu Thr Gly
            20                  25                  30

Lys Ile His Arg Leu Gly Glu Val His Asp Gly Ala Ser Thr Met Asp
        35                  40                  45

Phe Met Glu Gln Glu Ala Glu Arg Gly Ile Thr Ile Gln Ser Ala Ala
    50                  55                  60

Thr Thr Cys Phe Trp Lys Gly His Arg Phe Asn Val Ile Asp Thr Pro
65                  70                  75                  80

Gly His Val Asp Phe Thr Val Glu Val Tyr Arg Ser Leu Lys Val Leu
                85                  90                  95

Asp Gly Gly Ile Gly Val Phe Cys Gly Ser Gly Gly Val Glu Pro Gln
            100                 105                 110

Ser Glu Thr Asn Trp Arg Tyr Ala Asn Glu Ser Glu Val Ser Arg Leu
        115                 120                 125

Ile Phe Val Asn Lys Leu Asp Arg Met Gly Ala Asp Phe Phe Arg Val
    130                 135                 140

Val Glu Gln Val Lys Lys Val Leu Gly Ala Asn Pro Leu Val Met Thr
145                 150                 155                 160

Leu Pro Ile Gly Arg Glu Asp Glu Phe Val Gly Val Val Asp Val Leu
                165                 170                 175

Thr Arg Gln Ala Tyr Val Trp Asp Asp Ser Gly Leu Pro Glu Asn Phe
            180                 185                 190

Glu Val Lys Glu Val Pro Ala Asp Met Val Asp Gln Val Glu Glu Tyr
        195                 200                 205

Arg Glu Met Met Ile Glu Thr Ala Val Glu Gln Asp Asp Glu Leu Met
    210                 215                 220

Met Ala Tyr Met Glu Gly Glu Glu Pro Thr Val Glu Gln Ile Lys Ala
225                 230                 235                 240

Cys Ile Arg Lys Gly Thr Arg Asp Leu Ala Phe Phe Pro Thr Phe Cys
                245                 250                 255

Gly Ser Ala Phe Lys Asn Lys Gly Met Gln Leu Val Leu Asp Ala Val
            260                 265                 270

Val Asp Tyr Leu Pro Ser Pro Thr Glu Val Glu Pro Gln Pro Leu Thr
        275                 280                 285

Asp Pro Ala Thr Gly Glu Pro Thr Gly Glu Val Ala Thr Val Ser Val
    290                 295                 300

Asp Ala Pro Leu Lys Ala Leu Ala Phe Lys Ile Met Asp Asp Arg Phe
305                 310                 315                 320

Gly Ala Leu Thr Phe Val Arg Ile Tyr Ser Gly Lys Ile Lys Lys Gly
                325                 330                 335

Asp Thr Ile Leu Asn Ser Ala Thr Gly Lys Thr Glu Arg Ile Gly Arg
            340                 345                 350

Met Val Glu Met His Ala Asn Asp Arg Asn Glu Val Glu Ser Ala Gln
        355                 360                 365

Ala Ser Asp Ile Ile Ala Ile Val Gly Met Lys Asn Val Gln Thr Gly
    370                 375                 380

His Thr Leu Cys Asp Pro Lys His Glu Cys Thr Leu Glu Pro Met Ile
385                 390                 395                 400

Phe Pro Thr Pro Val Ile Ser Ile Ala Val Lys Pro Lys Asp Lys Asn
                405                 410                 415

Gly Ser Glu Lys Met Gly Ile Ala Ile Gly Lys Met Val Ala Glu Asp
            420                 425                 430

Pro Ser Phe Gln Val Glu Thr Asp Glu Asp Ser Gly Glu Thr Ile Leu
        435                 440                 445

Lys Gly Met Gly Glu Leu His Leu Asp Ile Lys Val Asp Ile Leu Lys
    450                 455                 460

Arg Thr Tyr Gly Val Glu Leu Glu Val Gly Ala Pro Gln Val Ala Tyr
465                 470                 475                 480

Arg Glu Thr Ile Thr Lys Ala Val Glu Asp Ser Tyr Thr His Lys Lys
                485                 490                 495

Gln Ser Gly Gly Ser Gly Gln Phe Gly Lys Ile Asp Tyr Arg Ile Arg
            500                 505                 510

Pro Gly Glu Gln Asn Ser Gly Phe Thr Phe Lys Ser Thr Val Val Gly
        515                 520                 525

Gly Asn Val Pro Lys Glu Phe Trp Pro Ala Val Glu Lys Gly Phe Lys
    530                 535                 540

Ser Met Met Asp Thr Gly Thr Leu Ala Gly Phe Pro Val Leu Asp Val
545                 550                 555                 560

Glu Val Glu Leu Phe Asp Gly Gly Phe His Ala Val Asp Ser Ser Ala
                565                 570                 575

Ile Ala Phe Glu Ile Ala Ala Lys Gly Ala Phe Arg Gln Ser Ile Pro
            580                 585                 590

Lys Ala Ala Pro Gln Leu Leu Glu Pro Ile Met Lys Val Asp Val Phe
        595                 600                 605

Thr Pro Glu Asp His Val Gly Asp Val Ile Gly Asp Leu Asn Arg Arg
    610                 615                 620

Arg Gly Met Ile Lys Asp Gln Glu Met Gly Leu Thr Gly Val Arg Val
625                 630                 635                 640

Lys Ala Asp Val Pro Leu Ser Glu Met Phe Gly Tyr Ile Gly Ser Leu
                645                 650                 655

Arg Thr Met Thr Ser Gly Arg Gly Gln Phe Ser Met Glu Phe Ser His
            660                 665                 670

Tyr Ala Pro Cys Pro Asn Asn Val Ala Glu Gln Val Ile Ala Glu Val
        675                 680                 685

Lys Glu Arg Asn Ala Lys Lys
    690                 695


<210> 90
<211> 898
<212> PRT
<213> Vibrio cholerae

<220>
<221> PEPTIDE
<222> (1)..(898)
<223> >sp|Q9KU80|IF2_VIBCH Translation initiation factor
      IF-2 - Vibrio cholerae. (898 aa)

<400> 90
Met Thr Gln Ile Thr Val Lys Ala Leu Ser Glu Glu Ile Gly Thr Pro
1               5                   10                  15

Val Asp Arg Leu Leu Glu Gln Leu Ala Asp Ala Gly Met Asn Lys Ala
            20                  25                  30

Val Ala Asp His Val Ser Glu Asp Glu Lys Gln Lys Leu Leu Ala His
        35                  40                  45

Leu Arg Lys Glu His Gly Asp Ala Thr Gly Ser Glu Pro Thr Arg Leu
    50                  55                  60

Thr Leu Gln Arg Lys Thr Arg Ser Thr Leu Ser Val Asn Ala Gly Gly
65                  70                  75                  80

Gly Lys Ser Lys Asn Val Gln Val Glu Val Arg Lys Lys Arg Thr Tyr
                85                  90                  95

Val Lys Arg Ser Ser Val Glu Asp Glu Ala Thr Arg Glu Ala Glu Glu
            100                 105                 110

Ala Ala Met Arg Ala Ala Glu Glu Gln Ala Lys Arg Glu Ala Glu Glu
        115                 120                 125

Ala Ala Gln Arg Ala Ala Glu Glu Lys Ala Lys Arg Glu Ala Glu Glu
    130                 135                 140

Ala Ala Lys Arg Glu Ala Glu Ala Lys Arg Met Ala Glu Glu Lys Ala
145                 150                 155                 160

Lys Arg Glu Thr Gln Ala Ala Thr Gln Pro Arg Ser Asp Glu Glu Lys
                165                 170                 175

Leu Lys Gln Glu Ala Ala Arg Lys Glu Ala Glu Ala Leu Lys Arg Arg
            180                 185                 190

Gln Glu Glu Glu Ala Arg Arg Lys Ala Glu Glu Glu Ser Arg Arg Gln
        195                 200                 205

Leu Glu Lys Val Arg Glu Leu Ala Glu Lys Asn Gly Glu Arg Trp Ser
    210                 215                 220

Ala Asp Lys Glu Thr Val Gly Asp Met Gln Glu Asn Thr Asp Tyr His
225                 230                 235                 240

Val Thr Thr Ser Arg Tyr Ala Arg Glu Ala Glu Asp Glu Ala Asp Leu
                245                 250                 255

His Glu Glu Gly Ala Arg Arg Arg Ser Thr Lys Ala Asn Lys Arg Lys
            260                 265                 270

Met Ser Ser Arg Asp Asp Asn Gln Glu Arg Asp Ser Arg Pro Arg Gly
        275                 280                 285

Gly Lys Ala Gly Arg Lys Gly Arg Ile Asn Lys Pro Met Ser Met Gln
    290                 295                 300

His Gly Phe Asp Lys Thr Ala Val Val Ala Lys Ala Asp Val Val Val
305                 310                 315                 320

Gly Glu Thr Ile Val Val Ser Glu Leu Ala Gln Lys Met Ser Val Lys
                325                 330                 335

Ala Thr Glu Val Ile Lys Val Met Met Lys Met Gly Ala Met Ala Thr
            340                 345                 350

Ile Asn Gln Val Ile Asp Gln Glu Thr Ala Gln Leu Val Ala Glu Glu
        355                 360                 365

Met Gly His Lys Val Val Leu Arg Lys Glu Asn Glu Leu Glu Glu Ala
    370                 375                 380

Ile Leu Ser Asp Arg Asp Asp Lys Phe Glu Glu Val Ser Arg Ala Pro
385                 390                 395                 400

Val Val Thr Ile Met Gly His Val Asp His Gly Lys Thr Ser Thr Leu

                405                 410                 415

Asp Tyr Ile Arg Arg Thr His Val Ala Ser Gly Glu Ala Gly Gly Ile
            420                 425                 430

Thr Gln His Ile Gly Ala Tyr His Val Glu Thr Pro Asn Gly Met Ile
        435                 440                 445

Thr Phe Leu Asp Thr Pro Gly His Ala Ala Phe Thr Ala Met Arg Ala
    450                 455                 460

Arg Gly Ala Gln Ala Thr Asp Ile Val Val Leu Val Val Ala Ala Asp
465                 470                 475                 480

Asp Gly Val Met Pro Gln Thr Val Glu Ala Ile Gln His Ala Lys Ala
                485                 490                 495

Ala Gly Val Pro Leu Ile Val Ala Val Asn Lys Ile Asp Lys Asp Thr
            500                 505                 510

Ala Asn Pro Asp Asn Val Lys Thr Glu Leu Ser Gln Tyr Asn Val Met
        515                 520                 525

Pro Glu Glu Trp Gly Gly Asp Asn Met Phe Val His Ile Ser Ala Lys
    530                 535                 540

Gln Gly Thr Asn Ile Asp Gly Leu Leu Glu Ala Ile Leu Leu Gln Ala
545                 550                 555                 560

Glu Val Leu Glu Leu Lys Ala Val Lys Gln Gly Met Ala Ser Gly Val
                565                 570                 575

Val Ile Glu Ser Arg Leu Asp Lys Gly Arg Gly Pro Val Ala Thr Val
            580                 585                 590

Leu Val Gln Ser Gly Thr Leu Arg Lys Gly Asp Ile Val Leu Cys Gly
        595                 600                 605

Gln Glu Tyr Gly Arg Val Arg Ala Met Arg Asp Glu Val Gly Asn Glu
    610                 615                 620

Val Glu Glu Ala Gly Pro Ser Ile Pro Val Glu Ile Leu Gly Leu Ser
625                 630                 635                 640

Gly Val Pro Ala Ala Gly Asp Glu Ala Thr Val Val Arg Asp Glu Arg
                645                 650                 655

Lys Ala Arg Glu Val Ala Asn Tyr Arg Ala Gly Lys Phe Arg Glu Val
            660                 665                 670

Lys Leu Ala Arg Gln Gln Lys Ser Lys Leu Glu Asn Met Phe Ser Asn
        675                 680                 685

Met Thr Ala Gly Asp Val Ala Glu Leu Asn Ile Val Leu Lys Ala Asp
    690                 695                 700

Val Gln Gly Ser Val Glu Ala Ile Ala Asp Ser Leu Thr Lys Leu Ser
705                 710                 715                 720

Thr Asp Glu Val Lys Val Asn Ile Val Gly Ser Gly Val Gly Gly Ile
                725                 730                 735

Thr Glu Thr Asp Ala Val Leu Ala Ala Ala Ser Asn Ala Ile Val Val
            740                 745                 750

Gly Phe Asn Val Arg Ala Asp Ala Ser Ala Arg Arg Met Ile Glu Ala
        755                 760                 765

Glu Asn Ile Asp Leu Arg Tyr Tyr Ser Ile Ile Tyr Gln Leu Ile Asp
    770                 775                 780

Glu Val Lys Gln Ala Met Ser Gly Met Leu Ser Pro Glu Phe Lys Gln
785                 790                 795                 800

Glu Ile Ile Gly Leu Ala Glu Val Arg Asp Val Phe Lys Ser Pro Lys
                805                 810                 815

Leu Gly Ala Ile Ala Gly Cys Met Val Thr Glu Gly Val Ile Lys Arg
            820                 825                 830

Asn Ala Pro Ile Arg Val Leu Arg Asp Asn Val Val Ile Tyr Glu Gly
        835                 840                 845

Glu Leu Glu Ser Leu Arg Arg Phe Lys Asp Asp Val Ala Glu Val Lys
    850                 855                 860

Asn Gly Tyr Glu Cys Gly Ile Gly Val Lys Asn Tyr Asn Asp Val Arg
865                 870                 875                 880

Val Gly Asp Gln Ile Glu Val Phe Glu Thr Ile Glu Ile Gln Arg Thr
                885                 890                 895

Ile Asp

<210> 91
<211> 256
<212> PRT
<213> Vibrio cholerae

<220>
<221> PEPTIDE
<222> (1)..(256)
<223> >tr|Q9KTQ6|Q9KTQ6_VIBCH Accessory colonization
      factor AcfC - Vibrio cholerae. (256 aa)

<400> 91
Met Tyr Phe Met Lys Ser Lys Asn Arg Phe Leu Leu Ile Ser Leu Leu
1               5                   10                  15

Ser Phe Ser Thr Ser Val Phe Ala Asp Val Asn Leu Tyr Gly Pro Gly
            20                  25                  30

Gly Pro His Val Pro Leu Ile Lys Val Ala Glu Ser Phe Glu Lys Ser
        35                  40                  45

Gln Ser Lys Arg Val Asn Ile Thr Phe Gly Pro Gln Ala Thr Trp Asn
    50                  55                  60

Asp Lys Ala Lys Lys Asn Ala Asp Ile Leu Phe Gly Ala Ser Glu His
65                  70                  75                  80

Ser Ala Leu Ala Ile Ala Glu Gly His Ser Glu Arg Phe Ser Lys Phe
                85                  90                  95

Asn Ile His Pro Val Phe Met Arg Glu Ala Ile Ile Leu Val Lys Lys
            100                 105                 110

Gly Asn Pro Lys Asn Ile Lys Gly Met Ala Asp Leu Leu Lys Pro Gly
        115                 120                 125

Ile Gly Ile Val Val Asn Asp Gly Ala Gly Val Ser Asn Thr Ser Gly
    130                 135                 140

Thr Ala Val Trp Glu Asp Ser Val Gly Arg Met Lys Asn Val Glu Lys
145                 150                 155                 160

Leu Gln Ala Phe Arg Ser Asn Ile His Val Phe Ala Pro Asn Ser Gly
                165                 170                 175

Ser Ala Arg Lys Ala Phe Val Asp Gly Glu Asp Ile Asp Ala Trp Ile
            180                 185                 190

Thr Trp Val Asp Trp Ala Ile Ala Asn Pro Thr Ile Gly Asp Met Val
        195                 200                 205

Arg Met Glu Asp Glu Tyr Arg Ile Tyr Arg Asp Phe Asn Val Val Leu
    210                 215                 220

Ala Lys Asn Pro Ser Ser Glu Ala Ile Asp Phe Phe Asp Tyr Leu Thr
225                 230                 235                 240

Lys Ser Lys Asp Ala Glu Ala Ile Phe Gln His Tyr Gly Trp Phe Lys
                245                 250                 255

<210> 92
<211> 588
<212> PRT
<213> Vibrio cholerae

<220>
<221> PEPTIDE
<222> (1)..(588)
<223> >tr|Q9KQB1|Q9KQB1_VIBCH Succinate dehydrogenase,
      flavoprotein subunit - Vibrio cholerae. (588 aa)

<400> 92
Met Thr Ile Pro Val Arg Ser Phe Asp Ala Val Val Ile Gly Ala Gly
1               5                   10                  15

Gly Ala Gly Met Arg Ala Ala Leu Gln Ile Ser Glu Gln Gly Leu Thr
            20                  25                  30

Cys Ala Leu Leu Ser Lys Val Phe Pro Thr Arg Ser His Thr Val Ser
        35                  40                  45

Ala Gln Gly Gly Ile Thr Val Ala Leu Gly Asn Ser His Lys Asp Asp

50                  55                  60

Trp Gln Trp His Met Tyr Asp Thr Val Lys Gly Ser Asp Tyr Ile Gly
65                  70                  75                  80

Asp Gln Asn Ala Ile Glu Tyr Met Cys Lys Asn Gly Pro Glu Ser Val
                85                  90                  95

Ile Glu Leu Glu Arg Met Gly Leu Pro Phe Ser Arg Phe Glu Asn Gly
            100                 105                 110

Thr Ile Tyr Gln Arg Pro Phe Gly Gly Gln Ser Lys Glu Phe Gly Gly
        115                 120                 125

Glu Gln Ala Ala Arg Thr Ala Ala Ala Ala Asp Arg Thr Gly His Ala
    130                 135                 140

Leu Leu His Thr Leu Tyr Gln Gln Asn Val Lys His Lys Thr Thr Ile
145                 150                 155                 160

Phe Ser Glu Trp Tyr Ala Leu Asp Leu Val Lys Asn Gln Asp Gly Ala
                165                 170                 175

Val Leu Gly Cys Thr Ala Ile Cys Met Glu Thr Gly Glu Ile Cys Tyr
            180                 185                 190

Phe Lys Ala Lys Ala Thr Val Leu Ala Thr Gly Gly Ala Gly Arg Ile
        195                 200                 205

Tyr Ala Ser Thr Thr Asn Ala His Ile Asn Thr Gly Asp Gly Val Gly
    210                 215                 220

Met Ala Leu Arg Ala Gly Val Pro Met Gln Asp Met Glu Met Trp Gln
225                 230                 235                 240

Phe His Pro Thr Gly Ile Ala Gly Ala Gly Val Leu Val Thr Glu Gly
                245                 250                 255

Cys Arg Gly Glu Gly Gly Tyr Leu Leu Asn Lys Asp Gly Glu Arg Phe
            260                 265                 270

Met Glu Arg Tyr Ala Pro Asn Ala Lys Asp Leu Ala Gly Arg Asp Val
        275                 280                 285

Val Ala Arg Ser Met Met Ile Glu Ile Arg Glu Gly Arg Gly Cys Asp
    290                 295                 300

Gly Pro Trp Gly Pro His Ile Lys Leu Lys Leu Asp His Leu Gly Arg
305                 310                 315                 320

Asp Val Leu Glu Ser Arg Leu Pro Gly Ile Cys Glu Leu Ser Arg Thr
                325                 330                 335

Phe Ala His Val Asp Pro Val Lys Glu Pro Ile Pro Val Ile Pro Thr
            340                 345                 350

Cys His Tyr Met Met Gly Gly Val Pro Thr Gln Val Ser Gly Gln Ala
        355                 360                 365

Ile Lys Gln Asp Ser His Gly Lys Asp Val Glu Val Gln Gly Leu Phe

370                 375                 380

Ala Cys Gly Glu Ile Ala Ser Val Ser Val His Gly Ala Asn Arg Leu
385                 390                 395                 400

Gly Gly Asn Ser Leu Leu Asp Leu Val Val Phe Gly Arg Ala Thr Gly
                405                 410                 415

Leu His Leu Gly Glu Thr Leu Arg Ala Gln Ala Glu Ala Arg Pro Ala
            420                 425                 430

Thr Ala Ser Asp Ile Glu Ala Ser Leu Ala Arg Thr Met Arg Trp Glu
        435                 440                 445

Asn Ser Lys Gly Gly Glu Asp Pro Val Val Ile Arg Lys Asp Leu Gln
    450                 455                 460

Arg Cys Met Gln Asn Asn Phe Ser Val Phe Arg Glu Gly Lys Ala Met
465                 470                 475                 480

Ala Glu Gly Leu Glu Glu Leu Lys Val Ile Arg Glu Arg Leu Lys Asn
                485                 490                 495

Ala His Leu Ala Asp Lys Ser Thr Glu Phe Asn Thr Gln Arg Ile Glu
            500                 505                 510

Cys Leu Glu Leu Asp Asn Leu Met Glu Thr Ala Phe Ser Thr Ala Val
        515                 520                 525

Ala Ala Asn Tyr Arg Thr Glu Ser Arg Gly Ala His Ala Arg Phe Asp
    530                 535                 540

Tyr Pro Glu Arg Asp Asp Glu Asn Trp Leu Cys His Ser Ile Tyr Asn
545                 550                 555                 560

Pro Glu Thr Glu Gln Met Ser Lys Arg Glu Val Asn Met Thr Pro Val
                565                 570                 575

Tyr Arg Asp Pro Phe Pro Pro Lys Val Arg Thr Tyr
            580                 585

<210> 93
<211> 336
<212> PRT
<213> Vibrio cholerae

<220>
<221> PEPTIDE
<222> (1)..(336)
<223> >tr|Q9KP35|Q9KP35_VIBCH
      Fructose-1,6-bisphosphatase - Vibrio cholerae.
      (336 aa)

<400> 93
Met Gln Lys Met Arg Thr Leu Gly Glu Phe Ile Val Glu Lys Gln His
1               5                   10                  15

Asp Phe Pro His Ala Ser Gly Glu Leu Ser Ser Leu Leu Ala Ser Ile
            20                  25                  30

Arg Leu Ala Ala Lys Ile Val Asn Arg Glu Ile Asn Lys Ala Gly Leu
        35                  40                  45

Ala Asp Ile Ile Gly Ala Ser Gly Asn Asp Asn Ile Gln Gly Glu Glu
    50                  55                  60

Gln Gln Lys Leu Asp Leu Tyr Ala Asn Glu Lys Phe Lys Ala Ala Leu
65                  70                  75                  80

Glu Ala Arg Asp Gln Val Cys Gly Val Ala Ser Glu Glu Glu Asp Glu
                85                  90                  95

Ala Val Ala Phe Ser Lys Glu Leu Asn Lys Asn Ala Lys Tyr Val Val
            100                 105                 110

Leu Met Asp Pro Leu Asp Gly Ser Ser Asn Ile Asp Val Asn Val Ser
        115                 120                 125

Val Gly Thr Ile Phe Ser Ile Tyr Arg Arg Val Ser Pro Val Gly Thr
    130                 135                 140

Pro Pro Thr Gln Glu Asp Phe Leu Gln Pro Gly Asn Lys Gln Val Ala
145                 150                 155                 160

Ala Gly Tyr Val Ile Tyr Gly Ser Ser Thr Met Leu Val Tyr Thr Thr
                165                 170                 175

Gly Asn Gly Val Asn Gly Phe Thr Tyr Asp Pro Ser Leu Gly Thr Phe
            180                 185                 190

Tyr Leu Ser His Glu Asn Met Arg Ile Pro Glu Asn Gly Lys Ile Tyr
        195                 200                 205

Ser Ile Asn Glu Gly Asn Tyr Ile Arg Phe Pro Thr Gly Val Lys Lys
    210                 215                 220

Tyr Ile Lys Phe Cys Gln Glu Asn Val Pro Glu Glu Gly Arg Pro Tyr
225                 230                 235                 240

Thr Ser Arg Tyr Ile Gly Ser Leu Val Ala Asp Phe His Arg Asn Leu
                245                 250                 255

Leu Lys Gly Gly Ile Tyr Leu Tyr Pro Ser Thr Gln Ser His Pro Asn
            260                 265                 270

Gly Lys Leu Arg Leu Leu Tyr Glu Cys Asn Pro Met Ala Phe Leu Ile
        275                 280                 285

Glu Gln Ala Gly Gly Leu Ala Ser Asp Gly Ala Arg Arg Ile Met Asp
    290                 295                 300

Ile Lys Pro Thr Glu Leu His Gln Arg Val Pro Phe Phe Val Gly Ser
305                 310                 315                 320

Lys Asn Met Val His Lys Val Glu Thr Phe Leu Glu Thr Tyr Pro Asp
                325                 330                 335

<210> 94
<211> 795
<212> PRT
<213> Vibrio cholerae

<220>
<221> PEPTIDE
<222> (1)..(795)
<223> >sp|Q9KSN6|SYFB_VIBCH Phenylalanyl-tRNA synthetase
      beta chain (EC 6.1.1.20) (Phenylalanine-- tRNA
      ligase beta chain) (PheRS) - Vibrio cholerae. (795
      aa)

<400> 94
Met Lys Phe Ser Glu Ser Trp Leu Arg Glu Trp Val Asn Pro Ala Ile
1               5                   10                  15

Thr Thr Asp Glu Leu Thr His Gln Ile Thr Met Ala Gly Leu Glu Val
            20                  25                  30

Asp Asp Val Leu Ala Val Ala Gly Val Phe Asp Gly Val Lys Val Gly
        35                  40                  45

His Val Val Glu Cys Ala Gln His Pro Asp Ala Asp Lys Leu Arg Val
    50                  55                  60

Thr Lys Val Asp Val Gly Glu Glu Glu Leu Leu Asp Ile Val Cys Gly
65                  70                  75                  80

Ala Ala Asn Cys Arg Gln Gly Leu Lys Val Ala Val Ala Thr Val Gly
                85                  90                  95

Ala Thr Leu Pro Gly Asp Phe Lys Ile Lys Lys Ala Lys Leu Arg Gly
            100                 105                 110

Gln Pro Ser His Gly Met Leu Cys Ser Phe Ser Glu Leu Gly Ile Asp
        115                 120                 125

Val Glu Ser Asn Gly Ile Met Glu Leu Ala Glu Asn Ala Pro Ile Gly
    130                 135                 140

Met Asp Phe Arg Asp Phe Leu Ser Leu Asn Asp Val Thr Ile Asp Val
145                 150                 155                 160

Asp Leu Thr Ser Asn Arg Ala Asp Cys Phe Ser Ile Arg Gly Leu Ala
                165                 170                 175

Arg Glu Val Gly Val Leu Asn Arg Ala Asp Val Thr Ala Pro Ala Val
            180                 185                 190

Asn Ala Ile Val Ala Thr Ile Asn Asp Thr Ile Ser Ile Asp Val Lys
        195                 200                 205

Ala Pro Ala Ala Cys Pro Arg Tyr Leu Gly Arg Ile Val Lys Asn Val
    210                 215                 220

Asn Val Gln Ala Gln Thr Pro Leu Trp Met Gln Glu Lys Leu Arg Arg
225                 230                 235                 240

Cys Gly Ile Arg Ser Ile Asp Pro Val Val Asp Ile Thr Asn Phe Val

                245                 250                 255

Met Leu Glu Gln Gly Gln Pro Met His Ala Phe Asp Leu Ala Lys Ile
            260                 265                 270

Glu Gly Gly Ile Val Val Arg Leu Ala Glu Gln Asp Glu Lys Leu Thr
        275                 280                 285

Leu Leu Asp Gly Ser Glu Ala Lys Leu Asn Ala Asp Thr Leu Val Ile
    290                 295                 300

Ala Asp Gln Gln Lys Ala Leu Ala Ile Ala Gly Val Phe Gly Gly Glu
305                 310                 315                 320

His Ser Gly Val Ser Thr Asp Thr Lys Asp Val Leu Leu Glu Cys Ala
                325                 330                 335

Phe Phe Ala Pro Asp His Ile Arg Gly Arg Ala Arg Ser Tyr Gly Leu
            340                 345                 350

His Thr Asp Ser Ser Met Arg Phe Glu Arg Gly Val Asp Tyr Ala Leu
        355                 360                 365

Gln His Ala Ala Met Glu Arg Ala Thr Gln Leu Leu Val Glu Ile Cys
    370                 375                 380

Gly Gly Asp Val Ala Pro Val Val Ala Ala Glu Ser Ala Ala Asp Leu
385                 390                 395                 400

Pro Lys Pro Asn Gln Val Ala Leu Arg Arg Ser Lys Leu Asp Lys Leu
                405                 410                 415

Leu Gly His Ala Ile Pro Asp Ala Asp Val Val Glu Ile Leu Glu Arg
            420                 425                 430

Leu Gly Met Gln Val Glu Thr Thr Ala Glu Gly Trp Gln Ala Thr Ala
        435                 440                 445

Pro Thr Trp Arg Phe Asp Ile Ala Ile Glu Gln Asp Leu Val Glu Glu
    450                 455                 460

Val Gly Arg Ile Tyr Gly Tyr Asn Asn Ile Pro Asn Gln Ala Pro Val
465                 470                 475                 480

Ala Ala Leu Asn Met Asn Leu His Asn Glu Ala Lys Leu Pro Leu Lys
                485                 490                 495

Arg Val Arg Asp Leu Leu Val Asp Arg Gly Tyr Gln Glu Ala Ile Thr
            500                 505                 510

Tyr Ser Phe Val Glu Pro Glu Gln Gln Lys Leu Val Val Pro Gly Val
        515                 520                 525

Asp Ala Leu Ile Leu Pro Asn Pro Ile Ser Ala Glu Met Ser Ala Met
    530                 535                 540

Arg Leu Ser Leu Ile Gln Gly Leu Leu Asn Thr Val Val His Asn Gln
545                 550                 555                 560

Lys Arg Gln Gln Pro Arg Val Arg Leu Phe Glu Tyr Gly Leu Arg Phe

                565                 570                 575

Ile Pro Asp Ala Ala Ala Glu Asn Gly Met Arg Gln Glu Pro Met Leu
            580                 585                 590

Ala Gly Val Ile Ser Gly Ala Arg Gly Glu Glu His Trp Asn Met Glu
        595                 600                 605

Thr Ala Thr Val Asp Phe Phe Asp Met Lys Gly Asp Leu Glu Ala Val
    610                 615                 620

Leu Glu Leu Thr Ala Lys Gly Lys Ala Tyr Ser Phe Ala Ala Thr Lys
625                 630                 635                 640

His Pro Ala Leu His Pro Gly Gln Ala Ala Ala Ile Met Val Asp Gly
                645                 650                 655

Lys Ala Ile Gly Val Ile Gly Thr Val His Pro Glu Leu Glu Arg Lys
            660                 665                 670

Phe Gly Leu Asn Gly Arg Thr Ile Val Phe Glu Ile Glu Trp Asn Ala
        675                 680                 685

Ile Asn Thr Arg Val Ile Pro Glu Ala Ala Ala Ile Ser Lys Phe Pro
    690                 695                 700

Ala Asn Arg Arg Asp Ile Ala Leu Val Val Asp Gly Asn Ile Ala Ser
705                 710                 715                 720

Gly Asp Ile Val Glu Ala Cys Arg Val Ala Gly Gly Glu Leu Leu Lys
                725                 730                 735

Asp Ala Lys Leu Phe Asp Val Tyr Val Gly Lys Gly Val Glu Glu Gly
            740                 745                 750

Lys Lys Ser Leu Ala Ile Ala Leu Thr Leu Gln Ser Val Glu Arg Thr
        755                 760                 765

Leu Glu Glu Ala Asp Ile Ala Ala Ala Val Glu Ala Ile Val Gln Ala
    770                 775                 780

Val Ser Ala Gln Phe Gly Ala Ala Leu Arg Asp
785                 790                 795

SEQUENCE LISTING

<110> CENTRO DE INGENIERIA GENETICA Y BIOTECNOLOGIA

<120> A METHOD FOR THE SELECTIVE ISOLATION OF MULTIPLY-CHARGED PEPTIDES
      APPLICABLE IN THE QUANTITATIVE PROTEOMICS.

<130> P79116EP00

<140> EP 06076889.2
<141> 2006-10-16

<150> CU-2005-0197
<151> 2005-10-17

<160> 94

<170> PatentIn Ver. 2.1

<210> 1
<211> 415
<212> PRT
<213> Streptococcus equisimilis

<220>
<221> PEPTIDE
<222> (1)..(415)

<400> 1
Met Ile Ala Gly Pro Glu Trp Leu Leu Asp Arg Pro Ser Val Asn Asn
  1               5                  10                  15

Ser Gln Leu Val Val Ser Val Ala Gly Thr Val Glu Gly Thr Asn Gln
             20                  25                  30

Asp Ile Ser Leu Lys Phe Phe Glu Ile Asp Leu Thr Ser Arg Pro Ala
         35                  40                  45

His Gly Gly Lys Thr Glu Gln Gly Leu Ser Pro Lys Ser Lys Pro Phe
     50                  55                  60

Ala Thr Asp Ser Gly Ala Met Pro His Lys Leu Glu Lys Ala Asp Leu
 65                  70                  75                  80

Leu Lys Ala Ile Gln Glu Gln Leu Ile Ala Asn Val His Ser Asn Asp
                 85                  90                  95

Asp Tyr Phe Glu Val Ile Asp Phe Ala Ser Asp Ala Thr Ile Thr Asp
            100                 105                 110

Arg Asn Gly Lys Val Tyr Phe Ala Asp Lys Asp Gly Ser Val Thr Leu
        115                 120                 125

Pro Thr Gln Pro Val Gln Glu Phe Leu Leu Ser Gly His Val Arg Val
    130                 135                 140

Arg Pro Tyr Lys Glu Lys Pro Ile Gln Asn Gln Ala Lys Ser Val Asp
145                 150                 155                 160

Val Glu Tyr Thr Val Gln Phe Thr Pro Leu Asn Pro Asp Asp Asp Phe

                165                 170                 175

Arg Pro Gly Leu Lys Asp Thr Lys Leu Leu Lys Thr Leu Ala Ile Gly
            180                 185                 190

Asp Thr Ile Thr Ser Gln Glu Leu Leu Ala Gln Ala Gln Ser Ile Leu
        195                 200                 205

Asn Lys Thr His Pro Gly Tyr Thr Ile Tyr Glu Arg Asp Ser Ser Ile
    210                 215                 220

Val Thr His Asp Asn Asp Ile Phe Arg Thr Ile Leu Pro Met Asp Gln
225                 230                 235                 240

Glu Phe Thr Tyr His Val Lys Asn Arg Glu Gln Ala Tyr Glu Ile Asn
                245                 250                 255

Lys Lys Ser Gly Leu Asn Glu Glu Ile Asn Asn Thr Asp Leu Ile Ser
            260                 265                 270

Glu Lys Tyr Tyr Val Leu Lys Lys Gly Glu Lys Pro Tyr Asp Pro Phe
        275                 280                 285

Asp Arg Ser His Leu Lys Leu Phe Thr Ile Lys Tyr Val Asp Val Asn
    290                 295                 300

Thr Asn Glu Leu Leu Lys Ser Glu Gln Leu Leu Thr Ala Ser Glu Arg
305                 310                 315                 320

Asn Leu Asp Phe Arg Asp Leu Tyr Asp Pro Arg Asp Lys Ala Lys Leu
                325                 330                 335

Leu Tyr Asn Asn Leu Asp Ala Phe Gly Ile Met Asp Tyr Thr Leu Thr
            340                 345                 350

Gly Lys Val Glu Asp Asn His Asp Asp Thr Asn Arg Ile Ile Thr Val
        355                 360                 365

Tyr Met Gly Lys Arg Pro Glu Gly Glu Asn Ala Ser Tyr His Leu Ala
    370                 375                 380

Tyr Asp Lys Asp Arg Tyr Thr Glu Glu Glu Arg Glu Val Tyr Ser Tyr
385                 390                 395                 400

Leu Arg Tyr Thr Gly Thr Pro Ile Pro Asp Asn Pro Asn Asp Lys
                405                 410                 415

<210> 2
<211> 153
<212> PRT
<213> Equus caballus

<220>
<221> PEPTIDE
<222> (1)..(153)

<400> 2
Val Leu Ser Glu Gly Glu Trp Gln Leu Val Leu His Val Trp Ala Lys
1               5                   10                  15

Val Glu Ala Asp Val Ala Gly His Gly Gln Asp Ile Leu Ile Arg Leu
            20                  25                  30

Phe Lys Ser His Pro Glu Thr Leu Glu Lys Phe Asp Arg Phe Lys His
        35                  40                  45

Leu Lys Thr Glu Ala Glu Met Lys Ala Ser Glu Asp Leu Lys Lys His
    50                  55                  60

Gly Val Thr Val Leu Thr Ala Leu Gly Ala Ile Leu Lys Lys Lys Gly
65                  70                  75                  80

His His Glu Ala Glu Leu Lys Pro Leu Ala Gln Ser His Ala Thr Lys
                85                  90                  95

His Lys Ile Pro Ile Lys Tyr Leu Glu Phe Ile Ser Glu Ala Ile Ile
            100                 105                 110

His Val Leu His Ser Arg His Pro Gly Asp Phe Gly Ala Asp Ala Gln
        115                 120                 125

Gly Ala Met Asn Lys Ala Leu Glu Leu Phe Arg Lys Asp Ile Ala Ala
    130                 135                 140

Lys Tyr Lys Glu Leu Gly Tyr Gln Gly
145                 150

<210> 3
<211> 104
<212> PRT
<213> Equus caballus

<220>
<221> PEPTIDE
<222> (1)..(104)

<400> 3

<210> 4

<211> 357
<212> PRT
<213> Vibrio cholerae

<220>
<221> PEPTIDE
<222> (1)..(357)
<223> >tr|Q9KU34|Q9KU34_VIBCH
      Phospho-2-dehydro-3-deoxyheptonate aldolase,
      tyr-sensitive - Vibrio cholerae (357 aa).

<400> 4
Met Lys Lys Ser Glu Leu Ser Asp Val Asn Ile Ser Asn Glu Gln Ile
1               5                   10                  15

Leu Ile Thr Pro Asp Ala Leu Lys Ala Lys Ile Pro Leu Ser Asp Lys
            20                  25                  30

Ala Arg Lys Phe Ile Arg Glu Ser Arg Gln Thr Val Ala Asp Ile Ile
        35                  40                  45

His Lys Arg Asp Pro Arg Leu Leu Ile Val Cys Gly Pro Cys Ser Ile
    50                  55                  60

His Asp Val Asp Ala Ala Lys Glu Tyr Ala Lys Lys Leu Lys Ala Leu
65                  70                  75                  80

Ser Ala Gln Leu Ser Asp Gln Leu Tyr Ile Val Met Arg Val Tyr Phe
                85                  90                  95

Glu Lys Pro Arg Thr Thr Val Gly Trp Lys Gly Leu Ile Asn Asp Pro
            100                 105                 110

His Leu Asp Gly Ser Phe Asp Ile Glu His Gly Leu His Val Gly Arg
        115                 120                 125

Gln Leu Leu Val Asp Leu Ala Glu Met Glu Ile Pro Leu Ala Thr Glu
    130                 135                 140

Ala Leu Asp Pro Ile Ser Pro Gln Tyr Leu Ala Asp Thr Phe Ser Trp
145                 150                 155                 160

Ala Ala Ile Gly Ala Arg Thr Thr Glu Ser Gln Thr His Arg Glu Met
                165                 170                 175

Ala Ser Gly Leu Ser Met Pro Ile Gly Phe Lys Asn Gly Thr Asp Gly
            180                 185                 190

Asn Leu Ala Thr Ala Ile Asn Ala Met Gln Ala Ala Ser Ser Ser His
        195                 200                 205

Arg Phe Met Gly Ile Asn Arg Glu Gly Gln Val Ala Leu Leu Thr Thr
    210                 215                 220

Gln Gly Asn Pro Asn Gly His Val Ile Leu Arg Gly Gly Lys Gln Thr
225                 230                 235                 240

Asn Tyr Asp Ser Val Ser Val Ala Glu Cys Glu Glu Glu Met Gln Lys
                245                 250                 255

Ala Arg Leu Glu Pro Ser Leu Met Val Asp Cys Ser His Ala Asn Ser

260                 265                 270

Arg Lys Asp Tyr Arg Arg Gln Pro Leu Val Ala Glu Asp Val Ile His
        275                 280                 285

Gln Ile Arg Glu Gly Asn Arg Ser Ile Ile Gly Leu Met Ile Glu Ser
    290                 295                 300

His Leu Asn Glu Gly Asn Gln Ser Ser Asp Leu Pro Arg Glu Lys Met
305                 310                 315                 320

Gln Tyr Gly Val Ser Ile Thr Asp Gly Cys Ile Asn Trp Ser Thr Thr
                325                 330                 335

Glu Ala Leu Leu Arg Arg Ala His Gln Glu Leu Ile Pro Phe Leu His
            340                 345                 350

Asn Arg Leu Gln Gly
        355


<210> 5
<211> 103
<212> PRT
<213> Vibrio cholerae

<220>
<221> PEPTIDE
<222> (1)..(103)
<223> >tr|Q9KUT0|Q9KUT0_VIBCH Ribosomal protein L21 -
      Vibrio cholerae. (103 aa)

<400> 5
Met Tyr Ala Val Phe Gln Ser Gly Gly Lys Gln His Arg Val Ser Glu
1               5                   10                  15

Gly Gln Thr Leu Arg Leu Glu Lys Leu Asp Val Glu Thr Gly Ala Thr
            20                  25                  30

Val Glu Phe Asp Lys Val Leu Leu Val Ala Asn Gly Glu Asp Ile Lys
        35                  40                  45

Val Gly Ala Pro Leu Val Glu Gly Gly Lys Val Val Ala Glu Val Val
    50                  55                  60

Gln His Gly Arg Gly Asp Lys Val Lys Ile Val Lys Phe Arg Arg Arg
65                  70                  75                  80

Lys His Ser Arg Lys Gln Gln Gly His Arg Gln Trp Phe Thr Glu Val
                85                  90                  95

Lys Ile Thr Gly Ile Asn Ala
            100


<210> 6
<211> 267
<212> PRT
<213> Vibrio cholerae

<220>

<221> PEPTIDE
<222> (1)..(267)
<223> >sp|Q9KTY5|SUHB_VIBCH Inositol-1-monophosphatase
      (EC 3.1.3.25) (IMPase) (Inositol-1- phosphatase)
      (I-1-Pase) - Vibrio cholerae. (267 aa)

<400> 6
Met His Pro Met Leu Asn Ile Ala Ile Arg Ala Ala Arg Lys Ala Gly
1               5                   10                  15

Asn His Ile Ala Lys Ser Leu Glu Asn Ala Glu Lys Ile Gln Thr Thr
            20                  25                  30

Gln Lys Gly Ser Asn Asp Phe Val Thr Asn Val Asp Lys Glu Ala Glu
        35                  40                  45

Ala Ile Ile Val Ser Thr Ile Lys Ser Ser Tyr Pro Glu His Cys Ile
    50                  55                  60

Ile Ala Glu Glu Gly Gly Leu Ile Glu Gly Lys Asp Lys Glu Val Gln
65                  70                  75                  80

Trp Ile Ile Asp Pro Leu Asp Gly Thr Thr Asn Phe Val Lys Gly Phe
                85                  90                  95

Pro His Phe Ala Val Ser Ile Ala Val Arg Phe Arg Gly Lys Thr Glu
            100                 105                 110

Val Ala Cys Val Tyr Asp Pro Met Thr Asn Glu Leu Phe Thr Ala Gln
        115                 120                 125

Arg Gly Ala Gly Ala Gln Leu Asn Asn Ala Arg Ile Arg Val Gln Pro
    130                 135                 140

Ile Lys Asp Leu Gln Gly Ala Val Leu Ala Thr Ala Phe Pro Phe Lys
145                 150                 155                 160

Gln Lys Gln His Ser Glu Ser Phe Met Lys Ile Leu Ser Ala Met Phe
                165                 170                 175

Val Glu Cys Ala Asp Phe Arg Arg Thr Gly Ser Ala Ala Leu Asp Leu
            180                 185                 190

Cys Tyr Leu Ala Ala Asn Arg Val Asp Gly Tyr Phe Glu Leu Gly Leu
        195                 200                 205

Lys Pro Trp Asp Met Ala Ala Gly Glu Leu Ile Ala Arg Glu Ala Gly
    210                 215                 220

Ala Ile Val Thr Asp Phe Ala Gly Gly Thr Asp Tyr Met Gln Ser Gly
225                 230                 235                 240

Asn Ile Val Ala Ser Ser Pro Arg Gly Val Lys Ala Ile Leu Gln His
                245                 250                 255

Ile Arg Glu Asn Gly Asn Ser Ala Ile Leu Lys
            260                 265


<210> 7
<211> 78

<212> PRT
<213> Vibrio cholerae

<220>
<221> PEPTIDE
<222> (1)..(76)
<223> >sp|Q9KVC8|RL28_VIBCH 50S ribosomal protein L28 -
      Vibrio cholerae. (76 aa)

<400> 7
Met Ser Arg Val Cys Gln Val Thr Gly Lys Arg Pro Ala Val Gly Asn
1               5                   10                  15

Asn Arg Ser His Ala Lys Asn Ala Thr Lys Arg Arg Phe Leu Pro Asn
            20                  25                  30

Leu Gln Thr His Arg Phe Trp Val Glu Ser Glu Lys Arg Phe Val Lys
        35                  40                  45

Leu Arg Leu Thr Ala Lys Gly Met Arg Ile Ile Asp Lys Lys Gly Ile
    50                  55                  60

Asp Ala Val Leu Val Glu Ile Arg Ala Arg Gly Glu Asn Val
65                  70                  75


<210> 8
<211> 108
<212> PRT
<213> Vibrio cholerae

<220>
<221> PEPTIDE
<222> (1)..(108)
<223> >sp|P52695|HLYU_VIBCH Transcriptional activator
      hlyU - Vibrio cholerae. (108 aa)

<400> 8
Met Pro Tyr Leu Lys Gly Ala Pro Met Asn Leu Gln Glu Met Glu Lys
1               5                   10                  15

Asn Ser Ala Lys Ala Val Val Leu Leu Lys Ala Met Ala Asn Glu Arg
            20                  25                  30

Arg Leu Gln Ile Leu Cys Met Leu Leu Asp Asn Glu Leu Ser Val Gly
        35                  40                  45

Glu Leu Ser Ser Arg Leu Glu Leu Ser Gln Ser Ala Leu Ser Gln His
    50                  55                  60

Leu Ala Trp Leu Arg Arg Asp Gly Leu Val Asn Thr Arg Lys Glu Ala
65                  70                  75                  80

Gln Thr Val Phe Tyr Thr Leu Ser Ser Thr Glu Val Lys Ala Met Ile
                85                  90                  95

Glu Leu Leu His Arg Leu Tyr Cys Gln Ala Asn Gln
            100                 105


<210> 9

<211> 394
<212> PRT
<213> Vibrio cholerae

<220>
<221> PEPTIDE
<222> (1)..(394)
<223> >sp|Q9KUZ6|EFTU2_VIBCH Elongation factor Tu-B
      (EF-Tu-B) - Vibrio cholerae. (394 aa)

<400> 9
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
        35                  40                  45

Ser Ile Asp Asn Ala Pro Glu Glu Arg Glu Arg Gly Ile Thr Ile Asn
    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Pro
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp
        195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala
        355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390


<210> 10
<211> 953
<212> PRT
<213> Vibrio cholerae

<220>
<221> PEPTIDE
<222> (1)..(953)
<223> >sp|Q9KP73|SYV_VIBCH Valyl-tRNA synthetase (EC
      6.1.1.9) (Valine--tRNA ligase) (ValRS) - Vibrio
      cholerae. (953 aa)

<400> 10
Met Glu Lys Thr Tyr Asn Pro Thr Ser Ile Glu Gln Asp Leu Tyr Lys
  1               5                  10                  15

Thr Trp Glu Glu Gln Gly Tyr Phe Lys Pro His Gly Asp Thr Ser Lys
             20                  25                  30

Asp Ala Tyr Ser Ile Met Ile Pro Pro Pro Asn Val Thr Gly Ser Leu
         35                  40                  45


His Met Gly His Ala Phe Gln Asp Thr Ile Met Asp Thr Leu Ile Arg
     50                  55                  60

Cys Gln Arg Met Lys Gly Lys Asn Thr Leu Trp Gln Val Gly Thr Asp
 65                  70                  75                  80

His Ala Gly Ile Ala Thr Gln Met Val Val Glu Arg Lys Ile Ala Ala
                 85                  90                  95

Glu Glu Gly Lys Thr Lys His Asp Tyr Gly Arg Asp Ala Phe Ile Asp
            100                 105                 110

Lys Ile Trp Glu Trp Lys Ala Glu Ser Gly Gly Thr Ile Thr Lys Gln
        115                 120                 125

Leu Arg Arg Leu Gly Ala Ser Val Asp Trp Asp Arg Glu Arg Phe Thr
    130                 135                 140

Met Asp Asp Gly Phe Tyr Lys Ala Val Gln Glu Val Phe Val Arg Leu
145                 150                 155                 160

Tyr Lys Asp Asp Leu Ile Tyr Arg Gly Lys Arg Leu Val Asn Trp Asp
                165                 170                 175

Pro Lys Leu His Thr Ala Ile Ser Asp Leu Glu Val Glu Asn Lys Glu
            180                 185                 190

Thr Lys Gly His Met Trp His Phe Arg Tyr Pro Leu Ala Asp Gly Val
        195                 200                 205

Lys Thr Ala Asp Gly Lys Asp Tyr Ile Val Val Ala Thr Thr Arg Pro
    210                 215                 220

Glu Thr Met Leu Gly Asp Thr Gly Val Ala Val Asn Pro Glu Asp Pro
225                 230                 235                 240

Arg Tyr Lys Asp Leu Ile Gly Lys Glu Ile Ile Leu Pro Ile Val Gly
                245                 250                 255

Arg Arg Ile Pro Ile Val Gly Asp Glu His Ala Asp Met Glu Lys Gly
            260                 265                 270

Thr Gly Cys Val Lys Ile Thr Pro Ala His Asp Phe Asn Asp Tyr Glu
        275                 280                 285

Val Gly Lys Arg His Gln Leu Pro Met Ile Asn Ile Leu Thr Phe Asp
    290                 295                 300

Ala Asn Ile Arg Asp Ala Ala Glu Val Phe Asn Ser Asn Gly Glu Ala
305                 310                 315                 320

Ser Asn Ala Tyr Gly Thr Glu Ile Pro Ala Lys Tyr Gln Gly Met Glu
                325                 330                 335

Arg Phe Ala Ala Arg Lys Ala Ile Val Ala Glu Phe Glu Glu Leu Gly
            340                 345                 350

Leu Leu Gln Glu Ile Lys Asp His Asp Leu Thr Val Pro Tyr Gly Asp
        355                 360                 365

Arg Gly Gly Val Val Ile Glu Pro Met Leu Thr Asp Gln Trp Tyr Val
    370                 375                 380

Arg Ala Gly Ile Leu Ala Lys Pro Ala Val Glu Ala Val Glu Asn Gly
385                 390                 395                 400

Asp Ile Gln Phe Val Pro Lys Gln Tyr Glu Asn Met Tyr Phe Ser Trp
                405                 410                 415

Met Arg Asp Ile Gln Asp Trp Cys Ile Ser Arg Gln Leu Trp Trp Gly
            420                 425                 430

His Arg Ile Pro Ala Trp Tyr Asp Glu Gln Gly Asn Val Phe Val Gly

435                 440                 445

Arg Asn Glu Glu Glu Val Arg Ala Glu Asn Asn Ile Ala Ala Asp Val
    450                 455                 460

Ala Leu Arg Gln Asp Asp Asp Val Leu Asp Thr Trp Phe Ser Ser Ala
465                 470                 475                 480

Leu Trp Thr Phe Gly Thr Leu Gly Trp Pro Glu Lys Thr Pro Glu Leu
                485                 490                 495

Lys Val Phe His Pro Thr Asp Val Leu Val Thr Gly Phe Asp Ile Ile
            500                 505                 510

Phe Phe Trp Val Ala Arg Met Ile Met Met Thr Met His Phe Cys Lys
        515                 520                 525

Asp Glu Asp Gly Lys Ala Gln Val Pro Phe Lys Thr Val Tyr Val Thr
    530                 535                 540

Gly Leu Ile Arg Asp Glu Asn Gly Asp Lys Met Ser Lys Ser Lys Gly
545                 550                 555                 560

Asn Val Leu Asp Pro Ile Asp Met Ile Asp Gly Ile Asp Leu Glu Ser
                565                 570                 575

Leu Val Ala Lys Arg Thr Gly Asn Met Met Gln Pro Gln Leu Ala Ala
            580                 585                 590

Lys Ile Glu Lys Asn Thr Arg Lys Thr Phe Glu Asn Gly Ile Glu Ala
        595                 600                 605

Tyr Gly Thr Asp Ser Leu Arg Phe Thr Leu Ala Ala Met Ala Ser Thr
    610                 615                 620

Gly Arg Asp Ile Asn Trp Asp Met Lys Arg Leu Glu Gly Tyr Arg Asn
625                 630                 635                 640

Phe Cys Asn Lys Leu Trp Asn Ala Ser Arg Tyr Val Leu Met Asn Thr
                645                 650                 655

Glu Glu Gln Asp Cys Gly Phe Ala Ala Gly Ala Glu Leu Glu Tyr Ser
            660                 665                 670

Leu Ala Asp Lys Trp Ile Glu Ser Gln Phe Glu Leu Ala Ala Lys Glu
        675                 680                 685

Phe Asn Gly His Ile Asp Asn Phe Arg Leu Asp Met Ala Ala Asn Thr
    690                 695                 700

Leu Tyr Glu Phe Ile Trp Asn Gln Phe Cys Asp Trp Tyr Leu Glu Leu
705                 710                 715                 720

Thr Lys Pro Val Leu Trp Lys Gly Thr Glu Ala Gln Gln Arg Ala Thr
                725                 730                 735

Arg Arg Thr Leu Ile Thr Val Leu Glu Lys Thr Leu Arg Leu Ala His
            740                 745                 750

Pro Val Ile Pro Tyr Ile Thr Glu Thr Ile Trp Gln Ser Val Lys Pro
        755                 760                 765

Leu Val Asp Gly Val Glu Gly Asp Thr Ile Met Leu Gln Ala Leu Pro
    770                 775                 780

Gln Tyr Asp Val Ala Asn Phe Asn Gln Glu Ala Leu Asp Asp Ile Glu
785                 790                 795                 800

Trp Val Lys Ala Phe Ile Thr Ser Ile Arg Asn Leu Arg Ala Glu Tyr
                805                 810                 815

Asp Ile Asn Pro Gly Lys Pro Leu Glu Val Met Leu Lys Ala Ala Asn
            820                 825                 830

Glu Gln Asp Ala Ala Arg Ile Glu Ala Asn Lys Pro Val Leu Val Ser
        835                 840                 845

Leu Ala Lys Leu Glu Ser Ile Arg Val Leu Ala Asp Gly Glu Ala Thr
    850                 855                 860

Pro Ala Cys Ala Thr Ala Leu Val Gly Lys Ser Glu Leu Met Ile Pro
865                 870                 875                 880

Met Ala Gly Leu Ile Asp Lys Asp Ala Glu Leu Asp Arg Leu Ala Lys
                885                 890                 895

Glu Ile Ala Lys Thr Gln Gly Glu Ile Ala Arg Ile Glu Gly Lys Leu
            900                 905                 910

Gly Asn Glu Gly Phe Val Ala Lys Ala Pro Glu Ala Val Ile Thr Lys
        915                 920                 925

Glu Arg Glu Lys Leu Ala Gly Tyr Gln Glu Ala Leu Val Lys Leu Glu
    930                 935                 940

Gln Gln Lys Ala Thr Ile Ala Ala Leu
945                 950


<210> 11
<211> 886
<212> PRT
<213> Vibrio cholerae

<220>
<221> PEPTIDE
<222> (1)..(886)
<223> >tr|Q9KPF4|Q9KPF4_VIBCH Pyruvate dehydrogenase, E1
      component - Vibrio cholerae. (886 aa)

<400> 11
Met Ser Asp Met Lys His Asp Val Asp Ala Leu Glu Thr Gln Glu Trp
1               5                   10                  15

Leu Ala Ala Leu Glu Ser Val Val Arg Glu Glu Gly Val Glu Arg Ala
            20                  25                  30

Gln Tyr Leu Leu Glu Gln Val Leu Glu Lys Ala Arg Leu Asp Gly Val
        35                  40                  45

Asp Met Pro Thr Gly Val Thr Thr Asn Tyr Ile Asn Thr Ile Pro Ala
    50                  55                  60

Ala Gln Glu Pro Ala Tyr Pro Gly Asp Thr Thr Ile Glu Arg Arg Ile
65                  70                  75                  80

Arg Ser Ile Ile Arg Trp Asn Ala Ile Met Ile Val Leu Arg Ala Ser
                85                  90                  95

Lys Lys Asp Leu Glu Leu Gly Gly His Met Ala Ser Phe Gln Ser Ser
            100                 105                 110

Ala Ala Phe Tyr Glu Thr Cys Phe Asn His Phe Phe Arg Ala Pro Asn
        115                 120                 125

Glu Lys Asp Gly Gly Asp Leu Val Tyr Tyr Gln Gly His Ile Ser Pro
    130                 135                 140

Gly Ile Tyr Ala Arg Ala Phe Val Glu Gly Arg Leu Thr Glu Glu Gln
145                 150                 155                 160

Leu Asp Asn Phe Arg Gln Glu Val Asp Gly Lys Gly Leu Pro Ser Tyr
                165                 170                 175

Pro His Pro Lys Leu Met Pro Glu Phe Trp Gln Phe Pro Thr Val Ser
            180                 185                 190

Met Gly Leu Gly Pro Ile Ser Ala Ile Tyr Gln Ala Arg Phe Leu Lys
        195                 200                 205

Tyr Leu Asn Gly Arg Gly Leu Lys Asp Thr Thr Ala Gln Arg Val Tyr
    210                 215                 220

Ala Phe Leu Gly Asp Gly Glu Met Asp Glu Pro Glu Ser Arg Gly Ala
225                 230                 235                 240

Ile Ser Phe Ala Ala Arg Glu Lys Leu Asp Asn Leu Cys Phe Leu Ile
                245                 250                 255

Asn Cys Asn Leu Gln Arg Leu Asp Gly Pro Val Met Gly Asn Gly Lys
            260                 265                 270

Ile Ile Gln Glu Leu Glu Gly Leu Phe Arg Gly Ala Gly Trp Asn Val
        275                 280                 285

Val Lys Val Ile Trp Gly Asn Gly Trp Asp Lys Leu Leu Ala Lys Asp
    290                 295                 300

Thr Thr Gly Lys Leu Leu Gln Leu Met Asn Glu Thr Ile Asp Gly Asp
305                 310                 315                 320

Tyr Gln Thr Phe Lys Ala Lys Asp Gly Ala Tyr Val Arg Glu His Phe
                325                 330                 335

Phe Gly Lys Tyr Pro Glu Thr Ala Ala Leu Val Ala Asp Met Thr Asp
            340                 345                 350

Asp Glu Ile Phe Ala Leu Lys Arg Gly Gly His Glu Ser Ser Lys Leu
        355                 360                 365

Tyr Ala Ala Phe Lys Asn Ala Gln Asp Thr Lys Gly Arg Pro Thr Val
    370                 375                 380

Ile Leu Ala Lys Thr Val Lys Gly Tyr Gly Met Gly Asp Ala Ala Glu
385                 390                 395                 400

Gly Lys Asn Ile Ala His Gln Val Lys Lys Met Asp Met Thr His Val
                405                 410                 415

Leu Ala Met Arg Asn Arg Leu Gly Leu Gln Asp Leu Ile Ser Asp Glu
            420                 425                 430

Glu Val Lys Asn Leu Pro Tyr Leu Lys Leu Glu Glu Gly Ser Lys Glu
        435                 440                 445

Phe Glu Tyr Leu His Ala Arg Arg Lys Ala Leu His Gly Tyr Thr Pro
    450                 455                 460

Gln Arg Leu Pro Asn Phe Thr Gly Glu Phe Ile Val Pro Ala Leu Glu
465                 470                 475                 480

Asp Phe Lys Pro Leu Leu Glu Glu Gln Ser Arg Glu Ile Ser Ser Thr
                485                 490                 495

Met Ala Tyr Val Arg Ser Leu Asn Ile Leu Leu Lys Asp Lys Asn Ile
            500                 505                 510

Gly Gln Asn Ile Val Pro Ile Ile Ala Asp Glu Ala Arg Thr Phe Gly
        515                 520                 525

Met Glu Gly Leu Phe Arg Gln Ile Gly Ile Tyr Asn Pro His Gly Gln
    530                 535                 540

Asn Tyr Thr Pro Gln Asp Arg Asp Ile Val Ser Tyr Tyr Lys Glu Ala
545                 550                 555                 560

Thr Ser Gly Gln Val Leu Gln Glu Gly Ile Asn Glu Leu Gly Ala Met
                565                 570                 575

Ser Ser Trp Val Ala Ala Ala Thr Ser Tyr Ser Thr Asn Asn Leu Pro
            580                 585                 590

Met Ile Pro Phe Tyr Ile Tyr Tyr Ser Met Phe Gly Phe Gln Arg Val
        595                 600                 605

Gly Asp Met Ala Trp Met Ala Gly Asp Gln Gln Ala Arg Gly Phe Leu
    610                 615                 620

Leu Gly Ala Thr Ala Gly Arg Thr Thr Leu Asn Gly Glu Gly Leu Gln
625                 630                 635                 640

His Glu Asp Gly His Ser His Ile Leu Ala Gly Thr Val Pro Asn Cys
                645                 650                 655

Ile Ser Tyr Asp Pro Thr Phe Ala Tyr Glu Val Ala Val Ile Leu Gln
            660                 665                 670

Asp Gly Ile Arg Arg Met Tyr Gly Glu Gln Glu Asn Val Phe Tyr Tyr
        675                 680                 685

Leu Thr Leu Met Asn Glu Ser Tyr Ala His Pro Ala Met Pro Ala Gly
    690                 695                 700

Ala Glu Glu Gly Ile Arg Lys Gly Ile Tyr Lys Leu Glu Thr His Ala

705                 710                 715                 720

Gly Asn Lys Ala Lys Val Gln Leu Met Ser Ser Gly Thr Ile Met Asn
                725                 730                 735

Glu Val Arg Lys Ala Ala Gln Ile Leu Ser Glu Glu Tyr Gly Val Ala
            740                 745                 750

Ser Asp Val Tyr Ser Val Thr Ser Phe Asn Glu Leu Ala Arg Asp Gly
        755                 760                 765

Gln Ala Cys Asp Arg Phe Asn Met Leu His Pro Glu Ala Glu Val Lys
    770                 775                 780

Val Pro Tyr Ile Ala Gln Val Met Gly Thr Glu Pro Ala Ile Ala Ala
785                 790                 795                 800

Thr Asp Tyr Met Lys Asn Tyr Ala Asp Gln Val Arg Ala Phe Ile Pro

                805                 810                 815

Ala Gln Ser Tyr Lys Val Leu Gly Thr Asp Gly Phe Gly Arg Ser Asp
            820                 825                 830

Ser Arg Glu Asn Leu Arg Arg His Phe Glu Val Asn Ala Gly Tyr Val
        835                 840                 845

Val Val Ala Ala Leu Asn Glu Leu Ala Lys Arg Gly Glu Val Glu Lys
    850                 855                 860

Ser Val Val Ala Ala Ala Ile Lys Lys Phe Asp Ile Asp Thr Glu Lys
865                 870                 875                 880

Thr Asn Pro Leu Tyr Ala
                885


<210> 12
<211> 635
<212> PRT
<213> Vibrio cholerae

<220>
<221> PEPTIDE
<222> (1)..(635)
<223> >tr|Q9KPF5|Q9KPF5_VIBCH Pyruvate dehydrogenase, E2
      component, dihydrolipoamide acetyltransferase -
      Vibrio cholerae. (635 aa)

<400> 12
Met Lys Val Gly Lys Val Met Ala Ile Glu Ile Tyr Val Pro Asp Ile
1               5                   10                  15

Gly Ala Asp Glu Val Glu Val Thr Glu Ile Leu Val Lys Val Gly Asp
            20                  25                  30

Lys Val Ala Glu Glu Gln Ser Leu Ile Thr Val Glu Gly Asp Lys Ala
        35                  40                  45

Ser Met Glu Val Pro Ala Ser Gln Ala Gly Ile Val Lys Glu Ile Lys
    50                  55                  60

Val Val Ala Gly Asp Lys Val Ser Thr Gly Ser Leu Ile Met Val Phe
65                  70                  75                  80

Glu Ala Glu Gly Ala Ala Ala Ala Ala Pro Ala Pro Ala Pro Gln Ala
                85                  90                  95

Ala Ala Pro Val Ala Ala Ala Pro Ala Ala Ala Ala Leu Lys Glu Val
            100                 105                 110

Gln Val Pro Asp Ile Gly Gly Asp Glu Val Glu Val Thr Glu Ile Met
        115                 120                 125

Val Lys Val Gly Asp Val Val Ala Glu Glu Gln Ser Leu Ile Thr Val
    130                 135                 140

Glu Gly Asp Lys Ala Ser Met Glu Val Pro Ala Pro Phe Ala Gly Thr
145                 150                 155                 160

Val Lys Glu Ile Lys Ile Ala Ala Gly Asp Lys Val Ser Thr Gly Ser
                165                 170                 175

Leu Ile Met Val Phe Glu Val Ala Gly Ala Ala Pro Leu Ala Ala Pro
            180                 185                 190

Val Gln Ala Ala Ala Pro Ala Ala Ala Ala Ala Pro Ala Val Ala Ala
        195                 200                 205

Leu Lys Glu Val Gln Val Pro Asp Ile Gly Gly Asp Glu Val Thr Val
    210                 215                 220

Thr Glu Ile Met Val Asn Val Gly Asp Ser Ile Ser Glu Glu Gln Ser
225                 230                 235                 240

Leu Ile Thr Val Glu Gly Asp Lys Ala Ser Met Glu Val Pro Ala Pro
                245                 250                 255

Phe Ala Gly Thr Leu Lys Glu Ile Lys Val Ala Thr Gly Asp Lys Val
            260                 265                 270

Lys Thr Gly Ser Leu Ile Met Val Phe Glu Val Ala Gly Ala Ala Pro
        275                 280                 285

Val Ala Ala Pro Val Gln Ala Ala Ala Ala Pro Ala Ala Ala Pro Ala
    290                 295                 300

Gln Ala Ala Thr Pro Ala Ala Ala Ala Pro Ala Thr Ser Gly Glu Phe
305                 310                 315                 320

Gln Glu Asn His Glu Tyr Ser His Ala Ser Pro Val Val Arg Arg Leu
                325                 330                 335

Ala Arg Glu Phe Gly Val Asn Leu Ala Lys Val Lys Gly Ser Gly Arg
            340                 345                 350

Lys Asn Arg Ile Leu Lys Glu Asp Val Gln Asn Tyr Val Lys Glu Ala
        355                 360                 365

Leu Lys Arg Leu Glu Ser Gly Ala Gln Ala Ala Ala Ser Gly Lys Gly
    370                 375                 380

Asp Gly Ala Ala Leu Gly Leu Leu Pro Trp Pro Lys Val Asp Phe Ser
385                 390                 395                 400

Lys Phe Gly Asp Thr Glu Val Gln Pro Leu Ser Arg Ile Lys Lys Ile
                405                 410                 415

Ser Gly Ala Asn Leu His Arg Asn Trp Val Met Ile Pro His Val Thr
            420                 425                 430

Gln Trp Asp Asn Ala Asp Ile Thr Glu Leu Glu Lys Phe Arg Gln Glu
        435                 440                 445

Gln Asn Ala Met Glu Ala Lys Arg Asp Thr Gly Met Lys Ile Thr Pro
    450                 455                 460

Leu Val Phe Ile Met Lys Ala Ala Ala Lys Ala Leu Glu Ala Phe Pro
465                 470                 475                 480

Ala Phe Asn Ser Ser Leu Ser Asp Asp Gly Glu Ser Leu Ile Leu Lys
                485                 490                 495

Lys Tyr Val Asn Ile Gly Ile Ala Val Asp Thr Pro Asn Gly Leu Val
            500                 505                 510

Val Pro Val Phe Lys Asp Val Asn Lys Lys Gly Ile Tyr Glu Leu Ser
        515                 520                 525

Lys Glu Leu Ala Glu Val Ser Lys Lys Ala Arg Gly Gly Lys Leu Thr
    530                 535                 540

Ala Ala Asp Met Gln Gly Gly Cys Phe Thr Ile Ser Ser Leu Gly Gly
545                 550                 555                 560

Ile Gly Gly Thr Ala Phe Thr Pro Ile Val Asn Ala Pro Glu Val Ala
                565                 570                 575

Ile Leu Gly Val Ser Lys Ser Glu Met Lys Pro Val Trp Asn Gly Lys
            580                 585                 590

Glu Phe Ala Pro Arg Leu Gln Leu Pro Leu Ser Leu Ser Tyr Asp His
        595                 600                 605

Arg Val Ile Asp Gly Ala Glu Gly Ala Arg Phe Ile Thr Tyr Leu Asn
    610                 615                 620

Glu Cys Leu Ser Asp Ile Arg Arg Leu Val Leu
625                 630                 635


<210> 13
<211> 172
<212> PRT
<213> Vibrio cholerae

<220>
<221> PEPTIDE
<222> (1)..(172)
<223> >tr|Q9KR12|Q9KR12_VIBCH Peptidoglycan-associated
      lipoprotein - Vibrio cholerae. (172 aa)

<400> 13

Met Gln Leu Asn Lys Val Leu Lys Ser Leu Leu Ile Ala Leu Pro Val
1               5                   10                  15

Leu Ala Val Thr Ala Cys Ser Ser Ser Asp Asp Ala Ala Asn Ser Gly
            20                  25                  30

Ser Gln Thr Asn Gln Ser Ala Val Ser Thr Val Asp Ser Asn Gly Leu
        35                  40                  45

Asn Ala Gln Gly Gln Leu Thr Glu Gln Glu Leu Lys Glu Gln Ala Leu
    50                  55                  60

Arg Glu Asn Gln Thr Ile Tyr Phe Ala Phe Asp Asn Ala Thr Ile Ala
65                  70                  75                  80

Ser Asp Tyr Glu Ala Met Leu Ala Ala His Ala Ala Tyr Leu Val Lys
                85                  90                  95

Asn Pro Ser Leu Arg Val Thr Ile Glu Gly His Ala Asp Glu Arg Gly
            100                 105                 110

Thr Pro Glu Tyr Asn Ile Ala Leu Gly Glu Arg Arg Ala Gln Ala Val
        115                 120                 125

Ala Lys Tyr Leu Glu Ala Leu Gly Val Gln Ala Gly Gln Leu Ser Ile
    130                 135                 140

Val Ser Tyr Gly Glu Glu Lys Pro Leu Val Leu Gly Gln Ser Glu Glu
145                 150                 155                 160

Ala Tyr Ala Lys Asn Arg Arg Ala Val Leu Val Tyr
                165                 170

<210> 14
<211> 1297
<212> PRT
<213> Vibrio cholerae

<220>
<221> PEPTIDE
<222> (1)..(1297)
<223> >sp|Q9KTN2|PUR4_VIBCH
      Phosphoribosylformylglycinamidine synthase (EC
      6.3.5.3) (FGAM synthase) (FGAMS)
      (Formylglycinamide ribotide amidotransferase)
      (FGARAT) (Formylglycinamide ribotide synthetase)

<400> 14
Met Arg Ile Leu Arg Gly Ser Pro Ala Leu Ser Glu Phe Arg Val Asn
1               5                   10                  15

Lys Leu Leu Thr Ala Cys Arg Glu Gln Gln Leu Pro Val Thr Gly Ile
            20                  25                  30

Tyr Ala Glu Phe Met His Phe Ala Asp Leu Lys Ala Glu Leu Asn Pro
        35                  40                  45

Gln Glu Leu Glu Lys Leu Glu Lys Leu Leu Thr Tyr Gly Pro Thr Ile

50                  55                  60

Gln Glu His Glu Pro Gln Gly Leu Leu Leu Leu Val Thr Pro Arg Pro
65                  70                  75                  80

Gly Thr Ile Ser Pro Trp Ser Ser Lys Ala Thr Asp Ile Ala His Asn
                85                  90                  95

Cys Gly Leu His Gly Ile Lys Arg Leu Glu Arg Gly Thr Ala Tyr Tyr
            100                 105                 110

Val Glu Ala Glu Thr Ala Leu Thr Ala Ala Gln Ile Ala Thr Leu Glu
        115                 120                 125

Ala Leu Leu His Asp Arg Met Met Glu Val Val Phe Ala Glu Leu Thr
    130                 135                 140

Asp Ala Gln Gln Leu Phe Ser Val Ala Glu Pro Ala Pro Met Ser Gln
145                 150                 155                 160

Val Asp Val Leu Ala Gly Gly Arg Arg Ala Leu Glu Glu Ala Asn Val
                165                 170                 175

Ser Leu Gly Leu Ala Leu Ala Glu Asp Glu Ile Asp Tyr Leu Val Glu
            180                 185                 190

Ser Phe Thr Lys Leu Gly Arg Asn Pro Asn Asp Ile Glu Leu Met Met
        195                 200                 205

Phe Ala Gln Ala Asn Ser Glu His Cys Arg His Lys Ile Phe Asn Ala
    210                 215                 220

Asp Trp Thr Ile Asp Gly Val Lys Gln Asp Lys Ser Leu Phe Lys Met
225                 230                 235                 240

Ile Lys Asn Thr Phe Glu Gln Thr Pro Asp Tyr Val Leu Ser Ala Tyr
                245                 250                 255

Lys Asp Asn Ala Ala Val Met Thr Gly Ser Thr Val Gly Arg Phe Phe
            260                 265                 270

Pro Asp Pro Glu Ser Arg Gln Tyr Thr Tyr His His Glu Asp Ala His
        275                 280                 285

Ile Leu Met Lys Val Glu Thr His Asn His Pro Thr Ala Ile Ser Pro
    290                 295                 300

Trp Pro Gly Ala Ser Thr Gly Ser Gly Gly Glu Ile Arg Asp Glu Gly
305                 310                 315                 320

Ala Thr Gly Ile Gly Gly Lys Pro Lys Ala Gly Leu Val Gly Phe Thr
                325                 330                 335

Thr Ser Asn Leu Arg Ile Pro Gly Phe Glu Gln Pro Trp Glu Ser Asp
            340                 345                 350

Phe Gly Lys Pro Ser Arg Ile Val Asn Ala Leu Asp Ile Met Leu Glu
        355                 360                 365

Gly Pro Leu Gly Gly Ala Ala Phe Asn Asn Glu Phe Gly Arg Pro Asn
    370                 375                 380

Leu Leu Gly Tyr Phe Arg Thr Tyr Glu Glu Lys Val Thr Ser His Ala
385                 390                 395                 400

Gly Glu Glu Val Arg Gly Tyr His Lys Pro Ile Met Ile Ala Gly Gly
                405                 410                 415

Met Gly Asn Ile Arg Ala Glu His Ile Gln Lys Lys Glu Ile Pro Val
            420                 425                 430

Gly Ala Lys Leu Ile Val Leu Gly Gly Pro Ala Met Asn Ile Gly Leu
        435                 440                 445

Gly Gly Gly Ala Ala Ser Ser Met Ala Ser Gly Gln Ser Ala Glu Asp
    450                 455                 460

Leu Asp Phe Ala Ser Val Gln Arg Glu Asn Pro Glu Met Glu Arg Arg
465                 470                 475                 480

Cys Gln Glu Val Ile Asp Arg Cys Trp Gln Leu Gly Asp Lys Asn Pro
                485                 490                 495

Ile Ala Phe Ile His Asp Val Gly Ala Gly Gly Ile Ser Asn Ala Leu
            500                 505                 510

Pro Glu Leu Val Asn Asp Gly Asp Arg Gly Gly Lys Phe Gln Leu Arg
        515                 520                 525

Asn Val Pro Asn Asp Glu Pro Gly Met Ser Pro Leu Glu Ile Trp Cys
    530                 535                 540

Asn Glu Ser Gln Glu Arg Tyr Val Leu Ala Val Ala Ala Glu Asp Met
545                 550                 555                 560

Pro Leu Phe Asp Ala Ile Cys Gln Arg Glu Arg Ala Pro Tyr Ala Val
                565                 570                 575

Val Gly Glu Ala Thr Glu Glu Arg His Leu Thr Leu Glu Asp Ser His
            580                 585                 590

Phe Ala Asn Thr Pro Ile Asp Met Pro Met Asp Ile Leu Leu Gly Lys
        595                 600                 605

Pro Pro Lys Met His Arg Glu Ala Ser Thr Leu Lys Val Ser Ser Pro
    610                 615                 620

Ala Leu Glu Arg Ser Gly Ile Glu Leu Asn Glu Ala Val Asp Arg Val
625                 630                 635                 640

Leu Arg Leu Pro Ala Val Ala Glu Lys Thr Phe Leu Ile Thr Ile Gly
                645                 650                 655

Asp Arg Ser Val Thr Gly Leu Val Ala Arg Asp Gln Met Val Gly Pro
            660                 665                 670

Trp Gln Val Pro Val Ala Asn Cys Ala Val Thr Ala Ala Ser Phe Asp
        675                 680                 685

Ser Tyr His Gly Glu Ala Met Ser Met Gly Glu Arg Thr Pro Val Ala
    690                 695                 700

Leu Leu Asp Phe Gly Ala Ser Ala Arg Leu Ala Val Gly Glu Ala Ile
705                 710                 715                 720

Thr Asn Ile Ala Ala Thr Asp Ile Gly Glu Leu Lys Arg Ile Lys Leu
                725                 730                 735

Ser Ala Asn Trp Met Ser Pro Ala Gly His Pro Gly Glu Asp Ala Gly
            740                 745                 750

Leu Tyr Glu Ala Val Lys Ala Val Gly Glu Glu Leu Cys Pro Ala Leu
        755                 760                 765

Gly Ile Thr Ile Pro Val Gly Lys Asp Ser Met Ser Met Lys Thr Lys
    770                 775                 780

Trp Gln Glu Asn Gly Glu Gln Lys Glu Val Thr Ser Pro Leu Ser Leu
785                 790                 795                 800

Ile Ile Thr Ala Phe Ala Arg Val Glu Asp Ile Arg Lys Thr Val Thr
                805                 810                 815

Pro Gln Leu Arg Thr Asp Leu Gly Glu Thr Ser Leu Ile Leu Ile Asp
            820                 825                 830

Leu Gly Asn Gly Gln Asn Arg Leu Gly Ala Thr Ala Leu Ala Gln Val
        835                 840                 845

Tyr Lys Gln Leu Gly Asp Lys Pro Ala Asp Val Asp Asn Ala Ala Gln
    850                 855                 860

Leu Lys Gly Phe Phe Asp Ala Val Gln Thr Leu Val Arg Asn Asp Lys
865                 870                 875                 880

Leu Val Ala Tyr His Asp Lys Gly Asp Gly Gly Leu Leu Val Thr Leu
                885                 890                 895

Ala Glu Met Ala Phe Ala Gly His Cys Gly Ile Lys Ala Asn Ile Glu
            900                 905                 910

Thr Leu Gly Asp Asp Ala Leu Ala Ala Leu Phe Asn Glu Glu Leu Gly
        915                 920                 925

Ala Val Val Gln Val Lys Asn Asp Glu Leu Asn Ala Val Leu Ala Thr
    930                 935                 940

Leu Ala Ala His Gly Leu Glu Ala Cys Ala His Val Ile Gly Glu Val
945                 950                 955                 960

Glu Ala Ser Asp Arg Leu Leu Ile Thr Cys Gly Glu Glu Val Leu Ile
                965                 970                 975

Glu Arg Ser Arg Thr Glu Leu Arg Thr Ile Trp Ala Glu Met Thr His
            980                 985                 990

Lys Met Gln Ala Leu Arg Asp Asn Ser Ala Cys Ala Asp Gln Glu Phe
        995                 1000                1005

Ala Ala Lys Gln Asp Asn Arg Asp Pro Gly Leu Asn Ala Lys Leu Thr
    1010                1015                1020

Tyr Asp Val Gln Ala Asp Val Ala Ala Pro Tyr Ile Ala Lys Gly Val

1025                1030                1035                1040

Arg Pro Lys Met Ala Ile Leu Arg Glu Gln Gly Val Asn Ser His Val
                1045                1050                1055

Glu Met Ala Ala Ala Phe Asp Arg Ala Gly Phe Asp Ala Val Asp Val
            1060                1065                1070

His Met Ser Asp Ile Leu Thr Gly Gln Thr Val Leu Asp Ala Tyr Gln
        1075                1080                1085

Gly Leu Val Ala Cys Gly Gly Phe Ser Tyr Gly Asp Val Leu Gly Ala
    1090                1095                1100

Gly Glu Gly Trp Ala Lys Ser Ile Leu Phe Asn Ala Gln Ala Arg Glu
1105                1110                1115                1120

Gln Phe Glu Gln Phe Phe Gln Arg Lys Asp Thr Phe Ser Leu Gly Val
                1125                1130                1135

Cys Asn Gly Cys Gln Met Leu Ser Asn Leu Arg Asp Leu Ile Pro Gly
            1140                1145                1150

Ala Glu Leu Trp Pro Arg Phe Val Arg Asn Glu Ser Asp Arg Phe Glu
        1155                1160                1165

Ala Arg Phe Ser Leu Val Glu Val Gln Lys Ser Pro Ser Leu Phe Phe
    1170                1175                1180

Ser Glu Met Ala Gly Ser Arg Met Pro Ile Ala Val Ser His Gly Glu
1185                1190                1195                1200

Gly Arg Val Glu Val Arg Asp Ala Gln His Leu Ala Ala Ile Glu Gln
                1205                1210                1215

Ser Gly Thr Val Ala Ile Arg Phe Val Asp Asn Phe Gly Gln Pro Thr
            1220                1225                1230

Gln Ala Tyr Pro Ser Asn Pro Asn Gly Ser Pro Asn Ala Ile Thr Gly
        1235                1240                1245

Leu Thr Thr Gln Asp Gly Arg Val Thr Ile Met Met Pro His Pro Glu
    1250                1255                1260

Arg Val Phe Arg Thr Val Ala Asn Ser Trp His Pro Asp Asn Trp Gly
1265                1270                1275                1280

Glu Asn Gly Ala Trp Met Arg Met Phe Gln Asn Ala Arg Lys Tyr Phe
                1285                1290                1295

Gly

<210> 15
<211> 110
<212> PRT
<213> Vibrio cholerae

<220>
<221> PEPTIDE

<222> (1)..(110)
<223> >sp|Q9KNY9|RL22_VIBCH 50S ribosomal protein L22 -
      Vibrio cholerae. (110 aa)

<400> 15
Met Glu Ala Ile Ala Lys His Asn Phe Ala Arg Ile Ser Pro Gln Lys
1               5                   10                  15

Ala Arg Leu Val Ala Asp Gln Ile Arg Gly Lys Ser Val Asp Gln Ala
            20                  25                  30

Leu Glu Leu Leu Thr Phe Ser Asn Lys Lys Ala Ala Glu Leu Val Lys
        35                  40                  45

Lys Val Leu Glu Ser Ala Ile Ala Asn Ala Glu His Asn Glu Gly Ala
    50                  55                  60

Asp Ile Asp Asp Leu Arg Val Ala Lys Ile Phe Val Asp Glu Gly Pro
65                  70                  75                  80

Val Met Lys Arg Ile Met Pro Arg Ala Lys Gly Arg Ala Asp Arg Ile
                85                  90                  95

Leu Lys Arg Ser Ser His Ile Thr Val Val Val Ala Asp Arg
            100                 105                 110

<210> 16
<211> 233
<212> PRT
<213> Vibrio cholerae

<220>
<221> PEPTIDE
<222> (1)..(233)
<223> >sp|Q9KV33|RL1_VIBCH 50S ribosomal protein L1 -
      Vibrio cholerae. (233 aa)

<400> 16
Met Ala Lys Leu Thr Lys Arg Met Arg Thr Ile Arg Ala Lys Val Asp
1               5                   10                  15

Val Thr Lys Glu Tyr Asp Ile Asn Glu Ala Val Ala Leu Leu Lys Glu
            20                  25                  30

Leu Ala Thr Ala Lys Phe Val Glu Ser Val Asp Val Ala Val Asn Leu
        35                  40                  45

Gly Ile Asp Ala Arg Lys Ser Asp Gln Asn Val Arg Gly Ala Thr Val
    50                  55                  60

Leu Pro His Gly Thr Gly Arg Asp Ile Arg Val Ala Val Phe Thr Gln
65                  70                  75                  80

Gly Ala Asn Ala Glu Ala Ala Lys Ala Ala Gly Ala Glu Leu Val Gly
                85                  90                  95

Met Glu Asp Leu Ala Asp Leu Val Lys Lys Gly Glu Met Asn Phe Asp
            100                 105                 110

Val Val Ile Ala Ser Pro Asp Ala Met Arg Val Val Gly Gln Leu Gly
        115                 120                 125

Thr Ile Leu Gly Pro Arg Gly Leu Met Pro Asn Pro Lys Val Gly Thr
    130                 135                 140

Val Thr Pro Asn Val Ala Glu Ala Val Lys Asn Ala Lys Ala Gly Gln
145                 150                 155                 160

Val Arg Tyr Arg Asn Asp Lys Asn Gly Ile Ile His Thr Thr Ile Gly
                165                 170                 175

Lys Val Thr Phe Glu Ala Asp Gln Leu Lys Glu Asn Leu Glu Ala Leu
            180                 185                 190

Leu Val Ala Leu Lys Lys Ala Lys Pro Ser Ser Ala Lys Gly Val Phe
        195                 200                 205

Val Lys Lys Val Ser Ile Ser Thr Thr Met Gly Ala Gly Val Ala Val
    210                 215                 220

Asp Gln Asn Thr Leu Ser Ala Gln Val
225                 230


<210> 17
<211> 330
<212> PRT
<213> Vibrio cholerae

<220>
<221> PEPTIDE
<222> (1)..(330)
<223> >sp|Q9KP08|RPOA_VIBCH DNA-directed RNA polymerase
      alpha chain (EC 2.7.7.6) (RNAP alpha subunit)
      (Transcriptase alpha chain) (RNA polymerase alpha
      subunit) - Vibrio cholerae. (330 aa)

<400> 17
Met Gln Gly Ser Val Thr Glu Phe Leu Lys Pro Arg Leu Val Asp Ile
1               5                   10                  15

Glu Gln Ile Ser Thr Thr His Ala Lys Val Thr Leu Glu Pro Leu Glu
            20                  25                  30

Arg Gly Phe Gly His Thr Leu Gly Asn Ala Leu Arg Arg Ile Leu Leu
        35                  40                  45

Ser Ser Met Pro Gly Cys Ala Val Thr Glu Val Glu Ile Glu Gly Val
    50                  55                  60

Leu His Glu Tyr Ser Thr Lys Glu Gly Val Gln Glu Asp Ile Leu Glu
65                  70                  75                  80

Ile Leu Leu Asn Leu Lys Gly Leu Ala Val Arg Val Ala Glu Gly Lys
                85                  90                  95

Asp Glu Val Phe Ile Thr Leu Asn Lys Ser Gly Ser Gly Pro Val Val
            100                 105                 110

Ala Gly Asp Ile Thr His Asp Gly Asp Val Glu Ile Val Asn Pro Glu

115                 120                 125

His Val Ile Cys His Leu Thr Ser Asp Asn Ala Ala Ile Ala Met Arg
    130                 135                 140

Ile Lys Val Glu Arg Gly Arg Gly Tyr Val Pro Ala Ser Ala Arg Ile
145                 150                 155                 160

His Thr Glu Glu Asp Glu Arg Pro Ile Gly Arg Leu Leu Val Asp Ala
                165                 170                 175

Thr Phe Ser Pro Val Asp Lys Ile Ala Tyr Ser Val Glu Ala Ala Arg
            180                 185                 190

Val Glu Gln Arg Thr Asp Leu Asp Lys Leu Val Ile Asp Met Glu Thr
        195                 200                 205

Asn Gly Thr Leu Glu Pro Glu Glu Ala Ile Arg Arg Ala Ala Thr Ile
    210                 215                 220

Leu Ala Glu Gln Leu Asp Ala Phe Val Asp Leu Arg Asp Val Arg Val
225                 230                 235                 240

Pro Glu Glu Lys Glu Glu Lys Pro Glu Phe Asp Pro Ile Leu Leu Arg
                245                 250                 255

Pro Val Asp Asp Leu Glu Leu Thr Val Arg Ser Ala Asn Cys Leu Lys
            260                 265                 270

Ala Glu Ala Ile His Tyr Ile Gly Asp Leu Val Gln Arg Thr Glu Val
        275                 280                 285

Glu Leu Leu Lys Thr Pro Asn Leu Gly Lys Lys Ser Leu Thr Glu Ile
    290                 295                 300

Lys Asp Val Leu Ala Ser Arg Gly Leu Ser Leu Gly Met Arg Leu Glu
305                 310                 315                 320

Asn Trp Pro Pro Ala Ser Ile Ala Glu Asp
                325                 330


<210> 18
<211> 274
<212> PRT
<213> Vibrio cholerae

<220>
<221> PEPTIDE
<222> (1)..(274)
<223> >sp|Q9KNY7|RL2_VIBCH 50S ribosomal protein L2 -
      Vibrio cholerae.(274 aa)


<400> 18
Met Ala Ile Val Lys Cys Lys Pro Thr Ser Ala Gly Arg Arg His Val
1               5                   10                  15

Val Lys Val Val Asn Ala Asp Leu His Lys Gly Lys Pro Tyr Ala Pro
            20                  25                  30

Leu Leu Glu Lys Asn Ser Lys Asn Gly Gly Arg Asn Asn Asn Gly Arg
        35                  40                  45

Ile Thr Val Arg His Ile Gly Gly Gly His Lys Gln His Tyr Arg Leu
    50                  55                  60

Val Asp Phe Lys Arg Thr Lys Asp Gly Ile Pro Ala Lys Val Glu Arg
65                  70                  75                  80

Leu Glu Tyr Asp Pro Asn Arg Ser Ala Asn Ile Ala Leu Val Leu Tyr
                85                  90                  95

Ala Asp Gly Glu Arg Arg Tyr Ile Ile Ala Pro Lys Gly Leu Gln Ala
            100                 105                 110

Gly Asp Val Ile Gln Ser Gly Pro Asp Ala Pro Ile Lys Ala Gly Asn
        115                 120                 125

Ala Met Pro Met Arg Asn Ile Pro Val Gly Ser Thr Ile His Asn Val
    130                 135                 140

Glu Leu Thr Pro Gly Lys Gly Ala Gln Leu Ala Arg Ser Ala Gly Ala
145                 150                 155                 160

Tyr Ala Gln Leu Val Ala Arg Asp Gly Ala Tyr Val Thr Leu Arg Leu
                165                 170                 175

Arg Ser Gly Glu Met Arg Lys Val Leu Ser Glu Gly Arg Ala Thr Ile
            180                 185                 190

Gly Glu Val Gly Asn Ala Glu His Met Leu Arg Glu Leu Gly Lys Ala
        195                 200                 205

Gly Ala Ala Arg Trp Arg Gly Val Arg Pro Thr Val Arg Gly Val Val
    210                 215                 220

Met Asn Pro Val Asp His Pro His Gly Gly Gly Glu Gly Arg Thr Ser
225                 230                 235                 240

Gly Gly Arg His Pro Val Ser Pro Trp Gly Val Pro Thr Lys Gly Tyr
                245                 250                 255

Lys Thr Arg Ser Asn Lys Arg Thr Asp Lys Tyr Ile Val Arg Arg Arg
            260                 265                 270

Asn Lys

<210> 19
<211> 504
<212> PRT
<213> Vibrio cholerae

<220>
<221> PEPTIDE
<222> (1)..(504)
<223> >tr|Q9KT99|Q9KT99_VIBCH
Amidophosphoribosyltransferase - Vibrio
cholerae.(504 aa)

<400> 19
Met Cys Gly Ile Val Gly Ile Val Gly Thr Thr Pro Val Asn Gln Ser
1               5                   10                  15

Ile Tyr Asp Ala Leu Thr Val Leu Gln His Arg Gly Gln Asp Ala Ala
            20                  25                  30

Gly Ile Cys Thr Ile Glu Ser Asn Arg Phe Arg Leu Arg Lys Ala Asn
        35                  40                  45

Gly Leu Val Arg Asp Val Phe Glu Ala Lys His Met Gln Arg Leu Gln
    50                  55                  60

Gly Asn Val Gly Ile Gly His Val Arg Tyr Pro Thr Ala Gly Ser Ser
65                  70                  75                  80

Ser Ala Ser Glu Ala Gln Pro Phe Tyr Val Asn Ser Pro Phe Gly Ile
                85                  90                  95

Thr Leu Ala His Asn Gly Asn Leu Thr Asn Ala Asn Gln Val Arg Gln
            100                 105                 110

Lys Leu Phe Glu Lys Asp Arg Arg His Val Asn Thr Thr Ser Asp Ser
        115                 120                 125

Glu Val Leu Leu Asn Val Leu Ala His Glu Ile Asp Thr Val Lys Gly
    130                 135                 140

Asn Val Thr Ala Asp Asp Val Phe Arg Ala Ile Ser Asn Val His Arg
145                 150                 155                 160

Thr Ile Arg Gly Ala Tyr Ala Val Ala Ala Met Ile Ile Gly His Gly
                165                 170                 175

Met Ile Ala Phe Arg Asp Pro His Gly Ile Arg Pro Leu Cys Leu Gly
            180                 185                 190

Lys Arg Glu Val Asn Gly Gln Leu Glu Tyr Met Val Ala Ser Glu Ser
        195                 200                 205

Val Ala Leu Asp Ala Val Gly Phe Asp Phe Val Arg Asp Val Ala Pro
    210                 215                 220

Gly Glu Ala Ile Tyr Ala Thr Phe Asp Gly Glu Leu Tyr Thr Lys Gln
225                 230                 235                 240

Cys Ala Asp Asn Pro Ala Leu Asn Pro Cys Ile Phe Glu Phe Val Tyr
                245                 250                 255

Phe Ala Arg Pro Asp Ser Phe Ile Asp Lys Ile Ser Val Tyr Ser Ala
            260                 265                 270

Arg Val Glu Met Gly Lys Arg Leu Gly Glu Arg Ile Lys Asn Asp Tyr
        275                 280                 285

Ser Asp Leu Asp Ile Asp Val Val Ile Pro Ile Pro Glu Thr Ser Cys
    290                 295                 300

Asp Ile Ala Leu Gln Ile Ala Gln Ala Ile Asp Lys Pro Tyr Arg Gln
305                 310                 315                 320

Gly Phe Val Lys Asn Arg Tyr Val Gly Arg Thr Phe Ile Met Pro Gly
                325                 330                 335

Gln Gln Gln Arg Lys Lys Ser Val Arg Arg Lys Leu Asn Ala Ile Arg
            340                 345                 350

Ser Glu Phe Lys Asp Lys Asn Val Leu Leu Val Asp Asp Ser Ile Val
        355                 360                 365

Arg Gly Thr Thr Ser Glu Gln Ile Ile Glu Met Ala Arg Asp Ser Gly
    370                 375                 380

Ala Lys Lys Val Tyr Ile Val Ser Ala Ala Pro Glu Ile Arg Phe Pro
385                 390                 395                 400

Asn Val Tyr Gly Ile Asp Met Pro Ser Ala Asn Glu Leu Ile Ala His
                405                 410                 415

Gly Arg Asp Asn Asp Ala Ile Cys Lys Gln Ile Gly Ala Asp Ala Leu
            420                 425                 430

Ile Phe Gln Thr Leu Glu Asp Leu Val Glu Ala Val Arg Cys Gly Asn
        435                 440                 445

Pro Asp Ile Val Lys Phe Glu Ala Ser Val Phe Asn Gly Glu Tyr Val
    450                 455                 460

Thr Gly Asp Ile Asp Gln Gln Tyr Leu Asp Phe Leu Glu Ser Met Arg
465                 470                 475                 480

Ser Asp Asp Ala Lys Val Gln Arg Glu Ile Gln Gln Asp Leu Ala Asn
                485                 490                 495

Leu Glu Leu His Asn Glu Gly Ala
            500


<210> 20
<211> 366
<212> PRT
<213> Vibrio cholerae

<220>
<221> PEPTIDE
<222> (1)..(366)
<223> >tr|Q9KS36|Q9KS36_VIBCH Spermidine/putrescine ABC
      transporter, periplasmic
      spermidine/putrescine-binding protein - Vibrio
      cholerae. (366 aa)


<400> 20
Met Thr Glu Val Ile Leu Phe Phe Phe Ala Thr Pro Lys Arg Ala Thr
1               5                   10                  15

Phe Ser Leu Glu Leu Thr Ser Met Lys Lys Leu Ala Thr Leu Leu Ala
            20                  25                  30

Gly Ser Ala Cys Ala Leu Ser Leu Leu Ser Gly Thr Ala Ala Ala Glu
        35                  40                  45

Asp Lys Glu Leu Val Phe Met Asn Trp Gly Pro Tyr Ile Asn Ser Gly
50                  55                  60

Ile Leu Glu Gln Phe Thr Lys Glu Thr Gly Ile Lys Val Ile Tyr Ser
65                  70                  75                  80

Thr Tyr Glu Ser Asn Glu Thr Leu Tyr Ala Lys Leu Lys Thr His Asn
                85                  90                  95

Gln Gly Tyr Asp Leu Val Val Pro Ser Thr Tyr Phe Val Ala Lys Met
            100                 105                 110

Arg Asp Glu Gly Met Leu Gln Lys Ile Asp Lys Ser Lys Leu Ser Asn
        115                 120                 125

Phe Lys Asn Leu Asp Ser Asn Tyr Leu Asp Lys Pro Tyr Asp Pro Asn
    130                 135                 140

Asn Asp Tyr Ser Ile Pro His Val Val Ala Ile Thr Gly Leu Ala Val
145                 150                 155                 160

Asn Thr Asp Met Tyr Asp Pro Asn Asp Phe Gln Ser Trp Gly Asp Leu
                165                 170                 175

Trp Lys Pro Glu Leu Lys Gly Gln Leu Met Leu Met Asp Asp Thr Arg
            180                 185                 190

Glu Val Phe His Ile Ala Leu Arg Lys Leu Gly Tyr Ser Gly Asn Ser
        195                 200                 205

Thr Asp Asp Lys Gln Ile Asp Glu Ala Tyr Ala Glu Leu Gln Lys Leu
    210                 215                 220

Met Pro Asn Val Leu Val Phe Asn Ser Asp Asn Pro Ala Ala Pro Tyr
225                 230                 235                 240

Leu Ser Gly Glu Val Gly Leu Gly Met Leu Trp Asn Gly Ser Ala Ala
                245                 250                 255

Ala Ala Gln Lys Glu Gly Leu Pro Ile Lys Leu Val Phe Pro Lys Glu
            260                 265                 270

Gly Gly Ile Gly Trp Val Asp Asn Phe Ala Ile Ser Ser Gly Ala Lys
        275                 280                 285

Asn Val Asp Ala Ala His Lys Met Ile Asp Phe Leu Leu Arg Pro Glu
    290                 295                 300

Ile Ala Glu Gln Ile Ser Arg Asp Thr Gly Tyr Leu Thr Gly Val Lys
305                 310                 315                 320

Ala Ser Asn Asp Lys Phe Gln Asp Val Ala Pro Leu Phe Pro Ser Gln
                325                 330                 335

Glu Asp Leu Asp Arg Val Glu Trp Gln Ser Ala Val Gly Asp Lys Thr
            340                 345                 350

Val Lys Tyr Glu Glu Tyr Phe Met Lys Leu Lys Ala Gly Gln
        355                 360                 365

<210> 21
<211> 635
<212> PRT
<213> Vibrio cholerae

<220>
<221> PEPTIDE
<222> (1)..(635)
<223> >sp|P22359|HTPG_VIBCH Chaperone protein htpG (Heat
      shock protein htpG) (High temperature protein G) -
      Vibrio cholerae. (635 aa)

<400> 21
Met Ser Glu Thr Ala Thr Thr Asn Lys Glu Thr Arg Gly Phe Gln Ser
1               5                   10                  15

Glu Val Lys Gln Leu Leu His Leu Met Ile His Ser Leu Tyr Ser Asn
            20                  25                  30

Lys Glu Ile Phe Leu Arg Glu Leu Ile Ser Asn Ala Ser Asp Ala Val
        35                  40                  45

Asp Lys Leu Arg Phe Gln Ala Leu Ser His Pro Asp Leu Tyr Gln Gly
    50                  55                  60

Asp Ala Glu Leu Gly Val Lys Leu Ser Phe Asp Lys Asp Lys Asn Thr
65                  70                  75                  80

Leu Thr Ile Ser Asp Asn Gly Ile Gly Met Thr Arg Asp Glu Val Ile
                85                  90                  95

Glu Asn Leu Gly Thr Ile Ala Lys Ser Gly Thr Ala Glu Phe Phe Ser
            100                 105                 110

Lys Leu Ser Gln Glu Gln Ser Lys Asn Ser Gln Leu Ile Gly Gln Phe
        115                 120                 125

Gly Val Gly Phe Tyr Ser Ala Phe Ile Val Ala Asp Ala Val Thr Val
    130                 135                 140

Arg Thr Arg Ala Ala Gly Ser Ala Pro Ala Asp Ala Val Gln Trp Tyr
145                 150                 155                 160

Ser Lys Gly Glu Gly Glu Tyr Thr Val Glu Thr Ile Asn Lys Glu Ser
                165                 170                 175

Arg Gly Thr Asp Ile Ile Leu His Leu Arg Glu Glu Gly Lys Glu Phe
            180                 185                 190

Leu Ser Glu Trp Arg Leu Arg Asp Val Ile Ser Lys Tyr Ser Asp His
        195                 200                 205

Ile Gly Ile Pro Val Tyr Ile Gln Thr Ser Val Met Asp Glu Glu Gly
    210                 215                 220

Lys Ala Thr Glu Glu Thr Lys Trp Glu Gln Ile Asn Lys Ala Gln Ala
225                 230                 235                 240

Leu Trp Thr Arg Ala Lys Ser Glu Val Thr Asp Glu Glu Tyr Lys Glu
                245                 250                 255

Phe Tyr Lys His Val Ser His Asp Phe Ala Asp Pro Leu Val Trp Ser
            260                 265                 270

His Asn Lys Val Glu Gly Lys Asn Asp Tyr Thr Ser Leu Leu Tyr Ile
        275                 280                 285

Pro Ala Lys Ala Pro Trp Asp Leu Phe Asn Arg Glu His Lys His Gly
    290                 295                 300

Leu Lys Leu Tyr Val Gln Arg Val Phe Ile Met Asp Asp Ala Ala Gln
305                 310                 315                 320

Phe Met Pro Ser Tyr Leu Arg Phe Val Arg Gly Leu Ile Asp Ser Asn
                325                 330                 335

Asp Leu Pro Leu Asn Val Ser Arg Glu Ile Leu Gln Asp Asn Lys Ile
            340                 345                 350

Thr Gln Ser Leu Arg Gln Ala Cys Thr Lys Arg Val Leu Thr Met Leu
        355                 360                 365

Glu Arg Met Ala Ser Asn Asp Ala Asp Asn Tyr Gln Lys Phe Trp Lys
    370                 375                 380

Glu Phe Gly Leu Val Met Lys Glu Gly Pro Ala Glu Asp Phe Ala Asn
385                 390                 395                 400

Arg Glu Lys Ile Ala Ser Leu Leu Arg Phe Ala Ser Thr His Ile Asp
                405                 410                 415

Ser Ala Glu Gln Thr Ile Ser Leu Ala Ser Tyr Val Glu Arg Met Lys
            420                 425                 430

Glu Gly Gln Asp Lys Ile Tyr Tyr Leu Thr Ala Asp Ser Tyr Thr Ala
        435                 440                 445

Ala Lys Asn Ser Pro His Leu Glu Gln Phe Lys Ser Lys Gly Ile Glu
    450                 455                 460

Val Ile Leu Met Phe Asp Arg Ile Asp Glu Trp Leu Met Asn Tyr Leu
465                 470                 475                 480

Pro Glu Phe Glu Gly Lys Ala Phe Gln Ser Ile Thr Lys Ala Gly Leu
                485                 490                 495

Asp Leu Ser Gln Phe Glu Asp Glu Ala Glu Lys Glu Lys His Lys Glu
            500                 505                 510

Thr Glu Glu Gln Phe Lys Ser Val Val Glu Arg Leu Lys Gly Tyr Leu
        515                 520                 525

Gly Ser Arg Val Lys Glu Val Arg Thr Thr Phe Lys Leu Ala Asn Thr
    530                 535                 540

Pro Ala Val Val Val Thr Asp Asp Tyr Glu Met Gly Thr Gln Met Ala
545                 550                 555                 560

Lys Leu Leu Ala Ala Ala Gly Gln Pro Val Pro Glu Val Lys Tyr Ile
                565                 570                 575

Leu Glu Val Asn Pro Glu His Ala Leu Val Lys Arg Met Ala Asp Glu

580                 585                 590

Ala Asp Glu Gln Thr Phe Gly Arg Trp Ala Glu Val Leu Leu Gly Gln
        595                 600                 605

Ala Met Leu Ala Glu Arg Gly Ser Met Glu Asp Pro Ser Gln Phe Leu
    610                 615                 620

Gly Ala Val Asn Gln Leu Leu Ala Pro Ser His
625                 630                 635


<210> 22
<211> 347
<212> PRT
<213> Vibrio cholerae

<220>
<221> PEPTIDE
<222> (1)..(347)
<223> >tr|Q9ZA92|Q9ZA92_VIBCH Rod shape-determining
      protein MreB - Vibrio cholerae. (347 aa)

<400> 22
Met Phe Lys Lys Leu Arg Gly Met Phe Ser Asn Asp Leu Ser Ile Asp
1               5                   10                  15

Leu Gly Thr Ala Asn Thr Leu Ile Tyr Val Lys Gly Gln Gly Ile Val
            20                  25                  30

Leu Asp Glu Pro Ser Val Val Ala Ile Arg Gln Asp Lys Gly Arg Gly
        35                  40                  45

Gly Lys Thr Val Ala Ala Val Gly His Ala Ala Lys Gln Met Leu Gly
    50                  55                  60

Arg Thr Pro Gly Asn Ile Ser Ala Ile Arg Pro Met Lys Asp Gly Val
65                  70                  75                  80

Ile Ala Asp Phe Tyr Val Thr Glu Lys Met Leu Gln His Phe Ile Arg
                85                  90                  95

Gln Val His Asp Asn Ser Val Leu Lys Pro Ser Pro Arg Val Leu Val
            100                 105                 110

Cys Val Pro Cys Gly Ser Thr Gln Val Glu Arg Arg Ala Ile Arg Glu
        115                 120                 125

Ser Ala Leu Gly Ala Gly Ala Arg Glu Val Tyr Leu Ile Asp Glu Pro
    130                 135                 140

Met Ala Ala Ala Ile Gly Ala Gly Leu Arg Val Ser Glu Pro Thr Gly
145                 150                 155                 160

Ser Met Val Ile Asp Ile Gly Gly Gly Thr Thr Glu Val Ala Val Ile
                165                 170                 175

Ser Leu Asn Gly Val Val Tyr Ser Ser Ser Val Arg Ile Gly Gly Asp
            180                 185                 190

Arg Phe Asp Glu Ala Ile Ile Asn Tyr Val Arg Arg Asn Tyr Gly Ser

195                 200                 205

Leu Ile Gly Glu Ala Thr Ala Glu Lys Ile Lys His Glu Ile Gly Ser
    210                 215                 220

Ala Tyr Pro Gly Asp Asp Val Gln Glu Ile Glu Val Arg Gly Arg Asn
225                 230                 235                 240

Leu Ala Glu Gly Val Pro Arg Ser Phe Thr Leu Asn Ser Asn Glu Ile
                245                 250                 255

Leu Glu Ala Leu Gln Glu Pro Leu Thr Gly Ile Val Ser Ala Val Met
            260                 265                 270

Val Ala Leu Glu Gln Cys Pro Pro Glu Leu Ala Ser Asp Ile Ser Glu
        275                 280                 285

Asn Gly Met Val Leu Thr Gly Gly Gly Ala Leu Leu Lys Asp Leu Asp
    290                 295                 300

Arg Leu Leu Met Glu Glu Thr Gly Ile Pro Val Val Ile Ala Asp Asp
305                 310                 315                 320

Pro Leu Thr Cys Val Ala Arg Gly Gly Gly Lys Ala Leu Glu Met Ile
                325                 330                 335

Asp Met His Gly Gly Asp Leu Phe Ser Glu Glu
            340                 345

<210> 23
<211> 109
<212> PRT
<213> Vibrio cholerae

<220>
<221> PEPTIDE
<222> (1)..(109)
<223> >sp|Q9KT50|Y1055_VIBCH Hypothetical UPF0133
      protein VC1055 - Vibrio cholerae.

<400> 23
Met Phe Gly Lys Gly Gly Met Gly Asn Leu Met Lys Gln Ala Gln Gln
1               5                   10                  15

Met Gln Glu Arg Met Gln Lys Leu Gln Glu Glu Ile Ala Asn Met Glu
            20                  25                  30

Val Thr Gly Glu Ser Gly Ala Gly Leu Val Lys Val Thr Val Thr Gly
        35                  40                  45

Ser His Ser Val Arg Arg Val Asn Ile Asp Glu Ser Leu Met Glu Asp
    50                  55                  60

Asp Lys Glu Met Leu Glu Asp Leu Ile Ala Ala Ala Phe Asn Asp Ala
65                  70                  75                  80

Ala Arg Arg Ile Glu Glu Thr Gln Lys Glu Lys Met Ala Ser Ile Thr
                85                  90                  95

Gly Gly Met Gln Leu Pro Pro Gly Met Lys Met Pro Phe
            100                 105

<210> 24
<211> 209
<212> PRT
<213> Vibrio cholerae

<220>
<221> PEPTIDE
<222> (1)..(209)
<223> >sp|Q9KNY4|RL3_VIBCH 50S ribosomal protein L3 -
      Vibrio cholerae. (209 aa)

<400> 24
Met Ile Gly Leu Ile Gly Arg Lys Val Gly Met Thr Arg Val Phe Thr
1               5                   10                  15

Glu Asp Gly Val Ser Ile Pro Val Thr Val Val Glu Val Glu Ala Asn
            20                  25                  30

Arg Val Ser Gln Val Lys Thr Leu Glu Thr Asp Gly Tyr Ala Ala Ile
        35                  40                  45

Gln Val Thr Thr Gly Ser Lys Lys Ala Asn Arg Val Thr Lys Pro Glu
    50                  55                  60

Ala Gly His Phe Ala Lys Ala Gly Val Glu Ala Gly Arg Gly Leu Trp
65                  70                  75                  80

Glu Phe Arg Leu Glu Asn Gly Glu Glu Phe Ala Val Gly Ser Glu Leu
                85                  90                  95

Thr Val Glu Leu Phe Asn Glu Val Lys Lys Val Asp Val Thr Gly Thr
            100                 105                 110

Ser Lys Gly Lys Gly Phe Gln Gly Ala Val Lys Arg Trp Asn Phe Arg
        115                 120                 125

Thr Gln Asp Met Thr His Gly Asn Ser Leu Ser His Arg Ala Pro Gly
    130                 135                 140

Ser Ile Gly Gln Cys Gln Thr Pro Gly Arg Val Phe Lys Gly Lys Lys
145                 150                 155                 160

Met Ala Gly His Met Gly Ala Glu Arg Val Thr Thr Gln Asn Leu Glu
                165                 170                 175

Ile Val Arg Val Asp Ala Glu Arg Asn Leu Leu Leu Ile Lys Gly Ala
            180                 185                 190

Val Pro Gly Ala Thr Gly Gly Asn Val Ile Val Lys Pro Ala Val Lys
        195                 200                 205

Ala

<210> 25

<211> 242
<212> PRT
<213> Vibrio cholerae

<220>
<221> PEPTIDE
<222> (1)..(242)
<223> >sp|Q9KPV2|RS2_VIBCH 30S ribosomal protein S2 -
      Vibrio cholerae. (242 aa)

<400> 25
Met Ala Ser Val Ser Met Arg Asp Met Leu Thr Ala Gly Val His Phe
1               5                   10                  15

Gly His Gln Thr Arg Tyr Trp Asn Pro Lys Met Lys Gln Phe Ile Phe
            20                  25                  30

Gly Ala Arg Asn Arg Val His Ile Ile Asn Leu Glu Lys Thr Val Pro
        35                  40                  45

Met Phe Asn Glu Ala Leu Ala Glu Leu Ala Lys Val Gly Glu Lys Lys
    50                  55                  60

Gly Lys Val Leu Phe Val Gly Thr Lys Arg Ala Ala Ser Glu Ser Val
65                  70                  75                  80

Lys Glu Ala Ala Leu Ala Ser Asn Gln Tyr Tyr Val Asn Asn Arg Trp
                85                  90                  95

Leu Gly Gly Met Leu Thr Asn Trp Lys Thr Val Arg Gln Ser Ile Lys
            100                 105                 110

Arg Leu Lys Glu Leu Glu Val Gln Ser Thr Asp Gly Thr Phe Asp Lys
        115                 120                 125

Leu Thr Lys Lys Glu Ala Leu Met Arg Thr Arg Glu Met Glu Lys Leu
    130                 135                 140

Glu Lys Ser Leu Gly Gly Ile Lys Asp Met Gly Gly Leu Pro Asp Ala
145                 150                 155                 160

Leu Phe Val Ile Asp Ala Asp His Glu His Ile Ala Ile Lys Glu Ala
                165                 170                 175

Asn Asn Leu Gly Ile Pro Val Phe Ala Val Val Asp Thr Asn Ser Ser
            180                 185                 190

Pro Asp Gly Val Asp Tyr Ile Ile Pro Gly Asn Asp Asp Ala Ile Arg
        195                 200                 205

Ala Val Gln Leu Tyr Leu Asn Ala Ala Ala Gln Ala Ile Asn Glu Gly
    210                 215                 220

Arg Asn Lys Asp Val Ala Ala Val Ala Glu Lys Asp Gly Phe Val Glu
225                 230                 235                 240

Ala Glu


<210> 26

<211> 556
<212> PRT
<213> Vibrio cholerae

<220>
<221> PEPTIDE
<222> (1)..(556)
<223> >tr|Q9KQT3|Q9KQT3_VIBCH Ribosomal protein S1 -
      Vibrio cholerae. (556 aa)

<400> 26
Met Thr Glu Ser Phe Ala Gln Leu Phe Glu Glu Phe Leu Asn Glu Thr
1               5                   10                  15

Gln Phe Gln Gln Gly Thr Ile Val Lys Gly Thr Val Val Ala Ile Glu
            20                  25                  30

Asn Gly Tyr Val Leu Val Asp Ala Gly Leu Lys Ser Glu Ser Ala Ile
        35                  40                  45

Pro Ala Glu Gln Phe Lys Asn Ala Ala Gly Glu Leu Glu Val Gln Val
    50                  55                  60

Gly Ser Gln Val Asp Val Ala Leu Asp Ala Val Glu Asp Gly Phe Gly
65                  70                  75                  80

Glu Thr Gln Leu Ser Arg Glu Lys Ala Lys Arg His Glu Ala Trp Ile
                85                  90                  95

Val Leu Glu Lys Ala Tyr Glu Glu Ala Ala Thr Val Ile Gly Ile Ile
            100                 105                 110

Asn Gly Lys Val Lys Gly Gly Phe Thr Val Glu Leu Asn Gly Ile Arg
        115                 120                 125

Ala Phe Leu Pro Gly Ser Leu Val Asp Val Arg Pro Ile Arg Asp Thr
    130                 135                 140

Ala His Leu Glu Asn Lys Glu Leu Glu Phe Lys Val Ile Lys Leu Asp
145                 150                 155                 160

Gln Lys Arg Asn Asn Val Val Val Ser Arg Arg Ala Val Ile Glu Ser
                165                 170                 175

Glu Ser Ser Val Glu Arg Asp Glu Leu Leu Glu Thr Leu Gln Glu Gly
            180                 185                 190

Thr Glu Val Lys Gly Ile Val Lys Asn Leu Thr Asp Tyr Gly Ala Phe
        195                 200                 205

Val Asp Leu Gly Gly Val Asp Gly Leu Leu His Ile Thr Asp Met Ala
    210                 215                 220

Trp Lys Arg Val Lys His Pro Ser Glu Ile Val Asn Val Gly Asp Glu
225                 230                 235                 240

Ile Leu Val Lys Val Leu Lys Phe Asp Arg Asp Arg Thr Arg Val Ser
                245                 250                 255

Leu Gly Leu Lys Gln Leu Gly Glu Asp Pro Trp Val Ala Ile Ala Lys
            260                 265                 270

Arg Tyr Pro Glu Gly His Lys Leu Ser Gly Arg Val Thr Asn Leu Thr
        275                 280                 285

Asp Tyr Gly Cys Phe Val Glu Ile Glu Glu Gly Val Glu Gly Leu Val
    290                 295                 300

His Val Ser Glu Met Asp Trp Thr Asn Lys Asn Ile His Pro Ser Lys
305                 310                 315                 320

Val Val Asn Val Gly Asp Glu Val Glu Val Met Val Leu Asp Ile Asp
                325                 330                 335

Glu Glu Arg Arg Arg Ile Ser Leu Gly Leu Lys Gln Cys Lys Ala Asn
            340                 345                 350

Pro Trp Gln Ser Phe Ala Glu Ala Gln Ala Lys Gly Asp Lys Val Thr
        355                 360                 365

Gly Lys Ile Lys Ser Ile Thr Asp Phe Gly Ile Phe Ile Gly Leu Glu
    370                 375                 380

Gly Gly Ile Asp Gly Leu Val His Leu Ser Asp Ile Ser Trp Asn Val
385                 390                 395                 400

Pro Gly Glu Glu Ala Val Arg Glu Phe Lys Lys Gly Asp Glu Ile Ser
                405                 410                 415

Ala Val Val Leu Ala Val Asp Ala Glu Arg Glu Arg Ile Ser Leu Gly
            420                 425                 430

Ile Lys Gln Met Glu Asn Asp Pro Phe Asn Ala Tyr Val Ser Asp Asn
        435                 440                 445

Lys Lys Gly Ala Leu Val Asn Gly Thr Val Thr Ala Val Asp Ala Lys
    450                 455                 460

Gly Ala Thr Ile Glu Leu Glu Asp Gly Val Glu Gly Tyr Ile Arg Ala
465                 470                 475                 480

Ser Glu Val Ser Arg Asp Arg Ile Glu Asp Ala Ser Leu Ile Leu Asn
                485                 490                 495

Val Gly Asp Lys Val Glu Ala Lys Phe Thr Gly Val Asp Arg Lys Asn
            500                 505                 510

Arg Val Ile Asn Leu Ser Ile Lys Ala Lys Asp Glu Ala Glu Glu Gln
        515                 520                 525

Glu Val Met Ala Thr Leu Asn Lys Gln Asp Glu Ala Ala Phe Gly Asn
    530                 535                 540

Ala Met Ala Asp Ala Phe Lys Ala Ala Lys Gly Glu
545                 550                 555

<210> 27
<211> 1341
<212> PRT
<213> Vibrio cholerae

<220>
<221> PEPTIDE
<222> (1)..(1341)
<223> >sp|Q9KV30|RPOB_VIBCH DNA-directed RNA polymerase
      beta chain (EC 2.7.7.6) (RNAP beta subunit)
      (Transcriptase beta chain) (RNA polymerase beta
      subunit) - Vibrio cholerae. (1341 aa)

<400> 27
Met Val Tyr Ser Tyr Thr Glu Lys Lys Arg Ile Arg Lys Asp Phe Gly
1               5                   10                  15

Thr Arg Pro Gln Val Leu Asp Ile Pro Tyr Leu Leu Ser Ile Gln Leu
            20                  25                  30

Asp Ser Phe Glu Lys Phe Ile Glu Gln Asp Pro Glu Gly Gln Tyr Gly
        35                  40                  45

Leu Glu Ala Ala Phe Arg Ser Val Phe Pro Ile Gln Ser Tyr Asn Gly
    50                  55                  60

Asn Ser Glu Leu Gln Tyr Val Ser Tyr Arg Leu Gly Glu Pro Val Phe
65                  70                  75                  80

Asp Val Lys Glu Cys Gln Ile Arg Gly Val Thr Tyr Ser Lys Pro Leu
                85                  90                  95

Arg Val Lys Leu Arg Leu Val Ile Phe Asp Lys Asp Ala Pro Ala Gly
            100                 105                 110

Thr Val Lys Asp Ile Lys Glu Gln Glu Val Tyr Met Gly Glu Ile Pro
        115                 120                 125

Leu Met Thr Glu Asn Gly Thr Phe Val Ile Asn Gly Thr Glu Arg Val
    130                 135                 140

Ile Val Ser Gln Leu His Arg Ser Pro Gly Val Phe Phe Asp Ser Asp
145                 150                 155                 160

Lys Gly Lys Thr His Ser Ser Gly Lys Val Leu Tyr Asn Ala Arg Ile
                165                 170                 175

Ile Pro Tyr Arg Gly Ser Trp Leu Asp Phe Glu Phe Asp Pro Lys Asp
            180                 185                 190

Asn Leu Tyr Val Arg Ile Asp Arg Arg Arg Lys Leu Pro Ala Ser Ile
        195                 200                 205

Ile Leu Arg Ala Leu Gly Lys Thr Ser Ala Glu Ile Leu Asp Ile Phe
    210                 215                 220

Phe Glu Lys Val Asn Phe Glu Val Lys Asp Gln Thr Leu Met Met Glu
225                 230                 235                 240

Leu Val Pro Glu Arg Leu Arg Gly Glu Thr Ala Thr Phe Asp Ile Glu
                245                 250                 255

Ala Asp Gly Lys Val Tyr Val Glu Lys Gly Arg Arg Val Thr Ala Arg
            260                 265                 270

His Ile Arg Gln Leu Glu Lys Asp Gly Val Asn Phe Ile Glu Val Pro

        275                 280                 285

Val Glu Tyr Ile Val Gly Lys Val Ser Ala Lys Asp Tyr Val Asn Glu
    290                 295                 300

Ala Thr Gly Glu Leu Ile Ile Thr Ala Asn Gln Glu Ile Ser Leu Glu
305                 310                 315                 320

Ala Leu Ala Asn Leu Ser Gln Ala Gly Tyr Lys Lys Leu Glu Val Leu
                325                 330                 335

Phe Thr Asn Asp Leu Asp His Gly Pro Phe Met Ser Glu Thr Leu Arg
            340                 345                 350

Val Asp Ser Thr Thr Asp Arg Ile Ser Ala Leu Val Glu Ile Tyr Arg
        355                 360                 365

Met Met Arg Pro Gly Glu Pro Pro Thr Lys Glu Ala Ala Glu Ser Leu
    370                 375                 380

Phe Glu Ser Leu Phe Phe Ser Ala Glu Arg Tyr Asp Leu Ser Thr Val
385                 390                 395                 400

Gly Arg Met Lys Phe Asn Ser Ser Ile Gly Arg Glu Asp Ala Glu Glu
                405                 410                 415

Gln Gly Thr Leu Asp Glu Val Asp Ile Ile Glu Val Met Lys Lys Leu
            420                 425                 430

Ile Ser Ile Arg Asn Gly Lys Gly Glu Val Asp Asp Ile Asp His Leu
        435                 440                 445

Gly Asn Arg Arg Ile Arg Ser Val Gly Glu Met Ala Glu Asn Gln Phe
    450                 455                 460

Arg Val Gly Leu Val Arg Val Glu Arg Ala Val Lys Glu Arg Leu Ser
465                 470                 475                 480

Leu Gly Asp Leu Asp Asn Val Met Pro Gln Asp Leu Ile Asn Ala Lys
                485                 490                 495

Pro Ile Ser Ala Ala Val Lys Glu Phe Phe Gly Ser Ser Gln Leu Ser
            500                 505                 510

Gln Phe Met Asp Gln Asn Asn Pro Leu Ser Glu Val Thr His Lys Arg
        515                 520                 525

Arg Ile Ser Ala Leu Gly Pro Gly Gly Leu Thr Arg Glu Arg Ala Gly
    530                 535                 540

Phe Glu Val Arg Asp Val His Val Thr His Tyr Gly Arg Leu Cys Pro
545                 550                 555                 560

Ile Glu Thr Pro Glu Gly Pro Asn Ile Gly Leu Ile Asn Ser Leu Ser
                565                 570                 575

Ala Phe Ala Arg Cys Asn Glu Tyr Gly Phe Leu Glu Thr Pro Tyr Arg
            580                 585                 590

Arg Val Val Asn Gly Val Val Thr Asp Glu Val Asp Tyr Leu Ser Ala
        595                 600                 605

Ile Glu Glu Gly Gln Phe Val Ile Ala Gln Ala Asn Ala Lys Leu Thr
    610                 615                 620

Glu Glu Gly Gly Phe Ala Asp Glu Leu Val Thr Ala Arg Gln Lys Gly
625                 630                 635                 640

Glu Ser Gly Leu His Pro Arg Glu His Val Asp Tyr Met Asp Val Ala
                645                 650                 655

Thr Asn Gln Val Val Ser Ile Ala Ala Ser Leu Ile Pro Phe Leu Glu
            660                 665                 670

His Asp Asp Ala Asn Arg Ala Leu Met Gly Ala Asn Met Gln Arg Gln
        675                 680                 685

Ala Val Pro Thr Leu Arg Ser Glu Lys Pro Leu Val Gly Thr Gly Ile
    690                 695                 700

Glu Arg Asn Val Ala Val Asp Ser Gly Val Thr Ala Val Ala Lys Arg
705                 710                 715                 720

Gly Gly Val Ile Gln Ser Val Asp Ala Ser Arg Ile Val Val Lys Val
                725                 730                 735

Asn Glu Glu Glu Leu Ile Pro Gly Glu Ala Gly Ile Asp Ile Tyr Asn
            740                 745                 750

Leu Thr Lys Tyr Thr Arg Ser Asn Gln Asn Thr Cys Ile Asn Gln Arg
        755                 760                 765

Pro Cys Val Met Pro Gly Glu Pro Val Ala Arg Gly Asp Val Leu Ala
    770                 775                 780

Asp Gly Pro Ser Thr Asp Leu Gly Glu Leu Ala Leu Gly Gln Asn Met
785                 790                 795                 800

Arg Ile Ala Phe Met Pro Trp Asn Gly Tyr Asn Phe Glu Asp Ser Ile
                805                 810                 815

Leu Val Ser Glu Arg Val Val Gln Asp Asp Arg Phe Thr Thr Ile His
            820                 825                 830

Ile Gln Glu Leu Ser Cys Val Ala Arg Asp Thr Lys Leu Gly Ala Glu

        835                 840                 845

Glu Ile Thr Ala Asp Ile Pro Asn Val Gly Glu Ala Ala Leu Ser Lys
    850                 855                 860

Leu Asp Glu Ser Gly Ile Val Tyr Ile Gly Ala Glu Val Lys Gly Gly
865                 870                 875                 880

Asp Ile Leu Val Gly Lys Val Thr Pro Lys Gly Glu Thr Gln Leu Thr
                885                 890                 895

Pro Glu Glu Lys Leu Leu Arg Ala Ile Phe Gly Glu Lys Ala Ser Asp
            900                 905                 910

Val Lys Asp Thr Ser Leu Arg Val Pro Asn Ser Val Ala Gly Thr Val
        915                 920                 925

Ile Asp Val Gln Val Phe Thr Arg Asp Gly Val Glu Lys Asp Lys Arg
    930                 935                 940

Ala Leu Glu Ile Glu Gln Met Gln Leu Lys Glu Ala Lys Lys Asp Leu
945                 950                 955                 960

Thr Glu Glu Phe Gln Ile Leu Glu Gly Gly Leu Leu Ala Arg Val Arg
                965                 970                 975

Ser Val Leu Leu Ala Gly Gly Tyr Thr Glu Ala Lys Leu Gly Ser Ile
            980                 985                 990

Glu Arg Lys Lys Trp Leu Glu Gln Thr Leu Glu Asn Glu Glu Leu Gln
        995                 1000                1005

Asn Gln Leu Glu Gln Leu Ala Glu Gln Tyr Asp Glu Leu Lys Ala Asp
    1010                1015                1020

Phe Asp Lys Lys Phe Glu Ala Lys Arg Arg Lys Ile Thr Gln Gly Asp
1025                1030                1035                1040

Asp Leu Ala Pro Gly Val Leu Lys Ile Val Lys Val Tyr Leu Ala Val
                1045                1050                1055

Lys Arg Arg Ile Gln Pro Gly Asp Lys Met Ala Gly Arg His Gly Asn
            1060                1065                1070

Lys Gly Val Ile Ser Lys Ile Asn Pro Val Glu Asp Met Pro Tyr Asp
        1075                1080                1085

Glu Asn Gly Gln Pro Val Asp Ile Val Leu Asn Pro Leu Gly Val Pro
    1090                1095                1100

Ser Arg Met Asn Ile Gly Gln Ile Leu Glu Val His Leu Gly Leu Ala
1105                1110                1115                1120

Ala Lys Gly Ile Gly Asp Lys Ile Asn Gln Met Ile Lys Glu Gln Gln
                1125                1130                1135

Glu Leu Ala Lys Leu Arg Glu Phe Leu Gln Lys Val Tyr Asp Leu Gly
            1140                1145                1150

Asp Thr Arg Gln Arg Val Asp Ile Ser Glu Leu Ser Asp Glu Asp Val
        1155                1160                1165

Arg Thr Leu Ala His Asn Leu Arg Ala Gly Leu Pro Val Ala Thr Pro
    1170                1175                1180

Val Phe Asp Gly Ala Pro Glu Ser Ser Ile Lys Ala Met Leu Glu Leu
1185                1190                1195                1200

Ala Asp Leu Pro Ala Ser Gly Gln Leu Thr Leu Phe Asp Gly Arg Thr
                1205                1210                1215

Gly Asp Ala Phe Glu Arg Pro Val Thr Val Gly Tyr Met Tyr Met Leu
            1220                1225                1230

Lys Leu Asn His Leu Val Asp Asp Lys Met His Ala Arg Ser Thr Gly
        1235                1240                1245

Ser Tyr Ser Leu Val Thr Gln Gln Pro Leu Gly Gly Lys Ala Gln Phe
1250                1255                1260

Gly Gly Gln Arg Phe Gly Glu Met Glu Val Trp Ala Leu Glu Ala Tyr
1265                1270                1275                1280

Gly Ala Ala Tyr Thr Leu Gln Glu Met Leu Thr Val Lys Ser Asp Asp
                1285                1290                1295

Val Asn Gly Arg Thr Lys Met Tyr Lys Asn Ile Val Asp Gly Asn His
            1300                1305                1310

Ala Met Glu Pro Gly Met Pro Glu Ser Phe Asn Val Leu Leu Lys Glu
        1315                1320                1325

Ile Arg Ser Leu Gly Ile Asn Ile Glu Leu Glu Asp Glu
    1330                1335                1340


<210> 28
<211> 358
<212> PRT
<213> Vibrio cholerae

<220>
<221> PEPTIDE
<222> (1)..(358)
<223> >tr|Q9KUN7|Q9KUN7_VIBCH Fructose-bisphosphate
      aldolase, class II - Vibrio cholerae. (358 aa)

<400> 28
Met Ser Lys Ile Phe Asp Phe Val Lys Pro Gly Val Ile Ser Gly Asp
1               5                   10                  15

Asp Val Gln Lys Val Phe Glu Val Ala Lys Gln Asn Gly Phe Ala Leu
            20                  25                  30

Pro Ala Val Asn Cys Val Gly Thr Asp Ser Val Asn Gly Val Leu Glu
        35                  40                  45

Ala Ala Ala Lys Val Lys Ala Pro Val Ile Val Gln Phe Ser Asn Gly
    50                  55                  60

Gly Ala Ala Phe Phe Ala Gly Lys Gly Leu Lys Leu Glu Gly Gln Gln
65                  70                  75                  80

Ala Gln Ile Leu Gly Ala Ile Ala Gly Ala Lys Tyr Val His Thr Val
                85                  90                  95

Ala Glu Ala Tyr Gly Val Pro Val Ile Leu His Thr Asp His Ala Ala
            100                 105                 110

Lys Asn Leu Leu Pro Trp Ile Asp Gly Leu Leu Asp Ala Gly Glu Lys
        115                 120                 125

His Phe Ala Glu Thr Gly Lys Pro Leu Phe Ser Ser His Met Ile Asp
    130                 135                 140

Leu Ser Glu Glu Ser Leu Glu Glu Asn Ile Glu Ile Ser Ala Lys Tyr
145                 150                 155                 160

Leu Glu Arg Met Ala Lys Met Asn Met Thr Leu Glu Ile Glu Leu Gly
                165                 170                 175

Cys Thr Gly Gly Glu Glu Asp Gly Val Asp Asn Ser Asp Met Asp Ser
            180                 185                 190

Ser Glu Leu Tyr Thr Ser Pro Glu Asp Val Ala Tyr Ala Tyr Glu Lys
        195                 200                 205

Leu Asn Ala Ile Ser His Arg Phe Thr Ile Ala Ala Ser Phe Gly Asn
    210                 215                 220

Val His Gly Val Tyr Lys Pro Gly Asn Val Val Leu Thr Pro Thr Ile
225                 230                 235                 240

Leu Arg Asp Ser Gln Lys Tyr Val Ser Glu Lys Phe Gly Leu Pro Glu
                245                 250                 255

Asn Ser Leu Asn Phe Val Phe His Gly Gly Ser Gly Ser Ser Leu Glu
            260                 265                 270

Glu Ile Arg Glu Ser Ile Ser Tyr Gly Val Ile Lys Met Asn Ile Asp
        275                 280                 285

Thr Asp Thr Gln Trp Ala Thr Trp Asp Gly Ile Arg Gln Tyr Glu Ala
    290                 295                 300

Lys Asn His Asp Tyr Leu Gln Gly Gln Ile Gly Asn Pro Thr Gly Glu
305                 310                 315                 320

Asp Ala Pro Asn Lys Lys Tyr Tyr Asp Pro Arg Val Trp Leu Arg Ala
                325                 330                 335

Gly Gln Ala Ala Met Val Ala Arg Leu Glu Lys Ala Phe Ser Asp Leu
            340                 345                 350

Asn Ala Ile Asp Val Leu
        355


<210> 29
<211> 433
<212> PRT
<213> Vibrio cholerae

<220>
<221> PEPTIDE
<222> (1)..(433)
<223> >sp|Q9KPC5|ENO_VIBCH Enolase (EC 4.2.1.11)
      (2-phosphoglycerate dehydratase) (2-phospho-D-
      glycerate hydro-lyase) - Vibrio cholerae.

<400> 29
Met Ser Lys Ile Val Lys Val Leu Gly Arg Glu Ile Ile Asp Ser Arg
  1               5                  10                  15

Gly Asn Pro Thr Val Glu Ala Glu Val His Leu Glu Gly Gly Phe Val

             20                  25                  30

Gly Met Ala Ala Ala Pro Ser Gly Ala Ser Thr Gly Ser Arg Glu Ala
        35                  40                  45

Leu Glu Leu Arg Asp Gly Asp Lys Ser Arg Phe Leu Gly Lys Gly Val
    50                  55                  60

Leu Lys Ala Leu Ala Ala Val Asn Gly Pro Ile Ala Asp Ala Leu Val
65                  70                  75                  80

Gly Lys Asp Ala Lys Asp Gln Ala Thr Ile Asp Gln Ile Met Ile Asp
                85                  90                  95

Leu Asp Gly Thr Glu Asn Lys Ser Asn Phe Gly Ala Asn Ala Ile Leu
            100                 105                 110

Ala Val Ser Leu Ala Asn Ala Lys Ala Ala Ala Ala Ala Lys Gly Met
        115                 120                 125

Pro Leu Tyr Glu His Ile Ala Glu Leu Asn Gly Thr Pro Gly Val Phe
    130                 135                 140

Ser Met Pro Leu Pro Met Met Asn Ile Ile Asn Gly Gly Glu His Ala
145                 150                 155                 160

Asp Asn Asn Val Asp Ile Gln Glu Phe Met Ile Gln Pro Val Gly Ala
                165                 170                 175

Lys Thr Leu Lys Glu Ala Val Arg Met Gly Ala Glu Val Phe His Asn
            180                 185                 190

Leu Ala Lys Val Leu Lys Ser Lys Gly Tyr Asn Thr Ala Val Gly Asp
        195                 200                 205

Glu Gly Gly Phe Ala Pro Asn Leu Lys Ser Asn Ala Glu Ala Leu Glu
    210                 215                 220

Val Ile Ala Glu Ala Val Ala Ala Ala Gly Tyr Lys Leu Gly Thr Asp
225                 230                 235                 240

Ile Thr Leu Ala Met Asp Cys Ala Ala Ser Glu Phe Tyr Asp Ala Glu
                245                 250                 255

Lys Lys Glu Tyr Asn Leu Lys Gly Glu Gly Arg Ile Phe Thr Ser Asn
            260                 265                 270

Gly Phe Ser Asp Phe Leu Glu Glu Leu Thr Glu Lys Phe Pro Ile Val
        275                 280                 285

Ser Ile Glu Asp Gly Leu Asp Glu Ser Asp Trp Glu Gly Phe Ala Tyr
    290                 295                 300

Gln Thr Glu Lys Leu Gly Lys Lys Ile Gln Ile Val Gly Asp Asp Leu
305                 310                 315                 320

Phe Val Thr Asn Thr Lys Ile Leu Lys Arg Gly Ile Asp Asn Gly Ile
                325                 330                 335

Ala Asn Ser Ile Leu Ile Lys Phe Asn Gln Ile Gly Ser Leu Thr Glu
            340                 345                 350

Thr Leu Ala Ala Ile Lys Met Ala Lys Asp Ala Gly Tyr Thr Ala Val

355                 360                 365

Ile Ser His Arg Ser Gly Glu Thr Glu Asp Ala Thr Ile Ala Asp Leu
    370                 375                 380

Ala Val Gly Thr Ala Ala Gly Gln Ile Lys Thr Gly Ser Met Ser Arg
385                 390                 395                 400

Ser Asp Arg Val Ala Lys Tyr Asn Gln Leu Ile Arg Ile Glu Glu Ala
                405                 410                 415

Leu Gly Ser Arg Ala Pro Phe Asn Gly Leu Lys Glu Val Lys Gly Gln
            420                 425                 430

Ala

<210> 30
<211> 206
<212> PRT
<213> Vibrio cholerae

<220>
<221> PEPTIDE
<222> (1)..(206)
<223> >sp|Q9KP07|RS4_VIBCH 30S ribosomal protein S4 -
      Vibrio cholerae. (206 aa)

<400> 30
Met Ala Arg Tyr Leu Gly Pro Lys Leu Lys Leu Ser Arg Arg Glu Gly
1               5                   10                  15

Thr Asp Leu Phe Leu Lys Ser Gly Val Arg Ala Ile Asp Thr Lys Cys
            20                  25                  30

Lys Ile Asp Asn Ala Pro Gly Val His Gly Ala Arg Arg Gly Arg Leu
        35                  40                  45

Ser Glu Tyr Gly Val Gln Leu Arg Glu Lys Gln Lys Val Arg Arg Ile
    50                  55                  60

Tyr Gly Val Leu Glu Lys Gln Phe Arg Asn Tyr Tyr Lys Glu Ala Ala
65                  70                  75                  80

Arg Leu Lys Gly Asn Thr Gly Glu Asn Leu Leu Gln Leu Leu Glu Gly
                85                  90                  95

Arg Leu Asp Asn Val Val Tyr Arg Met Gly Phe Gly Ala Thr Arg Ala
            100                 105                 110

Glu Ala Arg Gln Leu Val Ser His Lys Ala Ile Leu Val Asn Gly Lys
        115                 120                 125

Val Val Asn Val Pro Ser Phe Asn Val Ala Ala Asn Asp Val Val Ala
    130                 135                 140

Val Arg Glu Lys Ala Lys Lys Gln Ser Arg Ile Lys Ala Ala Leu Glu
145                 150                 155                 160

Val Ala Glu Gln Arg Glu Lys Pro Thr Trp Ile Glu Val Asp Val Asn

165                 170                 175

Thr Met Glu Gly Thr Phe Lys Arg Met Pro Glu Arg Ser Asp Leu Ser
            180                 185                 190

Ala Asp Ile Asn Glu Gln Leu Ile Val Glu Leu Tyr Ser Lys
        195                 200                 205


<210> 31
<211> 92
<212> PRT
<213> Vibrio cholerae

<220>
<221> PEPTIDE
<222> (1)..(92)
<223> >sp|Q9KRK0|RL25_VIBCH 50S ribosomal protein L25 -
      Vibrio cholerae.

<400> 31
Met Lys Phe Glu Ala Val Leu Arg Thr Asp Leu Gly Lys Gly Ala Ser
1               5                   10                  15

Arg Arg Leu Arg Asn Thr Gly Tyr Phe Pro Ala Ile Val Tyr Gly Gly
            20                  25                  30

Glu Ala Ala Pro Val Ser Ile Ser Leu Asn His Asp Asp Val Met Asn
        35                  40                  45

Gln Met Asp Lys Pro Glu Phe Tyr Glu Ala Ile Val Leu Val Ile Asp
    50                  55                  60

Gly Gln Glu Val Lys Val Lys Pro Gln Asp Val Gln Arg His Ala Tyr
65                  70                  75                  80

Lys Pro Lys Val Glu His Met Asp Phe Ile Arg Ile
                85                  90


<210> 32
<211> 55
<212> PRT
<213> Vibrio cholerae

<220>
<221> PEPTIDE
<222> (1)..(55)
<223> >sp|Q9KQH3|RL32_VIBCH 50S ribosomal protein L32 -
      Vibrio cholerae. (55 aa)

<400> 32
Ala Val Gln Gln Asn Arg Lys Thr Arg Ser Arg Arg Gly Met Arg Arg
1               5                   10                  15

Ser His Asp Ala Leu Thr Ala Ala Ala Leu Ser Val Asp Ala Thr Ser
            20                  25                  30

Gly Glu Thr His Leu Arg His Asn Val Thr Ala Glu Gly Tyr Tyr Arg
        35                  40                  45

Gly Lys Lys Val Ile Asn Lys
    50                  55

<210> 33
<211> 71
<212> PRT
<213> Vibrio cholerae

<220>
<221> PEPTIDE
<222> (1)..(71)
<223> >sp|P66532|RS21_VIBCH 30S ribosomal protein S21 -
      Vibrio cholerae. (71 aa)

<400> 33
Met Pro Val Val Lys Val Arg Glu Asn Glu Pro Phe Asp Val Ala Leu
  1               5                  10                  15

Arg Arg Phe Lys Arg Ser Cys Glu Lys Ala Gly Ile Leu Ser Glu Val
             20                  25                  30

Arg Arg Arg Glu His Tyr Glu Lys Pro Thr Thr Val Arg Lys Arg Ala
        35                  40                  45

Lys Ala Ala Ala Gln Lys Arg His Ala Lys Lys Leu Ala Arg Glu Asn
    50                  55                  60

Ala Arg Arg Val Arg Leu Tyr
 65                  70

<210> 34
<211> 373
<212> PRT
<213> Vibrio cholerae

<220>
<221> PEPTIDE
<222> (1)..(373)
<223> >sp|Q06952|GMD1_VIBCH Probable GDP-mannose
      4,6-dehydratase (EC 4.2.1.47) (GDP-D-mannose
      dehydratase) - Vibrio cholerae. (373 aa)

<400> 34
Met Asn Lys Lys Val Ala Leu Ile Thr Gly Ile Thr Gly Gln Asp Gly
  1               5                  10                  15

Ser Tyr Leu Ala Glu Phe Leu Leu Glu Lys Gly Tyr Glu Val His Gly
             20                  25                  30

Ile Lys Arg Arg Ser Ser Leu Phe Asn Thr Gln Arg Val Asp His Leu
        35                  40                  45

Tyr Lys Asp Pro His Glu Glu Asp Val Asn Phe Lys Leu His Tyr Gly
    50                  55                  60

Asp Leu Thr Asp Ser Ser Asn Leu Thr Arg Ile Leu Ala Glu Val Gln
 65                  70                  75                  80

Pro Asp Glu Val Tyr Asn Leu Gly Ala Gln Ser His Val Ala Val Ser
                85                  90                  95

Phe Gln Ser Pro Glu Tyr Thr Ala Asp Val Asp Ala Ile Gly Thr Leu
            100                 105                 110

Arg Leu Leu Glu Ala Ile Arg Phe Leu Gly Leu Thr Lys Lys Thr Lys
        115                 120                 125

Phe Tyr Gln Ala Ser Thr Ser Glu Leu Tyr Gly Leu Val Gln Glu Ile
    130                 135                 140

Pro Gln Lys Glu Thr Thr Pro Phe Tyr Pro Arg Ser Pro Tyr Ala Val
145                 150                 155                 160

Ala Lys Met Tyr Ala Tyr Trp Ile Thr Ile Asn Tyr Arg Glu Ser Tyr
                165                 170                 175

Gly Ile Tyr Ala Cys Asn Gly Ile Leu Phe Asn His Glu Ser Pro Arg
            180                 185                 190

Arg Gly Glu Thr Phe Val Thr Arg Lys Ile Thr Arg Gly Met Ala Asn
        195                 200                 205

Ile Ala Gln Gly Leu Glu Lys Cys Leu Phe Met Gly Asn Leu Asp Ala
    210                 215                 220

Leu Arg Asp Trp Gly His Ala Lys Asp Tyr Val Lys Met Gln Trp Met
225                 230                 235                 240

Met Leu Gln Gln Asp Glu Pro Arg Asp Phe Val Ile Ala Thr Gly Val
                245                 250                 255

Gln Tyr Ser Val Arg Glu Phe Ile Asp Met Ser Ala Arg Glu Leu Gly
            260                 265                 270

Ile Glu Leu Glu Phe Val Gly Lys Gly Val Asp Glu Lys Ala Val Val
        275                 280                 285

Lys Ser Val Ile Gly Thr Lys Ala Pro Ala Ile Lys Val Gly Asp Ile
    290                 295                 300

Ile Val Ala Val Asp Pro Ala Tyr Phe Arg Pro Ala Glu Val Glu Thr
305                 310                 315                 320

Leu Leu Gly Asp Pro Ser Leu Ala Lys Lys Glu Leu Gly Trp Val Pro
                325                 330                 335

Glu Ile Thr Leu Gln Gln Met Val Ser Glu Met Val Ala Ser Asp Leu
            340                 345                 350

Glu Gln Ala Gln Ser His Ala Leu Leu Lys Lys His Gly Tyr Asn Val
        355                 360                 365

Asn Val Ser Val Glu
    370

<210> 35
<211> 510
<212> PRT

<213> Vibrio cholerae

<220>
<221> PEPTIDE
<222> (1)..(510)
<223> >sp|Q9KU60|SYK_VIBCH Lysyl-tRNA synthetase (EC
      6.1.1.6) (Lysine--tRNA ligase) (LysRS) - Vibrio
      cholerae. (510 aa)

<400> 35
Met Thr Asp Ala Val Gln Asn Glu Ile Asn Gln Glu Gln Ile Ala Gln
1               5                   10                  15

Glu Glu Asn Lys Leu Ile Ala Glu Arg Arg Ser Lys Leu Asp His Ile
            20                  25                  30

Arg Lys Asn Cys Lys Ala Asn Gly His Pro Asn Ser Phe Arg Arg Asp
        35                  40                  45

Ser Leu Ala Gly Asp Leu Gln Lys Lys Phe Gly Glu Lys Ser Lys Glu
    50                  55                  60

Glu Leu Glu Ala Leu Asn His Val Val Ser Ile Ala Gly Arg Val Met
65                  70                  75                  80

Ala Lys Arg Gly Pro Phe Leu Val Ile Gln Glu Thr Ser Gly Arg Ile
                85                  90                  95

Gln Ala Tyr Ala Ser Lys Glu Val Gln Gln Glu Leu Lys Asp Lys Tyr
            100                 105                 110

Gln Gly Leu Asp Ile Gly Asp Ile Ile Gly Val Gln Gly Ala Leu His
        115                 120                 125

Lys Ser Gly Lys Gly Asp Leu Tyr Val Asn Met Glu Gln Phe Gln Leu
    130                 135                 140

Leu Thr Lys Ala Leu Arg Pro Leu Pro Glu Lys Phe His Gly Leu Thr
145                 150                 155                 160

Asp Gln Glu Met Arg Tyr Arg Gln Arg Tyr Val Asp Leu Ile Val Asn
                165                 170                 175

Glu Asn Ser Arg Asn Ala Phe Ile Val Arg Ser Lys Val Met Ser Ala
            180                 185                 190

Ile Arg Asn Phe Met Ile Ser Lys Gln Phe Met Glu Val Glu Thr Pro
        195                 200                 205

Met Met His Val Ile Pro Gly Gly Ala Ser Ala Arg Pro Phe Ile Thr
    210                 215                 220

His His Asn Ala Leu Asp Met Pro Met Tyr Leu Arg Ile Ala Pro Glu
225                 230                 235                 240

Leu Tyr Leu Lys Arg Leu Val Val Gly Gly Phe Asp Arg Val Phe Glu
                245                 250                 255

Ile Asn Arg Asn Phe Arg Asn Glu Gly Leu Ser Pro Arg His Asn Pro
            260                 265                 270

Glu Phe Thr Met Met Glu Phe Tyr Met Ala Tyr Ala Asp Tyr Lys Asp
        275                 280                 285

Leu Met Asp Leu Thr Glu Glu Leu Leu Ser Ser Val Ala Leu Glu Val
    290                 295                 300

Leu Gly Ser Thr Ser Met Pro Tyr Gly Glu His Thr Val Glu Phe Gly
305                 310                 315                 320

Gly Lys Tyr Ala Arg Met Ser Met Phe Asp Ala Ile Lys His Tyr Asn
                325                 330                 335

Pro Asn His Ala Gln Ile Gln Ala Leu Thr Glu Glu Asp Ile Gln Asn
            340                 345                 350

Arg Asp Leu Met Val Ser Ile Ala Lys Ser Val His Val Asp Val Glu
        355                 360                 365

Pro Phe Trp Thr Cys Gly Gln Leu Leu Glu Glu Ile Phe Gly Glu Thr
    370                 375                 380

Ala Glu Pro Lys Leu Met Gln Pro Thr Phe Ile Thr Gly Tyr Pro Ala
385                 390                 395                 400

Asp Ile Ser Pro Leu Ala Arg Arg Ser Asp Asp Asn Pro Phe Phe Thr
                405                 410                 415

Asp Arg Phe Glu Phe Phe Ile Gly Gly Arg Glu Val Ala Asn Gly Phe
            420                 425                 430

Ser Glu Leu Asn Asp Ala Glu Asp Gln Asp Ala Arg Phe Lys Ala Gln
        435                 440                 445

Val Glu Ala Lys Glu Ser Gly Asp Asp Glu Ala Met Phe Tyr Asp Ala
    450                 455                 460

Asp Tyr Ile Thr Ala Leu Glu His Gly Leu Pro Pro Thr Ala Gly Gln
465                 470                 475                 480

Gly Ile Gly Ile Asp Arg Leu Val Met Leu Leu Thr Asn Thr His Thr
                485                 490                 495

Ile Arg Asp Val Ile Leu Phe Pro Ala Met Arg Pro Gln Gln
            500                 505                 510


<210> 36
<211> 207
<212> PRT
<213> Vibrio cholerae

<220>
<221> PEPTIDE
<222> (1)..(207)
<223> >tr|Q9KTZ9|Q9KTZ9_VIBCH Antioxidant, AhpC/Tsa
      family - Vibrio cholerae (207 aa)

<400> 36
Met Leu Arg Ser Lys Lys Met Val Leu Val Gly Arg Lys Ala Pro Asp
1               5                   10                  15

Phe Thr Ala Ala Ala Val Leu Gly Asn Gly Glu Ile Val Asp Asn Phe
            20                  25                  30

Asn Phe Ala Glu Phe Thr Lys Gly Lys Lys Ala Val Val Phe Phe Tyr
        35                  40                  45

Pro Leu Asp Phe Thr Phe Val Cys Pro Ser Glu Leu Ile Ala Phe Asp
    50                  55                  60

Asn Arg Tyr Glu Asp Phe Leu Ala Lys Gly Val Glu Val Ile Gly Val
65                  70                  75                  80

Ser Ile Asp Ser Gln Phe Ser His Asn Ala Trp Arg Asn Thr Ala Val
                85                  90                  95

Glu Asn Gly Gly Ile Gly Gln Val Lys Tyr Pro Leu Val Ala Asp Val
            100                 105                 110

Lys His Glu Ile Cys Lys Ala Tyr Asp Val Glu His Pro Glu Ala Gly
        115                 120                 125

Val Ala Phe Arg Gly Ser Phe Leu Ile Asp Glu Glu Gly Met Val Arg
    130                 135                 140

His Gln Val Val Asn Asp Leu Pro Leu Gly Arg Asn Ile Asp Glu Met
145                 150                 155                 160

Leu Arg Met Val Asp Ala Leu Asn Phe His Gln Thr His Gly Glu Val
                165                 170                 175

Cys Pro Ala Gln Trp Glu Ala Gly Lys Ala Gly Met Glu Ala Ser Pro
            180                 185                 190

Lys Gly Val Ala Ala Phe Leu Ser Gln Tyr Ser Ala Asp Leu Lys
        195                 200                 205


<210> 37
<211> 129
<212> PRT
<213> Vibrio cholerae

<220>
<221> PEPTIDE
<222> (1)..(129)
<223> >sp|P66367|RS11_VIBCH 30S ribosomal protein S11 -
      Vibrio cholerae. (129 aa)

<400> 37
Met Ala Lys Gln Pro Thr Arg Ala Arg Lys Arg Val Arg Lys Gln Val
1               5                   10                  15

Ala Asp Gly Val Ala His Ile His Ala Ser Phe Asn Asn Thr Ile Val
            20                  25                  30

Thr Ile Thr Asp Arg Gln Gly Asn Ala Leu Ala Trp Ala Thr Ala Gly
        35                  40                  45

Gly Ser Gly Phe Arg Gly Ser Arg Lys Ser Thr Pro Phe Ala Ala Gln
    50                  55                  60

Val Ala Ala Glu Arg Cys Ala Glu Met Ala Lys Glu Tyr Gly Leu Lys
65                  70                  75                  80

Asn Leu Glu Val Met Val Lys Gly Pro Gly Pro Gly Arg Glu Ser Thr
                85                  90                  95

Val Arg Ala Leu Asn Ala Ala Gly Phe Arg Ile Thr Asn Ile Val Asp
            100                 105                 110

Ala Thr Pro Ile Pro His Asn Gly Cys Arg Pro Pro Lys Lys Arg Arg
        115                 120                 125

Val

<210> 38
<211> 394
<212> PRT
<213> Vibrio cholerae

<220>
<221> PEPTIDE
<222> (1)..(394)
<223> >sp|Q9KV37|EFTU1_VIBCH Elongation factor Tu-A
      (EF-Tu-A) - Vibrio cholerae. (394 aa)

<400> 38
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
        35                  40                  45

Ser Ile Asp Asn Ala Pro Glu Glu Arg Glu Arg Gly Ile Thr Ile Asn
    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Arg
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp
        195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala
        355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390


<210> 39
<211> 319
<212> PRT
<213> Vibrio cholerae


<220>
<221> PEPTIDE
<222> (1)..(319)
<223> >tr|Q9KPW8|Q9KPW8_VIBCH Acetyl-CoA carboxylase,
      carboxyl transferase alpha subunit - Vibrio
      cholerae. (319 aa)

<400> 39
Met Ser Leu Asn Phe Leu Asp Phe Glu Lys Pro Ile Val Glu Leu Glu
1               5                   10                  15

Thr Lys Ile Gln Ala Leu Arg Asp Val Ser Arg His Ser Thr Ser Ala

20                  25                  30

Ser Val Asp Leu Asp Lys Glu Leu Glu Gln Leu Glu Lys Lys Ser Leu
        35                  40                  45

Glu Leu Lys Lys Lys Ile Phe Ser Asp Leu Gly Ala Trp Gln Val Ala
    50                  55                  60

Gln Leu Ala Arg His Pro Gln Arg Pro Tyr Thr Leu Asp Tyr Leu Lys
65                  70                  75                  80

His Ile Phe Thr Glu Phe Asp Glu Leu Ala Gly Asp Arg Ala Tyr Ala
                85                  90                  95

Asp Asp Lys Ala Ile Val Gly Gly Ile Ala Arg Leu Glu Gly Arg Ser
            100                 105                 110

Val Met Val Ile Gly His Gln Lys Gly Arg Glu Thr Arg Glu Lys Val
        115                 120                 125

Lys Arg Asn Phe Gly Met Pro Lys Pro Glu Gly Tyr Arg Lys Ala Leu
    130                 135                 140

Arg Leu Met Glu Met Ala Glu Arg Phe Asn Met Pro Ile Ile Thr Phe
145                 150                 155                 160

Ile Asp Thr Ala Gly Ala Tyr Pro Gly Val Gly Ala Glu Glu Arg Gly
                165                 170                 175

Gln Ser Glu Ala Ile Ala Lys Asn Leu Lys Val Met Ser Gly Leu Lys
            180                 185                 190

Val Pro Val Ile Cys Asn Val Val Gly Glu Gly Gly Ser Gly Gly Ala
        195                 200                 205

Leu Ala Ile Gly Val Gly Asp Tyr Val Asn Met Leu Gln Tyr Ser Thr
    210                 215                 220

Tyr Ser Val Ile Ser Pro Glu Gly Cys Ala Ser Ile Leu Trp Arg Asp
225                 230                 235                 240

Ser Asp Lys Ala Pro Gln Ala Ala Glu Ala Met Gly Leu Ile Ala Pro
                245                 250                 255

Arg Leu Lys Glu Leu Glu Leu Ile Asp Glu Ile Ile Glu Glu Pro Leu
            260                 265                 270

Gly Gly Ala His Arg Asp His Lys Gln Thr Ala Glu Asn Val Lys Ala
        275                 280                 285

Thr Leu Leu Arg Gln Leu Ala Asp Leu Asp Ala Leu Asp His Glu Asn
    290                 295                 300

Leu Leu Glu Arg Arg Tyr Gln Arg Leu Met Asn Tyr Gly Tyr Cys
305                 310                 315

<210> 40
<211> 330
<212> PRT
<213> Vibrio cholerae

<220>
<221> PEPTIDE
<222> (1)..(330)
<223> >sp|Q9KP08|RPOA_VIBCH DNA-directed RNA polymerase
      alpha chain (EC 2.7.7.6) (RNAP alpha subunit)
      (Transcriptase alpha chain) (RNA polymerase alpha
      subunit) - Vibrio cholerae. (330 aa)

<400> 40
Met Gln Gly Ser Val Thr Glu Phe Leu Lys Pro Arg Leu Val Asp Ile
1               5                   10                  15

Glu Gln Ile Ser Thr Thr His Ala Lys Val Thr Leu Glu Pro Leu Glu
            20                  25                  30

Arg Gly Phe Gly His Thr Leu Gly Asn Ala Leu Arg Arg Ile Leu Leu
        35                  40                  45

Ser Ser Met Pro Gly Cys Ala Val Thr Glu Val Glu Ile Glu Gly Val
    50                  55                  60

Leu His Glu Tyr Ser Thr Lys Glu Gly Val Gln Glu Asp Ile Leu Glu
65                  70                  75                  80

Ile Leu Leu Asn Leu Lys Gly Leu Ala Val Arg Val Ala Glu Gly Lys
                85                  90                  95

Asp Glu Val Phe Ile Thr Leu Asn Lys Ser Gly Ser Gly Pro Val Val
            100                 105                 110

Ala Gly Asp Ile Thr His Asp Gly Asp Val Glu Ile Val Asn Pro Glu
        115                 120                 125

His Val Ile Cys His Leu Thr Ser Asp Asn Ala Ala Ile Ala Met Arg
    130                 135                 140

Ile Lys Val Glu Arg Gly Arg Gly Tyr Val Pro Ala Ser Ala Arg Ile
145                 150                 155                 160

His Thr Glu Glu Asp Glu Arg Pro Ile Gly Arg Leu Leu Val Asp Ala
                165                 170                 175

Thr Phe Ser Pro Val Asp Lys Ile Ala Tyr Ser Val Glu Ala Ala Arg
            180                 185                 190

Val Glu Gln Arg Thr Asp Leu Asp Lys Leu Val Ile Asp Met Glu Thr
        195                 200                 205

Asn Gly Thr Leu Glu Pro Glu Glu Ala Ile Arg Arg Ala Ala Thr Ile
    210                 215                 220

Leu Ala Glu Gln Leu Asp Ala Phe Val Asp Leu Arg Asp Val Arg Val
225                 230                 235                 240

Pro Glu Glu Lys Glu Glu Lys Pro Glu Phe Asp Pro Ile Leu Leu Arg
                245                 250                 255

Pro Val Asp Asp Leu Glu Leu Thr Val Arg Ser Ala Asn Cys Leu Lys
            260                 265                 270

Ala Glu Ala Ile His Tyr Ile Gly Asp Leu Val Gln Arg Thr Glu Val
        275                 280                 285

Glu Leu Leu Lys Thr Pro Asn Leu Gly Lys Lys Ser Leu Thr Glu Ile
    290                 295                 300

Lys Asp Val Leu Ala Ser Arg Gly Leu Ser Leu Gly Met Arg Leu Glu
305                 310                 315                 320

Asn Trp Pro Pro Ala Ser Ile Ala Glu Asp
                325                 330


<210> 41
<211> 513
<212> PRT
<213> Vibrio cholerae

<220>
<221> PEPTIDE
<222> (1)..(513)
<223> >sp|Q9KNH3|ATPA_VIBCH ATP synthase alpha chain (EC
      3.6.3.14) - Vibrio cholerae. (513 aa)

<400> 41
Met Gln Leu Asn Ser Thr Glu Ile Ser Asp Leu Ile Lys Gln Arg Ile
  1               5                  10                  15

Glu Ser Phe Asn Val Val Ser Glu Ala Arg Asn Glu Gly Thr Ile Val
             20                  25                  30

Ser Val Ser Asp Gly Ile Ile Arg Ile His Gly Leu Ala Asp Val Met
         35                  40                  45

Gln Gly Glu Met Ile Glu Leu Pro Gly Asn Arg Tyr Ala Leu Ala Leu
     50                  55                  60

Asn Leu Glu Arg Asp Ser Val Gly Ala Val Val Met Gly Pro Tyr Ala
 65                  70                  75                  80

Asp Leu Arg Glu Gly Met Lys Val Thr Gly Thr Gly Arg Ile Leu Glu
                 85                  90                  95

Val Pro Val Gly Pro Glu Leu Leu Gly Arg Val Val Asn Thr Leu Gly
            100                 105                 110

Glu Pro Ile Asp Gly Lys Gly Pro Ile Gly Ala Lys Gln Thr Ser Pro
        115                 120                 125

Val Glu Val Ile Ala Pro Gly Val Ile Asp Arg Lys Ser Val Asp Gln
    130                 135                 140

Pro Val Gln Thr Gly Tyr Lys Ser Val Asp Ser Met Ile Pro Ile Gly
145                 150                 155                 160

Arg Gly Gln Arg Glu Leu Ile Ile Gly Asp Arg Gln Thr Gly Lys Thr
                165                 170                 175

Ala Leu Ala Ile Asp Ala Ile Ile Asn Gln Lys Asn Ser Gly Ile Tyr
            180                 185                 190

Ser Ile Tyr Val Ala Ile Gly Gln Lys Ala Ser Thr Ile Ala Asn Val
        195                 200                 205

Val Arg Lys Leu Glu Glu His Gly Ala Leu Lys Asn Thr Ile Val Val
    210                 215                 220

Val Ala Ser Ala Ser Glu Ser Ala Ala Leu Gln Tyr Leu Ala Pro Tyr
225                 230                 235                 240

Ser Gly Cys Ala Met Gly Glu Tyr Phe Arg Asp Arg Gly Glu Asp Ala
                245                 250                 255

Leu Ile Val Tyr Asp Asp Leu Ser Lys Gln Ala Val Ala Tyr Arg Gln
            260                 265                 270

Ile Ser Leu Leu Leu Arg Arg Pro Pro Gly Arg Glu Ala Phe Pro Gly
        275                 280                 285

Asp Val Phe Tyr Leu His Ser Arg Leu Leu Glu Arg Ala Ala Arg Val
    290                 295                 300

Asn Glu Glu Tyr Val Glu Arg Phe Thr Lys Gly Glu Val Lys Gly Lys
305                 310                 315                 320

Thr Gly Ser Leu Thr Ala Leu Pro Ile Ile Glu Thr Gln Ala Gly Asp
                325                 330                 335

Val Ser Ala Phe Val Pro Thr Asn Val Ile Ser Ile Thr Asp Gly Gln
            340                 345                 350

Ile Phe Leu Gln Thr Glu Leu Phe Asn Ala Gly Val Arg Pro Ala Val
        355                 360                 365

Asp Pro Gly Ile Ser Val Ser Arg Val Gly Gly Ala Ala Gln Thr Lys
    370                 375                 380

Ile Val Lys Lys Leu Ser Gly Gly Ile Arg Thr Ala Leu Ala Ala Tyr

385                 390                 395                 400

Arg Glu Leu Ala Ala Phe Ala Gln Phe Ser Ser Asp Leu Asp Glu Ala
                405                 410                 415

Thr Lys Arg Gln Leu Thr His Gly Gln Lys Val Thr Glu Leu Met Lys
            420                 425                 430

Gln Lys Gln Tyr Ala Pro Met Ser Val Phe Asp Gln Ala Leu Val Ile
        435                 440                 445

Phe Ala Ala Glu Arg Gly Tyr Leu Thr Asp Val Glu Leu Asn Lys Val
    450                 455                 460

Leu Asp Phe Glu Ala Ala Leu Leu Ser Tyr Ala Arg Ala His Tyr Ala
465                 470                 475                 480

Glu Leu Ala Ala Gln Ile Asp Lys Thr Gly Ala Tyr Asn Asp Glu Ile
                485                 490                 495

Glu Ala Gln Leu Lys Lys Leu Val Asp Asp Phe Lys Ala Thr Gln Thr
            500                 505                 510

Trp

<210> 42
<211> 1401
<212> PRT
<213> Vibrio cholerae

<220>
<221> PEPTIDE
<222> (1)..(1401)
<223> >sp|Q9KV29|RPOC_VIBCH DNA-directed RNA polymerase
      beta' chain (EC 2.7.7.6) (RNAP beta' subunit)
      (Transcriptase beta' chain) (RNA polymerase beta'
      subunit) - Vibrio cholerae. (1401 aa)

<400> 42
Met Lys Asp Leu Leu Asn Phe Leu Lys Ala Gln His Lys Thr Glu Glu
1               5                   10                  15

Phe Asp Ala Ile Lys Ile Gly Leu Ala Ser Pro Asp Met Ile Arg Ser
            20                  25                  30

Trp Ser Phe Gly Glu Val Lys Lys Pro Glu Thr Ile Asn Tyr Arg Thr
        35                  40                  45

Phe Lys Pro Glu Arg Asp Gly Leu Phe Cys Ala Arg Ile Phe Gly Pro
    50                  55                  60

Val Lys Asp Tyr Glu Cys Leu Cys Gly Lys Tyr Lys Arg Leu Lys His
65                  70                  75                  80

Arg Gly Val Ile Cys Glu Lys Cys Gly Val Glu Val Thr Gln Thr Lys
                85                  90                  95

Val Arg Arg Asp Arg Met Gly His Ile Glu Leu Ala Ser Pro Val Ala
            100                 105                 110

His Ile Trp Phe Leu Lys Ser Leu Pro Ser Arg Ile Gly Leu Leu Met
        115                 120                 125

Asp Met Pro Leu Arg Asp Ile Glu Arg Val Leu Tyr Phe Glu Met Tyr
    130                 135                 140

Val Val Thr Glu Pro Gly Met Thr Asp Leu Glu Arg Gly Gln Met Leu
145                 150                 155                 160

Thr Glu Glu Glu Tyr Leu Asp Arg Leu Glu Glu Trp Gly Asp Glu Phe
                165                 170                 175

Thr Ala Lys Met Gly Ala Glu Ala Ile Lys Asp Leu Leu Ala Ser Met
            180                 185                 190

Asp Leu Pro Ala Glu Ala Glu Gln Met Arg Glu Glu Leu Asp Thr Thr
        195                 200                 205

Asn Ser Glu Thr Lys Arg Lys Lys Leu Thr Lys Arg Leu Lys Leu Val
    210                 215                 220

Glu Ala Phe Val Ala Ser Gly Asn Lys Pro Glu Trp Met Ile Leu Thr
225                 230                 235                 240

Val Leu Pro Val Leu Pro Pro Asp Leu Arg Pro Leu Val Pro Leu Asp
                245                 250                 255

Gly Gly Arg Phe Ala Thr Ser Asp Leu Asn Asp Leu Tyr Arg Arg Val
            260                 265                 270

Ile Asn Arg Asn Asn Arg Leu Lys Arg Leu Leu Glu Leu Ala Ala Pro
        275                 280                 285

Asp Ile Ile Val Arg Asn Glu Lys Arg Met Leu Gln Glu Ser Val Asp
    290                 295                 300

Ala Leu Leu Asp Asn Gly Arg Arg Gly Arg Ala Ile Thr Gly Ser Asn
305                 310                 315                 320

Lys Arg Pro Leu Lys Ser Leu Ala Asp Met Ile Lys Gly Lys Gln Gly
                325                 330                 335

Arg Phe Arg Gln Asn Leu Leu Gly Lys Arg Val Asp Tyr Ser Gly Arg
            340                 345                 350

Ser Val Ile Thr Val Gly Pro Tyr Leu Arg Leu His Gln Cys Gly Leu
        355                 360                 365

Pro Lys Lys Met Ala Leu Glu Leu Phe Lys Pro Phe Ile Tyr Ser Lys
    370                 375                 380

Leu Glu Thr Arg Gly Leu Ala Thr Thr Ile Lys Ala Ala Lys Lys Met
385                 390                 395                 400

Val Glu Arg Glu Glu Ala Val Val Trp Asp Ile Leu Asp Glu Val Ile
                405                 410                 415

Arg Glu His Pro Val Leu Leu Asn Arg Ala Pro Thr Leu His Arg Leu
            420                 425                 430

Gly Ile Gln Ala Phe Glu Pro Val Leu Ile Glu Gly Lys Ala Ile Gln
        435                 440                 445

Leu His Pro Leu Val Cys Ala Ala Tyr Asn Ala Asp Phe Asp Gly Asp
    450                 455                 460

Gln Met Ala Val His Val Pro Leu Thr Leu Glu Ala Gln Leu Glu Ala
465                 470                 475                 480

Arg Thr Leu Met Met Ser Thr Asn Asn Ile Leu Ser Pro Ala Ser Gly
                485                 490                 495

Asp Pro Ile Ile Val Pro Ser Gln Asp Val Val Leu Gly Leu Tyr Tyr
            500                 505                 510

Met Thr Arg Glu Lys Ile Asn Ala Lys Gly Glu Gly Met Tyr Leu Thr
        515                 520                 525

Gly Pro Ala Glu Ala Glu Lys Ala Tyr Arg Thr Lys Thr Ala Glu Leu
    530                 535                 540

His Ala Arg Val Lys Val Arg Ile Thr Glu Thr Ile Lys His Glu Asn

545                 550                 555                 560

Gly Lys Leu Thr Thr Glu Thr Lys Met Ile Asp Thr Thr Val Gly Arg
                565                 570                 575

Ala Met Leu Trp Gln Ile Val Pro Lys Gly Leu Pro Tyr Ser Leu Val
            580                 585                 590

Asn Gln Lys Leu Gly Lys Lys Gln Ile Ser Asn Leu Leu Asn Glu Ala
        595                 600                 605

Tyr Arg Lys Leu Gly Leu Lys Asp Thr Val Ile Phe Ala Asp Gln Ile
    610                 615                 620

Met Tyr Thr Gly Phe Ala Tyr Ala Ala Leu Ser Gly Val Ser Val Gly
625                 630                 635                 640

Ile Asp Asp Met Val Val Pro Ala Ala Lys Tyr Thr Glu Ile Ala Glu
                645                 650                 655

Ala Glu Glu Glu Val Arg Glu Ile Gln Glu Gln Phe Gln Ser Gly Leu
            660                 665                 670

Val Thr Ala Gly Glu Arg Tyr Asn Lys Val Ile Asp Ile Trp Ala Ser
        675                 680                 685

Thr Asn Asp Arg Val Ala Lys Ala Met Met Glu Asn Leu Ser Ser Glu
    690                 695                 700

Gln Val Ile Asn Arg Gln Gly Glu Gln Glu Lys Gln Glu Ser Phe Asn
705                 710                 715                 720

Ser Ile Tyr Met Met Ala Asp Ser Gly Ala Arg Gly Ser Ala Ala Gln
                725                 730                 735

Ile Arg Gln Leu Ala Gly Met Arg Gly Leu Met Ala Arg Pro Asp Gly
            740                 745                 750

Ser Ile Ile Glu Thr Pro Ile Thr Ala Asn Phe Lys Glu Gly Leu Asn
        755                 760                 765

Val Leu Gln Tyr Phe Ile Ser Thr His Gly Ala Arg Lys Gly Leu Ala
    770                 775                 780

Asp Thr Ala Leu Lys Thr Ala Asn Ser Gly Tyr Leu Thr Arg Arg Leu
785                 790                 795                 800

Val Asp Val Ala Gln Asp Val Val Val Thr Glu His Asp Cys Gly Thr
                805                 810                 815

Leu Glu Gly Val Val Met Thr Pro His Ile Glu Gly Gly Asp Val Lys
            820                 825                 830

Val Ala Leu Thr Glu Leu Ala Leu Gly Arg Val Val Ser Glu Asp Ile
        835                 840                 845

Leu Lys Pro Gly Thr Asp Glu Val Leu Ile Pro Arg Asn Thr Leu Leu
    850                 855                 860

Asp Glu Lys Trp Cys Lys Val Ile Asn Asp Asn Ser Val Asp Gln Ile
865                 870                 875                 880

Lys Val Arg Ser Val Val Thr Cys Asp Ser Asp Phe Gly Cys Cys Ala
                885                 890                 895

Gln Cys Tyr Gly Arg Asp Leu Ala Arg Gly His Leu Val Asn Gln Gly
            900                 905                 910

Glu Ala Val Gly Val Ile Ala Ala Gln Ser Ile Gly Glu Pro Gly Thr
        915                 920                 925

Gln Leu Thr Met Arg Thr Phe His Ile Gly Gly Ala Ala Ser Thr Ala
    930                 935                 940

Ala Ala Glu Asn Ser Ile Gln Ala Lys Asn Asn Gly Ser Val Lys Leu
945                 950                 955                 960

His Asn Ala Lys Phe Val Thr Asn Lys Asp Gly Lys Leu Val Ile Thr
                965                 970                 975

Ser Arg Ala Ser Glu Leu Thr Ile Ile Asp Glu Phe Gly Arg Thr Lys
            980                 985                 990

Glu Lys His Lys Leu Pro Tyr Gly Ser Met Leu Ser Lys Ala Asp Gly
        995                 1000                1005

Asp Ala Val Ala Ala Gly Glu Thr Val Ala Asn Trp Glu Ala His Thr
    1010                1015                1020

Met Pro Ile Ile Thr Glu Val Ala Gly Arg Val Gln Phe Val Asp Met
1025                1030                1035                1040

Ile Asp Gly Val Thr Val Ser Arg Gln Thr Asp Asp Leu Thr Gly Leu
                1045                1050                1055

Ser Ser Ser Glu Val Thr Glu Ala Ala Ala Arg Pro Ala Ala Gly Lys
            1060                1065                1070

Asp Met Arg Pro Ala Ile Lys Leu Val Asp Ala Asn Gly Lys Asp Val
        1075                1080                1085

Leu Ile Pro Gly Thr Asp Met Pro Ala Gln Tyr Phe Leu Pro Gly Lys
    1090                1095                1100

Ala Ile Val Asn Leu Asp Asp Gly Ala Glu Val Asn Val Gly Asp Thr
1105                1110                1115                1120

Leu Ala Arg Ile Pro Gln Lys Ser Gly Gly Asn Lys Asp Ile Thr Gly
                1125                1130                1135

Gly Leu Pro Arg Val Ala Asp Leu Phe Glu Ala Arg Lys Pro Lys Glu
            1140                1145                1150

Pro Ala Ile Leu Ala Glu His Ser Gly Thr Val Ser Phe Gly Lys Glu
        1155                1160                1165

Thr Lys Gly Lys Arg Arg Leu Ile Ile Thr Arg Asp Ser Gly Asp Thr
    1170                1175                1180

Tyr Glu Glu Met Ile Pro Lys His Arg Gln Leu Asn Val Phe Glu Gly
1185                1190                1195                1200

Glu Arg Ile Glu Arg Gly Asp Val Ile Ala Asp Gly Pro Glu Ser Pro
            1205                1210                1215

His Asp Ile Leu Arg Leu Arg Gly Ile His Ala Val Thr Thr Tyr Ile
        1220                1225                1230

Ala Asn Glu Val Gln Glu Val Tyr Arg Leu Gln Gly Val Lys Ile Asn
    1235                1240                1245

Asp Lys His Ile Glu Thr Ile Val Arg Gln Met Leu Arg Lys Cys Thr
  1250                1255                1260

Ile Thr Phe Ala Gly Asp Ser Glu Phe Leu Pro Gly Glu Thr Val Glu
1265                1270                1275                1280

Tyr Ser Gln Val Lys Ile Ala Asn Arg Lys Leu Val Glu Glu Gly Lys
            1285                1290                1295

Glu Pro Ala Arg Phe Glu Arg Glu Leu Leu Gly Ile Thr Lys Ala Ser
        1300                1305                1310

Leu Ala Thr Glu Ser Phe Ile Ser Ala Ala Ser Phe Gln Glu Thr Thr
    1315                1320                1325

Arg Val Leu Thr Glu Ala Ala Val Ser Gly Lys Arg Asp Asp Leu Arg
  1330                1335                1340

Gly Leu Lys Glu Asn Val Ile Val Gly Arg Leu Ile Pro Ala Gly Thr
1345                1350                1355                1360

Gly Phe Ala Tyr His Gln Asp Arg Gln Ala Lys Arg Ala Gln Glu Gln
            1365                1370                1375

Gln Gly Pro Ser Ala Glu Gln Ala Thr Asp Asn Leu Ala Ala Leu Leu
        1380                1385                1390

Asn Ala Gly Phe Ser Ser Asp Asp Glu
    1395                1400


<210> 43
<211> 82
<212> PRT
<213> Vibrio cholerae

<220>
<221> PEPTIDE
<222> (1)..(82)
<223> >sp|Q9KUG0|RS16_VIBCH 30S ribosomal protein S16 -
      Vibrio cholerae. (82 aa)

<400> 43
Met Val Thr Ile Arg Leu Ala Arg His Gly Ala Lys Lys Arg Pro Phe
1               5                   10                  15

Tyr Gln Ile Val Val Ala Asp Ser Arg Asn Ser Ala Thr Gly Arg Phe
            20                  25                  30

Ile Glu Lys Val Gly Phe Phe Asn Pro Thr Ala Thr Gly Gln Glu Glu
        35                  40                  45

Gly Leu Arg Leu Asp Leu Asp Arg Val Asn His Trp Val Ser Gln Gly
     50                  55                  60

Ala Ser Leu Ser Asp Arg Val Ala Gln Leu Val Lys Thr Ala Gln Lys
 65                  70                  75                  80

Ala Ala

<210> 44
<211> 122
<212> PRT
<213> Vibrio cholerae

<220>
<221> PEPTIDE
<222> (1)..(122)
<223> >sp|Q9KUZ2|RS6_VIBCH 30S ribosomal protein S6 -
      Vibrio cholerae. (122 aa)

<400> 44
Met Arg His Tyr Glu Ile Val Phe Met Val His Pro Asp Gln Ser Glu
  1               5                  10                  15

Gln Val Ala Gly Met Ile Glu Arg Tyr Thr Gly Ser Ile Thr Glu Ala
             20                  25                  30

Gly Gly Lys Ile His Arg Leu Glu Asp Trp Gly Arg Arg Gln Leu Ala
         35                  40                  45

Tyr Pro Ile Asn Lys Leu His Lys Ala His Tyr Val Leu Met Asn Val
     50                  55                  60

Glu Ala Asp Gln Ala Val Val Asp Glu Leu Glu Thr Ala Phe Arg Phe
 65                  70                  75                  80

Asn Asp Ala Val Leu Arg Asn Met Ile Met Arg Thr Lys Ala Ala Ile
                 85                  90                  95

Thr Glu Pro Ser Ile Met Leu Lys Ala Arg Glu Glu Arg Val Lys Arg
            100                 105                 110

Asp Glu Met Lys Phe Asp Ala Asp Val Glu
        115                 120

<210> 45
<211> 85
<212> PRT
<213> Vibrio cholerae

<220>
<221> PEPTIDE
<222> (1)..(85)
<223> >sp|Q9KTD6|PTHP_VIBCH Phosphocarrier protein HPr
      (Histidine-containing protein) - Vibrio cholerae.
      (85 aa)

<400> 45
Met Tyr Glu Lys Gln Val Glu Ile Thr Ala Glu Asn Gly Leu His Thr

1               5                   10                  15

Arg Pro Ala Ala Gln Phe Val Lys Glu Ala Lys Ala Phe Asp Ala Asp
            20                  25                  30

Ile Thr Val Thr Ser Asn Gly Lys Ser Ala Ser Ala Lys Ser Leu Phe
        35                  40                  45

Lys Leu Gln Thr Leu Gly Leu Val Lys Gly Thr Val Val Thr Ile Ser
    50                  55                  60

Ala Glu Gly Pro Gln Ala Lys Glu Ala Val Glu His Leu Val Ala Leu
65                  70                  75                  80

Met Asp Gln Leu His
                85


<210> 46
<211> 894
<212> PRT
<213> Vibrio cholerae

<220>
<221> PEPTIDE
<222> (1)..(894)
<223> >tr|Q9KQG5|Q9KQG5_VIBCH Alcohol
      dehydrogenase/acetaldehyde dehydrogenase - Vibrio
      cholerae.

<400> 46
Met Pro Val Thr Asn Leu Ala Glu Leu Asp Ala Leu Val Ala Arg Val
1               5                   10                  15

Lys Ala Ala Gln Ala Glu Phe Ala Thr Phe Ser Gln Glu Gln Val Asp
            20                  25                  30

Lys Ile Phe Arg Ala Ala Ser Leu Ala Ala Asn Gln Ala Arg Ile Pro
        35                  40                  45

Leu Ala Gln Met Ala Val Glu Glu Ser Gly Met Gly Ile Val Glu Asp
    50                  55                  60

Lys Val Ile Lys Asn His Phe Ala Ser Glu Phe Ile Tyr Asn Lys Tyr
65                  70                  75                  80

Lys Asp Glu Lys Thr Cys Gly Ile Leu Glu Glu Asp Asp Asn Leu Gly
                85                  90                  95

Thr Met Thr Ile Ala Glu Pro Val Gly Ile Ile Cys Gly Ile Val Pro
            100                 105                 110

Thr Thr Asn Pro Thr Ser Thr Ala Ile Phe Lys Ser Leu Ile Ser Leu
        115                 120                 125

Lys Thr Arg Asn Gly Ile Ile Phe Ser Pro His Pro Arg Ala Lys Asn
    130                 135                 140

Ser Thr Asn Ala Ala Ala Lys Leu Val Leu Asp Ala Ala Ile Ala Ala
145                 150                 155                 160

Gly Ala Pro Lys Asp Ile Ile Gly Trp Ile Asp Gln Pro Ser Val Glu
                165                 170                 175

Leu Ser Asn Ala Leu Met Lys His Asp Gly Ile Ala Leu Ile Leu Ala
            180                 185                 190

Thr Gly Gly Pro Gly Met Val Lys Ala Ala Tyr Ser Ser Gly Lys Pro
        195                 200                 205

Ala Ile Gly Val Gly Ala Gly Asn Val Pro Val Val Ile Asp Glu Thr
    210                 215                 220

Ala Asp Ile Lys Arg Ala Val Ala Ser Ile Leu Met Ser Lys Thr Phe
225                 230                 235                 240

Asp Asn Gly Val Val Cys Ala Ser Glu Gln Ala Ala Ile Val Val Ser
                245                 250                 255

Glu Val Tyr Asp Glu Val Lys Glu Arg Phe Ala Thr His Lys Ala His
            260                 265                 270

Val Leu Ser Lys Ala Asp Ala Asp Lys Val Arg Lys Val Leu Leu Ile
        275                 280                 285

Asp Gly Ala Leu Asn Ala Lys Ile Val Gly Gln Pro Ala Ala Ala Ile
    290                 295                 300

Ala Glu Met Ala Gly Val Lys Val Pro Ala Asp Thr Lys Val Leu Val

305                 310                 315                 320

Gly Glu Gly Leu Gly Lys Val Ser Tyr Asp Asp Glu Phe Ala His Glu
                325                 330                 335

Lys Leu Ser Pro Thr Leu Gly Leu Phe Arg Ala Asp Asn Phe Glu Asp
            340                 345                 350

Ala Val Ala Gln Ala Val Thr Met Val Glu Ile Gly Gly Ile Gly His
        355                 360                 365

Thr Ser Gly Leu Tyr Thr Asn Gln Asp Val Asn Ala Asp Arg Ile Arg
    370                 375                 380

Tyr Phe Gly Asp Lys Leu Lys Thr Ala Arg Ile Leu Val Asn Ile Pro
385                 390                 395                 400

Thr Thr His Gly Gly Ile Gly Asp Leu Tyr Asn Phe Asn Val Ala Pro
                405                 410                 415

Ser Leu Thr Leu Gly Cys Gly Ser Trp Gly Gly Asn Ser Ile Ser Glu
            420                 425                 430

Asn Val Gly Pro Lys His Leu Ile Asn Lys Lys Thr Val Ala Lys Arg
        435                 440                 445

Ala Glu Asn Met Leu Trp His Lys Leu Pro Lys Ser Ile Tyr Phe Arg
    450                 455                 460

Arg Gly Ser Leu Pro Ile Ala Leu Ser Asp Leu Glu Gly Lys Lys Arg
465                 470                 475                 480

Ala Phe Leu Val Thr Asp Arg Phe Leu Phe Asn Asn Gly Tyr Ala Asp
                485                 490                 495

Asp Val Val Ala Leu Leu Lys Ala Gln Gly Met Glu Val Gln Thr Phe
            500                 505                 510

Phe Glu Val Glu Ala Asp Pro Thr Leu Ser Val Val Glu Lys Gly Ala
        515                 520                 525

Ala Ala Met Gln Ser Phe Gln Pro Asp Val Ile Leu Ala Leu Gly Gly
    530                 535                 540

Gly Ser Pro Met Asp Ala Ala Lys Ile Met Trp Val Met Tyr Glu His
545                 550                 555                 560

Pro Asp Thr His Phe Glu Glu Leu Ala Met Arg Phe Met Asp Ile Arg
                565                 570                 575

Lys Arg Ile Tyr Lys Phe Pro Lys Met Gly Lys Lys Ala Glu Leu Val
            580                 585                 590

Cys Ile Thr Thr Thr Ser Gly Thr Gly Ser Glu Val Thr Pro Phe Ala
        595                 600                 605

Val Val Thr Asp Asp Lys Thr Gly Ala Lys Tyr Pro Leu Ala Asp Tyr
    610                 615                 620

Glu Leu Thr Pro Gln Met Ala Ile Val Asp Ala Asn Leu Val Met Asn
625                 630                 635                 640

Met Pro Lys Ser Leu Thr Ala Phe Gly Gly Tyr Asp Ala Val Thr His
                645                 650                 655

Ala Leu Glu Ala Tyr Val Ser Val Leu Ala Asn Glu Tyr Ser Asp Gly
            660                 665                 670

Gln Ala Leu Gln Ala Leu Lys Met Leu Lys Glu Tyr Leu Pro Ser Ser
        675                 680                 685

Tyr Ala Asn Gly Ala Lys Asp Pro Ile Ala Arg Glu Lys Val His Asn
    690                 695                 700

Ala Ala Thr Ile Ala Gly Ile Ala Phe Ala Asn Ala Phe Leu Gly Val
705                 710                 715                 720

Cys His Ser Met Ala His Lys Ile Gly Ala Glu Phe His Leu Pro His
                725                 730                 735

Gly Leu Ala Asn Ala Leu Leu Ile Ala Asn Val Val Arg Tyr Asn Ala
            740                 745                 750

Asn Asp Asn Pro Thr Lys Gln Thr Ala Phe Ser Gln Tyr Asp Arg Pro
        755                 760                 765

Gln Ala Arg Arg Arg Tyr Ala Glu Val Ala Asp His Leu Gly Leu Ser
    770                 775                 780

Gln Pro Gly Asp Arg Thr Ala Gln Lys Ile Glu Arg Leu Leu Thr Trp
785                 790                 795                 800

Leu Asp Glu Leu Lys Val Asn Leu Asp Ile Pro Lys Ser Ile Gln Ala

805                 810                 815

Ala Gly Val Ala Glu Ala Asp Phe Leu Ala Lys Val Asp Glu Leu Ala
            820                 825                 830

Val Glu Ala Phe Asp Asp Gln Cys Thr Gly Ala Asn Pro Arg Tyr Pro
        835                 840                 845

Leu Ile Ala Glu Leu Lys Glu Val Leu Leu Ala Ser Tyr Tyr Gly Lys
    850                 855                 860

Pro Phe Val Glu Gly Gln Thr Phe Glu Gly Thr Thr Val Ile Val Lys
865                 870                 875                 880

Lys Ala Asp Gln Glu Ala Ala Lys Ala Pro Lys Ala Lys Lys
                885                 890

<210> 47
<211> 200
<212> PRT
<213> Vibrio cholerae

<220>
<221> PEPTIDE
<222> (1)..(200)
<223> >sp|Q9KNY5|RL4_VIBCH 50S ribosomal protein L4 -
      Vibrio cholerae. (200 aa)

<400> 47
Met Glu Leu Met Val Lys Gly Ala Asn Ala Leu Thr Val Ser Glu Thr
1               5                   10                  15

Thr Phe Gly Arg Glu Phe Asn Glu Ala Leu Val His Gln Val Val Val
            20                  25                  30

Ala Tyr Ala Ala Gly Ala Arg Gln Gly Thr Arg Ala Gln Lys Thr Arg
        35                  40                  45

Ser Glu Val Ser Gly Gly Gly Ala Lys Pro Trp Arg Gln Lys Gly Thr
    50                  55                  60

Gly Arg Ala Arg Ala Gly Thr Ile Arg Ser Pro Ile Trp Arg Thr Gly
65                  70                  75                  80

Gly Val Thr Phe Ala Ala Lys Pro Gln Asp His Ser Gln Lys Val Asn
                85                  90                  95

Lys Lys Met Tyr Arg Gly Ala Met Lys Ser Ile Leu Ser Glu Leu Val
            100                 105                 110

Arg Gln Glu Arg Leu Ile Val Val Glu Asn Phe Ser Val Glu Ala Pro
        115                 120                 125

Lys Thr Lys Ala Leu Val Ala Lys Leu Lys Glu Leu Glu Leu Asn Asp
    130                 135                 140

Val Leu Ile Val Thr Gly Glu Val Asp Glu Asn Leu Phe Leu Ala Ala
145                 150                 155                 160

Arg Asn Leu Tyr Lys Val Asp Val Arg Asp Val Thr Gly Ile Asp Pro

165                 170                 175

Val Ser Leu Ile Ala Phe Asp Lys Val Leu Met Thr Ala Ala Ala Val
            180                 185                 190

Lys Gln Val Glu Glu Met Leu Ala
        195                 200


<210> 48
<211> 429
<212> PRT
<213> Vibrio cholerae

<220>
<221> PEPTIDE
<222> (1)..(429)
<223> >sp|Q9KV81|PUR2_VIBCH Phosphoribosylamine--glycine
      ligase (EC 6.3.4.13) (GARS) (Glycinamide
      ribonucleotide synthetase)
      (Phosphoribosylglycinamide synthetase) - Vibrio
      cholerae. (429 aa)

<400> 48
Met Gln Val Leu Ile Ile Gly Ser Gly Gly Arg Glu His Ala Leu Ala
1               5                   10                  15

Trp Lys Val Ala Gln Asn Pro Gln Val Asp Thr Ile Tyr Val Ala Pro
            20                  25                  30

Gly Asn Ala Gly Thr Ala Leu Glu His Lys Val Gln Asn Val Asn Ile
        35                  40                  45

Gly Ile Thr Asp Ile Pro Ala Leu Val Ala Phe Ala Gln Asp Lys Ala
    50                  55                  60

Ile Glu Leu Thr Ile Val Gly Pro Glu Ala Pro Leu Val Ile Gly Val
65                  70                  75                  80

Val Asp Ala Phe Arg Ala Ala Gly Leu Pro Ile Phe Gly Pro Thr Gln
                85                  90                  95

Gly Ala Ala Gln Leu Glu Gly Ser Lys Ala Phe Thr Lys Asp Phe Leu
            100                 105                 110

Ala Arg His Asn Ile Pro Thr Ala Ala Tyr Ala Asn Phe Thr Glu Ile
        115                 120                 125

Glu Pro Ala Leu Ala Tyr Val Arg Glu Lys Gly Ala Pro Ile Val Val
    130                 135                 140

Lys Ala Asp Gly Leu Ala Ala Gly Lys Gly Val Ile Val Ala Met Thr
145                 150                 155                 160

Leu Gln Glu Ala Glu Asp Ala Ile Gln Asp Met Leu Ala Gly Asn Ala
                165                 170                 175

Phe Gly Ser Ala Gly Ser Arg Val Val Val Glu Glu Phe Leu Asp Gly
            180                 185                 190

Glu Glu Ala Ser Phe Ile Val Met Val Asp Gly Glu Asn Val Leu Pro

195                 200                 205

Met Ala Thr Ser Gln Asp His Lys Arg Val Gly Asp Ala Asp Thr Gly
    210                 215                 220

Pro Asn Thr Gly Gly Met Gly Ala Tyr Ser Pro Ala Pro Val Val Thr
225                 230                 235                 240

Gln Asp Val His Asp Arg Val Met Arg Glu Val Ile Asp Pro Thr Val
                245                 250                 255

Arg Gly Met Ala Ala Glu Gly Asn Thr Tyr Thr Gly Phe Leu Tyr Ala
            260                 265                 270

Gly Leu Met Ile Asp Ser Thr Gly Ala Pro Lys Val Ile Glu Tyr Asn
        275                 280                 285

Cys Arg Phe Gly Asp Pro Glu Thr Gln Pro Ile Met Met Arg Leu Gln
    290                 295                 300

Ser Asp Leu Val Glu Leu Cys Gln Ala Ala Ile Ala Gly Lys Leu Asp
305                 310                 315                 320

Gln Val Glu Ser Lys Trp Asp Pro Arg Ala Ser Ile Gly Val Val Leu
                325                 330                 335

Ala Ala Gly Gly Tyr Pro Gly Asp Tyr Ala Lys Gly Glu Val Ile Ser
            340                 345                 350

Gly Leu Pro Thr Gln Glu Ser Ala Gly Gln Lys Val Phe His Ala Gly
        355                 360                 365

Thr Glu Thr Gln Gly Asp Gln Val Val Thr Asn Gly Gly Arg Val Leu
    370.                375                 380

Cys Ala Thr Ala Leu Gly Asn Thr Val Leu Glu Ala Gln Gln Arg Ala
385                 390                 395                 400

Tyr Gln Leu Ala Asp Gln Ile His Trp Asn Gly Met Phe Cys Arg Arg
                405                 410                 415

Asp Ile Gly Tyr Arg Ala Ile Ala Arg Glu Gln Ala Lys
            420                 425


<210> 49
<211> 645
<212> PRT
<213> Vibrio cholerae

<220>
<221> PEPTIDE
<222> (1)..(645)
<223> >sp|Q9KVS8|THIC_VIBCH Thiamine biosynthesis
      protein thiC - Vibrio cholerae. (645 aa)

<400> 49
Met Ser Asn Arg Lys Gln Ala Arg Leu Glu Ala Lys Arg Phe Ile Asp
1               5                   10                  15

Thr Leu Ser Val Glu Pro Tyr Pro Asn Ser Gln Lys Ser Tyr Leu Leu

            20                  25                  30

Gly Ser Arg Pro Asp Ile Arg Val Pro Val Arg Glu Ile Thr Leu Ser
        35                  40                  45

Asp Thr Leu Val Gly Gly Ser Lys Asp Ala Pro Ile Phe Glu Pro Asn
    50                  55                  60

Glu Pro Ile Cys Val Tyr Asp Thr Ser Gly Val Tyr Thr Asp Pro Ser
65                  70                  75                  80

His Asp Ile Asp Leu Tyr Lys Gly Leu Pro Lys Leu Arg Glu Glu Trp
                85                  90                  95

Ile Glu Glu Arg Arg Asp Thr His Ile Leu Pro Ser Met Ser Ser His
            100                 105                 110

Phe Ala Arg Glu Arg Leu Ala Asp Glu Thr Leu Asp Glu Leu Arg Tyr
        115                 120                 125

Gly His Leu Pro Arg Ile Arg Arg Ala Met Gly Gln His Arg Val Thr
    130                 135                 140

Gln Leu His Tyr Ala Arg Gln Gly Ile Ile Thr Pro Glu Met Glu Phe
145                 150                 155                 160

Val Ala Ile Arg Glu Asn Ser Arg Arg Leu Ala His Gln Asp Pro Ser
                165                 170                 175

Leu Leu Gln Gln His Ala Gly Gln Asn Phe Gly Ala His Leu Pro Asp
            180                 185                 190

Leu Ile Thr Pro Glu Phe Val Arg Arg Glu Ile Ala Glu Gly Arg Ala
        195                 200                 205

Ile Ile Pro Cys Asn Ile Asn His Pro Glu Ser Glu Pro Met Ile Ile
    210                 215                 220

Gly Arg Asn Phe Leu Val Lys Val Asn Ala Asn Ile Gly Asn Ser Ser
225                 230                 235                 240

Val Ser Ser Ser Ile Glu Glu Glu Val Glu Lys Leu Val Trp Ala Thr
                245                 250                 255

Arg Trp Gly Ala Asp Thr Val Met Asp Leu Ser Thr Gly Arg Asn Ile
            260                 265                 270

His Glu Thr Arg Glu Trp Ile Leu Arg Asn Ser Pro Val Pro Ile Gly
        275                 280                 285

Thr Val Pro Met Tyr Gln Ala Leu Glu Lys Val Asn Gly Val Ala Glu
    290                 295                 300

Asn Leu Thr Trp Glu Val Met Arg Asp Thr Leu Leu Glu Gln Ala Glu
305                 310                 315                 320

Gln Gly Val Asp Tyr Phe Thr Ile His Ala Gly Leu Leu Leu Arg Tyr
                325                 330                 335

Val Pro Met Thr Ala Lys Arg Val Thr Gly Ile Val Ser Arg Gly Gly

            340                 345                 350

Ser Ile Ile Ala Lys Trp Cys Leu Ser His His Gln Glu Asn Phe Leu
        355                 360                 365

Tyr Thr His Phe Arg Glu Ile Cys Glu Ile Cys Ala Gln Tyr Asp Val
    370                 375                 380

Ala Leu Ser Leu Gly Asp Gly Leu Arg Pro Gly Ser Ile Ala Asp Ala
385                 390                 395                 400

Asn Asp Glu Ala Gln Phe Ala Glu Leu Arg Thr Leu Gly Glu Leu Thr
                405                 410                 415

Gln Ile Ala Trp Glu Tyr Asp Val Gln Val Met Ile Glu Gly Pro Gly
            420                 425                 430

His Val Pro Met His Leu Ile Lys Ala Asn Met Asp Glu Gln Leu Lys
        435                 440                 445

His Cys His Glu Ala Pro Phe Tyr Thr Leu Gly Pro Leu Thr Thr Asp
    450                 455                 460

Ile Ala Pro Gly Tyr Asp His Ile Thr Ser Gly Ile Gly Ala Ala Met
465                 470                 475                 480

Ile Gly Trp Phe Gly Cys Ala Met Leu Cys Tyr Val Thr Pro Lys Glu
                485                 490                 495

His Leu Gly Leu Pro Asn Lys Glu Asp Val Lys Thr Gly Leu Ile Thr
            500                 505                 510

Tyr Lys Leu Ala Ala His Ala Ala Asp Leu Ala Lys Gly His Pro Gly
        515                 520                 525

Ala Gln Ile Arg Asp Asn Ala Leu Ser Lys Ala Arg Phe Glu Phe Arg
    530                 535                 540

Trp Glu Asp Gln Phe Asn Leu Ala Leu Asp Pro Val Thr Ala Arg Ala
545                 550                 555                 560

Phe His Asp Glu Thr Leu Pro Gln Glu Ser Gly Lys Val Ala His Phe
                565                 570                 575

Cys Ser Met Cys Gly Pro Lys Phe Cys Ser Met Lys Ile Ser Gln Glu
            580                 585                 590

Val Arg Asp Tyr Ala Asn Asn Gln Thr Leu Asp Thr Thr Val Ile Asp
        595                 600                 605

Leu Val Met Pro Ala Glu Ser Ile Gln Leu Ala Met Gln Asp Lys Ser
    610                 615                 620

Arg Glu Phe Leu Ala Ser Gly Ala Glu Leu Tyr His Pro Leu Val Lys
625                 630                 635                 640

Glu Pro Ile Glu Glu
                645

<210> 50

<211> 156
<212> PRT
<213> Vibrio cholerae

<220>
<221> PEPTIDE
<222> (1)..(156)
<223> >sp|Q9KUZ8|RS7_VIBCH 30S ribosomal protein S7 -
      Vibrio cholerae. (156 aa)

<400> 50
Met Pro Arg Arg Arg Val Ile Gly Gln Arg Lys Ile Leu Pro Asp Pro
1               5                   10                  15

Lys Phe Lys Ser Glu Leu Leu Ala Lys Phe Val Asn Ile Leu Met Val
            20                  25                  30

Asp Gly Lys Lys Ser Thr Ala Glu Lys Ile Val Tyr Thr Ala Leu Asp
        35                  40                  45

Thr Met Ala Glu Lys Ser Gly Lys Asp His Leu Ala Val Phe Glu Glu
    50                  55                  60

Ala Leu Glu Asn Val Arg Pro Ala Val Glu Val Lys Ser Arg Arg Val
65                  70                  75                  80

Gly Gly Ser Thr Tyr Gln Val Pro Val Glu Val Arg Pro Val Arg Arg
                85                  90                  95

Asn Ala Leu Ala Met Arg Trp Leu Val Glu Ala Ala Arg Lys Arg Gly
            100                 105                 110

Glu Lys Ser Met Ala Ala Arg Leu Ala Ala Glu Met Leu Asp Ala Ala
        115                 120                 125

Glu Asn Lys Gly Ser Ala Val Lys Lys Arg Glu Asp Val His Arg Met
    130                 135                 140

Ala Glu Ala Asn Lys Ala Phe Ala His Tyr Arg Trp
145                 150                 155


<210> 51
<211> 103
<212> PRT
<213> Vibrio cholerae

<220>
<221> PEPTIDE
<222> (1)..(103)
<223> >sp|Q9KNY3|RS10_VIBCH 30S ribosomal protein S10 -
      Vibrio cholerae. (103 aa)

<400> 51
Met Gln Asn Gln Arg Ile Arg Ile Arg Leu Lys Ala Phe Asp Tyr Lys
1               5                   10                  15

Leu Ile Asp Ala Ser Thr Ala Glu Ile Val Glu Thr Ala Lys Arg Thr
            20                  25                  30

Gly Ala Gln Val Arg Gly Pro Ile Pro Leu Pro Thr Arg Lys Glu Arg

35                  40                  45

Phe Thr Val Leu Ile Ser Pro His Val Asn Lys Asp Ala Arg Asp Gln
    50                  55                  60

Tyr Glu Ile Arg Thr His Lys Arg Leu Ile Asp Ile Val Glu Pro Thr
65                  70                  75                  80

Asp Lys Thr Val Asp Ala Leu Met Arg Leu Asp Leu Ala Ala Gly Val
                85                  90                  95

Asp Val Gln Ile Ser Leu Gly
            100

<210> 52
<211> 288
<212> PRT
<213> Vibrio cholerae

<220>
<221> PEPTIDE
<222> (1)..(288)
<223> >tr|Q9KNH4|Q9KNH4_VIBCH ATP synthase F1, gamma
      subunit - Vibrio cholerae. (288 aa)

<400> 52
Met Ala Gly Ala Lys Glu Ile Arg Thr Lys Ile Gly Ser Val Lys Ser
1               5                   10                  15

Thr Gln Lys Ile Thr Lys Ala Met Glu Met Val Ala Ala Ser Lys Met
            20                  25                  30

Arg Arg Ser Gln Asp Ala Met Glu Ser Ser Arg Pro Tyr Ala Gln Thr
        35                  40                  45

Ile Arg Lys Val Ile Gly His Val Ala Asn Ala Ser Leu Glu Tyr Arg
    50                  55                  60

His Pro Tyr Leu Glu Glu Arg Glu Ala Lys Arg Val Gly Tyr Ile Ile
65                  70                  75                  80

Ile Ser Thr Asp Arg Gly Leu Cys Gly Gly Leu Asn Ile Asn Leu Phe
                85                  90                  95

Lys Lys Ala Ile Thr Asp Met Gln Thr Trp Lys Glu Lys Gly Ala Gln
            100                 105                 110

Ile Glu Leu Ala Ile Ile Gly Ser Lys Ala Thr Ala Phe Phe Asn Asn
        115                 120                 125

Ser Gly Ala Lys Val Ala Ala Gln Val Ser Gly Leu Gly Asp Ser Pro
    130                 135                 140

Ser Leu Glu Asp Leu Ile Gly Ser Val Gly Val Met Leu Lys Lys Tyr
145                 150                 155                 160

Asp Lys Gly Glu Leu Asp Arg Leu Tyr Leu Val Phe Asn Gln Phe Val
                165                 170                 175

Asn Thr Met Val Gln Lys Pro Lys Ile Asp Gln Leu Leu Pro Leu Pro

180                 185                 190

Lys Ser Asp Ser Glu Asp Met Gln Arg Asp His Met Trp Asp Tyr Ile
        195                 200                 205

Tyr Glu Pro Glu Pro Lys Pro Leu Leu Asp Ala Leu Leu Leu Arg Phe
    210                 215                 220

Ile Glu Ser Gln Val Tyr Gln Gly Val Val Glu Asn Leu Ala Cys Glu
225                 230                 235                 240

Gln Ala Ala Arg Met Val Ala Met Lys Ala Ala Thr Asp Asn Ala Ser
                245                 250                 255

Asn Leu Ile Asp Asp Leu Gln Leu Val Tyr Asn Lys Ala Arg Gln Ala
            260                 265                 270

Ala Ile Thr Gln Glu Leu Ser Glu Ile Val Gly Gly Ala Ala Ala Val
        275                 280                 285

<210> 53
<211> 343
<212> PRT
<213> Vibrio cholerae

<220>
<221> PEPTIDE
<222> (1)..(343)
<223> >tr|Q9KPN7|Q9KPN7_VIBCH
      2,3,4,5-tetrahydropyridine-2-carboxylate
      N-succinyltransferase - Vibrio cholerae. (343 aa)

<400> 53
Met Ala Tyr Phe Ala Leu Gly Leu Ala Thr Ala Thr Lys Asn Arg Asp
1               5                   10                  15

Gly Lys Ile Ile Glu Ala Phe Phe Pro Thr Pro Ile Leu Ala Pro Ser
            20                  25                  30

Asp Ala Leu Val Ala Ala Leu Ala Pro Ile Ala Gly Tyr Gln Glu Gly
        35                  40                  45

Asn Gln Ala Leu Asp Ile Thr Ala Ala Gln Ser Ala Gln Leu Ala Ala
    50                  55                  60

Val Phe Ala Ala His Gln Gln Ala Ala Ser Ala Ala Phe Ala Asp Lys
65                  70                  75                  80

Ala Ala Asn Ala Lys Gln Pro Leu Val Leu Val Ile Leu Ala Ser Asp
                85                  90                  95

Asp Lys Pro Gln Ser Val Ala Glu Gly Tyr Leu Lys Leu Gln Leu Ile
            100                 105                 110

Ser His Arg Leu Val Lys Pro His Gly Thr Val Leu Asp Gly Ile Phe
        115                 120                 125

Gly Leu Leu His Asn Ile Ala Trp Thr Asn Glu Gly Pro Ile Asp Leu
    130                 135                 140

Pro Glu Leu Ala Glu Arg Gln Ile Glu Ala Arg Leu Ala Gly Arg Val
145                 150                 155                 160

Leu Thr Val Asp Cys Val Asp Lys Phe Pro Lys Met Val Asp Tyr Val
                165                 170                 175

Val Pro Ala Gly Ile Arg Ile Ala Asp Thr Ser Arg Val Arg Leu Gly
            180                 185                 190

Ala His Val Gly Glu Gly Thr Thr Val Met His Glu Gly Phe Ile Asn
        195                 200                 205

Phe Asn Ala Gly Thr Thr Gly Val Ser Met Val Glu Gly Arg Ile Ser
    210                 215                 220

Ala Gly Val Val Val Gly Asn Gly Ser Asp Ile Gly Gly Gly Ala Ser
225                 230                 235                 240

Ile Met Gly Thr Leu Ser Gly Gly Gly Lys Val Val Val Ser Ile Gly
                245                 250                 255

Glu Asn Ser Leu Leu Gly Ala Asn Ala Gly Leu Gly Phe Pro Leu Gly
            260                 265                 270

Asp Arg Cys Thr Val Glu Ser Gly Leu Tyr Val Thr Ala Gly Thr Lys
        275                 280                 285

Val Arg Thr Leu Asp Lys Asp Gly Asn Gln Val Asp Ile Val Lys Ala
    290                 295                 300

Arg Asp Leu Ala Gly Val Ser Asp Leu Leu Phe Arg Arg Asn Ser Leu
305                 310                 315                 320

Thr Gly Gln Ile Glu Cys Leu Ala Asn Lys Ser Ala Val Glu Leu Asn
                325                 330                 335

Ser Glu Leu His Lys Asn Asn
            340


<210> 54
<211> 466
<212> PRT
<213> Vibrio cholerae

<220>
<221> PEPTIDE
<222> (1)..(466)
<223> >sp|Q9KSF9|SYN_VIBCH Asparaginyl-tRNA synthetase
      (EC 6.1.1.22) (Asparagine--tRNA ligase) (AsnRS) -
      Vibrio cholerae. (466 aa)

<400> 54
Met Thr Tyr Ala Pro Val Asn Asp Val Leu Ser Gly Lys Leu Ala Val
1               5                   10                  15

Asp Ser Glu Val Thr Val Arg Gly Trp Ile Arg Thr Arg Arg Asp Ser
            20                  25                  30

Lys Ala Gly Ile Ser Phe Leu Ala Ile Tyr Asp Gly Ser Cys Phe Asn
        35                  40                  45

Pro Ile Gln Ala Val Val Pro Asn Asn Leu Asn Asn Tyr Asp Asn Glu

    50                  55                  60

Val Leu Lys Leu Thr Thr Gly Cys Ser Val Glu Val Thr Gly Lys Ile
65                  70                  75                  80

Val Glu Ser Pro Ala Ser Gly Gln Ala Phe Glu Leu Ala Ala Ser Asp
                85                  90                  95

Val Lys Val Val Gly Trp Val Glu Asp Ala Asp Thr Tyr Pro Met Ala
            100                 105                 110

Lys Thr Arg His Ser Ile Glu Tyr Leu Arg Glu Val Ala His Leu Arg
        115                 120                 125

Pro Arg Thr Asn Val Ile Gly Ala Val Ala Arg Val Arg Asn Cys Leu
    130                 135                 140

Ala Gln Ala Ile His Arg Phe Tyr His Glu Gln Gly Tyr Phe Trp Val
145                 150                 155                 160

Ser Ala Pro Leu Ile Thr Ala Ser Asp Ala Glu Gly Ala Gly Glu Met
                165                 170                 175

Phe Arg Val Ser Thr Leu Asp Met Glu Asn Leu Pro Arg Thr Asp Ala
            180                 185                 190

Gly Lys Val Asp Tyr Asn Gln Asp Phe Phe Gly Lys Glu Thr Phe Leu
        195                 200                 205

Thr Val Ser Gly Gln Leu Asn Ala Glu Ala Tyr Ala Cys Ala Ile Ser
    210                 215                 220

Lys Val Tyr Thr Phe Gly Pro Thr Phe Arg Ala Glu Asn Ser Asn Thr
225                 230                 235                 240

Ser Arg His Leu Ala Glu Phe Trp Met Val Glu Pro Glu Val Ala Phe
                245                 250                 255

Ala Asp Leu Asn Thr Val Ala Lys Leu Ala Glu Asp Met Leu Lys Tyr
            260                 265                 270

Val Phe Lys Ala Val Leu Ala Glu Arg Arg Asp Asp Leu Glu Phe Phe
        275                 280                 285

Asn Asp Arg Ile Asn Asn Glu Val Ile Ala Arg Leu Glu Gln Phe Val
    290                 295                 300

Glu Ser Asp Phe Ala Gln Val Asp Tyr Thr Asp Ala Ile Glu Ile Leu
305                 310                 315                 320

Lys Asn Cys Gly Lys Thr Phe Glu Phe Pro Val Glu Trp Gly Ile Asp
                325                 330                 335

Leu Ala Ser Glu His Glu Arg Phe Leu Ala Glu Glu His Phe Lys Ala
            340                 345                 350

Pro Val Ile Val Lys Asn Tyr Pro Lys Asp Ile Lys Ala Phe Tyr Met
        355                 360                 365

Arg Met Asn Glu Asp Gly Lys Thr Val Ala Ala Met Asp Val Leu Ala
    370                 375                 380

Pro Gly Ile Gly Glu Ile Ile Gly Gly Ser Gln Arg Glu Glu Arg Leu
385                 390                 395                 400

Asp Ile Leu Asp Ala Arg Met Arg Glu Phe Gly Ile Asp Pro Glu His
                405                 410                 415

Met Asp Trp Tyr Arg Asp Leu Arg Arg Tyr Gly Thr Val Pro His Ala
            420                 425                 430

Gly Phe Gly Leu Gly Phe Glu Arg Leu Val Ser Tyr Val Thr Gly Met
        435                 440                 445

Gly Asn Val Arg Asp Val Ile Pro Phe Pro Arg Thr Pro Arg Ser Ala
    450                 455                 460

Ser Phe
465

<210> 55
<211> 90
<212> PRT
<213> Vibrio cholerae

<220>
<221> PEPTIDE
<222> (1)..(90)
<223> >sp|Q9KNM3|RPOZ_VIBCH DNA-directed RNA polymerase
omega chain (EC 2.7.7.6) (RNAP omega subunit)
(Transcriptase omega chain) (RNA polymerase omega
subunit) - Vibrio cholerae. (90 aa)

<400> 55
Met Ala Arg Val Thr Val Gln Asp Ala Val Glu Lys Ile Gly Asn Arg
1               5                   10                  15

Phe Asp Leu Val Leu Val Ala Ala Arg Arg Ala Arg Gln Met Gln Ser
            20                  25                  30

Gly Gly Lys Asp Ala Leu Val Pro Glu Glu Asn Asp Lys Pro Thr Val
        35                  40                  45

Ile Ala Leu Arg Glu Ile Glu Glu Gly Leu Ile Thr Lys Asp Val Leu
    50                  55                  60

Asp Ala Arg Glu Arg Gln Glu Gln Gln Glu Gln Glu Ala Ala Glu Leu
65                  70                  75                  80

Ala Ala Val Ser Ser Ile Met His Asn Arg
                85                  90

<210> 56
<211> 544

<212> PRT
<213> Vibrio cholerae

<220>
<221> PEPTIDE
<222> (1)..(544)
<223> >sp|Q9KNR7|CH601_VIBCH 60 kDa chaperonin 1
      (Protein Cpn60 1) (groEL protein 1) - Vibrio
      cholerae. (544 aa)

<400> 56
Met Ala Ala Lys Asp Val Arg Phe Gly Asn Asp Ala Arg Val Lys Met
1               5                   10                  15

Leu Glu Gly Val Asn Ile Leu Ala Asp Ala Val Lys Val Thr Leu Gly
            20                  25                  30

Pro Lys Gly Arg Asn Val Val Leu Asp Lys Ser Phe Gly Ala Pro Thr
        35                  40                  45

Ile Thr Lys Asp Gly Val Ser Val Ala Arg Glu Ile Glu Leu Glu Asp
    50                  55                  60

Lys Phe Gln Asn Met Gly Ala Gln Met Val Lys Glu Val Ala Ser Gln
65                  70                  75                  80

Ala Asn Asp Ala Ala Gly Asp Gly Thr Thr Thr Ala Thr Val Leu Ala
                85                  90                  95

Gln Ala Ile Val Asn Glu Gly Leu Lys Ala Val Ala Ala Gly Met Asn
            100                 105                 110

Pro Met Asp Leu Lys Arg Gly Ile Asp Lys Ala Val Ile Ala Ala Val
        115                 120                 125

Glu Glu Leu Lys Ala Leu Ser Val Pro Cys Ala Asp Thr Lys Ala Ile
    130                 135                 140

Ala Gln Val Gly Thr Ile Ser Ala Asn Ser Asp Ser Ser Val Gly Asn
145                 150                 155                 160

Ile Ile Ala Glu Ala Met Glu Lys Val Gly Arg Asp Gly Val Ile Thr
                165                 170                 175

Val Glu Glu Gly Gln Ala Leu Gln Asp Glu Leu Asp Val Val Glu Gly
            180                 185                 190

Met Gln Phe Asp Arg Gly Tyr Leu Ser Pro Tyr Phe Ile Asn Asn Gln
        195                 200                 205

Glu Ser Gly Ser Val Glu Leu Asp Asn Pro Phe Ile Leu Leu Val Asp
    210                 215                 220

Lys Lys Ile Ser Asn Ile Arg Glu Leu Leu Pro Val Leu Glu Gly Val
225                 230                 235                 240

Ala Lys Ala Ser Arg Pro Leu Leu Ile Val Ala Glu Asp Val Glu Gly

                245                 250                 255

Glu Ala Leu Ala Thr Leu Val Val Asn Asn Met Arg Gly Ile Val Lys

260                 265                 270

Val Ala Ala Val Lys Ala Pro Gly Phe Gly Asp Arg Arg Lys Ala Met
        275                 280                 285

Leu Gln Asp Ile Ala Ile Leu Thr Gly Gly Val Val Ile Ser Glu Glu
    290                 295                 300

Ile Gly Leu Glu Leu Glu Lys Ala Thr Leu Glu Asp Leu Gly Gln Ala
305                 310                 315                 320

Lys Arg Val Ser Ile Thr Lys Glu Asn Ser Thr Ile Ile Asp Gly Ala
                325                 330                 335

Gly Asp Gln Ala Ala Ile Gln Gly Arg Val Ala Gln Ile Arg Gln Gln
            340                 345                 350

Ile Glu Glu Ala Thr Ser Asp Tyr Asp Lys Glu Lys Leu Gln Glu Arg
        355                 360                 365

Val Ala Lys Leu Ala Gly Gly Val Ala Val Ile Lys Val Gly Ala Ala
    370                 375                 380

Thr Glu Val Glu Met Lys Glu Lys Lys Asp Arg Val Glu Asp Ala Leu
385                 390                 395                 400

His Ala Thr Arg Ala Ala Val Glu Glu Gly Val Val Ala Gly Gly Gly
                405                 410                 415

Val Ala Leu Ile Arg Ala Ala Ser Lys Leu Ser Ser Leu Val Gly Asp
            420                 425                 430

Asn Glu Glu Gln Asn Val Gly Ile Arg Val Ala Leu Arg Ala Met Glu
        435                 440                 445

Ala Pro Leu Arg Gln Ile Val Lys Asn Ala Gly Asp Glu Glu Ser Val
    450                 455                 460

Val Ala Asn Asn Val Arg Ala Gly Glu Gly Asn Tyr Gly Tyr Asn Ala
465                 470                 475                 480

Ala Thr Gly Val Tyr Gly Asp Met Ile Glu Met Gly Ile Leu Asp Pro
                485                 490                 495

Thr Lys Val Thr Arg Ser Ala Leu Gln Phe Ala Ala Ser Val Ala Gly
            500                 505                 510

Leu Met Ile Thr Thr Glu Ala Met Ile Thr Glu Leu Pro Lys Lys Asp
        515                 520                 525

Ala Pro Ala Met Pro Asp Met Gly Met Gly Gly Met Gly Gly Met Met
    530                 535                 540

<210> 57
<211> 130
<212> PRT
<213> Vibrio cholerae

<220>
<221> PEPTIDE
<222> (1)..(130)
<223> >sp|Q9KUF0|RS9_VIBCH 30S ribosomal protein S9 -
      Vibrio cholerae. (130 aa)

<400> 57
Met Ala Glu Asn Gln Tyr Tyr Gly Thr Gly Arg Arg Lys Ser Ser Ala
1               5                   10                  15

Ala Arg Val Phe Ile Lys Pro Gly Ser Gly Asn Ile Val Ile Asn Lys
            20                  25                  30

Arg Ser Leu Glu Glu Tyr Phe Gly Arg Pro Thr Ser Cys Met Val Val
        35                  40                  45

Lys Gln Pro Leu Glu Leu Val Asp Met Val Glu Lys Leu Asp Leu Tyr
    50                  55                  60

Ile Thr Val Lys Gly Gly Gly Ile Ser Gly Gln Ala Gly Ala Ile Arg
65                  70                  75                  80

His Gly Ile Thr Arg Ala Leu Met Glu Tyr Asp Glu Ser Leu Arg Pro
                85                  90                  95

Val Leu Arg Ala Ala Gly Tyr Val Thr Arg Asp Ala Arg Arg Val Glu
            100                 105                 110

Arg Lys Lys Val Gly Leu Arg Lys Ala Arg Arg Arg Pro Gln Phe Ser
        115                 120                 125

Lys Arg
    130


<210> 58
<211> 787
<212> PRT
<213> Vibrio cholerae

<220>
<221> PEPTIDE
<222> (1)..(787)
<223> >tr|Q9KQY1|Q9KQY1_VIBCH Formate acetyltransferase
      - Vibrio cholerae. (787 aa)

<400> 58
Met Lys Ser Val Leu Leu Pro Ser Gly Ile Ala Gly Ser Thr Ala Lys
1               5                   10                  15

Thr Val Arg Val Phe Ser Thr His Lys Gly Arg Tyr Val Met Ala Glu
            20                  25                  30

Gln Phe Ala Lys Ala Trp Glu Gly Phe Ala Ala Gly Asp Trp Gln Asn
        35                  40                  45

Glu Val Asn Val Arg Asp Phe Ile Gln Lys Asn Tyr Thr Pro Tyr Glu
    50                  55                  60

Gly Asp Glu Ser Phe Leu Val Ser Glu Gly Thr Glu Ala Thr Asn Lys

65                  70                  75                  80

Leu Trp Ala Lys Val Met Glu Gly Ile Arg Gln Glu Asn Ser Thr His
                85                  90                  95

Ala Pro Val Asp Phe Asp Thr Ser Leu Ile Ser Thr Ile Thr Ala His
            100                 105                 110

Asp Ala Gly Tyr Ile Asn Lys Asp Leu Glu Lys Ile Val Gly Leu Gln
        115                 120                 125

Thr Asp Ala Pro Leu Lys Arg Ala Ile Ile Pro Asn Gly Gly Ile Arg
    130                 135                 140

Met Val Glu Gly Ser Cys Lys Ala Tyr Gly Arg Glu Leu Asp Pro Gln
145                 150                 155                 160

Val Ser Lys Ile Tyr Ser Glu Tyr Arg Lys Thr His Asn Ala Gly Val
                165                 170                 175

Phe Asp Ile Tyr Thr Pro Glu Ile Leu Ala Cys Arg Lys Ser Gly Val
            180                 185                 190

Leu Thr Gly Leu Pro Asp Ala Tyr Gly Arg Gly Arg Ile Ile Gly Asp
        195                 200                 205

Tyr Arg Arg Val Ala Leu Tyr Gly Ile Asp Phe Leu Met Lys Asp Lys
    210                 215                 220

Leu Ala Gln Phe Lys Ser Leu Gln Glu Lys Phe Glu Asn Gly Glu Asp
225                 230                 235                 240

Leu Gln Met Thr Met Gln Leu Arg Glu Glu Ile Ala Glu Gln His Arg
                245                 250                 255

Ala Leu Gly Gln Met Lys Gln Met Ala Ala Lys Tyr Gly Tyr Asp Ile
            260                 265                 270

Ser Arg Pro Ala Glu Thr Ala Gln Glu Ala Ile Gln Trp Thr Tyr Phe
        275                 280                 285

Gly Tyr Leu Ala Ala Val Lys Ser Gln Asn Gly Ala Ala Met Ser Leu
    290                 295                 300

Gly Arg Thr Ser Thr Phe Leu Asp Val Tyr Ile Glu Arg Asp Met Lys
305                 310                 315                 320

Ala Gly Lys Ile Thr Glu Val Glu Ala Gln Glu Met Ile Asp His Phe
                325                 330                 335

Val Met Lys Leu Arg Met Val Arg Phe Leu Arg Thr Pro Glu Tyr Asp
            340                 345                 350

Glu Leu Phe Ser Gly Asp Pro Ile Trp Ala Thr Glu Ser Met Gly Gly
        355                 360                 365

Met Gly Leu Asp Gly Arg Thr Leu Val Thr Arg Thr Asn Phe Arg Phe
    370                 375                 380

Leu Asn Ser Leu Tyr Thr Met Gly Pro Ser Pro Glu Pro Asn Ile Thr
385                 390                 395                 400

Val Leu Trp Ser Glu Gln Leu Pro Glu Gly Phe Lys Lys Phe Cys Ala
                405                 410                 415

Lys Val Ser Ile Asp Thr Ser Ser Ile Gln Tyr Glu Asn Asp Asp Leu
            420                 425                 430

Met Arg Pro Asp Phe Asn Asn Asp Asp Tyr Ala Ile Ala Cys Cys Val
        435                 440                 445

Ser Pro Met Val Ile Gly Lys His Met Gln Phe Phe Gly Ala Arg Ala
    450                 455                 460

Asn Leu Ala Lys Thr Leu Leu Tyr Val Ile Asn Gly Gly Val Asp Glu
465                 470                 475                 480

Lys Leu Lys Ile Gln Val Gly Pro Lys Met Pro Lys Ile Thr Asp Glu
                485                 490                 495

Val Leu Asp Phe Asp Asp Val Trp Gly Lys Leu Asp His Phe Met Gly
            500                 505                 510

Trp Leu Ala Thr Gln Tyr Val Thr Ala Leu Asn Ala Ile His Tyr Met
        515                 520                 525

His Asp Lys Tyr Ser Tyr Glu Ala Ala Leu Met Ala Leu His Asp Arg
    530                 535                 540

Asp Val Arg Arg Thr Met Ala Cys Gly Ile Ala Gly Leu Ser Val Ala
545                 550                 555                 560

Ala Asp Ser Leu Ser Ala Ile Lys Tyr Ala Lys Val Lys Pro Ile Arg
                565                 570                 575

Asp Glu Asp Gly Val Ala Ile Asp Phe Glu Ile Glu Gly Asp Tyr Pro
            580                 585                 590

Lys Phe Gly Asn Asn Asp Ser Arg Val Asp Asp Ile Ala Cys Glu Leu
        595                 600                 605

Val Glu Arg Phe Met Asn Lys Ile Arg Ser Leu Lys Thr Tyr Arg Asn
    610                 615                 620

Ala Val Pro Thr Gln Ser Ile Leu Thr Ile Thr Ser Asn Val Val Tyr
625                 630                 635                 640

Gly Lys Lys Thr Gly Asn Thr Pro Asp Gly Arg Arg Ala Gly Ala Pro
                645                 650                 655

Phe Ala Pro Gly Ala Asn Pro Met His Gly Arg Asp Glu Lys Gly Ala
            660                 665                 670

Val Ala Ser Leu Thr Ser Val Gly Lys Leu Pro Phe Ala His Ala Lys
        675                 680                 685

Asp Gly Ile Ser Tyr Thr Phe Ser Ile Val Pro Asn Ala Leu Gly Lys
    690                 695                 700

Asp Glu Asn Ser Gln Arg Ala Asn Leu Ala Gly Leu Met Asp Gly Tyr
705                 710                 715                 720

Phe His His Glu Ala Gly Ile Glu Gly Gly Gln His Leu Asn Val Asn
                725                 730                 735

Val Leu Asn Arg Asp Thr Leu Leu Asp Ala Val Lys His Pro Glu Lys
            740                 745                 750

Tyr Pro Gln Leu Thr Ile Arg Val Ser Gly Tyr Ala Val Arg Phe Asn
        755                 760                 765

Ser Leu Thr Ala Glu Gln Gln Gln Asp Val Ile Ala Arg Thr Phe Thr
    770                 775                 780

Glu Ser Leu
785

<210> 59
<211> 320
<212> PRT
<213> Vibrio cholerae

<220>
<221> PEPTIDE
<222> (1)..(320)
<223> >sp|Q9KNP2|K6PF_VIBCH 6-phosphofructokinase (EC
      2.7.1.11) (Phosphofructokinase)
      (Phosphohexokinase) - Vibrio cholerae. (320 aa)

<400> 59
Met Ile Lys Lys Ile Gly Val Leu Thr Ser Gly Gly Asp Ala Pro Gly
1               5                   10                  15

Met Asn Ala Ala Ile Arg Gly Val Val Arg Thr Ala Leu Gly Ala Gly
            20                  25                  30

Leu Glu Val Phe Gly Ile Tyr Asp Gly Tyr Gln Gly Leu Tyr Glu Asp
        35                  40                  45

Arg Ile Lys Gln Leu Asp Arg Ser Ser Val Ser Asp Val Ile Asn Arg
    50                  55                  60

Gly Gly Thr Phe Leu Gly Ser Ala Arg Phe Pro Gln Phe Arg Glu Val
65                  70                  75                  80

Glu Val Arg Glu Lys Ala Ile Glu Asn Leu Lys Lys His Gly Ile Glu
                85                  90                  95

Ala Leu Val Val Ile Gly Gly Asp Gly Ser Tyr Met Gly Ala Lys Lys
            100                 105                 110

Leu Thr Glu Met Gly Phe Pro Cys Ile Gly Leu Pro Gly Thr Ile Asp
        115                 120                 125

Asn Asp Ile Ala Gly Thr Asp Tyr Thr Ile Gly Tyr Leu Thr Ala Leu
    130                 135                 140

Asn Thr Val Ile Glu Ala Ile Asp Arg Leu Arg Asp Thr Ser Ser Ser
145                 150                 155                 160

His Gln Arg Ile Ser Ile Val Glu Ile Met Gly Arg His Cys Gly Asp

165                 170                 175

Leu Thr Leu Thr Ser Ala Ile Ala Gly Gly Cys Glu Tyr Ile Ile Thr
            180                 185                 190

Pro Glu Thr Gly Leu Asn Met Glu Gln Leu Ile Ser Asn Ile Lys Asp
        195                 200                 205

Gly Ile Ala Lys Gly Lys Lys His Ala Ile Ile Ala Leu Thr Glu Leu
    210                 215                 220

Met Met Asp Ala Asn Lys Leu Ala Lys Glu Ile Glu Ser Ala Thr Gly
225                 230                 235                 240

Arg Glu Thr Arg Ala Thr Val Leu Gly His Ile Gln Arg Gly Gly Lys
                245                 250                 255

Pro Thr Ala Phe Asp Arg Val Leu Ala Ser Arg Met Gly Asn Tyr Ala
            260                 265                 270

Val His Leu Leu Met Glu Gly His Gly Gly Arg Cys Val Gly Ile Gln
        275                 280                 285

Lys Glu Gln Leu Val His His Asp Ile Ile Asp Ala Ile Glu Asn Met
    290                 295                 300

Lys Arg Pro Val Arg Thr Asp Leu Tyr Lys Val Ala Glu Glu Leu Phe
305                 310                 315                 320

<210> 60
<211> 130
<212> PRT
<213> Vibrio cholerae

<220>
<221> PEPTIDE
<222> (1)..(130)
<223> >sp|Q9KNZ8|RS8_VIBCH 30S ribosomal protein S8 -
      Vibrio cholerae. (130 aa)

<400> 60
Met Ser Met Gln Asp Pro Ile Ser Asp Met Leu Thr Arg Ile Arg Asn
1               5                   10                  15

Gly Gln Ala Ala Asn Lys Val Ala Val Lys Met Pro Ser Ser Lys Leu
            20                  25                  30

Lys Val Ala Ile Ala Ala Leu Leu Lys Ala Glu Gly Tyr Ile Ala Asp
        35                  40                  45

Phe Ala Val Glu Gly Glu Val Lys Ala Glu Leu Glu Ile Thr Leu Lys
    50                  55                  60

Tyr Phe Gln Ala Lys Pro Val Ile Glu Gln Ile Lys Arg Val Ser Arg
65                  70                  75                  80

Pro Gly Leu Arg Val Tyr Lys Lys Lys Asp Glu Leu Pro Ser Val Met

85                  90                  95

Gly Gly Leu Gly Val Ala Val Val Ser Thr Ser Lys Gly Leu Met Ser
            100                 105                 110

Asp Arg Ala Ala Arg Lys Ala Gly Leu Gly Gly Glu Ile Ile Cys Tyr
        115                 120                 125

Val Ala
    130


<210> 61
<211> 257
<212> PRT
<213> Vibrio cholerae

<220>
<221> PEPTIDE
<222> (1)..(257)
<223> >sp|Q9KNR1|TPIS_VIBCH Triosephosphate isomerase
      (EC 5.3.1.1) (TIM) (Triose-phosphate isomerase) -
      Vibrio cholerae. (257 aa)

<400> 61
Met Arg Arg Pro Val Val Met Gly Asn Trp Lys Leu Asn Gly Ser Lys
1               5                   10                  15

Ala Met Val Thr Asp Leu Leu Asn Gly Leu Asn Ala Glu Leu Glu Gly
            20                  25                  30

Val Glu Gly Val Asp Val Val Val Ala Pro Pro Ala Met Tyr Leu Asp
        35                  40                  45

Leu Ala Glu Arg Leu Ile Lys Glu Gly Gly Asn Lys Leu Ile Leu Gly
    50                  55                  60

Ala Gln Asn Thr Asp Thr His Asn Ser Gly Ala Tyr Thr Gly Asp Met
65                  70                  75                  80

Ser Pro Ala Met Leu Lys Asp Phe Gly Ala Ser His Ile Ile Ile Gly
                85                  90                  95

His Ser Glu Arg Arg Asp Tyr His Lys Glu Ser Asp Glu Phe Val Ala
            100                 105                 110

Lys Lys Phe Ala Phe Leu Lys Glu Asn Gly Leu Thr Pro Val Phe Cys
        115                 120                 125

Ile Gly Glu Thr Glu Ala Gln Asn Glu Ala Gly Glu Thr Glu Ala Val
    130                 135                 140

Cys Ala Arg Gln Ile Asn Ala Val Ile Asp Ala Tyr Gly Val Glu Ala
145                 150                 155                 160

Leu Asn Gly Ala Ile Ile Ala Tyr Glu Pro Ile Trp Ala Ile Gly Thr
                165                 170                 175

Gly Lys Ala Ala Thr Ala Asp Asp Ala Gln Arg Ile His Ala Ser Ile
            180                 185                 190

Arg Ala Leu Ile Ala Ala Lys Asp Ala Ala Val Ala Glu Gln Val Ile
        195                 200                 205

Ile Gln Tyr Gly Gly Ser Val Lys Pro Glu Asn Ala Ala Ser Tyr Phe
    210                 215                 220

Ala Gln Pro Asp Ile Asp Gly Ala Leu Val Gly Gly Ala Ser Leu Asp
225                 230                 235                 240

Ala Lys Gly Phe Ala Ala Ile Ala Lys Ala Ala Ala Glu Ala Lys Lys
                245                 250                 255

Ala

<210> 62
<211> 176
<212> PRT
<213> Vibrio cholerae

<220>
<221> PEPTIDE
<222> (1)..(176)
<223> >sp|Q9KP34|IPYR_VIBCH Inorganic pyrophosphatase
      (EC 3.6.1.1) (Pyrophosphate phospho- hydrolase)
      (PPase) - Vibrio cholerae. (176 aa)

<400> 62
Met Ser Leu Asn Asn Val Pro Ala Gly Lys Ser Leu Pro Asp Asp Ile
1               5                   10                  15

Tyr Val Val Ile Glu Ile Pro Ala Asn Ala Asp Pro Ile Lys Tyr Glu
            20                  25                  30

Val Asp Lys Glu Ser Gly Ala Val Phe Val Asp Arg Phe Met Ser Ala
        35                  40                  45

Pro Met Phe Tyr Pro Cys Asn Tyr Gly Tyr Val Asn His Thr Leu Ser
    50                  55                  60

Leu Asp Gly Asp Pro Val Asp Val Leu Val Pro Thr Pro Tyr Pro Leu
65                  70                  75                  80

Ile Pro Gly Ser Val Ile Arg Cys Arg Pro Val Gly Val Leu Lys Met
                85                  90                  95

Thr Asp Glu Ser Gly Glu Asp Ala Lys Val Val Ala Val Pro His Thr
            100                 105                 110

Lys Ile Ser Lys Glu Tyr Asp His Ile Gln Asp Val Asn Asp Leu Pro
        115                 120                 125

Ala Leu Leu Lys Ala Gln Ile Thr His Phe Phe Glu Arg Tyr Lys Glu
    130                 135                 140

Leu Glu Ser Gly Lys Trp Val Lys Val Asp Gly Trp Glu Asp Ala Ala
145                 150                 155                 160

Ser Ala Arg Ala Glu Ile Leu Ser Ser Tyr Glu Arg Ala Gln Asn Lys
                165                 170                 175

<210> 63
<211> 116
<212> PRT
<213> Vibrio cholerae


<220>
<221> PEPTIDE
<222> (1)..(116)
<223> >sp|Q9KUF7|RL19_VIBCH 50S ribosomal protein L19 -
      Vibrio cholerae. (116 aa)

<400> 63
Ser Asn Ile Ile Lys Ala Leu Glu Gln Glu Gln Met Lys Gln Asp Leu
1               5                   10                  15

Pro Gln Phe Ala Pro Gly Asp Thr Val Val Val Gln Val Lys Val Lys
            20                  25                  30

Glu Gly Asp Arg Glu Arg Leu Gln Ala Phe Glu Gly Ile Val Ile Ala
        35                  40                  45

Ile Arg Asn Arg Gly Leu His Ser Ala Phe Thr Val Arg Lys Ile Ser
    50                  55                  60

Asn Gly Glu Gly Val Glu Arg Thr Phe Gln Thr His Ser Pro Val Val
65                  70                  75                  80

Asp Ser Ile Glu Val Lys Arg Arg Gly Ala Val Arg Arg Ala Lys Leu
                85                  90                  95

Tyr Tyr Leu Arg Asp Leu Ser Gly Lys Ala Ala Arg Ile Lys Glu Lys
            100                 105                 110

Leu Ala Lys Lys
        115


<210> 64
<211> 394
<212> PRT
<213> Vibrio cholerae

<220>
<221> PEPTIDE
<222> (1)..(394)
<223> >sp|Q9KV37|EFTU1_VIBCH Elongation factor Tu-A
      (EF-Tu-A) - Vibrio cholerae. (394 aa)

<400> 64
Met Ser Lys Glu Lys Phe Glu Arg Thr Lys Pro His Val Asn Val Gly
1               5                   10                  15

Thr Ile Gly His Val Asp His Gly Lys Thr Thr Leu Thr Ala Ala Ile
            20                  25                  30

Cys Thr Val Leu Ala Lys Val Tyr Gly Gly Lys Ala Arg Asp Phe Ala
            35                  40                  45

Ser Ile Asp Asn Ala Pro Glu Glu Arg Glu Arg Gly Ile Thr Ile Asn
    50                  55                  60

Thr Ser His Val Glu Tyr Asp Thr Pro Asn Arg His Tyr Ala His Val
65                  70                  75                  80

Asp Cys Pro Gly His Ala Asp Tyr Val Lys Asn Met Ile Thr Gly Ala
                85                  90                  95

Ala Gln Met Asp Gly Gly Ile Leu Val Val Ala Ala Thr Asp Gly Arg
            100                 105                 110

Met Pro Gln Thr Arg Glu His Ile Leu Leu Gly Arg Gln Val Gly Ile
        115                 120                 125

Pro Tyr Ile Ile Val Phe Met Asn Lys Cys Asp Met Val Asp Asp Glu
    130                 135                 140

Glu Leu Leu Glu Leu Val Glu Met Glu Val Arg Glu Leu Leu Ser Glu
145                 150                 155                 160

Tyr Asp Phe Pro Gly Asp Asp Leu Pro Val Ile Gln Gly Ser Ala Leu
                165                 170                 175

Gly Ala Leu Asn Gly Glu Ala Gln Trp Glu Ala Lys Ile Val Glu Leu
            180                 185                 190

Ala Glu Ala Leu Asp Thr Tyr Ile Pro Glu Pro Glu Arg Ala Val Asp
        195                 200                 205

Met Ala Phe Leu Met Pro Ile Glu Asp Val Phe Ser Ile Gln Gly Arg
    210                 215                 220

Gly Thr Val Val Thr Gly Arg Ile Glu Arg Gly Ile Leu Lys Val Gly
225                 230                 235                 240

Asp Glu Val Ala Ile Val Gly Ile Lys Glu Thr Val Lys Thr Thr Cys
                245                 250                 255

Thr Gly Val Glu Met Phe Arg Lys Leu Leu Asp Glu Gly Arg Ala Gly
            260                 265                 270

Glu Asn Val Gly Ala Leu Leu Arg Gly Thr Lys Arg Glu Glu Val Glu
        275                 280                 285

Arg Gly Gln Val Leu Ala Lys Pro Gly Ser Ile Thr Pro His Thr Lys
    290                 295                 300

Phe Glu Ser Glu Val Tyr Val Leu Ser Lys Asp Glu Gly Gly Arg His
305                 310                 315                 320

Thr Pro Phe Phe Lys Gly Tyr Arg Pro Gln Phe Tyr Phe Arg Thr Thr
                325                 330                 335

Asp Val Thr Gly Ser Ile Glu Leu Pro Glu Gly Val Glu Met Val Met
            340                 345                 350

Pro Gly Asp Asn Val Lys Met Val Val Asp Leu Ile Ala Pro Ile Ala

355                 360                 365

Met Asp Glu Gly Leu Arg Phe Ala Ile Arg Glu Gly Gly Arg Thr Val
    370                 375                 380

Gly Ala Gly Val Val Ala Lys Ile Ile Ala
385                 390

<210> 65
<211> 389
<212> PRT
<213> Vibrio cholerae

<220>
<221> PEPTIDE
<222> (1)..(389)
<223> >tr|Q9KUV8|Q9KUV8_VIBCH MSHA biogenesis protein
      MshN - Vibrio cholerae. (389 aa)

<400> 65
Met Gly Met Glu Gln Thr Met Ser Val Val Asn Glu Ala Leu Ala Lys
1               5                   10                  15

Ser Ala Gln Arg Ser His Ser Arg Leu Ser Asn Ile Glu Arg Ile Asp
            20                  25                  30

Val Ala Lys Pro Lys Ala Arg Pro Ala Trp Leu Trp Val Met Leu Gly
        35                  40                  45

Phe Gly Val Ser Leu Ala Val Gly Gly Trp Ser Ile Ser Leu Gln Ser
    50                  55                  60

Val Asp Thr Ile Pro Ser Ser Ala Glu Val Val Arg Pro Glu Val Pro
65                  70                  75                  80

Ser Pro Thr Gln Lys Thr Thr Gln Gln Ser Ile Ala Leu Tyr Gln Ala
                85                  90                  95

Pro Val Asn Ser Glu Ala Leu Val Lys Asn Glu Thr Leu Val Gln Lys
            100                 105                 110

Glu Thr Leu Pro Lys Asn Glu Thr Ser Val Lys Asn Ala Val Leu Ala
        115                 120                 125

Lys Val Glu Gly Ser Pro Thr Ser Leu Lys Gln Thr Ala Asn Arg Gln
    130                 135                 140

Arg Glu Gln Thr Ala Leu Gly Ser Glu Leu Asn Ala Glu Pro Val Leu
145                 150                 155                 160

Asn Leu Val Asp Asn Ser Glu Pro Ser Phe Phe Glu Glu Glu Val Ser
                165                 170                 175

Ser Leu Pro Ser Ser Ser Pro Val Met Ile Val Glu Gln Val Ser Leu
            180                 185                 190

Thr Pro Glu Gln Leu Ala Gln Lys Ala Leu Gln Arg Ala Gln Lys Ala
        195                 200                 205

Met Glu Ser Asn Glu Leu Gln Thr Ala Val Ser Ala Tyr Thr Glu Ala

210                 215                 220

Leu Arg Tyr Thr Pro His Asp Glu Met Ala Arg Gln Lys Leu Ala Ala
225                 230                 235                 240

Leu Tyr Tyr Gly Lys Gly Asp Gly Arg Lys Ala Phe Asp Leu Met Gln
                245                 250                 255

Ala Gly Ile Glu Arg Asn Pro Asp Gly Glu Val Leu Arg Leu Ala Leu
            260                 265                 270

Ala Lys Leu Leu Val Lys Glu Lys Gln Glu Ala Ser Ala Leu Val Pro
        275                 280                 285

Leu Ala Tyr Leu Pro Ser Gln Pro Ser Ile Glu Tyr Leu Ser Leu Arg
    290                 295                 300

Ala Ala Leu Ala Gln Lys Thr Lys Gln Asp Glu Ile Ala Arg Glu Ser
305                 310                 315                 320

Tyr Gln Gln Leu Thr Glu Lys Asp Pro Asn Asn Gly Arg Trp Trp Leu
                325                 330                 335

Gly Leu Ala Ile Gln Gln Glu Arg Ala Leu Gln Trp Pro Ala Ala Gln
            340                 345                 350

His Ala Tyr Gln Gln Ala Leu Asn Lys Val Gly Leu Ser Ser Gln Ser
        355                 360                 365

Gln Ala Phe Ile His Gln Arg Leu Gln Leu Leu Ala Ser Leu Glu Glu
    370                 375                 380

Thr Gln Ser Ala Asn
385


<210> 66
<211> 642
<212> PRT
<213> Vibrio cholerae

<220>
<221> PEPTIDE
<222> (1)..(642)
<223> >sp|Q9KMN7|SYT_VIBCH Threonyl-tRNA synthetase (EC
      6.1.1.3) (Threonine--tRNA ligase) (ThrRS) - Vibrio
      cholerae. (642 aa)

<400> 66
Met Pro Ile Ile Thr Leu Pro Asp Gly Ser Gln Arg His Phe Asp Asn
1               5                   10                  15

Pro Val Ser Thr Leu Glu Val Ala Gln Ser Ile Gly Pro Gly Leu Ala
            20                  25                  30

Lys Ala Thr Ile Ala Gly Arg Val Asn Gly Ala Arg Val Asp Ala Cys
        35                  40                  45

Asp Leu Ile Glu His Asp Ala Ser Leu Glu Ile Ile Thr Thr Lys Asp
    50                  55                  60

Glu Val Asp Gly Leu Glu Ile Val Arg His Ser Cys Ala His Leu Leu
65                  70                  75                  80

Gly His Ala Leu Lys Gln Leu Tyr Pro Asn Ala Lys Met Ala Ile Gly
                85                  90                  95

Pro Thr Ile Asp Ser Gly Phe Tyr Tyr Asp Ile Asp Leu Glu Gln Ser
            100                 105                 110

Leu Ser Gln Glu Asp Leu Glu Lys Ile Glu Ala Arg Met Val Glu Leu
        115                 120                 125

Ala Lys Thr Lys Tyr Ala Val Val Lys Lys Lys Val Ser Trp Gln Glu
    130                 135                 140

Ala Arg Asp Thr Phe Glu Ser Arg Gly Glu Ser Tyr Lys Met Glu Ile
145                 150                 155                 160

Leu Asp Glu Asn Val Ala Arg Asp Asp Arg Pro Gly Leu Tyr His His
                165                 170                 175

Glu Glu Tyr Ile Asp Met Cys Arg Gly Pro His Val Pro His Met Gly
            180                 185                 190

Phe Cys Gln Asn Phe Lys Leu Leu Asn Ile Ala Gly Ala Tyr Trp Arg
        195                 200                 205

Gly Asn Ser Asp Asn Lys Met Leu Gln Arg Ile Tyr Gly Thr Ala Phe
    210                 215                 220

His Asp Lys Lys Ala Leu Gln Ala His Leu Thr Arg Leu Glu Glu Ala
225                 230                 235                 240

Ala Lys Arg Asp His Arg Lys Ile Gly Lys Gln Leu Asp Leu Phe His
                245                 250                 255

Met Gln Gln Glu Ala Pro Gly Met Val Phe Trp His His Asn Gly Trp
            260                 265                 270

Ser Ile Phe Arg Asp Leu Glu Ile Phe Ile Arg Gln Lys Leu Asn Glu
        275                 280                 285

Tyr Gly Tyr Gln Glu Val Lys Gly Pro Leu Met Met Asp Arg Val Leu
    290                 295                 300

Trp Glu Arg Ser Gly His Trp Asp Lys Tyr Ala Asp Ala Met Phe Thr
305                 310                 315                 320

Thr Ser Ser Glu Asn Arg Glu Tyr Ala Ile Lys Pro Met Asn Cys Pro
                325                 330                 335

Gly His Ile Gln Ile Phe Asn Gln Gly Leu Lys Ser Tyr Arg Asp Leu
            340                 345                 350

Pro Leu Arg Met Ala Glu Phe Gly Ser Cys His Arg Asn Glu Pro Ser
        355                 360                 365

Gly Ser Leu His Gly Ile Met Arg Val Arg Gly Phe Thr Gln Asp Asp
    370                 375                 380

Ala His Ile Phe Cys Thr Glu Asp Gln Ile Gln Gln Glu Val Thr Ser

385                 390                 395                 400

Cys Ile Lys Met Val Tyr Asp Thr Tyr Thr Thr Phe Gly Phe Gln Asn
                405                 410                 415

Ile Val Val Lys Leu Ser Thr Arg Pro Glu Lys Arg Val Gly Ser Asp
            420                 425                 430

Glu Ile Trp Asp Lys Ser Glu Gln Ala Leu Ile Asp Ser Leu Lys Ala
        435                 440                 445

Met Asp Ile Pro Phe Glu Ile Gln Glu Gly Glu Gly Ala Phe Tyr Gly
    450                 455                 460

Pro Lys Ile Glu Phe Thr Leu Tyr Asp Cys Leu Asp Arg Ala Trp Gln
465                 470                 475                 480

Cys Gly Thr Val Gln Leu Asp Phe Asn Leu Pro Thr Arg Leu Gly Ala
                485                 490                 495

Thr Tyr Val Gly Glu Ser Asn Glu Arg Leu Ile Pro Val Met Ile His
            500                 505                 510

Arg Ala Ile Leu Gly Ser Leu Glu Arg Phe Ile Gly Ile Leu Ile Glu
        515                 520                 525

Glu Tyr Ala Gly Phe Phe Pro Thr Trp Leu Ala Pro Glu Gln Ala Val
    530                 535                 540

Val Val Asn Ile Thr Asp Lys Gln Ala Asp Tyr Ala His Glu Val Ala
545                 550                 555                 560

Gln Lys Leu Gln Lys Cys Gly Ile Arg Ala Lys Ala Asp Leu Arg Asn
                565                 570                 575

Glu Lys Ile Gly Phe Lys Ile Arg Glu His Thr Leu Lys Arg Val Pro
            580                 585                 590

Tyr Met Leu Val Cys Gly Asp Gln Glu Met Glu Ala Gly Glu Ile Ala
        595                 600                 605

Val Arg Thr Arg Lys Gly Lys Asp Leu Gly Lys Phe Lys Leu Asp Asp
    610                 615                 620

Phe Ile Ala His Ile Gln Ala Glu Ile Ala Ser Arg Lys Leu Asn Leu
625                 630                 635                 640

Glu Glu

<210> 67
<211> 645
<212> PRT
<213> Vibrio cholerae

<220>
<221> PEPTIDE
<222> (1)..(645)
<223> >sp|Q9KVS8|THIC_VIBCH Thiamine biosynthesis
      protein thiC - Vibrio cholerae. (645 aa)

<400> 67
Met Ser Asn Arg Lys Gln Ala Arg Leu Glu Ala Lys Arg Phe Ile Asp
1               5                   10                  15

Thr Leu Ser Val Glu Pro Tyr Pro Asn Ser Gln Lys Ser Tyr Leu Leu
            20                  25                  30

Gly Ser Arg Pro Asp Ile Arg Val Pro Val Arg Glu Ile Thr Leu Ser
        35                  40                  45

Asp Thr Leu Val Gly Gly Ser Lys Asp Ala Pro Ile Phe Glu Pro Asn
    50                  55                  60

Glu Pro Ile Cys Val Tyr Asp Thr Ser Gly Val Tyr Thr Asp Pro Ser
65                  70                  75                  80

His Asp Ile Asp Leu Tyr Lys Gly Leu Pro Lys Leu Arg Glu Glu Trp
                85                  90                  95

Ile Glu Glu Arg Arg Asp Thr His Ile Leu Pro Ser Met Ser Ser His
            100                 105                 110

Phe Ala Arg Glu Arg Leu Ala Asp Glu Thr Leu Asp Glu Leu Arg Tyr
        115                 120                 125

Gly His Leu Pro Arg Ile Arg Arg Ala Met Gly Gln His Arg Val Thr
    130                 135                 140

Gln Leu His Tyr Ala Arg Gln Gly Ile Ile Thr Pro Glu Met Glu Phe
145                 150                 155                 160

Val Ala Ile Arg Glu Asn Ser Arg Arg Leu Ala His Gln Asp Pro Ser
                165                 170                 175

Leu Leu Gln Gln His Ala Gly Gln Asn Phe Gly Ala His Leu Pro Asp
            180                 185                 190

Leu Ile Thr Pro Glu Phe Val Arg Arg Glu Ile Ala Glu Gly Arg Ala
        195                 200                 205

Ile Ile Pro Cys Asn Ile Asn His Pro Glu Ser Glu Pro Met Ile Ile
    210                 215                 220

Gly Arg Asn Phe Leu Val Lys Val Asn Ala Asn Ile Gly Asn Ser Ser
225                 230                 235                 240

Val Ser Ser Ser Ile Glu Glu Glu Val Glu Lys Leu Val Trp Ala Thr
                245                 250                 255

Arg Trp Gly Ala Asp Thr Val Met Asp Leu Ser Thr Gly Arg Asn Ile
            260                 265                 270

His Glu Thr Arg Glu Trp Ile Leu Arg Asn Ser Pro Val Pro Ile Gly
        275                 280                 285

Thr Val Pro Met Tyr Gln Ala Leu Glu Lys Val Asn Gly Val Ala Glu
    290                 295                 300

Asn Leu Thr Trp Glu Val Met Arg Asp Thr Leu Leu Glu Gln Ala Glu

305                 310                 315                 320

Gln Gly Val Asp Tyr Phe Thr Ile His Ala Gly Leu Leu Leu Arg Tyr
                325                 330                 335

Val Pro Met Thr Ala Lys Arg Val Thr Gly Ile Val Ser Arg Gly Gly
            340                 345                 350

Ser Ile Ile Ala Lys Trp Cys Leu Ser His His Gln Glu Asn Phe Leu
        355                 360                 365

Tyr Thr His Phe Arg Glu Ile Cys Glu Ile Cys Ala Gln Tyr Asp Val
    370                 375                 380

Ala Leu Ser Leu Gly Asp Gly Leu Arg Pro Gly Ser Ile Ala Asp Ala
385                 390                 395                 400

Asn Asp Glu Ala Gln Phe Ala Glu Leu Arg Thr Leu Gly Glu Leu Thr
                405                 410                 415

Gln Ile Ala Trp Glu Tyr Asp Val Gln Val Met Ile Glu Gly Pro Gly
            420                 425                 430

His Val Pro Met His Leu Ile Lys Ala Asn Met Asp Glu Gln Leu Lys
        435                 440                 445

His Cys His Glu Ala Pro Phe Tyr Thr Leu Gly Pro Leu Thr Thr Asp
    450                 455                 460

Ile Ala Pro Gly Tyr Asp His Ile Thr Ser Gly Ile Gly Ala Ala Met
465                 470                 475                 480

Ile Gly Trp Phe Gly Cys Ala Met Leu Cys Tyr Val Thr Pro Lys Glu
                485                 490                 495

His Leu Gly Leu Pro Asn Lys Glu Asp Val Lys Thr Gly Leu Ile Thr
            500                 505                 510

Tyr Lys Leu Ala Ala His Ala Ala Asp Leu Ala Lys Gly His Pro Gly
        515                 520                 525

Ala Gln Ile Arg Asp Asn Ala Leu Ser Lys Ala Arg Phe Glu Phe Arg
    530                 535                 540

Trp Glu Asp Gln Phe Asn Leu Ala Leu Asp Pro Val Thr Ala Arg Ala
545                 550                 555                 560

Phe His Asp Glu Thr Leu Pro Gln Glu Ser Gly Lys Val Ala His Phe
                565                 570                 575

Cys Ser Met Cys Gly Pro Lys Phe Cys Ser Met Lys Ile Ser Gln Glu
            580                 585                 590

Val Arg Asp Tyr Ala Asn Asn Gln Thr Leu Asp Thr Thr Val Ile Asp
        595                 600                 605

Leu Val Met Pro Ala Glu Ser Ile Gln Leu Ala Met Gln Asp Lys Ser
    610                 615                 620

Arg Glu Phe Leu Ala Ser Gly Ala Glu Leu Tyr His Pro Leu Val Lys

625                 630                 635                 640

Glu Pro Ile Glu Glu
                645


<210> 68
<211> 311
<212> PRT
<213> Vibrio cholerae

<220>
<221> PEPTIDE
<222> (1)..(311)
<223> >tr|Q9KQQ6|Q9KQQ6_VIBCH Methylenetetrahydrofolate
      dehydrogenase/methenyltetrahydrofolate
      cyclohydrolase - Vibrio cholerae. (311 aa)

<400> 68
Met Pro Val Asp Val Ile Lys Asn Ile Cys Asp Asn Val Arg Lys Asn
1               5                   10                  15

Phe Arg Ser Lys Pro Val Arg Lys Val Met Thr Ala Gln Asn Ile Asp
            20                  25                  30

Gly Thr Leu Ile Ser Gln Thr Val Arg Ser Glu Val Ala Ala Arg Val
        35                  40                  45

Lys Ala Arg Val Gln Ala Gly Leu Arg Ala Pro Gly Leu Ala Val Val
    50                  55                  60

Leu Val Gly Glu Asp Pro Ala Ser Gln Val Tyr Val Gly Ser Lys Arg
65                  70                  75                  80

Arg Ala Cys Glu Glu Val Gly Phe Val Ser Lys Ser Phe Asp Leu Pro
                85                  90                  95

Ala Thr Ala Ser Glu Glu Ala Leu Leu Ser Leu Val Glu Glu Leu Asn
            100                 105                 110

Asn Asp Pro Gln Ile Asp Gly Ile Leu Val Gln Leu Pro Leu Pro Ala
        115                 120                 125

Gly Met Asp Thr Thr Lys Val Leu Glu Ser Ile His Pro Glu Lys Asp
    130                 135                 140

Val Asp Gly Phe His Pro Tyr Asn Val Gly Arg Leu Ala Gln Arg Ile
145                 150                 155                 160

Pro Lys Leu Arg Ser Cys Thr Pro Lys Gly Ile Ile Thr Leu Leu Glu
                165                 170                 175

Arg Tyr Asn Ile Pro Leu Arg Gly Lys His Ala Val Ile Val Gly Ala
            180                 185                 190

Ser Asn Ile Val Gly Arg Pro Met Thr Leu Glu Leu Leu Leu Ala Gly
        195                 200                 205

Cys Thr Thr Thr Thr Cys His Arg Phe Thr Gln Asp Leu Glu Gly His
    210                 215                 220

Ile Arg Gln Ala Asp Ile Leu Val Val Ala Val Gly Lys Pro Asn Phe
225                 230                 235                 240

Ile Pro Gly Ala Trp Ile Lys Glu Gly Ala Val Val Val Asp Val Gly
                245                 250                 255

Ile Asn Arg Leu Asp Thr Gly Lys Leu Val Gly Asp Val Glu Tyr Asp
            260                 265                 270

Val Ala Arg Thr Arg Ala Ser Phe Ile Thr Pro Val Pro Gly Gly Val
        275                 280                 285

Gly Pro Met Thr Val Ala Ser Leu Ile Glu Asn Thr Met Met Ala Cys
    290                 295                 300

Glu Gln Phe His Thr Gln Gly
305                 310


<210> 69
<211> 446
<212> PRT
<213> Vibrio cholerae

<220>
<221> PEPTIDE
<222> (1)..(446)
<223> >sp|Q9KPS1|NQRA_VIBCH Na(+)-translocating
      NADH-quinone reductase subunit A (EC 1.6.5.-)
      (Na(+)-translocating NQR subunit A) (Na(+)-NQR
      subunit A) (NQR complex subunit A) (NQR-1 subunit
      A) - Vibrio cho

<400> 69

<210> 70
<211> 635

<212> PRT
<213> Vibrio cholerae

<220>
<221> PEPTIDE
<222> (1)..(635)
<223> >sp|O34241|DNAK_VIBCH Chaperone protein dnaK (Heat
      shock protein 70) (Heat shock 70 kDa protein)
      (HSP70) - Vibrio cholerae. (635 aa)

<400> 70
Met Gly Lys Ile Ile Gly Ile Asp Leu Gly Thr Thr Asn Ser Cys Val
1               5                   10                  15

Ala Val Leu Asp Gly Asp Lys Pro Arg Val Ile Glu Asn Ala Glu Gly
            20                  25                  30

Glu Arg Thr Thr Pro Ser Val Ile Ala Tyr Thr Asp Gly Glu Thr Leu
        35                  40                  45

Val Gly Gln Pro Ala Lys Arg Gln Ala Val Thr Asn Pro Gln Asn Thr
    50                  55                  60

Leu Phe Ala Ile Lys Arg Leu Ile Gly Arg Arg Phe Glu Asp Glu Glu
65                  70                  75                  80

Val Gln Arg Asp Ile Lys Ile Met Pro Tyr Lys Ile Val Lys Ala Asp
                85                  90                  95

Asn Gly Asp Ala Trp Val Glu Ala Lys Gly Gln Lys Met Ala Ala Pro
            100                 105                 110

Gln Val Ser Ala Glu Val Leu Lys Lys Met Lys Lys Thr Ala Glu Asp
        115                 120                 125

Phe Leu Gly Glu Pro Val Thr Ala Ala Val Ile Thr Val Pro Ala Tyr
    130                 135                 140

Phe Asn Asp Ala Gln Arg Gln Ala Thr Lys Asp Ala Gly Arg Ile Ala
145                 150                 155                 160

Gly Leu Glu Val Lys Arg Ile Ile Asn Glu Pro Thr Ala Ala Ala Leu
                165                 170                 175

Ala Tyr Gly Leu Asp Lys Gln Gly Gly Asp Arg Thr Ile Ala Val Tyr
            180                 185                 190

Asp Leu Gly Gly Gly Thr Phe Asp Ile Ser Ile Ile Glu Ile Asp Glu
        195                 200                 205

Val Glu Gly Glu Lys Thr Phe Glu Val Leu Ser Thr Asn Gly Asp Thr
    210                 215                 220

His Leu Gly Gly Glu Asp Phe Asp Asn Arg Met Ile Asn Tyr Leu Val
225                 230                 235                 240

Asp Glu Phe Lys Lys Asp Gln Gly Ile Asp Leu Arg Asn Asp Pro Leu
                245                 250                 255

Ala Met Gln Arg Leu Lys Glu Ala Ala Glu Lys Ala Lys Ile Glu Leu
            260                 265                 270

Ser Ser Ala Gln Gln Thr Asp Val Asn Leu Pro Tyr Ile Thr Ala Asp
        275                 280                 285

Ala Thr Gly Pro Lys His Met Asn Ile Lys Val Thr Arg Ala Lys Leu
    290                 295                 300

Glu Ala Leu Val Glu Asp Leu Val Gln Arg Ser Leu Glu Pro Leu Lys
305                 310                 315                 320

Val Ala Leu Ala Asp Ala Asp Leu Ser Val Asn Asp Ile Thr Asp Val
                325                 330                 335

Ile Leu Val Gly Gly Gln Thr Arg Met Pro Met Val Gln Lys Lys Val
            340                 345                 350

Ala Glu Phe Phe Gly Lys Glu Pro Arg Lys Asp Val Asn Pro Asp Glu
        355                 360                 365

Ala Val Ala Val Gly Ala Ala Val Gln Gly Gly Val Leu Ala Gly Glu
    370                 375                 380

Val Lys Asp Val Leu Leu Leu Asp Val Thr Pro Leu Ser Leu Gly Ile
385                 390                 395                 400

Glu Thr Met Gly Gly Val Met Thr Lys Leu Ile Glu Lys Asn Thr Thr
                405                 410                 415

Ile Pro Thr Lys Ala Asn Gln Val Phe Ser Thr Ala Glu Asp Asn Gln
            420                 425                 430

Ser Ala Val Thr Ile His Val Leu Gln Gly Glu Arg Lys Gln Ala Met
        435                 440                 445

Tyr Asn Lys Ser Leu Gly Gln Phe Asn Leu Glu Gly Ile Asn Pro Ala
    450                 455                 460

Pro Arg Gly Met Pro Gln Ile Glu Val Ile Phe Asp Leu Asp Ala Asp
465                 470                 475                 480

Gly Ile Leu His Val Ser Ala Lys Asp Lys Gln Thr Gly Lys Glu Gln
                485                 490                 495

Lys Ile Thr Ile Gln Ala Ser Gly Gly Leu Ser Asp Ala Glu Ile Glu
            500                 505                 510

Lys Met Val Gln Glu Ala Glu Ala Asn Lys Glu Ala Asp Lys Lys Phe
        515                 520                 525

Glu Glu Leu Ala Thr Ala Arg Asn Gln Ala Asp Gln Met Ile His Ala
    530                 535                 540

Thr Arg Lys Gln Ile Thr Glu Ala Gly Glu Ala Leu Pro Ala Asp Glu
545                 550                 555                 560

Lys Ala Lys Ile Glu Thr Ala Ile Asn Glu Leu Glu Thr Ala Lys Lys
                565                 570                 575

Gly Glu Asp Lys Ala Glu Ile Asp Ala Lys Val Gln Ala Leu Met Ala
            580                 585                 590

Ala Ala Gln Lys Leu Met Glu Ile Ala Gln Gln Gln Ala Gln Ala Gln
        595                 600                 605

Gly Ala Asn Ala Gly Gln Ser Ser Ala Lys Glu Asp Asp Val Val Asp
    610                 615                 620

Ala Glu Phe Glu Glu Val Asn Asp Asp Lys Lys
625                 630                 635

<210> 71
<211> 117
<212> PRT
<213> Vibrio cholerae

<220>
<221> PEPTIDE
<222> (1)..(117)
<223> >sp|Q9KP00|RL18_VIBCH 50S ribosomal protein L18 -
Vibrio cholerae. (117 aa)

<400> 71
Met Asp Lys Lys Ala Ser Arg Ile Arg Arg Ala Thr Arg Ala Arg Arg
1               5                   10                  15

Lys Ile Ala Glu Leu Gly Ala Thr Arg Leu Val Val His Arg Thr Pro
            20                  25                  30

Arg His Val Tyr Ala Gln Val Ile Ala Ala Asn Gly Ser Glu Val Ile
        35                  40                  45

Ala Ala Ala Ser Thr Val Glu Lys Ala Ile Arg Glu Gln Val Lys Tyr
    50                  55                  60

Thr Gly Asn Ile Asp Ala Ala Lys Ala Val Gly Lys Ala Val Ala Glu
65                  70                  75                  80

Arg Ala Leu Glu Lys Gly Val Thr Val Val Ala Phe Asp Arg Ser Gly
                85                  90                  95

Phe Gln Tyr His Gly Arg Val Ala Ala Leu Ala Asp Ser Ala Arg Glu
            100                 105                 110

Ala Gly Leu Lys Phe
        115

<210> 72
<211> 345
<212> PRT
<213> Vibrio cholerae

<220>
<221> PEPTIDE
<222> (1)..(345)
<223> >tr|Q9KMH9|Q9KMH9_VIBCH Hypothetical protein
VCA0374 - Vibrio cholerae. (345 aa)

<400> 72
Met Ile Leu Arg Gln Leu Asn Arg Ile Asp Met Ser Ser Phe Asp Phe
1               5                   10                  15

Pro Lys Tyr Gln Ala Asn Gln Glu Val Gly Asn Thr Gly Glu Ala Phe
            20                  25                  30

Phe Glu His Phe Val Thr Lys Glu Leu Gly Cys Ile Tyr Arg Lys Val
        35                  40                  45

His Arg Glu His Asp Phe Gly Ile Asp Gly Tyr Ile Asp Ile Val Asn
    50                  55                  60

Asn Gly Val Val Ser Gly Arg Ser Val Ala Val Gln Ile Lys Cys Gly
65                  70                  75                  80

Asp Ser Tyr Ile Ser Lys Val Thr Ser Gly Gly Ile Lys Tyr Leu Gly
                85                  90                  95

Asp Asn Lys His Leu Asn Phe Leu Ile Asn His Asn Val Pro Val Ile
            100                 105                 110

Leu Val Val Leu Asn Ser Asp Cys Thr Lys Gly Tyr Trp Val Glu Phe
        115                 120                 125

Ser Val Asn Ala Thr Asn Pro Ser Ser Lys Ser Trp Trp Ile Glu Ile
    130                 135                 140

Pro Phe Glu Asn Gln Leu Gly Thr Leu Asp Met Thr Ser Leu Cys Asp
145                 150                 155                 160

Ile Ala Gly Ile Ala Glu Asp Tyr Ser Glu Asp Ile Lys Val Ser Trp
                165                 170                 175

Ala Ile Asn Ser Ala Met Asp Glu Gln Ser Asp Ile Ser Phe Val Leu
            180                 185                 190

Ser Lys Glu Glu Val Glu Glu Leu Asp Leu Ser Gly Ile Asn Gln Met
        195                 200                 205

Met Glu Arg Tyr Phe Arg Thr Lys Asp Leu Leu Ile Ser Asn Arg Asn
    210                 215                 220

Lys Thr Val Phe Trp Ile Phe Gly Tyr Asp Asp Asp Ser Arg Glu Leu
225                 230                 235                 240

Tyr Glu Ile Pro Glu Val Arg Arg Trp Phe Lys Phe Thr Leu Glu Gln
                245                 250                 255

Gly Val Pro Trp Phe Tyr Leu Leu Asp Val Gly Ala Asp His Met Ser
            260                 265                 270

Leu Pro Ile Asn Met Tyr Ser Cys Cys Asn Ile Ser Val Asn Lys Leu
        275                 280                 285

Ser Cys Gly Ser Lys Ser Val Val Ile Ser Ser Val Ser Asp Ile Asn
    290                 295                 300

Asn Trp Val Glu Val Asn Phe Glu Asn Leu Asn Arg Phe Ala Asp Glu
305                 310                 315                 320

Lys Lys Ile Pro Asp Asn Ile Leu Lys Glu Val Ser Glu Lys Val Ile
                325                 330                 335

Thr Ser Val His Arg Leu Val Ala Gly
            340                 345

<210> 73
<211> 331
<212> PRT
<213> Vibrio cholerae

<220>
<221> PEPTIDE
<222> (1)..(331)
<223> >tr|Q9KQJ8|Q9KQJ8_VIBCH Glyceraldehyde 3-phosphate
      dehydrogenase - Vibrio cholerae. (331 aa)

<400> 73
Met Thr Ile Lys Val Gly Ile Asn Gly Phe Gly Arg Ile Gly Arg Phe
1               5                   10                  15

Val Phe Arg Ala Ala Gln Glu Arg Asn Asp Ile Glu Ile Val Gly Ile
            20                  25                  30

Asn Asp Leu Ile Asp Val Asp Tyr Met Ala Tyr Met Leu Lys Tyr Asp
        35                  40                  45

Ser Thr His Gly Arg Phe Asn Gly Thr Val Glu Val Lys Asp Gly Asn
    50                  55                  60

Leu Ile Val Asn Gly Lys Thr Val Arg Val Thr Ala Glu Arg Asn Pro
65                  70                  75                  80

Ala Asp Leu Lys Trp Gly Glu Ile Gly Val Asp Val Val Ala Glu Ala
                85                  90                  95

Thr Gly Leu Phe Leu Thr Asp Glu Thr Ala Arg Lys His Ile Glu Ala
            100                 105                 110

Gly Ala Lys Lys Val Val Leu Thr Gly Pro Ser Lys Asp Lys Thr Pro
        115                 120                 125

Met Phe Val Met Gly Val Asn His Lys Thr Tyr Ala Gly Gln Asp Ile
    130                 135                 140

Val Ser Asn Ala Ser Cys Thr Thr Asn Cys Leu Ala Pro Ile Ala Lys
145                 150                 155                 160

Val Leu Asn Asp Asn Phe Gly Ile Glu Ser Gly Leu Met Thr Thr Val
                165                 170                 175

His Ala Thr Thr Ala Thr Gln Lys Thr Val Asp Gly Pro Ser Ala Lys
            180                 185                 190

Asp Trp Arg Gly Gly Arg Gly Ala Ser Gln Asn Ile Ile Pro Ser Ser
        195                 200                 205

Thr Gly Ala Ala Lys Ala Val Gly Val Val Leu Pro Glu Leu Asn Gly
    210                 215                 220

Lys Leu Thr Gly Met Ala Phe Arg Val Pro Thr Ala Asn Val Ser Val
225                 230                 235                 240

Val Asp Leu Thr Val Asn Leu Gln Lys Ala Ala Ser Tyr Asp Asp Ile
                245                 250                 255

Lys Ala Ala Met Lys Ala Ala Ser Glu Gly Glu Leu Ala Gly Ile Leu
            260                 265                 270

Gly Tyr Thr Glu Asp Ala Val Val Ser Thr Asp Phe Asn Gly Asp Thr
        275                 280                 285

Arg Thr Ser Ile Phe Asp Ala Asp Ala Gly Ile Ala Leu Thr Asp Lys
    290                 295                 300

Phe Val Lys Val Val Ser Trp Tyr Asp Asn Glu Ile Gly Tyr Ser Asn
305                 310                 315                 320

Lys Val Leu Asp Leu Ile Ala His Ile Ser Lys
                325                 330


<210> 74
<211> 513
<212> PRT
<213> Vibrio cholerae

<220>
<221> PEPTIDE
<222> (1)..(513)
<223> >sp|Q9KNH3|ATPA_VIBCH ATP synthase alpha chain (EC
      3.6.3.14) - Vibrio cholerae. (513 aa)

<400> 74
Met Gln Leu Asn Ser Thr Glu Ile Ser Asp Leu Ile Lys Gln Arg Ile
  1               5                  10                  15

Glu Ser Phe Asn Val Val Ser Glu Ala Arg Asn Glu Gly Thr Ile Val
             20                  25                  30

Ser Val Ser Asp Gly Ile Ile Arg Ile His Gly Leu Ala Asp Val Met
         35                  40                  45

Gln Gly Glu Met Ile Glu Leu Pro Gly Asn Arg Tyr Ala Leu Ala Leu
     50                  55                  60

Asn Leu Glu Arg Asp Ser Val Gly Ala Val Val Met Gly Pro Tyr Ala
 65                  70                  75                  80

Asp Leu Arg Glu Gly Met Lys Val Thr Gly Thr Gly Arg Ile Leu Glu
                 85                  90                  95

Val Pro Val Gly Pro Glu Leu Leu Gly Arg Val Val Asn Thr Leu Gly
            100                 105                 110

Glu Pro Ile Asp Gly Lys Gly Pro Ile Gly Ala Lys Gln Thr Ser Pro
        115                 120                 125

Val Glu Val Ile Ala Pro Gly Val Ile Asp Arg Lys Ser Val Asp Gln
    130                 135                 140

Pro Val Gln Thr Gly Tyr Lys Ser Val Asp Ser Met Ile Pro Ile Gly
145                 150                 155                 160

Arg Gly Gln Arg Glu Leu Ile Ile Gly Asp Arg Gln Thr Gly Lys Thr
                165                 170                 175

Ala Leu Ala Ile Asp Ala Ile Ile Asn Gln Lys Asn Ser Gly Ile Tyr
            180                 185                 190

Ser Ile Tyr Val Ala Ile Gly Gln Lys Ala Ser Thr Ile Ala Asn Val
        195                 200                 205

Val Arg Lys Leu Glu Glu His Gly Ala Leu Lys Asn Thr Ile Val Val
    210                 215                 220

Val Ala Ser Ala Ser Glu Ser Ala Ala Leu Gln Tyr Leu Ala Pro Tyr
225                 230                 235                 240

Ser Gly Cys Ala Met Gly Glu Tyr Phe Arg Asp Arg Gly Glu Asp Ala
                245                 250                 255

Leu Ile Val Tyr Asp Asp Leu Ser Lys Gln Ala Val Ala Tyr Arg Gln
            260                 265                 270

Ile Ser Leu Leu Leu Arg Arg Pro Pro Gly Arg Glu Ala Phe Pro Gly
        275                 280                 285

Asp Val Phe Tyr Leu His Ser Arg Leu Leu Glu Arg Ala Ala Arg Val
    290                 295                 300

Asn Glu Glu Tyr Val Glu Arg Phe Thr Lys Gly Glu Val Lys Gly Lys
305                 310                 315                 320

Thr Gly Ser Leu Thr Ala Leu Pro Ile Ile Glu Thr Gln Ala Gly Asp
                325                 330                 335

Val Ser Ala Phe Val Pro Thr Asn Val Ile Ser Ile Thr Asp Gly Gln
            340                 345                 350

Ile Phe Leu Gln Thr Glu Leu Phe Asn Ala Gly Val Arg Pro Ala Val
        355                 360                 365

Asp Pro Gly Ile Ser Val Ser Arg Val Gly Gly Ala Ala Gln Thr Lys
    370                 375                 380

Ile Val Lys Lys Leu Ser Gly Gly Ile Arg Thr Ala Leu Ala Ala Tyr
385                 390                 395                 400

Arg Glu Leu Ala Ala Phe Ala Gln Phe Ser Ser Asp Leu Asp Glu Ala
                405                 410                 415

Thr Lys Arg Gln Leu Thr His Gly Gln Lys Val Thr Glu Leu Met Lys
            420                 425                 430

Gln Lys Gln Tyr Ala Pro Met Ser Val Phe Asp Gln Ala Leu Val Ile
        435                 440                 445

Phe Ala Ala Glu Arg Gly Tyr Leu Thr Asp Val Glu Leu Asn Lys Val
    450                 455                 460

Leu Asp Phe Glu Ala Ala Leu Leu Ser Tyr Ala Arg Ala His Tyr Ala
465                 470                 475                 480

Glu Leu Ala Ala Gln Ile Asp Lys Thr Gly Ala Tyr Asn Asp Glu Ile

485                 490                 495

Glu Ala Gln Leu Lys Lys Leu Val Asp Asp Phe Lys Ala Thr Gln Thr
            500                 505                 510

Trp

<210> 75
<211> 128
<212> PRT
<213> Vibrio cholerae

<220>
<221> PEPTIDE
<222> (1)..(128)
<223> >tr|Q9KP09|Q9KP09_VIBCH Ribosomal protein L17 -
      Vibrio cholerae. (128 aa)

<400> 75
Met Arg His Arg Lys Ser Gly Arg Gln Leu Asn Arg Asn Ser Ser His
1               5                   10                  15

Arg Lys Ala Met Phe Ser Asn Met Ala Ser Ser Leu Val Arg His Glu
            20                  25                  30

Val Ile Lys Thr Thr Leu Pro Lys Ala Lys Glu Leu Arg Arg Val Val
        35                  40                  45

Glu Pro Leu Ile Thr Leu Ala Lys Thr Asp Ser Val Ala Asn Arg Arg
    50                  55                  60

Leu Ala Phe Ala Arg Thr Arg Asp Asn Glu Val Val Ala Lys Leu Phe
65                  70                  75                  80

Asn Glu Leu Gly Pro Arg Phe Ala Ala Arg Gln Gly Gly Tyr Thr Arg
                85                  90                  95

Ile Leu Lys Cys Gly Phe Arg Ala Gly Asp Lys Ala Pro Met Ala Tyr
            100                 105                 110

Ile Glu Leu Val Asp Arg Pro Glu Ala Ala Val Glu Ala Ala Ala Glu
        115                 120                 125

<210> 76
<211> 233
<212> PRT
<213> Vibrio cholerae

<220>
<221> PEPTIDE
<222> (1)..(233)
<223> >sp|Q9KNZ0|RS3_VIBCH 30S ribosomal protein S3 -
      Vibrio cholerae. (233 aa)

<400> 76

Met Gly Gln Lys Val His Pro Asn Gly Ile Arg Leu Gly Ile Val Lys
1               5                   10                  15

Pro Trp Asn Ala Thr Trp Phe Ala Asn Thr Lys Asp Phe Ala Asp Asn
            20                  25                  30

Leu Asp Gly Asp Phe Lys Val Arg Gln Tyr Leu Ser Lys Glu Leu Ala
        35                  40                  45

Asn Ala Ser Leu Ser Arg Ile Val Ile Glu Arg Pro Ala Lys Ser Ile
    50                  55                  60

Arg Val Thr Ile His Thr Ala Arg Pro Gly Val Val Ile Gly Lys Lys
65                  70                  75                  80

Gly Glu Asp Val Glu Lys Leu Arg Ala Ala Val Ala Lys Ile Ala Gly
                85                  90                  95

Val Pro Ala Gln Ile Asn Ile Ala Glu Val Arg Lys Pro Glu Leu Asp
            100                 105                 110

Gly Gln Leu Val Ala Asp Ser Ile Ala Ser Gln Leu Glu Arg Arg Val
        115                 120                 125

Met Phe Arg Arg Ala Met Lys Arg Ala Val Gln Asn Ala Met Arg Leu
    130                 135                 140

Gly Ala Lys Gly Ile Lys Val Glu Val Ser Gly Arg Leu Gly Gly Ala
145                 150                 155                 160

Glu Ile Ala Arg Ser Glu Trp Tyr Arg Glu Gly Arg Val Pro Leu His
                165                 170                 175

Thr Leu Arg Ala Asp Ile Asp Tyr Ala Thr Ser Ser Ala His Thr Thr
            180                 185                 190

Tyr Gly Val Ile Gly Val Lys Val Trp Ile Phe Lys Gly Glu Ile Leu
        195                 200                 205

Gly Gly Met Pro Ala Ala Thr Glu Ala Ala Glu Pro Lys Ala Asp Lys
    210                 215                 220

Pro Lys Lys Gln Arg Lys Gly Arg Lys
225                 230


<210> 77
<211> 194
<212> PRT
<213> Vibrio cholerae

<220>
<221> PEPTIDE
<222> (1)..(94)

<400> 77
Met Ala Phe Glu Leu Pro Ala Leu Pro Tyr Ala Lys Asp Ala Leu Glu
1               5                   10                  15

Pro His Ile Ser Ala Glu Thr Leu Asp Phe His His Gly Lys His His
            20                  25                  30

Asn Thr Tyr Val Val Lys Leu Asn Gly Leu Ile Pro Gly Thr Glu Phe
        35                  40                  45

Glu Asn Lys Ser Leu Glu Glu Ile Ile Lys Thr Ser Thr Gly Gly Ile
    50                  55                  60

Phe Asn Asn Ala Ala Gln Val Trp Asn His Thr Phe Tyr Trp His Cys
65                  70                  75                  80

Leu Ser Pro Asn Gly Gly Gly Glu Pro Thr Gly Ala Val Ala Glu Ala
                85                  90                  95

Ile Asn Ala Ala Phe Gly Ser Phe Ala Asp Phe Lys Ala Lys Phe Thr
            100                 105                 110

Asp Ser Ala Ile Asn Asn Phe Gly Ser Ser Trp Thr Trp Leu Val Lys
        115                 120                 125

Lys Ala Asp Gly Thr Leu Ala Ile Thr Asn Thr Ser Asn Ala Ala Thr
    130                 135                 140

Pro Leu Thr Glu Glu Gly Val Thr Pro Leu Leu Thr Val Asp Leu Trp
145                 150                 155                 160

Glu His Ala Tyr Tyr Ile Asp Tyr Arg Asn Val Arg Pro Asp Tyr Met
                165                 170                 175

Asn Gly Phe Trp Ala Leu Val Asn Trp Asp Phe Val Ala Gln Asn Leu
            180                 185                 190

Ala Lys

<210> 78
<211> 1052
<212> PRT
<213> Vibrio cholerae

<220>
<221> PEPTIDE
<222> (1)..(1052)
<223> >tr|Q9KQG8|Q9KQG8_VIBCH Ribonuclease E - Vibrio
      cholerae. (1052 aa)

<400> 78
Met Lys Arg Met Leu Ile Asn Ala Thr Gln Lys Glu Glu Leu Arg Val
1               5                   10                  15

Ala Leu Val Asp Gly Gln Arg Leu Phe Asp Leu Asp Ile Glu Ser Pro
            20                  25                  30

Gly His Glu Ser Lys Lys Ala Asn Ile Tyr Lys Gly Arg Ile Thr Arg
        35                  40                  45

Ile Glu Pro Ser Leu Glu Ala Ala Phe Val Asp Tyr Gly Ala Glu Arg
    50                  55                  60

His Gly Phe Leu Pro Leu Lys Glu Ile Ala Arg Glu Tyr Phe Pro Asp

65                  70                  75                  80

Gly Tyr Ser Tyr Gln Gly Arg Pro Asn Ile Lys Asp Val Leu Asn Glu
                85                  90                  95

Gly Gln Glu Val Ile Val Gln Ile Glu Lys Glu Glu Arg Gly Ser Lys
            100                 105                 110

Gly Ala Ala Leu Thr Thr Phe Ile Ser Leu Ala Gly Ser Tyr Leu Val
        115                 120                 125

Leu Met Pro Asn Asn Pro Arg Ala Gly Gly Ile Ser Arg Arg Ile Glu
    130                 135                 140

Gly Asp Glu Arg Thr Glu Leu Lys Ser Ala Leu Ser Ser Leu Glu Leu
145                 150                 155                 160

Pro Gln Gly Met Gly Leu Ile Val Arg Thr Ala Gly Val Gly Lys Ser
                165                 170                 175

Ala Glu Glu Leu Glu Trp Asp Leu Asn Val Leu Leu Lys His Trp Ser
            180                 185                 190

Ala Ile Lys Gln Ala Ser Asp Ser Asn Pro Ala Pro Phe Leu Ile His
        195                 200                 205

Gln Glu Ser Asn Val Ile Val Arg Ala Ile Arg Asp Tyr Leu Arg Arg
    210                 215                 220

Asp Ile Gly Glu Ile Leu Ile Asp Ser Asn Ser Ile Tyr Glu Arg Ala
225                 230                 235                 240

Leu Glu His Ile Arg Leu Val Arg Pro Asp Phe Val Asn Arg Val Lys
                245                 250                 255

Lys Tyr Glu Gly Glu Val Pro Leu Phe Ser His Phe Gln Ile Glu Ser
            260                 265                 270

Gln Ile Glu Ser Ala Phe Gln Arg Glu Val Arg Leu Pro Ser Gly Gly
        275                 280                 285

Ser Ile Val Ile Asp Pro Thr Glu Ala Leu Thr Ser Ile Asp Ile Asn
    290                 295                 300

Ser Ala Arg Ala Thr Lys Gly Gly Asp Ile Glu Glu Thr Ala Leu Asn
305                 310                 315                 320

Thr Asn Leu Glu Ala Ala Asp Glu Ile Ala Arg Gln Leu Arg Leu Arg
                325                 330                 335

Asp Leu Gly Gly Leu Val Val Ile Asp Phe Ile Asp Met Thr Pro Val
            340                 345                 350

Arg His Gln Arg Glu Val Glu Asn Arg Leu Arg Glu Ala Val Arg Val
        355                 360                 365

Asp Arg Ala Arg Val Gln Ile Gly Arg Ile Ser Arg Phe Gly Leu Leu
    370                 375                 380

Glu Met Ser Arg Gln Arg Leu Ser Pro Ser Leu Ala Glu Ala Ser His
385                 390                 395                 400

His Ile Cys Pro Arg Cys Lys Gly Thr Gly Val Ile Arg Asp Asn Glu
                405                 410                 415

Ser Leu Ala Leu Ser Val Leu Arg Leu Ile Glu Glu Glu Ala Leu Lys
            420                 425                 430

Asp Asn Thr Ser Gln Val Leu Ala Val Val Pro Val Ser Ile Ala Ser
        435                 440                 445

Tyr Leu Leu Asn Glu Lys Arg Arg Ser Ile Asn His Ile Glu Lys Ala
    450                 455                 460

Gln Gln Val Arg Ile Thr Ile Val Pro Asn Ser Asp Met Glu Thr Pro
465                 470                 475                 480

His Phe Glu Val Ile Arg Val Arg Glu Gly Glu Glu Gln Asp Val Leu
                485                 490                 495

Ser Tyr Leu Ile Pro Lys Lys Leu Glu Ala Met Lys Glu Ala Glu Gly
            500                 505                 510

Lys Glu Val Val Asp Val Glu Leu Lys Pro Lys Arg Ile Glu Glu Pro
        515                 520                 525

Val Leu Lys Gly Phe Ala Ala Pro Ala Glu Ala Val Pro Ala Pro Thr
    530                 535                 540

Pro Lys Pro Lys Ala Glu Pro Gln Pro Val Ala Glu Val Gln Gln Pro
545                 550                 555                 560

Ala Gln Pro Gly Phe Phe Ser Arg Val Phe Lys Ala Ile Ala Ser Leu
                565                 570                 575

Phe Ser Ala Thr Pro Glu Ala Ala Lys Val Glu Ala Pro Val Thr Gln
            580                 585                 590

Asn Ser Glu Gln Asp Lys Pro Arg Arg Glu Arg Asn Asp Arg Asn Asn
        595                 600                 605

Asp Arg Arg Arg Asn Pro Arg Asp Lys Asn Arg Arg Arg Gly Asn Glu
    610                 615                 620

Glu Asn Asn Glu Arg Asn Asp Lys Glu Thr Ser Asn Ser Asn Arg Lys
625                 630                 635                 640

Pro Gln Glu Arg Lys Pro Lys Gln Glu Arg Gly Glu Arg Asn Glu Arg
                645                 650                 655

Asn Asp Arg Asn Glu Arg Pro Glu Thr Glu Arg Pro Glu Arg Gln Asp
            660                 665                 670

Arg Arg Asn Lys Arg Asp Glu Ser Lys Thr Ala Lys Leu Leu Glu Gln
        675                 680                 685

Gly Arg Gln Leu Ala Ala Glu Ala Gln Gln Glu Val Lys Ala Val Glu
    690                 695                 700

Pro Lys Glu Glu Lys Ala Ala Val Val Lys Glu Arg Arg Gln Arg Arg
705                 710                 715                 720

Lys Leu Ser Lys Gln Ile Arg Val Lys Asp Gln Leu Ala Ala Glu Glu
                725                 730                 735

Leu Asp Asn Leu Ser Thr Ala Pro Val Asp Asn Ala Phe Asp Ala Pro
            740                 745                 750

Ala Pro Val Asn Leu Pro Val Thr Asp Asp Phe Ala Asp Ser Ala Gln
        755                 760                 765

Glu Asp Asn Glu Gln Asp Asp Gln Lys Gln Arg Arg Asn Arg Arg Ser
    770                 775                 780

Pro Arg His Leu Arg Ala Ser Gly Gln Arg Arg Arg Arg Gly Arg Asp
785                 790                 795                 800

Arg Arg Pro Asn Pro Phe Arg Leu Arg Lys Gly Gly Val Ala Ser Pro
                805                 810                 815

Glu Met Ala Met Gly Lys Val Met Pro Arg Tyr Asp Leu Ala Pro Arg
            820                 825                 830

Pro Gln Ser Arg His Glu Thr Ala Glu Gly Ala Gln His Val Thr His
        835                 840                 845

Val Thr Pro Thr Ala Ala Thr Ala Val Val Glu Ser Glu Lys Val Ala
    850                 855                 860

Ala Pro Arg Val Leu Gly Gly Val Ala Phe Pro Glu Met Ala Met Gly
865                 870                 875                 880

Lys Val Ile Val Arg Arg Glu Thr Ala Ala Val Gln Pro Glu Pro Val
                885                 890                 895

Val Gln Glu Pro Val Ile Thr Asp Val Ile Ala Glu Thr Leu Ile Ala
            900                 905                 910

Pro Val Glu Pro Val Val Val Glu Ala Thr Val Ile Glu Ala Pro Val
        915                 920                 925

Ile Glu Glu Ser Ala Thr Glu Thr Ala Ala Leu Glu Thr Val Val Ala
    930                 935                 940

Lys Val Ala Glu Val Glu Glu Pro Ala Thr Ile Ser Asp Glu Val Val
945                 950                 955                 960

Ala Gln Thr Thr Val Glu Val Ile Ala Asp Lys Met Ala Glu Pro Ile
                965                 970                 975

Lys Val Val Lys Pro Val Thr Val Ser Ala Thr Ser Leu Thr Ala Lys
            980                 985                 990

Ala Val Ile Gln Gln Pro Tyr Ala Ser Ser Pro Met Thr Lys Ala Pro
        995                 1000                1005

Gly Ser Asp Asp Ile Arg Glu Val Gln Val Asn Ala Ala Pro Leu Arg
    1010                1015                1020

Ala Glu Arg Tyr Gln Ala Arg Gly Ala Gly Ser Gln Val Ala Arg Asn
1025                1030                1035                1040

Gln Ala Ser Ala Gly Met Ser Lys Pro Gln Ser Phe
                1045                1050

<210> 79
<211> 466
<212> PRT
<213> Vibrio cholerae

<220>
<221> PEPTIDE
<222> (1)..(466)
<223> >sp|Q9KNH5|ATPB_VIBCH ATP synthase beta chain (EC
      3.6.3.14) - Vibrio cholerae. (466 aa)

<400> 79
Ala Thr Gly Lys Ile Val Gln Ile Ile Gly Ala Val Val Asp Val Glu
1               5                   10                  15

Phe Pro Gln Ser Glu Val Pro Ser Val Tyr Asp Ala Leu Asn Val Val
            20                  25                  30

Asp Ser Lys Glu Arg Leu Val Leu Glu Val Gln Gln Gln Leu Gly Gly
        35                  40                  45

Gly Val Ile Arg Ala Ile Val Met Gly Ser Ser Asp Gly Leu Arg Arg
    50                  55                  60

Gly Met Thr Val Gln Asn Thr Gly Ala Pro Ile Ser Val Pro Val Gly
65                  70                  75                  80

Thr Lys Thr Leu Gly Arg Ile Met Asn Val Leu Gly Asp Ala Ile Asp
                85                  90                  95

Glu Arg Gly Asp Ile Gly Ala Glu Glu Val Tyr Ser Ile His Arg Pro
            100                 105                 110

Ala Pro Ser Tyr Glu Glu Gln Ser Ser Ala Thr Glu Leu Leu Glu Thr
        115                 120                 125

Gly Val Lys Val Ile Asp Leu Ile Cys Pro Phe Ala Lys Gly Gly Lys
    130                 135                 140

Ile Gly Leu Phe Gly Gly Ala Gly Val Gly Lys Thr Val Asn Met Met
145                 150                 155                 160

Glu Leu Ile Asn Asn Ile Ala Leu Gln His Ser Gly Leu Ser Val Phe
                165                 170                 175

Ala Gly Val Gly Glu Arg Thr Arg Glu Gly Asn Asp Phe Tyr His Glu
            180                 185                 190

Met Gln Glu Ala Gly Val Val Asn Val Glu Gln Pro Glu Leu Ser Lys
        195                 200                 205

Val Ala Met Val Tyr Gly Gln Met Asn Glu Pro Pro Gly Asn Arg Leu
    210                 215                 220

Arg Val Ala Leu Thr Gly Leu Thr Met Ala Glu Lys Phe Arg Asp Glu
225                 230                 235                 240

Gly Arg Asp Val Leu Leu Phe Ile Asp Asn Ile Tyr Arg Tyr Thr Leu
                245                 250                 255

Ala Gly Thr Glu Val Ser Ala Leu Leu Gly Arg Met Pro Ser Ala Val
            260                 265                 270

Gly Tyr Gln Pro Thr Leu Ala Glu Glu Met Gly Val Leu Gln Glu Arg
        275                 280                 285

Ile Thr Ser Thr Lys Lys Gly Ser Ile Thr Ser Val Gln Ala Val Tyr
    290                 295                 300

Val Pro Ala Asp Asp Leu Thr Asp Pro Ser Pro Ala Thr Thr Phe Ala
305                 310                 315                 320

His Leu Asp Ala Thr Val Val Leu Asn Arg Asn Ile Ala Ala Met Gly
                325                 330                 335

Leu Tyr Pro Ala Ile Asp Pro Leu Asp Ser Thr Ser Arg Gln Leu Asp
            340                 345                 350

Pro Leu Val Val Gly Gln Glu His Tyr Asp Val Ala Arg Gly Val Gln
        355                 360                 365

Ala Thr Leu Gln Arg Tyr Lys Glu Leu Lys Asp Ile Ile Ala Ile Leu
    370                 375                 380

Gly Met Asp Glu Leu Ser Glu Ala Asp Lys Gln Val Val Ala Arg Ala
385                 390                 395                 400

Arg Lys Ile Glu Arg Phe Leu Thr Gln Pro Tyr His Val Ala Glu Val
                405                 410                 415

Phe Thr Gly Asp Pro Gly Val Tyr Val Pro Leu Lys Glu Thr Leu Arg
            420                 425                 430

Gly Phe Lys Gly Leu Leu Ala Gly Asp Tyr Asp Asp Ile Pro Glu Gln
        435                 440                 445

Ala Phe Met Tyr Cys Gly Thr Ile Asp Asp Ala Ile Glu Asn Ala Lys
    450                 455                 460

Lys Leu
465


<210> 80
<211> 517
<212> PRT
<213> Vibrio cholerae

<220>
<221> PEPTIDE
<222> (1)..(517)
<223> >sp|Q9KTW2|GUAA_VIBCH GMP synthase
      [glutamine-hydrolyzing] (EC 6.3.5.2) (Glutamine
      amidotransferase) (GMP synthetase) - Vibrio
      cholerae. (517 aa)

<400> 80
Met Thr Lys Asn Ile His Asp Gln Arg Ile Leu Ile Leu Asp Phe Gly

1               5                   10                  15

Ser Gln Tyr Thr Gln Leu Val Ala Arg Arg Val Arg Glu Ile Gly Val
            20                  25                  30

Tyr Cys Glu Leu Trp Ser Trp Asp Val Glu Glu Ala Asp Ile Arg Glu
        35                  40                  45

Phe Asn Pro Asp Gly Ile Ile Leu Ser Gly Gly Pro Glu Ser Val Thr
    50                  55                  60

Glu Ala Asn Ser Pro Arg Ala Pro Gln Tyr Val Phe Asp Ser Gly Val
65                  70                  75                  80

Pro Val Phe Gly Val Cys Tyr Gly Met Gln Thr Met Ala Glu Gln Leu
                85                  90                  95

Gly Gly Arg Val Ala Thr Ser Asp Glu Arg Glu Phe Gly Tyr Ala Gln
            100                 105                 110

Val Lys Ile Ser Gly Glu Ser Ala Leu Phe Lys Asp Leu Asp Leu Thr
        115                 120                 125

Gln Asp Val Trp Met Ser His Gly Asp Lys Val Val Glu Ile Pro Ala
    130                 135                 140

Asp Phe Val Lys Ile Gly Glu Thr Asp Thr Cys Pro Tyr Ala Ala Met
145                 150                 155                 160

Ala Asn Glu Glu Lys Lys Tyr Tyr Gly Val Gln Phe His Pro Glu Val
                165                 170                 175

Thr His Thr Lys Asn Gly Leu Gln Met Leu Glu Asn Phe Val Leu Gly
            180                 185                 190

Val Cys Gly Cys Glu Arg Leu Trp Thr Ser Glu Ser Ile Ile Glu Asp
        195                 200                 205

Ala Val Ala Arg Ile Lys Glu Gln Val Gly Asn Asp Glu Val Ile Leu
    210                 215                 220

Gly Leu Ser Gly Gly Val Asp Ser Ser Val Val Ala Met Leu Val His
225                 230                 235                 240

Arg Ala Ile Gly Ser Lys Leu Thr Cys Val Phe Val Asp Asn Gly Leu
                245                 250                 255

Leu Arg Leu Asn Glu Gly Glu Gln Val Met Glu Met Phe Gly Asp Lys
            260                 265                 270

Phe Gly Leu Asn Ile Ile Lys Val Asp Ala Glu Glu Arg Phe Leu Lys
        275                 280                 285

Ala Leu Glu Gly Ile Asp Glu Pro Glu Ala Lys Arg Lys Thr Ile Gly
    290                 295                 300

Arg Val Phe Val Glu Val Phe Asp Glu Glu Ser Lys Lys Leu Ser Asn
305                 310                 315                 320

Ala Lys Trp Leu Ala Gln Gly Thr Ile Tyr Pro Asp Val Ile Glu Ser
                325                 330                 335

Ala Ala Ser Lys Thr Gly Lys Ala His Val Ile Lys Ser His His Asn
            340                 345                 350

Val Gly Gly Leu Pro Asp Asp Met Lys Met Gly Leu Val Glu Pro Leu
        355                 360                 365

Arg Glu Leu Phe Lys Asp Glu Val Arg Lys Ile Gly Leu Glu Leu Gly
    370                 375                 380

Leu Pro Tyr Asn Met Leu Tyr Arg His Pro Phe Pro Gly Pro Gly Leu
385                 390                 395                 400

Gly Val Arg Val Leu Gly Glu Val Lys Lys Glu Tyr Cys Asp Leu Leu
                405                 410                 415

Arg Arg Ala Asp Ala Ile Phe Ile Glu Glu Leu His Ala Ala Asp Leu
            420                 425                 430

Tyr Asn Lys Val Ser Gln Ala Phe Thr Val Phe Leu Pro Val Arg Ser
        435                 440                 445

Val Gly Val Met Gly Asp Gly Arg Lys Tyr Asp Trp Val Val Ser Leu
    450                 455                 460

Arg Ala Val Glu Thr Ile Asp Phe Met Thr Ala His Trp Ala His Leu
465                 470                 475                 480

Pro Tyr Glu Phe Leu Gly Lys Val Ser Asn Arg Ile Ile Asn Glu Val
                485                 490                 495

Asn Gly Ile Ser Arg Val Val Tyr Asp Ile Ser Gly Lys Pro Pro Ala
            500                 505                 510

Thr Ile Glu Trp Glu
        515


<210> 81
<211> 512
<212> PRT
<213> Vibrio cholerae

<220>
<221> PEPTIDE
<222> (1)..(512)
<223> >tr|Q9KM26|Q9KM26_VIBCH NAD(P) transhydrogenase,
      alpha subunit - Vibrio cholerae. (512 aa)

<400> 81
Met Gln Ile Gly Val Pro Arg Glu Gln Leu Ala Gly Glu Thr Arg Val
1               5                   10                  15

Ala Ala Ser Pro Ser Ser Val Glu Gln Leu Ile Lys Leu Gly Phe Glu
            20                  25                  30

Val Cys Ile Glu Ser Lys Ala Gly Ala Leu Ala Ser Phe Asp Asp Ala
        35                  40                  45

Ala Tyr Thr Ala Ala Gly Ala Thr Ile Gly Ser Arg Glu Glu Ile Trp
    50                  55                  60

Ala Cys Pro Leu Ile Leu Lys Val Asn Ala Pro Ser Asp Asp Glu Ile
65                  70                  75                  80

Ala Leu Leu Lys Glu Gly Ala Thr Leu Val Ser Phe Ile Trp Pro Ala
                85                  90                  95

Gln Asn Pro Ala Leu Met Glu Lys Leu Ser Ser Lys Asn Ile Asn Val
            100                 105                 110

Leu Ala Met Asp Ala Val Pro Arg Ile Ser Arg Ala Gln Ala Leu Asp
        115                 120                 125

Ala Leu Ser Ser Met Ala Asn Ile Ala Gly Tyr Arg Ala Val Val Glu
    130                 135                 140

Ala Ala His Glu Phe Gly Arg Phe Phe Thr Gly Gln Ile Thr Ala Ala
145                 150                 155                 160

Gly Lys Val Ser Pro Ala Lys Val Phe Val Ala Gly Ala Gly Val Ala
                165                 170                 175

Gly Leu Ala Ala Ile Gly Ala Ala Gly Ser Leu Gly Ala Ile Val Arg
            180                 185                 190

Ala Phe Asp Val Arg Pro Glu Val Lys Glu Gln Val Gln Ser Met Gly
        195                 200                 205

Ala Glu Phe Leu Glu Val Asn Phe Gln Glu Thr Ala Gly Ser Gly Asp
    210                 215                 220

Gly Tyr Ala Lys Glu Met Ser Asp Glu Phe Asn Arg Lys Ala Ala Glu
225                 230                 235                 240

Leu Tyr Ala Ala Gln Ala Lys Asp Val Asp Ile Ile Ile Thr Thr Ala
                245                 250                 255

Leu Ile Pro Gly Lys Pro Ala Pro Lys Leu Ile Thr Lys Glu Met Val
            260                 265                 270

Asp Ser Met Lys Ala Gly Ser Val Ile Val Asp Leu Ala Ala Ala Asn
        275                 280                 285

Gly Gly Asn Cys Glu Tyr Thr Val Lys Asp Gln Val Ile Thr Thr Asp
    290                 295                 300

Asn Gly Val Lys Val Ile Gly Tyr Thr Asp Met Val Gly Arg Leu Pro
305                 310                 315                 320

Thr Gln Ser Ser Gln Leu Tyr Ala Thr Asn Leu Val Asn Leu Leu Lys
                325                 330                 335

Leu Leu Cys Lys Glu Lys Asp Gly Asn Ile Asp Ile Asn Phe Glu Asp
            340                 345                 350

Val Val Leu Arg Gly Val Thr Val Val Lys Ala Gly Glu Ile Thr Trp
        355                 360                 365

Pro Ala Pro Pro Ile Gln Val Ser Ala Gln Pro Gln Gln Lys Ala Lys
    370                 375                 380

Val Gln Pro Thr Lys Ala Gln Lys Lys Glu Pro Glu Pro Thr Ser Pro
385                 390                 395                 400

Val Lys Lys Leu Val Gly Leu Ala Val Gly Val Gly Leu Phe Ala Trp
                405                 410                 415

Val Ala Ser Val Ala Pro Ala Ala Phe Leu Gly His Phe Thr Val Phe
            420                 425                 430

Val Leu Ala Cys Val Val Gly Tyr Tyr Val Val Trp Asn Val Thr His
        435                 440                 445

Ala Leu His Thr Pro Leu Met Ser Val Thr Asn Ala Ile Ser Gly Ile
    450                 455                 460

Ile Val Val Gly Ala Leu Leu Gln Ile Gly Gln Gly Ser Gly Ile Val
465                 470                 475                 480

Thr Phe Leu Ala Phe Ile Ala Val Leu Ile Ala Ser Ile Asn Ile Phe
                485                 490                 495

Gly Gly Phe Thr Val Thr Lys Arg Met Leu Glu Met Phe Arg Lys Asn
            500                 505                 510

<210> 82
<211> 280
<212> PRT
<213> Vibrio cholerae

<220>
<221> PEPTIDE
<222> (1)..(280)
<223> >sp|Q9KPV3|EFTS_VIBCH Elongation factor Ts (EF-Ts)
      - Vibrio cholerae. (280 aa)

<400> 82
Met Ala Val Thr Ala Ala Leu Val Lys Glu Leu Arg Glu Arg Thr Gly
1               5                   10                  15

Ala Gly Met Met Glu Cys Lys Lys Ala Leu Val Glu Thr Asn Gly Asp
            20                  25                  30

Ile Glu Leu Ala Ile Glu Asn Met Arg Lys Ser Gly Ala Ala Lys Ala
        35                  40                  45

Ala Lys Lys Ala Gly Asn Ile Ala Ala Glu Gly Thr Ile Met Ile Lys
    50                  55                  60

Glu Gly Glu Gly Ile Ala Ala Leu Val Glu Val Asn Cys Gln Thr Asp
65                  70                  75                  80

Phe Val Ala Lys Asp Ser Asn Phe Val Ala Phe Ala Asn Gln Val Thr
                85                  90                  95

Asp Ala Ala Leu Ala Ser Lys Ala Ser Val Glu Glu Leu Gln Ala Gln
            100                 105                 110

Phe Glu Glu Ala Arg Val Ala Leu Val Ala Lys Ile Gly Glu Asn Ile
        115                 120                 125

Asn Ile Arg Arg Val Gln Tyr Val Glu Gly Glu Ala Leu Ala Thr Tyr
    130                 135                 140

Arg His Gly Asp Arg Ile Gly Val Val Val Ala Gly Ser Ala Asp Val
145                 150                 155                 160

Glu Thr Leu Lys His Val Ala Met His Val Ala Ala Ser Arg Pro Glu
                165                 170                 175

Phe Leu Thr Pro Asp Asp Val Pro Ala Glu Val Val Ala Lys Glu Arg
            180                 185                 190

Glu Val Gln Val Gly Ile Ala Met Asn Glu Gly Lys Ser Lys Glu Ile
        195                 200                 205

Ala Glu Lys Met Val Glu Gly Arg Met Lys Lys Phe Thr Gly Glu Val
    210                 215                 220

Ser Leu Thr Gly Gln Pro Phe Val Met Glu Pro Lys Lys Thr Val Gly
225                 230                 235                 240

Glu Ile Leu Ala Glu Lys Gly Ala Thr Val Ser Ala Phe Ile Arg Leu
                245                 250                 255

Glu Val Gly Glu Gly Ile Glu Lys Gln Glu Gly Leu Ser Phe Ala Glu
            260                 265                 270

Glu Val Ala Leu Ala Gln Lys Gly
        275                 280


<210> 83
<211> 554

<212> PRT
<213> Vibrio cholerae

<220>
<221> PEPTIDE
<222> (1)..(554)
<223> >tr|Q9KTB2|Q9KTB2_VIBCH Asparagine synthetase B,
      glutamine-hydrolyzing - Vibrio cholerae. (554 aa)

<400> 83
Met Cys Ser Ile Phe Gly Ile Leu Asp Ile Lys Thr Asp Ala Ala Thr
1               5                   10                  15

Leu Arg Pro Ile Ala Leu Glu Met Ser Lys Lys Leu Arg His Arg Gly
            20                  25                  30

Pro Asp Trp Ser Gly Ile Tyr Ala Ser Asp Lys Ala Ile Leu Ala His
        35                  40                  45

Glu Arg Leu Ala Ile Val Gly Leu Asn Ser Gly Ala Gln Pro Leu Tyr
    50                  55                  60

Ser Pro Asp Arg Lys Leu Ile Leu Ala Val Asn Gly Glu Ile Tyr Asn
65                  70                  75                  80

His Lys Glu Ile Arg Glu Arg Tyr Lys Gly Gln Tyr Glu Phe Gln Thr
                85                  90                  95

Asp Ser Asp Cys Glu Val Ile Leu Ala Leu Tyr Arg Asp Lys Gly Ala
            100                 105                 110

Asp Leu Leu Glu Glu Leu Asn Gly Ile Phe Ala Phe Val Leu Tyr Asp
        115                 120                 125

Glu Glu Lys Asp Glu Tyr Leu Ile Gly Arg Asp His Ile Gly Ile Ile
    130                 135                 140

Pro Leu Tyr Gln Gly His Asp Glu His Gly Asn Phe Tyr Val Ala Ser
145                 150                 155                 160

Glu Met Lys Ala Leu Val Pro Val Cys Lys Thr Leu Ser Glu Phe Pro
                165                 170                 175

Pro Gly Ser Tyr Leu Ser Ser Lys Asp Gln Ala Val Thr Arg Tyr Tyr
            180                 185                 190

Val Arg Asp Trp Asn Ser Phe Asp Glu Val Lys Asp Asn Val Ser Ser
        195                 200                 205

Lys Glu Glu Leu Thr Gln Ala Leu Glu Ala Ala Val Lys Arg Gln Leu
    210                 215                 220

Met Thr Asp Val Pro Tyr Gly Val Leu Leu Ser Gly Gly Leu Asp Ser
225                 230                 235                 240

Ser Ile Thr Ser Ala Ile Ala Lys Arg Phe Ala Ala Met Arg Ile Glu
                245                 250                 255

Asp Asp Glu Lys Ser Ala Ala Trp Trp Pro Gln Leu His Ser Phe Ala
            260                 265                 270

Ile Gly Leu Glu Gly Ala Pro Asp Leu Lys Ala Ala Arg Glu Val Ala
        275                 280                 285

Glu Lys Ile Gly Thr Val His His Glu Met Thr Tyr Thr Ile Gln Glu
    290                 295                 300

Gly Leu Asp Ala Ile Arg Asp Val Ile Tyr His Ile Glu Thr Tyr Asp
305                 310                 315                 320

Val Thr Thr Ile Arg Ala Ser Thr Pro Met Phe Leu Met Gly Arg Lys
                325                 330                 335

Ile Lys Ala Met Gly Ile Lys Met Val Leu Ser Gly Glu Gly Ala Asp
            340                 345                 350

Glu Ile Phe Gly Gly Tyr Leu Tyr Phe His Lys Ala Pro Asn Ala Lys
        355                 360                 365

Glu Phe His Glu Glu Thr Val Arg Lys Leu Leu Ala Leu Asn Leu Phe
    370                 375                 380

Asp Cys Ala Arg Ala Asn Lys Ser Leu Ala Ala Trp Gly Val Glu Gly
385                 390                 395                 400

Arg Val Pro Phe Leu Asp Lys Glu Phe Ile Asp Val Ala Met Arg Leu
                405                 410                 415

Asn Pro Ala Asp Lys Met Cys Gly Asn Gly Lys Met Glu Lys His Ile
            420                 425                 430

Leu Arg Glu Cys Phe Glu His Tyr Leu Pro Glu Ser Ile Ala Trp Arg
        435                 440                 445

Gln Lys Glu Gln Phe Ser Asp Gly Val Gly Tyr Gly Trp Ile Asp Thr
    450                 455                 460

Leu Lys Ala Thr Ala Glu Ala Lys Ile Ser Asp Gln Gln Met Glu Thr
465                 470                 475                 480

Ala Lys Phe Arg Phe Pro Tyr Asn Thr Pro Thr Thr Lys Glu Gly Tyr
                485                 490                 495

Val Tyr Arg Glu Ile Phe Glu Glu Leu Phe Pro Leu Glu Ser Ala Ala
            500                 505                 510

Arg Cys Val Pro Gly Gly Pro Ser Val Ala Cys Ser Ser Ala Lys Ala
        515                 520                 525

Ile Glu Trp Asp Glu Ser Phe Lys Asn Cys Ile Asp Pro Ser Gly Arg
    530                 535                 540

Ala Val Lys Ala Val His Lys Gln Ala Tyr
545                 550


<210> 84
<211> 64
<212> PRT
<213> Vibrio cholerae

<220>
<221> PEPTIDE
<222> (1)..(64)
<223> >sp|O68845|RL35_VIBCH 50S ribosomal protein L35 -
      Vibrio cholerae. (64 aa)

<400> 84
Met Pro Lys Met Lys Asn Asn Lys Gly Ala Ala Lys Arg Phe Lys Lys
1               5                   10                  15

Thr Ala Gly Gly Ile Lys Tyr Lys His Ala Thr Lys Arg His Ile Leu
            20                  25                  30

Thr Lys Arg Thr Thr Lys Asn Lys Arg Gln Leu Arg Pro Asn Ala Ile
        35                  40                  45

Leu Pro Lys Cys Glu Leu Ala Ala Val Ala Arg Met Leu Pro Tyr Ala
    50                  55                  60


<210> 85
<211> 681

<212> PRT
<213> Vibrio cholerae

<220>
<221> PEPTIDE
<222> (1)..(681)
<223> >tr|Q9KP17|Q9KP17_VIBCH 2`,3`-cyclic-nucleotide
      2`-phosphodiesterase - Vibrio cholerae. (681 aa)

<400> 85
Met Lys Pro Leu Phe His Arg Leu Leu Leu His Leu Ser Ser Cys Phe
1               5                   10                  15

Tyr Phe Leu Leu Leu Ser Leu Ile Leu Glu Ser Val Met Thr Met Met
            20                  25                  30

Ile Lys Pro Val Ser Leu Ala Val Leu Gly Gly Leu Leu Ala Met Ala
        35                  40                  45

Gly Pro Val Met Ala Asp Thr Ile Lys Leu Arg Ile Val Glu Thr Thr
    50                  55                  60

Asp Ile His Thr Asn Leu Met Asp Tyr Asp Tyr Tyr Lys Asp Lys Ala
65                  70                  75                  80

Ser Asp Gln Ile Gly Leu Thr Arg Ala Ala Thr Leu Val Lys Gln Ala
                85                  90                  95

Arg Ala Glu Val Glu Asn Ser Val Leu Val Asp Asn Gly Asp Leu Ile
            100                 105                 110

Gln Gly Ser Pro Met Gly Asp Tyr Met Ala Ala Lys Gly Ile Lys Ala
        115                 120                 125

Gly Glu Val His Pro Val Tyr Lys Ala Met Asn Pro Leu Gly Tyr Asp
    130                 135                 140

Val Gly Asn Ile Gly Asn His Glu Phe Asn Tyr Gly Leu Glu Phe Leu
145                 150                 155                 160

Lys Glu Thr Ile Asn Asp Ala Ala Phe Pro Tyr Ile Asn Ala Asn Val
                165                 170                 175

Phe Asp Ala Lys Thr Gly Gln His Leu Phe Thr Pro Tyr Val Ile Lys
            180                 185                 190

Glu His Ser Phe Lys Asp Thr Asp Gly Glu Ala His Thr Ile Lys Val
        195                 200                 205

Gly Tyr Ile Gly Phe Val Pro Pro Gln Ile Met Val Trp Asp Lys Lys
    210                 215                 220

Asn Leu Glu Gly Lys Val Thr Ala Ala Asp Ile Lys Gln Thr Ala Glu
225                 230                 235                 240

Lys Leu Ile Pro Glu Met Lys Ala Lys Gly Ala Asp Val Ile Val Ala
                245                 250                 255

Ile Pro His Ser Gly Ile Ser Thr Asp Glu Tyr Gln Ala Gly Ala Glu
            260                 265                 270

Asn Ser Val Tyr Tyr Leu Thr Gln Val Lys Gly Ile Asp Ala Ile Ala
        275                 280                 285

Phe Gly His Ser His Ala Val Phe Pro Gly Lys Asp Phe Ala Asn Val
    290                 295                 300

Pro Gly Ala Asp Ile Asp Lys Gly Thr Ile Asn Gly Val Thr Ala Val
305                 310                 315                 320

Met Pro Gly Arg Trp Gly Ser His Val Gly Val Met Asp Leu Thr Leu
                325                 330                 335

Glu Lys Gln Gln Gly Arg Trp Ala Val Thr Ser Gly Gln Ser Glu Ala
            340                 345                 350

Arg Pro Ile Phe Asp Lys Ala Asn Lys Lys Ala Leu Val Glu Ala Asp
        355                 360                 365

Ala Thr Met Val Gln Ala Leu Gln His Asp His Gln Gly Thr Arg Asp
    370                 375                 380

Phe Val Asn Gln Pro Ile Gly Lys Ala Asn Asp Val Met Tyr Ser Phe
385                 390                 395                 400

Leu Ser Leu Val Gln Asp Asp Pro Thr Val Gln Ile Val Asn Leu Ala
                405                 410                 415

Gln Lys Asp Tyr Val Glu Arg Phe Ile Gln Gly Asp Pro Asn Leu Asp
            420                 425                 430

Gly Leu Pro Val Leu Ser Ala Ala Ala Pro Phe Lys Ala Gly Gly Arg
        435                 440                 445

Lys Asp Asp Pro Asn Gly Phe Thr Glu Val Glu Ala Gly Glu Leu Thr
    450                 455                 460

Phe Arg Asn Ala Ala Asp Leu Tyr Leu Tyr Pro Asn Thr Leu Val Thr
465                 470                 475                 480

Met Lys Val Ser Gly Gln Gln Leu Lys Glu Trp Leu Glu Cys Ser Ala

                485                 490                 495

Gly Gln Phe Asn Gln Ile Asp Val Thr Ser Ser Ala Pro Gln Ser Leu
            500                 505                 510

Ile Asn Trp Asp Gly Phe Arg Thr Tyr Asn Phe Asp Val Ile Asp Gly
        515                 520                 525

Val Asn Tyr Gln Ile Asp Val Thr Gln Pro Ala Arg Tyr Asp Gly Asp
    530                 535                 540

Cys Lys Leu Ile Asn Pro Asn Ser Gln Arg Ile Val Gln Leu Thr Tyr
545                 550                 555                 560

Gln Gly Lys Pro Ile Asp Thr Gln Gln Thr Phe Leu Ile Ala Thr Asn
                565                 570                 575

Asn Tyr Arg Ala Tyr Ser Asn Lys Phe Pro Gly Thr Gly Ala Glu Phe
            580                 585                 590

Val Ala Phe Asp Ser Pro Asp Glu Asn Arg Thr Val Val Ala Asp Tyr
        595                 600                 605

Ile Ala Arg Val Ser Lys Asp Lys Gly Glu Val Thr Pro Ser Ala Asp
    610                 615                 620

Asn Asn Trp Ser Phe Ala Pro Ile Ser Ser Lys Thr Lys Leu Asp Ile
625                 630                 635                 640

Arg Phe Glu Thr Ser Pro Ser Asp Lys Ala Thr Gln Phe Ile Lys Gln
                645                 650                 655

Lys Gly Gln Tyr Pro Met Thr Arg Val Ala Ser Asp Asp Val Gly Phe
            660                 665                 670

Ala Val Tyr Arg Ile Asp Leu Gln Lys
        675                 680

<210> 86
<211> 469
<212> PRT
<213> Vibrio cholerae

<220>
<221> PEPTIDE
<222> (1)..(469)
<223> >sp|Q9KNJ2|GLNA_VIBCH Glutamine synthetase (EC
6.3.1.2) (Glutamate--ammonia ligase) - Vibrio
cholerae. (469 aa)

<400> 86
Met Ser Val Glu Asn Val Leu Ser Leu Ile Gln Glu Asn Glu Val Lys
1               5                   10                  15

Phe Val Asp Leu Arg Phe Thr Asp Thr Lys Gly Lys Glu Gln His Ile
            20                  25                  30

Ser Ile Pro Ala His Gln Ile Asp Ala Asp Phe Phe Glu Asp Gly Lys
        35                  40                  45

Met Phe Asp Gly Ser Ser Val Ala Gly Trp Lys Gly Ile Asn Glu Ser
    50                  55                  60

Asp Met Val Met Met Pro Asp Pro Ser Ser Ala Val Leu Asp Pro Phe
65                  70                  75                  80

Thr Glu Asp Ala Thr Leu Asn Ile Arg Cys Asp Ile Leu Glu Pro Ala
                85                  90                  95

Thr Met Gln Gly Tyr Asp Arg Asp Pro Arg Ser Ile Ala Lys Arg Ala
            100                 105                 110

Glu Glu Tyr Met Arg Ser Thr Gly Ile Ala Asp Thr Val Leu Val Gly
        115                 120                 125

Pro Glu Pro Glu Phe Phe Leu Phe Asp Asp Val Lys Phe Ala Thr Asn
    130                 135                 140

Met Ser Gly Ser Phe Phe Lys Ile Asp Asp Val Glu Ala Ala Trp Asn

145                 150                 155                 160

Thr Gly Thr Glu Tyr Glu Asp Gly Asn Lys Gly His Arg Pro Gly Val
                165                 170                 175

Lys Gly Gly Tyr Phe Pro Val Ala Pro Val Asp Ser Ser Gln Asp Ile
            180                 185                 190

Arg Ser Ala Met Cys Leu Ile Met Glu Glu Met Gly Leu Val Val Glu
        195                 200                 205

Ala His His His Glu Val Ala Thr Ala Gly Gln Asn Glu Ile Ala Thr
    210                 215                 220

Arg Phe Asn Thr Leu Thr Thr Lys Ala Asp Glu Ile Gln Ile Tyr Lys
225                 230                 235                 240

Tyr Val Val His Asn Val Ala His Ala Phe Gly Lys Thr Ala Thr Phe
                245                 250                 255

Met Pro Lys Pro Leu Val Gly Asp Asn Gly Ser Gly Met His Val His
            260                 265                 270

Gln Ser Leu Ala Lys Asp Gly Val Asn Leu Phe Ala Gly Asp Lys Tyr
        275                 280                 285

Gly Gly Leu Ser Glu Thr Ala Leu Tyr Tyr Ile Gly Gly Ile Ile Lys
    290                 295                 300

His Ala Arg Ala Leu Asn Ala Ile Thr Asn Pro Ser Thr Asn Ser Tyr
305                 310                 315                 320

Lys Arg Leu Val Pro His Tyr Glu Ala Pro Val Met Leu Ala Tyr Ser
                325                 330                 335

Ala Arg Asn Arg Ser Ala Ser Ile Arg Ile Pro Val Val Pro Ser Pro
            340                 345                 350

Lys Ala Arg Arg Ile Glu Val Arg Phe Pro Asp Pro Ala Ala Asn Pro
        355                 360                 365

Tyr Leu Ala Phe Ala Ala Met Leu Met Ala Gly Leu Asp Gly Ile Lys
    370                 375                 380

Asn Lys Ile His Pro Gly Glu Ala Met Asp Lys Asp Leu Tyr Asp Leu
385                 390                 395                 400

Pro Ala Glu Glu Ala Ala Glu Ile Pro Lys Val Ala Glu Ser Leu Gln
                405                 410                 415

Gln Ala Leu Gln Tyr Leu Asp Ala Asp Arg Glu Phe Leu Thr Ala Gly
            420                 425                 430

Gly Val Phe Ser Asp Asp Phe Ile Asp Ser Tyr Ile Ala Leu Lys Thr
        435                 440                 445

Lys Asp Val Glu Arg Val Asn Val Ala Val His Pro Leu Glu Phe Glu
    450                 455                 460

Leu Tyr Tyr Ser Val

465

<210> 87
<211> 123
<212> PRT
<213> Vibrio cholerae

<220>
<221> PEPTIDE
<222> (1)..(123)
<223> >tr|Q9KNZ4|Q9KNZ4_VIBCH Ribosomal protein L14 -
      Vibrio cholerae. (123 aa)

<400> 87
Met Ile Gln Met Gln Thr Met Leu Asp Ala Ala Asp Asn Ser Gly Ala
1               5                   10                  15

Arg Ser Val Met Cys Ile Lys Val Leu Gly Gly Ser His Arg Arg Tyr
            20                  25                  30

Ala His Val Gly Asp Ile Ile Lys Val Thr Val Lys Glu Ala Ile Pro
        35                  40                  45

Arg Gly Lys Val Lys Lys Gly Asp Val Leu Lys Ala Val Val Val Arg
    50                  55                  60

Thr Arg Lys Gly Val Arg Arg Pro Asp Gly Ser Val Ile Arg Phe Asp
65                  70                  75                  80

Arg Asn Ala Cys Val Leu Leu Asn Asn Asn Ser Glu Gln Pro Ile Gly
                85                  90                  95

Thr Arg Ile Phe Gly Pro Val Thr Arg Glu Leu Arg Asn Ala Lys Phe
            100                 105                 110

Met Lys Ile Val Ser Leu Ala Pro Glu Val Leu
        115                 120


<210> 88
<211> 1076
<212> PRT
<213> Vibrio cholerae

<220>
<221> PEPTIDE
<222> (1)..(1076)
<223> >sp|Q9KPH9|CARB_VIBCH Carbamoyl-phosphate synthase
      large chain (EC 6.3.5.5) (Carbamoyl- phosphate
      synthetase ammonia chain) - Vibrio cholerae. (1076
      aa)

<400> 88
Met Pro Lys Arg Thr Asp Ile Gln Ser Ile Leu Ile Leu Gly Ala Gly
1               5                   10                  15

Pro Ile Val Ile Gly Gln Ala Cys Glu Phe Asp Tyr Ser Gly Ala Gln
            20                  25                  30

Ala Cys Lys Ala Leu Arg Glu Glu Gly Tyr Arg Val Ile Leu Val Asn

35                  40                  45

Ser Asn Pro Ala Thr Ile Met Thr Asp Pro Glu Met Ala Asp Ala Thr
    50                  55                  60

Tyr Ile Glu Pro Ile His Trp Glu Val Val Arg Lys Ile Ile Glu Lys
65                  70                  75                  80

Glu Arg Pro Asp Ala Ile Leu Pro Thr Met Gly Gly Gln Thr Ala Leu
                85                  90                  95

Asn Cys Ala Leu Ala Leu Glu Lys His Gly Val Leu Ala Glu Phe Gly
            100                 105                 110

Val Glu Met Ile Gly Ala Thr Ala Asp Ala Ile Asp Lys Ala Glu Asp
        115                 120                 125

Arg Ser Arg Phe Asp Lys Ala Met Lys Ser Ile Gly Leu Glu Cys Pro
    130                 135                 140

Arg Ala Asp Thr Ala Lys Ser Met Glu Glu Ala Tyr Lys Val Leu Asp
145                 150                 155                 160

Met Val Gly Phe Pro Cys Ile Ile Arg Pro Ser Phe Thr Met Gly Gly
                165                 170                 175

Ser Gly Gly Gly Ile Ala Tyr Asn Arg Glu Glu Phe Glu Glu Ile Cys
            180                 185                 190

Thr Arg Gly Leu Asp Leu Ser Pro Thr Asn Glu Leu Leu Ile Asp Glu
        195                 200                 205

Ser Leu Ile Gly Trp Lys Glu Tyr Glu Met Glu Val Val Arg Asp Lys
    210                 215                 220

Asn Asp Asn Cys Ile Ile Val Cys Ala Ile Glu Asn Phe Asp Pro Met
225                 230                 235                 240

Gly Ile His Thr Gly Asp Ser Ile Thr Val Ala Pro Ala Gln Thr Leu
                245                 250                 255

Thr Asp Lys Glu Tyr Gln Ile Met Arg Asn Ala Ser Leu Ala Val Leu
            260                 265                 270

Arg Glu Ile Gly Val Glu Thr Gly Gly Ser Asn Val Gln Phe Gly Ile
        275                 280                 285

Asn Pro Lys Asp Gly Arg Met Val Ile Ile Glu Met Asn Pro Arg Val
    290                 295                 300

Ser Arg Ser Ser Ala Leu Ala Ser Lys Ala Thr Gly Phe Pro Ile Ala
305                 310                 315                 320

Lys Val Ala Ala Lys Leu Ala Val Gly Phe Thr Leu Asp Glu Leu Met
                325                 330                 335

Asn Asp Ile Thr Gly Gly Ala Thr Pro Ala Ser Phe Glu Pro Thr Ile
            340                 345                 350

Asp Tyr Val Val Thr Lys Ile Pro Arg Phe Asn Phe Glu Lys Phe Ala
        355                 360                 365

Gly Ala Asn Asp Arg Leu Thr Thr Gln Met Lys Ser Val Gly Glu Val
    370                 375                 380

Met Ala Ile Gly Arg Asn Gln Gln Glu Ser Leu Gln Lys Ala Leu Arg
385                 390                 395                 400

Gly Leu Glu Val Gly Ala Ala Gly Leu Asp Glu Lys Val Asp Leu Asp
                405                 410                 415

Ala Pro Asp Ala Leu Thr Lys Ile Arg Tyr Glu Leu Lys Glu Ala Gly
            420                 425                 430

Ala Glu Arg Ile Trp Tyr Ile Ala Asp Ala Phe Arg Ala Gly Met Ser
        435                 440                 445

Val Asp Gly Val Phe Asn Leu Thr Asn Ile Asp Arg Trp Phe Leu Val
    450                 455                 460

Gln Ile Glu Glu Leu Val Lys Leu Glu Ala Glu Val Lys Ala Gly Gly
465                 470                 475                 480

Phe Ala Gly Leu Asn Gln Asp Val Leu Arg Lys Met Lys Arg Lys Gly
                485                 490                 495

Phe Ser Asp Ala Arg Leu Ser Lys Leu Leu Gly Val Ser Glu Asn Glu
            500                 505                 510

Ile Arg Arg Leu Arg Asp Gln Tyr Asn Ile His Pro Val Tyr Lys Arg
        515                 520                 525

Val Asp Thr Cys Ala Ala Glu Phe Lys Ser Asp Thr Ala Tyr Met Tyr
    530                 535                 540

Ser Thr Tyr Asp Glu Glu Cys Glu Ala Asn Pro Thr Asp Lys Asp Lys
545                 550                 555                 560

Ile Met Val Leu Gly Gly Gly Pro Asn Arg Ile Gly Gln Gly Ile Glu
                565                 570                 575

Phe Asp Tyr Cys Cys Val His Ala Ala Leu Ala Leu Arg Glu Asp Gly
            580                 585                 590

Tyr Glu Thr Ile Met Val Asn Cys Asn Pro Glu Thr Val Ser Thr Asp
        595                 600                 605

Tyr Asp Thr Ser Asp Arg Leu Tyr Phe Glu Pro Val Thr Leu Glu Asp
    610                 615                 620

Val Leu Ala Ile Val Arg Val Glu Lys Pro Lys Gly Val Ile Val Gln
625                 630                 635                 640

Tyr Gly Gly Gln Thr Pro Leu Lys Leu Ala Arg Ala Leu Glu Ala Ala
                645                 650                 655

Gly Val Pro Val Ile Gly Thr Ser Pro Asp Ala Ile Asp Arg Ala Glu
            660                 665                 670

Asp Arg Glu Arg Phe Gln Gln Ala Val Gln Arg Leu Gly Leu Lys Gln
        675                 680                 685

Pro Asp Asn Ala Thr Val Thr Ala Ile Glu Gln Ala Ile Glu Lys Ser
    690                 695                 700

Arg Glu Ile Gly Phe Pro Leu Val Val Arg Pro Ser Tyr Val Leu Gly
705                 710                 715                 720

Gly Arg Ala Met Glu Ile Val Tyr Asp Glu Gln Asp Leu Arg Arg Tyr
                725                 730                 735

Phe Asn Glu Ala Val Ser Val Ser Asn Glu Ser Pro Val Leu Leu Asp
            740                 745                 750

Arg Phe Leu Asp Asp Ala Thr Glu Val Asp Val Asp Ala Ile Cys Asp
        755                 760                 765

Gly Glu Arg Val Val Ile Gly Gly Ile Met Glu His Ile Glu Gln Ala
    770                 775                 780

Gly Val His Ser Gly Asp Ser Ala Cys Ser Leu Pro Ala Tyr Thr Leu
785                 790                 795                 800

Ser Gln Glu Ile Gln Asp Lys Met Arg Glu Gln Val Glu Lys Leu Ala
                805                 810                 815

Phe Glu Leu Gly Val Arg Gly Leu Met Asn Ile Gln Phe Ala Val Lys
            820                 825                 830

Asp Asn Glu Val Tyr Leu Ile Glu Val Asn Pro Arg Ala Ala Arg Thr
        835                 840                 845

Val Pro Phe Val Ser Lys Ala Thr Gly Ala Pro Leu Ala Lys Ile Ala
    850                 855                 860

Ala Arg Val Met Val Gly Gln Thr Leu Glu Gln Gln Gly Phe Thr Lys
865                 870                 875                 880

Glu Ile Ile Pro Pro Tyr Tyr Ser Val Lys Glu Val Val Leu Pro Phe
                885                 890                 895

Asn Lys Phe Pro Gly Val Asp Pro Leu Leu Gly Pro Glu Met Arg Ser
            900                 905                 910

Thr Gly Glu Val Met Gly Val Gly Ala Thr Phe Ala Glu Ala Tyr Ala
        915                 920                 925

Lys Ala Glu Leu Gly Cys Gly Ser Val Tyr Pro Glu Gly Gly Arg Ala
    930                 935                 940

Leu Leu Ser Val Arg Glu Gly Asp Lys Gln Arg Val Val Asp Leu Ala
945                 950                 955                 960

Ser Lys Leu Val Lys Leu Gly Tyr Gln Leu Asp Ala Thr His Gly Thr
                965                 970                 975

Ala Val Ile Leu Gly Glu Ala Gly Ile Asn Pro Arg Leu Val Asn Lys
            980                 985                 990

Val His Glu Gly Arg Pro His Ile Leu Asp Arg Ile Lys Asn His Glu
        995                 1000                1005

Tyr Thr Tyr Ile Val Asn Thr Ala Ser Gly Arg Gln Ala Ile Glu Asp

1010                1015                1020

Ser Lys Val Leu Arg Arg Gly Ala Leu Ala His Lys Val Asn Tyr Thr
1025                1030                1035                1040

Thr Thr Leu Asn Ala Ala Phe Ala Thr Cys Met Ser His Thr Ala Asp
                1045                1050                1055

Ala Lys Ala Ser Val Thr Ser Val Gln Glu Leu His Ala Arg Val Lys
            1060                1065                1070

Ala Asn Gln Ala
        1075


<210> 89
<211> 695
<212> PRT
<213> Vibrio cholerae

<220>
<221> PEPTIDE
<222> (1)..(695)
<223> >sp|Q9KPM5|EFG2_VIBCH Elongation factor G 2 (EF-G
      2) - Vibrio cholerae. (695 aa)

<400> 89
Met Ala Asp Leu Ser Leu Tyr Arg Asn Ile Gly Ile Phe Ala His Val
1               5                   10                  15

Asp Ala Gly Lys Thr Thr Thr Thr Glu Arg Ile Leu Lys Leu Thr Gly
            20                  25                  30

Lys Ile His Arg Leu Gly Glu Val His Asp Gly Ala Ser Thr Met Asp
        35                  40                  45

Phe Met Glu Gln Glu Ala Glu Arg Gly Ile Thr Ile Gln Ser Ala Ala
    50                  55                  60

Thr Thr Cys Phe Trp Lys Gly His Arg Phe Asn Val Ile Asp Thr Pro
65                  70                  75                  80

Gly His Val Asp Phe Thr Val Glu Val Tyr Arg Ser Leu Lys Val Leu
                85                  90                  95

Asp Gly Gly Ile Gly Val Phe Cys Gly Ser Gly Gly Val Glu Pro Gln
            100                 105                 110

Ser Glu Thr Asn Trp Arg Tyr Ala Asn Glu Ser Glu Val Ser Arg Leu
        115                 120                 125

Ile Phe Val Asn Lys Leu Asp Arg Met Gly Ala Asp Phe Phe Arg Val
    130                 135                 140

Val Glu Gln Val Lys Lys Val Leu Gly Ala Asn Pro Leu Val Met Thr
145                 150                 155                 160

Leu Pro Ile Gly Arg Glu Asp Glu Phe Val Gly Val Val Asp Val Leu
                165                 170                 175

Thr Arg Gln Ala Tyr Val Trp Asp Asp Ser Gly Leu Pro Glu Asn Phe

            180                 185                 190

Glu Val Lys Glu Val Pro Ala Asp Met Val Asp Gln Val Glu Glu Tyr
        195                 200                 205

Arg Glu Met Met Ile Glu Thr Ala Val Glu Gln Asp Asp Glu Leu Met
    210                 215                 220

Met Ala Tyr Met Glu Gly Glu Glu Pro Thr Val Glu Gln Ile Lys Ala
225                 230                 235                 240

Cys Ile Arg Lys Gly Thr Arg Asp Leu Ala Phe Phe Pro Thr Phe Cys
                245                 250                 255

Gly Ser Ala Phe Lys Asn Lys Gly Met Gln Leu Val Leu Asp Ala Val
            260                 265                 270

Val Asp Tyr Leu Pro Ser Pro Thr Glu Val Glu Pro Gln Pro Leu Thr
        275                 280                 285

Asp Pro Ala Thr Gly Glu Pro Thr Gly Glu Val Ala Thr Val Ser Val
    290                 295                 300

Asp Ala Pro Leu Lys Ala Leu Ala Phe Lys Ile Met Asp Asp Arg Phe
305                 310                 315                 320

Gly Ala Leu Thr Phe Val Arg Ile Tyr Ser Gly Lys Ile Lys Lys Gly
                325                 330                 335

Asp Thr Ile Leu Asn Ser Ala Thr Gly Lys Thr Glu Arg Ile Gly Arg
            340                 345                 350

Met Val Glu Met His Ala Asn Asp Arg Asn Glu Val Glu Ser Ala Gln
        355                 360                 365

Ala Ser Asp Ile Ile Ala Ile Val Gly Met Lys Asn Val Gln Thr Gly
    370                 375                 380

His Thr Leu Cys Asp Pro Lys His Glu Cys Thr Leu Glu Pro Met Ile
385                 390                 395                 400

Phe Pro Thr Pro Val Ile Ser Ile Ala Val Lys Pro Lys Asp Lys Asn
                405                 410                 415

Gly Ser Glu Lys Met Gly Ile Ala Ile Gly Lys Met Val Ala Glu Asp
            420                 425                 430

Pro Ser Phe Gln Val Glu Thr Asp Glu Asp Ser Gly Glu Thr Ile Leu
        435                 440                 445

Lys Gly Met Gly Glu Leu His Leu Asp Ile Lys Val Asp Ile Leu Lys
    450                 455                 460

Arg Thr Tyr Gly Val Glu Leu Glu Val Gly Ala Pro Gln Val Ala Tyr
465                 470                 475                 480

Arg Glu Thr Ile Thr Lys Ala Val Glu Asp Ser Tyr Thr His Lys Lys
                485                 490                 495

Gln Ser Gly Gly Ser Gly Gln Phe Gly Lys Ile Asp Tyr Arg Ile Arg
            500                 505                 510

Pro Gly Glu Gln Asn Ser Gly Phe Thr Phe Lys Ser Thr Val Val Gly
        515                 520                 525

Gly Asn Val Pro Lys Glu Phe Trp Pro Ala Val Glu Lys Gly Phe Lys
    530                 535                 540

Ser Met Met Asp Thr Gly Thr Leu Ala Gly Phe Pro Val Leu Asp Val
545                 550                 555                 560

Glu Val Glu Leu Phe Asp Gly Gly Phe His Ala Val Asp Ser Ser Ala
                565                 570                 575

Ile Ala Phe Glu Ile Ala Ala Lys Gly Ala Phe Arg Gln Ser Ile Pro
                580                 585                 590

Lys Ala Ala Pro Gln Leu Leu Glu Pro Ile Met Lys Val Asp Val Phe
        595                 600                 605

Thr Pro Glu Asp His Val Gly Asp Val Ile Gly Asp Leu Asn Arg Arg
    610                 615                 620

Arg Gly Met Ile Lys Asp Gln Glu Met Gly Leu Thr Gly Val Arg Val
625                 630                 635                 640

Lys Ala Asp Val Pro Leu Ser Glu Met Phe Gly Tyr Ile Gly Ser Leu
                645                 650                 655

Arg Thr Met Thr Ser Gly Arg Gly Gln Phe Ser Met Glu Phe Ser His
                660                 665                 670

Tyr Ala Pro Cys Pro Asn Asn Val Ala Glu Gln Val Ile Ala Glu Val
        675                 680                 685

Lys Glu Arg Asn Ala Lys Lys
    690                 695


<210> 90
<211> 898
<212> PRT
<213> Vibrio cholerae

<220>
<221> PEPTIDE
<222> (1)..(898)
<223> >sp|Q9KU80|IF2_VIBCH Translation initiation factor
      IF-2 - Vibrio cholerae. (898 aa)

<400> 90
Met Thr Gln Ile Thr Val Lys Ala Leu Ser Glu Glu Ile Gly Thr Pro
1               5                   10                  15

Val Asp Arg Leu Leu Glu Gln Leu Ala Asp Ala Gly Met Asn Lys Ala
            20                  25                  30

Val Ala Asp His Val Ser Glu Asp Glu Lys Gln Lys Leu Leu Ala His
        35                  40                  45

Leu Arg Lys Glu His Gly Asp Ala Thr Gly Ser Glu Pro Thr Arg Leu
    50                  55                  60

Thr Leu Gln Arg Lys Thr Arg Ser Thr Leu Ser Val Asn Ala Gly Gly
65                  70                  75                  80

Gly Lys Ser Lys Asn Val Gln Val Glu Val Arg Lys Lys Arg Thr Tyr
                85                  90                  95

Val Lys Arg Ser Ser Val Glu Asp Glu Ala Thr Arg Glu Ala Glu Glu
            100                 105                 110

Ala Ala Met Arg Ala Ala Glu Glu Gln Ala Lys Arg Glu Ala Glu Glu
        115                 120                 125

Ala Ala Gln Arg Ala Ala Glu Glu Lys Ala Lys Arg Glu Ala Glu Glu
    130                 135                 140

Ala Ala Lys Arg Glu Ala Glu Ala Lys Arg Met Ala Glu Glu Lys Ala
145                 150                 155                 160

Lys Arg Glu Thr Gln Ala Ala Thr Gln Pro Arg Ser Asp Glu Glu Lys
                165                 170                 175

Leu Lys Gln Glu Ala Ala Arg Lys Glu Ala Glu Ala Leu Lys Arg Arg
            180                 185                 190

Gln Glu Glu Glu Ala Arg Arg Lys Ala Glu Glu Glu Ser Arg Arg Gln
        195                 200                 205

Leu Glu Lys Val Arg Glu Leu Ala Glu Lys Asn Gly Glu Arg Trp Ser
    210                 215                 220

Ala Asp Lys Glu Thr Val Gly Asp Met Gln Glu Asn Thr Asp Tyr His
225                 230                 235                 240

Val Thr Thr Ser Arg Tyr Ala Arg Glu Ala Glu Asp Glu Ala Asp Leu
                245                 250                 255

His Glu Glu Gly Ala Arg Arg Arg Ser Thr Lys Ala Asn Lys Arg Lys
            260                 265                 270

Met Ser Ser Arg Asp Asp Asn Gln Glu Arg Asp Ser Arg Pro Arg Gly
        275                 280                 285

Gly Lys Ala Gly Arg Lys Gly Arg Ile Asn Lys Pro Met Ser Met Gln
    290                 295                 300

His Gly Phe Asp Lys Thr Ala Val Val Ala Lys Ala Asp Val Val Val
305                 310                 315                 320

Gly Glu Thr Ile Val Val Ser Glu Leu Ala Gln Lys Met Ser Val Lys
                325                 330                 335

Ala Thr Glu Val Ile Lys Val Met Met Lys Met Gly Ala Met Ala Thr
            340                 345                 350

Ile Asn Gln Val Ile Asp Gln Glu Thr Ala Gln Leu Val Ala Glu Glu
        355                 360                 365

Met Gly His Lys Val Val Leu Arg Lys Glu Asn Glu Leu Glu Glu Ala
    370                 375                 380

Ile Leu Ser Asp Arg Asp Asp Lys Phe Glu Glu Val Ser Arg Ala Pro
385                 390                 395                 400

Val Val Thr Ile Met Gly His Val Asp His Gly Lys Thr Ser Thr Leu
                405                 410                 415

Asp Tyr Ile Arg Arg Thr His Val Ala Ser Gly Glu Ala Gly Gly Ile
            420                 425                 430

Thr Gln His Ile Gly Ala Tyr His Val Glu Thr Pro Asn Gly Met Ile
        435                 440                 445

Thr Phe Leu Asp Thr Pro Gly His Ala Ala Phe Thr Ala Met Arg Ala
    450                 455                 460

Arg Gly Ala Gln Ala Thr Asp Ile Val Val Leu Val Val Ala Ala Asp
465                 470                 475                 480

Asp Gly Val Met Pro Gln Thr Val Glu Ala Ile Gln His Ala Lys Ala
                485                 490                 495

Ala Gly Val Pro Leu Ile Val Ala Val Asn Lys Ile Asp Lys Asp Thr
            500                 505                 510

Ala Asn Pro Asp Asn Val Lys Thr Glu Leu Ser Gln Tyr Asn Val Met
        515                 520                 525

Pro Glu Glu Trp Gly Gly Asp Asn Met Phe Val His Ile Ser Ala Lys
    530                 535                 540

Gln Gly Thr Asn Ile Asp Gly Leu Leu Glu Ala Ile Leu Leu Gln Ala
545                 550                 555                 560

Glu Val Leu Glu Leu Lys Ala Val Lys Gln Gly Met Ala Ser Gly Val
                565                 570                 575

Val Ile Glu Ser Arg Leu Asp Lys Gly Arg Gly Pro Val Ala Thr Val
            580                 585                 590

Leu Val Gln Ser Gly Thr Leu Arg Lys Gly Asp Ile Val Leu Cys Gly
        595                 600                 605

Gln Glu Tyr Gly Arg Val Arg Ala Met Arg Asp Glu Val Gly Asn Glu
    610                 615                 620

Val Glu Glu Ala Gly Pro Ser Ile Pro Val Glu Ile Leu Gly Leu Ser
625                 630                 635                 640

Gly Val Pro Ala Ala Gly Asp Glu Ala Thr Val Val Arg Asp Glu Arg
                645                 650                 655

Lys Ala Arg Glu Val Ala Asn Tyr Arg Ala Gly Lys Phe Arg Glu Val
            660                 665                 670

Lys Leu Ala Arg Gln Gln Lys Ser Lys Leu Glu Asn Met Phe Ser Asn
        675                 680                 685

Met Thr Ala Gly Asp Val Ala Glu Leu Asn Ile Val Leu Lys Ala Asp
    690                 695                 700

Val Gln Gly Ser Val Glu Ala Ile Ala Asp Ser Leu Thr Lys Leu Ser

705                 710                 715                 720

Thr Asp Glu Val Lys Val Asn Ile Val Gly Ser Gly Val Gly Gly Ile
                725                 730                 735

Thr Glu Thr Asp Ala Val Leu Ala Ala Ala Ser Asn Ala Ile Val Val
            740                 745                 750

Gly Phe Asn Val Arg Ala Asp Ala Ser Ala Arg Arg Met Ile Glu Ala
        755                 760                 765

Glu Asn Ile Asp Leu Arg Tyr Tyr Ser Ile Ile Tyr Gln Leu Ile Asp
    770                 775                 780

Glu Val Lys Gln Ala Met Ser Gly Met Leu Ser Pro Glu Phe Lys Gln
785                 790                 795                 800

Glu Ile Ile Gly Leu Ala Glu Val Arg Asp Val Phe Lys Ser Pro Lys
                805                 810                 815

Leu Gly Ala Ile Ala Gly Cys Met Val Thr Glu Gly Val Ile Lys Arg
            820                 825                 830

Asn Ala Pro Ile Arg Val Leu Arg Asp Asn Val Val Ile Tyr Glu Gly
        835                 840                 845

Glu Leu Glu Ser Leu Arg Arg Phe Lys Asp Asp Val Ala Glu Val Lys
    850                 855                 860

Asn Gly Tyr Glu Cys Gly Ile Gly Val Lys Asn Tyr Asn Asp Val Arg
865                 870                 875                 880

Val Gly Asp Gln Ile Glu Val Phe Glu Thr Ile Glu Ile Gln Arg Thr
                885                 890                 895

Ile Asp

<210> 91
<211> 256
<212> PRT
<213> Vibrio cholerae

<220>
<221> PEPTIDE
<222> (1)..(256)
<223> >tr|Q9KTQ6|Q9KTQ6_VIBCH Accessory colonization
      factor AcfC - Vibrio cholerae. (256 aa)

<400> 91
Met Tyr Phe Met Lys Ser Lys Asn Arg Phe Leu Leu Ile Ser Leu Leu
1               5                   10                  15

Ser Phe Ser Thr Ser Val Phe Ala Asp Val Asn Leu Tyr Gly Pro Gly
            20                  25                  30

Gly Pro His Val Pro Leu Ile Lys Val Ala Glu Ser Phe Glu Lys Ser
        35                  40                  45

Gln Ser Lys Arg Val Asn Ile Thr Phe Gly Pro Gln Ala Thr Trp Asn

50                  55                  60

Asp Lys Ala Lys Lys Asn Ala Asp Ile Leu Phe Gly Ala Ser Glu His
65                  70                  75                  80

Ser Ala Leu Ala Ile Ala Glu Gly His Ser Glu Arg Phe Ser Lys Phe
                85                  90                  95

Asn Ile His Pro Val Phe Met Arg Glu Ala Ile Ile Leu Val Lys Lys
            100                 105                 110

Gly Asn Pro Lys Asn Ile Lys Gly Met Ala Asp Leu Leu Lys Pro Gly
        115                 120                 125

Ile Gly Ile Val Val Asn Asp Gly Ala Gly Val Ser Asn Thr Ser Gly
    130                 135                 140

Thr Ala Val Trp Glu Asp Ser Val Gly Arg Met Lys Asn Val Glu Lys
145                 150                 155                 160

Leu Gln Ala Phe Arg Ser Asn Ile His Val Phe Ala Pro Asn Ser Gly
                165                 170                 175

Ser Ala Arg Lys Ala Phe Val Asp Gly Glu Asp Ile Asp Ala Trp Ile
            180                 185                 190

Thr Trp Val Asp Trp Ala Ile Ala Asn Pro Thr Ile Gly Asp Met Val
        195                 200                 205

Arg Met Glu Asp Glu Tyr Arg Ile Tyr Arg Asp Phe Asn Val Val Leu
    210                 215                 220

Ala Lys Asn Pro Ser Ser Glu Ala Ile Asp Phe Phe Asp Tyr Leu Thr
225                 230                 235                 240

Lys Ser Lys Asp Ala Glu Ala Ile Phe Gln His Tyr Gly Trp Phe Lys
                245                 250                 255

<210> 92
<211> 588
<212> PRT
<213> Vibrio cholerae

<220>
<221> PEPTIDE
<222> (1)..(588)
<223> >tr|Q9KQB1|Q9KQB1_VIBCH Succinate dehydrogenase,
      flavoprotein subunit - Vibrio cholerae. (588 aa)

<400> 92
Met Thr Ile Pro Val Arg Ser Phe Asp Ala Val Val Ile Gly Ala Gly
1               5                   10                  15

Gly Ala Gly Met Arg Ala Ala Leu Gln Ile Ser Glu Gln Gly Leu Thr
            20                  25                  30

Cys Ala Leu Leu Ser Lys Val Phe Pro Thr Arg Ser His Thr Val Ser
        35                  40                  45

Ala Gln Gly Gly Ile Thr Val Ala Leu Gly Asn Ser His Lys Asp Asp
    50                  55                  60

Trp Gln Trp His Met Tyr Asp Thr Val Lys Gly Ser Asp Tyr Ile Gly
65                  70                  75                  80

Asp Gln Asn Ala Ile Glu Tyr Met Cys Lys Asn Gly Pro Glu Ser Val
                85                  90                  95

Ile Glu Leu Glu Arg Met Gly Leu Pro Phe Ser Arg Phe Glu Asn Gly
            100                 105                 110

Thr Ile Tyr Gln Arg Pro Phe Gly Gly Gln Ser Lys Glu Phe Gly Gly
        115                 120                 125

Glu Gln Ala Ala Arg Thr Ala Ala Ala Ala Asp Arg Thr Gly His Ala
    130                 135                 140

Leu Leu His Thr Leu Tyr Gln Gln Asn Val Lys His Lys Thr Thr Ile
145                 150                 155                 160

Phe Ser Glu Trp Tyr Ala Leu Asp Leu Val Lys Asn Gln Asp Gly Ala
                165                 170                 175

Val Leu Gly Cys Thr Ala Ile Cys Met Glu Thr Gly Glu Ile Cys Tyr
            180                 185                 190

Phe Lys Ala Lys Ala Thr Val Leu Ala Thr Gly Gly Ala Gly Arg Ile
        195                 200                 205

Tyr Ala Ser Thr Thr Asn Ala His Ile Asn Thr Gly Asp Gly Val Gly
    210                 215                 220

Met Ala Leu Arg Ala Gly Val Pro Met Gln Asp Met Glu Met Trp Gln
225                 230                 235                 240

Phe His Pro Thr Gly Ile Ala Gly Ala Gly Val Leu Val Thr Glu Gly
                245                 250                 255

Cys Arg Gly Glu Gly Gly Tyr Leu Leu Asn Lys Asp Gly Glu Arg Phe
            260                 265                 270

Met Glu Arg Tyr Ala Pro Asn Ala Lys Asp Leu Ala Gly Arg Asp Val
        275                 280                 285

Val Ala Arg Ser Met Met Ile Glu Ile Arg Glu Gly Arg Gly Cys Asp
    290                 295                 300

Gly Pro Trp Gly Pro His Ile Lys Leu Lys Leu Asp His Leu Gly Arg
305                 310                 315                 320

Asp Val Leu Glu Ser Arg Leu Pro Gly Ile Cys Glu Leu Ser Arg Thr
                325                 330                 335

Phe Ala His Val Asp Pro Val Lys Glu Pro Ile Pro Val Ile Pro Thr
            340                 345                 350

Cys His Tyr Met Met Gly Gly Val Pro Thr Gln Val Ser Gly Gln Ala
        355                 360                 365

Ile Lys Gln Asp Ser His Gly Lys Asp Val Glu Val Gln Gly Leu Phe
    370                 375                 380

Ala Cys Gly Glu Ile Ala Ser Val Ser Val His Gly Ala Asn Arg Leu
385                 390                 395                 400

Gly Gly Asn Ser Leu Leu Asp Leu Val Val Phe Gly Arg Ala Thr Gly
                405                 410                 415

Leu His Leu Gly Glu Thr Leu Arg Ala Gln Ala Glu Ala Arg Pro Ala
            420                 425                 430

Thr Ala Ser Asp Ile Glu Ala Ser Leu Ala Arg Thr Met Arg Trp Glu
        435                 440                 445

Asn Ser Lys Gly Gly Glu Asp Pro Val Val Ile Arg Lys Asp Leu Gln
    450                 455                 460

Arg Cys Met Gln Asn Asn Phe Ser Val Phe Arg Glu Gly Lys Ala Met
465                 470                 475                 480

Ala Glu Gly Leu Glu Glu Leu Lys Val Ile Arg Glu Arg Leu Lys Asn
                485                 490                 495

Ala His Leu Ala Asp Lys Ser Thr Glu Phe Asn Thr Gln Arg Ile Glu
            500                 505                 510

Cys Leu Glu Leu Asp Asn Leu Met Glu Thr Ala Phe Ser Thr Ala Val
        515                 520                 525

Ala Ala Asn Tyr Arg Thr Glu Ser Arg Gly Ala His Ala Arg Phe Asp
    530                 535                 540

Tyr Pro Glu Arg Asp Asp Glu Asn Trp Leu Cys His Ser Ile Tyr Asn
545                 550                 555                 560

Pro Glu Thr Glu Gln Met Ser Lys Arg Glu Val Asn Met Thr Pro Val
                565                 570                 575

Tyr Arg Asp Pro Phe Pro Pro Lys Val Arg Thr Tyr
            580                 585

<210> 93
<211> 336
<212> PRT
<213> Vibrio cholerae

<220>
<221> PEPTIDE
<222> (1)..(336)
<223> >tr|Q9KP35|Q9KP35_VIBCH
      Fructose-1,6-bisphosphatase - Vibrio cholerae.
      (336 aa)

<400> 93
Met Gln Lys Met Arg Thr Leu Gly Glu Phe Ile Val Glu Lys Gln His

1               5                   10                  15

Asp Phe Pro His Ala Ser Gly Glu Leu Ser Ser Leu Leu Ala Ser Ile
                20                  25                  30

Arg Leu Ala Ala Lys Ile Val Asn Arg Glu Ile Asn Lys Ala Gly Leu
            35                  40                  45

Ala Asp Ile Ile Gly Ala Ser Gly Asn Asp Asn Ile Gln Gly Glu Glu
    50                  55                  60

Gln Gln Lys Leu Asp Leu Tyr Ala Asn Glu Lys Phe Lys Ala Ala Leu
65                  70                  75                  80

Glu Ala Arg Asp Gln Val Cys Gly Val Ala Ser Glu Glu Glu Asp Glu
                85                  90                  95

Ala Val Ala Phe Ser Lys Glu Leu Asn Lys Asn Ala Lys Tyr Val Val
            100                 105                 110

Leu Met Asp Pro Leu Asp Gly Ser Ser Asn Ile Asp Val Asn Val Ser
        115                 120                 125

Val Gly Thr Ile Phe Ser Ile Tyr Arg Arg Val Ser Pro Val Gly Thr
    130                 135                 140

Pro Pro Thr Gln Glu Asp Phe Leu Gln Pro Gly Asn Lys Gln Val Ala
145                 150                 155                 160

Ala Gly Tyr Val Ile Tyr Gly Ser Ser Thr Met Leu Val Tyr Thr Thr
                165                 170                 175

Gly Asn Gly Val Asn Gly Phe Thr Tyr Asp Pro Ser Leu Gly Thr Phe
            180                 185                 190

Tyr Leu Ser His Glu Asn Met Arg Ile Pro Glu Asn Gly Lys Ile Tyr
        195                 200                 205

Ser Ile Asn Glu Gly Asn Tyr Ile Arg Phe Pro Thr Gly Val Lys Lys
    210                 215                 220

Tyr Ile Lys Phe Cys Gln Glu Asn Val Pro Glu Glu Gly Arg Pro Tyr
225                 230                 235                 240

Thr Ser Arg Tyr Ile Gly Ser Leu Val Ala Asp Phe His Arg Asn Leu
                245                 250                 255

Leu Lys Gly Gly Ile Tyr Leu Tyr Pro Ser Thr Gln Ser His Pro Asn
            260                 265                 270

Gly Lys Leu Arg Leu Leu Tyr Glu Cys Asn Pro Met Ala Phe Leu Ile
        275                 280                 285

Glu Gln Ala Gly Gly Leu Ala Ser Asp Gly Ala Arg Arg Ile Met Asp
    290                 295                 300

Ile Lys Pro Thr Glu Leu His Gln Arg Val Pro Phe Phe Val Gly Ser
305                 310                 315                 320

Lys Asn Met Val His Lys Val Glu Thr Phe Leu Glu Thr Tyr Pro Asp
                325                 330                 335

<210> 94
<211> 795
<212> PRT
<213> Vibrio cholerae

<220>
<221> PEPTIDE
<222> (1)..(795)
<223> >sp|Q9KSN6|SYFB_VIBCH Phenylalanyl-tRNA synthetase
      beta chain (EC 6.1.1.20) (Phenylalanine-- tRNA
      ligase beta chain) (PheRS) - Vibrio cholerae. (795
      aa)

<400> 94
Met Lys Phe Ser Glu Ser Trp Leu Arg Glu Trp Val Asn Pro Ala Ile
1               5                   10                  15

Thr Thr Asp Glu Leu Thr His Gln Ile Thr Met Ala Gly Leu Glu Val
            20                  25                  30

Asp Asp Val Leu Ala Val Ala Gly Val Phe Asp Gly Val Lys Val Gly
        35                  40                  45

His Val Val Glu Cys Ala Gln His Pro Asp Ala Asp Lys Leu Arg Val
    50                  55                  60

Thr Lys Val Asp Val Gly Glu Glu Glu Leu Leu Asp Ile Val Cys Gly
65                  70                  75                  80

Ala Ala Asn Cys Arg Gln Gly Leu Lys Val Ala Val Ala Thr Val Gly
                85                  90                  95

Ala Thr Leu Pro Gly Asp Phe Lys Ile Lys Lys Ala Lys Leu Arg Gly
            100                 105                 110

Gln Pro Ser His Gly Met Leu Cys Ser Phe Ser Glu Leu Gly Ile Asp
        115                 120                 125

Val Glu Ser Asn Gly Ile Met Glu Leu Ala Glu Asn Ala Pro Ile Gly
    130                 135                 140

Met Asp Phe Arg Asp Phe Leu Ser Leu Asn Asp Val Thr Ile Asp Val
145                 150                 155                 160

Asp Leu Thr Ser Asn Arg Ala Asp Cys Phe Ser Ile Arg Gly Leu Ala
                165                 170                 175

Arg Glu Val Gly Val Leu Asn Arg Ala Asp Val Thr Ala Pro Ala Val
            180                 185                 190

Asn Ala Ile Val Ala Thr Ile Asn Asp Thr Ile Ser Ile Asp Val Lys
        195                 200                 205

Ala Pro Ala Ala Cys Pro Arg Tyr Leu Gly Arg Ile Val Lys Asn Val
    210                 215                 220

Asn Val Gln Ala Gln Thr Pro Leu Trp Met Gln Glu Lys Leu Arg Arg
225                 230                 235                 240

Cys Gly Ile Arg Ser Ile Asp Pro Val Val Asp Ile Thr Asn Phe Val
                245                 250                 255

Met Leu Glu Gln Gly Gln Pro Met His Ala Phe Asp Leu Ala Lys Ile
            260                 265                 270

Glu Gly Gly Ile Val Val Arg Leu Ala Glu Gln Asp Glu Lys Leu Thr
        275                 280                 285

Leu Leu Asp Gly Ser Glu Ala Lys Leu Asn Ala Asp Thr Leu Val Ile
    290                 295                 300

Ala Asp Gln Gln Lys Ala Leu Ala Ile Ala Gly Val Phe Gly Gly Glu
305                 310                 315                 320

His Ser Gly Val Ser Thr Asp Thr Lys Asp Val Leu Leu Glu Cys Ala
                325                 330                 335

Phe Phe Ala Pro Asp His Ile Arg Gly Arg Ala Arg Ser Tyr Gly Leu
            340                 345                 350

His Thr Asp Ser Ser Met Arg Phe Glu Arg Gly Val Asp Tyr Ala Leu
        355                 360                 365

Gln His Ala Ala Met Glu Arg Ala Thr Gln Leu Leu Val Glu Ile Cys
    370                 375                 380

Gly Gly Asp Val Ala Pro Val Val Ala Ala Glu Ser Ala Ala Asp Leu
385                 390                 395                 400

Pro Lys Pro Asn Gln Val Ala Leu Arg Arg Ser Lys Leu Asp Lys Leu
                405                 410                 415

Leu Gly His Ala Ile Pro Asp Ala Asp Val Val Glu Ile Leu Glu Arg
            420                 425                 430

Leu Gly Met Gln Val Glu Thr Thr Ala Glu Gly Trp Gln Ala Thr Ala
        435                 440                 445

Pro Thr Trp Arg Phe Asp Ile Ala Ile Glu Gln Asp Leu Val Glu Glu
    450                 455                 460

Val Gly Arg Ile Tyr Gly Tyr Asn Asn Ile Pro Asn Gln Ala Pro Val
465                 470                 475                 480

Ala Ala Leu Asn Met Asn Leu His Asn Glu Ala Lys Leu Pro Leu Lys
                485                 490                 495

Arg Val Arg Asp Leu Leu Val Asp Arg Gly Tyr Gln Glu Ala Ile Thr
            500                 505                 510

Tyr Ser Phe Val Glu Pro Glu Gln Gln Lys Leu Val Val Pro Gly Val
        515                 520                 525

Asp Ala Leu Ile Leu Pro Asn Pro Ile Ser Ala Glu Met Ser Ala Met
    530                 535                 540

Arg Leu Ser Leu Ile Gln Gly Leu Leu Asn Thr Val Val His Asn Gln

545                 550                 555                 560

Lys Arg Gln Gln Pro Arg Val Arg Leu Phe Glu Tyr Gly Leu Arg Phe

                565                 570                 575

Ile Pro Asp Ala Ala Ala Glu Asn Gly Met Arg Gln Glu Pro Met Leu
            580                 585                 590

Ala Gly Val Ile Ser Gly Ala Arg Gly Glu Glu His Trp Asn Met Glu
        595                 600                 605

Thr Ala Thr Val Asp Phe Phe Asp Met Lys Gly Asp Leu Glu Ala Val
    610                 615                 620

Leu Glu Leu Thr Ala Lys Gly Lys Ala Tyr Ser Phe Ala Ala Thr Lys
625                 630                 635                 640

His Pro Ala Leu His Pro Gly Gln Ala Ala Ala Ile Met Val Asp Gly
                645                 650                 655

Lys Ala Ile Gly Val Ile Gly Thr Val His Pro Glu Leu Glu Arg Lys
            660                 665                 670

Phe Gly Leu Asn Gly Arg Thr Ile Val Phe Glu Ile Glu Trp Asn Ala
        675                 680                 685

Ile Asn Thr Arg Val Ile Pro Glu Ala Ala Ala Ile Ser Lys Phe Pro
    690                 695                 700

Ala Asn Arg Arg Asp Ile Ala Leu Val Val Asp Gly Asn Ile Ala Ser
705                 710                 715                 720

Gly Asp Ile Val Glu Ala Cys Arg Val Ala Gly Gly Glu Leu Leu Lys
                725                 730                 735

Asp Ala Lys Leu Phe Asp Val Tyr Val Gly Lys Gly Val Glu Glu Gly
            740                 745                 750

Lys Lys Ser Leu Ala Ile Ala Leu Thr Leu Gln Ser Val Glu Arg Thr
        755                 760                 765

Leu Glu Glu Ala Asp Ile Ala Ala Ala Val Glu Ala Ile Val Gln Ala
    770                 775                 780

Val Ser Ala Gln Phe Gly Ala Ala Leu Arg Asp
785                 790                 795